# EUROPEAN PATENT SPECIFICATION

(11) **EP 4 168 398 B1**
(45) Date of publication and mention of the grant of the patent: **03.06.2026**
(21) Application number: 21829711.7
(22) Date of filing: 21.06.2021
(51) Int. Cl.: C07D 401/14, C07D 403/14, A61K 31/444, A61K 31/44, A61P 29/00, A61P 37/00, C07D 401/04, C07D 213/75, C07D 405/14, C07D 409/14, C07D 413/14, C07D 417/14, C07D 487/04, C07D 491/147, C07D 493/04, C07D 495/04, C07D 497/04, C07D 498/04, C07D 498/10, C07D 513/14

(54) **TYK-2 INHIBITOR**
TYK-2-INHIBITOR
INHIBITEUR DE TYK -2

(30) Priority: 22.06.2020 WO PCT/CN2020/097557; 30.09.2020 WO PCT/CN2020/119750; 14.05.2021 WO PCT/CN2021/093815
(43) Date of publication of application: 26.04.2023
(73) Proprietor: BeOne Medicines I GmbH, 4051 Basel (CH)
(72) Inventor: GUO, Yunhang, Beijing 102206 (CN); WANG, Zhiwei, Beijing 102206 (CN); WANG, Qiuwen, Beijing 102206 (CN)
(74) Representative: Mewburn Ellis LLP
(86) International application number: PCT/CN2021/101282
(87) International publication number: WO 2021/259208

(56) References cited:
- WO-A1-2012/117059
- WO-A1-2018/071794
- WO-A1-2020/086616

## Description

### FIELD OF THE DISCLOSURE

Disclosed herein is a compound of Formula (I) for inhibiting TYK2 and treating a disease associated with the undesirable tyk-2 activity (tyk-2 related diseases), a method of using the compounds disclosed herein for treating inflammatory or autoimmune diseases, and a pharmaceutical composition comprising the same.

### BACKGROUND OF THE DISCLOSURE

Janus family of kinases includes JAK1, JAK2, JAK3, and tyrosine kinase 2 (Tyk2) and are nonreceptor tyrosine kinases that bind to the intracellular portion of cell surface cytokine receptors. In response to the stimulation of these receptors, the Janus kinases phosphorylate signal transducer and activator of transcription (STAT) proteins, which then dimerize, translocate to the nucleus, and activate gene transcription. Tyrosine kinase 2 (Tyk2) is a member of the Janus kinase (JAK)family of nonreceptor tyrosine kinases and has been shown to be critical in regulating the signal transduction cascade downstream of receptors for IL-12, IL-23 and type I interferons in both mice (Ishizaki, M. et al., "Involvement of Tyrosine Kinase-2 in Both the IL-12/TH1 and IL-23/TH17 Axes in vivo", J. Immunol., 187: 181-189 (2011); Prchal-Murphyl, M. et al., "TYK2 kinase activity is required for functional type I interferon responses in vivo", PloS one, 7:e39141 (2012)) and humans (Minegishi, Y. et al., "Human tyrosine kinase 2 deficiency reveals its requisite roles in multiple cytokine signals involved in innate acquired immunity", Immunity, 25:745-755 (2006)). Tyk2 mediates the receptor-induced phosphorylation of members of the STAT family of transcription factors, an essential signal that leads to the dimerization of STAT proteins and the transcription of STAT-dependent pro-inflammatory genes. Tyk2-deficient mice are resistant to experimental model of colitis, psoriasis and multiple sclerosis, demonstrating the importance of Tyk2-mediated signaling in autoimmunity and related disorders (Ishizaki, M. et al., "Involvement of Tyrosine Kinase-2 in Both the IL-12/TH1 and IL-23/TH17 Axes in vivo", J. Immunol., 187: 181-189 (2011); Oyamada, A. et al., "Tyrosine kinase 2 plays critical roles in the pathogenic CD4 T cell responses for the development of experimental autoimmune encephalomyelitis", J. Immunol., 2009, 183, 7539-7546).

To date, most of the known small molecule JAK inhibitors that have progressed into development are active site-directed inhibitors that bind to the adenosine triphosphate (ATP) site of the catalytic domain (also referred to as the JH1 or "Janus Homology 1" domain) of the JAK protein, which prevents catalytic activity of the kinase by blocking ATP, downstream phosphorylation, and resulting pathway signal transduction (Bryan, M. et al., "Kinase Inhibitors for the Treatment of Immunological Disorders: Recent Advances", J. Med. Chem. 2018, 61, 9030-9058). It's well-known that JAK2 is involved in hematopoiesis (Neubauer, H.; et al., "JAK2 deficiency defines an essential developmental checkpoint in definitive hematopoiesis", Cell 1998, 93, 397-409) and the inhibition of JAK2 can cause side effects such as anemia, neutropenia, and increased infection risk and dyslipidemia (Wollenhaupt, J., et al., "Safety and efficacy of tofacitinib, an oral Janus Kinase Inhibitor, for the treatment of rheumatoid arthritis in open-label. J. Rheumatol. 2014, 41, 837-852; He, Y., et al., Efficacy and safety of tofacitinib in the treatment of rheumatoid arthritis: a systematic review and meta-analysis. BMC Musculoskelet. Disord. 2013, 14, 298; Zerbini, C. A, et al., Tofacitinib for the treatment of rheumatoid arthritis. Expert Rev. Clin. Immunol. 2012, 8, 319-331).

Small molecule inhibitors of TYK2-JH2 domain are being developed for treating autoimmune diseases. BMS986165 (WO2014074661A1, WO2018183649A1, WO2018183656A1 and WO2019232138A1) is a first-in-class of TYK2-JH2 inhibitor, currently undergoing multiple clinical trials in psoriasis, ulcerative colitis (UC), lupus and systemic lupus erythematosus. The other TYK2-JH2 inhibitor which entered clinical trials is ABBV-712 (See, for example, WO2019178079A1, WO2019178079A9, JP6557436B1, and US2019276450A1) and it is in a clinical trial for psoriasis. WO2020086616A1 and WO2018071794A1 describe TYK2 inhibitors and their use in treating a TYK2-mediated disorder.

### SUMMARY OF THE DISCLOSURE

In one aspect, the invention provides a compound, or stereoisomer or pharmaceutically acceptable salt thereof, as defined by the claims. In another aspect, the invention provides a compound of the invention for use in treating an inflammatory or autoimmune disease. In another aspect, the invention provides a pharmaceutical composition as defined by the claims.
Accordingly, subject matter for which protection is sought is as defined by the claims. Any reference to an "aspect", "case", "disclosure", or "embodiment" which does not fall within the scope of the claims is present for explanatory purposes only and does not form part of the invention.
References to methods of treatment by therapy or surgery or in vivo diagnosis methods disclosed herein are to be interpreted as references to compounds of the present invention for use in those methods.
Disclosed herein provides a serial of compounds which inhibit the pseudokinase (JH2) domain of TYK2. These compounds showed picomolar to nanomolar biochemical activity in TYK2-JH2 binding assay and also showed nanomolar activity in cellular assay. In the meanwhile, these compounds showed excellent selectivity against JAK1 in biochemical assay and excellent selectivity against JAK2 in cellular assay.

In the present discourse, compounds bind to the pseudokinase (JH2) domain of TYK2 and inhibit its function through an allosteric mechanism. In the meanwhile, these compounds have greatly improved selectivity over other JAK family members (JAK1, JAK2 and JAK3).

**In** the first aspect, disclosed herein a compound of Formula (I) 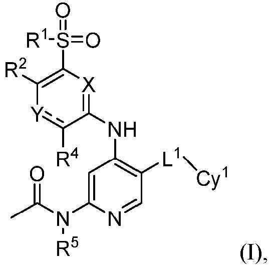 or a stereoisomer or a pharmaceutically acceptable salt thereof, wherein:
X is N or CH;
Y is N, NR³ or CR³;
L¹ is a direct bond;
R¹ is -C₁₋₆alkyl, -haloC₁₋₆alkyl, -C₁₋₆ alkoxy, -haloC₁₋₆ alkoxyl, -C₃₋₆ cycloalkyl, aryl, or -NR^{c}R^{d};
each of R², R³, and R⁴ is independently hydrogen, cyano, halogen, -C₁₋₆alkyl, -C₂₋₆alkenyl, -C₂₋₆alkynyl, - C₃₋₈cycloalkyl, heterocyclyl, aryl, heteroaryl, oxo, -NO₂, -OR^{e}, -SO₂R^{e}, -COR^{e}, -CO₂R^{e}, -CONR^{e}R^{f}, - C(=NR^{e})NR^{f}R^{g}, -NR^{e}R^{f}, -NR^{e}COR^{f}, -NR^{e}CONR^{f}R³, -NR^{e}CO₂R^{f}, -NR^{e}SONR^{f}R^{g}, -NR^{e}SO₂NR^{f}R^{g}, or - NR^{e}SO₂R^{f},
wherein each of -C₁₋₆alkyl, -C₂₋₆alkenyl, -C₂₋₆alkynyl, -C₃₋₈cycloalkyl, heterocyclyl, aryl, or heteroaryl is independently and optionally substituted with at least one substituent selected from
   i) cyano, -oxo-, halogen, -NR^{m}Rⁿ, -OR^{h}, -C(O)NR^{m}Rⁿ;
   ii) heterocyclyl optionally substituted with at least one substituent independently selected from cyano, - oxo, halogen, hydroxy, -NR^{m}Rⁿ, substituted or unsubstituted -C₁₋₆alkyl, substituted or unsubstituted -C₁₋₆alkoxy or -C(O)NR^{m}R; or,
   iii) C₁₋₆alkyl optionally substituted with at least one substitution independently selected from cyano, halogen, hydroxy, - NH₂ or C₁₋₆alkoxy;
wherein R^{h} is hydrogen, hydroxy, -NH₂, -C₁₋₆alkyl, C₁₋₆alkyl substituted with hydroxy, or heterocyclyl, R^{e}, R^{f}, and R^{g} are each independently hydrogen, -C₁₋₆alkyl, -C₁₋₆alkoxy, -C₂₋₆alkenyl, -C₂₋₆alkynyl, cycloalkyl, heterocyclyl, aryl, or heteroaryl, wherein each of -C₁₋₆alkyl, -C₂₋₆alkenyl, -C₂₋₆alkynyl, cycloalkyl, heterocyclyl, aryl, or heteroaryl is optionally independently substituted with one to three substituents selected from cyano, -oxo-, halogen, hydroxy, -NR^{m}Rⁿ, -C₁₋₆alkyl, -C₁₋₆alkoxy, -C₃₋₆cycloalkyl optionally substituted with halogen, hydroxy or -C₁₋₆alkoxy, -C(O)NR^{m}Rⁿ, or heterocyclyl;
R⁵ is hydrogen or C₁₋₆alkyl;
Cy¹ is 6- to 12- membered aryl or 5- to 14- membered heteroaryl, or 5- to 14- membered heterocyclyl, each of which is optionally substituted with at least one substituent Rⁱ,
Rⁱ is independently halogen, cyano, -C₁₋₆alkyl, -C₂₋₆alkenyl, -C₂₋₆alkynyl, -C₃₋₈cycloalkyl, heterocyclyl, aryl, heteroaryl, oxo, cyano (-CN), -NO₂, -OR^{j}, -SO₂R^{j}, -COR^{j}, -CO₂R^{k}, -CONR^{j}R^{k}, -C(=NR^{j})NR^{k}R^{l}, - NR^{j}R^{k}, -NR^{j}COR^{k}, -NR^{j}CONR^{k}R^{l}, -NR^{j}CO₂R^{k}, -NR^{j}SONR^{k}R^{l}, -NR^{j}SO₂NR^{k}R^{l}, or -NR^{j}SO₂R^{k}
wherein each of -C₁₋₆alkyl, -C₂₋₆alkenyl, -C₂₋₆alkynyl, -C₃₋₈cycloalkyl, heterocyclyl, aryl, or heteroaryl is optionally substituted with halogen, -OR^{m}, -C(O)R^{m}, -NR^{m}Rⁿ, -C₁₋₆alkyl, C₁₋₆alkoxy-, C₁₋₆alkyl substituted with -C₁₋₆alkoxy or -oxo-;
R^{j}, R^{k}, R^{l}, R^{m}, Rⁿ are each independently hydrogen, -C₁₋₆alkyl, C₁₋₆alkoxy-C₁₋₆alkyl-, -C₂₋₆alkenyl, -C₂₋₆alkynyl, cycloalkyl, heterocyclyl, aryl, or heteroaryl;
or (R¹ and R²), or (R² and R³), or (R³ and R⁴), together with the atoms to which they are attached, form a fused ring system, said fused ring system comprises 0-4 heteroatoms selected from oxygen (O), nitrogen (N) or sulfur (S) as ring member(s) and is optionally and independently substituted with halogen, -C₁₋₆alkyl, -C₁₋₆alkoxy, C₁₋₆alkyl substituted with halogen, C₁₋₆alkoxy substituted with halogen or -C₃₋₆cycloalkyl;
any of the said alkyl or alkoxy is optionally enriched in deuterium.

In some embodiments, disclosed herein is a compound of Formula (I-A) 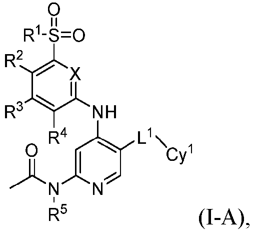 or a stereoisomer or a pharmaceutically acceptable salt thereof, wherein:
X is N or CH;
L¹ is a direct bond;
R¹ is -C₁₋₆alkyl, -haloC₁₋₆alkyl, -C₁₋₆ alkoxy, -haloC₁₋₆ alkoxyl, -C₃₋₆ cycloalkyl, aryl, or -NR^{m}Rⁿ;
each of R², R³, and R⁴ is independently hydrogen, halogen, -C₁₋₆alkyl, -C₂₋₆alkenyl, -C₂₋₆alkynyl, -C₃₋₈cycloalkyl, heterocyclyl, aryl, heteroaryl, -oxo-, -CN, -NO₂, -OR^{e}, -SO₂R^{e}, -COR^{e}, -CO₂R^{e}, -CONR^{e}R^{f}, - C(=NR^{e})NR^{f}R^{g}, -NR^{e}R^{f}, -NR^{e}COR^{f}, -NR^{e}CONR^{f}R^{g}, -NR^{e}CO₂R^{f}, -NR^{e}SONR^{f}R^{g}, -NR^{e}SO₂NR^{f}R^{g}, or - NR^{e}SO₂R^{f},
wherein each of -C₁₋₆alkyl, -C₂₋₆alkenyl, -C₂₋₆alkynyl, -C₃₋₈cycloalkyl, heterocyclyl, aryl, or heteroaryl is independently and optionally substituted with at least one substituents selected from cyano, oxo, halogen, C₁₋₆alkyl optionally substituted with halogen, C₁₋₆alkyl substituted with hydroxy (preferably, hydroxymethyl, hydroxyethyl), -OR^{h}, -C(O)NR^{m}Rⁿ, -NH₂, -C₁₋₆alkyl substituted with -NH₂ or -C₁₋₆alkyl substituted with -C₁₋₆alkoxy -;
wherein R¹¹ is hydrogen, alkyl, hydroxy-C₁₋₆alkyl or heterocyclyl,
R^{e}, R^{f}, and R^{g} are each independently hydrogen, -C₁₋₆alkyl, -C₁₋₆alkoxy, -C₂₋₆alkenyl, -C₂₋₆alkynyl, cycloalkyl, heterocyclyl, aryl, or heteroaryl, wherein each of -C₁₋₆alkyl, -C₂₋₆alkenyl, -C₂₋₆alkynyl, cycloalkyl, heterocyclyl, aryl, or heteroaryl is optionally independently substituted with one to three substituents selected from halogen, hydroxy, cyano, or -C₁₋₆alkoxy; -C₃₋₆cycloalkyl optionally substituted with halogen, hydroxy, or C₁₋₆alkoxy, or heterocyclyl;
R⁵ is hydrogen or C₁₋₆alkyl;
Cy¹ is 6- to 12- membered aryl or 5- to 14- membered heteroaryl, or 5- to 14- membered heterocyclyl, each of which is optionally substituted with at least one substituent Rⁱ,
Rⁱ is independently halogen, cyano, -C₁₋₆alkyl, -C₂₋₆alkenyl, -C₂₋₆alkynyl, -C₃₋₈cycloalkyl, heterocyclyl, aryl, heteroaryl, oxo, -CN, -NO₂, -OR^{j}, -SO₂R^{j}, -COR^{j}, -CO₂R^{k}, -CONR^{j}R^{k}, -C(=NR^{j})NR^{k}R^{l}, -NR^{j}R^{k}, - NR^{j}COR^{k}, -NR^{j}CONR^{k}R^{l}, -NR^{j}CO₂R^{k}, -NR^{j}SONR^{k}R^{l}, -NR^{j}SO₂NR^{k}R^{l}, or -NR^{j}SO₂R^{k},
wherein each of said -C₁₋₆alkyl, -C₂₋₆alkenyl, -C₂₋₆alkynyl, -C₃₋₈cycloalkyl, heterocyclyl, aryl, or heteroaryl is optionally substituted with halogen, OR^{m}, C(O)R^{m}, -NR^{m}Rⁿ, -C₁₋₆alkyl, C₁₋₆alkoxy-, C₁₋₆alkoxy-C₁₋₆alkyl-, or oxo;
R^{j}, R^{k}, R^{l}, R^{m}, Rⁿ are each independently hydrogen, -C₁₋₆alkyl, -C₁₋₆alkyl substituted with C₁₋₆alkoxy, -C₂₋₆alkenyl, -C₂₋₆alkynyl, cycloalkyl, heterocyclyl, aryl, or heteroaryl;
or (R¹ and R²), or (R² and R³), or (R³ and R⁴), together with the atoms to which they are attached, form a fused ring system, said fused ring system comprises 0-4 heteroatoms selected from oxygen, nitrogen or sulfur as ring member(s) and is optionally and independently substituted with halogen, -C₁₋₆alkyl, -C₁₋₆alkoxy, -haloC₁₋₆alkyl, -haloC₁₋₆alkoxy, or -C₃₋₆cycloalkyl;
any of the said alkyl or alkoxy is optionally enriched in deuterium.

In some embodiments, X is N and Y is CR³. In some embodiments, X is N and Y is N. In some embodiments, X is CH and Y is N.

In some embodiments, R¹ is -C₁₋₃ alkyl, -NR^{c}R^{d} or -C₃₋₆ cycloalkyl, preferably -NH₂, methyl, ethyl, propyl, isopropyl, cyclopropyl or cyclopentyl.

In some embodiments, R² and R⁴ are each independently hydrogen, halogen, -C₁₋₆alkyl, or -C₁₋₆alkoxy, preferably hydrogen, fluoro, methyl, methoxy, ethoxy, or isopropoxy.

In some embodiments, R³ is
- hydrogen;
- cyano;
- halogen;
- -C₁₋₄ alkyl optionally substituted with at least one substituent independently selected from halogen, 3- to 6-membered heterocyclyl or -OR^{h}, wherein
   3- to 6-membered heterocyclyl comprises one or two heteroatoms selected from oxygen (O), nitrogen (N) or sulfur (O) as ring member(s), optionally substituted with at least one substituent independently selected from cyano, -oxo, halogen, hydroxy, -NR^{m}Rⁿ, C₁₋₆alkyl, -C₁₋₆alkoxy or -C(O)NR^{m}Rⁿ, wherein -C₁₋₆alkyl or -C₁₋₆alkoxy is substituted with at least one substitution independently selected from cyano, halogen, hydroxy, -NH₂, -C₁₋₆alkyl or C₁₋₆alkoxy; and
   R¹¹ is hydrogen, alkyl, or heterocyclyl;
- -C₃₋₆cycloalkyl, optionally substituted with at least one substituent independently selected from cyano, -oxo, halogen, -NR^{m}Rⁿ, hydroxy, -C₁₋₆alkyl, -C₁₋₆alkoxy or -C(O)NR^{m}Rⁿ, wherein -C₁₋₆alkyl or C₁₋₆alkoxy is substituted with at least one substitution independently selected from cyano, halogen, hydroxy, - NH₂, -C₁₋₆alkyl or C₁₋₆alkoxy;
- heterocyclyl comprising one or two heteroatoms selected from oxygen (O), nitrogen (N) or sulfur (S) as ring member(s), optionally substituted with at least one substituent independently selected from cyano, -oxo-, halogen, hydroxy, C₁₋₆alkyl, alkoxy, -NRR^{m}Rⁿ, or -C(O)NR^{m}Rⁿ, and wherein -C₁₋₆alkyl or C₁₋₆alkoxy is substituted with at least one substitution independently selected from cyano, halogen, hydroxy, -NH₂, -C₁₋₆alkyl or C₁₋₆alkoxy;
- -OR^{e}, wherein R^{e} is -C₁₋₆alkyl, -C₃₋₆cycloalkyl, 3- to 6-membered heterocyclyl , or aryl, wherein
   i) -C₁₋₆alkyl is optionally substituted with cyano, -oxo-, halogen, hydroxy, -NR^{m}Rⁿ, -C₁₋₆alkoxy-, -C₃₋₆cycloalkyl optionally substituted with cyano, -oxo, halogen, hydroxy, -NR^{m}Rⁿ, -C₁₋₆alkyl, C₁₋₆alkoxy or - C(O)NR^{m}Rⁿ, 4- to 6-membered heterocyclyl optionally substituted with cyano, halogen, hydroxy, C₁₋₆alkyl or C₁₋₆alkoxy; and,
   ii) -C₃₋₆cycloalkyl or 3- to 6-membered heterocyclyl is optionally substituted with cyano, -oxo, halogen, hydroxy, -NR^{m}Rⁿ, C₁₋₆alkyl, C₁₋₆alkoxy or -C(O)NR^{m}Rⁿ, wherein -C₁₋₆alkyl or C₁₋₆alkoxy is substituted with at least one substitution independently selected from cyano, halogen, hydroxy, -NH₂, -C₁₋₆alkyl or C₁₋₆alkoxy;
- -C₅₋₁₀aryl; or
- heteroaryl comprising one oxygen (O), nitrogen (N) or sulfur (S) heteroatom as ring member, optionally substituted with at least one substitution independently selected from cyano, -oxo, halogen, hydroxy, -NR^{m}Rⁿ, -C₁₋₆alkyl, -C₁₋₆alkoxy or -C(O)NR^{m}Rⁿ, wherein -C₁₋₆alkyl or C₁₋₆alkoxy is substituted with at least one substitution independently selected from cyano, halogen, hydroxy, -NH₂, -C₁₋₆alkyl or C₁₋₆alkoxy;
   and wherein R^{m} and Rⁿ are independently selected from hydrogen or C₁₋₃alkyl;
   any of the said alkyl or alkoxy is optionally enriched in deuterium.

In some embodiments, R³ is
- hydrogen;
- cyano;
- halogen;
- -C₁₋₄ alkyl optionally substituted with at least one substituent independently selected from halogen, 3- to 6-membered heterocyclyl or -OR^{h}, wherein
   said 3- to 6-membered heterocyclyl comprises one or two heteroatoms selected from oxygen (O), nitrogen (N) or sulfur (O) as ring member(s), optionally substituted with at least one substituent independently selected from cyano, -oxo, halogen, hydroxy, -NR^{m}Rⁿ, C₁₋₆alkyl, -C₁₋₆alkoxy or -C(O)NR^{m}Rⁿ, wherein -C₁₋₆alkyl or C₁₋₆alkoxy is substituted with at least one substitution independently selected from cyano, halogen, hydroxy, -NH₂, -C₁₋₆alkyl or C₁₋₆alkoxy, and,
   R^{h} is hydrogen, alkyl or heterocyclyl (preferably 3- to 6-membered heterocyclyl, e.g., tetrahydrofuranyl, thiazolidinyl);
- -C₃₋₆cycloalkyl, optionally substituted with at least one substituent independently selected from cyano, -oxo, halogen, -NR^{m}Rⁿ, hydroxy, -C₁₋₆alkyl, -C₁₋₆alkoxy or -C(O)NR^{m}Rⁿ, wherein -C₁₋₆alkyl or C₁₋₆alkoxy is substituted with at least one substitution independently selected from cyano, halogen, hydroxy, - NH₂, -C₁₋₆alkyl or C₁₋₆alkoxy;
- heterocyclyl, preferably 4- to 6-membered monocyclic saturated heterocyclyl, saturated mono-spiro heterocyclyl, saturated bicyclic fused heterocyclyl, or saturated bridged heterocyclyl, comprising one or two heteroatoms selected from oxygen (O), nitrogen (N) or sulfur (S) as ring member(s), more preferably morpholinyl, tetrahydrofuranyl, tetrahydropyranyl, pyrrolidinyl, 1,4-dioxanyl, piperidinyl or azetidinyl, optionally substituted with at least one substituent independently selected from cyano, -oxo, halogen, hydroxy, -C₁₋₆alkyl, alkoxy, -NR^{m}Rⁿ, or -C(O)NR^{m}Rⁿ, and wherein -C₁₋₆alkyl or -C₁₋₆alkoxy is substituted with at least one substitution independently selected from cyano, halogen, hydroxy, -NH₂, -C₁₋₆alkyl or C₁₋₆alkoxy;
- -OR^{e}, wherein R^{e} is -C₁₋₆alkyl, -C₃₋₆cycloalkyl, 3- to 6-membered heterocyclyl (preferably 4- to 6-membered monocyclic saturated heterocyclyl comprising one oxygen heteroatom as ring member), or aryl, wherein
   i) -C₁₋₆alkyl is optionally substituted with cyano, -oxo-, halogen, hydroxy, -NR^{m}Rⁿ, -C₁₋₆alkoxy-, -C₃₋₆cycloalkyl optionally substituted with cyano, -oxo, halogen, hydroxy, -NR^{m}Rⁿ, -C₁₋₆alkyl, C₁₋₆alkoxy or - C(O)NR^{m}Rⁿ, 4- to 6-membered heterocyclyl optionally substituted with cyano, halogen, hydroxy, -C₁₋₆alkyl or -C₁₋₆alkoxy; and,
   ii) -C₃₋₆cycloalkyl or 3- to 6-membered heterocyclyl is optionally substituted with cyano, -oxo, halogen, hydroxy, NR^{m}Rⁿ, C₁₋₆alkyl, C₁₋₆alkoxy or -C(O)NR^{m}Rⁿ, wherein -C₁₋₆alkyl or C₁₋₆alkoxy is substituted with at least one substitution independently selected from cyano, halogen, hydroxy, -NH₂, -C₁₋₆alkyl or C₁₋₆alkoxy;
- -C₅₋₁₀aryl; or
- heteroaryl, preferably 5- to 6-membered heteroaryl comprising one oxygen (O), nitrogen (N) or sulfur (S) heteroatom as ring member, optionally substituted with at least one substitution independently selected from cyano, -oxo, halogen, hydroxy, -NR^{m}Rⁿ, -C₁₋₆alkyl, -C₁₋₆alkoxy or -C(O)NR^{m}Rⁿ, wherein - C₁₋₆alkyl or C₁₋₆alkoxy is substituted with at least one substitution independently selected from cyano, halogen, hydroxy, -NH₂, -C₁₋₆alkyl or C₁₋₆alkoxy;
   and wherein R^{m} and Rⁿ are independently selected from hydrogen or C₁₋₃alkyl.

In some embodiments, R³ is
- hydrogen, cyano, halogen;
- -C₁₋₄ alkyl optionally substituted with at least one substituent independently selected from halogen, 3- to 6-membered heterocyclyl or -OR^{h}, wherein
   Said 3- to 6-membered heterocyclyl is selected from morpholinyl, tetrahydrofuranyl, tetrahydropyranyl, pyrrolidinyl, 1,4-dioxanyl, piperidinyl, thiazolidinyl or azetidinyl, each of which is optionally substituted with at least one substituent independently selected from cyano, -oxo, halogen, hydroxy, -NR^{m}Rⁿ, C₁₋₆alkyl, -C₁₋₆alkoxy or -C(O)NR^{m}Rⁿ, wherein -C₁₋₆alkyl or C₁₋₆alkoxy is substituted with at least one substitution independently selected from cyano, halogen, hydroxy, -NH₂, -C₁₋₆alkyl or C₁₋₆alkoxy, and, R^{h} is hydrogen, C₁₋₆alkyl or 3- to 6-membered heterocyclyl (e.g., tetrahydrofuranyl or thiazolidinyl);
- -C₃₋₆cycloalkyl, optionally substituted with at least one substituent independently selected from cyano, -oxo, halogen, NR^{m}Rⁿ, hydroxy, -C₁₋₆alkyl, -C₁₋₆alkoxy or -C(O)NR^{m}Rⁿ, hydroxy, or C₁₋₆alkoxy, wherein -C₁₋₆alkyl or C₁₋₆alkoxy is substituted with at least one substitution independently selected from cyano, halogen, hydroxy, -NH₂, -C₁₋₆alkyl or C₁₋₆alkoxy;
- heterocyclyl is selected from morpholinyl, tetrahydrofuranyl, tetrahydropyranyl, pyrrolidinyl, 1,4-dioxanyl, piperidinyl or azetidinyl, optionally substituted with at least one substituent independently selected from cyano, -oxo, halogen, hydroxy, C₁₋₆alkyl, alkoxy, -NRR^{m}Rⁿ, or -C(O)NR^{m}Rⁿ, and wherein - C₁₋₆alkyl or -C₁₋₆alkoxy is substituted with at least one substitution independently selected from cyano, halogen, hydroxy, -NH₂, -C₁₋₆alkyl or -C₁₋₆alkoxy;
- -OR^{e}, wherein R^{e} is -C₁₋₆alkyl, -C₃₋₆cycloalkyl, 4- to 6-membered monocyclic saturated heterocyclyl comprising one oxygen heteroatom as ring member, or C₆₋₁₀aryl, wherein
   i) -C₁₋₆alkyl is optionally substituted with cyano, -oxo-, halogen, hydroxy, -NR^{m}Rⁿ, -C₁₋₆alkoxy-, -C₃₋₆cycloalkyl optionally substituted with cyano, -oxo, halogen, hydroxy, -NR^{m}Rⁿ, -C₁₋₆alkyl, -C₁₋₆alkoxy or - C(O)NR^{m}Rⁿ, 4- to 6-membered heterocyclyl optionally substituted with cyano, halogen, hydroxy, -C₁₋₆alkyl or -C₁₋₆alkoxy; and,
   ii) -C₃₋₆cycloalkyl or 3- to 6-membered heterocyclyl is optionally substituted with cyano, -oxo, halogen, hydroxy, NR^{m}Rⁿ, C₁₋₆alkyl, C₁₋₆alkoxy or -C(O)NR^{m}Rⁿ, wherein -C₁₋₆alkyl or C₁₋₆alkoxy is substituted with at least one substitution independently selected from cyano, halogen, hydroxy, -NH₂, -C₁₋₆alkyl or C₁₋₆alkoxy;
- -C₆₋₁₀aryl; or
- 5- to 6-membered heteroaryl selected from pyridinyl, pyridazinyl, pyrazinyl, thiazolyl or isoxazolyl, each of which is optionally substituted with at least one substitution independently selected from cyano, -oxo, halogen, hydroxy, -NR^{m}Rⁿ, -C₁₋₆alkyl, -C₁₋₆alkoxy or -C(O)NR^{m}Rⁿ, wherein -C₁₋₆alkyl or C₁₋₆alkoxy is substituted with at least one substitution independently selected from cyano, halogen, hydroxy, -NH₂, -C₁₋₆alkyl or C₁₋₆alkoxy;
   and wherein R^{m} and Rⁿ are independently selected from hydrogen or -C₁₋₃alkyl.

In some embodiments, R³ is
- hydrogen, cyano, halogen;
- -C₁₋₄ alkyl optionally substituted with at least one substituent independently selected from halogen, hydroxy, C₁₋₃alkoxy, thiazolidin-3-yl optionally substituted with at least one substituent independently selected from cyano, -oxo, halogen, hydroxy, -NR^{m}Rⁿ, or -C₁₋₃alkyl;
- -C₃₋₆cycloalkyl, optionally substituted with at least one substituent independently selected from cyano, -oxo, halogen, -NR^{m}Rⁿ, hydroxy, -C₁₋₆alkyl, or -C₁₋₆alkoxy, wherein -C₁₋₆alkyl or C₁₋₆alkoxy is substituted with at least one substitution independently selected from cyano, halogen, hydroxy, -NH₂, -C₁₋₆alkyl or -C₁₋₃alkoxy;
- heterocyclyl is selected from morpholinyl, tetrahydrofuranyl, tetrahydropyranyl, pyrrolidinyl, 1,4-dioxanyl, piperidinyl or azetidinyl, optionally substituted with at least one substituent independently selected from cyano, -oxo, halogen, hydroxy, C₁₋₆alkyl, alkoxy, -NR^{m}Rⁿ, or -C(O)NR^{m}Rⁿ, and wherein -C₁₋₆alkyl or C₁₋₆alkoxy is substituted with at least one substitution independently selected from cyano, halogen, hydroxy, -NH₂, -C₁₋₆alkyl or -C₁₋₆alkoxy;
- -OR^{e}, wherein R^{e} is -C₁₋₆alkyl, -C₃₋₆cycloalkyl, 4- to 6-membered monocyclic saturated heterocyclyl comprising one oxygen heteroatom as ring member, or C₆₋₁₀aryl, wherein
   i) -C₁₋₆alkyl is optionally substituted with cyano, -oxo-, halogen, hydroxy, -NR^{m}Rⁿ, -C₁₋₆alkoxy-, -C₃₋₆cycloalkyl optionally substituted with cyano, -oxo, halogen, hydroxy, -NR^{m}Rⁿ, -C₁₋₆alkyl, -C₁₋₆alkoxy or - C(O)NR^{m}Rⁿ, 4- to 6-membered heterocyclyl optionally substituted with cyano, halogen, hydroxy, -C₁₋₆alkyl or -C₁₋₆alkoxy; and,
   ii) -C₃₋₆cycloalkyl or 3- to 6-membered heterocyclyl is optionally substituted with cyano, -oxo, halogen, hydroxy, -NR^{m}Rⁿ, -C₁₋₆alkyl, -C₁₋₆alkoxy or -C(O)NR^{m}Rⁿ, wherein -C₁₋₆alkyl or -C₁₋₆alkoxy is substituted with at least one substitution independently selected from cyano, halogen, hydroxy, -NH₂, -C₁₋₆alkyl or -C₁₋₆alkoxy;
- -C₆₋₁₀aryl; or
- 5- to 6-membered heteroaryl selected from pyridinyl, pyridazinyl, pyrazinyl, thiazolyl or isoxazolyl, each of which is optionally substituted with at least one substitution independently selected from cyano, -oxo, halogen, hydroxy, -NR^{m}Rⁿ, -C₁₋₆alkyl, -C₁₋₆alkoxy or -C(O)NR^{m}Rⁿ, wherein -C₁₋₆alkyl or
- C₁₋₆alkoxy is substituted with at least one substitution independently selected from cyano, halogen, hydroxy, -NH₂, -C₁₋₆alkyl or -C₁₋₆alkoxy;
   and wherein R^{m} and Rⁿ are independently selected from hydrogen or -C₁₋₃alkyl.

In some embodiments, R³ is
- hydrogen, cyano, halogen;
- methyl, ethyl, propyl or butyl, each of which optionally substituted with at least one substituent independently selected from halogen, hydroxy, methoxy, ethoxy, propoxy, or 2,4-dioxothiazolidin-3-yl;
- cyclopropyl, cyclobutyl, cyclopentyl or cyclohexyl, each of which is optionally substituted with at least one substituent independently selected from cyano, -oxo, halogen, -NR^{m}Rⁿ, hydroxy, -C₁₋₃alkyl, or - C₁₋₃alkoxy, wherein -C₁₋₃alkyl or C₁₋₃alkoxy is optionally substituted with at least one substitution independently selected from cyano, halogen, hydroxy, -NH₂, -C₁₋₃alkyl or -C₁₋₃alkoxy;
- heterocyclyl is selected from morpholin-2-yl, morpholin-3-yl, morpholin-4-yl, tetrahydrofuran-2-yl, tetrahydrofuran-3-yl, tetrahydropyran-2-yl, tetrahydropyran-3-yl, tetrahydropyran-4-yl, pyrrolidine-1-yl, pyrrolidin-2-yl, pyrrolidin-3-yl, 1,4-dioxan-2-yl, piperidin-1-yl, piperidin-2-yl, piperidin-3-yl, piperidin-4-yl, azetidin-1-yl, azetidine-2-yl, azetidin-3-yl, 5-azaspiro[2.4]heptanyl, 3-azabicyclo[3.1.0]hexan-3-yl or 2-azabicyclo[3.1.0]hexan-2-yl, each of which is optionally substituted with at least one substituent independently selected from cyano, -oxo, halogen, hydroxy, -C₁₋₆alkyl, -C₁₋₆alkoxy, -NR^{m}Rⁿ, or - C(O)NR^{m}Rⁿ, and wherein -C₁₋₆alkyl or C₁₋₆alkoxy is substituted with at least one substitution independently selected from cyano, halogen, hydroxy, -NH₂, -C₁₋₆alkyl or -C₁₋₆alkoxy;
- -OR^{e}, wherein R^{e} is
   i) methyl, ethyl, propyl (iso-propyl), butyl, pentyl or hexyl, each of which is optionally substituted with deuterium, cyano, -oxo-, halogen, hydroxy, -NR^{m}Rⁿ, -C₁₋₃alkoxy-, -C₃₋₆cycloalkyl optionally substituted with cyano, -oxo, halogen, hydroxy, -NR^{m}Rⁿ, -C₁₋₃alkyl, -C₁₋₃alkoxy or -C(O)NR^{m}Rⁿ, or 4- to 6-membered heterocyclyl optionally substituted with cyano, halogen, hydroxy, -C₁₋₃alkyl or -C₁₋₃alkoxy; or,
   ii) cyclopropyl, cyclobutyl, cyclopentyl, cyclohexyl, morpholin-2-yl, morpholin-3-yl, morpholin-4-yl, tetrahydrofuran-2-yl, tetrahydrofuran-3-yl, tetrahydropyran-2-yl, tetrahydropyran-3-yl, tetrahydropyran-4-yl, pyrrolidin-1-yl, pyrrolidin-2-yl, pyrrolidin-3-yl, 1,4-dioxan-2-yl, piperidin-1-yl, piperidin-2-yl, piperidin-3-yl, piperidin-4-yl, azetidin-1-yl, azetidine-2-yl or azetidin-3-yl, each of which is optionally substituted with cyano, -oxo, halogen, hydroxy, -NR^{m}Rⁿ, -C₁₋₃alkyl, -C₁₋₃alkoxy or -C(O)NR^{m}Rⁿ, wherein - C₁₋₃alkyl or -C₁₋₃alkoxy is substituted with at least one substitution independently selected from cyano, halogen, hydroxy, -NH₂, -C₁₋₃alkyl or -C₁₋₃alkoxy;
- -C₆₋₁₀aryl; or
- 5- to 6-membered heteroaryl selected from pyridin-1-yl, pyridin-2-yl, pyridin-3-yl, pyridin-4-yl, pyridazin-1-yl, pyridazin-2-yl, pyridazin-3-yl, pyridazin-4-yl, pyrazin-1-yl, pyrazin-2-yl, thiazol-2-yl, thiazol-3-yl, thiazol-4-yl, isoxazol-2-yl, isoxazol-3-yl or isoxazol-4-yl, each of which is optionally substituted with at least one substitution independently selected from cyano, -oxo, halogen, hydroxy, - NR^{m}Rⁿ, -C₁₋₃alkyl, -C₁₋₃alkoxy or -C(O)NR^{m}Rⁿ, wherein -C₁₋₃alkyl or -C₁₋₃alkoxy is substituted with at least one substitution independently selected from cyano, halogen, hydroxy, -NH₂, -C₁₋₃alkyl or C₁₋₃alkoxy;
   and wherein R^{m} and Rⁿ are independently selected from hydrogen or -C₁₋₃alkyl.

In some embodiments, R³ is
- Hydrogen;
- Methyl, 1-methoxyethyl, 2-hydroxypropan-2-yl, 1-methoxyethyl, or (2,4-dioxothiazolidin-3-yl)methyl;
- Isopropoxy, methoxy-d3, methoxy, ethoxy, difluoromethoxy, 2-methoxyethoxy, 2-methoxy-2-methylpropoxy, 2-hydroxy-2-methylpropoxy, cyclopropylmethoxy, (1,4-dioxan-2-yl)methoxy, (4-hydroxycyclohexyl)oxy, (cis-4-hydroxycyclohexyl)oxy, (trans-4-hydroxycyclohexyl)oxy, (4-methoxycyclohexyl)oxy, (cis-4-methoxycyclohexyl)oxy, (trans-4-methoxycyclohexyl)oxy, or (3-methyloxetan-3-yl)methoxy;
- cyano
- 3-methoxycyclobutyl, (trans)-3-methoxycyclobutyl, (cis)-3-methoxycyclobutyl, 2,2-dichlorocyclopropyl, or 1-cyanocyclopropyl;
- Morpholino, 3-methyl-morpholino, 3(R)-methyl-morpholino, 3(S)-methyl-morpholino, 3,3-dimethylmorpho;
- tetrahydro-2H-pyran-4-yl, tetrahydro-2H-pyran-3-yl, (R)-tetrahydro-2H-pyran-3-yl, (S)-tetrahydro-2H-pyran-3-yl, 2,2,6,6-tetramethyltetrahydro-2H-pyran-4-yl;
- 3-methoxypyrrolidin-1-yl, 3(R)-methoxypyrrolidin-1-yl, 3(S)-methoxypyrrolidin-1-yl, 3-hydroxy-3-methylpyrrolidin-1-yl, 3-(2-hydroxyethoxy)pyrrolidin-1-yl, 3-(trifluoromethoxy)pyrrolidin-1-yl, 3(R)-(trifluoromethoxy)pyrrolidin-1-yl, 3(S)-(trifluoromethoxy)pyrrolidin-1-yl, 2-(aminocarbonyl)pyrrolidin-1-yl, 2(R)-(aminocarbonyl)pyrrolidin-1-yl, 2(S)-(aminocarbonyl)pyrrolidin-1-yl, 3-(methoxymethyl)pyrrolidin-1-yl, 3(R)-(methoxymethyl)pyrrolidin-1-yl, 3(S)-(methoxymethyl)pyrrolidin-1-yl, 3-cyano-4-hydroxypyrrolidin-1-yl, cis-3-cyano-4-hydroxypyrrolidin-1-yl, trans-3-cyano-4-hydroxypyrrolidin-1-yl, 3-cyano-4-methoxypyrrolidin-1-yl, cis-3-cyano-4-methoxypyrrolidin-1-yl, trans-3-cyano-4-methoxypyrrolidin-1-yl, 2-(methoxymethyl)pyrrolidin-1-yl, 2(R)-(methoxymethyl)pyrrolidin-1-yl, 2(S)-(methoxymethyl)pyrrolidin-1-yl, 3-methylpyrrolidin-1-yl, 3(R)-methylpyrrolidin-1-yl, 3(S)-methylpyrrolidin-1-yl, pyrrolidin-1-yl, 3-(cyanomethoxy)pyrrolidin-1-yl;
- 5-azaspiro[2.4]heptan-5-yl;
- tetrahydrofuran-3-yl;
- 3-methoxyazetidin-1-yl, 3-hydroxy-3-methylazetidin-1-yl;
- 1,4-dioxan-2-yl;
- 4-aminotetrahydro-2H-pyran-4-yl, 4-(aminomethyl)tetrahydro-2H-pyran-4-yl,
- 4-methoxypiperidin-1-yl, 4-hydroxy-4-methylpiperidin-1-yl, 1-(2,2,2-trifluoroethyl)piperidin-4-yl, 3-methoxypiperidin-1-yl, 3(R)-methoxypiperidin-1-yl, 3(S)-methoxypiperidin-1-yl, 3-ethoxypiperidin-1-yl, 3(R)-ethoxypiperidin-1-yl, 3(S)-ethoxypiperidin-1-yl;
- 3-cyano-2-azabicyclo[3.1.0]hexan-2-yl, (3R)-3-cyano-2-azabicyclo[3.1.0]hexan-2-yl, (3S)-3-cyano-2-azabicyclo[3.1.0]hexan-2-yl, 3-azabicyclo[3.1.0]hexan-3-yl;4-methylpyridin-3-yl, 5-methylpyridazin-4-yl, 5-methoxypyridazin-4-yl, 3,5-dimethylisoxazol-4-yl, 4-methoxypyridin-3-yl, 4-(2-hydroxypropan-2-yl)pyridin-3-yl, 6-cyanopyridin-3-yl, 4-cyanopyridin-3-yl, 2-cyanopyridin-3-yl, 3-methylpyrazin-2-yl, 5-cyanopyridazin-4-yl, 5-fluoropyridazin-4-yl, 4-fluoropyridin-3-yl, 4-isopropylpyridin-3-yl, 4-(1-hydroxyethyl)pyridin-3-yl, 4-(1-methoxyethyl)pyridin-3-yl, pyridin-2-yl, or thiazol-4-yl.

In some embodiments, (R¹ and R²), or (R² and R³), or (R³ and R⁴), together with the atoms to which they are attached, form a fused 5- to 7-membered ring system, said fused ring system comprises 0-2 oxygen heteroatoms as ring member(s) and is optionally and independently substituted with halogen, -C₁₋₆alkyl, -C₁₋₆alkoxy, -haloC₁₋₆alkyl, -haloC₁₋₆alkoxy, or -C₃₋₆cycloalkyl.

In some embodiments, R¹ and R², together with the atoms to which they are attached, form a fused ring system selected from 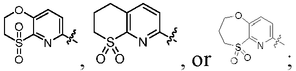 or R² and R³, together with the atoms to which they are attached, form a fused ring system 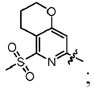 R³ and R⁴, together with the atoms to which they are attached, form a fused ring system selected from 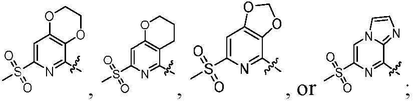 and wherein each of fused ring system is optionally and independently substituted with halogen, -C₁₋₆alkyl, -C₁₋₆alkoxy, -haloC₁₋₆alkyl, -haloC₁₋₆alkoxy, or -C₃₋₆cycloalkyl .

In some embodiments, Cy¹ is
- a 5- to 7-membered monocyclic heterocyclyl or heteroaryl comprising 1, 2, 3 or 4 heteroatoms selected from oxygen, nitrogen or sulfur as ring member(s), or
- 7- to 14- membered bicyclic or tricyclic heterocyclyl or heteroaryl having 1, 2, or 3 heteroatoms selected from oxygen, nitrogen or sulfur as ring member(s),
each of which is optionally substituted with at least one substituent Rⁱ.

In some embodiments, Cy¹ is
- said 5- to 7-membered monocyclic heteroaryl comprising 1, 2, 3 or 4 heteroatom(s) selected from oxygen (O), nitrogen (N) or sulfur (S) as ring member(s), preferably pyrazolyl, triazolyl, imidazolyl, thiazolyl, oxazolyl, furanyl, pyridinyl, pyridazinyl, pyrazinyl or pyrimidinyl, said monocyclic heteroaryl is optionally substituted with one or two substituents selected from
   i. halogen;
   ii. cyano;
   iii. -C₁₋₆alkyl optionally substituted with halogen, hydroxy, -C₁₋₆alkoxy, -C(O)R^{m} (preferably R^{m} is morpholinyl), or -NR^{m}Rⁿ;
   iv. heterocyclyl optionally substituted with halogen, C₁₋₆alkyl-, -C₁₋₆alkyl substituted with -C₁₋₆alkoxy, or oxo; preferably said heterocyclyl is selected from tetrahydrofuranyl (preferably tetrahydrofuran-3-yl), morpholinyl (preferably morpholino), 2-oxa-5-azabicyclo[2.2.1]heptanyl (preferably 2-oxa-5-azabicyclo[2.2.1]heptan-2-yl), 8-oxa-3-azabicyclo[3.2.1]octanyl (preferably 8-oxa-3-azabicyclo[3.2.1]octan-8-yl), isoindolinyl (preferably isoindolin-2-yl), each of which is optionally substituted with methyl, ethyl, propyl, isopropyl, isobutyl, tert-butyl, n-butyl, methoxymethyl, methoxyethyl, ethoxymethyl, ethoxyethyl, or oxo;
   v. -C₃₋₆cycloalkyl optionally substituted with halogen,- oxo, -C₁₋₆alkyl, C₁₋₆alkoxy-, or -C₁₋₆alkyl substituted with -C₁₋₆alkoxy; or
   vi. -OR^{j}, wherein R^{j} is -C₁₋₆alkyl, -C₁₋₆alkyl substituted with -C₁₋₆alkoxy, or heterocyclyl;
   vii. oxo;
- said 7- to 14-membered bicyclic or tricyclic heteroaryl comprising 1, 2, or 3 heteroatom(s) as ring member(s), preferably benzoimidazolyl, imidazopyrimidinyl, pyrazolopyrazinyl, pyrazolopyrimidinyl, benzothiophenyl, benzothiazolyl, benzoisoxazolyl, benzooxazolyl, benzoisothiazolyl, imidazopyridazinyl, imidazopyridazinyl; dihydro-4H-furo[3,2-c]pyranyl, 6,7-dihydro-4H-thieno[3,2-c]pyranyl, 2,3-dihydropyrazolo[5,1-b]oxazolyl, 4,5,6,7-tetrahydrofuro[3,2-c]pyridinyl, 1,3a,4,6,7,7a-hexahydropyrano[4,3-c]pyrazolyl, 4,5,6,7-tetrahydropyrazolo[1,5-a]pyrazinyl, 4,5,6,7-tetrahydropyrazolo[1,5-a]pyrimidinyl, 4,5,6,7-tetrahydropyrazolo[1,5-a]pyrazinyl, 4,5,6,7-tetrahydrothiazolo[5,4-c]pyridinyl, 4,5,6,7-tetrahydropyrazolo[1,5-a]pyrazinyl, 6,7-dihydro-4H-pyrazolo[5,1-c][1,4]oxazinyl, 2,3-dihydropyrazolo[5,1-b]oxazolyl, 2,3-dihydropyrazolo[5,1-b]oxazolyl, 1,3a,4,6,7,7a-hexahydropyrano[4,3-c]pyrazolyl, 6,7-dihydro-4H-pyrano[4,3-d]thiazolyl, [1,3]dioxolo[4,5-c]pyridinyl, 2,3-dihydro-[1,4]dioxino[2,3-c]pyridinyl, 3,4-dihydro-2H-pyrido[3,2-b][1,4]oxazinyl, 2,3-dihydro-[1,4]dioxino[2,3-b]pyridinyl, 2,3-dihydro-[1,4]dioxino[2,3-b]pyridin-6-yl, 3,4-dihydro-2H-pyrido[4,3-b][1,4]oxazinyl, or 2H-pyrido[3,2-b][1,4]oxazin-4(3H)-yl, each of which is optionally substituted with halogen, -C₁₋₆alkyl, -NH₂, or -C(O)R^{m}, wherein R^{m} is C₁₋₆alkyl.

In some embodiments, Cy¹ is a 7- to 14-membered bicyclic heteroaryl which is a pyridinyl, pyrazolyl, thienyl, or thiazolyl ring fused with a 5- or 6-membered heterocyclyl ring, wherein said 5- or 6-membered heterocyclyl ring comprising one or two heteroatoms selected from oxygen or nitrogen as ring member(s) and said 5- or 6-membered heterocyclyl ring is optionally substituted with one or two C₁₋₆alkyl or oxo, preferably two C₁₋₆alkyl, more preferably two methyl, most preferably two methyl on the same carbon atom. In some embodiments, Cy¹ is a 7- to 14-membered bicyclic heteroaryl which is a pyridinyl ring fused with a 5- or 6-membered heterocyclyl ring, wherein said 5- or 6-membered heterocyclyl ring comprising one or two heteroatoms selected from oxygen (O) or nitrogen (N) as ring member(s) and said 5- or 6-membered heterocyclyl ring is optionally substituted with one or two C₁₋₆alkyl or oxo, preferably two C₁₋₆alkyl, more preferably two methyl, most preferably two methyl on the same carbon atom. In some embodiments, Cy¹ is a 7- to 14-membered bicyclic heteroaryl which is a pyridinyl ring fused with a 5- or 6-membered heterocyclyl ring, wherein said 5- or 6-membered heterocyclyl ring comprising two oxygen atoms as ring member(s) and said 5- or 6-membered heterocyclyl ring is optionally substituted with one or two C₁₋₆alkyl, preferably two C₁₋₆alkyl, more preferably two methyl, most preferably two methyl on the same carbon atom. In some embodiments, Cy¹ is a 7- to 14-membered bicyclic heteroaryl which is a pyridinyl ring fused with 1,4-dioxane ring, wherein said 1,4-dioxane ring is optionally substituted with one or two C₁₋₆alkyl, preferably two C₁₋₆alkyl, more preferably two methyl, most preferably two methyl on the same carbon atom; any of the said alkyl or alkoxy is optionally enriched in deuterium. **In** some preferred embodiments, Cy¹ is 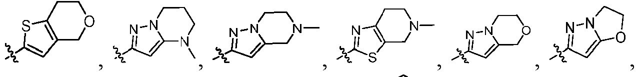 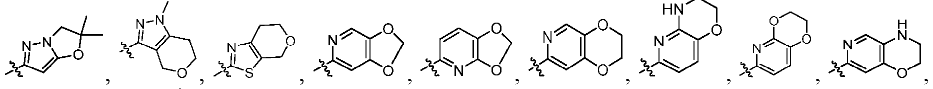 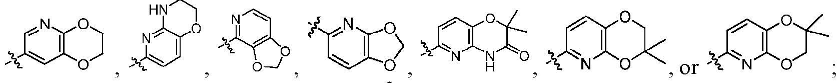 preferably

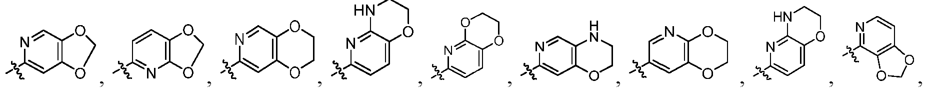

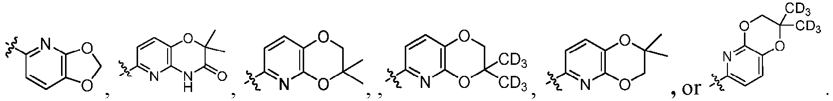

In some embodiments, Cy¹ is

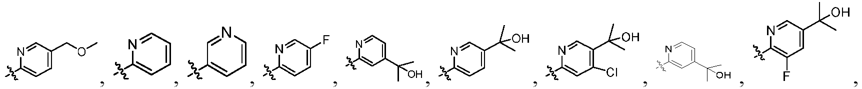

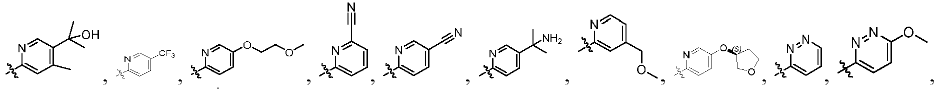

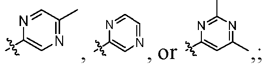

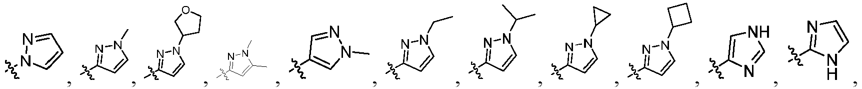

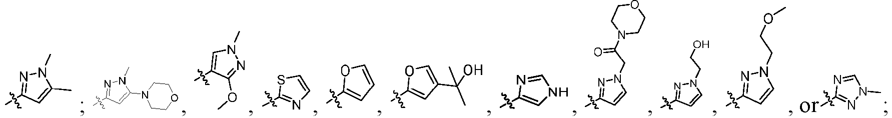

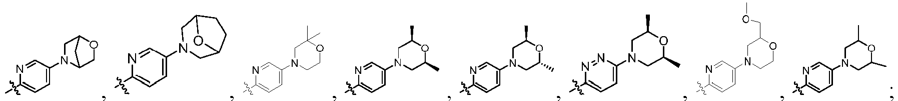

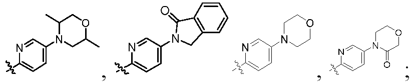

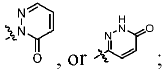

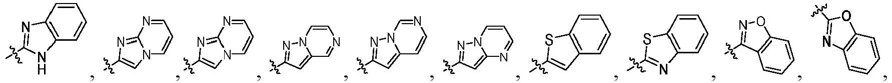

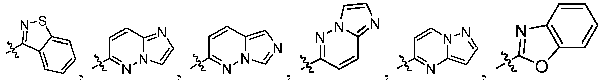

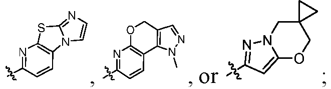

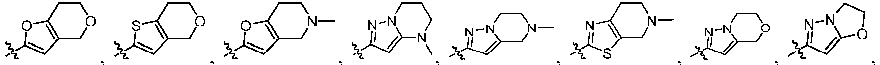

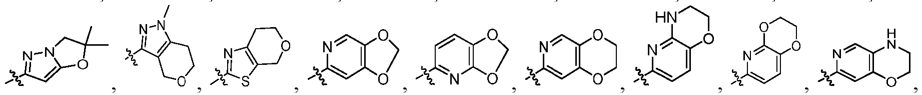

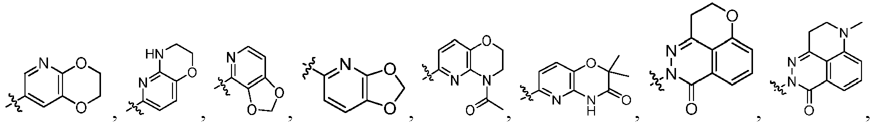

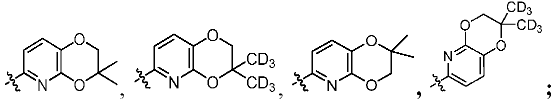

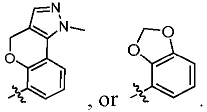

In one embodiment, disclosed herein is a compound of Formula (I-B): 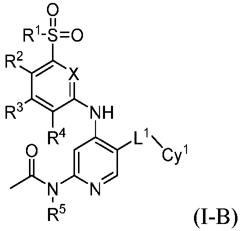 or a stereoisomer or a pharmaceutically acceptable salt thereof, wherein:
X is N or CH;
L¹ is a direct bond;
R¹ is -C₁₋₆alkyl, -haloC₁₋₆alkyl, -C₁₋₆ alkoxy, -haloC₁₋₆ alkoxyl, -C₃₋₆ cycloalkyl, aryl, or -NR^{c}R^{d};
each of R², R³, and R⁴ is independently hydrogen, halogen, -C₁₋₆alkyl, -C₂₋₆alkenyl, -C₂₋₆alkynyl, -C₃₋₈cycloalkyl, heterocyclyl (preferably, morpholin-4-yl, tetrahydrofuran-3-yl), aryl, heteroaryl, oxo, -CN, - NO₂, -OR^{e}, -SO₂R^{e}, -COR^{e}, -CO₂R^{e}, -CONR^{e}R^{f}, -C(=NR^{e})NR^{f}R^{g}, -NR^{e}R^{f}, -NR^{e}COR^{f}, -NR^{e}CONR^{f}R^{g}, - NR^{e}CO₂R^{f}, -NR^{e}SONR^{f}R^{g}, -NR^{e}SO₂NR^{f}R^{g}, or -NR^{e}SO₂R^{f},
wherein each of -C₁₋₆alkyl, -C₂₋₆alkenyl, -C₂₋₆alkynyl, -C₃₋₈cycloalkyl, heterocyclyl, aryl, or heteroaryl is independently and optionally substituted with at least one substituents selected from halogen, hydroxy-C₁₋₆alkyl (preferably, hydroxymethyl), -OR^{h}, or C₁₋₆alkoxy-C₁₋₆alkyl-;
wherein R¹¹ is hydrogen, alkyl, or heterocyclyl,
R^{e}, R^{f}, and R^{g} are each independently hydrogen, -C₁₋₆alkyl, -C₁₋₆alkoxy, -C₂₋₆alkenyl, -C₂₋₆alkynyl, cycloalkyl, heterocyclyl, aryl, or heteroaryl, wherein each of -C₁₋₆alkyl, -C₂₋₆alkenyl, -C₂₋₆alkynyl, cycloalkyl, heterocyclyl, aryl, or heteroaryl is optionally independently substituted with one to three substituents selected from halogen, hydroxy, cyano, -C₁₋₆alkoxy; -C₃₋₆cycloalkyl optionally substituted with halogen, hydroxy, or C₁₋₆alkoxy;
R⁵ is hydrogen or C₁₋₆alkyl;
Cy¹ is 6- to 12-membered aryl or 5- to 14- membered heteroaryl, or 5- to 14- membered heterocyclyl, each of which is optionally substituted with at least one substituent Rⁱ,
Rⁱ is independently halogen, cyano, -C₁₋₆alkyl, -C₂₋₆alkenyl, -C₂₋₆alkynyl, -C₃₋₈cycloalkyl, heterocyclyl, aryl, heteroaryl, oxo, -CN, -NO₂, -OR^{j}, -SO₂R^{j}, -COR^{j}, -CO₂R^{k}, -CONR^{j}R^{k}, -C(=NR^{j})NR^{k}R^{l}, -NR^{j}R^{k}, - NR^{j}COR^{k}, -NR^{j}CONR^{k}R^{l}, -NR^{j}CO₂R^{k}, -NR^{j}SONR^{k}R^{l}, -NR^{j}SO₂NR^{k}R^{l}, or -NR^{j}SO₂R^{k},
wherein each of said -C₁₋₆alkyl, -C₂₋₆alkenyl, -C₂₋₆alkynyl, -C₃₋₈cycloalkyl, heterocyclyl, aryl, or heteroaryl is optionally substituted with halogen, OR^{m}, C(O)R^{m}, -NR^{m}Rⁿ, -C₁₋₆alkyl, C₁₋₆alkoxy-, C₁₋₆alkoxy-C₁₋₆alkyl-, or oxo;
R^{j}, R^{k}, R^{l}, R^{m}, Rⁿ are each independently hydrogen, -C₁₋₆alkyl, C₁₋₆alkoxy-C₁₋₆alkyl-, -C₂₋₆alkenyl, -C_{2- 6}alkynyl, cycloalkyl, heterocyclyl, aryl, or heteroaryl;
or (R¹ and R²), or (R² and R³), or (R³ and R⁴), together with the atoms to which they are attached, form a fused ring system, said fused ring system comprises 0-4 heteroatoms selected from oxygen, nitrogen or sulfur as ring member(s) and is optionally and independently substituted with halogen, -C₁₋₆alkyl, -C₁₋₆alkoxy, -haloC₁₋₆alkyl, -haloC₁₋₆alkoxy, or -C₃₋₆cycloalkyl;
any of the said alkyl or alkoxy can be optionally enriched in deuterium.

In some embodiments, disclosed here is a compound of Formula (I-C) 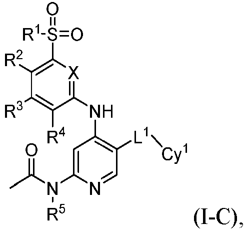 or a stereoisomer or a pharmaceutically acceptable salt thereof, wherein:
X is N or CH;
L¹ is a direct bond;
R¹ is -C₁₋₆alkyl, or -haloC₁₋₆alkyl;
each of R² and R⁴ is independently hydrogen, halogen, -C₁₋₆alkyl or -C₁₋₆alkoxy;
R³ is independently hydrogen, halogen, -C₁₋₆alkyl, -C₂₋₆alkenyl, -C₂₋₆alkynyl, -C₃₋₈cycloalkyl, heterocyclyl, aryl, heteroaryl, -oxo-, -CN, -NO₂, -OR^{e}, -SO₂R^{e}, -COR^{e}, -CO₂R^{e}, -CONR^{e}R^{f}, -C(=NR^{e})NR^{f}R^{g}, -NR^{e}R^{f}, - NR^{e}COR^{f}, -NR^{e}CONR^{f}R^{g}, -NR^{e}CO₂R^{f}, -NR^{e}SONR^{f}R^{g}, -NR^{e}SO₂NR^{f}R^{g}, or -NR^{e}SO₂R^{f},
wherein each of -C₁₋₆alkyl, -C₂₋₆alkenyl, -C₂₋₆alkynyl, -C₃₋₈cycloalkyl, heterocyclyl, aryl, or heteroaryl is independently and optionally substituted with at least one substituents selected from i) cyano, oxo, halogen, -NH₂, -OR^{h}, -C(O)NR^{m}Rⁿ;
ii) heterocyclyl optionally substituted with at least one substituent independently selected from cyano, - oxo, halogen, hydroxy, -NR^{m}Rⁿ, substituted or unsubstituted -C₁₋₆alkyl, substituted or unsubstituted -C₁₋₆alkoxy or -C(O)NR^{m}R; or,
iii) C₁₋₆alkyl optionally substituted with halogen, -C₁₋₆alkyl substituted with hydroxy (preferably, hydroxymethyl, hydroxyethyl), -C₁₋₆alkyl substituted with -NH₂, - NH₂ or -C₁₋₆alkyl substituted with C₁₋₆alkoxy;
wherein R^{h} is hydrogen, hydroxy, alkyl, substituted with hydroxy, or heterocyclyl,
R^{e}, R^{f}, and R^{g} are each independently hydrogen, -C₁₋₆alkyl, -C₁₋₆alkoxy, -C₂₋₆alkenyl, -C₂₋₆alkynyl, cycloalkyl, heterocyclyl, aryl, or heteroaryl, wherein each of -C₁₋₆alkyl, -C₂₋₆alkenyl, -C₂₋₆alkynyl, cycloalkyl, heterocyclyl, aryl, or heteroaryl is optionally independently substituted with one to three substituents selected from halogen, hydroxy, cyano, -oxo-, -NR^{m}Rⁿ, -C₁₋₆alkyl, -C₁₋₆alkoxy; -C₃₋₆cycloalkyl optionally substituted with halogen, hydroxy, or C₁₋₆alkoxy, -C(O)NR^{m}Rⁿ, or heterocyclyl;
R⁵ is hydrogen or C₁₋₆alkyl;
Cy¹ is a 7- to 14- membered bicyclic or tricyclic heterocyclyl or heteroaryl having 1, 2, or 3 heteroatoms selected from oxygen (O), nitrogen (N) or sulfur (S) as ring member(s), which is optionally substituted with at least one substituent Rⁱ,
Rⁱ is independently halogen, cyano, -C₁₋₆alkyl, -C₂₋₆alkenyl, -C₂₋₆alkynyl, -C₃₋₈cycloalkyl, heterocyclyl, aryl, heteroaryl, oxo, -CN, -NO₂, -OR^{j}, -SO₂R^{j}, -COR^{j}, -CO₂R^{k}, -CONR^{j}R^{k}, -C(=NR^{j})NR^{k}R^{l}, -NR^{j}R^{k}, - NR^{j}COR^{k}, -NR^{j}CONR^{k}R^{l}, -NR^{j}COR^{k}, -NR^{j}SONR^{k}R^{l}, -NR^{j}SO₂NR^{k}R^{l}, or -NR^{j}SO₂R^{k},
wherein each of said -C₁₋₆alkyl, -C₂₋₆alkenyl, -C₂₋₆alkynyl, -C₃₋₈cycloalkyl, heterocyclyl, aryl, or heteroaryl is optionally substituted with halogen, OR^{m}, C(O)R^{m}, -NR^{m}Rⁿ, -C₁₋₆alkyl, C₁₋₆alkoxy-, C₁₋₆alkyl substituted with -C₁₋₆alkoxy, or oxo;
R^{j}, R^{k}, R^{l}, R^{m}, Rⁿ are each independently hydrogen, -C₁₋₆alkyl, -C₁₋₆alkyl substituted with C₁₋₆alkoxy, -C₂₋₆alkenyl, -C₂₋₆alkynyl, cycloalkyl, heterocyclyl, aryl, or heteroaryl;
or (R¹ and R²), or (R² and R³), or (R³ and R⁴), together with the atoms to which they are attached, form a fused ring system, said fused ring system comprises 0-4 heteroatoms selected from oxygen (O), nitrogen (N) or sulfur (S) as ring member(s) and is optionally and independently substituted with halogen, -C₁₋₆alkyl, -C₁₋₆alkoxy, -haloC₁₋₆alkyl, -haloC₁₋₆alkoxy, or -C₃₋₆cycloalkyl;
any of the said alkyl or alkoxy is optionally enriched in deuterium.

In some embodiments, R¹ is -C₁₋₃ alkyl, preferably methyl, ethyl, propyl, or isopropyl.

In some embodiments, R² and R⁴ are each independently hydrogen, halogen, -C₁₋₃alkyl, or -C₁₋₃alkoxy, preferably hydrogen, fluoro, methyl, methoxy, ethoxy, or isopropoxy.

In some embodiments, R³ is
- hydrogen;
- cyano;
- halogen;
- -C₁₋₄ alkyl optionally substituted with at least one substituent independently selected from halogen, 3- to 6-membered heterocyclyl or -OR^{h}, wherein
   3- to 6-membered heterocyclyl comprises one or two heteroatoms selected from oxygen (O), nitrogen (N) or sulfur (O) as ring member(s), optionally substituted with at least one substituent independently selected from cyano, -oxo, halogen, hydroxy, -NR^{m}Rⁿ, C₁₋₆alkyl, -C₁₋₆alkoxy or -C(O)NR^{m}Rⁿ, wherein -C₁₋₆alkyl or -C₁₋₆alkoxy is substituted with at least one substitution independently selected from cyano, halogen, hydroxy, -NH₂, -C₁₋₆alkyl or C₁₋₆alkoxy; and
   R^{h} is hydrogen, alkyl, or heterocyclyl;
- -C₃₋₆cycloalkyl, optionally substituted with at least one substituent independently selected from cyano, -oxo, halogen, -NR^{m}Rⁿ, hydroxy, -C₁₋₆alkyl, -C₁₋₆alkoxy or -C(O)NR^{m}Rⁿ, wherein -C₁₋₆alkyl or C₁₋₆alkoxy is substituted with at least one substitution independently selected from cyano, halogen, hydroxy, - NH₂, -C₁₋₆alkyl or C₁₋₆alkoxy;
- heterocyclyl comprising one or two heteroatoms selected from oxygen (O), nitrogen (N) or sulfur (S) as ring member(s), optionally substituted with at least one substituent independently selected from cyano, -oxo-, halogen, hydroxy, C₁₋₆alkyl, alkoxy, -NRR^{m}Rⁿ, or -C(O)NR^{m}Rⁿ, and wherein -C₁₋₆alkyl or C₁₋₆alkoxy is substituted with at least one substitution independently selected from cyano, halogen, hydroxy, -NH₂, -C₁₋₆alkyl or C₁₋₆alkoxy;
- -OR^{e}, wherein R^{e} is -C₁₋₆alkyl, -C₃₋₆cycloalkyl, 3- to 6-membered heterocyclyl , or aryl, wherein
   i) -C₁₋₆alkyl is optionally substituted with cyano, -oxo-, halogen, hydroxy, -NR^{m}Rⁿ, -C₁₋₆alkoxy-, -C₃₋₆cycloalkyl optionally substituted with cyano, -oxo, halogen, hydroxy, -NR^{m}Rⁿ, -C₁₋₆alkyl, C₁₋₆alkoxy or - C(O)NR^{m}Rⁿ, 4- to 6-membered heterocyclyl optionally substituted with cyano, halogen, hydroxy, C₁₋₆alkyl or C₁₋₆alkoxy; and,
   ii) -C₃₋₆cycloalkyl or 3- to 6-membered heterocyclyl is optionally substituted with cyano, -oxo, halogen, hydroxy, -NR^{m}Rⁿ, C₁₋₆alkyl, C₁₋₆alkoxy or -C(O)NR^{m}Rⁿ, wherein -C₁₋₆alkyl or C₁₋₆alkoxy is substituted with at least one substitution independently selected from cyano, halogen, hydroxy, -NH₂, -C₁₋₆alkyl or C₁₋₆alkoxy;
- -C₅₋₁₀aryl; or
- heteroaryl comprising one oxygen (O), nitrogen (N) or sulfur (S) heteroatom as ring member, optionally substituted with at least one substitution independently selected from cyano, -oxo, halogen, hydroxy, -NR^{m}Rⁿ, -C₁₋₆alkyl, -C₁₋₆alkoxy or -C(O)NR^{m}Rⁿ, wherein -C₁₋₆alkyl or C₁₋₆alkoxy is substituted with at least one substitution independently selected from cyano, halogen, hydroxy, -NH₂, -C₁₋₆alkyl or C₁₋₆alkoxy;
   and wherein R^{m} and Rⁿ are independently selected from hydrogen or C₁₋₃alkyl;
   any of the said alkyl or alkoxy is optionally enriched in deuterium.
   In some preferred embodiments, R³ is heterocyclyl comprising one or two heteroatoms selected from oxygen (O), nitrogen (N) or sulfur (S) as ring member(s), optionally substituted with at least one substituent independently selected from cyano, -oxo-, halogen, hydroxy, C₁₋₆alkyl, alkoxy, -NRR^{m}Rⁿ, or - C(O)NR^{m}Rⁿ, and wherein -C₁₋₆alkyl or C₁₋₆alkoxy is substituted with at least one substitution independently selected from cyano, halogen, hydroxy, -NH₂, -C₁₋₆alkyl or C₁₋₆alkoxy.

In some embodiments, R³ is
- hydrogen;
- cyano;
- halogen;
- -C₁₋₄ alkyl optionally substituted with at least one substituent independently selected from halogen, 3- to 6-membered heterocyclyl or -OR^{h}, wherein
   Said 3- to 6-membered heterocyclyl comprises one or two heteroatoms selected from oxygen (O), nitrogen (N) or sulfur (O) as ring member(s), optionally substituted with at least one substituent independently selected from cyano, -oxo, halogen, hydroxy, -NR^{m}Rⁿ, C₁₋₆alkyl, -C₁₋₆alkoxy or -C(O)NR^{m}Rⁿ, wherein -C_{1 6}alkyl or C₁₋₆alkoxy is substituted with at least one substitution independently selected from cyano, halogen, hydroxy, -NH₂, -C₁₋₆alkyl or C₁₋₆alkoxy, and,
   R^{h} is hydrogen, alkyl or heterocyclyl (preferably 3- to 6-membered heterocyclyl, e.g., tetrahydrofuranyl, thiazolidinyl);
- -C₃₋₆cycloalkyl, optionally substituted with at least one substituent independently selected from cyano, -oxo, halogen, -NR^{m}Rⁿ, hydroxy, -C₁₋₆alkyl, -C₁₋₆alkoxy or -C(O)NR^{m}Rⁿ, wherein -C₁₋₆alkyl or C₁₋₆alkoxy is substituted with at least one substitution independently selected from cyano, halogen, hydroxy, - NH₂, -C₁₋₆alkyl or C₁₋₆alkoxy;
- heterocyclyl, preferably 4- to 6-membered monocyclic saturated heterocyclyl, saturated mono-spiro heterocyclyl, saturated bicyclic fused heterocyclyl, or saturated bridged heterocyclyl, comprising one or two heteroatoms selected from oxygen (O), nitrogen (N) or sulfur (S) as ring member(s), more preferably morpholinyl, tetrahydrofuranyl, tetrahydropyranyl, pyrrolidinyl, 1,4-dioxanyl, piperidinyl or azetidinyl, optionally substituted with at least one substituent independently selected from cyano, -oxo, halogen, hydroxy, -C₁₋₆alkyl, alkoxy, -NR^{m}Rⁿ, or -C(O)NR^{m}Rⁿ, and wherein -C₁₋₆alkyl or -C₁₋₆alkoxy is substituted with at least one substitution independently selected from cyano, halogen, hydroxy, -NH₂, -C₁₋₆alkyl or C₁₋₆alkoxy;
- -OR^{e}, wherein R^{e} is -C₁₋₆alkyl, -C₃₋₆cycloalkyl, 3- to 6-membered heterocyclyl (preferably 4- to 6-membered monocyclic saturated heterocyclyl comprising one oxygen heteroatom as ring member), or aryl, wherein
   i) -C₁₋₆alkyl is optionally substituted with cyano, -oxo-, halogen, hydroxy, -NR^{m}Rⁿ, -C₁₋₆alkoxy-, -C₃₋₆cycloalkyl optionally substituted with cyano, -oxo, halogen, hydroxy, -NR^{m}Rⁿ, -C₁₋₆alkyl, C₁₋₆alkoxy or - C(O)NR^{m}Rⁿ, 4- to 6-membered heterocyclyl optionally substituted with cyano, halogen, hydroxy, -C₁₋₆alkyl or -C₁₋₆alkoxy; and,
   ii) -C₃₋₆cycloalkyl or 3- to 6-membered heterocyclyl is optionally substituted with cyano, -oxo, halogen, hydroxy, NR^{m}Rⁿ, C₁₋₆alkyl, C₁₋₆alkoxy or -C(O)NR^{m}Rⁿ, wherein -C₁₋₆alkyl or C₁₋₆alkoxy is substituted with at least one substitution independently selected from cyano, halogen, hydroxy, -NH₂, -C₁₋₆alkyl or C₁₋₆alkoxy;
- -C₅₋₁₀aryl; or
- heteroaryl, preferably 5- to 6-membered heteroaryl comprising one oxygen (O), nitrogen (N) or sulfur (S) heteroatom as ring member, optionally substituted with at least one substitution independently selected from cyano, -oxo, halogen, hydroxy, -NR^{m}Rⁿ, -C₁₋₆alkyl, -C₁₋₆alkoxy or -C(O)NR^{m}Rⁿ, wherein - C₁₋₆alkyl or C₁₋₆alkoxy is substituted with at least one substitution independently selected from cyano, halogen, hydroxy, -NH₂, -C₁₋₆alkyl or C₁₋₆alkoxy;
   and wherein R^{m} and Rⁿ are independently selected from hydrogen or C₁₋₃alkyl.
   In some preferred embodiments, R³ is heterocyclyl, preferably 4- to 6-membered monocyclic saturated heterocyclyl, saturated mono-spiro heterocyclyl, saturated bicyclic fused heterocyclyl, or saturated bridged heterocyclyl, comprising one or two heteroatoms selected from oxygen (O), nitrogen (N) or sulfur (S) as ring member(s), more preferably morpholinyl, tetrahydrofuranyl, tetrahydropyranyl, pyrrolidinyl, 1,4-dioxanyl, piperidinyl or azetidinyl, optionally substituted with at least one substituent independently selected from cyano, -oxo, halogen, hydroxy, -C₁₋₆alkyl, alkoxy, -NR^{m}Rⁿ, or -C(O)NR^{m}Rⁿ, and wherein - C₁₋₆alkyl or -C₁₋₆alkoxy is substituted with at least one substitution independently selected from cyano, halogen, hydroxy, -NH₂, -C₁₋₆alkyl or C₁₋₆alkoxy.

**In** some embodiments, R³ is
- hydrogen, cyano, halogen;
- -C₁₋₄ alkyl optionally substituted with at least one substituent independently selected from halogen, 3- to 6-membered heterocyclyl or -OR^{h}, wherein
   Said 3- to 6-membered heterocyclyl is selected from morpholinyl, tetrahydrofuranyl, tetrahydropyranyl, pyrrolidinyl, 1,4-dioxanyl, piperidinyl, thiazolidinyl or azetidinyl, each of which is optionally substituted with at least one substituent independently selected from cyano, -oxo, halogen, hydroxy, -NR^{m}Rⁿ, C₁₋₆alkyl, -C₁₋₆alkoxy or -C(O)NR^{m}Rⁿ, wherein -C₁₋₆alkyl or C₁₋₆alkoxy is substituted with at least one substitution independently selected from cyano, halogen, hydroxy, -NH₂, -C₁₋₆alkyl or C₁₋₆alkoxy, and, R^{h} is hydrogen, C₁₋₆alkyl or 3- to 6-membered heterocyclyl (e.g., tetrahydrofuranyl or thiazolidinyl);
- -C₃₋₆cycloalkyl, optionally substituted with at least one substituent independently selected from cyano, -oxo, halogen, NR^{m}Rⁿ, hydroxy, -C₁₋₆alkyl, -C₁₋₆alkoxy or -C(O)NR^{m}Rⁿ, hydroxy, or C₁₋₆alkoxy, wherein -C₁₋₆alkyl or C₁₋₆alkoxy is substituted with at least one substitution independently selected from cyano, halogen, hydroxy, -NH₂, -C₁₋₆alkyl or C₁₋₆alkoxy;
- heterocyclyl is selected from morpholinyl, tetrahydrofuranyl, tetrahydropyranyl, pyrrolidinyl, 1,4-dioxanyl, piperidinyl or azetidinyl, optionally substituted with at least one substituent independently selected from cyano, -oxo, halogen, hydroxy, C₁₋₆alkyl, alkoxy, -NRR^{m}Rⁿ, or -C(O)NR^{m}Rⁿ, and wherein - C₁₋₆alkyl or -C₁₋₆alkoxy is substituted with at least one substitution independently selected from cyano, halogen, hydroxy, -NH₂, -C₁₋₆alkyl or -C₁₋₆alkoxy;
- -OR^{e}, wherein R^{e} is -C₁₋₆alkyl, -C₃₋₆cycloalkyl, 4- to 6-membered monocyclic saturated heterocyclyl comprising one oxygen heteroatom as ring member, or C₆₋₁₀aryl, wherein
   i) -C₁₋₆alkyl is optionally substituted with cyano, -oxo-, halogen, hydroxy, -NR^{m}Rⁿ, -C₁₋₆alkoxy-, -C₃₋₆cycloalkyl optionally substituted with cyano, -oxo, halogen, hydroxy, -NR^{m}Rⁿ, -C₁₋₆alkyl, -C₁₋₆alkoxy or - C(O)NR^{m}Rⁿ, 4- to 6-membered heterocyclyl optionally substituted with cyano, halogen, hydroxy, -C₁₋₆alkyl or -C₁₋₆alkoxy; and,
   ii) -C₃₋₆cycloalkyl or 3- to 6-membered heterocyclyl is optionally substituted with cyano, -oxo, halogen, hydroxy, NR^{m}Rⁿ, C₁₋₆alkyl, C₁₋₆alkoxy or -C(O)NR^{m}Rⁿ, wherein -C₁₋₆alkyl or C₁₋₆alkoxy is substituted with at least one substitution independently selected from cyano, halogen, hydroxy, -NH₂, -C₁₋₆alkyl or C₁₋₆alkoxy;
- -C₆₋₁₀aryl; or
- 5- to 6-membered heteroaryl selected from pyridinyl, pyridazinyl, pyrazinyl, thiazolyl or isoxazolyl, each of which is optionally substituted with at least one substitution independently selected from cyano, -oxo, halogen, hydroxy, -NR^{m}Rⁿ, -C₁₋₆alkyl, -C₁₋₆alkoxy or -C(O)NR^{m}Rⁿ, wherein -C₁₋₆alkyl or C₁₋₆alkoxy is substituted with at least one substitution independently selected from cyano, halogen, hydroxy, -NH₂, -C₁₋₆alkyl or C₁₋₆alkoxy;
   and wherein R^{m} and Rⁿ are independently selected from hydrogen or -C₁₋₃alkyl.
   In some preferred embodiments, R³ is heterocyclyl is selected from morpholinyl, tetrahydrofuranyl, tetrahydropyranyl, pyrrolidinyl, 1,4-dioxanyl, piperidinyl or azetidinyl, optionally substituted with at least one substituent independently selected from cyano, -oxo, halogen, hydroxy, C₁₋₆alkyl, alkoxy, -NRR^{m}Rⁿ, or -C(O)NR^{m}Rⁿ, and wherein -C₁₋₆alkyl or -C₁₋₆alkoxy is substituted with at least one substitution independently selected from cyano, halogen, hydroxy, -NH₂, -C₁₋₆alkyl or -C₁₋₆alkoxy.

**In** some embodiments, R³ is
- hydrogen, cyano, halogen;
- -C₁₋₄ alkyl optionally substituted with at least one substituent independently selected from halogen, hydroxy, C₁₋₃alkoxy, thiazolidin-3-yl optionally substituted with at least one substituent independently selected from cyano, -oxo, halogen, hydroxy, -NR^{m}Rⁿ, or -C₁₋₃alkyl;
- -C₃₋₆cycloalkyl, optionally substituted with at least one substituent independently selected from cyano, -oxo, halogen, -NR^{m}Rⁿ, hydroxy, -C₁₋₆alkyl, or -C₁₋₆alkoxy, wherein -C₁₋₆alkyl or C₁₋₆alkoxy is substituted with at least one substitution independently selected from cyano, halogen, hydroxy, -NH₂, -C₁₋₆alkyl or -C₁₋₃alkoxy;
- heterocyclyl is selected from morpholinyl, tetrahydrofuranyl, tetrahydropyranyl, pyrrolidinyl, 1,4-dioxanyl, piperidinyl or azetidinyl, optionally substituted with at least one substituent independently selected from cyano, -oxo, halogen, hydroxy, C₁₋₆alkyl, alkoxy, -NR^{m}Rⁿ, or -C(O)NR^{m}Rⁿ, and wherein -C₁₋₆alkyl or C₁₋₆alkoxy is substituted with at least one substitution independently selected from cyano, halogen, hydroxy, -NH₂, -C₁₋₆alkyl or -C₁₋₆alkoxy;
- -OR^{e}, wherein R^{e} is -C₁₋₆alkyl, -C₃₋₆cycloalkyl, 4- to 6-membered monocyclic saturated heterocyclyl comprising one oxygen heteroatom as ring member, or C₆₋₁₀aryl, wherein
   i) -C₁₋₆alkyl is optionally substituted with cyano, -oxo-, halogen, hydroxy, -NR^{m}Rⁿ, -C₁₋₆alkoxy-, -C₃₋₆cycloalkyl optionally substituted with cyano, -oxo, halogen, hydroxy, -NR^{m}Rⁿ, -C₁₋₆alkyl, -C₁₋₆alkoxy or - C(O)NR^{m}Rⁿ, 4- to 6-membered heterocyclyl optionally substituted with cyano, halogen, hydroxy, -C₁₋₆alkyl or -C₁₋₆alkoxy; and,
   ii) -C₃₋₆cycloalkyl or 3- to 6-membered heterocyclyl is optionally substituted with cyano, -oxo, halogen, hydroxy, -NR^{m}Rⁿ, -C₁₋₆alkyl, -C₁₋₆alkoxy or -C(O)NR^{m}Rⁿ, wherein -C₁₋₆alkyl or -C₁₋₆alkoxy is substituted with at least one substitution independently selected from cyano, halogen, hydroxy, -NH₂, -C₁₋₆alkyl or -C₁₋₆alkoxy;
- -C₆₋₁₀aryl; or
- 5- to 6-membered heteroaryl selected from pyridinyl, pyridazinyl, pyrazinyl, thiazolyl or isoxazolyl, each of which is optionally substituted with at least one substitution independently selected from cyano, -oxo, halogen, hydroxy, -NR^{m}Rⁿ, -C₁₋₆alkyl, -C₁₋₆alkoxy or -C(O)NR^{m}Rⁿ, wherein -C₁₋₆alkyl or -C₁₋₆alkoxy is substituted with at least one substitution independently selected from cyano, halogen, hydroxy, -NH₂, -C₁₋₆alkyl or -C₁₋₆alkoxy;
   and wherein R^{m} and Rⁿ are independently selected from hydrogen or -C₁₋₃alkyl.
   In some preferred embodiments, R³ is heterocyclyl is selected from morpholinyl, tetrahydrofuranyl, tetrahydropyranyl, pyrrolidinyl, 1,4-dioxanyl, piperidinyl or azetidinyl, optionally substituted with at least one substituent independently selected from cyano, -oxo, halogen, hydroxy, C₁₋₆alkyl, alkoxy, -NR^{m}Rⁿ, or - C(O)NR^{m}Rⁿ, and wherein -C₁₋₆alkyl or C₁₋₆alkoxy is substituted with at least one substitution independently selected from cyano, halogen, hydroxy, -NH₂, -C₁₋₆alkyl or -C₁₋₆alkoxy

**In** some embodiments, R³ is
- hydrogen, cyano, halogen;
- methyl, ethyl, propyl or butyl, each of which optionally substituted with at least one substituent independently selected from halogen, hydroxy, methoxy, ethoxy, propoxy, or 2,4-dioxothiazolidin-3-yl;
- cyclopropyl, cyclobutyl, cyclopentyl or cyclohexyl, each of which is optionally substituted with at least one substituent independently selected from cyano, -oxo, halogen, -NR^{m}Rⁿ, hydroxy, -C₁₋₃alkyl, or - C₁₋₃alkoxy, wherein -C₁₋₃alkyl or C₁₋₃alkoxy is optionally substituted with at least one substitution independently selected from cyano, halogen, hydroxy, -NH₂, -C₁₋₃alkyl or -C₁₋₃alkoxy;
- heterocyclyl is selected from morpholin-2-yl, morpholin-3-yl, morpholin-4-yl, tetrahydrofuran-2-yl, tetrahydrofuran-3-yl, tetrahydropyran-2-yl, tetrahydropyran-3-yl, tetrahydropyran-4-yl, pyrrolidine-1-yl, pyrrolidin-2-yl, pyrrolidin-3-yl, 1,4-dioxan-2-yl, piperidin-1-yl, piperidin-2-yl, piperidin-3-yl, piperidin-4-yl, azetidin-1-yl, azetidine-2-yl, azetidin-3-yl, 5-azaspiro[2.4]heptanyl, 3-azabicyclo[3.1.0]hexan-3-yl or 2-azabicyclo[3.1.0]hexan-2-yl, each of which is optionally substituted with at least one substituent independently selected from cyano, -oxo, halogen, hydroxy, -C₁₋₆alkyl, -C₁₋₆alkoxy, -NR^{m}Rⁿ, or - C(O)NR^{m}Rⁿ, and wherein -C₁₋₆alkyl or C₁₋₆alkoxy is substituted with at least one substitution independently selected from cyano, halogen, hydroxy, -NH₂, -C₁₋₆alkyl or -C₁₋₆alkoxy;
- -OR^{e}, wherein R^{e} is
   i) methyl, ethyl, propyl (iso-propyl), butyl, pentyl or hexyl, each of which is optionally substituted with deuterium, cyano, -oxo-, halogen, hydroxy, -NR^{m}Rⁿ, -C₁₋₃alkoxy-, -C₃₋₆cycloalkyl optionally substituted with cyano, -oxo, halogen, hydroxy, -NR^{m}Rⁿ, -C₁₋₃alkyl, -C₁₋₃alkoxy or -C(O)NR^{m}Rⁿ, or 4- to 6-membered heterocyclyl optionally substituted with cyano, halogen, hydroxy, -C₁₋₃alkyl or -C₁₋₃alkoxy; or,
   ii) cyclopropyl, cyclobutyl, cyclopentyl, cyclohexyl, morpholin-2-yl, morpholin-3-yl, morpholin-4-yl, tetrahydrofuran-2-yl, tetrahydrofuran-3-yl, tetrahydropyran-2-yl, tetrahydropyran-3-yl, tetrahydropyran-4-yl, pyrrolidin-1-yl, pyrrolidin-2-yl, pyrrolidin-3-yl, 1,4-dioxan-2-yl, piperidin-1-yl, piperidin-2-yl, piperidin-3-yl, piperidin-4-yl, azetidin-1-yl, azetidine-2-yl or azetidin-3-yl, each of which is optionally substituted with cyano, -oxo, halogen, hydroxy, -NR^{m}Rⁿ, -C₁₋₃alkyl, -C₁₋₃alkoxy or -C(O)NR^{m}Rⁿ, wherein - C₁₋₃alkyl or -C₁₋₃alkoxy is substituted with at least one substitution independently selected from cyano, halogen, hydroxy, -NH₂, -C₁₋₃alkyl or -C₁₋₃alkoxy;
- -C₆₋₁₀aryl; or
- 5- to 6-membered heteroaryl selected from pyridin-1-yl, pyridin-2-yl, pyridin-3-yl, pyridin-4-yl, pyridazin-1-yl, pyridazin-2-yl, pyridazin-3-yl, pyridazin-4-yl, pyrazin-1-yl, pyrazin-2-yl, thiazol-2-yl, thiazol-3-yl, thiazol-4-yl, isoxazol-2-yl, isoxazol-3-yl or isoxazol-4-yl, each of which is optionally substituted with at least one substitution independently selected from cyano, -oxo, halogen, hydroxy, - NR^{m}Rⁿ, -C₁₋₃alkyl, -C₁₋₃alkoxy or -C(O)NR^{m}Rⁿ, wherein -C₁₋₃alkyl or -C₁₋₃alkoxy is substituted with at least one substitution independently selected from cyano, halogen, hydroxy, -NH₂, -C₁₋₃alkyl or C₁₋₃alkoxy;
   and wherein R^{m} and Rⁿ are independently selected from hydrogen or -C₁₋₃alkyl.
   In some preferred embodiments, R³ is heterocyclyl is selected from morpholin-2-yl, morpholin-3-yl, morpholin-4-yl, tetrahydrofuran-2-yl, tetrahydrofuran-3-yl, tetrahydropyran-2-yl, tetrahydropyran-3-yl, tetrahydropyran-4-yl, pyrrolidine-1-yl, pyrrolidin-2-yl, pyrrolidin-3-yl, 1,4-dioxan-2-yl, piperidin-1-yl, piperidin-2-yl, piperidin-3-yl, piperidin-4-yl, azetidin-1-yl, azetidine-2-yl, azetidin-3-yl, 5-azaspiro[2.4]heptanyl, 3-azabicyclo[3.1.0]hexan-3-yl or 2-azabicyclo[3.1.0]hexan-2-yl, each of which is optionally substituted with at least one substituent independently selected from cyano, -oxo, halogen, hydroxy, -C₁₋₆alkyl, -C₁₋₆alkoxy, -NR^{m}Rⁿ, or -C(O)NR^{m}Rⁿ, and wherein -C₁₋₆alkyl or C₁₋₆alkoxy is substituted with at least one substitution independently selected from cyano, halogen, hydroxy, -NH₂, -C₁₋₆alkyl or -C₁₋₆alkoxy

**In** some embodiments, R³ is
- Hydrogen;
- Methyl, 1-methoxyethyl, 2-hydroxypropan-2-yl, 1-methoxyethyl, or (2,4-dioxothiazolidin-3-yl)methyl;
- Isopropoxy, methoxy-d3, methoxy, ethoxy, difluoromethoxy, 2-methoxyethoxy, 2-methoxy-2-methylpropoxy, 2-hydroxy-2-methylpropoxy, cyclopropylmethoxy, (1,4-dioxan-2-yl)methoxy, (4-hydroxycyclohexyl)oxy, (cis-4-hydroxycyclohexyl)oxy, (trans-4-hydroxycyclohexyl)oxy, (4-methoxycyclohexyl)oxy, (cis-4-methoxycyclohexyl)oxy, (trans-4-methoxycyclohexyl)oxy, or (3-methyloxetan-3-yl)methoxy;
- cyano
- 3-methoxycyclobutyl, (trans)-3-methoxycyclobutyl, (cis)-3-methoxycyclobutyl, 2,2-dichlorocyclopropyl, or 1-cyanocyclopropyl;
- Morpholino, 3-methyl-morpholino, 3(R)-methyl-morpholino, 3(S)-methyl-morpholino;
- tetrahydro-2H-pyran-4-yl, tetrahydro-2H-pyran-3-yl, (R)-tetrahydro-2H-pyran-3-yl, (S)-tetrahydro-2H-pyran-3-yl, 2,2,6,6-tetramethyltetrahydro-2H-pyran-4-yl, 3,3-dimethylmorpho;
- 3-methoxypyrrolidin-1-yl, 3(R)-methoxypyrrolidin-1-yl, 3(S)-methoxypyrrolidin-1-yl, 3-hydroxy-3-methylpyrrolidin-1-yl, 3-(2-hydroxyethoxy)pyrrolidin-1-yl, 3-(trifluoromethoxy)pyrrolidin-1-yl, 3(R)-(trifluoromethoxy)pyrrolidin-1-yl, 3(S)-(trifluoromethoxy)pyrrolidin-1-yl, 2-(aminocarbonyl)pyrrolidin-1-yl, 2(R)-(aminocarbonyl)pyrrolidin-1-yl, 2(S)-(aminocarbonyl)pyrrolidin-1-yl, 3-(methoxymethyl)pyrrolidin-1-yl, 3(R)-(methoxymethyl)pyrrolidin-1-yl, 3(S)-(methoxymethyl)pyrrolidin-1-yl, 3-cyano-4-hydroxypyrrolidin-1-yl, cis-3-cyano-4-hydroxypyrrolidin-1-yl, trans-3-cyano-4-hydroxypyrrolidin-1-yl, 3-cyano-4-methoxypyrrolidin-1-yl, cis-3-cyano-4-methoxypyrrolidin-1-yl, trans-3-cyano-4-methoxypyrrolidin-1-yl, 2-(methoxymethyl)pyrrolidin-1-yl, 2(R)-(methoxymethyl)pyrrolidin-1-yl, 2(S)-(methoxymethyl)pyrrolidin-1-yl, 3-methylpyrrolidin-1-yl, 3(R)-methylpyrrolidin-1-yl, 3(S)-methylpyrrolidin-1-yl, pyrrolidin-1-yl, 3-(cyanomethoxy)pyrrolidin-1-yl;
- 5-azaspiro[2.4]heptan-5-yl;
- tetrahydrofuran-3-yl;
- 3-methoxyazetidin-1-yl, 3-hydroxy-3-methylazetidin-1-yl;
- 1,4-dioxan-2-yl;
- 4-aminotetrahydro-2H-pyran-4-yl, 4-(aminomethyl)tetrahydro-2H-pyran-4-yl,
- 4-methoxypiperidin-1-yl, 4-hydroxy-4-methylpiperidin-1-yl, 1-(2,2,2-trifluoroethyl)piperidin-4-yl, 3-methoxypiperidin-1-yl, 3(R)-methoxypiperidin-1-yl, 3(S)-methoxypiperidin-1-yl, 3-ethoxypiperidin-1-yl, 3(R)-ethoxypiperidin-1-yl, 3(S)-ethoxypiperidin-1-yl;
- 3-cyano-2-azabicyclo[3.1.0]hexan-2-yl, (3R)-3-cyano-2-azabicyclo[3.1.0]hexan-2-yl, (3S)-3-cyano-2-azabicyclo[3.1.0]hexan-2-yl, 3-azabicyclo[3.1.0]hexan-3-yl;
- 4-methylpyridin-3-yl, 5-methylpyridazin-4-yl, 5-methoxypyridazin-4-yl, 3,5-dimethylisoxazol-4-yl, 4-methoxypyridin-3-yl, 4-(2-hydroxypropan-2-yl)pyridin-3-yl, 6-cyanopyridin-3-yl, 4-cyanopyridin-3-yl, 2-cyanopyridin-3-yl, 3-methylpyrazin-2-yl, 5-cyanopyridazin-4-yl, 5-fluoropyridazin-4-yl, 4-fluoropyridin-3-yl, 4-isopropylpyridin-3-yl, 4-(1-hydroxyethyl)pyridin-3-yl, 4-(1-methoxyethyl)pyridin-3-yl, pyridin-2-yl, or thiazol-4-yl.

In some preferred embodiments, R³ is
- Morpholino, 3-methyl-morpholino, 3(R)-methyl-morpholino, 3(S)-methyl-morpholino;
- tetrahydro-2H-pyran-4-yl, tetrahydro-2H-pyran-3-yl, (R)-tetrahydro-2H-pyran-3-yl, (S)-tetrahydro-2H-pyran-3-yl, 2,2,6,6-tetramethyltetrahydro-2H-pyran-4-yl;
- 3-methoxypyrrolidin-1-yl, 3(R)-methoxypyrtrolidin-1-yl, 3(S)-methoxypyrrolidin-1-yl, 3-hydroxy-3-methylpyrrolidin-1-yl, 3-(2-hydroxyethoxy)pyrrolidin-1-yl, 3-(trifluoromethoxy)pyrrolidin-1-yl, 3(R)-(trifluoromethoxy)pyrrolidin-1-yl, 3(S)-(trifluoromethoxy)pyrrolidin-1-yl, 2-(aminocarbonyl)pyrrolidin-1-yl, 2(R)-(aminocarbonyl)pyrrolidin-1-yl, 2(S)-(aminocarbonyl)pyrrolidin-1-yl, 3-(methoxymethyl)pyrrolidin-1-yl, 3(R)-(methoxymethyl)pyrrolidin-1-yl, 3(S)-(methoxymethyl)pyrrolidin-1-yl, 3-cyano-4-hydroxypyrrolidin-1-yl, cis-3-cyano-4-hydroxypyrrolidin-1-yl, trans-3-cyano-4-hydroxypyrrolidin-1-yl, 3-cyano-4-methoxypyrrolidin-1-yl, cis-3-cyano-4-methoxypyrrolidin-1-yl, trans-3-cyano-4-methoxypyrrolidin-1-yl, 2-(methoxymethyl)pyrrolidin-1-yl, 2(R)-(methoxymethyl)pyrrolidin-1-yl, 2(S)-(methoxymethyl)pyrrolidin-1-yl, 3-methylpyrrolidin-1-yl, 3(R)-methylpyrrolidin-1-yl, 3(S)-methylpyrrolidin-1-yl, pyrrolidin-1-yl, 3-(cyanomethoxy)pyrrolidin-1-yl;
- 5-azaspiro[2.4]heptan-5-yl;
- tetrahydrofuran-3-yl;
- 3-methoxyazetidin-1-yl, 3-hydroxy-3-methylazetidin-1-yl;
- 1,4-dioxan-2-yl;
- 4-aminotetrahydro-2H-pyran-4-yl, 4-(aminomethyl)tetrahydro-2H-pyran-4-yl,
- 4-methoxypiperidin-1-yl, 4-hydroxy-4-methylpiperidin-1-yl, 1-(2,2,2-trifluoroethyl)piperidin-4-yl, 3-methoxypiperidin-1-yl, 3(R)-methoxypiperidin-1-yl, 3(S)-methoxypiperidin-1-yl, 3-ethoxypiperidin-1-yl, 3(R)-ethoxypiperidin-1-yl, 3(S)-ethoxypiperidin-1-yl;
- 3-cyano-2-azabicyclo[3.1.0]hexan-2-yl, (3R)-3-cyano-2-azabicyclo[3.1.0]hexan-2-yl, (3S)-3-cyano-2-azabicyclo[3.1.0]hexan-2-yl, or 3-azabicyclo[3.1.0]hexan-3-yl.

In some further preferred embodiments, R³ is morpholino, 3-methyl-morpholino, 3(R)-methyl-morpholino, or 3(S)-methyl-morpholino.

**In** some embodiments, (R¹ and R²), or (R² and R³), or (R³ and R⁴), together with the atoms to which they are attached, form a fused 5- to 7-membered ring system, said fused ring system comprises 0-2 oxygen heteroatoms as ring member(s) and is optionally and independently substituted with halogen, -C₁₋₆alkyl, -C₁₋₆alkoxy, -haloC₁₋₆alkyl, -haloC₁₋₆alkoxy, or -C₃₋₆cycloalkyl.

**In** some embodiments, R¹ and R², together with the atoms to which they are attached, form a fused ring system selected from 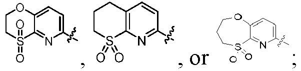 or R² and R³, together with the atoms to which they are attached, form a fused ring system 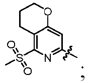 R³ and R⁴, together with the atoms to which they are attached, form a fused ring system selected from 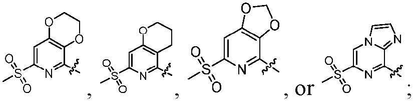 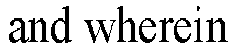 each of fused ring system is optionally and independently substituted with halogen, -C₁₋₆alkyl, -C₁₋₆alkoxy, -haloC₁₋₆alkyl, -haloC₁₋₆alkoxy, or -C₃₋₆cycloalkyl .

**In** some embodiments, Cy¹ is
- said 7- to 14-membered bicyclic or tricyclic heteroaryl comprising 1, 2, or 3 heteroatom(s) selected from oxygen, nitrogen or sulfur as ring member(s), preferably benzoimidazolyl, imidazopyrimidinyl, pyrazolopyrazinyl, pyrazolopyrimidinyl, benzothiophenyl, benzothiazolyl, benzoisoxazolyl, benzooxazolyl, benzoisothiazolyl, imidazopyridazinyl, imidazopyridazinyl; dihydro-4H-furo[3,2-c]pyranyl, 6,7-dihydro-4H-thieno[3,2-c]pyranyl, 2,3-dihydropyrazolo[5,1-b]oxazolyl, 4,5,6,7-tetrahydrofuro[3,2-c]pyridinyl, 1,3a,4,6,7,7a-hexahydropyrano[4,3-c]pyrazolyl, 4,5,6,7-tetrahydropyrazolo[1,5-a]pyrazinyl, 4,5,6,7-tetrahydropyrazolo[1,5-a]pyrimidinyl, 4,5,6,7-tetrahydropyrazolo[1,5-a]pyrazinyl, 4,5,6,7-tetrahydrothiazolo[5,4-c]pyridinyl, 4,5,6,7-tetrahydropyrazolo[1,5-a]pyrazinyl, 6,7-dihydro-4H-pyrazolo[5,1-c][1,4]oxazinyl, 2,3-dihydropyrazolo[5,1-b]oxazolyl, 2,3-dihydropyrazolo[5,1-b]oxazolyl, 1,3a,4,6,7,7a-hexahydropyrano[4,3-c]pyrazolyl, 6,7-dihydro-4H-pyrano[4,3-d]thiazolyl, [1,3]dioxolo[4,5-c]pyridinyl, 2,3-dihydro-[1,4]dioxino[2,3-c]pyridinyl, 3,4-dihydro-2H-pyrido[3,2-b][1,4]oxazinyl, 2,3-dihydro-[1,4]dioxino[2,3-b]pyridinyl, 2,3-dihydro-[1,4]dioxino[2,3-b]pyridin-6-yl, 3,4-dihydro-2H-pyrido[4,3-b][1,4]oxazinyl, or 2H-pyrido[3,2-b][1,4]oxazin-4(3H)-yl, each of which is optionally substituted with halogen, -C₁₋₆alkyl, - NH₂, or -C(O)R^{m}, wherein R^{m} is C₁₋₆alkyl; any of the said alkyl is optionally enriched in deuterium.

**In** some embodiments, Cy¹ is a 7- to 14-membered bicyclic heteroaryl which is a pyridinyl, pyrazolyl, thienyl, or thiazolyl ring fused with a 5- or 6-membered heterocyclyl ring, wherein said 5- or 6-membered heterocyclyl ring comprising one or two heteroatoms selected from oxygen or nitrogen as ring member(s) and said 5- or 6-membered heterocyclyl ring is optionally substituted with one or two C₁₋₆alkyl or oxo, preferably two C₁₋₆alkyl, more preferably two methyl, most preferably two methyl on the same carbon atom. In some embodiments, Cy¹ is a 7- to 14-membered bicyclic heteroaryl which is a pyridinyl ring fused with a 5- or 6-membered heterocyclyl ring, wherein said 5- or 6-membered heterocyclyl ring comprising one or two heteroatoms selected from oxygen or nitrogen as ring member(s) and said 5- or 6-membered heterocyclyl ring is optionally substituted with one or two C₁₋₆alkyl or oxo, preferably two C₁₋₆alkyl, more preferably two methyl, most preferably two methyl on the same carbon atom. In some embodiments, Cy¹ is a 7- to 14-membered bicyclic heteroaryl which is a pyridinyl ring fused with a 5- or 6-membered heterocyclyl ring, wherein said 5- or 6-membered heterocyclyl ring comprising two oxygen atoms as ring member(s) and said 5- or 6-membered heterocyclyl ring is optionally substituted with one or two C₁₋₆alkyl, preferably two C₁₋₆alkyl, more preferably two methyl, most preferably two methyl on the same carbon atom. In some embodiments, Cy¹ is a 7- to 14-membered bicyclic heteroaryl which is a pyridinyl ring fused with 1,4-dioxane ring, wherein said 1,4-dioxane ring is optionally substituted with one or two C₁₋₆alkyl, preferably two C₁₋₆alkyl, more preferably two methyl, most preferably two methyl on the same carbon atom. In some preferred embodiments, Cy¹ is 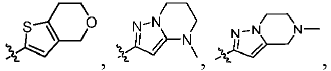 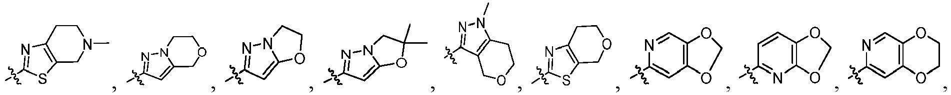 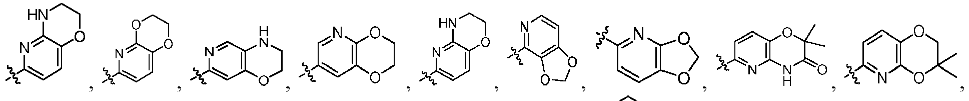 or 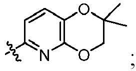 preferably

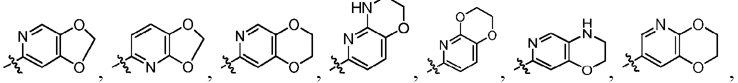

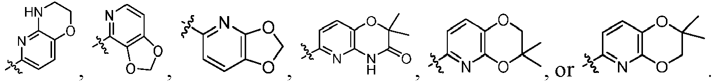

In some embodiments, Cy¹ is

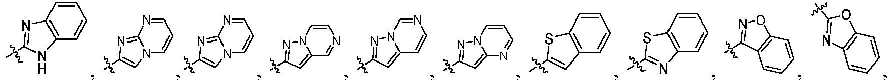

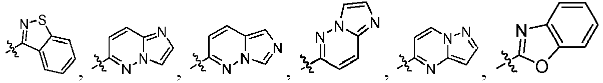

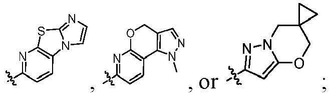

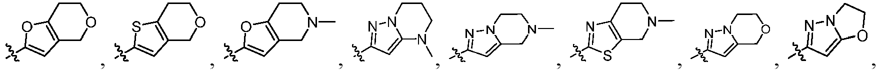

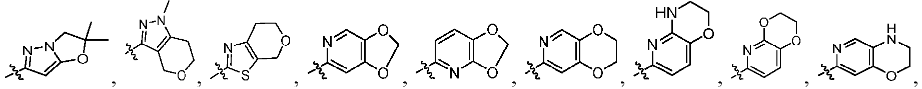

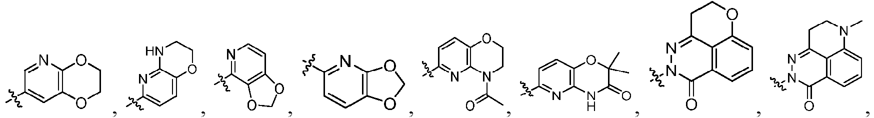

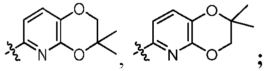

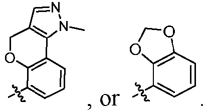

In some embodiments, the compound is selected from the exemplified compounds in Examples.

In the second aspect, disclosed herein provides a pharmaceutical composition comprising one or more compounds in the present disclosure or a stereoisomer or pharmaceutically acceptable salt thereof, and a pharmaceutically acceptable excipient.

In the third aspect, disclosed herein provides a compound of the invention for use in a method for treating a disease associated with undesirable TYK2 activity (TYK2-related diseases), comprising administrating to a subject in need of such treatment a therapeutically effective amount of the compound or a stereoisomer or pharmaceutically acceptable salt thereof.

In one embodiment, the disease is inflammatory or autoimmune.

### DETAILED DESCRIPTION OF THE DISCLOSURE

### Definitions

The following terms have the indicated meanings throughout the specification:

As used herein, including the appended claims, the singular forms of words such as "a," "an," and "the," include their corresponding plural references unless the context clearly dictates otherwise.

The term **"or"** is used to mean, and is used interchangeably with, the term **"and/or"** unless the context clearly dictates otherwise.

The term **"alkyl"** refers to a hydrocarbon group selected from linear and branched saturated hydrocarbon groups comprising from 1 to 18, such as from 1 to 12, further such as from 1 to 10, more further such as from 1 to 8, or from 1 to 6, or from 1 to 4, carbon atoms. Examples of alkyl groups comprising from 1 to 6 carbon atoms (i.e., C₁₋₆ alkyl) include, but not limited to, methyl, ethyl, 1-propyl or n-propyl ("n-Pr"), 2-propyl or isopropyl ("i-Pr"), 1-butyl or n-butyl ("n-Bu"), 2-methyl-1-propyl or isobutyl ("i-Bu"), 1-methylpropyl or s-butyl ("s-Bu"), 1,1-dimethylethyl or t-butyl ("t-Bu"), 1-pentyl, 2-pentyl, 3-pentyl, 2-methyl-2-butyl, 3-methyl-2-butyl, 3-methyl-1-butyl, 2-methyl-1-butyl, 1-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl and 3,3-dimethyl-2-butyl groups. The alkyl group can be optionally enriched in deuterium, e.g., -CD₃, -CD₂CD₃ and the like.

The term **"halogen"** refers to fluoro (F), chloro (Cl), bromo (Br) and iodo (I).

The term **"haloalkyl"** refers to an alkyl group in which one or more hydrogen is/are replaced by one or more halogen atoms such as fluoro, chloro, bromo, and iodo. Examples of the haloalkyl include haloC₁₋₈alkyl, haloC₁₋₆alkyl or halo C₁₋₄alkyl, but not limited to -CF₃, -CH₂Cl, -CH₂CF₃, -CCl₂, CF₃, and the like.

The term **"alkyloxy"** or **"alkoxy"** refers to an alkyl group as defined above attached to the parent molecular moiety through an oxygen atom. Examples of an alkyloxy, e.g., C₁₋₆alkyloxy or C₁₋₄ alkyloxy include, but not limited to, methoxy, ethoxy, isopropoxy, propoxy, n-butoxy, tert-butoxy, pentoxy and hexoxy and the like.

The term **"alkoxy-alkyl-"** refers to an alkyl group as defined above further substituted with an alkoxy as defined above. Examples of an alkoxy-alkyl-, e.g., C₁-₈alkoxy-C₁-₈alkyl- or C₁-₆alkoxy-C₁₋₆alkyl-include, but not limited to, methoxymethyl, ethoxymethyl, ethoxyethyl, isopropoxymethyl, or propoxymethyl and the like.

The term **"amino"** refers to -NH₂. The term **"alkylamino"** refers to -NH(alkyl). The term **"dialkylamino"** refers to -N(alkyl)₂.

The term **"alkenyl"** herein refers to a hydrocarbon group selected from linear and branched hydrocarbon groups comprising at least one C = C double bond and from 2 to 18, such as from 2 to 8, further such as from 2 to 6, carbon atoms. Examples of the alkenyl group, e.g., C2-6 alkenyl, include, but not limited to ethenyl or vinyl, prop-1-enyl, prop-2-enyl, 2-methylprop-1-enyl, but-1-enyl, but-2-enyl, but-3-enyl, buta-1,3-dienyl, 2-methylbuta-1,3-dienyl, hex-1-enyl, hex-2-enyl, hex-3-enyl, hex-4-enyl, and hexa-1,3-dienyl groups.

The term **"alkynyl"** herein refers to a hydrocarbon group selected from linear and branched hydrocarbon group, comprising at least one C≡C triple bond and from 2 to 18, such as 2 to 8, further such as from 2 to 6, carbon atoms. Examples of the alkynyl group, e.g., C2-6 alkynyl, include, but not limited to ethynyl, 1-propynyl, 2-propynyl (propargyl), 1-butynyl, 2-butynyl, and 3-butynyl groups.

The term **"cycloalkyl"** refers to a hydrocarbon group selected from saturated cyclic hydrocarbon groups, comprising monocyclic and polycyclic (e.g., bicyclic and tricyclic) groups including fused, bridged or spiro cycloalkyl.

For example, the cycloalkyl group may comprise from 3 to 12, such as from 3 to 10, further such as 3 to 8, further such as 3 to 6, 3 to 5, or 3 to 4 carbon atoms. Even further for example, the cycloalkyl group may be selected from monocyclic group comprising from 3 to 12, such as from 3 to 10, further such as 3 to 8, 3 to 6 carbon atoms. Examples of the monocyclic cycloalkyl group include cyclopropyl, cyclobutyl, cyclopentyl, 1-cyclopent-1-enyl, 1-cyclopent-2-enyl, 1-cyclopent-3-enyl, cyclohexyl, 1-cyclohex-1-enyl, 1-cyclohex-2-enyl, 1-cyclohex-3-enyl, cyclohexadienyl, cycloheptyl, cyclooctyl, cyclononyl, cyclodecyl, cycloundecyl, and cyclododecyl groups. In particular, Examples of the saturated monocyclic cycloalkyl group, e.g., C₃₋₈cycloalkyl, include, but not limited to cyclopropyl, cyclobutyl, cyclopentyl, cyclohexyl, cycloheptyl, and cyclooctyl groups. In a preferred embedment, the cycloalkyl is a monocyclic ring comprising 3 to 6 carbon atoms (abbreviated as C₃₋₆ cycloalkyl), including but not limited to, cyclopropyl, cyclobutyl, cyclopentyl, and cyclohexyl. Examples of the bicyclic cycloalkyl groups include those having from 7 to 12 ring atoms arranged as a fused bicyclic ring selected from [4,4], [4,5], [5,5], [5,6] and [6,6] ring systems, or as a bridged bicyclic ring selected from bicyclo[2.2.1]heptane, bicyclo[2.2.2]octane, and bicyclo[3.2.2]nonane. Further Examples of the bicyclic cycloalkyl groups include those arranged as a bicyclic ring selected from [5,6] and [6,6] ring systems.

The term "cycloalkenyl" refers to non-aromatic cyclic alkyl groups of from 3 to 10 carbon atoms having single or multiple rings and having at least one double bond and preferably from 1 to 2 double bonds. In one embodiment, the cycloalkenyl is cyclopentenyl or cyclohexenyl, preferably cyclohexenyl.

The term **"cycloalkynyl"** refers to non-aromatic cycloalkyl groups of from 5 to 10 carbon atoms having single or multiple rings and having at least one triple bond.

The term **"deuterated"** is used herein to modify a chemical structure or an organic group or radical, wherein one or more carbon-bound hydrogen(s) are replaced by one or more deuterium(s), e.g., "deuterated-alkyl", "deuterated-cycloalkyl", "deuterated-heterocycloalkyl", "deuterated-aryl", "deuterated-morpholinyl", and the like. For example, the term "deuterated-alkyl" defined above refers to an alkyl group as defined herein, wherein at least one hydrogen atom bound to carbon is replaced by a deuterium. In a deuterated alkyl group, at least one carbon atom is bound to a deuterium; and it is possible for a carbon atom to be bound to more than one deuterium; it is also possible that more than one carbon atom in the alkyl group is bound to a deuterium.

The term **"aryl"** used alone or in combination with other terms refers to a group selected from:
- 5- and 6-membered carbocyclic aromatic rings, e.g., phenyl;
- bicyclic ring systems such as 7 to 12 membered bicyclic ring systems, wherein at least one ring is carbocyclic and aromatic, e.g., naphthyl and indanyl; and,
- tricyclic ring systems such as 10 to 15 membered tricyclic ring systems wherein at least one ring is carbocyclic and aromatic, e.g., fluorenyl.

The terms **"aromatic hydrocarbon ring" and "aryl"** are used interchangeably throughout the disclosure herein. In some embodiments, a monocyclic or bicyclic aromatic hydrocarbon ring has 5 to 10 ring-forming carbon atoms (i.e., C5-10 aryl). Examples of a monocyclic or bicyclic aromatic hydrocarbon ring include, but not limited to, phenyl, naphth-1-yl, naphth-2-yl, anthracenyl, phenanthrenyl, and the like. In some embodiments, the aromatic hydrocarbon ring is a naphthalene ring (naphth-1-yl or naphth-2-yl) or phenyl ring. In some embodiments, the aromatic hydrocarbon ring is a phenyl ring.

The term **"heteroaryl"** herein refers to a group selected from:
- 5-, 6- or 7-membered aromatic, monocyclic rings comprising at least one heteroatom, for example, from 1 to 4, or, in some embodiments, from 1 to 3, in some embodiments, from 1 to 2, heteroatoms, selected from nitrogen (N), sulfur (S) and oxygen (O), with the remaining ring atoms being carbon;
- 7- to 12-membered bicyclic rings comprising at least one heteroatom, for example, from 1 to 4, or, in some embodiments, from 1 to 3, or, in other embodiments, 1 or 2, heteroatoms, selected from nitrogen, oxygen or optionally oxidized sulfur as ring member(s), with the remaining ring atoms being carbon and wherein at least one ring is aromatic and at least one heteroatom is present in the aromatic ring; and
- 11- to 14-membered tricyclic rings comprising at least one heteroatom, for example, from 1 to 4, or in some embodiments, from 1 to 3, or, in other embodiments, 1 or 2, heteroatoms, selected from nitrogen, oxygen or optionally oxidized sulfur as ring member(s), with the remaining ring atoms being carbon and wherein at least one ring is aromatic and at least one heteroatom is present in an aromatic ring.

When the total number of S and O atoms in the heteroaryl group exceeds 1, those heteroatoms are not adjacent to one another. In some embodiments, the total number of S and O atoms in the heteroaryl group is not more than 2. In some embodiments, the total number of S and O atoms in the aromatic heterocycle is not more than 1. When the heteroaryl group contains more than one heteroatom ring member, the heteroatoms may be the same or different. The nitrogen atoms in the ring(s) of the heteroaryl group can be oxidized to form N-oxides.

The term **"optionally oxidized sulfur"** used herein refers to S, SO or SO2.

The terms **"aromatic heterocyclic ring"** and **"heteroaryl"** are used interchangeably throughout the disclosure herein. In some embodiments, a monocyclic or bicyclic aromatic heterocyclic ring has 5-, 6-, 7-, 8-, 9- or 10-ring forming members with 1, 2, 3, or 4 heteroatom ring members independently selected from nitrogen (N), sulfur (S) and oxygen (O) and the remaining ring members being carbon. In some embodiments, the monocyclic or bicyclic aromatic heterocyclic ring is a monocyclic or bicyclic ring comprising 1 or 2 heteroatom ring members independently selected from nitrogen (N), sulfur (S) and oxygen (O). In some embodiments, the monocyclic or bicyclic aromatic heterocyclic ring is a 5- to 6-membered heteroaryl ring, which is monocyclic and which has 1 or 2 heteroatom ring members independently selected from nitrogen (N), sulfur (S) and oxygen (O). In some embodiments, the monocyclic or bicyclic aromatic heterocyclic ring is an 8- to 10-membered heteroaryl ring, which is bicyclic and which has 1 or 2 heteroatom ring members independently selected from nitrogen, sulfur and oxygen.

Examples of the heteroaryl group or the monocyclic or bicyclic aromatic heterocyclic ring include, but are not limited to, (as numbered from the linkage position assigned priority 1) pyridyl (such as 2-pyridyl, 3-pyridyl, or 4-pyridyl), cinnolinyl, pyrazinyl, 2,4-pyrimidinyl, 3,5-pyrimidinyl, 2,4-imidazolyl, imidazopyridinyl, isoxazolyl, oxazolyl, thiazolyl, isothiazolyl, thiadiazolyl (such as 1,2,3-thiadiazolyl, 1,2,4-thiadiazolyl, or 1,3,4-thiadiazolyl), tetrazolyl, thienyl (such as thien-2-yl, thien-3-yl), triazinyl, benzothienyl, furyl or furanyl, benzofuryl, benzoimidazolyl, indolyl, isoindolyl, indolinyl, oxadiazolyl (such as 1,2,3-oxadiazolyl, 1,2,4-oxadiazolyl, or 1,3,4-oxadiazolyl), phthalazinyl, pyrazinyl, pyridazinyl, pyrrolyl, triazolyl (such as 1,2,3-triazolyl, 1,2,4-triazolyl, or 1,3,4-triazolyl), quinolinyl, isoquinolinyl, pyrazolyl, pyrrolopyridinyl (such as 1H-pyrrolo[2,3-b]pyridin-5-yl), pyrazolopyridinyl (such as 1H-pyrazolo[3,4-b]pyridin-5-yl), benzoxazolyl (such as benzo[d]oxazol-6-yl), pteridinyl, purinyl, 1-oxa-2,3-diazolyl, 1-oxa-2,4-diazolyl, 1-oxa-2,5-diazolyl, 1-oxa-3,4-diazolyl, 1-thia-2,3-diazolyl, 1-thia-2,4-diazolyl, 1-thia-2,5-diazolyl, 1-thia-3,4-diazolyl, furazanyl (such as furazan-2-yl, furazan-3-yl), benzofurazanyl, benzothiophenyl, benzothiazolyl, benzoxazolyl, quinazolinyl, quinoxalinyl, naphthyridinyl, furopyridinyl, benzothiazolyl (such as benzo[d]thiazol-6-yl), indazolyl (such as 1H-indazol-5-yl) and 5,6,7,8-tetrahydroisoquinoline.

Also, a **"heteroaryl"** which is further fused with a **"Heterocyclyl"** is defined as a **"heteroaryl".**

**"Heterocyclyl," "heterocycle"** or **"heterocyclic"** are interchangeable and refer to a non-aromatic heterocyclyl group comprising one or more heteroatoms selected from nitrogen, oxygen or optionally oxidized sulfur as ring members, with the remaining ring members being carbon, including monocyclic, fused, bridged, and spiro ring, i.e., containing monocyclic heterocyclyl, bridged heterocyclyl, spiro heterocyclyl, and fused heterocyclic groups.

The term **"monocyclic heterocyclyl"** refers to monocyclic groups in which at least one ring member is a heteroatom selected from nitrogen, oxygen or optionally oxidized sulfur. A heterocycle may be saturated or partially saturated.

Exemplary monocyclic 4 to 9-membered heterocyclyl groups include, but not limited to, (as numbered from the linkage position assigned priority 1) pyrrolidin-1-yl, pyrrolidin-2-yl, pyrrolidin-3-yl, imidazolidin-2-yl, imidazolidin-4-yl , pyrazolidin-2-yl, pyrazolidin-3-yl, piperidin-1-yl, piperidin-2-yl, piperidin-3-yl, piperidin-4-yl, 2,5-piperazinyl, pyranyl, morpholinyl, morpholino, morpholin-2-yl, morpholin-3-yl, oxiranyl, aziridin-1-yl, aziridin-2-yl, azocan-1-yl, azocan-2-yl, azocan-3-yl, azocan-4-yl, azocan-5-yl, thiiranyl, azetidin-1-yl, azetidin-2-yl, azetidin-3-yl, oxetanyl, thietanyl, 1,2-dithietanyl, 1,3-dithietanyl, dihydropyridinyl, tetrahydropyridinyl, thiomorpholinyl, thioxanyl, piperazinyl, homopiperazinyl, homopiperidinyl, azepan-1-yl, azepan-2-yl, azepan-3-yl, azepan-4-yl, oxepanyl, thiepanyl, 1,4-oxathianyl, 1,4-dioxepanyl, 1,4-oxathiepanyl, 1,4-oxaazepanyl, 1,4-dithiepanyl, 1,4-thiazepanyl and 1,4-diazepanyl, 1,4-dithianyl, 1,4-azathianyl, oxazepinyl, diazepinyl, thiazepinyl, dihydrothienyl, dihydropyranyl, dihydrofuranyl, tetrahydrofuranyl, tetrahydrothienyl, tetrahydropyranyl, tetrahydrothiopyranyl, 1-pyrrolinyl, 2-pyrrolinyl, 3-pyrrolinyl, indolinyl, 2H-pyranyl, 4H-pyranyl, 1,4-dioxanyl, 1,3-dioxolanyl, pyrazolinyl, pyrazolidinyl, dithianyl, dithiolanyl, pyrazolidinyl, imidazolinyl, pyrimidinonyl, or 1,1-dioxo-thiomorpholinyl.

The term **"spiro heterocyclyl"** refers to a 5 to 20-membered polycyclic heterocyclyl with rings connected through one common carbon atom (called a spiro atom), comprising one or more heteroatoms selected from nitrogen, oxygen or optionally oxidized sulfur as ring members, with the remaining ring members being carbon. One or more rings of a spiro heterocyclyl group may contain one or more double bonds, but none of the rings has a completely conjugated pi-electron system. Preferably a spiro heterocyclyl is 6 to 14-membered, and more preferably 7 to 12-membered. According to the number of common spiro atoms, a spiro heterocyclyl is divided into mono-spiro heterocyclyl, di-spiro heterocyclyl, or poly-spiro heterocyclyl, and preferably refers to mono-spiro heterocyclyl or di-spiro heterocyclyl, and more preferably 4-membered/4-membered, 3-membered/5-membered, 4-membered/5-membered, 4-membered/6-membered, 5-membered/5-membered, or 5-membered/6-membered mono-spiro heterocyclyl.

The term **"fused heterocyclic group"** refers to a 5 to 20-membered polycyclic heterocyclyl group, wherein each ring in the system shares an adjacent pair of atoms (carbon and carbon atoms or carbon and nitrogen atoms) with another ring, comprising one or more heteroatoms selected from nitrogen, oxygen or optionally oxidized sulfur as ring members, with the remaining ring members being carbon. One or more rings of a fused heterocyclic group may contain one or more double bonds, but none of the rings has a completely conjugated pi-electron system. Preferably, a fused heterocyclyl is 6 to 14-membered, and more preferably 7 to 10-membered. According to the number of membered rings, a fused heterocyclyl is divided into bicyclic, tricyclic, tetracyclic, or polycyclic fused heterocyclyl, preferably refers to bicyclic or tricyclic fused heterocyclyl, and more preferably 5-membered/5-membered, or 5-membered/6-membered bicyclic fused heterocyclyl. Representative examples of fused heterocycles include, but not limited to, the following groups octahydrocyclopenta[c]pyrrole (e.g., octahydrocyclopenta[c]pyrrol-2-yl), octahydropyrrolo[3,4-c]pyrrolyl, octahydroisoindolyl, isoindolinyl (e.g., isoindoline-2-yl), octahydro-benzo[b][1,4]dioxin.

The term **"bridged heterocyclyl"** refers to a 5- to 14-membered polycyclic heterocyclic alkyl group, wherein every two rings in the system share two disconnected atoms, comprising one or more heteroatoms selected from nitrogen, oxygen or optionally oxidized sulfur as ring members, with the remaining ring members being carbon. One or more rings of a bridged heterocyclyl group may contain one or more double bonds, but none of the rings has a completely conjugated pi-electron system. Preferably, a bridged heterocyclyl is 6 to 14-membered, and more preferably 7 to 10-membered. According to the number of membered rings, a bridged heterocyclyl is divided into bicyclic, tricyclic, tetracyclic or polycyclic bridged heterocyclyl, and preferably refers to bicyclic, tricyclic or tetracyclic bridged heterocyclyl, and more preferably bicyclic or tricyclic bridged heterocyclyl. Representative examples of bridged heterocyclyls include, but not limited to, the following groups: 2-azabicyclo[2.2.1]heptyl, azabicyclo[3.1.0]hexyl, 2-azabicyclo[2.2.2]octyl and 2-azabicyclo[3.3.2]decyl.

Compounds disclosed herein may contain an asymmetric center and may thus exist as enantiomers. **"Enantiomers"** refer to two stereoisomers of a compound which are non-superimposable mirror images of one another. Where the compounds disclosed herein possess two or more asymmetric centers, they may additionally exist as diastereomers. Enantiomers and diastereomers fall within the broader class of stereoisomers. All such possible stereoisomers as substantially pure resolved enantiomers, racemic mixtures thereof, as well as mixtures of diastereomers are intended to be included. All stereoisomers of the compounds disclosed herein and /or pharmaceutically acceptable salts thereof are intended to be included. Unless specifically mentioned otherwise, reference to one isomer applies to any of the possible isomers. Whenever the isomeric composition is unspecified, all possible isomers are included.

The term **"substantially pure"** as used herein means that the target stereoisomer contains no more than 35%, such as no more than 30%, further such as no more than 25%, even further such as no more than 20%, by weight of any other stereoisomer(s). In some embodiments, the term "substantially pure" means that the target stereoisomer contains no more than 10%, for example, no more than 5%, such as no more than 1%, by weight of any other stereoisomer(s).

When compounds disclosed herein contain olefinic double bonds, unless specified otherwise, such double bonds are meant to include both E and Z geometric isomers.

When compounds disclosed herein contain a di-substituted cyclohexyl or cyclobutyl group, substituents found on cyclohexyl or cyclobutyl ring may adopt cis and trans formations. Cis formation means that both substituents are found on the upper side of the 2 substituent placements on the carbon, while trans would mean that they were on opposing sides.

It may be advantageous to separate reaction products from one another and /or from starting materials. The desired products of each step or series of steps is separated and /or purified (hereinafter separated) to the desired degree of homogeneity by the techniques common in the art. Typically such separations involve multiphase extraction, crystallization from a solvent or solvent mixture, distillation, sublimation, or chromatography. Chromatography can involve any number of methods including, for example: reverse-phase and normal phase; size exclusion; ion exchange; high, medium and low pressure liquid chromatography methods and apparatus; small scale analytical; simulated moving bed ("SMB") and preparative thin or thick layer chromatography, as well as techniques of small scale thin layer and flash chromatography. One skilled in the art will apply techniques most likely to achieve the desired separation.

"Diastereomers" refers to stereoisomers of a compound with two or more chiral centers but which are not mirror images of one another. Diastereomeric mixtures can be separated into their individual diastereomers on the basis of their physical chemical differences by methods well known to those skilled in the art, such as by chromatography and /or fractional crystallization. Enantiomers can be separated by converting the enantiomeric mixture into a diastereomeric mixture by reaction with an appropriate optically active compound (e.g., chiral auxiliary such as a chiral alcohol or Mosher's acid chloride), separating the diastereomers and converting (e.g., hydrolyzing) the individual diastereoisomers to the corresponding pure enantiomers. Enantiomers can also be separated by use of a chiral HPLC column.

A single stereoisomer, e.g., a substantially pure enantiomer, may be obtained by resolution of the racemic mixture using a method such as formation of diastereomers using optically active resolving agents *[*Eliel, E. and Wilen, S. Stereochemistry of Organic Compounds. New York: John Wiley & Sons, Inc., 1994*;* Lochmuller, C. H., et al. "Chromatographic resolution of enantiomers: Selective review. "J. Chromatogr, 113(3) (1975): pp. 283-302*].* Racemic mixtures of chiral compounds of the invention can be separated and isolated by any suitable method, including: (1) formation of ionic, diastereomeric salts with chiral compounds and separation by fractional crystallization or other methods, (2) formation of diastereomeric compounds with chiral derivatizing reagents, separation of the diastereomers, and conversion to the pure stereoisomers, and (3) separation of the substantially pure or enriched stereoisomers directly under chiral conditions. See: Wainer, Irving W, Ed. Drug Stereochemistry: Analytical Methods and Pharmacology. New York: Marcel Dekker, Inc., 1993*.*

**"Pharmaceutically acceptable salts"** refers to those salts which are, within the scope of sound medical judgment, suitable for use in contact with the tissues of humans and lower animals without undue toxicity, irritation, allergic response and the like, and are commensurate with a reasonable benefit/risk ratio. A pharmaceutically acceptable salt may be prepared in situ during the final isolation and purification of the compounds disclosed herein, or separately by reacting the free base function with a suitable organic acid or by reacting the acidic group with a suitable base.

In addition, if a compound disclosed herein is obtained as an acid addition salt, the free base can be obtained by basifying a solution of the acid salt. Conversely, if the product is a free base, an addition salt, such as a pharmaceutically acceptable addition salt, may be produced by dissolving the free base in a suitable organic solvent and treating the solution with an acid, in accordance with conventional procedures for preparing acid addition salts from base compounds. Those skilled in the art will recognize various synthetic methodologies that may be used without undue experimentation to prepare non-toxic pharmaceutically acceptable addition salts.

As defined herein, **"a pharmaceutically acceptable salt thereof" include** salts of at least one compound of Formula (I), and salts of the stereoisomers of the compound of Formula (I), such as salts of enantiomers, and /or salts of diastereomers.

The terms **"administration", "administering", "treating"** and **"treatment"** herein, when applied to an animal, human, experimental subject, cell, tissue, organ, or biological fluid, mean contact of an exogenous pharmaceutical, therapeutic, diagnostic agent, or composition to the animal, human, subject, cell, tissue, organ, or biological fluid. Treatment of a cell encompasses contact of a reagent to the cell, as well as contact of a reagent to a fluid, where the fluid is in contact with the cell. The term "administration" and "treatment" also means in vitro and ex vivo treatments, e.g., of a cell, by a reagent, diagnostic, binding compound, or by another cell. The term "subject" herein includes any organism, preferably an animal, more preferably a mammal (e.g., rat, mouse, dog, cat, rabbit) and most preferably a human.

The term **"effective amount"** or **"therapeutically effective amount"** refers to an amount of the active ingredient, such as compound that, when administered to a subject for treating a disease, or at least one of the clinical symptoms of a disease or disorder, is sufficient to affect such treatment for the disease, disorder, or symptom. The "therapeutically effective amount" can vary with the compound, the disease, disorder, and/or symptoms of the disease or disorder, severity of the disease, disorder, and/or symptoms of the disease or disorder, the age of the subject to be treated, and/or the weight of the subject to be treated. An appropriate amount in any given instance can be apparent to those skilled in the art or can be determined by routine experiments. In some embodiments, "therapeutically effective amount" is an amount of at least one compound and /or at least one stereoisomer thereof, and /or at least one pharmaceutically acceptable salt thereof disclosed herein effective to "treat" as defined above, a disease or disorder in a subject. In the case of combination therapy, the "therapeutically effective amount" refers to the total amount of the combination objects for the effective treatment of a disease, a disorder or a condition.

The pharmaceutical composition comprising the compound disclosed herein can be administrated via oral, inhalation, rectal, parenteral or topical administration to a subject in need thereof. For oral administration, the pharmaceutical composition may be a regular solid Formulation such as tablets, powder, granule, capsules and the like, a liquid Formulation such as water or oil suspension or other liquid Formulation such as syrup, solution, suspension or the like; for parenteral administration, the pharmaceutical composition may be solution, water solution, oil suspension concentrate, lyophilized powder or the like. Preferably, the Formulation of the pharmaceutical composition is selected from tablet, coated tablet, capsule, suppository, nasal spray or injection, more preferably tablet or capsule. The pharmaceutical composition can be a single unit administration with an accurate dosage. In addition, the pharmaceutical composition may further comprise additional active ingredients.

All Formulations of the pharmaceutical composition disclosed herein can be produced by the conventional methods in the pharmaceutical field. For example, the active ingredient can be mixed with one or more excipients, then to make the desired Formulation. The "pharmaceutically acceptable excipient" refers to conventional pharmaceutical carriers suitable for the desired pharmaceutical Formulation, for example: a diluent, a vehicle such as water, various organic solvents, etc., a filler such as starch, sucrose, etc. a binder such as cellulose derivatives, alginates, gelatin and polyvinylpyrrolidone (PVP); a wetting agent such as glycerol; a disintegrating agent such as agar, calcium carbonate and sodium bicarbonate; an absorption enhancer such as quaternary ammonium compound; a surfactant such as hexadecanol; an absorption carrier such as Kaolin and soap clay; a lubricant such as talc, calcium stearate, magnesium stearate, polyethylene glycol, etc. In addition, the pharmaceutical composition further comprises other pharmaceutically acceptable excipients such as a decentralized agent, a stabilizer, a thickener, a complexing agent, a buffering agent, a permeation enhancer, a polymer, aromatics, a sweetener, and a dye.

The term **"disease"** refers to any disease, discomfort, illness, symptoms or indications, and can be interchangeable with the term "disorder" or "condition".

Throughout this specification and the claims which follow, unless the context requires otherwise, the term **"comprise,"** and variations such as **"comprises"** and **"comprising"** are intended to specify the presence of the features thereafter, but do not exclude the presence or addition of one or more other features. When used herein the term "comprising" can be substituted with the term **"containing", "including"** or sometimes "having".

Throughout this specification and the claims which follow, the term "Cn-m" indicates a range which includes the endpoints, wherein n and m are integers and indicate the number of carbons. Examples include C1-8, C1-6, and the like.

Unless specifically defined elsewhere in this document, all other technical and scientific terms used herein have the meaning commonly understood by one of ordinary skill in the art to which this invention belongs.

### EXAMPLE

A compound according to the present invention has the features as defined by the claims. Any compound described in this section that does not comprise these features is disclosed for reference only. The examples below are intended to be purely exemplary and should not be considered to be limiting in any way. Efforts have been made to ensure accuracy with respect to numbers used (for example, amounts, temperature, etc.), but some experimental errors and deviations should be accounted for. Unless indicated otherwise, temperature is in degrees Centigrade. Reagents were purchased from commercial suppliers such as Sigma-Aldrich, Alfa Aesar, or TCI, and were used without further purification unless indicated otherwise.

Unless indicated otherwise, the reactions set forth below were performed under a positive pressure of nitrogen or argon or with a drying tube in anhydrous solvents; the reaction flasks were fitted with rubber septa for the introduction of substrates and reagents via syringe; and glassware was oven dried and/or heat dried.

Unless otherwise indicated, the reactions set forth below were performed under a positive pressure of nitrogen or argon or with a drying tube in anhydrous solvents; the reaction flasks were fitted with rubber septa for the introduction of substrates and reagents via syringe; and glassware was oven dried and /or heat dried.

Unless otherwise indicated, column chromatography purification was conducted on a Biotage system (Manufacturer: Dyax Corporation) having a silica gel column or on a silica SepPak cartridge (Waters), or was conducted on a Teledyne Isco Combiflash purification system using prepacked silica gel cartridges.

¹H NMR spectra were recorded on a Varian instrument operating at 400 MHz. ¹H-NMR spectra were obtained using CDCl₃, CD₂Cl₂, CD₃OD, D₂O, *d*₆-DMSO, *d*₆-acetone or (CD₃)₂CO as solvent and tetramethylsilane (0.00 ppm) or residual solvent (CDCl₃: 7.25 ppm; CD₃OD: 3.31 ppm; D₂O: 4.79 ppm; *d*₆-DMSO: 2.50 ppm; *d*₆-acetone: 2.05; (CD₃)₂CO: 2.05) as the reference standard. When peak multiplicities are reported, the following abbreviations are used: s (singlet), d (doublet), t (triplet), q (quartet), qn (quintuplet), sx (sextuplet), m (multiplet), br (broadened), dd (doublet of doublets), dt (doublet of triplets). Coupling constants, when given, are reported in Hertz (Hz). Compound names except the reagents were generated by ChemDraw version 12.0.

### Abbreviations:

- AcOH: Acetic acid
- Aq: Aqueous
- Brine: Saturated aqueous sodium chloride solution
- Bn: Benzyl
- BnBr: Benzyl Bromide
- (Boc)₂O: di-tert-butyl dicarbonate
- DMF: N,N-Dimethylformamide
- Dppf: 1,1"-bis(diphenylphosphino)ferrocene
- DBU: 1,8-diazabicyclo[5.4.0]undec-7-ene
- DIEA or DIPEA: N-ethyl-N-isopropylpropan-2-amine
- DMAP: 4-N,N-dimethylaminopyridine
- DMSO: Dimethyl sulfoxide
- EtOAc or EA: EA
- EtOH: Ethanol
- Et₂O or ether: Diethyl ether
- Et₃N: Triethyl amine
- HATU: O-(7-Azabenzotriazol-1-yl)-N,N,N',N'-tetramethyluronium hexafluorophosphate
- HPLC: High-performance liquid chromatography
- IPA: 2-propanol
- i-PrOH: Isopropyl alcohol
- ms or MS: Mass spectrum
- NaHMDS: Sodium Hexamethylenedisilazane
- PE: petroleum ether
- PPA: Polyphosphoric acid
- p-TSA: p-Tolunesulfonic acid
- Rt: Retention time
- Rt or rt: Room temperature
- TBAF: Tetra-butyl ammonium fluoride
- TBSCl: tert-Butyldimethylsilyl chloride
- TFA: Trifluoroacetic acid
- THF: tetrahydrofuran
- TLC: thin layer chromatography

**Example BB1: Synthesis of 2-bromo-4-(methoxymethyl)-6-(methylsulfonyl)pyridine**

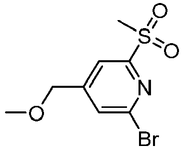

### Step 1: 2,6-dibromo-4-(bromomethyl)pyridine

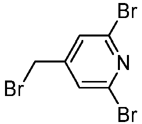

A mixture of 2,6-dibromo-4-methylpyridine (10.0 g, 39.85 mmol), CCl₄ (100 mL), AIBN (1.31 g, 7.98 mmol) and NBS (10.64 g, 59.78 mmol) was stirred for 15 h at 80 °C. After cooled to room temperature, DCM (100 mL) was added and the resulting mixture was washed with H₂O (150 mL x 3). The mixture was dried over anhydrous Na₂SO₄ and concentrated under vacuum. The residue was purified by combi-flash (EA/PE = 0-20%) give 2,6-dibromo-4-(bromomethyl)pyridine (10.0 g, 76 %). LCMS (ESI) m/e [M+1]⁺ 328.

### Step 2: 2,6-dibromo-4-(methoxymethyl)pyridine

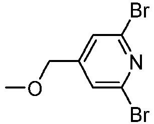

A mixture of 2,6-dibromo-4-(bromomethyl)pyridine (10.0 g, 30.32 mmol), MeOH (100 mL) and K₂CO₃ (8.38 g, 60.63 mmol) was stirred for 2 h at RT. Upon completion of the reaction, EA (200 mL) was added and the resulting mixture was washed with H₂O (200 mL x 3). The organic layer was dried over anhydrous Na₂SO₄ and concentrated under vacuum. The residue was purified by combi-flash (EA /PE = 0 - 25%) to give 2,6-dibromo-4-(methoxymethyl)pyridine (2.00 g, 23%). LCMS (ESI) m/e [M+1]⁺ 280.

### Step 3: 2-bromo-4-(methoxymethyl)-6-(methylsulfanyl)pyridine

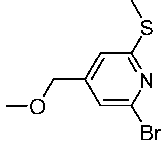

A mixture of 2,6-dibromo-4-(methoxymethyl)pyridine (2.00 g, 7.12 mmol), DMF (20 mL) and NaSCH₃ (0.50 g, 7.14 mmol) was stirred for 1 h at RT. Upon completion of the reaction, EA (100 mL) was added and the resulting mixture was washed with H₂O (200 mL x 3). The organic layer was dried over anhydrous Na₂SO₄ and concentrated under vacuum. The residue was purified by combi-flash (EA/PE = 0 - 25%) to give 2-bromo-4-(methoxymethyl)-6-(methylsulfanyl)pyridine (1.40 g, 79%). LCMS (ESI) m/e [M+1]⁺ 248.

### Step 4: 2-bromo-6-methanesulfonyl-4-(methoxymethyl)pyridine

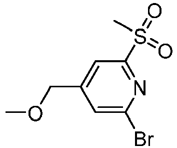

A mixture of 2-bromo-4-(methoxymethyl)-6-(methylsulfanyl)pyridine (1.40 g, 5.64 mmol), THF (10 mL), H₂O (10.00 mL), NaIO₄ (2.41 g, 11.26 mmol) and RuCl₃.H₂O (127.19 mg, 0.56 mmol) was stirred for 1h at 0 °C. Upon completion of the reaction, EA (100 mL) was added and the resulting mixture was washed with H₂O (200 mL x 3). The organic layer was dried over anhydrous Na₂SO₄, concentrated and purified by combi-flash (EA/PE = 0-30%) give 2-bromo-6-methanesulfonyl-4-(methoxymethyl)pyridine (1.10 **g,** 69%). ¹H NMR (300 MHz, CDCl₃) δ 7.99 (s, 1H), 7.74 (s, 1H), 4.56 (s, 2H), 3.50 (s, 3H), 3.27 (s, 3H). LCMS (ESI) m/e [M+1]⁺ 280.

### Example BB2: Synthesis of 2-bromo-4-[2-[(tert-butyldimethylsilyl)oxy]ethoxy]-6-methanesulfonyl pyridine

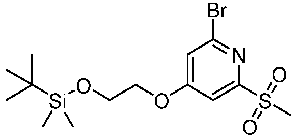

### Step 1: 2-bromo-6-(methylthio)pyridin-4-ol

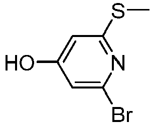

A mixture of 2, 6-dibromopyridin-4-ol (10.00 g, 39.542 mmol), DIEA (10.22 g, 0.079 mmol), Xantphos (0.23 g, 0.39 mmol), NaSCH₃ (2.93 g, 39.54 mmol) and Pd₂(dba)₃ (0.18 g, 0.20 mmol) in 1,4-dioxane (200 mL) was stirred overnight at 75 °C under nitrogen atmosphere. After cooled to room temperature, water was added and the resulting mixture was extracted with EA (100 mL x 3). The combined organic layers were washed with water, dried over anhydrous Na₂SO₄. After filtration, the filtrate was concentrated under vacuum and the crude product 2-bromo-6-(methylthio)pyridin-4-ol was used directly for next step without further purification. LCMS (ESI) m/e [M+1]⁺ 220.

### Step 2: 2-bromo-6-methanesulfonylpyridin-4-ol

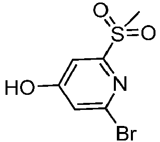

To a stirred solution of 2-bromo-6-(methylsulfanyl)pyridin-4-ol (6.00 g, 27.26 mmol) in H₂O (100 mL)/ THF (100 mL) was added RuCl₃.H₂O (0.18 g, 0.82 mmol) in water (30 mL) dropwise at 0 °C, then NaIO₄ (11.66 g, 0.055 mmol) was added dropwise successively at 0 °C. The resulting mixture was stirred for additional 30 mins at 0 °C. Upon completion of the reaction, the resulting mixture was extracted with EA (100 mL x 3). The combined organic layers were washed with water, dried over anhydrous Na₂SO₄. After filtration, the filtrate was concentrated under vacuum. The residue was purified by silica gel column chomatography (DCM/ MeOH = 12:1) to give 2-bromo-6-methanesulfonylpyridin-4-ol (3 g, 44%). LCMS (ESI) m/e [M+1]⁺ 252.

### Step 3: 2-bromo-4-[2-[(tert-butyldimethylsilyl)oxy]ethoxy]-6-methanesulfonylpyridine

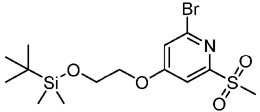

A mixture of 2-bromo-6-methanesulfonylpyridin-4-ol (1.00 g, 3.97 mmol), K₂CO₃ (1.10 g, 79.59 mmol) and (2-bromoethoxy)(tert-butyl)dimethylsilane (1.90 g, 79.42 mmol) in DMF (20 mL) was stirred for 3 h at 60 °C. After cooled to room temperature, water was added and the resulting mixture was extracted with EA (100 mL x 3). The combined organic layers were washed with brine, dried over anhydrous Na₂SO₄. After filtration, the filtrate was concentrated under vacuum. The residue was purified by Prep-TLC (hexane/EA = 5:1) to give the product (1.08 g, 68%). ¹H NMR (300 MHz, DMSO-*d*₆) δ 7.62 (s 1H), 7.54 (s 1H), 4.30 - 4.25 (m, 2H), 3.90 - 3.87 (m, 2H), 3.28 (s, 3H), 0.85 (s, 9H), 0.06 (s, 6H).

### Example BB3: Synthesis of 2-bromo-4-((2,2-dimethyl-1,3-dioxolan-4-yl)methoxy)-6-(methyl sulfonyl)pyridine

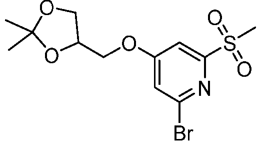

A mixture of 2-bromo-6-methanesulfonylpyridin-4-ol (200 mg, 0.79 mmol), Cs₂CO₃ (517.01 mg, 1.59 mmol) and (2,2-dimethyl-1,3-dioxolan-4-yl)methyl 4-methylbenzenesulfonate (272.62 mg, 0.95 mmol) in DMF (5 mL) was stirred for 3 h at 80 °C under nitrogen atmosphere. After cool to room temperature, water was added and the resulting mixture was extracted with EA (100 mL x 3). The combined organic layers were washed with water, dried over anhydrous Na₂SO₄. After filtration, the filtrate was concentrated under vacuum. The residue was purified by Prep-TLC (PE/EA = 5:1) to give 2-bromo-4-((2,2-dimethyl-1,3-dioxolan-4-yl)methoxy)-6-(methylsulfonyl)pyridine (260 mg, 89%). ¹H NMR (300 MHz, MeOD-*d*₄) δ 7.64 (s, 1H), 7.49 (s, 1H), 4.59 - 4.40 (m, 1H), 4.32 - 4.13 (m, 3H), 3.89 (d, *J =* 8.6 Hz, 1H), 3.23 (s, 3H), 1.41 (s, 3H), 1.37 (s, 3H). LCMS (ESI) m/e [M+1]⁺ 366.

### Example BB4: Synthesis of 2-bromo-4-(2-((tert-butyldimethylsilyl)oxy)propoxy)-6-(methylsulfonyl)pyridine

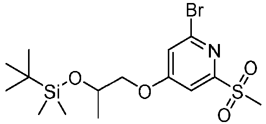

### Step 1: 1-((2-Bromo-6-(methylsulfonyl)pyridin-4-yl)oxy)propan-2-ol

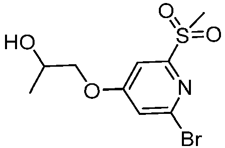

A mixture of 2-bromo-6-methanesulfonylpyridin-4-ol (500 mg, 1.98 mmol), 2-propanol-1-bromo (4.14 g, 29.75 mmol) and K₂CO₃ (548 mg, 3.97 mmol) in DMF (5 mL) was stirred for 12 h at 70 °C. After cool to room temperature, water was added and the resulting mixture was extracted with EA (100 mL x 3). The combined organic layers were washed with brine, dried over anhydrous Na₂SO₄. After filtration, the filtrate was concentrated to give the crude product 1-((2-Bromo-6-(methylsulfonyl)pyridin-4-yl)oxy)propan-2-ol was used for the next step directly without further purification. LCMS (ESI) m/e [M+1]⁺ 310.

### Step 2: 2-Bromo-4-(2-((tert-butyldimethylsilyl)oxy)propoxy)-6-(methylsulfonyl)pyridine

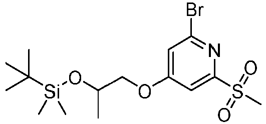

A solution of 1-[(2-bromo-6-methanesulfonylpyridin-4-yl)oxy]propan-2-ol (600 mg, 1.93 mmol), TBSCl (437.4 mg, 2.90 mmol) and DIEA (500 mg, 3.87 mmol) in DCM (10 mL) was stirred for 12 h at RT. Upon completion of the reaction, the solvent was removed and the residue was purified by Prep-TLC (EA/PE = 1:3) to give 2-bromo-4-[2-[(tert-butyldimethylsilyl)oxy]propoxy]-6-methanesulfonylpyridine (635 mg, 77%). ¹H NMR (300 MHz, DMSO-*d*₆) δ 7.61 (s, 1H), 7.52 (s, 1H), 4.24 - 4.05 (m, 1H), 3.35 (s, 3H), 3.28 (d, *J =* 4.4 Hz, 2H), 1.25 - 1.20 (m, 3H), 0.82 (s, 9H), 0.08 (s, 6H). LCMS (ESI) m/e [M+1]⁺ 425.

### Example BBS: Synthesis of 2-[(2-bromo-6-methanesulfonylpyridin-4-yl)oxy]acetonitrile

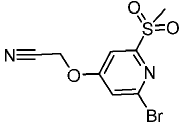

To a stirred solution of 2-bromo-6-methanesulfonylpyridin-4-ol (500 mg, 1.98 mmol) and 2-bromoacetonitrile (475 mg, 3.97 mmol) in DMF (10 mL) was added K₂CO₃ (548 mg, 3.97 mmol) in portions at room temperature, then the resulting mixture was stirred overnight at 80 °C. After cooled to room temperature, water was added and the resulting mixture was extracted with EA (40 mL x 3). The combined organic layers were washed with brine (3x30 mL), dried over anhydrous Na₂SO₄, concentrated and purified by Prep-TLC (PE/EA = 3:1) to give 2-[(2-bromo-6-methanesulfonylpyridin-4-yl)oxy]acetonitrile (254.3 mg, 44 %). ¹H NMR (300 MHz, MeOD-*d*₄) δ 7.73 (s, 1H), 7.63 (s, 1H), 5.27 (s, 2H), 3.27 (s, 3H). LCMS (ESI) m/e [M+1]⁺ 291.

### Example BB6: Synthesis of 2-bromo-4-isopropoxy-6-methanesulfonylpyridine

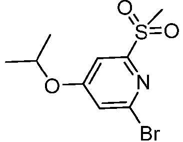

To a stirred solution of 2-bromo-6-methanesulfonylpyridin-4-ol (600 mg, 2.38 mmol) and 2-iodopropane (809 mg, 4.76 mmol) in DMF (10 mL) was added K₂CO₃ (658 mg, 4.76 mmol) in portions at room temperature. After cooled to room temperature, water was added and the resulting mixture was extracted with EA (40 mL x 3). The combined organic layers were washed with brine (30 mL x 3), dried over anhydrous Na₂SO₄. After filtration, the filtrate was concentrated under vacuum. The residue was purified by Prep-TLC (PE/EA = 3:1) to give the product (570 mg, 82%). ¹H NMR (400 MHz, MeOD-*d*₄) δ 7.54 (s, 1H), 7.41 (s, 1H), 4.96 - 4.85 (m, 1H), 3.24 (s, 3H), 1.40 *(d, J=* 6.1 Hz, 6H). LCMS (ESI) m/e [M+1]⁺ 294.

### Example BB7: Synthesis of 2-bromo-6-methanesulfonyl-4-[(trans)-3-[(tert-butyldimethylsilyl)oxy]cyclobutoxy]pyridine

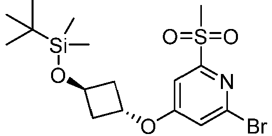

A solution of 2-bromo-6-methanesulfonylpyridin-4-ol (2.00 g, 7.93 mmol), PPh₃ (3.12 g, 11.89 mmol), DIAD (2.41 g, 11.92 mmol) and *trans*-3-[(tert-butyldimethylsilyl)oxy]cyclobutan-1-ol (1.61 g, 7.95 mmol) in THF (100 mL) was stirred overnight at 50 °C. After cooled to room temperature, water was added and the resulting mixture was extracted with EA (40 mL x 3). The combined organic layers were washed with brine, dried over anhydrous Na₂SO₄. After filtration, the filtrate was concentrated under vacuum. The residue was purified by Prep-TLC (hexane/EA = 5:1) to give the product (2.06 g, 60%). ¹H NMR (300 MHz, MeOD-*d*₄) δ 7.48 (s, 1H), 7.29 (s, 1H), 5.03 (t, *J =* 6.3 Hz, 1H), 4.70 - 4.52 (m, 1H), 3.23 (s, 3H), 2.59 - 2.37 (m, 4H), 0.92 (s, 9H), 0.08 (s, 6H). LCMS (ESI) m/e [M+1]⁺ 436.

### Example BB8: Synthesis of 2-bromo-6-methanesulfonylpyridine-4-carbonitrile

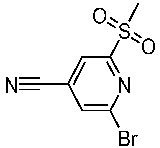

### Step1: 2-bromo-6-(methylsulfanyl)pyridine-4-carbonitrile

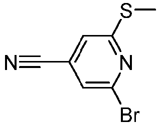

A mixture of 2,6-dibromopyridine-4-carbonitrile (4.00 g, 15.27 mmol), DMF (40 mL) and CH₃SNa (1.28 g, 18.33 mmol) was stirred overnight at rt. Upon completion of the reaction, water was added and the resulting solution was extracted with EA (60 mL x 3). The combined organic layer was washed with brine, dried over anhydrous Na₂SO₄. The solid were filtered out and the resulting mixture was concentrated under vacuum. The residue was purified by combi-flash (EA/PE = 0 - 5%) to give the product (1.50 g, 43%). GCMS (ESI) [M] 228.

### Step 2: 2-bromo-6-methanesulfonylpyridine-4-carbonitrile

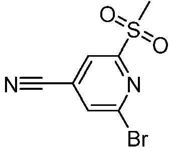

To a solution of 2-bromo-6-(methylsulfanyl)pyridine-4-carbonitrile (1.50 g, 6.55 mmol) in THF (15 mL) was added NaIO₄ (2.80 g, 13.10 mmol) in H₂O (20 mL) in portions at 0 °C, then to this was added RuCl₃.H₂O (147.60 mg, 0.655 mmol) in H₂O (10 mL) in portions at 0 °C. The resulting solution was stirred for 2 h at RT. Upon completion of the reaction, the resulting solution was extracted with EA (60 mL x 3) and the combined organic layer was washed with brine, dried over anhydrous Na₂SO₄. The solid was filtered out and the resulting mixture was concentrated under vacuum and the residue was purified by Prep-TLC (EA/PE = 1:2) to give the product (1.06 g, 62%).¹H NMR (300 MHz, DMSO-*d*₆) δ 8.65 (s, 1H), 8.55 (s, 1H), 3.40 (s, 3H); GC-MS (ESI) [M] 260.

### Example BB9: Synthesis of 2-bromo-6-methanesulfonyl-4-methoxypyridine

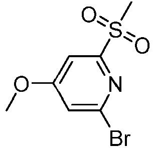

To a mixture of 2-bromo-6-methanesulfonylpyridin-4-ol (5.00 g, 19.84 mmol) and K₂CO₃ (5.49 g, 0.04 mmol) in DMF (100 mL) was added CH₃I (4.20 g, 29.59 mmol) dropwise at 60 °C for 2 h. After cooled to rt, water was added and the resulting mixture was extracted with EA (150 mL x 3). The combined organic layers were washed with brine, dried over anhydrous Na₂SO₄. After filtration, the filtrate was concentrated under vacuum. The residue was purified by combi-flash (EA/PE = 25 - 35%) to give the product (4.00 g, 76%). ¹H NMR (300 MHz, CDCl₃) δ 7.56 (s, 1H), 7.18 (s, 1H), 3.95 (s, 3H), 3.24 (s, 3H). LCMS (ESI) m/e [M+1]⁺ 266.

### Example BB10: Synthesis of 6-bromo-4-methylpyridine-2-sulfonamide

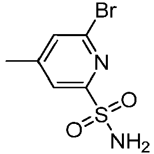

### Step 1: 2-(benzylsulfanyl)-6-bromo-4-methylpyridine

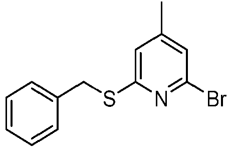

A mixture of 2,6-dibromo-4-methylpyridine (2.00 g, 7.97 mmol), DMF (30 mL), benzyl mercaptan (1.09 g, 8.78 mmol) and Cs₂CO₃ (5.19 g, 15.93 mmol) was stirred for 2 h at RT. Upon completion of the reaction, EA (100 mL) was added and the resulting mixture was washed with water (100 mL x 3). Then the mixture was dried over anhydrous Na₂SO₄ and concentrated under vacuum. The residue was purified by combi-flash (EA/PE = 0 - 25%) give the product (2.30 g, 98%). LCMS (ESI) m/e [M+1]⁺ 294.

### Step 2: 6-bromo-4-methylpyridine-2-sulfonyl chloride

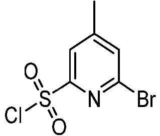

A mixture of 2-(benzylsulfanyl)-6-bromo-4-methylpyridine (2.30 g, 7.82 mmol), AcOH (36 mL), H₂O (4 mL) and NCS (3.65 g, 27.33 mmol) was stirred for 1 h at RT. Upon completion of the reaction, EA (100 mL) was added and the resulting mixture was washed with water (100 mL x 3). The mixture was dried over anhydrous Na₂SO₄ and concentrated under vacuum. The residue was purified by combi-flash (EA/PE = 0 - 25%) give the product (2.00 g, 94%).

### Step 3: 4-methylpyridine-2-sulfonamide

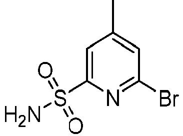

A solution of 6-bromo-4-methylpyridine-2-sulfonyl chloride (2.00 g, 7.39 mmol) in THF (20 mL) was added ammonium (10 mL, 33% wt) dropwise at 0 °C and was stirred for 1 h at RT. Upon completion of the reaction, EA (100 mL) was added and the resulting mixture was washed with water (100 mL x 3). The mixture was dried over anhydrous Na₂SO₄ and concentrated. The residue was purified by combi-flash (EA/PE = 0 - 50%) give the product (1.02 g, 55%). ¹H NMR (300 MHz, DMSO-*d*₆) δ 7.84 (s, 1H), 7.62 (s, 2H), 7.37 (s, 1H), 2.43 (s, 3H). LCMS (ESI) m/e [M+1]⁺ 251.

### Example BB11: Synthesis of 6-bromo-4-(2-methoxyethoxy)pyridine-2-sulfonamide

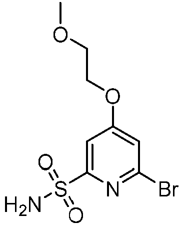

### Step 1: 2,6-dibromo-4-(2-methoxyethoxy)pyridine

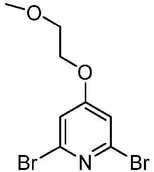

A mixture of 2, 6-dibromopyridin-4-ol (2.02 g, 7.90 mmol), 2-bromoethyl methyl ether (1.65 g, 11.86 mmol) and K₂CO₃ (2.19 g, 15.82 mmol) in DMF (10.00 mL) was stirred at 80 °C for 3 h. After cooled to RT, water was added and the resulting mixture was extracted with EA (150 mL x 3). The combined organic layers were washed with brine, dried over anhydrous Na₂SO₄. After filtration, the filtrate was concentrated under vacuum to afford crude product. The crude product was used for the next step directly without further purification. LCMS (ESI) m/e [M+1]⁺ 310.

### Step 2: 6-bromo-4-(2-methoxyethoxy)pyridine-2-sulfonyl chloride

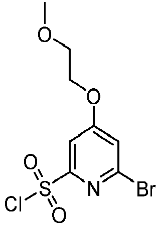

To a stirred solution of 2,6-dibromo-4-(2-methoxyethoxy)pyridine (1.90 g, 6.11 mmol) in THF (10 mL) was added i-PrMgCl (4.0 mL, 8.00 mmol, 2M) dropwise at 0 °C and the resulting mixture was stirred for 1 h at RT. under nitrogen atmosphere. SO₂Cl₂ (1.2 mL) in hexane (3 mL) was added dropwise at 0 °C and the resulting mixture was stirred for 1h at rt under nitrogen atmosphere. Upon completion of the reaction, the reaction was quenched with cool water. The resulting mixture was concentrated under vacuum to afford crude product 6-bromo-4-(2-methoxyethoxy) pyridine-2-sulfonyl chloride (2.0 g). It was used directly for next step without purification.

### Step 3: 6-bromo-4-(2-methoxyethoxy)pyridine-2-sulfonamide

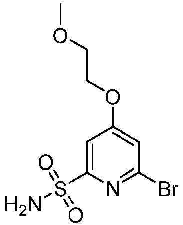

A mixture of 6-bromo-4-(2-methoxyethoxy) pyridine-2-sulfonyl chloride (1.80 g, 5.44 mmol) in NH₃/THF (0.5M, 33 mL) was stirred at 1 h at RT. Upon completion of the reaction, the solvent was removed under vacuum and the residue was purified by prep-HPLC (column: Phenomenex Gemini-NX; phase: A-H₂O; B-Acetonitrile, B%: 15%-25% in 15 min) to give the product (128 mg, 81%). ¹H NMR (300 MHz, DMSO-*d*₆) δ 7.63 (s, 2H), 7.52 (s, 1H), 7.43 (s, 1H), 4.34 - 4.32 (m, 2H), 3.68 - 3.66 (m, 2H), 3.31 (s, 3H). LCMS (ESI) m/e [M+1]⁺ 312.

### Example BB12: Synthesis of 2-bromo-4-(difluoromethyl)-6-(methylsulfonyl)pyridine

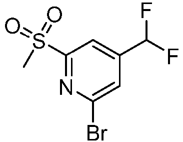

### Step 1: (2,6-dibromopyridin-4-yl)methanol

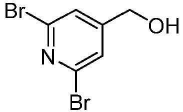

A solution of 2,6-dibromopyridine-4-carboxylic acid (2.00 g, 7.12 mmol) in THF (20 mL) was added BH₃.THF (14 mL, 14.00 mmol, 1 M in THF) dropwise and the resulting mixture was stirred for 48 h at RT. Upon completion of the reaction, water was added slowly to quench the reaction and the resulting mixture was extracted with EA (3 x 200 mL). The combined organic layers were dried over anhydrous Na₂SO₄. After filtration, the filtrate was concentrated under vacuum. The crude product was used directly for the next step without further purification. LCMS (ESI) m/e [M+1]⁺ 266.

### Step 2: 2,6-dibromoisonicotinaldehyde

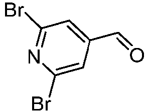

A solution of (2, 6-dibromopyridin-4-yl)methanol (1.50 g, 5.62 mmol) and DMP (3.58 g, 8.43 mmol) in DCM (15 mL) was stirred for 12 h at RT. Upon completion of the reaction, water was added and the resulting mixture was extracted with DCM (200 mL x 3). The combined organic layers were dried over anhydrous Na₂SO₄. After filtration, the filtrate was concentrated under vacuum. The residue was purified by Prep-TLC (DCM/PE = 1:1) to give the product (900 mg, 60%).

### Step 3: 2,6-dibromo-4-(difluoromethyl)pyridine

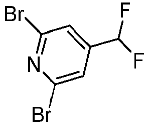

To a stirred solution of 2,6-dibromopyridine-4-carbaldehyde (850 mg, 3.21 mmol) in DCM (8.5 mL) was added DAST (1.55 g, 9.63 mmol) dropwise at rt. The resulting mixture was stirred for additional 1 h at RT. Upon completion of the reaction, EtOH was added and the resulting mixture was concentrated under vacuum. The crude product was used directly for the next step without further purification. LCMS (ESI) m/e [M+1]⁺ 286.

### Step 4: 2-bromo-4-(difluoromethyl)-6-(methylthio)pyridine

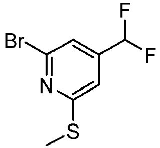

A solution of 2,6-dibromo-4-(difluoromethyl)pyridine (1.00 g, 3.49 mmol) and MeSNa (195 mg, 2.79 mmol) in DMF (10 mL) was stirred for 2 h at RT. Upon completion of the reaction, water was added and the resulting mixture was extracted with EA (100 mL x 3). The combined organic layers were dried over anhydrous Na₂SO₄. After filtration, the filtrate was concentrated under vacuum. The crude product was used directly for the next step without further purification. LCMS (ESI) m/e [M+1]⁺ 254.

### Step 5: 2-bromo-4-(difluoromethyl)-6-(methylsulfonyl)pyridine

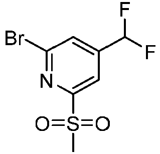

To a stirred solution of 2-bromo-4-(difluoromethyl)-6-(methylsulfanyl)pyridine (450 mg, 1.77 mmol) in H₂O (5 mL) amd THF (5 mL) was added RuCl₃.H₂O (12 mg, 0.053 mmol) in water (2 mL) dropwise at 0 °C. To the above mixture was added NaIO₄ (1515 mg, 7.08 mmol) dropwise at 0 °C. The resulting mixture was stirred for additional 30 mins at 0 °C. Upon completion of the reaction, the resulting mixture was extracted with EA (3 x 100 mL) and the combined organic layers were dried over anhydrous Na₂SO₄. After filtration, the filtrate was concentrated under vacuum. The residue was purified by Prep-TLC (EA/PE = 1: 4) to give the product (317 mg, 63%). ¹H NMR (300 MHz, DMSO-*d*₆) δ 8.28 (s, 1H), 8.21 (s, 1H), 7.40 - 7.05 (m, 1H), 3.37 (s, 3H). LCMS (ESI) m/e [M+1]⁺ 286.

### Example BB13: Synthesis of 2-bromo-6-methanesulfonyl-4-(oxetan-3-ylmethoxy) pyridine

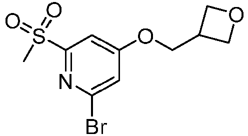

A mixture of 2-bromo-6-methanesulfonylpyridin-4-ol (1.00 g, 3.97 mmol), PPh₃ (3.12 g, 11.89 mmol), DIAD (2.41 g, 11.92 mmol) and oxetan-3-ylmethanol (0.35 g, 3.97 mmol) in THF (20 mL) was stirred overnight at 50 °C. After cooled to RT, water was added and the resulting mixture was extracted with EA (100 mL x 3). The combined organic layers were washed with brine, dried over anhydrous Na₂SO₄. After filtration, the filtrate was concentrated under vacuum. The residue was purified by Prep-TLC (hexane/EA = 5:1) to give the product (996 mg, 78%). ¹H NMR (300 MHz, DMSO-*d*₆) δ 7.65 (s, 1H), 7.58 (s, 1H), 4.71 (d, *J =* 7.9 Hz, 2H), 4.52 - 4.37 (m, 4H), 3.49 - 3.37 (m, 1H), 2.54 (s, 3H). LCMS (ESI) m/e [M+1]⁺ 322.

### Example BB14: Synthesis of 2-bromo-6-methanesulfonyl-4-(oxetan-3-yloxy) pyridine

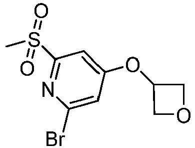

A solution of 2-bromo-6-methanesulfonylpyridin-4-ol (826 mg, 3.27 mmol), PPh₃ (3.12 g, 11.89 mmol), DIAD (2.41 g, 11.92 mmol)) and oxetan-3-ol (0.59 g, 7.93 mmol) in THF (20 mL) was stirred overnight at 50 °C. After cooled to RT, water was added, and the resulting mixture was extracted with EA (50 mL x 3). The combined organic layers were washed with brine, dried over anhydrous Na₂SO₄. After filtration, the filtrate was concentrated under vacuum. The residue was purified by Prep-TLC (hexane/EA = 5:1) to give (899 mg, 89 %) of the product . ¹H NMR (300 MHz, DMSO-*d*₆) δ 7.50 (s, 1H), 7.45 (s, 1H), 5.59 - 5.50 (m, 1H), 4.99 - 4.95 (m, 2H), 4.60 - 4.55 (m, 2H), 3.29 (s, 3H). LCMS (ESI) m/e [M+1]⁺ 308.

### Example BB15: Synthesis of 2-bromo-6-methanesulfonyl-4-(oxetan-3-ylmethoxy) pyridine

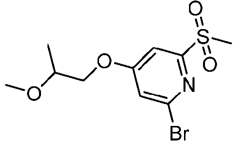

A solution of 2-bromo-6-methanesulfonylpyridin-4-ol (1.00 g, 3.97 mmol), PPh₃ (3.12 g, 11.89 mmol), DIAD (2.41 g, 11.92 mmol) and 2-methoxypropan-1-ol (0.44 g, 3.97 mmol) in THF (20 mL) was stirred overnight at 50 °C. After cooled to RT, water was added and the resulting mixture was extracted with EA (100 mL x 3). The combined organic layers were washed with brine, dried over anhydrous Na₂SO₄. After filtration, the filtrate was concentrated under vacuum. The residue was purified by Prep-TLC (hexane/EA = 5:1) to give the product (900 mg, 70%) of the product . ¹H NMR (300 MHz, DMSO-*d*₆) δ 7.63 (s, 1H), 7.56 (s, 1H), 4.31- 4.14 (m, 2H), 3.70 - 3.65 (m, 1H), 3.39 (m, 3H), 3.25 (m, 3H), 1.17 - 1.14 (m, 3H). LCMS (ESI) m/e [M+1]⁺ 324.

### Example BB16: Synthesis of 2-bromo-6-methanesulfonyl-3-methoxypyridine

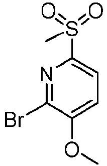

### Step1: 2-bromo-3-methoxy-6-(methylsulfanyl)pyridine

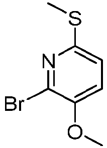

A mixture of 2-bromo-6-iodo-3-methoxypyridine (4.00 g, 12.74 mmol), (methylsulfanyl)sodium (0.80 g, 11.41 mmol), Pd₂(dba)₃ (0.58 g, 0.63 mmol), Xantphos (0.74 g, 1.27 mmol) in dioxane (64 mL) was stirred for 3 h at 75 °C under nitrogen atmosphere. After cooled to rt, water was added, and the resulting mixture was extracted with EA (200 mL x 3). The combined organic layers were washed with brine, dried over anhydrous Na₂SO₄. After filtration, the filtrate was concentrated under vacuum and purified by combi-flash (EA/PE = 0 - 4%) to give the product (1.72 g, 52%). LCMS (ESI, *m*/*e)* [M+1]⁺ 234.

### Step 2: 2-bromo-6-methanesulfonyl-3-methoxypyridine

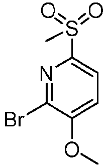

To a mixture of 2-bromo-3-methoxy-6-(methylsulfanyl)pyridine (1.72 g, 7.34 mmol) in THF (15 mL) were added NaIO₄ (4.73 g, 22.14 mmol) and RuCl₃ (0.05 g, 0.22 mmol) in H₂O (15 mL) dropwise at 0 °C. The resulting solution was stirred for 1 h at RT. Upon completion of the reaction, the resulting mixture was concentrated under vacuum. The residue was purified by combi-flash (EA/PE = 0-39%) to give the product (1.78 g, 82%). ¹H NMR (300 MHz, DMSO-*d*₆) δ 8.06 (d, *J= 8.5* Hz, 1H), 7.75 (d, *J=* 8.5 Hz, 1H), 4.01 (s, 3H), 3.24 (s, 3H). LCMS (ESI, *m*/*e)* [M+1]⁺ 266.

### Example BB17: Synthesis of 1-bromo-3-methanesulfonyl-5-(2-methoxyethoxy) benzene

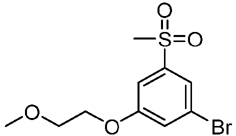

### Step1: 1,3-dibromo-5-(2-methoxyethoxy) benzene

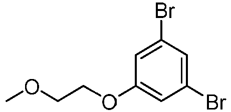

A mixture of 3,5-dibromophenol (11.00 g, 43.66 mmol), 2-bromoethyl methyl ether (15.17 g, 109.14 mmol) and Cs₂CO₃ (28.46 g, 87.33 mmol) in DMF (165 mL) was stirred for 4 h at 80 °C. After cooled to RT, water was added then extracted with EA (500 mL x 3). The combined organic layers were washed with brine, dried over anhydrous Na₂SO₄. After filtration, the filtrate was concentrated to give the crude product and used directly for the next step without further purification. LCMS (ESI, m/e) [M+1]⁺ 311.

### Step 2: 1-bromo-3-(2-methoxyethoxy)-5-(methylsulfanyl) benzene

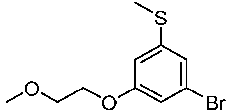

A mixture of 1,3-dibromo-5-(2-methoxyethoxy) benzene (15.00 g, 48.39 mmol), (methylsulfanyl)sodium (2.71 g, 38.67 mmol), Pd₂(dba)₃ (2.22 g, 2.42 mmol), Xantphos (2.80 g, 4.83 mmol) in dioxane (150 mL) was stirred overnight at 75 °C under nitrogen atmosphere. After cooled to RT, the resulting mixture was concentrated under vacuum. The residue was purified by combi-flash (EA/PE = 0-1%) to give the product (5.71 g, 38%). LCMS (ESI, m/e) [M+1]⁺ 277.

### Step 3: 1-bromo-3-methanesulfonyl-5-(2-methoxyethoxy) benzene

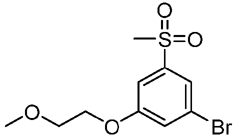

To a mixture of 1-bromo-3-(2-methoxyethoxy)-5-(methylsulfanyl) benzene (5.71 g, 20.60 mmol) in THF (60 mL) and H₂O (30 mL) was added the solution of NaIO₄ (13.22 g, 61.81 mmol) in H₂O (15 mL) in portions at 0 °C. Then to the mixture was added the solution of RuCl₃ (0.14 g, 0.62 mmol) in H₂O (15 mL) in portions at 0 °C. The resulting solution was stirred for 1 h at RT. Upon completion of the reaction, the resulting solution was extracted with EA (50 mL x 3). The solvent was removed under vacuum and the residue was purified by combi-flash (EA/PE = 0 - 31%) to give the product (4.99 g, 71%). ¹H NMR (300 MHz, DMSO-*d*₆) δ 7.65 (s, 1H), 7.56 (s, 1H), 7.46 (s, 1H), 4.30 - 4.22 (m, 2H), 3.70 - 3.65 (m, 2H), 3.31 (s, 3H), 3.29 (s, 3H). LCMS (ESI, m/e) [M+1]⁺ 309.

### Example BB18: Synthesis of [2-(3-bromo-5-methanesulfonylphenoxy)ethoxy](tert-butyl)dimethylsilane

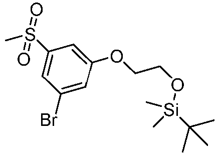

### Step 1: tert-butyl[2-(3,5-dibromophenoxy)ethoxy]dimethylsilane

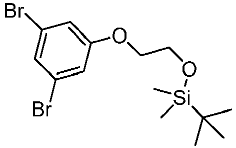

A mixture of 3,5-dibromophenol (5.00 g, 19.85 mmol), (2-bromoethoxy)(tert-butyl)dimethyl silane (7.12 g, 29.77 mmol), Cs₂CO₃ (12.93 g, 39.69 mmol) in DMF (50 mL) was stirred for 2 h at 80 °C. After cooled to RT, water was added and the resulting mixture was extracted with EA (80 mL x 3). The combined organic layers were washed with water, dried over anhydrous Na₂SO₄. After filtration, the filtrate was concentrated under vacuum. The residue was purified by combi-flash (EA/PE = 0 - 20%) to give the product (5.00 g, 61%).

### Step 2: [2-[3-bromo-5-(methylsulfanyl)phenoxy]ethoxy](tert-butyl)dimethylsilane

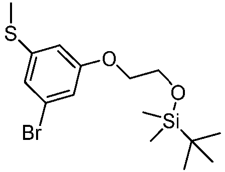

A mixture of tert-butyl[2-(3,5-dibromophenoxy)ethoxy]dimethylsilane (5.00 g, 12.19 mmol), (methylsulfanyl)sodium (768 mg, 10.97 mmol), Xantphos (705 mg, 1.22 mmol), DIEA (3.15 g, 24.38 mmol), Pd₂(dba)₃ (558 mg, 0.61 mmol) and 1,4-dioxane (50 mL) was stirred overnight at 90 °C under nitrogen atmosphere. After cooled to RT, water was added, and the resulting mixture was extracted with EA (120 mL x 3). The combined organic layers were washed with brine (50 mL x 3), dried over anhydrous Na₂SO₄. After filtration, the filtrate was concentrated under vacuum. The residue was purified by combi-flash (EA/PE = 0-10%) to give the product (2.80 g, 60%). LCMS (ESI, m/e) [M+1]⁺ 377.

### Step 3: [2-(3-bromo-5-methanesulfonylphenoxy)ethoxy](tert-butyl)dimethylsilane

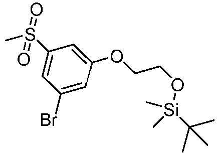

To a stirred solution of [2-[3-bromo-5-(methylsulfanyl)phenoxy]ethoxy](tert-butyl)dimethylsilane (2.80 g, 7.41 mmol) in THF (30 mL) were added RuCl₃.H₂O (50 mg, 0.22 mmol) and NaIO₄ (3.17 g, 14.84 mmol) in H₂O (20 mL) at 0 °C and the resulting solution was stirred for 2 h at this temperature. Upon completion of the reaction, the reaction mixture was filtered and the filtrate was concentrated under vacuum. The residue was purified by combi-flash (EA/PE = 0 - 30%) to give the product (1.07 g, 35%). ¹H NMR (300 MHz, DMSO-*d*₆) δ 7.64 (s, 1H), 7.57 - 7.52 (m, 1H),7.44 (s, 1H), 4.20 - 4.15 (m, 2H), 3.96 - 3.90 (m, 2H), 3.29 (s, 3H), 0.86 (s, 9H), 0.07 (s, 6H). LCMS (ESI) m/e [M+1]⁺ 409.

### Example BB19: Synthesis of 1-bromo-3-isopropoxy-5-(methylsulfonyl)benzene

### Step 1: 1,3-dibromo-5-isopropoxybenzene

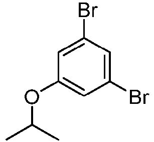

A mixture of 3,5-dibromophenol (10 g, 39.69 mmol), DMF (200 mL), 2-iodopropane (20.24 g, 119.06 mmol) and K₂CO₃ (10.97 g, 79.38 mmol) was stirred for 2 h at 80 °C. After cooled to RT, EA (200 mL) was added and the resulting mixture was washed with H₂O (200 mL x 3). The mixture was dried over anhydrous Na₂SO₄ and concentrated under vacuum. The residue was purified by combi-flash (EA /PE = 0-15%) give the product (7.6 g, 65%).

### Step 2: 1-bromo-3-isopropoxy-5-(methylsulfanyl)benzene

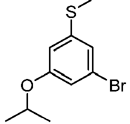

A mixture of 1,3-dibromo-5-isopropoxybenzene (7.60 g, 25.85 mmol), dioxane (100 mL), Pd₂(dba)₃ (1.18 g, 1.29 mmol), Xantphos (1.50 g, 2.59 mmol), MeSNa (1.81 g, 25.85 mmol) was stirred for 3 h at 75 °C. After cooled to RT, EA (200 mL) was added and the resulting mixture was washed with H₂O (200 mL x 3). The mixture was dried over anhydrous Na₂SO₄ and concentrated under vacuum. The residue was purified by combi-flash (EA/PE = 0 - 15%) give the product (7.1 g, crude). GCMS (ESI) m/e [M+1] 260.

### Step 3: 1-bromo-3-isopropoxy-5-(methylsulfonyl)benzene

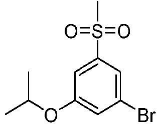

A mixture of 1-bromo-3-isopropoxy-5-(methylsulfanyl)benzene (7.10 g, 27.18 mmol), THF (60 mL), H₂O (60 mL), NaIO₄ (23.26 g, 108.75 mmol) and RuCl₃.H₂O (1.23 g, 5.46 mmol) was stirred for 1 h at 0 °C. Upon completion of the reaction, the resulting solution was diluted with EA (200 mL). The resulting mixture was washed with H₂O (100 mL x 3). The mixture was dried over anhydrous Na₂SO₄ and concentrated. The residue was purified by combi-flash (EA/PE = 0 - 25%) give the product (4.25 g, 53%). ¹H NMR (300 MHz, CDCl₃) δ 7.63 (s, 1H), 7.37 (s, 1H), 7.30 (s, 1H), 4.62 (q, *J=* 6.1 Hz, 1H), 3.08 (s, 3H), 1.38 (d, *J=* 6.0 Hz, 6H). GCMS (ESI) m/e [M] 292.

### Example BB20: Synthesis of 1-bromo-3-methoxy-5-(methylsulfonyl)benzene

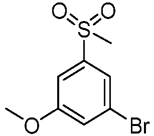

### Step 1: (3-bromo-5-methoxyphenyl)(methyl)sulfane

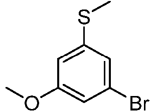

A mixture of 1,3-dibromo-5-methoxybenzene (9.00 g, 33.16 mmol), dioxane (100 mL), NaSCH₃ (3.19 g, 33.19 mmol), Pd₂(dba)₃ (1.52 g, 1.66 mmol) and Xantphos (1.92 g, 3.31 mmol) was stirred overnight at 75 °C under nitrogen atmosphere. After cooled to RT, the solvent was removed under vacuum and the residue was purified by combi-flash (EA/PE = 0-10%) to give the product (3.2 g, 39%). GCMS (ESI) m/e [M] 232.

### Step 2: 1-bromo-3-methoxy-5-(methylsulfonyl)benzene

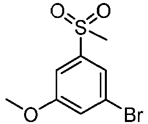

To a mixture of 1-bromo-3-methoxy-5-(methylsulfanyl)benzene (3.10 g, 12.63 mmol) and THF (30 mL)/H₂O (30 mL) was added NaIO₄ (10.81 g, 50.54 mmol) in H₂O (15 mL) in portions at 0 °C, then RuCl₃ (0.14 g, 0.62 mmol) in H₂O (15 mL) was added in portions successively at 0 °C. The resulting solution was stirred for 1 h at RT. The resulting solution was diluted with H₂O (50 mL), extracted with EA (30 mL x 3), the organic layers were concentrated and purified by combi-flash (EA/PE = 0-30%) to give the product (3.22 g, 91%). ¹H NMR (300 MHz, DMSO-d6) δ 7.66 (s, 1H), 7.54 (s, 1H), 7.45 (s, 1H), 3.89 (s, 3H), 3.30 (s, 3H). GCMS (ESI) m/e [M] 264.

### Example BB21: Synthesis of 1-bromo-3-methyl-5-(methylsulfonyl)benzene

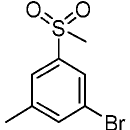

### Step 1: (3-bromo-5-methylphenyl)(methyl)sulfane

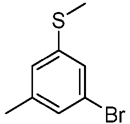

A mixture of 1,3-dibromo-5-methylbenzene (11.00 g, 43.13 mmol), dioxane (150 mL), NaSCH₃ (3.02 g, 43.14 mmol), Pd₂(dba)₃ (1.97 g, 2.15 mmol and Xantphos (2.50 g, 4.32 mmol) was stirred overnight at 75 °C under nitrogen atmosphere. After cooled to RT, the solvent was removed under vacuum and the residue was purified by combi-flash (EA/PE = 0 - 8%) to give the product (4.40 g, 42%). GCMS (ESI) m/e [M] 216

### Step 2: 1-bromo-3-methyl-5-(methylsulfonyl)benzene

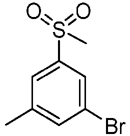

To a mixture of 1-bromo-3-methyl-5-(methylsulfanyl)benzene (4.30 g, 17.82 mmol) and THF (20 mL) was added NaIO₄ (15.25 g, 71.29 mmol) in H₂O (10 mL) in portions at 0 °C, then RuCl₃ (0.20 g, 0.89 mmol) in H₂O (10 mL) was added in portions successively at 0 °C. The resulting solution was stirred for 1 h at RT. Upon completion of the reaction, the resulting solution was diluted with 50 mL of H₂O, extracted with 3 x 30 mL of EA and the organic layers were combined and concentrated. The residue was purified by combi-flash (EA/PE = 0-20%) to give the product (4.51 g, 96%). ¹H NMR (300 MHz, DMSO-*d*₆) δ 7.91 (s, 1H), 7.84 -7.72 (m, 2H), 3.28 (s, 3H), 2.46 - 2.40 (m, 3H). GCMS (ESI) m/e [M] 248.

### Example BB22: Synthesis of 1-bromo-3-methanesulfonyl-5-(methoxymethyl)benzene

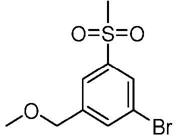

### Step 1: 1,3-dibromo-5-(methoxymethyl)benzene

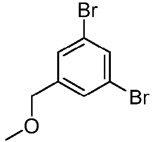

A mixture of 1,3-dibromo-5-(bromomethyl)benzene (7.00 g, 21.28 mmol), MeONa (5.75 g, 106.40 mmol) in MeOH (70 mL) and was stirred for 1 h at 70 °C. After cooled to rt, the solvent was removed under vacuum and the residue was diluted with water. The resulting solution was extracted with EA (100 mL x 3) and the combined organic layer was washed with brine. The organic layer was dried over anhydrous Na₂SO₄ and concentrated under vacuum. The residue was purified by combi-flash (EA/PE = 0-5%) to give the product (5.00 g, 84%). GCMS (ESI) m/e [M] 280.

### Step 2: 1-bromo-3-(methoxymethyl)-5-(methylsulfanyl)benzene

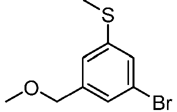

A mixture of 1,3-dibromo-5-(methoxymethyl)benzene (5.00 g, 17.86 mmol), DMF (50 mL) and CH₃SNa (1.50 g, 21.43 mmol) was stirred overnight at RT. Upon completion of the reaction, water (20 mL) was added and the resulting solution was extracted with EA (100 mL x 3). The combined organic layers were washed with brine and dried over anhydrous Na₂SO₄. The solids were filtered out and the resulting mixture was concentrated under vacuum. The residue was purified by combi-flash (EA/PE = 0 - 2%) to give the product (3.50 g, 79%). GCMS (ESI) m/e [M] 246.

### Step 3: 1-bromo-3-methanesulfonyl-5-(methoxymethyl)benzene

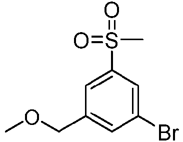

A mixture of 1-bromo-3-(methoxymethyl)-5-(methylsulfanyl)benzene (3.50 g, 14.16 mmol), RuCl₃.H₂O (319.26 mg, 1.416 mmol) and NaIO₄ (6.06 g, 28.32 mmol) in THF (35 mL) and H₂O (35 mL) was stirred for 2 h at RT. Upon completion of the reaction, the resulting solution was extracted with EA (100 mL x 3) and the organic layers combined. The resulting mixture was washed with brine, dried over anhydrous Na₂SO₄. The resulting mixture was concentrated under vacuum and the residue was purified by combi-flash (EA/PE = 0 - 10%) to give the product (2.21 g, 56%). ¹H NMR (300 MHz, DMSO-*d*₆) δ 8.05 (s, 1H), 7.75 (s, 1H), 7.70 (s, 1H), 4.50 (s, 2H), 3.45 (s, 3H), 3.03 (s, 3H). GCMS (ESI) m/e [M] 278.

### Example BB23: Synthesis of 1-bromo-3-(difluoromethyl)-5-methanesulfonylbenzene

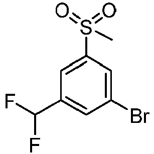

### Step 1: 1-bromo-3-(difluoromethyl)-5-(methylsulfanyl)benzene

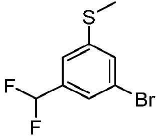

A mixture of 1,3-dibromo-5-(difluoromethyl)benzene (4.20 g, 14.69 mmol), (methylsulfanyl) sodium (927 mg, 13.22 mmol), DIEA (3.80 g, 29.38 mmol), Xantphos (849.97 mg, 1.47 mmol), Pd₂(dba)₃ (672.58 mg, 0.74 mmol) and 1,4-dioxane (50 mL) was stirred overnight at 80 °C under nitrogen atmosphere. After cooled to RT, the resulting mixture was extracted with EA (150 mL x 3) and the combined organic layers were washed with brine, dried over anhydrous Na₂SO₄. After filtration, the filtrate was concentrated under vacuum. The residue was purified by combi-flash (EA/PE = 0-10%) to give the product (2.60 g, 69%). GCMS (ESI) m/e [M] 252

### Step 2: 1-bromo-3-(difluoromethyl)-5-methanesulfonylbenzene

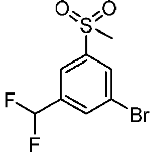

To a stirred solution of 1-bromo-3-(difluoromethyl)-5-(methylsulfanyl)benzene (2.60 g, 10.27 mmol) in THF (40 ml) and H₂O (40 mL) were added RuCl₃.H₂O (69.48 mg, 0.31 mmol) and NaIO₄ (4.39 g, 20.55 mmol) in portions at 0 °C. The resulting mixture was stirred for 2 h at 0 °C. Upon completion of the reaction, the resulting mixture was filtered out and the filtrate was concentrated under vacuum. The residue was purified by combi-flash (EA/PE = 0 - 40%) to give the product (1.79 g, 61%). ¹H NMR (300 MHz, DMSO-*d*₆) δ 8.31 (s, 1H), 8.19 (s, 1H), 8.13 (s, 1H), 7.20 - 7.10 (m, 1H), 3.37 (s, 3H).

### Example BB24: Synthesis of 3-bromo-5-methanesulfonylbenzonitrile

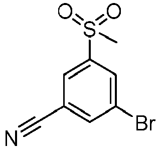

### Step1: 3-bromo-5-(methylsulfanyl)benzonitrile

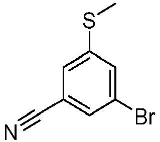

A mixture of 3,5-dibromobenzonitrile (10.00 g, 38.32 mmol), dioxane (160 mL), (methylsulfanyl) sodium (2.42 g, 34.53 mmol), Pd₂(dba)₃ (1.75 g, 1.91 mmol) and Xantphos (2.22 g, 3.83 mmol) was stirred overnight at 75°C in an oil bath. After cooled to room temperature, the solvent was removed in vacuo and the residue was diluted with water (20 mL). The resulting solution was extracted with EA (100 mL x 3) and the combined organic layers were washed with brine, dried over anhydrous sodium sulfate. The solvent was removed in vacuo and the residue was purified by combi-flash (EA/PE = 0 - 9%) to give the product (8.52 g, 88%). LCMS (ESI, m/e) [M+1]⁺ 228.

### Step2: 3-bromo-5-methanesulfonylbenzonitrile

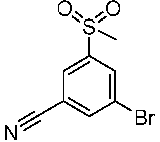

To a mixture of 3-bromo-5-(methylsulfanyl) benzonitrile (8.52 g, 37.35 mmol) and THF (75 mL) were added NaIO₄ (23.97 g, 112.06 mmol) in H₂O (35 mL) and RuCl₃ (0.25 g, 1.12 mmol) in H₂O (35 mL) dropwise with stirring at 0 °C. The resulting solution was stirred for 1 h at RT. Upon completion of the reaction, the resulting solution was extracted with EA (100 mL x 3) and then concentrated under vacuum. The residue was purified by combi-flash (EA/PE = 0-38%) to give the product (4.40 g, 40%). ¹H NMR (300 MHz, DMSO-*d*₆) δ 8.55 (s, 1H), 8.45 - 8.32 (m, 2H), 3.36 (s, 3H). LCMS (ESI, m/e) [M+1]⁺ 260.

### Example BB25: Synthesis of 1-bromo-3-methanesulfonyl-5-(trifluoromethoxy)benzene

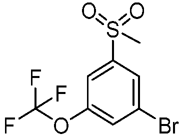

### Step 1: 1-bromo-3-(methylsulfanyl)-5-(trifluoromethoxy)benzene

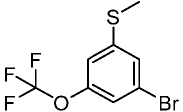

A mixture of 1,3-dibromo-5-(trifluoromethoxy)benzene (5.00 g, 15.63 mmol), 1,4-dioxane (50 mL), CH₃SNa (984.67 mg, 14.07 mmol), Pd₂(dba)₃ (715.62 mg, 0.78mmol) and XantPhos (904.37 mg, 1.56 mmol) was stirred for 2 h at 75 °C under nitrogen atmosphere. After cooled to room temperature, the solvent was removed and the residue was diluted with water. The resulting solution was extracted with of EA (150 mL) and the combined organic layers were washed with brine, dried over anhydrous sodium sulfate. The solvent was removed in vacuo and the residue was purified by combi-flash (EA/PE = 0-5%) to give the product (3.00 g, 67%). GCMS (ESI) m/e [M] 286.

### Step 2: 1-bromo-3-methanesulfonyl-5-(trifluoromethoxy)benzene

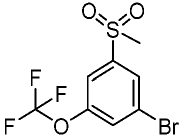

A mixture of 1-bromo-3-(methylsulfanyl)-5-(trifluoromethoxy)benzene (3.00 g, 10.45 mmol), RuCl₃.H₂O (235.58 mg, 1.05 mmol), NaIO₄ (6.71 g, 31.35 mmol), H₂O (30 mL) in THF (30 mL) was stirred for 2 h at RT. Upon completion of the reaction, the resulting solution was extracted with EA (100 mL x 3) and the organic layers combined. The resulting mixture was washed with brine, dried over anhydrous Na₂SO₄. The resulting mixture was concentrated under vacuum and the residue was purified by combi-flash (EA/PE = 0 - 15%) to give the product (2.31 g, 69%). ¹H NMR (300 MHz, DMSO-*d*₆) δ 8.05 (s, 1H), 7.75 (s, 1H), 7.70 (s, 1H), 3.10 (s, 3H). GCMS (ESI) m/e [M] 318.

### Example BB26: Synthesis of 2-bromo-4-[(2R)-2-[(tert-butyldimethylsilyl)oxy]propo xy]-6-methanesulfonylpyridine

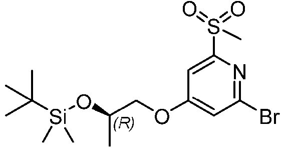

A mixture of 2-bromo-6-methanesulfonylpyridin-4-ol (1.00 g, 3.97 mmol), (2R)-2-[(tert-butyldimethylsilyl)oxy]propyl 4-methylbenzenesulfonate (2.73 g, 7.94 mmol) and Cs₂CO₃ (2.59 g, 7.90 mmol) in DMF (10 mL) was stirred at 100 °C overnight under nitrogen atmosphere. After cooled to room temperature, the solvent was removed and the residue was diluted with water. The resulting solution was extracted with EA (50 mL x 3) and the combined organic layers were washed with brine, dried over anhydrous sodium sulfate. The solvent was removed in vacuo and the residue was purified by Prep-TLC (PE/EA = 5:1) to give the product (644 mg, 82%). ¹H NMR (300 MHz, CDCl₃) δ 7.55 (s, 1H), 7.18 (s, 1H), 4.26 - 4.11 (m, 1H), 4.03 - 3.89 (m, 2H), 3.24 (s, 3H), 1.24 (d, *J =* 6.3 Hz, 3H), 0.88 (s, 9H), 0.11 (s, 3H), 0.08 (s, 3H). LCMS (ESI) m/e [M+1]⁺ 424.

### Example BB27: Synthesis of 7-bromo-2H,3H-[1,4]dioxino[2,3-c]pyridine

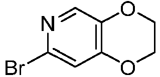

### Step1: 2-[(6-bromo-4-iodopyridin-3-yl) oxy] ethanol

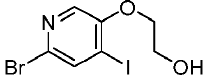

To a mixture of 2-bromo-5-fluoro-4-iodopyridine (11.00 g, 36.43 mmol) in NMP (135 mL) were added ethylene glycol (10.81 g, 174.16 mmol) and t-BuOK (4.50 g, 40.08 mmol) in NMP (30 mL) dropwise at 0 °C. The resulting solution was stirred for 1 h at 80 °C. After cooled to room temperature, the solvent was removed in vacuo and the residue was diluted with water (300 mL). The resulting solution was extracted with EA (200 mL x 3) and the combined organic layers were washed with brine, dried over anhydrous sodium sulfate. The solvent was removed in vacuo and the residue was purified by combi-flash (EA/PE = 0-41%) to give the product (12.00 g, 86%). LCMS (ESI, m/e) [M+1]⁺ 344.

### Step 2: 7-bromo-2H,3H-[1,4]dioxino[2,3-c]pyridine

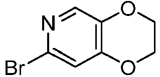

A mixture of 2-[(6-bromo-4-iodopyridin-3-yl) oxy] ethanol (12.00 g, 34.89 mmol), i-PrOH (180.00 mL), 3,4,7,8-tetramethyl-1,10-phenantholine (0.68 g, 2.88 mmol), CuI (0.41 g, 2.13 mmol) and t-BuOK (5.48 g, 48.85 mmol) was stirred for 1 h at 80 °C under nitrogen atmosphere. After cooled to room temperature, the solvent was removed in vacuo and the residue was diluted with water (20 mL). The resulting solution was extracted with EA (300 mL x 3) and the combined organic layers were washed with brine, dried over anhydrous sodium sulfate. The solvent was removed in vacuo and the residue was purified by combi-flash (EA/PE = 0-7%) to give the product (1.36 g, 16 %). ¹H NMR (300 MHz, DMSO-*d*₆) δ 7.95 (s, 1H), 7.19 (s, 1H), 4.44 - 4.24 (m, 4H). LCMS (ESI, m/e) [M+1]⁺ 216.

### Example BB29: Synthesis of 2-bromo-4-chloro-6-(methylsulfonyl)pyridine

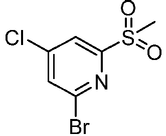

### Step 1: 2-bromo-6-(methylthio)pyridin-4-amine

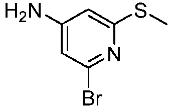

A mixture of 2,6-dibromopyridin-4-amine (5.00 g, 19.85 mmol), Cs₂CO₃ (9.70 g, 29.77 mmol) and MeSNa (1.53 g, 21.83 mmol) in DMSO (50 mL) was stirred for 12 h at 80 °C. After cooled to room temperature, water was added and the resulting mixture was extracted with EA (500 mL x 3). The combined organic layers were washed with brine, dried over anhydrous Na₂SO₄. After filtration, the filtrate was concentrated under vacuum and the residue was used in the next step directly without further purification. LCMS (ESI) m/e [M+1]⁺ 219.

### Step 2: 2-bromo-6-(methylsulfonyl)pyridin-4-amine

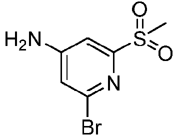

To a solution of 2-bromo-6-(methylsulfanyl)pyridin-4-amine (2.50 g, 11.41 mmol) in THF (25 mL) was added RuCl₃.H₂O (77.17 mg, 0.34 mmol), NaIO₄ (9.76 g, 45.64 mmol) and H₂O (25 mL) at 0 °C and the resulting mixture was stirred at room temperature for 1 h. Upon completion of the reaction, the resulting mixture was extracted with EA (300 mL x 3). The combined organic layers were dried over anhydrous Na₂SO₄. After filtration, the filtrate was concentrated under vacuum. The residue was purified by combi-flash (EA/PE = 1:1) to give the product (1.02 g, 35%). LCMS (ESI) m/e [M+1]⁺ 251.

### Step 3: 2-bromo-4-chloro-6-(methylsulfonyl)pyridine

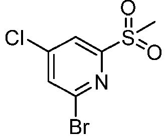

To a solution of 2-bromo-6-methanesulfonylpyridin-4-amine (800 mg, 3.18 mmol) in HCl (12M, 14.4 mL) was added NaNO₂ (1.10 g, 15.93 mmol) at 0 °C and the mixture was stirred for 1h at this temperature. Upon completion of the reaction, the resulting mixture was extracted with EA (100 mL x 3). The combined organic layers were dried over anhydrous Na₂SO₄. After filtration, the filtrate was concentrated under vacuum. The residue was purified by Prep-TLC (EA/PE = 1:4) to give the product (468 mg, 54%). ¹H NMR (300 MHz, DMSO-*d*₆) δ 8.33 (s, 1H), 8.17 (s, 1H), 3.33 (s, 3H). LCMS (ESI) m/e [M+1]⁺ 270.

### Example BB30: Synthesis of 1-bromo-3-fluoro-5-methanesulfonylbenzene

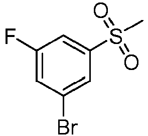

### Step 1: 1-bromo-3-fluoro-5-(methylsulfanyl)benzene

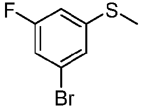

A mixture of 1-bromo-3,5-difluorobenzene (5.00 g, 25.91 mmol), (methylsulfanyl)sodium (1.82 g, 25.91 mmol) in DMF (50 mL) was stirred for 2 h at RT. Upon completion of the reaction, water was added and the resulting solution was extracted with EA (100 mL x 3) and the organic layers combined. The resulting mixture was washed with brine, dried over anhydrous Na₂SO₄. The solid were filtered out and the resulting mixture was concentrated under vacuum. The residue was purified by combi-flash (EA/PE = 0 - 2%) to give the product (4.80 g, 84%). GCMS (ESI) m/e [M] 220.

### Step 2: 1-bromo-3-fluoro-5-methanesulfonylbenzene

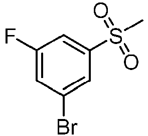

To a mixture of 1-bromo-3-fluoro-5-(methylsulfanyl)benzene (4.80 g, 21.71mmol) and THF (50 mL)/ H₂O (50 mL) were added RuCl₃.H₂O (489 mg, 2.17 mmol) and NaIO₄ (13.93 g, 65.13 mmol) in several batches at 0 °C. The resulting solution was stirred for 2 h at RT. Upon completion of the reaction, water was added and the resulting solution was extracted with EA (100 mL x 3) and the combined organic layers were washed with brine, dried over anhydrous sodium sulfate. The solvent was removed in vacuo and the residue was purified by combi-flash (EA/PE = 0-15%) to give the product (4.60 g, 80%). ¹H NMR (300 MHz, CDCl₃) δ 7.91 (s, 1H), 7.60 - 7.50 (m, 2 H), 3.13 (s, 3H). GCMS (ESI) m/e [M] 252.

### Example BB31: Synthesis of 1-bromo-3-chloro-5-(methylsulfonyl)benzene

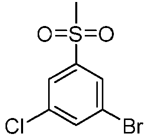

### Step 1: 1-bromo-3-chloro-5-(methylsulfanyl)benzene

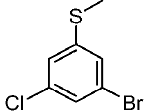

A mixture of 1,3-dibromo-5-chlorobenzene (10.00 g, 36.98 mmol), DMF (200 mL) and MeSNa (2.59 g, 37.00 mmol) was stirred for 1 h at RT. Upon completion of the reaction, the resulting solution was diluted with EA (200 mL) and the resulting mixture was washed with H₂O (200 mL x 3). The mixture was dried over anhydrous Na₂SO₄ and concentrated. The residue was purified by combi-flash (EA/PE = 0 - 15%) give the product (7.6 g, 87%). GCMS (ESI) m/e [M] 236.

### Step 2: 1-bromo-3-chloro-5-methanesulfonylbenzene

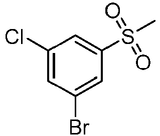

A mixture of 1-bromo-3-chloro-5-(methylsulfanyl)benzene (7.60 g, 31.99 mmol), THF (60 mL), H₂O (60 mL), NaIO₄ (27.37 g, 127.96 mmol) and RuCl₃.H₂O (1.44 g, 6.39 mmol) was stirred for 1 h at 0 °C. Upon completion of the reaction, the resulting solution was diluted with EA (200 mL) and the resulting mixture was washed with H₂O (200 mL x 3). The mixture was dried over anhydrous Na₂SO₄ and concentrated. The residue was purified by combi-flash (EA/PE = 0 - 30%) give the product (1.13 g, 13%). ¹H NMR (400 MHz, CDCl₃) δ 7.91 (s, 1H), 7.81 (s, 1H), 7.73 (s, 1H), 3.02 (s, 3H). GCMS (ESI) m/e [M] 268.

### Example BB32: Synthesis of 1-bromo-3-cyclopropoxy-5-methanesulfonylbenzene

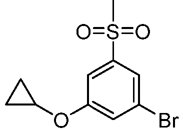

### Step 1: 1,3-dibromo-5-cyclopropoxybenzene

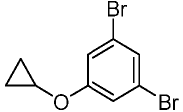

A mixture of 3,5-dibromophenol (5.00 g, 19.85 mmol), cyclopropyltrifluoro-lambda4-borane potassium (11.75 g, 79.40 mmol), K₂CO₃ (5.49 g, 39.70 mmol), 1,10-phenantholine (357.69 mg, 1.99 mmol) and Cu(OAc)₂ (360.5 mg, 1.985 mmol) in toluene (45 mL) and H₂O (15 mL) was stirred for overnight at 70 °C under nitrogen atmosphere. After cooled to room temperature, water was added and the resulting solution was extracted with EA (200 mL x 3) and the combined organic layers were washed with brine, dried over anhydrous Na₂SO₄. The solids were filtered out and the resulting mixture was concentrated under vacuum. The residue was purified by combi-flash (EA/PE = 0-5%) to give the product (1.00 g, 17%). GCMS (ESI) m/e [M] 290.

### Step 2: 1-bromo-3-cyclopropoxy-5-(methylsulfanyl)benzene

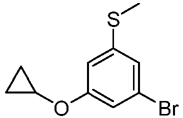

A mixture of 1,3-dibromo-5-cyclopropoxybenzene (1.00 g, 3.43 mmol), 1,4-dioxane (10 mL), CH₃SNa (191.8 mg, 2.74 mmol), Xantphos (396.35 mg, 0.69 mmol) and Pd₂(dba)₃ (313.6 mg, 0.34 mmol) was stirred for 2 h at 75 °C under nitrogen atmosphere. After cooled to room temperature, the solvent was removed in vacuo and the residue was diluted with 40 mL of water. The resulting solution was extracted with EA (60 mL x 3) and the combined organic layers were washed with brine, dried over anhydrous sodium sulfate. The solvent was removed in vacuo and the residue was purified by combi-flash (EA/PE = 0-5%) to give the product (300 mg, 34%). GCMS (ESI) m/e [M] 258.

### Step 3: 1-bromo-3-cyclopropoxy-5-methanesulfonylbenzene

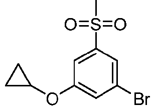

To a mixture of 1-bromo-3-cyclopropoxy-5-(methylsulfanyl)benzene (300 mg, 1.16 mmol) in THF (5 mL)/H₂O (5 mL) were added RuCl₃.H₂O (26 mg, 0.12 mmol) at 0 °C. Then NaIO₄ (742.79 mg, 3.47 mmol) was added in several batches at 0 °C. The resulting solution was stirred for 2 h at RT. Upon completion of the reaction, water was added and the resulting solution was extracted with EA (30 mL x 3) and the organic layers combined. The resulting mixture was washed with brine, dried over anhydrous Na₂SO₄. The resulting mixture was concentrated under vacuum and the residue was purified by Prep-TLC (EA/PE = 1:2) to give the product (165 mg, 49%). ¹H NMR (300 MHz, CDCl₃) δ 7.69 (s, 1H), 7.55 (s, 1H), 7.49 (s, 1H), 3.85 - 3.81 (m, 1H), 3.09 (s, 3H), 0.94 - 0.76 (m, 4H). GCMS (ESI) m/e [M] 290.

### Example BB33: Synthesis of of 2-bromo-3-isopropoxy-6-methanesulfonylpyridine

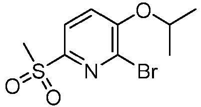

### Step1: 2-bromo-6-iodo-3-isopropoxypyridine

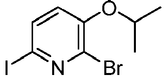

A mixture of 2-bromo-6-iodopyridin-3-ol (10.00 g, 33.34 mmol), DMF (150 mL), 2-iodopropane (13.04 g, 76.70 mmol) and Cs₂CO₃ (21.73 g, 66.69 mmol) was stirred for 3 h at 80 °C. After cooled to room temperature, water was added and the resulting solution was extracted with EA (100 mL x 3). The resulting mixture was concentrated under vacuum to give the product (11.08 g, 87%). LCMS (ESI, m/e) [M+1]⁺ 342.

### Step 2: 2-bromo-3-isopropoxy-6-(methylsulfanyl)pyridine

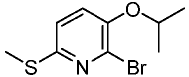

A mixture of 2-bromo-6-iodo-3-isopropoxypyridine (11.08 g, 32.40 mmol), 1,4-dioxane (166 mL), (methylsulfanyl)sodium (1.82 g, 25.97 mmol), Pd₂(dba)₃ (1.48 g, 1.62 mmol), Xantphos (1.87 g, 3.24 mmol) was stirred for 3 h at 75 °C nitrogen atmosphere. After cooled to room temperature, the solvent was removed in vacuo and the residue was diluted with water (20 mL). The resulting solution was extracted with EA (30 mL x 3) and the combined organic layers were washed with brine, dried over anhydrous sodium sulfate. The solvent was removed in vacuo and the residue was purified by combi-flash (EA/PE = 10: 1) to give the product (8.00 g, 84%). LCMS (ESI, m/e) [M+1]⁺ 262.

### Step 3: 2-bromo-3-isopropoxy-6-methanesulfonylpyridine

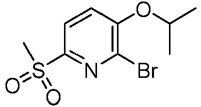

To a mixture of 2-bromo-3-isopropoxy-6-(methylsulfanyl)pyridine (5.00 g, 19.07 mmol) in THF (50.00 mL) was added NaIO₄ (12.24 g, 57.22 mmol) and RuCl₃ (0.13 g, 0.57 mmo) in H₂O (30 mL) dropwise at 0 °C and the resulting solution was stirred for 1 h at RT. Upon completion of the reaction, the resulting solution was extracted EA (30 mL x 3) and the combined organic layers were concentrated under vacuum. The residue was purified by combi-flash (EA/PE = 0-24%) to give the product (3.10 g, 50%). ¹H NMR (300 MHz, DMSO-*d*₆) δ 8.02 (d, *J =* 8.5 Hz, 1H), 7.79 (d, *J =* 8.5 Hz, 1H), 4.95 - 4.91 (m, 1H), 3.25 (s, 3H), 1.37 (d, *J =* 6.0 Hz, 6H). LCMS (ESI, m/e) [M+1]⁺ 294.

### Example BB34: Synthesis of 2-bromo-4-(cyclopropylmethoxy)-6-methanesulfonyl pyridine

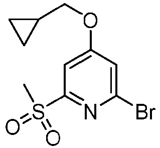

A mixture of 2-bromo-6-methanesulfonylpyridin-4-ol (1.00 g, 3.97 mmol), Cs₂CO₃ (2.59 g, 7.94 mmol) and (bromomethyl)cyclopropane (0.80 g, 5.95 mmol) in DMF (10 mL) was stirred for 5 h at 80 °C under nitrogen atmosphere. After cooled to room temperature, the solvent was removed in vacuo and the residue was diluted with 50 mL of water. The resulting solution was extracted with EA (30 mL x 3) and the combined organic layers were washed with brine, dried over anhydrous sodium sulfate. The solvent was removed in vacuo and the residue was purified by Prep-TLC (PE/EA = 5:1) to give the product (793 mg, 65%). ¹H NMR (300 MHz, CDCl₃) δ 7.54 (s, 1H), 7.16 (s, 1H), 3.94 (d, *J =* 7.1 Hz, 2H), 3.23 (s, 3H), 1.35 - 1.22 (m, 1H), 0.77- 0.65 (m, 2H), 0.43 - 0.34 (m, 2H). LCMS (ESI) m/e [M+1]⁺ 306.

### Example BB35: Synthesis of 1-bromo-3-(cyclopropylmethoxy)-5-(methylsulfonyl) benzene

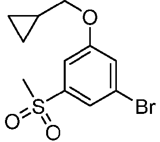

### Step 1: 1,3-dibromo-5-(cyclopropylmethoxy)benzene

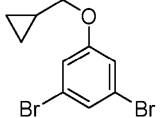

A mixture of 3,5-dibromophenol (5.00 g, 19.85 mmol), DMF (50 mL), (bromomethyl)cyclopropane (8.04 g, 59.55 mmol) and K₂CO₃ (5.49 g, 39.72 mmol) was stirred for 2 h at 80 °C. After cooled to room temperature, the solvent was removed in vacuo and the residue was diluted with water (100 mL). The resulting solution was extracted with EA (30 mL x 3) and the combined organic layers were washed with brine, dried over anhydrous sodium sulfate. The solvent was removed in vacuo and the residue was purified by combi-flash (EA/PE = 0-15%) give the product (2.80 g, 46%). GCMS (ESI) m/e [M+1]⁺ 304.

### Step 2: (3-bromo-5-(cyclopropylmethoxy)phenyl)(methyl)sulfane

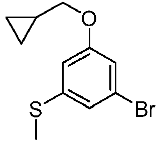

A mixture of 1,3-dibromo-5-(cyclopropylmethoxy)benzene (2.80 g, 9.15 mmol), 1,4-dioxane (20 mL), Pd₂(dba)₃ (418 mg, 0.46 mmol), Xantphos (529 mg, 0.915 mmol) and MeSNa (641 mg, 9.15 mmol) was stirred for 4 h at 80 °C. After cooled to room temperature, the solvent was removed in vacuo and the residue was diluted with water (20 mL). The resulting solution was extracted with EA (30 mL x 3) and the combined organic layers were washed with brine, dried over anhydrous sodium sulfate. The solvent was removed in vacuo and the residue was purified by combi-flash (EA/PE = 0-15%) give the product (2.0 g, 80%). LCMS (ESI) m/e [M+1]⁺ 272.

### Step 3: 1-bromo-3-(cyclopropylmethoxy)-5-(methylsulfonyl)benzene

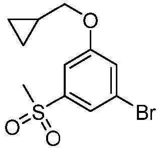

A mixture of 1-bromo-3-(cyclopropylmethoxy)-5-(methylsulfanyl)benzene (2.00 g, 7.32 mmol), NaIO₄ (6.26 g, 29.27 mmol), RuCl₃.H₂O (330 mg, 1.46 mmol) in THF (20 mL) and H₂O (20 mL) was stirred for 1 h at 0 °C. Upon completion of the reaction, EA (100 mL) was added and the resulting mixture was washed with water (100 mL x 3). The mixture was dried over anhydrous Na₂SO₄ and concentrated. The residue was purified by combi-flash (EA/PE = 0-20%) to give the product (1.07 g, 48%). ¹H NMR (300 MHz, DMSO-*d*₆) δ 7.61 (s, 1H), 7.52 (s, 1H), 7.42 (s, 1H), 3.96 (d, *J* = 7.1 Hz, 2H), 3.28 (s, 3H), 1.30 - 1.13 (m, 1H), 0.63 - 0.53 (m, 2H), 0.39 - 0.30 (m, 2H).

### Example BB36: Synthesis of 2-bromo-4-cyclobutoxy-6-(methylsulfonyl)pyridine

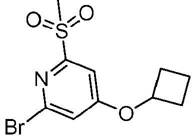

A solution of 2-bromo-6-methanesulfonylpyridin-4-ol (700 mg, 2.78 mmol), bromocyclobutane (749 mg, 5.55 mmol) and K₂CO₃ (767 mg, 5.55 mmol) in DMF (7 mL) was stirred for 8 h at 80 °C. After cooled to room temperature, water was added and the resulting mixture was extracted with EA (200 mL x 3). The combined organic layers were washed with brine (200 mL x 3), dried over anhydrous Na₂SO₄. After filtration, the filtrate was concentrated under vacuum. The residue was purified by Prep-TLC (EA/PE = 1:3) to give the product (382 mg, 45%). ¹H NMR (300 MHz, DMSO-*d*₆) δ 7.49 (s, 1H), 7.41 (s, 1H), 5.07 - 4.95 (m, 1H), 3.28 (s, 3H), 2.51 - 2.41 (m, 1H), 2.16 - 2.02 (m, 2H), 1.83 - 1.80 (m, 1H), 1.74 - 1.58 (m, 1H). LCMS (ESI) m/e [M+1]⁺ 306.

### Example BB37: Synthesis of 2-bromo-4-(cyclopentyloxy)-6-methanesulfonylpyridine

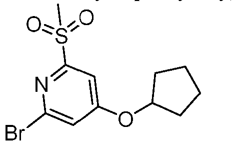

To a stirred solution of 2-bromo-6-methanesulfonylpyridin-4-ol (500 mg, 1.98 mmol), PPh₃ (780 mg, 2.97 mmol) and cyclopentanol (188 mg, 2.18 mmol) in THF (10 mL) was added DIAD (642 mg, 3.17 mmol) dropwise at 0 °C and the resulting mixture was stirred overnight at 50 °C. After cooled to room temperature, the solvent was removed in vacuo and the residue was diluted with water (20 mL). The resulting solution was extracted with EA (30 mL x 3) and the combined organic layers were washed with brine, dried over anhydrous sodium sulfate. The solvent was removed in vacuo and the residue was purified by Prep-TLC (PE/EA = 5:1) to give the product (300 mg, 47%). ¹H NMR (300 MHz, DMSO-*d*₆) δ 7.53 (s, 1H), 7.46 (s, 1H), 5.14 - 5.10 (m, 1H), 3.27 (s, 3H), 2.04 - 1.89 (m, 3H), 1.82 - 1.51 (m, 5H). LCMS (ESI) m/e [M+1]⁺ 320.

### Example BB38: Synthesis of 2-bromo-6-methanesulfonyl-4-(oxolan-3-yloxy)pyridine

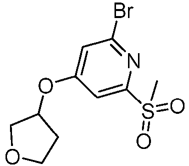

To a stirred solution of 2-bromo-6-methanesulfonylpyridin-4-ol (400 mg, 1.59 mmol) and 3-hydroxytetrahydrofuran (154 mg, 1.75 mmol) in THF were added PPh₃ (832 mg, 3.17 mmol) and DIAD (642 mg, 3.17 mmol) dropwise portions at 0 °C. The resulting mixture was stirred overnight at RT. Upon completion of the reaction, water was added and the resulting mixture was extracted with EA (50 mL x 3). The combined organic layers were washed with brine (30 mL x 3), dried over anhydrous Na₂SO₄, concentrated under vacuum. The residue was purified by Prep-TLC (PE/EA = 1:1) to give the product (250 mg, 47%). ¹H NMR (300 MHz, DMSO-*d*₆) δ 7.60 (s, 1H), 7.51 (s, 1H), 5.38 - 5.34 (m, 1H), 3.90 - 3.82 (m, 3H), 3.82 - 3.72 (m, 1H), 3.28 (s, 3H), 2.29 - 2.25 (m, 1H), 2.00 - 1.95 (m, 1H). LCMS (ESI) m/e [M+1]⁺ 322.

### Example BB39: Synthesis of 2-bromo-4-(1-methoxyethyl)-6-(methylsulfonyl)pyridine

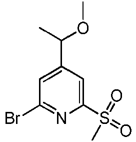

### Step 1: 2,6-dibromo-N-methoxy-N-methylisonicotinamide

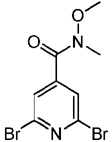

A mixture of 2,6-dibromoisonicotinic acid (9 g, 32.0 mmol) and CDI (5.7 g, 35.0 mmol) in DCM (100 mL) was stirred at 20 °C for 2 h, N,O-dimethylhydroxylamine hydrochloride (3.4 g, 35.0 mmol) was added and stirred at 20 °C for 12 h. The mixture was diluted with H₂O (100 mL) and extracted with DCM (100 mL x 3). The combined organic layers were dried over Na₂SO₄ and concentrated to give the product (7.3 g, 70%). ¹H NMR (400 MHz, CDCl₃) δ 7.68 (s, 2H), 3.58 (s, 3H), 3.37 (s, 3H). MS (ESI) m/e [M+1]⁺ 324.

### Step 2: 1-(2,6-dibromopyridin-4-yl)ethanone

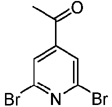

A mixture of 2,6-dibromo-N-methoxy-N-methylisonicotinamide (4.0 g, 12.3 mmol) in THF (40 mL) was added MgBrCH₃ (41 mL, 123 mmol, 3M) dropwise at 0 °C. The mixture was stirred at 20 °C for 2 h. The mixture was diluted with H₂O (50 ml) and extracted with EA (50 mL x 3). The combined organic layers were dried over Na₂SO₄ and concentrated to give the product (3.2 g, 93%). ¹H NMR (400 MHz, CDCl₃) δ 7.87 (s, 2H), 2.61 (s, 3H). MS (ESI) m/e [M+1]⁺ 278.

### Step 3: 1-(2,6-dibromopyridin-4-yl)ethanol

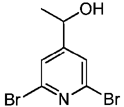

A solution of 1-(2,6-dibromopyridin-4-yl)ethanone (3.2 g, 11.5 mmol) in MeOH (30 mL) at 0 °C, was added NaBH₄ (434 mg, 11.5 mmol). The mixture was stirred at 0 °C for 30 mins. The mixture was diluted with H₂O (50 ml) and extracted with EA (50 mL x 3). The combined organic layers were dried over Na₂SO₄ and concentrated to give the product (3.2 g, 99%). MS (ESI) m/e [M+1]⁺ 282.

### Step 4: 2,6-dibromo-4-(1-methoxyethyl)pyridine

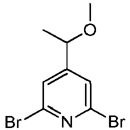

A solution of 1-(2,6-dibromopyridin-4-yl)ethanol (3.2 g, 11.4 mmol) in THF (50 mL) at 0 °C, was added NaH (60% in mineral oil , 592 mg, 14.8 mmol). The mixture was stirred at 0 °C for 20 min. MeI (4.9 g, 34.2 mmol) was added and stirred at 20 °C for 2 h. The mixture was diluted with sat. NH₄Cl (50 ml) and extracted with EA (50 mL x 3). The combined organic layers were dried over Na₂SO₄ and concentrated to give the crude product (3.2 g, crude) , used directly. MS (ESI) m/e [M+1]⁺ 294.

### Step 5: 2-bromo-4-(1-methoxyethyl)-6-(methylthio)pyridine

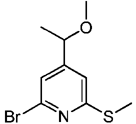

A solution of 2,6-dibromo-4-(1-methoxyethyl)pyridine (1.5 g, 5.1 mmol) in DMF (20 mL) at 20 °C, was added NaSCH₃ (392 mg, 5.6 mmol). The mixture was stirred at 20 °C for 1 h. The mixture was diluted with brine (20 mL) and extracted with EA (20 mL x 3). The combined organic layers were dried over Na₂SO₄ and concentrated to give the product (1.5 g, crude) , used directly. MS (ESI) m/e [M+1]⁺ 262.0.

### Step 6: 2-bromo-4-(1-methoxyethyl)-6-(methylsulfonyl)pyridine

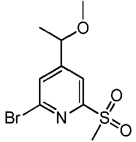

A mixture of 2-bromo-4-(1-methoxyethyl)-6-(methylthio)pyridine (1.5 g, 5.1 mmol) and oxone (12.5 g, 20.4 mmol) in MeOH (16 mL) and H₂O (8 ml) was stirred at 20 °C for 12 h. The mixture was filtered, added brine (20 ml) and extracted with EA (20 mL x 3). The combined organic layers were dried over Na₂SO₄ and concentrated. The crude was purified by column chomatography (PE/EA = 20:1 - 3:1) to give the product (1.1 g, 65%). ¹H NMR (400 MHz, CDCl₃) δ 7.97 (s, 1H), 7.70 (s, 1H), 4.39 - 4.35 (m, 1H), 3.33 (s, 3H), 3.28 (s, 3H), 1.45 (d, *J* = 6.4 Hz, 3H). MS (ESI) m/e [M+1]⁺ 294.

### Example BB40: Synthesis of 2-bromo-4-[(2S)-butan-2-yloxy]-6-methanesulfonyl pyridine

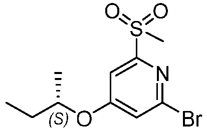

To a stirred solution of 2-bromo-6-methanesulfonylpyridin-4-ol (400 mg, 1.59 mmol), (S)-2-butanol (176 mg, 2.38 mmol) and PPh₃ (624 mg, 2.38 mmol) in THF (10 mL) was added DIAD (513 mg, 2.54 mmol) dropwise at 0 °C under air atmosphere. The resulting mixture was stirred overnight at 50 °C. The resulting mixture was extracted with EA (100 mL x 3). The combined organic layers were washed with brine (100 mL), dried over anhydrous Na₂SO₄. After filtration, the filtrate was concentrated and purified by silica gel column chomatography (PE/EA = 8:1) to give 2-bromo-4-[(2S)-butan-2-yloxy]-6-methanesulfonylpyridine (358 mg, 73% yield). ¹H NMR (400 MHz, DMSO-*d*₆) δ 7.58 (s, 1H), 7.48 (s, 1H), 4.86 - 4.71 (m, 1H), 3.27 (s, 3H), 1.77 - 1.52 (m, 2H), 1.27 (d, *J =* 6.1 Hz, 3H), 0.91 (t, *J =* 7.4 Hz, 3H). LCMS (ESI) m/e [M+1]⁺ 308.

### Example BB41: Synthesis of 2-bromo-4-(difluoromethoxy)-6-methanesulfonylpyridine

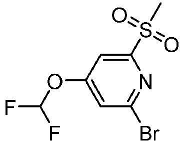

A mixture solution of KOH (1.11 g, 19.78 mmol), CH₃CN (5 mL) and H₂O (5 mL) was cooled to approximately -10 °C. 2-bromo-6-methanesulfonylpyridin-4-ol (500 mg, 1.98 mmol) was added dropwise followed by diethyl bromodifluoromethylphosphonate (1.06 g, 3.97 mmol) over 15 mins. The mixture was allowed to warm to RT over 1h. The mixture was poured into water and extracted with EA (50 mL x 3), washed with brine and dried over Na₂SO₄. The residue was purified by Prep-TLC (EA/PE = 1: 10) to give 2-bromo-4-(difluoromethoxy)-6-methanesulfonyl pyridine (310 mg, 52% yield). ¹H NMR (400 MHz, DMSO-*d*₆) δ 7.94 (*t, J =* 1.8 Hz, 1H), 7.88 - 7.50 (m, 2H), 3.34 (s, 3H). LCMS (ESI) m/e [M+1]⁺ 301.

### Example BB42: Synthesis of 2-bromo-4-ethoxy-6-methanesulfonylpyridine

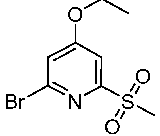

A mixture solution of 2-bromo-6-methanesulfonylpyridin-4-ol (3.50 g, 13.88 mmol), Cs₂CO₃ (9.10 g, 27.84 mmol) and ethyl iodide (3.26 g, 20.90 mmol) in DMF (40 mL) was stirred for 1 h at 80 °C. The mixture was allowed to cool down to rt. The resulting mixture was extracted with EA (50 mL x 3). The combined organic layers were washed with brine (50 mL x 2), dried over anhydrous Na₂SO₄. After filtration, the filtrate was concentrated under reduced pressure. The residue was purified by combi-flash (EA/PE = 20-25%) to give 2-bromo-4-ethoxy-6-methanesulfonylpyridine (3.05 g, 78% yield). 1H NMR (300 MHz, CDCl₃) δ 7.55 (d, *J=* 2.2 Hz, 1H), 7.17 (d, *J* = 2.2 Hz, 1H), 4.19 (m, 2H), 3.25 (s, 3H), 1.49 (t, *J= 7.0* Hz, 3H). LCMS (ESI) m/e [M+1]⁺ 281.

### Example BB43: Synthesis of [[(2R)-1-(3-bromo-5-methanesulfonylphenoxy)propan-2-yl]oxy](tert-butyl)dimethylsilane

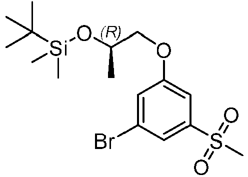

### Step 1: (2R)-1-[(4-methylbenzenesulfonyl)oxy]propan-2-ol

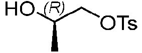

To a stirred solution of R-1, 2-propanediol (5.00 g, 65.71 mmol) in Py (50 mL) was added TsCl (13.78 g, 72.28 mmol) in portions at 0 °C. The resulting mixture was stirred overnight at RT. The resulting mixture was extracted with EA (100 mL x 3). The combined organic layers were washed with brine (100 mL x 3), dried over anhydrous Na₂SO₄. After filtration, the filtrate was concentrated and purified by combi-flash (EA/PE = 28-32%) to give (2R)-1-[(4-methylbenzenesulfonyl)oxy]propan-2-ol (10.0 g, 66% yield). LCMS (ESI) m/e [M+1]⁺ 230.

### Step 2: (2R)-2-[(tert-butyldimethylsilyl)oxy]propyl 4-methylbenzenesulfonate

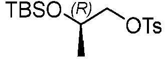

To a stirred mixture of (2R)-1-[(4-methylbenzenesulfonyl)oxy]propan-2-ol (10 g, 43.42 mmol), Imidazole (8.87 g, 130.28 mmol) in DCM (100 mL) was added TBSCl (19.64 g, 130.28 mmol) in portions at 0 °C. The resulting mixture was stirred for 1 h at RT. The resulting mixture was concentrated under reduced pressure. The residue was purified by combi-flash (EA/PE = 3 - 5%) to give the crude product (15.00 g, 100% yield). LCMS (ESI) m/e [M+1]⁺ 344.

### Step 3: tert-butyl([[(2R)-1-(3,5-dibromophenoxy)propan-2-yl]oxy])dimethylsilane

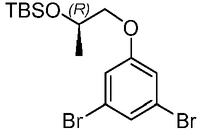

A mixture of 3,5-dibromophenol (2.0 g, 7.94 mmol) and (2R)-2-[(tert-butyldimethylsilyl)oxy]propyl 4-methylbenzenesulfonate (5.46 g, 15.85 mmol) and Cs₂CO₃ (5.17 g, 15.86 mmol) in DMF (20 mL) was stirred for one night at 100 °C. The mixture was allowed to cool down to RT. The resulting mixture was extracted with EA (40 mL x 3). The combined organic layers were washed with brine (40 mL x 2), dried over anhydrous Na₂SO₄. After filtration, the filtrate was concentrated under reduced pressure. The residue was purified by combi-flash (EA/PE = 3 - 5%) to give tert-butyl([[(2R)-1-(3,5-dibromophenoxy)propan-2-yl]oxy])dimeth ylsilane (1.90 g, 56% yield).

### Step 4: [[(2R)-1-[3-bromo-5-(methylsulfanyl)phenoxy]propan-2-yl]oxy](tert-butyl) dimethylsilane

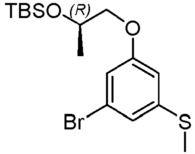

A mixture of tert-butyl([[(2R)-1-(3,5-dibromophenoxy)propan-2-yl]oxyl)dimethylsilane (1.00 g, 2.36 mmol), (methylsulfanyl)sodium (166 mg, 2.37 mmol), DIEA (0.82 mL, 4.71 mmol), Pd₂(dba)₃ (108 mg, 0.12 mmol) and Xantphos (137 mg, 0.24 mmol) in dioxane (10 mL) was stirred for 4 h at 70 °C under N₂ atmosphere. The mixture was allowed to cool down to RT. The resulting mixture was extracted with EA (40 mL x 3). The combined organic layers were washed with brine (30 mL x 3), dried over anhydrous Na₂SO₄. After filtration, the filtrate was concentrated under reduced pressure. The residue was purified by Prep-TLC (PE/EA =20:1) to give [[(2R)-1-[3-bromo-5-(methylsulfanyl)phenoxy]propan-2-yl]oxy](tert-butyl)dime thylsilane (470 mg, 50% yield).

### Step 5: [[(2R)-1-(3-bromo-5-methanesulfonylphenoxy)propan-2-yl]oxy](tert-butyl) dimethylsilane

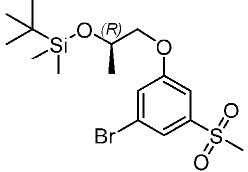

To a stirred solution of [[(2R)-1-[3-bromo-5-(methylsulfanyl)phenoxy]propan-2-yl]oxy](tert-butyl)dimethylsilane (470 mg, 1.20 mmol) in THF (30 mL) were added RuCl₃•H₂O (7.5 mg, 0.04 mmol) and NaIO₄ (513.6 mg, 2.40 mmol) in H₂O (20 mL) at 0 °C and the resulting solution was stirred for 1 h at 0 °C. Then the mixture was extracted with EA (30 mL x 3). The combined organic layers were washed with brine (30 mL x 3), dried over Na₂SO₄. After filtration, the filtrate was concentrated and purified by Prep-TLC (PE/EA = 10:1) to give [[(2R)-1-(3-bromo-5-methanesulfonyl phenoxy)propan-2-yl]oxy](tert-butyl)dimethylsilane (344.2 mg, 67% yield). ¹H NMR (400 MHz, CDCl₃) δ 7.66 (s, 1H), 7.38 (s, 1H), 7.33 (s, 1H), 4.23 - 4.15 (m, 1H), 3.96 - 3.81 (m, 2H), 3.08 (s, 3H), 1.29 - 1.23 (d, *J =* 8.0Hz, 3H), 0.91 (s, 9H), 0.10 (s, 6H). LCMS (ESI) m/e [M+1]⁺ 422.

### Example BB44: Synthesis of 2-bromo-6-(ethanesulfonyl)-4-isopropoxypyridine

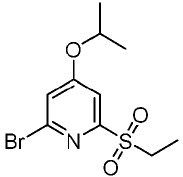

### Step 1: 2-bromo-6-(ethylsulfanyl)pyridin-4-ol

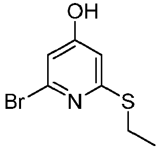

A stirred mixture of 2, 6-dibromopyridin-4-ol (5.00 g, 19.77 mmol), EtSNa (1.66 g, 19.77 mmol), Pd₂(dba)₃ (181 mg, 0.20 mmol), Xantphos (114.4 mg, 0.20 mmol) and DIEA (6.9 mL, 53.29 mmol) in dioxane (50 mL) was stirred for 3 h at 80 °C. The mixture was allowed to cool down to RT. The resulting mixture was extracted with EA (50 mL x 3). The combined organic layers were washed with brine (50 mL x 2), dried over anhydrous Na₂SO₄. After filtration, the filtrate was concentrated under reduced pressure to give 2-bromo-6-(ethylsulfanyl)pyridin-4-ol as a crude product, and used in the next step directly without further purification. LCMS (ESI) m/e [M+1]⁺ 234.

### Step 2: 2-bromo-6-(ethanesulfonyl)pyridin-4-ol

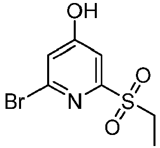

To a stirred mixture of 2-bromo-6-(ethylsulfanyl)pyridin-4-ol (5.00 g, 21.36 mmol) in THF was added RuCl₃•H₂O (133 mg, 0.64 mmol) in water for a moment and NaIO₄ (9.14 g, 42.72 mmol) was added in THF and water in portions at 0 °C for 30 mins. The resulting mixture was extracted with EA (100 mL x 3). The combined organic layers were washed with brine (2 x 50 mL), dried over anhydrous Na₂SO₄. After filtration, the filtrate was concentrated under reduced pressure. The residue was purified by combi-flash (EA/PE = 28-32%) to 2-bromo-6-(ethanesulfonyl)pyridin-4-ol (2.00 g, 35% yield). LCMS (ESI) m/e [M+1]⁺ 266.

### Step 3: 2-bromo-6-(ethanesulfonyl)-4-isopropoxypyridine

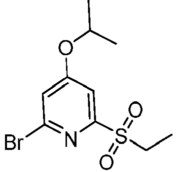

A stirred solution of 2-bromo-6-(ethanesulfonyl)pyridin-4-ol (1.50 g, 5.64 mmol), Cs₂CO₃ (3.68 g. 11.27 mmol) and 2-iodopropane (1.44 g. 8.46 mmol) in dimethylformamide (10 mL) at 80 °C for 1 h. The mixture was allowed to cool down to rt. The resulting mixture was extracted with EA (40 mL x 3). The combined organic layers were washed with brine (30 mL x 2), dried over anhydrous Na₂SO₄. After filtration, the filtrate was concentrated under reduced pressure. The residue was purified by combi-flash (EA/PE = 28-32%) to give 2-bromo-6-(ethanesulfonyl)-4-isopropoxypyridine (1.40 g, 78% yield). ¹H NMR (400 MHz, CDCl₃) δ 7.53 (s, 1H), 7.13 (s, 1H), 4.75 - 4.70 (m, 1H), 3.46 - 3.42 (m, 2H), 1.41 (d*, J =* 6.1 Hz, 6H), 1.35 - 1.32 (m, 3H). LCMS (ESI) m/e [M+1]⁺ 307.

### Example BB45: Synthesis of [[(2S)-1-(3-bromo-5-methanesulfonylphenoxy)propan-2-yl]oxy](tert-butyl)dimethylsilane

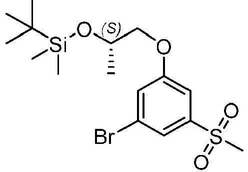

### Step 1: (2S)-1-[(4-methylbenzenesulfonyl)oxy]propan-2-ol

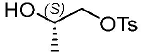

To a stirred solution of (S)-1, 2-propanediol (5.00 g, 65.71 mmol) in Py (50 mL) was added TsCl (13.78 g, 72.28 mmol) in portions at 0 °C. The resulting mixture was stirred overnight at RT. The resulting mixture was extracted with EA (100 mL x 3). The combined organic layers were washed with brine (100 x 3 mL), dried over anhydrous Na₂SO₄, concentrated under reduced pressure. The residue was purified by combi-flash (EA/PE = 28 - 32%) to give (2S)-1-[(4-methylbenzenesulfonyl)oxy]propan-2-ol (10.00 g, 66% yield). LCMS (ESI) m/e [M+1]⁺ 230.

### Step 2: (2S)-2-[(tert-butyldimethylsilyl)oxy]propyl 4-methylbenzenesulfonate

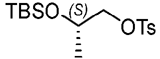

To a stirred mixture of (2S)-1-[(4-methylbenzenesulfonyl)oxy]propan-2-ol (10.00 g, 43.42 mmol), Imidazole (8.87 g, 130.28 mmol) and in DCM (100 mL) was added TBSCl (19.64 g, 130.28 mmol) in portions at 0 °C. The resulting mixture was stirred for 1 h at RT. The mixture was concentrated and purified by combi-flash (EA/PE = 3-5%) to give (2S)-2-[(tert-butyldimethylsilyl)oxy] propyl-4-methylbenzenesulfonate (15.00g, 100% yield). LCMS (ESI) m/e [M+1]⁺ 344.

### Step 3: tert-butyl([[(2S)-1-(3,5-dibromophenoxy)propan-2-yl[oxy])dimethylsilane

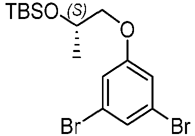

A mixture of 3, 5-dibromophenol (2.0 g, 7.94 mmol) and (2S)-2-[(tertbutyldimethylsilyl)oxy]propyl 4-methylbenzenesulfonate (5.46 g, 15.85 mmol) and Cs₂CO₃ (5.17 g, 15.86 mmol) in DMF (20 mL) was stirred overnight at 100 °C. The mixture was allowed to cool down to RT. The resulting mixture was extracted with EA (40 mL x 3). The combined organic layers were washed with brine (40 mL x 2), dried over anhydrous Na₂SO₄. After filtration, the filtrate was concentrated and purified by combi-flash (EA/PE = 3-5%) to give tert-butyl([[(2S)-1-(3,5-dibromophenoxy)propan-2-yl]oxy])dimethylsilane (1.90 g, 56% yield).

### Step 4: [[(2S)-1-[3-bromo-5-(methylsulfanyl)phenoxy]propan-2-yl]oxy](tertbutyl)dimethylsilane

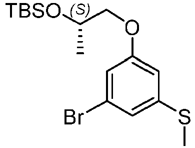

A mixture of tert-butyl([[(2S)-1-(3,5-dibromophenoxy)propan-2-yl]oxy])dimethylsilane (2.00 g, 4.72 mmol), (methylsulfanyl)sodium (332.00 mg, 4.74 mmol), DIEA (1.64 mL, 3.48 mmol), Pd₂(dba)₃ (216.00 mg, 0.24 mmol) and Xantphos (274.00 mg, 0.48 mmol) in dioxane (20 mL) was stirred for 4 h at 75 °C under N₂ atmosphere. The mixture was allowed to cool down to RT. The resulting mixture was extracted with EA (100 mL x 3). The combined organic layers were washed with brine (100 mL x 3), dried over anhydrous Na₂SO₄, concentrated to give the reside and purified by Prep-TLC (PE/EA = 20:1) to give [[(2S)-1-[3-bromo-5-(methylsulfanyl)phenoxy]propan-2-yl]oxy](tert-butyl)dimethylsilane (940 mg, 50% yield).

### Step 5: [[(2S)-1-(3-bromo-5-methanesulfonylphenoxy)propan-2-yl]oxy](tert-butyl)dimethylsilane

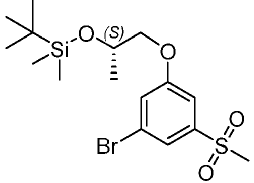

To a stirred solution of [[(2S)-1-[3-bromo-5-(methylsulfanyl)phenoxy]propan-2-yl]oxy](tert-butyl)dimethylsilane (940 mg, 2.40 mmol) in THF (10 mL) were added RuCl₃•H₂O (15 mg, 0.08 mmol) and NaIO₄ (1.03 g, 4.80 mmol) in H₂O (10 mL) at 0 °C and the resulting solution was stirred for 1 h at 0 °C. The resulting mixture was extracted with EA (100 mL x 3). The combined organic layers were washed with brine (100 mL x 3), dried over anhydrous Na₂SO₄, concentrated and purified by Prep-TLC (PE/EA = 10:1) to give [[(2S)-1-(3-bromo-5-methanesulfonylphenoxy)propan-2-yl]oxy](tert-butyl)dimethyl silane (646.4 mg, 63% yield). ¹H NMR (400 MHz, CDCl₃) δ 7.58 (m, 1H), 7.34 (m, 1H), 7.31 (m, 1H), 4.24 - 4.12 (m, 1H), 3.89 - 3.72 (m, 2H), 3.06 (s, 3H), 1.29 - 1.23 (d, *J=* 8.0 Hz, 3H), 0.91 (s, 9H), 0.10 (s, 6H). LCMS (ESI) m/e [M+1]⁺ 422.

### Example BB46: Synthesis of 2-bromo-4-isobutoxy-6-(methylsulfonyl)pyridine

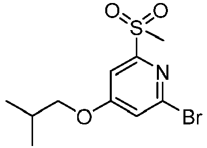

A solution of 2-bromo-6-methanesulfonylpyridin-4-ol (500 mg, 1.98 mmol), isobutyl bromide (815 mg, 5.95 mmol) and K₂CO₃ (548 mg, 3.97 mmol) in DMF (5 mL) was stirred for 2 h at 80 °C. The resulting mixture was extracted with EA (100 mL x 3). The combined organic layers were washed with brine (100 mL x 2), dried over anhydrous Na₂SO₄. After filtration, the filtrate was concentrated under reduced pressure. The residue was purified by Prep-TLC (EA/PE = 1:5) to give 2-bromo-4-isobutoxy-6-(methylsulfonyl)pyridine (547 mg, 89% yield). ¹H NMR (400 MHz, DMSO-*d₆*) δ 7.61 (s, 1H), 7.54 (s, 1H), 4.02 (d, *J* = 6.5 Hz, 2H), 3.29 (s, 3H), 2.06 - 2.02 (m, 1H), 0.98 (d, *J* = 6.7 Hz, 6H). LCMS (ESI) m/e [M+1]⁺ 309.

### Example BB48: Synthesis of 2-bromo-3-(difluoromethoxy)-6-(methylsulfonyl)pyridine

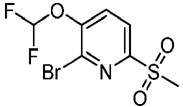

### Step 1: 2-bromo-3-(difluoromethoxy)-6-iodopyridine

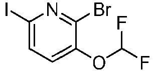

A solution of 2-bromo-6-iodopyridin-3-ol (5.00 g, 16.67 mmol), sodium 2-chloro-2,2-difluoroacetate (5.08 g, 33.34 mmol) and Cs₂CO₃ (10.86 g, 33.34 mmol) in DMF (50 mL) was stirred for 4 h at 80 °C. The resulting mixture was extracted with EA (200 mL x 3). The combined organic layers were washed with brine (200 mL x 3), dried over anhydrous Na₂SO₄. After filtration, the filtrate was concentrated under reduced pressure to give the crude product 2-bromo-3-(difluoromethoxy)-6-iodopyridine (5.00 g) and used in the next step directly without further purification. LCMS (ESI) m/e [M]⁺ 350.

### Step 2: 2-bromo-3-(difluoromethoxy)-6-(methylsulfanyl)pyridine

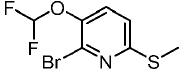

To a solution of 2-bromo-3-(difluoromethoxy)-6-iodopyridine (2.00 g, 5.72 mmol) and CH₃SNa (320 mg, 4.57 mmol) in dioxane (20 mL) were added Xantphos (331 mg, 0.57 mmol) and Pd₂(dba)₃ (523 mg, 0.57 mmol). After stirring for 4 h at 70 °C under a nitrogen atmosphere, the resulting mixture was extracted with EA (200 mL x 3). The combined organic layers were dried over anhydrous Na₂SO₄. After filtration, the filtrate was concentrated to give the crude product 2-bromo-3-(difluoromethoxy)-6-(methylsulfanyl)pyridine (2.0 g) and used in the next step directly without further purification. LCMS (ESI) m/e [M]⁺ 270.

### Step 3: 2-bromo-3-(difluoromethoxy)-6-methanesulfonylpyridine

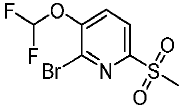

To a solution of 2-bromo-3-(difluoromethoxy)-6-(methylsulfanyl)pyridine (2.00 g, 7.41 mmol) in THF (20 mL) was added RuCl₃•H₂O (50 mg, 0.22 mmol) at 0 °C. NaIO₄ (6.34 g, 29.62 mmol) and H₂O (20 mL) was added and the mixture was allowed to warm to RT and stirred for 1 h. The resulting mixture was extracted with EA (200 mL x 3). The combined organic layers were washed with brine (200 mL x 3), dried over anhydrous Na₂SO₄. After filtration, the filtrate was concentrated under reduced pressure. The residue was purified by Prep-TLC (EA/PE = 1:3) to give 2-bromo-3-(difluoromethoxy)-6-methanesulfonylpyridine (556.8 mg, 25% yield). ¹H NMR (400 MHz, DMSO-*d₆*) δ 8.17 (d, *J* = 8.4 Hz, 4H), 8.08 - 8.01 (m, 1H), 7.71-7.35 (m 1H), 3.32 (s, 3H). LCMS (ESI) m/e [M]⁺ 301.

### Example BB49: Synthesis of 5-bromo-7-methanesulfonyl-2H,3H- [1,4]dioxino[2,3-c] pyridine

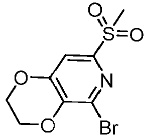

### Step 1: 2-bromo-6-iodo-3-methoxypyridine

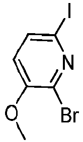

A mixture of 2-bromo-6-iodopyridin-3-ol (11.00 g, 36.68 mmol), CH₃I (10.41 g, 73.34 mmol), K₂CO₃ (10.14 g, 73.35 mmol) in DMF (165 mL) was stirred for 1 h at RT. The resulting solution was added to H₂O (200 mL) and extracted with EA (100 mL x 3), The combined organic layers were washed with brine (200 mL x 3), dried over anhydrous Na₂SO₄, concentrated to give the residue and purified by combi-flash (EA/PE = 0 - 6 %) to give 2-bromo-6-iodo-3-methoxypyridine (11.00 g, 85% yield). LCMS (ESI) m/e [M+1]⁺ 314.

### Step 2: 2-bromo-6-iodo-3-methoxypyridin-4-ol

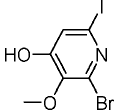

Into a 250-mL 3-necked round-bottom flask purged and maintained with an inert atmosphere of nitrogen, were placed 2-bromo-6-iodo-3-methoxypyridine (5.00 g, 15.92 mmol), THF (100 mL). This was followed by the addition of LDA (2.0 M, 9.6 mL, 19.11 mmol) dropwise with stirring at -78 °C. The resulting solution was stirred for 1 h at -78 °C. To the mixture was added trimethyl borate (2.48 g, 23.86 mmol) dropwise with stirring at -78°C. The resulting solution was stirred for 2 h at -78 °C. To the mixture was added H₂O₂ (0.15 mL) dropwise with stirring at -60°C. The resulting solution was allowed to react, with stirring, for an additional 1 h at RT. The reaction was then quenched by the addition of Na₂S₂O₃. The resulting solution was extracted with EA (50 mL x 3), dried over anhydrous Na₂SO₄, concentrated under reduced pressure to give the residue and purified by combi-flash (EA/PE = 0 - 36%) to give 2-bromo-6-iodo-3-methoxypyridin-4-ol (1.92 g, 32% yield). LCMS (ESI) m/e [M+1]⁺ 330.

### Step 3: 2-bromo-6-iodopyridine-3,4-diol

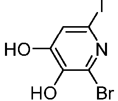

To a solution of 2-bromo-6-iodo-3-methoxypyridin-4-ol (1.92 g, 5.82 mmol) in DCM (35 mL) was followed by the addition of BBr₃ (4.37 g, 17.45 mmol) dropwise with stirring at 0 °C. The resulting solution was stirred for 3 h at rt. The reaction was then quenched by the addition of MeOH (5 mL). The resulting mixture was concentrated under vacuum and purified by combi-flash (EA/PE = 0 - 8%) to give 2-bromo-6-iodopyridine-3,4-diol (2.12 g crude). LCMS (ESI) m/e [M+1]⁺ 316.

### Step 4: 5-bromo-7-iodo-2H,3H- [1,4]dioxino[2,3-c] pyridine

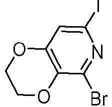

A mixture of 2-bromo-6-iodopyridine-3,4-diol (2.12 g, 6.71 mmol), 1,2-dibromoethane (1.89 g, 10.06 mmol), K₂CO₃ (4.64 g, 33.55 mmol) in DMF (40 mL) was stirred for 2 h at 90 °C. The resulting solution was added to H₂O (100 mL) and extracted with EA (50 mL x 3), dried over anhydrous Na₂SO₄, concentrated to give 5-bromo-7-iodo-2H,3H-[1,4]dioxino[2,3-c] pyridine (1.26 g, 49% yield). LCMS (ESI) m/e [M+1]⁺ 342.

### Step 5: 5-bromo-7-(methylsulfanyl)-2H,3H- [1,4]dioxino[2,3-c] pyridine

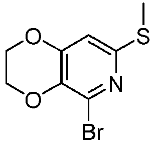

A mixture of 5-bromo-7-iodo-2H,3H- [1,4]dioxino[2,3-c] pyridine (1.26 g, 3.68 mmol), (methylsulfanyl)sodium (0.23 g, 3.28 mmol), Pd₂(dba)₃ (0.17 g, 0.18 mmol), Xantphos (0.21 g, 0.36 mmol) in dioxane (20 mL) was stirred for 3 h at 75 °C. The resulting mixture was filtrated and filtrate was concentrated under vacuum. The residue was purified by combi-flash (EA / PE = 0-5%) to give 5-bromo-7-(methylsulfanyl)-2H,3H- [1,4]dioxino[2,3-c] pyridine (492mg, 45% yield). LCMS (ESI) m/e [M+1]⁺ 262.

### Step 6: 5-bromo-7-methanesulfonyl-2H,3H- [1,4]dioxino[2,3-c] pyridine

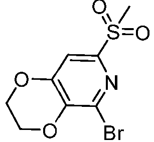

To a solution of 2-bromo-3-[(4-methoxyphenyl)methoxy]-6-(methylsulfanyl)pyridine (492.8 mg, 1.88 mmol) in THF (10 mL) was added RuCl₃•H₂O (12.72 mg, 0.05 mmol) at 0 °C. NaIO₄ (1206.4 mg, 5.64 mmol) in H₂O (10 mL) was added at 0 °C. The resulting solution was stirred for 1 h at RT. The resulting mixture was concentrated under vacuum. The residue was purified by combi-flash (EA/PE = 0-25%) to give 5-bromo-7-methanesulfonyl-2H,3H- [1,4]dioxino[2,3-c] pyridine (207.0 mg, 33%). ¹H NMR (400 MHz, DMSO-*d₆*) δ 7.56 (s, 1H), 4.54 - 4.48 (m, 4H), 3.23 (s, 3H). LCMS (ESI) m/e [M+1]⁺ 294.

### Example BB50: Synthesis of (R)-2-bromo-4-(2-methoxypropoxy)-6-(methylsulfonyl)pyridine

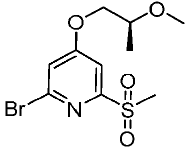

### Step 1: (R)-2-methoxypropyl 4-methylbenzenesulfonate

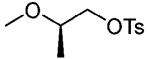

To a mixture of NaH (60% in mineral oil, 1.2 g, 30 mmol) in DMF (20 mL) was added 4-(R)-2-methoxypropan-1-ol ( 1.8 g, 20 mmol ) at 0 ~ 5 °C, after 30 mins, methylbenzenesulfonyl chloride (5.7 g, 30 mmol) was added to the mixture and stirred for 16 h at RT. The mixture was poured into water (50 mL), and extraced with EA (20 mL × 3). The combined organic layers were dried over Na₂SO₄, concentrated under reduced pressure to give (R)-2-methoxypropyl 4-methylbenzenesulfonate (3.95 g, 82.3 %). MS (ESI) m/e [M+1]⁺ 245.

### Step 2: (R)-2,6-dibromo-4-(2-methoxypropoxy)pyridine

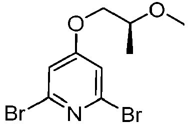

A mixture of (R)-2-methoxypropyl 4-methylbenzenesulfonate (3.95 g, 16.2 mmol), 2,6-dibromopyridin-4-ol (3.37 g, 13.5 mmol), K₂CO₃ (3.72 g, 27.0 mmol) in DMF (20 mL) was heated to 80 °C 16 h. The mixture was poured into water (50 mL), and extraced with EA (20 mL × 3). The combined organic layers were dried over Na₂SO₄, concentrated under reduced pressure to give (R)-2,6-dibromo-4-(2-methoxypropoxy)pyridine (3.05 g, 71.3 %). MS (ESI) m/e [M+1]⁺ 325.

### Step 3: (R)-2-bromo-4-(2-methoxypropoxy)-6-(methylthio)pyridine

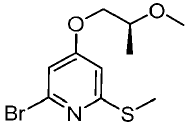

A mixture of (R)-2,6-dibromo-4-(2-methoxypropoxy)pyridine (3.0 g, 9.23 mmol), Sodium methyl mercaptan (wt : 20 %, 7.9 g, 27.7 mmol) in DMF (20 mL) was stirred for 16 h at RT. The mixture was poured into water (50 mL), and extraced with EA (20 mL × 3). The combined organic layers were dried over Na₂SO₄, concentrated under reduced pressure to give (R)-2-bromo-4-(2-methoxypropoxy)-6-(methylthio)pyridine (2.4 g, 88.9 %). MS (ESI) m/e [M+1]⁺ 292.

### Step 4: (R)-2-bromo-4-(2-methoxypropoxy)-6-(methylsulfonyl)pyridine

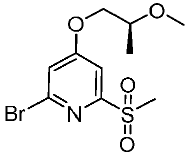

A mixture of (R)-2-bromo-4-(2-methoxypropoxy)-6-(methylthio)pyridine (2.4 g, 8.22 mmol), 3-Chloroperbenzoic acid (2.12 g, 12.33 mmol) in DCM (30 mL) was stirred for 16 h at RT. The DCM was exchanged by EA (30 mL), then the organic layer was washed with the mixture solution NaHCO₃ (10 mL) and Na₂S₂O₃ (10 mL), dried over Na₂SO₄, concentrated to give the crude product and purification by column chomatograph on silica gel using EA/PE (1/2) as eluant to afford (R)-2-bromo-4-(2-methoxypropoxy)-6-(methylsulfonyl)pyridine (2.5 g, 93.6 %). MS (ESI) m/e [M+1]⁺ 324.

### Example BB51: Synthesis of 6-bromo-3-methoxy-2-(methylsulfonyl)pyridine

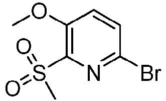

### Step 1: 6-bromo-3-methoxy-2-(methylthio)pyridine

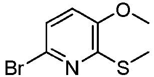

Into a 100-mL round-bottom flask, were placed 6-bromo-2-fluoro-3-methoxypyridine (1.00 g, 4.85 mmol), DMF (10 mL), NaSCH₃ (339.78 mg, 4.85 mmol). The resulting solution was stirred for 1 h at RT. The resulting solution was diluted with EA (100 mL). The resulting mixture was washed with H₂O (100 mL x 3). The mixture was dried over anhydrous sodium sulfate and concentrated. The residue was purified by combi-flash (EA/PE = 0 - 15%) give the product (0.90 g, 79% yield). LCMS (ESI) m/e [M+1]⁺ 234.

### Step 2: 6-bromo-3-methoxy-2-(methylsulfonyl)pyridine

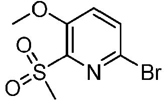

Into a 100-mL round-bottom flask purged and maintained with an inert atmosphere of nitrogen, were placed 6-bromo-3-methoxy-2-(methylsulfanyl)pyridine (1.00 g, 4.27 mmol), THF (10 mL), H₂O (10 mL), NaIO₄ (3.65 g, 17.07 mmol), RuCl₃.H₂O (192.59 mg, 0.85 mmol). The resulting solution was stirred for 1 h at 0 °C. The resulting solution was diluted with EA (100 mL). The resulting mixture was washed with H₂O (100 mL x 3). The mixture was dried over anhydrous sodium sulfate and concentrated. The residue was purified by combi-flash (EA/PE = 0 - 20%) give the product (705.30 mg, 62% yield). ¹H NMR (400 MHz, CDCl₃) δ 7.66 (d, *J* = 8.7 Hz, 1H), 7.37 (d, *J* = 8.7 Hz, 1H), 4.03 (s, 3H), 3.35 (s, 3H). LCMS (ESI) m/e [M+1]⁺ 266.

### Example BB52: Synthesis of 2-bromo-6-(methylsulfonyl)-4-phenylpyridine

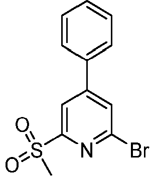

### Step 1: Synthesis of 2,6-dibromo-4-phenylpyridine

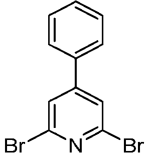

Into a 250 mL round-bottom flask purged and maintained with an inert atmosphere of nitrogen, were placed 2,6-dibromo-4-iodopyridine (5.20 g, 14.33 mmol), dioxane (30 mL), H₂O (10 mL), phenyl boronic acid (1.75 g, 14.35 mmol), Na₂CO₃ (3.04 g, 28.68 mmol), Pd(PPh₃)₄ (1656.31 mg, 1.43 mmol). The resulting solution was stirred for 2 h at 90 °C. The resulting solution was diluted with (100 mL). The resulting mixture was washed with H₂O (100 mL x 3). The mixture was dried over anhydrous sodium sulfate and concentrated. The residue was purified by combi-flash (EA/PE = 0 - 15%) give the product (0.77 g, 17% yield). LCMS (ESI) m/e [M+1]⁺ 312.

### Step 2: 2,6-dibromo-4-phenylpyridine

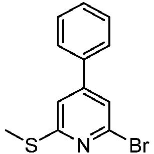

Into a 100 mL round-bottom flask, were placed 2,6-dibromo-4-phenylpyridine (770.00 mg, 2.460 mmol), DMF (10 mL, NaSCH₃ (172.21 mg, 2.46 mmol). The resulting solution was stirred for 1 h at rt. The resulting solution was diluted with EA (100 mL). The resulting mixture was washed with H₂O (100 mL x 3). The mixture was dried over anhydrous sodium sulfate and concentrated. The residue was purified by combi-flash (EA/PE = 0 - 10%) give the product (0.43 g, 62% yield). LCMS (ESI) m/e [M+1]⁺ 280.

### Step 3: 2-bromo-6-(methylsulfonyl)-4-phenylpyridine

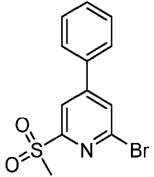

Into a 100 mL round-bottom flask purged and maintained with an inert atmosphere of nitrogen, were placed 2-bromo-6-(methylsulfanyl)-4-phenylpyridine (430.00 mg, 1.54 mmol), THF (10 mL), H₂O (10 mL), NaIO₄ (1313.06 mg, 6.14 mmol), RuCl₃.H₂O (69.20 mg, 0.31 mmol). The resulting solution was stirred for 1 h at 0 °C. The resulting solution was diluted with EA (50 mL). The resulting mixture was washed with H₂O (50 mL x 3). The mixture was dried over anhydrous sodium sulfate and concentrated. The residue was purified by combi-flash (EA/PE = 0 - 25%) give the product (386.50 mg, 80% yield). 1H NMR (400 MHz, DMSO-*d₆*) δ 8.37 (d, *J =* 1.5 Hz, 1H), 8.29 (*d, J =* 1.5 Hz, 1H), 8.01 - 7.92 (m, 2H), 7.63 - 7.54 (m, 3H), 3.37 (s, 3H). LCMS (ESI) m/e [M+1]⁺ 312.

### Example BB53: Synthesis of 2-bromo-4-methyl-6-(methylsulfonyl)pyridine

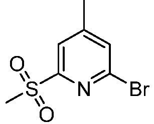

### Step 1: 2-bromo-4-methyl-6-(methylthio)pyridine

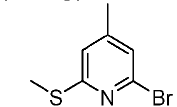

Into a 250-mL round-bottom flask, were placed 2,6-dibromo-4-methylpyridine (4.00 g, 15.62 mmol), DMF (50 mL), NaSCH₃ (0.98 g, 14.00 mmol). The resulting solution was stirred for 3 h at 25 °C. The resulting solution was diluted with H₂O (100 mL), extracted with 3x80 mL of EA (100 mL x 3) and the organic layers were combined and concentrated. The residue was purified by combi-flash (EA/PE = 0 - 20%) to give the product (3.3 g, 87% yield). LCMS (ESI, m/z) [M+1]⁺ 218.

### Step 2: 2-bromo-4-methyl-6-(methylsulfonyl)pyridine

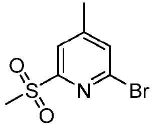

Into a 250-mL round-bottom flask, were placed 2-bromo-4-methyl-6-(methylsulfanyl)pyridine (3.30 g, 13.61 mmol), THF (30 mL), H₂O (10 mL). This was followed by the addition of NaIO₄ (11.65 g, 0.05 mmol) in H₂O (10 mL) in portions at 0 °C. To the mixture was added RuCl₃ (0.31 g, 1.37 mmol) in H₂O (10 mL) in portions at 0 °C. The resulting solution was stirred for 1 h at 25 °C. The resulting solution was diluted with H₂O, extracted with EA (100 mL x 3) and the organic layers were combined and concentrated. The residue was purified by combi-flash (EA/PE = 0-30%) to give the product (2.45 g, 65% yield). ¹H NMR (300 MHz, DMSO-*d*₆) δ 7.97 (t*, J =* 0.9 Hz, 1H), 7.92 (t*, J =* 1.1 Hz, 1H), 3.30 (s, 3H), 2.47 (s, 3H). LCMS (ESI, m/z): [M+H] 250.

### Example BB54: Synthesis of 2-bromo-6-methanesulfonyl-4-phenoxypyridine

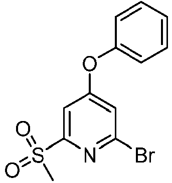

### Step 1: 2,6-dibromo-4-phenoxypyridine

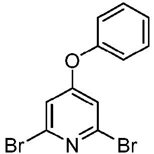

Into a 250-mL round-bottom flask, were placed 2,6-dibromo-4-chloropyridine (4.80 g, 17.69 mmol), DMF (75 mL), phenol (1.50 g, 15.93 mmol), Cs₂CO₃ (11.53 g, 35.38 mmol). The resulting solution was stirred for 2 h at 90 °C in an oil bath. The resulting solution was extracted with EA (50 mL x 3) concentrated. The residue was purified by prep-MPLC (column: C18 spherical 20-35 um, 100A, 330g; phase: A-H₂O (0.05% TFA); B-Acetonitrile, B%:60%-70% in 40 min) to give the product (0.96 g, 14% yield). LCMS (ESI) m/e [M+1]⁺ 330.

### Step2: 2-bromo-6-(methylsulfanyl)-4-phenoxypyridine

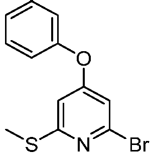

Into a 50-mL round-bottom flask purged and maintained with an inert atmosphere of nitrogen, were placed 2,6-dibromo-4-phenoxypyridine (0.96 g, 2.91 mmol), dioxane (15 mL), (methylsulfanyl)sodium (0.18 g, 2.56 mmol), Pd₂(dba)₃ (0.13 g, 0.14 mmol), XantPhos (0.17 g, 0.29 mmol). The resulting solution was stirred for 3 h at 75°C in an oil bath. The resulting mixture was concentrated under vacuum. The residue was purified by combi-flash (EA/PE = 0 - 1%) to give the product (0.82 g, 85%). LCMS (ESI) m/e [M+1]⁺ 296.

### Step 3: 2-bromo-6-methanesulfonyl-4-phenoxypyridine

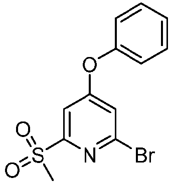

Into a 25-mL round-bottom flask, were placed 2-bromo-6-(methylsulfanyl)-4-phenoxypyridine (0.82 g, 2.76 mmol), THF (7 mL). This was followed by the addition of NaIO₄ (1.78 g, 8.32 mmol) and RuCl₃ (0.020 g, 0.080 mmol) in H₂O (7 mL) dropwise with stirring at 0°C. The resulting solution was stirred for 1 h at RT. The resulting mixture was concentrated under vacuum. The residue was purified by combi-flash (EA/PE = 0 - 11%) to give the product (488.80 mg, 48% yield). ¹H NMR (300 MHz, DMSO-*d*₆) δ 7.60 - 7.51 (m, 3H), 7.43 - 7.36 (m, 2H), 7.34 -7.27 (m, 2H), 3.28 (s, 3H). LCMS (ESI) m/e [M+1]⁺ 328.

### Example BB55: Synthesis of 2-chloro-6-methanesulfonyl-4-(trifluoromethyl)pyridine

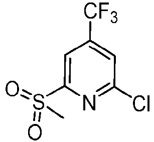

### Step 1: 2-chloro-6-(methylsulfanyl)-4-(trifluoromethyl)pyridine

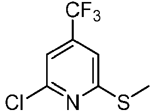

Into a 250-mL round-bottom flask, were placed 2,6-dichloro-4-(trifluoromethyl) pyridine (6.00 g, 27.78 mmol), DMF (90.00 mL), (methylsulfanyl)sodium (1.75 g, 24.97 mmol). The resulting solution was stirred for 1 h at RT. The resulting solution was extracted with 3x50 mL of EA concentrated. The residue was purified by combi-flash to give the product (2.56 g, 36% yield). GCMS (ESI) m/e [M] 227.

### Step 2: 2-chloro-6-methanesulfonyl-4-(trifluoromethyl)pyridine

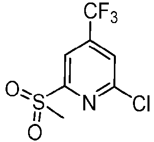

Into a 100-mL 3-necked round-bottom flask, were placed 2-chloro-6-(methylsulfanyl)-4-(trifluoromethyl) pyridine (2.56 g, 11.24 mmol), THF (20 mL). This was followed by the addition of NaIO₄ (7.22 g, 33.75 mmol) and RuCl₃ (0.08 g, 0.337 mmol) in H₂O (20 mL) dropwise with stirring at 0 °C. The resulting solution was stirred for 1 h at RT. The resulting mixture was concentrated under vacuum. The residue was purified by combi-flash (EA/PE = 0 - 11%) to give the product (1.08 g, 33% yield). ¹H NMR (300 MHz, DMSO-*d*₆) δ 8.49 - 8.46 (m, 1H), 8.35 - 8.30 (m, 1H), 3.38 (s, 3H). LCMS (ESI) m/e [M+1]⁺ 260.

### Example BB56: Synthesis of 6-bromo-3-methyl-2-(methylsulfonyl)pyridine

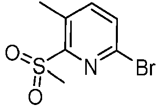

### Step: 1: 6-bromo-2-fluoro-3-methylpyridine

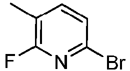

Into a 500-mL 3-necked round-bottom flask purged and maintained with an inert atmosphere of nitrogen, were placed 2-bromo-6-fluoropyridine (10.00 g, 56.82 mmol), THF (200 mL). This was followed by the addition of LDA (7.91 g, 73.87 mmol) dropwise with stirring at -78 °C in 15 mins. The resulting solution was stirred for 3 h at -78 °C in a liquid nitrogen bath. To this was added methyl iodide (8.87 g, 62.50 mmol) dropwise with stirring at -78 °C in 5 min. The resulting solution was allowed to react, with stirring, for an additional 1 h at RT. The reaction was then quenched by the addition of aq. NH₄Cl (150 mL). The resulting solution was extracted with EA (100 mL x 3). The resulting mixture was washed with H₂O. The resulting mixture was concentrated under vacuum. The residue was purified by combi-flash (PE /EA = 0 - 10%) give the product (4.4 g, 40% yield). LCMS (ESI) m/e [M+1]⁺ 190.

### Step: 2: 6-bromo-3-methyl-2-(methylthio)pyridine

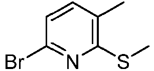

Into a 100-mL round-bottom flask, were placed 6-bromo-2-fluoro-3-methylpyridine (3.40 g, 17.89 mmol), DMF (40 mL), NaSCH₃ (1.00 g, 14.31 mmol). The resulting solution was stirred for 1.5 h at RT. The reaction was then quenched by the addition of aq. NH₄Cl (50 mL). The resulting solution was extracted with EA (50 mL x 3). The resulting mixture was washed with H₂O. The resulting mixture was concentrated under vacuum. The residue was purified by combi-flash (PE /EA = 0 - 1%) give the product (2.9 g, 74% yield). LCMS (ESI) m/e [M+1]⁺ 218.

### Step: 3: 6-bromo-3-methyl-2-(methylsulfonyl)pyridine

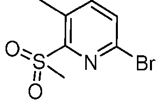

Into a 250-mL round-bottom flask, were placed 6-bromo-3-methyl-2- (methylsulfanyl)pyridine (2.90 g, 13.29 mmol), THF (30 mL). This were followed by the addition of NaIO₄ (8.53 g, 39.88 mmol), H₂O (30 mL) and RuCl₃ (82.57 mg, 0.40 mmol), in portions at 0 °C in 5 min. The resulting solution was stirred for 1 h at RT. The resulting solution was extracted with 50 mL of EA. The resulting mixture was washed with H₂O (25 mL x 2). The resulting mixture was concentrated under vacuum. The residue was purified by combi-flash (PE /EA = 0 - 13%) give the product (2.48 g, 75% yield). ¹H NMR (400 MHz, DMSO-*d*₆) δ 7.93 - 7.89 (m, 2H), 3.38 (s, 3H), 2.57 (s, 3H). LCMS (ESI) m/e [M+1]⁺ 250.

### Example BB57: Synthesis of 2-bromo-6-(methylsulfonyl)-4-(tetrahydro-2H-pyran-4-yl)pyridine

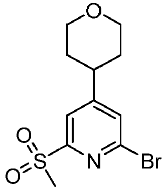

### Step 1: Synthesis of 2,6-dibromo-4-iodopyridine

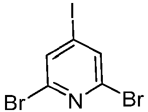

In a flame-dried and nitrogen-flushed 3-necked Schlenk tube equipped with a rubber septum and a magnetic stirring bar, 2,6-dibromopyridine (5.0 g, 21.11 mmol) was dissolved in dry THF (20 mL). The mixture was cooled to -30 °C. Then 2,2,6,6-tetramethylpiperidinylmagnsium chloride lithium chloride complex (32 mL, 31.70 mmol, 1 M in THF) was added dropwise via a syringe and stirred for a further 30 min at that temperature. Then I₂ (5.9 g, 23.24 mmol) was added quickly to the mixture at -30 °C under the protection of nitrogen gas and stirred for 30 min at the same temperature. The reaction mixture was quenched by adding 30 mL of sat. NH₄Cl followed by extraction with EtOAc, dryness over anhydrous Na₂SO₄ and concentration under reduced pressure. The residue was purified by silica gel column chromatography, eluted with EtOAc/PE (1/10, v/v) to afford the product (3.0 g, 39% yield). LCMS (ESI) m/e [M+1]⁺ = 362.

### Step 2: Synthesis of 2,6-dibromo-4-(3,6-dihydro-2H-pyran-4-yl)pyridine

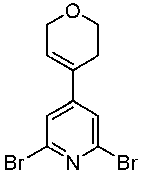

Into a stirred solution of 2,6-dibromo-4-iodopyridine (2.0 g, 5.51 mmol) and 2-(3,6-dihydro-2H-pyran-4-yl)- 4,4,5,5-tetramethyl-1,3,2-dioxaborolane (1.2 g, 5.71 mmol) in 1,4-dioxane (40 mL) and water (8 mL), were added Pd(dppf)Cl₂ (403 mg, 0.55 mmol) and K₂CO₃ (1.5 g, 10.85 mmol) at rt under nitrogen atmosphere. The resulting mixture was stirred for 2 hours at 90 °C under nitrogen atmosphere. The resulting mixture was concentrated under reduced pressure. The resulting mixture was extracted with EtOAc (2 x100 mL). The combined organic layers were washed with water (100 mL) and brine (100 mL) and then dried over anhydrous Na₂SO₄. After filtration, the filtrate was concentrated under reduced pressure. The residue was purified by silica gel column chromatography, eluted with EtOAc/PE (1/12, v/v) to give the product (1.2 g, 68% yield). LCMS (ESI) m/e [M+1]⁺ = 318.

### Step 3: Synthesis of 2,6-dibromo-4-(tetrahydro-2H-pyran-4-yl)pyridine

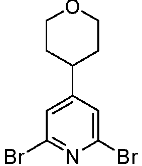

Into a solution of 2,6-dibromo-4-(3,6-dihydro-2H-pyran-4-yl)pyridine (1.0 g, 3.13 mmol) in EtOAc (30 mL) was added 5% Rh/C (200 mg, 5% wt). The mixture was stirred at room temperature overnight under hydrogen atmosphere. The resulting mixture was filtered, the filter cake was washed with EtOAc. The filtrate was concentrated under reduced pressure to afford the product (1.0 g, 100% yield). LCMS (ESI) m/e [M+1]⁺ = 320.

### Step 4: Synthesis of 2-bromo-6-(methylthio)-4-(tetrahydro-2H-pyran-4-yl)pyridine

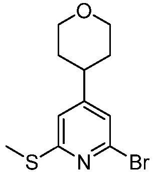

A solution of 2,6-dibromo-4-(tetrahydro-2H-pyran-4-yl)pyridine (1.0 g, 3.11 mmol) and Sodium thiomethoxide (195 mg, 2.79 mmol) in DMF (10 mL) was stirred overnight at room temperature. Water was added to the reaction mixture, and the resulting mixture was extracted with EtOAc. The combined organic layers were dried over anhydrous Na₂SO₄. After filtration, the filtrate was concentrated under reduced pressure. The residue was purified by silica gel column chromatography, eluted with EtOAc/PE (1/4, v/v) to give the product (550 mg, 61% yield). LCMS (ESI) m/e [M+1]⁺ = 288.

### Step 5: Synthesis of 2-bromo-6-(methylsulfonyl)-4-(tetrahydro-2H-pyran-4-yl)pyridine

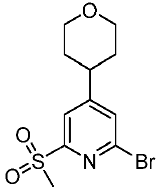

To a stirred solution of 2-bromo-6-(methylthio)-4-(tetrahydro-2H-pyran-4-yl)pyridine (550 mg, 1.91 mmol) in water (5 mL) amd THF (5 mL) was added RuCl₃.H₂O (12 mg, 0.05 mmol) in water (2 mL) dropwise at 0 °C. To the above mixture was added NaIO₄ (1.5 g, 7.01 mmol) in water (10 mL) dropwise at 0 °C. The resulting mixture was stirred for additional 30 min at 0 °C. The resulting mixture was extracted with EtOAc. The combined organic layers were dried over anhydrous Na₂SO₄. After filtration, the filtrate was concentrated under reduced pressure. The residue was purified by Prep-TLC (EtOAc:PE = 1:5) to give the product (361 mg, 59% yield). ¹H NMR (300 MHz, DMSO-*d*₆) δ 7.97 (s, 1H), 7.95 (s, 1H), 3.97-3.94 (m, 2H), 3.44-3.39 (m, 2H), 3.33 (s, 3H), 3.08-3.00 (m, 1H), 1.78-1.69 (m, 4H). LCMS (ESI) m/e [M+1]⁺ = 320.

### Example BB58: Synthesis of 2-bromo-3,4-dimethoxy-6-(methylsulfonyl)pyridine

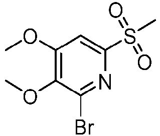

### Step: 1: 2-bromo-6-iodo-3,4-dimethoxypyridine

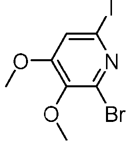

Into a 250-mL round-bottom flask, were placed 2-bromo-6-iodo-3-methoxypyridin-4-ol (2.95 g, 8.94 mmol), DMF (90 mL) and K₂CO₃ (2.47 g, 17.88 mmol). This was followed by the addition of CH₃I (2.54 g, 17.88 mmol) at 0 °C in 5 mins. The resulting solution was stirred for 1 hr at rt. The reaction was then quenched by the addition of 40 mL of NH₄Cl (aq). The resulting solution was extracted with of EtOAc (40 mL x2) and combined organic layer was washed with H₂O and brine. The resulting mixture was concentrated under vacuum. The residue was purified by combi-flash (PE/EtOAc = 0-11%) to give the product (2.6 g, 84.5% yield). LCMS (ESI) m/e [M+1]⁺ 344.

### Step: 2: 2-bromo-3,4-dimethoxy-6-(methylthio)pyridine

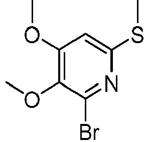

Into a 100-mL round-bottom flask purged and maintained with an inert atmosphere of nitrogen, were placed 2-bromo-6-iodo-3,4-dimethoxypyridine (2.60 g, 7.56 mmol), Pd₂(dba)₃ (346.1 mg, 0.38 mmol), Xant-phos (437.4 mg, 0.76 mmol) and dioxane (60 mL). This was followed by the addition of CH₃SNa (529.6 mg, 7.56 mmol). The resulting solution was stirred overnight at 75 °C in an oil bath. The resulting mixture was concentrated under vacuum. The residue was purified by combi-flash (PE/EtOAc = 0-6%) to give the product (1.64 g, 82%). LCMS (ESI) m/e [M+1]⁺ 264.

### Step: 3: 2-bromo-3,4-dimethoxy-6-(methylsulfonyl)pyridine

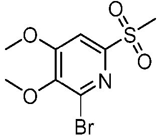

Into a 100-mL round-bottom flask, were placed 2-bromo-3,4-dimethoxy-6-(methylsulfanyl)pyridine (1.60 g, 6.06 mmol), H₂O (35 mL), THF (35 mL). This was followed by the addition of RuCl₃ (126 mg, 0.61 mmol) at 0 °C in 2 min. To this was added NaIO₄ (3.89 g, 18.17 mmol) at 0 °C in 2 min. The resulting solution was stirred for 1 RT at rt. The reaction was then quenched by the addition of 50 mL of NH₄Cl (aq). The resulting solution was extracted with EtOAc (80 mL) and the combined organic layer was washed with brine. The resulting mixture was concentrated under vacuum and the residue was purified by combi-flash (PE/EtOAc = 0-18%) to give the product (354.5 mg, 19.8%). ¹H NMR (400 MHz, DMSO-*d*₆) δ 7.72 (s, 1H), 4.06 (s, 3H), 3.88 (s, 3H), 3.28 (s, 3H). LCMS (ESI) m/e [M+1]⁺ 296.

### Example BB59: Synthesis of 2-bromo-4-(methoxy-d3)-6-(methylsulfonyl)pyridine

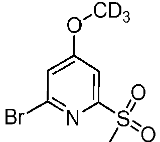

### Step 1: 2,6-dibromo-4-(methoxy-d3)pyridine

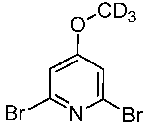

A mixture of 2,6-dibromopyridin-4-ol (2.4 g, 9.5 mmol), CD₃I (1.4 g, 9.5 mmol), K₂CO₃ (3.9 g, 28.5 mmol) in DMF (30 mL) was stirred at 25 °C for 2 hr. Upon completion of the reaction, H₂O (200 mL) was added and the resulting solution was extracted with EA (30 mL x 3). The combined organic layers were dried over Na₂SO₄ and concentrated in vacuo to give the crude product (2.56 g, crude). MS(ESI) m/e [M+1]⁺ 269.

### Step 2: 2-bromo-4-(methoxy-d3)-6-(methylthio)pyridine

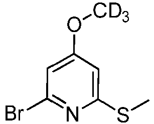

To a solution of 2,6-dibromo-4-(methoxy-d3)pyridine (2.56 g, 9.5 mmol) in DMF (30 ml) was added NaSMe (731 mg, 10.4 mmol) in one portion at 25 °C. The mixture was stirred at 25°C for 16 h. The mixture was poured into H₂O (200 ml) and extracted with EA (30 mL x 3). The combined organic layers were dried over Na₂SO₄ and concentrated to give crude product (2.4 g, crude). MS(ESI) m/e [M+1]⁺ 237.

### Step 3: 2-bromo-4-(methoxy-d3)-6-(methylsulfonyl)pyridine

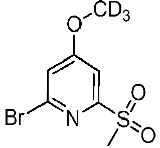

To a solution of 2-bromo-4-(methoxy-d3)-6-(methylthio)pyridine (2.4 g, 10.1 mmol) in MeOH (40 ml)/H₂O(20 ml) was added Oxone (12.4 g, 20.1 mmol) at 25 °C. The mixture was stirred at RT for 2 hr. Upon completion of the reaction, the solid was filtered out and the filtrate was concentrated. The crude product was purified by silica gel column chromatography eluted with (PE/EtOAc 100:1 to 20: 1) to give the product (1.3 g, 47% yield). ¹H NMR (400 MHz, CDCl₃) δ 7.57 (s, 1 H), 7.18 (s, 1 H), 3.25 (s, 3 H). MS(ESI) m/e [M+1]⁺ 269.

### Example BB60: Synthesis of (R and S)-2-chloro-4-(1-methoxyethyl)-6-(methylsulfonyl)pyridine

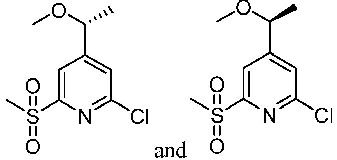

### Step1. Synthesis of 1-(2,6-dichloropyridin-4-yl)ethan-1-ol

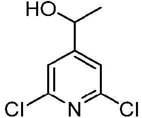

To a stirred solution of 2,6-dichloro-4-iodopyridine (10.00 g, 36.51 mmol) in THF (100 mL) was added n-BuLi in n-hexane (21.9 mL, 2.5 M, 54.77 mmol) dropwise at -78°C under N₂ atmosphere. The resulting mixture was stirred for 1 h at -78 °C. To the above mixture was added acetaldehyde (4.83 g, 0.11 mmol) dropwise over 15 min at -78 °C. The resulting mixture was stirred for additional 1 h at -78 °C. The reaction was quenched with sat. NH₄Cl (aq.) at -78 °C. The resulting mixture was extracted with EtOAc (2 x 300 mL). The combined organic layers were washed with brine (2 x 200 mL), dried over anhydrous Na₂SO₄. After filtration, the filtrate was concentrated under reduced pressure. The crude product (5.50 g, 78% yield) was used in the next step directly without further purification. LCMS (ESI) m/e [M+1] ⁺ 191.99.

### Step2. Synthesis of 2,6-dichloro-4-(1-methoxyethyl)pyridine

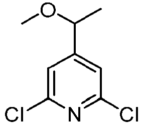

To a stirred solution of 1-(2,6-dichloropyridin-4-yl)ethanol (5.50 g, 28.64 mmol) and CH₃I (6.10 g, 42.98 mmol) in THF (100 mL) were added NaH (1.37 g, 60% in mineral oil, 57.28 mmol) in portions at 0 °C. The resulting mixture was stirred for 2 h at rt under N₂ atmosphere. The reaction was quenched with Water/Ice at 0 °C. The resulting mixture was extracted with EtOAc (2 x 200 mL). The combined organic layers were washed with brine (1 x 200 mL), dried over anhydrous Na₂SO₄. After filtration, the filtrate was concentrated under reduced pressure. The crude product (5 g, crude) was used in the next step directly without further purification. LCMS (ESI) m/e [M+1] ⁺ 206.

### Step 3. Synthesis of 2-chloro-4-(1-methoxyethyl)-6-(methylthio)pyridine

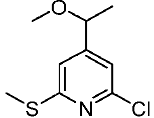

To a stirred solution of 2,6-dichloro-4-(1-methoxyethyl)pyridine (5.00 g, 24.26 mmol) in DMF (50 mL,) was added MeSNa (2.55 g, 36.43 mmol) in portions at 0 °C. The resulting mixture was stirred for 3 h at rt. The resulting mixture was extracted with EtOAc (2 x 200 mL). The combined organic layers were washed with brine (2 x 200 mL), dried over anhydrous Na₂SO₄. After filtration, the filtrate was concentrated under reduced pressure. The crude product (7 g, crude) was used in the next step directly without further purification. LCMS (ESI) m/e [M+1] ⁺ 218.

### Step 4. Synthesis of 2-chloro-4-(1-methoxyethyl)-6-(methylsulfonyl)pyridine

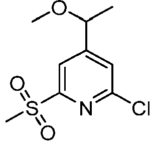

To a stirred solution of 2-chloro-4-(1-methoxyethyl)-6-(methylsulfanyl)pyridine (7.00 g, 32.15 mmol) and RuCl₃.H₂O (0.22 g, 0.97 mmol) in THF (70 mL) and H₂O (70 mL) were added NaIO₄ (13.75 g, 64.29 mmol) in portions at 0 °C and the resulting mixture was stirred at rt for 10 h. The resulting mixture was extracted with EtOAc (2 x 200 mL). The combined organic layers were washed with brine (2 x 200 mL), dried over anhydrous Na₂SO₄. After filtration, the filtrate was concentrated under reduced pressure. The residue was purified by combi-flash (PE/EtOAc = 0-20%) to give the product (5 g, 62%). LCMS (ESI) m/e [M+1] ⁺ 250.

### Step 5. Synthesis of (R and S)-2-chloro-4-(1-methoxyethyl)-6-(methylsulfonyl)pyridine

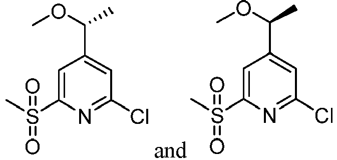

Racemic 2-chloro-4-(1-methoxyethyl)-6-(methylsulfonyl)pyridine) (3 g) was separated by chiral-HPLC with the following conditions (Column: CHIRAL ART Cellulose-SB, 5 x 25cm, 5um; Mobile Phase A: Hex (0.5% 2M NH₃-MeOH), Mobile Phase B:EtOH; Flow rate: 95 mL/min; Gradient:20% B to 20% B in 15 min; 220 nm; RT1: 8.55 min; RT2: 9.50 min), the faster peak was collected and concentrated to give one pure isomer (1.32 g, 44% yield). For faster peak (retention time: 8.55 min): ¹H NMR (400 MHz, CDCl₃) δ 7.96 (dd, *J =* 1.3 Hz, 1H), 7.56 (dd*, J =* 1.3 Hz, 1H), 4.43 - 4.38 (m, 1H), 3.35 (s, 3H), 3.29 (s, 3H), 1.47 (d, *J =* 6.6 Hz, 3H). LCMS (ESI) m/e [M+1] ⁺ = 249.85. The slower peak was collected and concentrated to afford the product (1.22 g, 41% yield). For slower peak (retention time: 9.50 min): ¹H NMR (400 MHz, CDCl₃) δ 7.96 (dd, *J* = 1.2, Hz, 1H), 7.56 (dd, *J* = 1.3 Hz, 1H), 4.46 - 4.36 (m, 1H), 3.35 (s, 3H), 3.29 (s, 3H), 1.47 (d, *J* = 6.5 Hz, 3H). LCMS (ESI) m/e [M+1] ⁺ 250.

### Example BB61: Synthesis of (S)-4-(2-chloro-6-(methylsulfonyl)pyridin-4-yl)-3-methylmorpholine

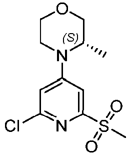

### Step 1: Synthesis of (S)-4-(2,6-dichloropyridin-4-yl)-3-methylmorpholine

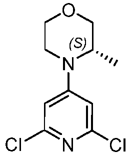

Into a 250 mL round-bottom flask purged and maintained with an inert atmosphere of nitrogen, were placed 2,6-dichloro-4-iodopyridine (5.00 g, 18.25 mmol), dioxane (100 mL), (3S)-3-methylmorpholine (1.85 g, 18.29 mmol), Pd₂(dba)₃·CHCl₃ (1.89 g, 1.82 mmol), XantPhos (2.11 g, 3.64 mmol) and Cs₂CO₃ (17.84 g, 54.75 mmol). The resulting solution was stirred for 3 hr at 110 °C in an oil bath. After cooling to room temperature, the reaction was concentrated. The residue was purified by combi-flash (EtOAc/PE = 0-15%) to give the product (1.20 g, 24%). LCMS (ESI, m/z): [M+H]⁺ 247.

### Step 2: Synthesis of (S)-4-(2-chloro-6-(methylthio)pyridin-4-yl)-3-methylmorpholine

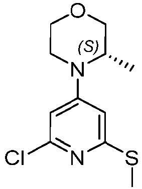

Into a 100 mL round-bottom flask, were placed (3S)-4-(2,6-dichloropyridin-4-yl)-3-methylmorpholine (1.90 g, 7.68 mmol), DMF (30 mL) and CH₃SNa (1.08 g, 15.42 mmol). The resulting solution was stirred for 5 hr at 25 °C. The resulting solution was diluted with 50 mL of H₂O, extracted with 3x30 mL of EtOAc and the organic layers were combined and concentrated. The residue was purified by combi-flash (EtOAc/PE = 0-10%) to give the product (1.40 g, 63%). LCMS (ESI, m/z): [M+H] ⁺ 259.

### Step 3: Synthesis of (S)-4-(2-chloro-6-(methylsulfonyl)pyridin-4-yl)-3-methylmorpholine

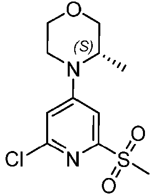

Into a 100 mL round-bottom flask, were placed (3S)-4-[2-chloro-6-(methylsulfanyl)pyridin-4-yl]-3-methylmorpholine (1.40 g, 5.41 mmol), THF (20 mL), H₂O (20 mL), NaIO₄ (4.63 g, 21.64 mmol). To the mixture was added RuCl₃·H₂O (0.12 g, 0.53 mmol) in portions at 0 °C. The resulting solution was stirred for 3 hr at 25 °C. The resulting solution was diluted with 50 mL of H₂O, extracted with 3x30 mL of ethyl acetate and the organic layers were combined and concentrated. The residue was purified by combi-flash (EtOAc/PE = 0-45%) to give the product (1.30 g, 79%). ¹H NMR (300 MHz, DMSO-*d*₆) δ 7.31 (d, *J=* 2.3 Hz, 1H), 7.11 (d, *J =* 2.3 Hz, 1H), 4.17 *(d, J =* 7.2 Hz, 1H), 4.02 - 3.90 (m, 1H), 3.81 - 3.58 (m, 3H), 3.58 - 3.45 (m, 1H), 3.24 -3.15 (m, 4 H), 1.18 (d, *J* = 6.7 Hz, 3H). LCMS (ESI, m/z): [M+H] ⁺ 291.

### Example BB62: Synthesis of (R)-4-(2-chloro-6-(methylsulfonyl)pyridin-4-yl)-3-methylmorpholine

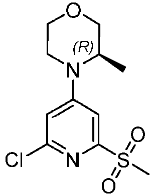

### Step 1: Synthesis of (R)-4-(2,6-dichloropyridin-4-yl)-3-methylmorpholine

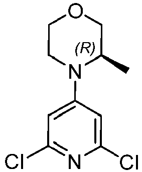

Into a 250 mL round-bottom flask purged and maintained with an inert atmosphere of nitrogen, were placed 2,6-dichloro-4-iodopyridine (2.50 g, 9.13 mmol), dioxane (50 mL), (3R)-3-methylmorpholine (0.97 g, 9.13 mmol), Pd₂(dba)₃·CHCl₃ (0.97 g, 0.91 mmol), XantPhos (1.06 g, 1.82 mmol), Cs₂CO₃ (8.92 g, 27.38 mmol). The resulting solution was stirred for 3 hr at 110 °C in an oil bath. After cooling to room temperature, the reaction was concentrated and the residue was purified by combi-flash (EtOAc/PE = 0-15%) to give the product (1.9 g, 84%). LCMS (ESI, m/z): [M+H] ⁺ 247.

### Step 2: Synthesis of (R)-4-(2-chloro-6-(methylthio)pyridin-4-yl)-3-methylmorpholine

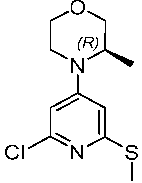

Into a 100 mL round-bottom flask, were placed (3R)-4-(2,6-dichloropyridin-4-yl)-3-methylmorpholine (1.90 g, 7.68 mmol), DMF (30 mL), CH₃SNa (1.08 g, 15.42 mmol). The resulting solution was stirred for 5 h at 25 °C. The resulting solution was diluted with 50 mL of H₂O, extracted with 3x30 mL of ethyl acetate and the organic layers were combined and concentrated. The residue was purified by combi-flash (EtOAc/ PE = 0-10%) to give the product (1.70 g, 86%). LCMS (ESI, m/z): [M+H] ⁺ 259.

### Step 3: Synthesis of (R)-4-(2-chloro-6-(methylsulfonyl)pyridin-4-yl)-3-methylmorpholine

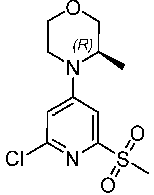

Into a 100 mL round-bottom flask, were placed (3R)-4-[2-chloro-6-(methylsulfanyl)pyridin-4-yl]-3-methylmorpholine (1.70 g, 6.59 mmol), THF (20 mL), H₂O (20 mL), NaIO₄ (5.64 g, 26.36 mmol). To the mixture was added RuCl₃·H₂O (0.17 g, 0.66 mmol) in portions at 0 °C. The resulting solution was stirred for 3 h at 25 °C. The resulting solution was diluted with 50 mL of H₂O, extracted with 3x30 mL of ethyl acetate and the organic layers were combined and concentrated. The residue was purified by combi-flash (EtOAc/ PE = 0-45%) to give the product (1.20 g, 63%). ¹H NMR (300 MHz, DMSO-*d*₆) δ 7.30 (d, *J* = 2.3 Hz, 1H), 7.12 (d, *J =* 2.3 Hz, 1H), 4.16 (d, *J =* 7.2 Hz, 1H), 4.05 - 3.93 (m, 1H), 3.82 - 3.56 (m, 3H), 3.57 - 3.45 (m, 1H), 3.23 - 3.15 (m, 4H), 1.19 (d, *J* = 6.7 Hz, 3H). LCMS (ESI, m/z): [M+H]⁺ 291.

### Example BB63: Synthesis of 2-chloro-6-methanesulfonyl-4-(3-methoxyazetidin-1-yl)pyridine

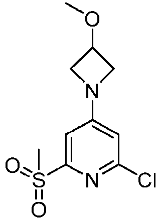

### Step 1: Synthesis of 2,6-dichloro-4-(3-methoxyazetidin-1-yl)pyridine

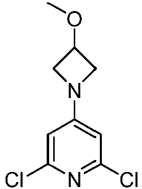

To a stirred mixture of 2,6-dichloro-4-iodopyridine (3.0 g, 10.95 mmol), 3-methoxyazetidine (1.15 g, 13.14 mmol ), Xantphos (633.80 mg, 1.09 mmol ) and Cs₂CO₃ (7.14 g, 21.91 mmol ) in 1,4-dioxane (30 mL) was added Pd₂(dba)₃ (501.53 mg, 0.55 mmol ) at rt. The resulting mixture was stirred for 2 h at 100 °C under N₂ atmosphere. The mixture was allowed to cool down to rt. The resulting mixture was extracted with EtOAc (2 x 50 mL) and the combined organic layers were dried over anhydrous Na₂SO₄. The solid was removed by filtration and the filtrate was concentrated under reduced pressure. The residue was purified by combi-flash (EtOAc/PE = 20 %) to give the product (1.2 g, 47% yield). LCMS (ESI) m/e [M+1]⁺ = 233.

### Step 2: Synthesis of 2-chloro-4-(3-methoxyazetidin-1-yl)-6-(methylsulfanyl)pyridine

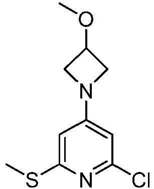

To a stirred solution of 2,6-dichloro-4-(3-methoxyazetidin-1-yl)pyridine (1.2 g, 5.15 mmol ) in DMF (10 mL) was added CH₃SNa (900.94 mg, 12.87 mmol) at 0 °C. The resulting mixture was stirred for 2 h at rt. Upon completion of the reaction, water was added and the resulting mixture was extracted with EtOAc (2 x 50 mL). The combined organic layers were washed with water (2 x 50 mL), dried over anhydrous Na₂SO₄. After filtration, the filtrate was concentrated under reduced pressure to give the crude product (1.2 g). It was used in the next step directly without further purification. LCMS (ESI) m/e [M+1]⁺ = 245.

### Step 3: Synthesis of 2-chloro-6-methanesulfonyl-4-(3-methoxyazetidin-1-yl)pyridine

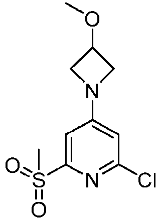

To a stirred solution of 2-chloro-4-(3-methoxyazetidin-1-yl)-6-(methylsulfanyl)pyridine (1.2 g, 4.90 mmol) and RuCl₃.H₂O (33.16 mg, 0.15 mmol ) in THF(10 mL) and H₂O (10 mL) was added NaIO₄ (2.1 g, 9.81 mmol) at 0 °C. the mixture was stirred for 1h at rt. The resulting mixture was extracted with EtOAc (2 x 50 mL). The combined organic layers were dried over anhydrous Na₂SO₄. After filtration, the filtrate was concentrated under reduced pressure. The residue was purified by combi-flash (EtOAc/PE = 50%) to give the product (806.4 mg, 59% yield). ¹H NMR (300 MHz, Chloroform-d) δ 6.96 (s, 1H), 6.35 (s, 1H), 4.48-4.37 (m, 1H), 4.30-4.20 (m, 2H), 3.99-3.90 (m, 2H), 3.38 (s, 3H), 3.21 (s, 3H). LCMS (ESI) m/e [M+1]⁺ = 277.

### Example BB64: Synthesis of 2-chloro-6-methanesulfonyl-4-(4-methoxypiperidin-1-yl)pyridine

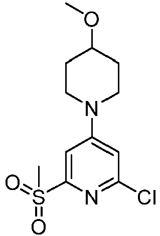

### Step 1: Synthesis of 2,6-dichloro-4-(4-methoxypiperidin-1-yl)pyridine

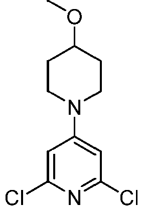

To a stirred mixture of 2,6-dichloro-4-iodopyridine (3.0 g, 10.95 mmol), 4-methoxypiperidine (1.51 g, 13.14 mmol), Xantphos (633.80 mg, 1.09 mmol) and Cs₂CO₃ (7.14 g, 21.91 mmol) in 1,4-dioxane (30 mL) was added Pd₂(dba)₃ (501.53 mg, 0.55 mmol) at rt. The resulting mixture was stirred for 3 h at 100 °C under N₂ atmosphere. After cooled to rt, the resulting mixture was extracted with EtOAc (2 x 50 mL). The combined organic layers were dried over anhydrous Na₂SO₄. After filtration, the filtrate was concentrated under reduced pressure. The residue was purified by combi-flash (EtOAc/PE = 25%) to give the product (1.3 g, 45% yield). LCMS (ESI) m/e [M+1]⁺ = 261.

### Step 2: Synthesis of 2-chloro-4-(4-methoxypiperidin-1-yl)-6-(methylsulfanyl)pyridine

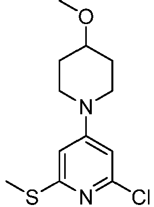

To a stirred solution of 2,6-dichloro-4-(4-methoxypiperidin-1-yl)pyridine (1.3 g, 4.98 mmol) in DMF (10 mL) was added CH₃SNa (522.69 mg, 7.47 mmol) at 0 °C. The resulting mixture was stirred for 2 h at rt. Water was added and the resulting mixture was extracted with EtOAc (2 x 50 mL). The combined organic layers were washed with water (2 x 50 mL), dried over anhydrous Na₂SO₄. After filtration, the filtrate was concentrated under reduced pressure to give the crude product. It was used in the next step directly without further purification. LCMS (ESI) m/e [M+1]⁺ = 273.

### Step 3: Synthesis of 2-chloro-6-methanesulfonyl-4-(4-methoxypiperidin-1-yl)pyridine

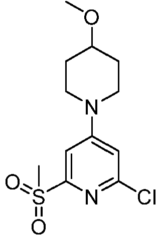

To a stirred solution of 2-chloro-4-(4-methoxypiperidin-1-yl)-6-(methylsulfanyl)pyridine (1.48 g, 5.42 mmol) and RuCl₃.H₂O (36.69 mg, 0.16 mmol) in THF (15 mL) and H₂O (15 mL) was added NaIO₄ (2.32 g, 10.85 mmol) at 0 °C. The resulting mixture was stirred for 1h at rt. Upon completion of the reaction, the resulting mixture was extracted with EtOAc (2 x 50 mL). The combined organic layers were dried over anhydrous Na₂SO₄. After filtration, the filtrate was concentrated under reduced pressure. The residue was purified by combi-flash (EtOAc/PE = 50%) to give the product (613 mg, 37% yield). ¹H NMR (400 MHz, Chloroform-d) δ 7.36 (s, 1H), 6.75 (s, 1H), 3.70-3.62 (m, 2H), 3.59-3.50 (m, 1H), 3.40 (s, 3H), 3.40-3.31 (m, 2H), 3.22 (s, 3H), 1.98-1.88 (m, 2H), 1.84 -1.69 (m, 2H). LCMS (ESI) m/e [M+1]⁺ = 305.

### Example BB65: Synthesis of 2-chloro-4-(1,4-dioxan-2-yl)-6-methanesulfonylpyridine, R or S 2-chloro-4-(1,4-dioxan-2-yl)-6-methanesulfonylpyridine

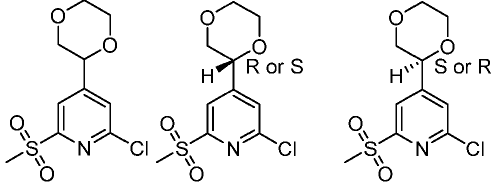

### Step 1: Synthesis of 2,6-dichloro-4-ethenylpyridine

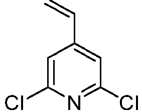

Into a 1000-mL round-bottom flask purged and maintained with an inert atmosphere of nitrogen, were placed 2,6-dichloro-4-iodopyridine (20.00 g, 73.02 mmol), dioxane (200 mL), H₂O (100 mL), 2-ethenyl-4,4,5,5-tetramethyl-1,3,2-dioxaborolane (13.50 g, 87.65 mmol), Pd(dppf)Cl₂ (5.34 g, 7.30 mmol) and CsF (33.28 g, 219.07 mmol). The resulting solution was stirred for 2 h at 90 °C. The resulting solution was extracted with EtOAc (200 mL x 3). The resulting mixture was concentrated under vacuum. The residue was purified by combi-flash (EtOAc/PE = 0.1%) to give the product (10.90 g, 86%). LCMS (ESI) m/e [M+1]⁺ = 174.

### Step 2: Synthesis of 1-(2,6-dichloropyridin-4-yl) ethane-1,2-diol

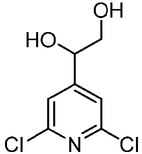

Into a 500-mL round-bottom flask, were placed 2,6-dichloro-4-ethenylpyridine (10.90 g, 62.63 mmol), acetone (110 mL), H₂O (55 mL), NMO (8.07 g, 68.90 mmol) and OsO₄ (7.95 mL, 1 g/mL, 313.20 mmol). The resulting solution was stirred for 4 h at rt. The reaction was then quenched by the addition of 500 mg of florisil. The resulting solution was extracted with EtOAc (3 x 100 mL). The resulting mixture was concentrated under vacuum. The residue was purified by combi-flash (EtOAc/PE = 32%) to give the product (3.20 g, 24.56%). LCMS (ESI) m/e [M+1]⁺ = 208.

### Step 3: Synthesis of 2,6-dichloro-4-(1,4-dioxan-2-yl) pyridine

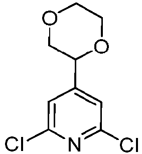

Into a 1000-mL round-bottom flask, were placed 1-(2,6-dichloropyridin-4-yl) ethane-1,2-diol (3.00 g, 14.42 mmol), ethylene dichloride (250 mL) and TBAB (1.29 g, 4.00 mmol). This was followed by the addition of NaOH (1 M aq., 50 mL) dropwise with stirring at 0 °C. The resulting solution was stirred for 48 h at 35 °C in an oil bath. The resulting solution was extracted with EtOAc (3 x 150 mL). The resulting mixture was concentrated under vacuum. The residue was purified by combi-flash (EtOAc/PE = 7.90%) to give the product (1.45 g, 43%). LCMS (ESI) m/e [M+1]⁺ = 234.

### Step 4: Synthesis of 2-chloro-4-(1,4-dioxan-2-yl)-6-(methylsulfanyl)pyridine

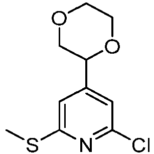

A mixture of 2,6-dichloro-4-(1,4-dioxan-2-yl) pyridine (1.45 g, 6.19 mmol), DMF (17 mL) and (methylsulfanyl)sodium (0.52 g, 7.42 mmol) was stirred for overnight at rt. Water was added and the resulting solution was extracted with EtOAc (3 x 50 mL). The resulting mixture was concentrated under vacuum. The residue was purified by combi-flash (EtOAc/PE = 0.6%-5.3%) to give the product (1.48 g, 97%). LCMS (ESI) m/e [M+1]⁺ = 246.

### Step 5: Synthesis of 2-chloro-4-(1,4-dioxan-2-yl)-6-(methylsulfonyl)pyridine and (R or S)-2-chloro-4-(1,4-dioxan-2-yl)-6-(methylsulfonyl)pyridine

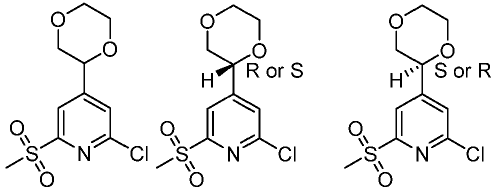

Into a 100-mL 3-necked round-bottom flask, were placed 2-chloro-4-(1,4-dioxan-2-yl)-6-(methylsulfanyl)pyridine (1.48 g, 6.02 mmol), THF (12 mL) and H₂O (12 mL). This was followed by the addition of NaIO₄ (3.86 g, 18.06 mmol) in portions with stirring at 0 °C. To this was added RuCl₃·H₂O (0.20 g, 0.90 mmol) with stirring at 0 °C. The resulting solution was stirred for 1 h at rt. The resulting solution was extracted with EtOAc (3 x 50 mL) and the combined organic layer was concentrated under vacuum. The residue was purified by combi-flash (EtOAc/PE = 15-22%) to give the racemic product (1.10 g). The racemic product was purified by Prep-SFC with the following conditions (Column: CHIRALPAK AD-3, 3.0 x 50 mm, 3 µm; Mobile Phase B: MeOH (0.1%DEA); Flow rate: 2 mL/min; Gradient: isocratic 10% B; Wave Length: 220 nm) to give the product.

First peak (434.90 mg, 26.00%, RT₁: 0.959 min): ¹H NMR (300 MHz, DMSO-*d*₆) δ 8.06 (s, 1H), 7.90 (s, 1H), 4.86 - 4.83 (m, 1H), 4.15 - 3.92 (m, 2H), 3.87-3.73 (m, 2H), 3.61 - 3.59 (m, 1H), 3.33 (s, 4H). LCMS (ESI) m/e [M+1]⁺ = 278.
Second peak (413.3 mg, 24.71%, RT₂: 1.605 min): ¹H NMR (300 MHz, DMSO-*d*₆) δ 8.05 (s, 1H), 7.89 (s, 1H), 4.85 - 4.82 (m, J = 9.9 Hz, 1H), 4.14 - 3.92 (m, 2H), 3.86 - 3.73 (m, 2H), 3.60 - 3.57 (m, 1H), 3.33 - 3.25 (m, 4H). LCMS (ESI) m/e [M+1]⁺ = 278.

### Example BB66: Synthesis of 1-(2-bromo-6-(methylsulfonyl)pyridin-4-yl)-4-methylpiperidin-4-ol

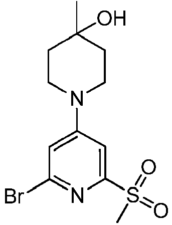

### Step 1: 1-(2,6-dibromopyridin-4-yl)-4-methylpiperidin-4-ol

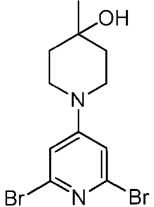

To a solution of 2,6-dibromo-4-nitropyridine (2.0 g, 7.1 mmol) in DMSO (30 mL) was added K₂CO₃ (2.0 g, 14.2 mmol) and 4-methylpiperidin-4-ol (899 mg, 7.8 mmol) at 0 °C under N₂. The mixture was stirred for 2 hr at 25°C under N₂. The mixture was poured into water (20 mL) and extracted with EtOAc (30 mL, 20 mL). The combined organic phase was washed with brine (50 mL, 30 mL), dried over Na₂SO₄, filtered and concentrated to give the residue which was purified by column chromatography (SiO₂, PE/EA = 100/1 to 0/1). Compound of 1-(2,6-dibromopyridin-4-yl)-4-methylpiperidin-4-ol to give the product (1.4 g, 56% yiled) was obtained as a gray solid. LCMS (ESI) m/e [M+1]⁺ = 350.8.

### Step 2: 1-(2-bromo-6-(methylthio)pyridin-4-yl)-4-methylpiperidin-4-ol

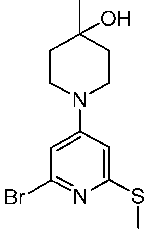

To a solution of 1-(2,6-dibromopyridin-4-yl)-4-methylpiperidin-4-ol (1.4 g, 4.0 mmol) in DMF (20 mL) was added sodium methanethiolate (336 mg, 4.8 mmol) at 0 °C under N₂. The reaction mixture was stirred for 3 hr at 25 °C under N₂. The mixture was poured into water (10 mL) and extracted with EtOAc (30 mL, 20 mL). The combined organic phase was washed with brine (30 mL, 20 mL), dried over Na₂SO₄, filtered and concentrated to give the residue. The crude product was purified by column chromatography (SiO₂, PE : EA = 100/1 to 0/1). Compound of 1-(2-bromo-6-(methylthio)pyridin-4-yl)-4-methylpiperidin-4-ol (1.4 g, 95% yield) was obtained as a light yellow solid. LCMS (ESI) m/e [M+1]⁺ = 319.

### Step 3: 1-(2-bromo-6-(methylsulfonyl)pyridin-4-yl)-4-methylpiperidin-4-ol

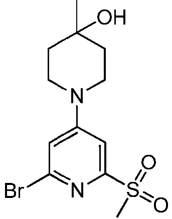

To a solution of 1-(2-bromo-6-(methylthio)pyridin-4-yl)-4-methylpiperidin-4-ol (1.1 g, 8.7 mmol) in H₂O/MeOH (1/1, 30 mL) was added oxone (4.3 g, 6.9 mmol) at 25 °C under N₂. The reaction mixture was stirred for 5 hr at 25 °C under N₂. The mixture was filtered to give the filtrate, which was poured into Na₂SO₃ aqueous solution (20 mL) and extracted with EtOAc (30 mL, 20 mL). The combined organic phase was washed with brine (10 mL), dried over Na₂SO₄, filtered and concentrated to give the residue. The crude was purified by column chromatography (SiO₂, PE : EA = 100/1 to 0/1). Compound of 1-(2-bromo-6-(methylsulfonyl)pyridin-4-yl)-4-methylpiperidin-4-ol (412 mg, 34% yield) was obtained as a white solid. ¹H NMR (DMSO) δ 7.30-7.31 (d, *J =* 2.4, 1H) 7.21-7.22 (d, *J =* 2.0, 1H) 4.48 (s, 1H) 3.5-3.7 (m, 2H) 3.31-3.34 (m, 2H) 3.21 (s, 3H) 1.51-1.52 (m, 4H) 1.14 (s, 3H). LCMS (ESI) m/e [M+1]⁺ = 349.

### Example BB67: Synthesis of 2-chloro-4-((trans)-3-methoxycyclobutyl)-6-(methylsulfonyl)pyridine and 2-chloro-4-((cis)-3-methoxycyclobutyl)-6-(methylsulfonyl)pyridine

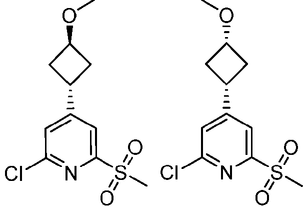

### Step 1: Synthesis of 3-hydroxycyclobutane-1-carbonitrile

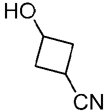

A solution of 3-oxocyclobutane-1-carbonitrile (5 g, 52.57 mmol) in MeOH was treated with NaBH₄ (2.98 g, 78.86 mmol) for 2h at rt under nitrogen atmosphere. The reaction was quenched with water/ice at 0 °C. The resulting mixture was extracted with EtOAc (3 x 80 mL). The combined organic layers were washed with water (3 x 50 mL), dried over anhydrous Na₂SO₄. After filtration, the filtrate was concentrated under reduced pressure. The crude product was used in the next step directly without further purification.

### Step 2: Synthesis of 3-(benzyloxy)cyclobutane-1-carbonitrile

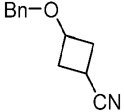

To a solution of 3-hydroxycyclobutane-1-carbonitrile (3.90 g, 40.15 mmol) in DMF (40mL) was added NaH (2.01 g, 52.20 mmol, 60% wt) at 0 °C. The mixture was stirred for 15 min. BnBr (8.24 g, 48.18 mmol) was added and the mixture was allowed to warm to rt and stirred at rt for 2 h. The resulting mixture was extracted with EtOAc (3 x 80 mL). The combined organic layers were washed with brine (3 x 40 mL), dried over anhydrous Na₂SO₄. After filtration, the filtrate was concentrated under reduced pressure. The residue was purified by combi-flash (EtOAc/PE = 0-20%) to give the product (7.01 g, 93% yield). ¹H NMR (400 MHz, Chloroform-d) δ 7.43-7.34 (m, 2H), 7.34 (m, 3H), 4.45 (s, 2H), 4.01 (m, 1H), 2.74 - 2.63 (m, 2H), 2.67 - 2.56 (m, 1H), 2.49 - 2.38 (m, 1H), 2.38 (m, 1H).

### Step 3: Synthesis of 3-(benzyloxy)-1-(2,6-dichloropyridin-4-yl)cyclobutane-1-carbonitrile

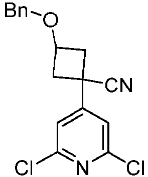

To a stirred mixture of 2,4,6-trichloropyridine (3g, 16.44 mmol) and 3-(benzyloxy)cyclobutane-1-carbonitrile (3.08 g, 16.44 mmol) in THF (60 mL) was added LiHMDS (23.02 mL, 23.02 mmol, 1M in THF) dropwise portions at -10°C under nitrogen atmosphere. The resulting mixture was stirred for 2 h at rt under nitrogen atmosphere. The reaction was quenched with water/ice at 0 °C and the resulting mixture was extracted with EtOAc (3 x 80 mL). The combined organic layers were washed with water (3 x 60 mL), dried over anhydrous Na₂SO₄. After filtration, the filtrate was concentrated under reduced pressure. The residue was purified by combi-flash (EtOAc/PE = 0-20%) to give the product (4.5 g, 82% yield). LCMS (ESI) m/e [M+1]⁺= 333.

### Step 4: Synthesis of 3-(benzyloxy)-1-(2,6-dichloropyridin-4-yl)cyclobutane-1-carboxylic acid

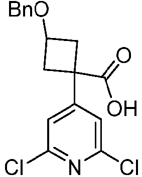

A solution of 3-(benzyloxy)-1-(2,6-dichloropyridin-4-yl)cyclobutane-1-carbonitrile (4.50 g, 13.50 mmol) in H₂O (50mL) was treated with KOH (2.27 g, 40.51 mmol) for 2 h at 105 °C. The resulting mixture was concentrated under reduced pressure. The crude product was used in the next step directly without further purification. LCMS (ESI) m/e [M+1]⁺= 352.

### Step 5: Synthesis of 4-[3-(benzyloxy)cyclobutyl]-2,6-dichloropyridine

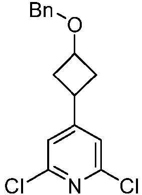

A solution of 3-(benzyloxy)-1-(2,6-dichloropyridin-4-yl)cyclobutane-1-carboxylic acid (4.50 g, 12.77 mmol) in pyridine (40 mL) was treated with Py-HCl (1.47 g, 12.77 mmol) for 2 h at 90 °C. The resulting mixture was concentrated under reduced pressure. The resulting mixture was extracted with EtOAc (3 x 80 mL). The combined organic layers were washed with brine (3 x 50 mL), dried over anhydrous Na₂SO₄. After filtration, the filtrate was concentrated under reduced pressure. The residue was purified by combi-flash (EtOAc/PE = 0-40%) to give the product (2.2 g, 55% yield). LCMS (ESI) m/e [M+1]⁺= 308.

### Step 6: Synthesis of 3-(2,6-dichloropyridin-4-yl)cyclobutan-1-ol

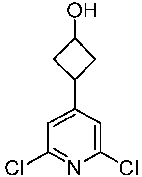

A solution of 4-[3-(benzyloxy)cyclobutyl]-2,6-dichloropyridine (2.20 g, 7.13 mmol) in HCl (12 N) (20 mL) was stirred 2 h at 50 °C. The resulting mixture was extracted with EtOAc (3 x 50 mL). The combined organic layers were washed with water (3 x 30 mL), dried over anhydrous Na₂SO₄. After filtration, the filtrate was concentrated under reduced pressure. The residue was purified by combi-flash (PE/EtOAc = 50%) to give the product (880 mg, 56% yield). LCMS (ESI) m/e [M+1]⁺= 218.

### Step 7: Synthesis of 2,6-dichloro-4-(3-methoxycyclobutyl)pyridine

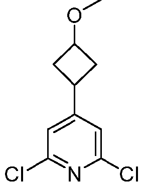

To a solution of 3-(2,6-dichloropyridin-4-yl)cyclobutan-1-ol (830 mg, 3.80 mmol) in DMF was added NaH (182 mg, 4.56 mmol, 60% wt) at 0 °C. The mixture was stirred for 15 min at this temperature and then CH₃I (810 mg, 5.70 mmol) was added and the mixture was allowed to warm to rt and stirred for 2 h. The reaction was quenched with water/ice at 0 °C. The resulting mixture was extracted with EtOAc (3 x 30 mL). The combined organic layers were washed with water (3 x 20 mL), dried over anhydrous Na₂SO₄. After filtration, the filtrate was concentrated under reduced pressure. The residue was purified by Prep-TLC (PE/EtOAc = 5:1) to give the product (520 mg, 58% yield). LCMS (ESI) m/e [M+1]⁺= 232.

### Step 8: Synthesis of 2-chloro-4-(3-methoxycyclobutyl)-6-(methylsulfanyl)pyridine

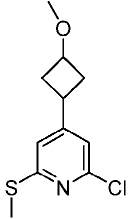

A mixture of 2,6-dichloro-4-(3-methoxycyclobutyl)pyridine (520 mg, 2.24 mmol) and (methylsulfanyl)sodium (235 mg, 3.36 mmol) in DMF (6 mL) was stirred for 2 h at rt. The resulting mixture was extracted with EtOAc (3 x 30 mL). The combined organic layers were washed with water (3 x 20 mL), dried over anhydrous Na₂SO₄. After filtration, the filtrate was concentrated under reduced pressure. The crude product was used in the next step directly without further purification. LCMS (ESI) m/e [M+1]⁺= 244.

### Step 9: Synthesis of 2-chloro-6-methanesulfonyl-4-(3-methoxycyclobutyl)pyridine; 2-chloro-4-((trans)-3-methoxycyclobutyl)-6-(methylsulfonyl)pyridine; 2-chloro-4-((cis)-3-methoxycyclobutyl)-6-(methylsulfonyl)pyridine

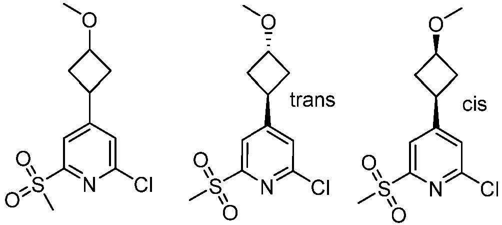

A solution of 2-chloro-4-(3-methoxycyclobutyl)-6-(methylsulfanyl)pyridine (520 mg, 2.13 mmol) in THF (10 mL) was treated with NaIO₄ (912.61 mg, 4.26 mmol) for 2 h at 0 °C under nitrogen atmosphere followed by the addition of RuCl₃.H₂O (24.05 mg, 0.10 mmol) in H₂O (5 mL) dropwise portions at 0 °C. The resulting mixture was extracted with EtOAc (3 x 30 mL). The combined organic layers were washed with water (3 x 30 mL), dried over anhydrous Na₂SO₄. After filtration, the filtrate was concentrated under reduced pressure. The residue was purified by Prep-TLC (PE/EtOAc = 3:1) to give the product (268 mg, 45% yield). ¹H NMR (400 MHz, DMSO-*d*₆) δ 7.92 - 7.81 (s, 1H), 7.77 (s, 1H), 3.88 (tt, *J =* 7.7, 6.6 Hz, 1H), 3.30 (s, 3H), 3.34 - 3.20 (m, 1H), 3.17 (s, 3H), 2.70 (m, 2H), 1.98 (m, 2H). LCMS (ESI) m/e [M+1]⁺ = 276.

The racemic compound was separated by ACHIRAL-SFC (Column: DAICEL DCpak P4VP, 3 x 25 cm, 5 µm; Mobile Phase A: CO₂, Mobile Phase B: IPA (0.5% 2M NH₃-MeOH); Flow rate: 60 mL/min; Gradient: isocratic 15% B; Column Temperature: 35 °C; Back Pressure: 100 bar; Wave Length: 254 nm;;) to give the trans product (179 mg, RT₁: 4.78 min). ¹H NMR (300 MHz, DMSO-*d*₆) δ 7.94 - 7.88 (m, 1H), 7.84 (s, 1H), 4.12 - 3.98 (m, 1H), 3.76 (p, *J* = 7.9 Hz, 1H), 3.31 (s, 3H), 3.19 (s, 3H), 2.51 - 2.33 (m, 4H). LCMS (ESI) m/e [M+1]⁺= 276.

Cis product (1.31 g, RT₂: 5.28 min): ¹H NMR (300 MHz, DMSO-*d*₆) δ 7.91 - 7.86 (m, 1H), 7.86 - 7.74 (m, 1H), 3.97 - 3.81 (m, 1H), 3.31 (s, 3H), 3.32 - 3.20 (m, 1H), 3.18 (s, 3H), 2.71 (m, 2H), 2.08 - 1.90 (m, 2H). LCMS (ESI) m/e [M+1]⁺= 276.

### Example A1: Synthesis of N-(4-((3-(methylsulfonyl)phenyl)amino)-5-(pyridazin-3-yl)pyridin-2-yl)acetamide

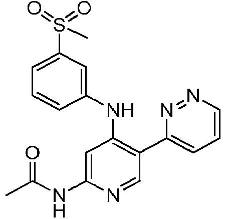

### Step 1: 4-chloro-5-(4,4,5,5-tetramethyl-1,3,2-dioxaborolan-2-yl)pyridin-2-amine

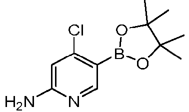

A mixture of 5-bromo-4-chloropyridin-2-amine (1.0 g, 4.8 mmol), 4,4,4',4',5,5,5',5'-octamethyl-2,2'-bi(1,3,2-dioxaborolane) (1.3 g, 5.3 mmol), KOAc (1.4 g, 14.5 mmol) and Pd(dppf)Cl₂-CH₂Cl₂ (197 mg, 0.2 mmol) in 1,4-dioxane (20 mL) was stirred at 115 °C under N₂ for 6 h. After cooled to room temperature, the mixture was diluted with H₂O (30 mL) and the resulting solution was extracted with EA (30 mL x 3). The combined organic layers were dried over anhydrous Na₂SO₄ and concentrated under vacuum. The crude product was purified by silica gel column chromatography (PE/EA = 1:1 to 0:1) to give 4-chloro-5-(4,4,5,5-tetramethyl-1,3,2-dioxaborolan-2-yl)pyridin-2-amine (1.5 g, crude). MS (ESI) m/e [M+1]⁺ 173.

### Step 2: 4-chloro-5-(pyridazin-3-yl)pyridin-2-amine

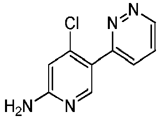

A mixture of 4-chloro-5-(4,4,5,5-tetramethyl-1,3,2-dioxaborolan-2-yl)pyridin-2-amine (478 mg, 1.9 mmol), 3-bromopyridazine (100 mg, 0.6 mmol), Na₂CO₃ (133 mg, 1.3 mmol) and Pd(dppf)Cl₂ (44 mg, 0.06 mmol) in CH₃CN (10 mL) and H₂O (2 mL) was stirred at 120 °C for 10 mins in microwave reactor. After cooled to room temperature, the mixture was diluted with H₂O (20 mL) and the resulting solution was extracted with EA (30 mL x 3). The combined organic layers were dried over anhydrous Na₂SO₄ and concentrated under vacuum. The crude product was purified by silica gel column chromatography (PE/EA = 1: 1 to 0:1) to give 4-chloro-5-(pyridazin-3-yl)pyridin-2-amine (51 mg, 39.4%). MS (ESI) m/e [M+1]⁺ 207.

### Step 3: N-(4-chloro-5-(pyridazin-3-yl)pyridin-2-yl)acetamide

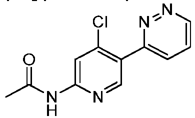

To a solution of *N*-(4-chloro-5-(pyridazin-3-yl)pyridin-2-yl)acetamide (40 mg, 0.2 mmol) in pyridine (2 mL) was added acetyl chloride (17 mg, 0.2 mmol) dropwise at 0 °C. Then the mixture was stirred at RT for 2 h. Upon completion of the reaction, the mixture was concentrate and the residue was diluted with water (5 mL), then the mixture was extracted with EA (10 mL x 3) and citric acid (10 mL), dried over anhydrous Na₂SO₄, concentrated under vacuum to give *N*-(4-chloro-5-(pyridazin-3-yl)pyridin-2-yl)acetamide (45 mg, 93%). (ESI) m/e [M+1]⁺ 249.

### Step 4: N-(4-((3-(methylsulfonyl)phenyl)amino)-5-(pyridazin-3-yl)pyridin-2-yl)acetamide

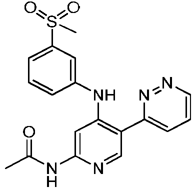

A mixture of *N*-(4-chloro-5-(pyridazin-3-yl)pyridin-2-yl)acetamide (20.0 mg, 0.08 mmol), 3-(methylsulfonyl)aniline (27.0 mg, 0.16 mmol), Cs₂CO₃ (78 mg, 0.24 mmol), Xantphos (5 mg, 8 µmol) and Pd₂(dba)₃ (7 mg, 8 µmol) in 1,4-dioxane (2 mL) was stirred at 110 °C for 12 h under nitrogen atmosphere. After cooled to room temperature, the mixture was diluted with H₂O (20 mL) and the resulting solution was extracted with EA (30 mL x 3). The combined organic layers were dried over anhydrous Na₂SO₄ and concentrated under vacuum. The crude product was purified by Prep-HPLC (column: Phenomenex Gemini-NX 150 x 30mm x 5um; phase: A-H₂O (10mM NH₄HCO₃); B-ACN; B%: 15%-35% in 20min) to give *N*-(4-((3-(methylsulfonyl) phenyl)amino)-5-(pyridazin-3-yl)pyridin-2-yl)acetamide (1.8 mg, 6%). ¹H NMR (400 MHz, MeOD-*d₄*) δ 9.32 (d, *J* = 4.4 Hz, 1H), 8.59 (s, 1H), 8.36 (d, *J =* 8.8 Hz, 1H), 8.05 (s, 1H), 8.03 - 7.96 (m, 2H), 7.86 - 7.78 (m, 2H), 6.70 (s, 1H), 3.20 (s, 3H), 2.24 (s, 3H). MS (ESI) m/e [M+1]⁺ 384.

### Example A2: Synthesis of N-[4-[(3-methanesulfonylphenyl)amino]-5-(oxolan-2-yl)pyridin-2-yl]acetamide

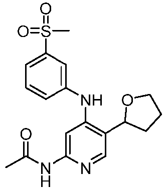

### Step 1: 5-Bromo-2-chloro-N-(3-methanesulfonylphenyl)pyridin-4-amine

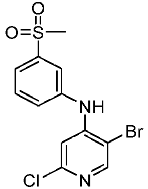

A mixture of 5-bromo-2-chloro-4-iodopyridine (3.00 g, 9.42 mmol), Cs₂CO₃ (6.14 g, 18.848 mmol), Xantphos (1.09 g, 1.88 mmol), 3-methanesulfonylaniline (1.77 g, 10.37 mmol), Pd₂(dba)₃ (0.86 g, 0.942 mmol) in 1,4-dioxane (30 mL) was stirred for overnight at 100 °C under nitrogen atmosphere. After cooled to room temperature, the mixture was diluted with H₂O (20 mL) and the resulting solution was extracted with EA (80 mL x 3). The resulting mixture was washed with brine (10 mL), dried over anhydrous sodium sulfate. The resulting mixture was concentrated under vacuum and the residue was purified by combi-flash (EA/PE = 0-15%) to give 5-Bromo-2-chloro-N-(3-methanesulfonylphenyl)pyridin-4-amine (1.6 g, 46.95% yield). LCMS (ESI) m/e [M+1]⁺ 362.

### Step 2: 5-bromo-N²-(4-methoxybenzyl)-N⁴-(3-(methylsulfonyl)phenyl)pyridine-2,4-diamine

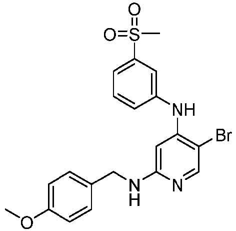

Into a 20-mL sealed tube, were placed 5-bromo-2-chloro-N-(3-methanesulfonylphenyl)pyridin-4-amine (1.5 g, 4.148mmol) and PMBNH₂ (5 mL). The resulting solution was stirred overnight at 135 °C. After cooled to room temperature, the residue was purified by prep-HPLC (column: Phenomenex Gemini-NX; phase: A-H₂O (0.05% TFA); B-Acetonitrile, B%: 40%-60% in 20 min) to give 5-bromo-N2-(4-methoxybenzyl)-N4-(3-(methylsulfonyl)phenyl)pyridine-2,4-diamine (600 mg, 31%). LCMS (ESI) m/e [M+1]⁺ 462.

### Step 3: 5-(furan-2-yl)-N⁴-(3-methanesulfonylphenyl)-N²-[(4-methoxyphenyl)methyl]pyridine-2,4-diamine

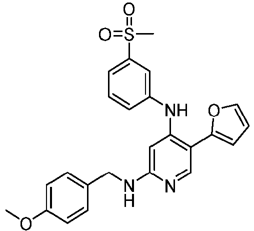

A mixture of 5-bromo-N4-(3-methanesulfonylphenyl)-N2-[(4-methoxyphenyl)methyl]pyridine-2,4-diamine (420 mg, 0.91 mmol), K₂CO₃ (251 mg, 1.82 mmol), furan-2-ylboronic acid (152 mg, 1.36 mmol) and Pd(PPh₃)₄ (105 mg, 0.091 mmol) in 1,4-dioxane (4.00 mL) and H₂O (0.80 mL) was stirred for 2 h at 100 °C under nitrogen atmosphere. After cooled to room temperature, the mixture was diluted with H₂O (20 mL) and the resulting solution was extracted with EA (80 mL x 3). The combined organic layer was washed with 100 mL of brine, dried over anhydrous sodium sulfate. The resulting mixture was concentrated under vacuum and the residue was purified by Prep-TLC (MeOH/DCM = 1: 15) to give 5-(furan-2-yl)-N⁴-(3-methanesulfonylphenyl)-N²-[(4-methoxyphenyl)methyl]pyri dine-2,4-diamine (270 mg, 66%). LCMS (ESI) m/e [M+1]⁺ 450.

### Step 4: N²-(4-methoxybenzyl)-N4-(3-(methylsulfonyl)phenyl)-5-(tetrahydrofuran-2-yl)pyridine-2,4-diamine

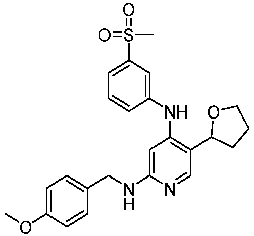

Into a 20-mL pressure tank reactor were placed 5-(furan-2-yl)-N4-(3-methanesulfonylphenyl)-N2-[(4-methoxyphenyl)methyl]pyridine-2,4-diamine (270 mg, 0.60 mmol), i-PrOH (5 mL), hydrochloric acid (12 M, 0.05 mL) and Pd(OH)₂/C (10%, 168 mg), the resulting solution was stirred overnight at 65 °C under hydrogen atmosphere (10 atm). After cooled to room temperature, the mixture was diluted with H₂O (20 mL) and the resulting solution was extracted with EA (50 mL x 3). The reaction mixture was cooled to rt with a water bath. The resulting solution was extracted with EA (50 mL x 3) and the organic layers combined. The combined organic layers were washed with 100 mL of brine, dried over anhydrous sodium sulfate. The resulting mixture was concentrated under vacuum and the residue was purified by Prep-TLC (MeOH/DCM = 1: 12) to give N²-(4-methoxybenzyl)-N⁴-(3-(methylsulfonyl)phenyl)-5-(tetrahydrofuran-2-yl) pyridine-2,4-diamine (80 mg, 29%). LCMS (ESI) m/e [M+1]⁺ 454.

### Step 5: N⁴-(3-(methylsulfonyl)phenyl)-5-(tetrahydrofuran-2-yl)pyridine-2,4-diamine

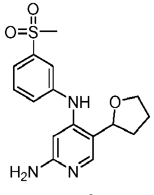

A mixture of N⁴-(3-methanesulfonylphenyl)-N²-[(4-methoxyphenyl)methyl]-5-(oxolan-2-yl)pyridine-2,4-diamine (80 mg) and TFA (2 mL) was stirred for 1 h at 70 °C. After cooled to room temperature, the solvent was removed in vacuo and the residue was purified by prep-HPLC (column: Phenomenex Gemini-NX; phase: A-H₂O (0.05% TFA); B-Acetonitrile, B%: 13%-20% in 20 min) to give N⁴-(3-(methylsulfonyl)phenyl)-5-(tetrahydrofuran-2-yl)pyridine-2,4-diamine (50 mg, 85%). LCMS (ESI) m/e [M+1]⁺ 334.

### Step 6: N-[4-[(3-methanesulfonylphenyl)amino]-5-(oxolan-2-yl)pyridin-2-yl]acetamide

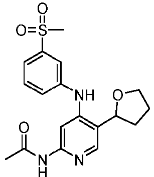

A mixture of N4-(3-methanesulfonylphenyl)-5-(oxolan-2-yl)-1,6-dihydropyridine-2,4-diamine (50 mg, 0.15 mmol), pyridine (1 mL) and acetic anhydride (12 mg, 0.12 mmol) was stirred for 1 h at 80 °C. After cooled to room temperature, the solvent was removed in vacuo and the residue was diluted with 20 mL of water. The resulting solution was extracted with EA (30 mL x 3) and the combined organic layers were washed with 50 mL of brine, dried over anhydrous sodium sulfate. The solvent was removed in vacuo and the residue was purified by prep-HPLC (column: Phenomenex Gemini-NX; phase: A-H₂O (0.1% FA); B-Acetonitrile, B%: 35%-55% in 8 min) to give N-[4-[(3-methanesulfonylphenyl)amino]-5-(oxolan-2-yl)pyridin-2-yl]acetamide (1.8 mg, 3%). ¹H NMR (400 MHz, DMSO-*d*₆) δ 10.34 (s, 1H), 8.20 *(d, J =* 6.7 Hz, 1H), 8.11 (s, 1H), 8.00 (s, 1H), 7.73 (s, 1H), 7.60 (d, *J* = 8.9 Hz, 1H), 7.58 - 7.50 (m, 2H), 5.08 - 5.03 (m, 1H), 4.10 - 4.05 (m, 1H), 3.82 - 3.78 (m, 1H), 3.35 (s, 3H), 2.38 - 2.34 (m, 1H), 2.03 (s, 3H), 2.00 - 1.95 (m, 2H), 1.78 - 1.72 (m, 1H). MS (ES, *m*/*z*): [M+H]⁺ 376.

### Example A3: Synthesis of N-(5-(1H-imidazol-4-yl)-4-((6-(methylsulfonyl)pyridin-2-yl)amino)pyridin-2-yl)acetamide

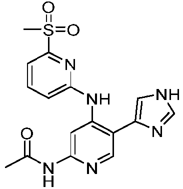

### Step 1: 4-iodo-1-((2-(trimethylsilyl)ethoxy)methyl)-1H-imidazole and 5-iodo-1-((2-(trimethylsilyl) ethoxy)methyl)-1H-imidazole and 5-iodo-1-((2-(trimethylsilyl)ethoxy)methyl)-1H-imidazole

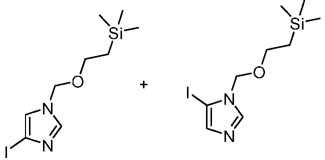

To a solution of 5-iodo-1*H*-imidazole (2 g, 10.3 mmol) in THF (15 mL) was added NaH (60% in mineral oil, 454 mg, 11.3 mmol) in portions at 0 °C. The resulting mixture was stirred for 30 mins at 0 °C. Then added a solution of (2-(chloromethoxy)ethyl)trimethylsilane (1.72 g, 10.8 mmol) in THF (5 mL) dropwise with stirring at 0 °C. The reaction was warmed to room temperature with stirring for 1 h. The reaction was quenched by saturated NH₄Cl at 0 °C and extracted with EA. The organic layer was washed with brine, dried over Na₂SO₄, concentrated to give the residue and purification by silica gel column with EA/DCM (1: 1) to afford a mixture of 4-iodo-1-((2-(trimethylsilyl)ethoxy)methyl)-1*H*-imidazole and 5-iodo-1-((2-(trimethylsilyl)ethoxy)methyl)-1*H*-imidazole (2.66 g, 79.6%). MS (ESI) m/e [M+1]⁺ 325.

### Step 2: 2-chloro-5-(1-((2-(trimethylsilyl)ethoxy)methyl)-1H-imidazol-4-yl)pyridin-4-amine and 2-chloro-5-(1-((2-(trimethylsilyl)ethoxy)methyl)-1H-imidazol-5-yl)pyridin-4-amine

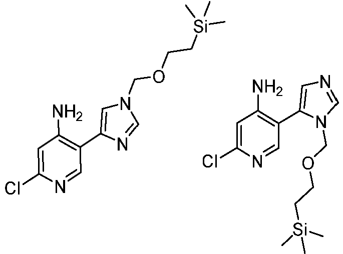

A mixture of 2-chloro-5-(1-((2-(trimethylsilyl)ethoxy)methyl)-1*H*-imidazol-4-yl)pyridin-4-amine and 2-chloro-5-(1-((2-(trimethylsilyl)ethoxy)methyl)-1H-imidazol-5-yl)pyridin-4-amine (966 mg, 4.98 mmol), 2-chloro-5-(4,4,5,5-tetramethyl-1,3,2-dioxaborolan-2-yl)pyridin-4-amine (1.39 g, 5.48 mmol), Pd(dppf)Cl₂ (182 mg, 0.25 mmol) and K₂CO₃ (1.37 g, 9.96 mmol) in 1,4-dioxane (18 mL) and H₂O (3 mL) was charged with nitrogen and heated to 90 °C stirred for 1 h. The reaction was cooled to room temperature and diluted with EA, washed with brine, dried and concentrated. The residue was applied onto a silica gel column with EA/PE (1:2) to afford the product (862 mg, 89.2%). MS (ESI) m/e [M+1]⁺ 325.

### Step 3: N-(2-chloro-5-(1-((2-(trimethylsilyl)ethoxy)methyl)-1H-imidazol-4-yl)pyridin-4-yl)-6-(methylsulfonyl)pyridin-2-amine and N-(2-chloro-5-(1-((2-(trimethylsilyl)ethoxy)methyl)-1H-imidazol-5-yl)pyridin-4-yl)-6-(methylsulfonyl)pyridin-2-amine

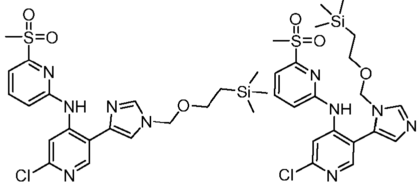

A mixture of 2-chloro-5-(1-((2-(trimethylsilyl)ethoxy)methyl)-1*H*-imidazol-4-yl)pyridin-4-amine and 2-chloro-5-(1-((2-(trimethylsilyl)ethoxy)methyl)-1H-imidazol-5-yl)pyridin-4-amine (412 mg, 1.27 mmol), 2-bromo-6-(methylsulfonyl)pyridine (358 mg, 1.5 mmol), Pd₂dba₃ (58 mg, 0.06 mmol), BINAP (118 mg, 0.19 mmol) and Cs₂CO₃ (829 mg, 2.5 mmol) in1,4-dioxane (10 mL) was heated to 130 °C and stirred at this temperature for 3 h under nitrogen atmosphere. The reaction was cooled to room temperature, filtered and the filtration was concentrated under vacuum. The residue was applied onto Prep-TLC with DCM/MeOH (30 : 1) to afford the product (139 mg, 22.9%). MS (ESI) m/e [M+1]⁺ 480.

### Step 4: N-(4-((6-(methylsulfonyl)pyridin-2-yl)amino)-5-(1-((2-(trimethylsilyl)ethoxy)methyl)-1H-imidazol-4-yl)pyridin-2-yl)acetamide and N-(4-((6-(methylsulfonyl)pyridin-2-yl)amino)-5-(1-((2-(trimethylsilyl)ethoxy)methyl)-1H-imidazol-5-yl)pyridin-2-yl)acetamide

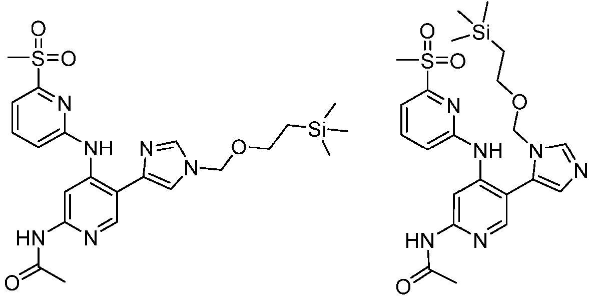

A mixture of *N*-(2-chloro-5-(1-((2-(trimethylsilyl)ethoxy)methyl)-1*H*-imidazol-4-yl)pyridin-4-yl)-6-(methylsulfonyl)pyridin-2-amine and N-(2-chloro-5-(1-((2-(trimethylsilyl)ethoxy)methyl)-1H-imidazol-5-yl)pyridin-4-yl)-6-(methylsulfonyl)pyridin-2-amine (139 mg, 0.29 mmol), acetamide (51 mg, 0.87 mmol), Pd₂dba₃ (26.6 mg, 0.03 mmol), Xantphos (50 mg, 0.09 mmol) and Cs₂CO₃ (189 mg, 0.58 mmol) in 1,4-dioxane (4 mL) was heated at 130 °C for 3 h under nitrogen atmosphere. The reaction was cooled to room temperature, filtered and the filtration was concentrated under vacuum. The residue was applied onto a silica gel column with DCM/MeOH (25: 1) to afford the product (94 mg, 64.5%). MS (ESI) m/e [M+1]⁺ 502.

### Step 5: N-(5-(1H-imidazol-4-yl)-4-((6-(methylsulfonyl)pyridin-2-yl)amino)pyridin-2-yl)acetamide

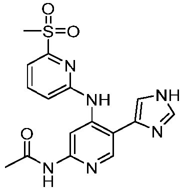

A solution of *N*-(4-((6-(methylsulfonyl)pyridin-2-yl)amino)-5-(1-((2-(trimethylsilyl)ethoxy)methyl)-1*H*-imidazol-4-yl)pyridin-2-yl)acetamide and N-(4-((6-(methylsulfonyl)pyridin-2-yl)amino)-5-(1-((2-(trimethylsilyl)ethoxy)methyl)-1H-imidazol-5-yl)pyridin-2-yl)acetamide (94 mg, 0.19 mmol) in TFA (5 mL) was heated to 50 °C stirred for 1 h. Then TFA was removed under vacuum and the residue was treated with NH₃ in MeOH (4 mL, 7M). The solvent was removed under vacuum and the residue was applied onto a C¹⁸ column with CH₃CN/water to afford *N*-(5-(1*H*-imidazol-4-yl)-4-((6-(methylsulfonyl)pyridin-2-yl)amino)pyridin-2-yl)acetamide (31.04 mg, 43.9%). ¹H NMR (400 MHz, DMSO-*d*₆) δ 12.71 (s, 1H), 12.67 (s, 1H), 10.38 (s, 1H), 9.21 (s, 1H), 8.63 (s, 1H), 8.04 - 7.93 (m, 2H), 7.88 (s, 1H), 7.54 (d, *J =* 8.2 Hz, 1H), 7.23 (d, *J* = 8.2 Hz, 1H), 3.49 (s, 3H), 2.10 (s, 3H). MS (ESI) m/e [M+1]⁺ 373.

### Example A25: synthesis of N-(4-((4-(cyclopropylmethoxy)-6-(methylsulfonyl)pyridin-2-yl)amino)-5-(6-methoxypyridazin-3-yl)pyridin-2-yl)acetamide

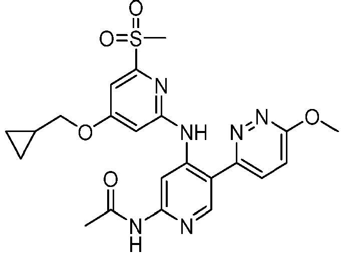

A mixture of N-(4-amino-5-(6-methoxypyridazin-3-yl)pyridin-2-yl)acetamide (50 mg, 0.19 mmol), 2-bromo-4-(cyclopropylmethoxy)-6-(methylsulfonyl)pyridine (71 mg, 0.23 mmol), Pd₂(dba)₃ (17 mg, 0.019 mmol), BINAP ( 12 mg, 0.019 mmol) and Cs₂CO₃ (186 mg, 0.57 mmol) in dioxane (5 mL) was stirred for 16 h at 120 °C under nitrogen atmosphere. After cooled to room temperature, the resulting mixture was filtered. The filtrate was concentrated under reduced pressure. The residue was purified by combi-flash (MeOH/DCM = 0- 10%) to give the product (0.84 mg, 22%). ¹H NMR (400 MHz, DMSO-*d*₆) δ 11.39 (s, 1H), 10.58 (s, 1H), 8.96 (s, 1H), 8.65 (s, 1H), 8.25 (d, *J =* 8.0 Hz, 1H), 7.41 (d, *J* = 8.0 Hz, 1H), 7.10 (s, 1H), 6.82 (s, 1H), 4.11 (s, 3H), 4.06 - 4.02 (m, 2H), 3.37 (s, 3H), 2.12 (s, 3H), 1.26 - 1.24 (m, 1H), 0.61 - 0.60 (m, 2H), 0.38 - 0.37 (m, 2H). MS (ESI) m/e [M+1]⁺ 485.

### Example A26: synthesis of N-(4-((4-isopropoxy-6-(methylsulfonyl)pyridin-2-yl)amino)-5-(6-methoxypyridazin-3-yl)pyridin-2-yl)acetamide

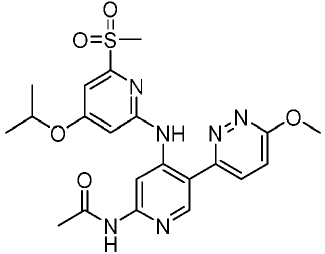

A mixture of N-(4-amino-5-(6-methoxypyridazin-3-yl)pyridin-2-yl)acetamide (50 mg, 0.19 mmol), 2-bromo-4-isopropoxy-6-(methylsulfonyl)pyridine (67 mg, 0.23 mmol), Pd₂(dba)₃ (17 mg, 0.019 mmol), BINAP ( 12 mg, 0.019 mmol) and Cs₂CO₃ (186 mg, 0.57 mmol) in dioxane (5 mL) was stirred for 16 h at 120 °C under nitrogen atmosphere. The mixture was allowed to cool down to room temperature. The resulting mixture was filtered. The filtrate was concentrated under reduced pressure. The residue was purified by combi-flash (MeOH/DCM = 0- 10%) to give the product (4.20 mg, 4%). ¹H NMR (400 MHz, DMSO-*d*₆) δ 11.25 (s, 1H), 10.52 (s, 1H), 8.91 (s, 1H), 8.59 (s, 1H), 8.19 (d*, J =* 8.0 Hz, 1H), 7.36 (d*, J=* 8.0 Hz, 1H), 7.02 (s, 1H), 6.74 (s, 1H), 4.95 - 4.66 (m, 1H), 4.07 (s, 3H), 3.32 (s, 3H), 2.07 (s, 3H), 1.28 (d, *J=* 4.0 Hz, 6H). MS (ESI) m/e [M+1]⁺ 473.

The following Examples were prepared in a similar manner to the product Example A3:

| **Example** | **Compound** | **Chemical Name** | **¹H NMR and LC /MS *m*/*z* (M+1)** |
|---|---|---|---|
| A4 | 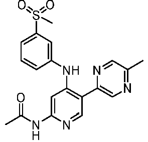 | N-(5-(5-methylpyrazin-2-yl)-4-((3-(methylsulfonyl)phenyl)amino)pyridin-2-yl)acetamide | ¹H NMR (400 MHz, CD₃OD) δ: 9.00 (s, 1 H), 8.60 (s, 1 H), 8.58 (s, 1 H), 8.16 (s, 1 H), 7.88 (s, 1 H), 7.69 - 7.56 (m, 3 H), 3.20 (s, 3 H), 2.58 (s, 3 H), 2.12 (s, 3 H). MS (ESI) m/e [M+1]⁺ 398. |
| A5 | 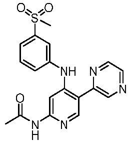 | N-(4-((3-(methylsulfonyl)phenyl)amino)-5-(pyrazin-2-yl)pyridin-2-yl)acetamide | ¹H NMR (400 MHz, DMSO-d6) δ 10.55 (s, 1H), 10.43 (s, 1H), 9.25 (s, 1H), 8.74 - 8.68 (m, 2H), 8.61 (s, 1H), 8.17 (s, 1H), 7.80 (s, 1H), 7.66 - 7.58 (m, 3H), 3.27 (s, 3H), 2.07 (s, 3H). MS (ESI) m/e [M+1]⁺ 384. |
| A6 | 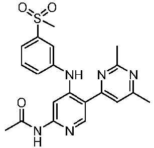 | N-(5-(2,6-dimethylpyrimidin-4-yl)-4-((3-(methylsulfonyl)phenyl)amino)pyridin-2-yl)acetamide | ¹H NMR (400 MHz, DMSO-d6) δ 11.73 (s, 1H), 10.60 (s, 1H), 8.82 (s, 1H), 8.19 (s, 1H), 7.90 - 7.85 (m, 2H), 7.74 - 7.58 (m, 3H), 3.33 (s, 3H), 3.30 (s, 3H), 2.68 (s, 3H), 2.07 (s, 3H). MS (ESI) m/e [M+1]⁺ 412. |
| A7 | 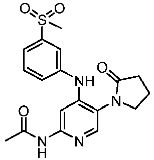 | N-(4-((3-(methylsulfonyl)phenyl)amino)-5-(2-oxopyrrolidin-1-yl)pyridin-2-yl)acetamide | ¹H NMR (300 MHz, DMSO-*d*₆) δ 10.40 (s, 1H), 8.45 (s, 1H), 8.05 - 8.00 (m, 2H), 7.75 - 7.70 (m, 1H), 7.68 - 7.47 (m, 3H), 3.65 - 3.60 (m, 2H), 2.45 (s, 3H), 2.10 - 2.05 (m, 2H), 2.06 (s, 3H). 2.05 - 2.00 (m, 2H), MS (ESI) m/e [M+1]⁺ 389. |
| A8 | 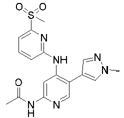 | N-(5-(1-methyl-1H-pyrazol-4-yl)-4-((6-(methylsulfonyl)pyridin-2-yl)amino)pyridin-2-yl)acetamide | ¹H NMR (400 MHz, DMSO-*d*₆) δ 10.42 (s, 1H), 8.91 (s, 1H), 8.71 (s, 1H), 8.21 (s, 1H), 8.02 (s, 1H), 7.95 - 7.85 (m, 1H), 7.69 (s, 1H), 7.50 - 7.40 (m, 1H), 7.35 - 7.25 (m, 1H), 3.86 (s, 3H), 3.28 (s, 3H), 2.06 (s, 3H). MS (ESI) m/e [M+1]⁺ 387. |
| A9 | 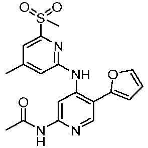 | N-(5-(furan-2-yl)-4-((4-methyl-6-(methylsulfonyl)pyridin-2-yl)amino)pyridin-2-yl)acetamide | ¹H NMR (400 MHz, DMSO-*d*₆) δ 10.52 (s, 1H), 9.06 (s, 1H), 8.74 (s, 1H), 8.44 (s, 1H), 7.77 (d, *J =* 0.7 Hz, 1H), 7.38 - 7.33 (m, 1H), 7.17 - 7.11 (m, 1H), 6.78 (d, *J* = 3.4 Hz, 1H), 6.61 (d, *J =* 3.4 Hz, 1H), 3.24 (s, 3H), 2.35 (s, 3H), 2.07 (s, 3H). MS (ESI) m/e [M+1]⁺ 387. |
| A10 | 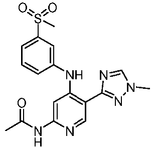 | N-(5-(1-methyl-1H-1,2,4-triazol-3-yl)-4-((3-(methylsulfonyl)phenyl)amino)pyridin-2-yl)acetamide | ¹H NMR (400 MHz, CDCl₃) δ = 10.16 (s, 1H), 8.98 (s, 1H), 8.25 (s, 1H), 8.12 (s, 2H), 7.97 (s, 1H), 7.71 (d, *J =* 7.2 Hz, 1H), 7.66 - 7.58 (m, 2H), 4.03 (s, 3H), 3.21 (s, 3H), 2.17 (s, 3H). MS (ESI) m/e [M+1]⁺ 387. |
| A11 | 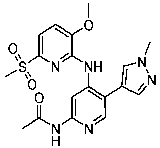 | N-(4-((3-methoxy-6-(methylsulfonyl)pyridin-2-yl)amino)-5-(1-methyl-1H-pyrazol-4-yl)pyridin-2-yl)acetamide | ¹H NMR (400 MHz, DMSO-*d*₆) δ 10.45 (s, 1H), 9.25 (s, 1H), 8.19 (s, 1H), 8.15 (s, 1H), 8.12 (s, 1H), 7.77 (s, 1H), 7.58 (d, *J* = 8.2 Hz, 1H), 7.51 (d, *J* = 8.2 Hz, 1H), 3.95 (s, 3H), 3.92 (s, 3H), 3.43 (s, 3H), 2.11 (s, 3H). MS (ESI) m/e [M+1]⁺ 417. |
| A12 | 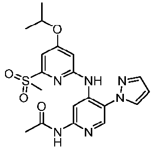 | N-(4-((4-isopropoxy-6-(methylsulfonyl)pyridin-2-yl)amino)-5-(1H-pyrazol-1-yl)pyridin-2-yl)acetamide | ¹H NMR (400 MHz, DMSO-*d*₆) δ 10.59 (s, 1H), 9.65 (s, 1H), 9.02 (s, 1H), 8.34 (s, 1H), 8.30 (d, *J =* 2.4 Hz, 1H), 7.90 (s, 1H), 7.03 (d, *J =* 2.4 Hz, 1H), 6.85 (s, 1H), 6.70 - 6.60 (m, 1H), 4.81 (s, 1H), 3.37 (s, 3H), 2.11 (s, 3H), 1.31 (d, *J =* 6.0 Hz, 6H). MS (ESI) m/e [M+1]⁺ 431. |
| A13 | 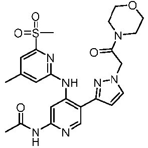 | N-(4-((4-methyl-6-(methylsulfonyl)pyridin-2-yl)amino)-5-(1-(2-morpholino-2-oxoethyl)-1H-pyrazol-3-yl)pyridin-2-yl)acetamide | ¹H NMR (400 MHz, DMSO-d₆) δ 11.44 (s, 1H), 10.50 (s, 1H), 9.26 (s, 1H), 8.63 (s, 1H), 7.89 (d, *J =* 4.0 Hz, 1H), 7.46 (s, 1H), 7.16 (s, 1H), 6.93 (d, *J =* 4.0 Hz, 1H), 5.38 (s, 2H), 3.68 - 3.64 (m, 2H), 3.60 - 3.55 (m, 4H), 3.54 - 3.50 (m, 2H), 3.48 (s, 3H), 2.42 (s, 3H), 2.12 (s, 3H). MS (ESI) m/e [M+1]⁺ 514. |
| A14 | 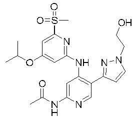 | N-(5-(1-(2-hydroxyethyl)-1H-pyrazol-3-yl)-4-((4-isopropoxy-6-(methylsulfonyl)pyridin-2-yl)amino)pyridin-2-yl)acetamide | ¹H NMR (400 MHz, DMSO-*d*₆) δ 11.43 (s, 1H), 10.42 (s, 1H), 9.19 (s, 1H), 8.62 (s, 1H), 7.90 (d, *J =* 2.3 Hz, 1H), 7.06 (s, 1H), 6.91 (d, *J =* 2.3 Hz, 1H), 6.74 (s, 1H), 5.00 (s, 1H), 4.90 - 4.83 (m, 1H), 4.36 - 4.29 (m, 2H), 3.90 - 3.83 (m, 2H), 3.46 (s, 3H), 2.11 (s, 3H), 1.34 (d, *J =* 6.0 Hz, 6H). MS (ESI) m/e [M+1]⁺ 475. |
| A15 | 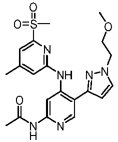 | N-(5-(1-(2-methoxyethyl)-1H-pyrazol-3-yl)-4-((4-methyl-6-(methylsulfonyl)pyridin-2-yl)amino)pyridin-2-yl)acetamide | ¹H NMR (400 MHz, DMSO-*d*₆) δ 11.57 (s, 1H), 10.54 (s, 1H), 9.21 (s, 1H), 8.65 (s, 1H), 7.92 (d, *J =* 4.1 Hz, 1H), 7.47 (s, 1H), 7.17 (s, 1H), 6.95 (d, *J =* 4.1 Hz, 1H), 4.49 - 4.45 (m, 2H), 3.80 - 3.75 (m, 2H), 3.47 (s, 3H), 3.28 (s, 3H), 2.45 (s, 3H), 2.12 (s, 3H). MS (ESI) m/e [M+1]⁺ 445. |
| A16 | 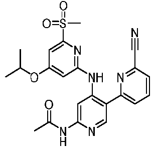 | N-(6-cyano-4'-((4-isopropoxy-6-(methylsulfonyl)pyridin-2-yl)amino)-[2,3'-bipyridin]-6'-yl)acetamide | ¹H NMR (400 MHz, DMSO-*d*₆) δ 11.03 (s, 1H), 10.61 (s, 1H), 9.04 (s, 1H), 8.68 (s, 1H), 8.28 (d, *J =* 8.4 Hz, 1H), 8.20 - 8.15 (m, 1H), 8.04 (d, *J* = 8.4 Hz, 1H), 7.16 - 7.04 (m, 1H), 6.75 - 6.70 (m, 1H), 4.80 - 4.46 (m, 1H), 3.39 (s, 3H), 2.13 (s, 3H), 1.34 (d, *J =* 5.9 Hz, 6H). MS (ESI) m/e [M+1]⁺ 467. |
| A17 | 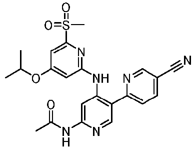 | N-(5-cyano-4'-((4-isopropoxy-6-(methylsulfonyl)pyridin-2-yl)amino)-[2,3'-bipyridin]-6'-yl)acetamide | ¹H NMR (400 MHz, DMSO-*d*₆) δ 11.92 (s, 1H), 10.57 (s, 1H), 9.18 (s, 1H), 9.10 (s, 1H), 8.77 (s, 1H), 8.39 (d, *J =* 8.6 Hz, 1H), 8.22 (d, *J =* 8.6 Hz, 1H), 7.04 (s, 1H), 6.84 (s, 1H), 4.89 - 4.83 (m, 1H), 3.37 (s, 3H), 2.08 (s, 3H), 1.29 (d, *J* = 5.9 Hz, 6H). MS (ESI) m/e [M+1]⁺ 467. |
| A18 | 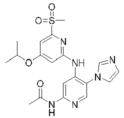 | N-(5-(1H-imidazol-1-yl)-4-((4-isopropoxy-6-(methylsulfonyl)pyridin-2-yl)amino)pyridin-2-yl)acetamide | ¹H NMR (400 MHz, DMSO-*d*₆) δ 10.59 (s, 1H), 8.91 (s, 1H), 8.83 (s, 1H), 8.13 (s, 1H), 7.94 (d, *J =* 1.9 Hz, 1H), 7.42 (s, 1H), 7.14 (s, 1H), 7.00 (d, *J =* 1.9 Hz, 1H), 6.86 (s, 1H), 4.73 - 4.67 (m, 1H), 3.33 (s, 3H), 2.10 (s, 3H), 1.30 (d, *J =* 6.0 Hz, 6H). MS (ESI) m/e [M+1]⁺ 431. |
| A19 | 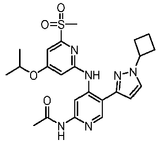 | N-(5-(1-cyclobutyl-1H-pyrazol-3-yl)-4-((4-isopropoxy-6-(methylsulfonyl)pyridin-2-yl)amino)pyridin-2-yl)acetamide | ¹H NMR (400 MHz, DMSO-*d*₆) δ 11.49 (s, 1H), 10.50 (s, 1H), 9.12 (s, 1H), 8.66 (s, 1H), 8.00 (d*, J =* 2.2 Hz, 1H), 7.07 (s, 1H), 6.95 *(d, J =* 2.2 Hz, 1H), 6.72 (s, 1H), 5.07 - 4.96 (m, 1H), 4.95 - 4.79 (m, 1H), 3.45 (s, 5H), 2.71 - 2.53 (m, 2H), 2.50 - 2.42 (m, 3H), 2.12 (s, 3H), 1.91 - 1.81 (m, 2H), 1.35 (d, *J =* 5.9 Hz, 6H). MS (ESI) m/e [M+1]⁺ 485. |
| A20 | 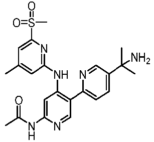 | N-(5-(2-aminopropan-2-yl)-4'-((4-methyl-6-(methylsulfonyl)pyridin-2-yl)amino)-[2,3'-bipyridin]-6'-yl)acetamide | ¹H NMR (400 MHz, DMSO-*d*₆) δ 12.88 (s, 1H), 10.54 (s, 1H), 9.23 (s, 1H), 8.99 - 8.95 (m, 1H), 8.85 (s, 1H), 8.45 - 8.40 (m, 2H), 8.26 - 8.18 (m, 2H), 7.46 (s, 1H), 7.24 (s, 1H), 3.45 (s, 3H), 2.44 (s, 3H), 2.13 (s, 3H), 1.73 (s, 6H). MS (ESI) m/e [M+1]⁺ 455. |
| A21 | 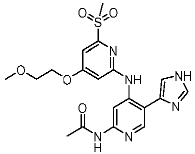 | N-(5-(1H-imidazol-4-yl)-4-((4-(2-methoxyethoxy)-6-(methylsulfonyl)pyridin-2-yl)amino)pyridin-2-yl)acetamide | MS (ESI) m/e [M+1]⁺ 447. |
| A22 | 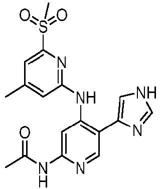 | N-(5-(1H-imidazol-4-yl)-4-((4-methyl-6-(methylsulfonyl)pyridin-2-yl)amino)pyridin-2-yl)acetamide | MS (ESI) m/e [M+1]⁺ 387. |
| A23 | 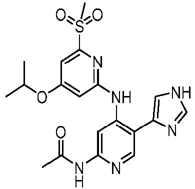 | N-(5-(1H-imidazol-4-yl)-4-((4-isopropoxy-6-(methylsulfonyl)pyridin-2-yl)amino)pyridin-2-yl)acetamide | MS (ESI) m/e [M+1]⁺ 431. |
| A24 | 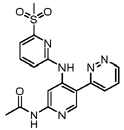 | N-(4-((6-(methylsulfonyl)pyridin-2-yl)amino)-5-(pyridazin-3-yl)pyridin-2-yl)acetamide | Molecular Weight: 384 |
| A27 | 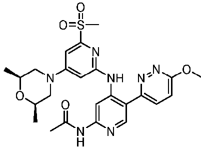 | N-(4-((4-((2S,6R)-2,6-dimethylmorpholino)-6-(methylsulfonyl)pyridin-2-yl)amino)-5-(6-methoxypyridazin-3-yl)pyridin-2-yl)acetamide | Molecular Weight: 528 |
| A28 | 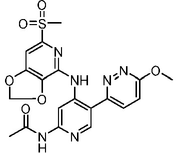 | N-(5-(6-methoxypyridazin-3-yl)-4-((6-(methylsulfonyl)-[1,3]dioxolo[4,5-c]pyridin-4-yl)amino)pyridin-2-yl)acetamide | Molecular Weight: 459 |
| A29 | 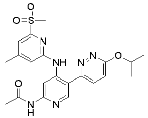 | N-(5-(6-isopropoxypyridazin-3-yl)-4-((4-methyl-6-(methylsulfonyl)pyridin-2-yl)amino)pyridin-2-yl)acetamide | Molecular Weight:457 |
| A30 | 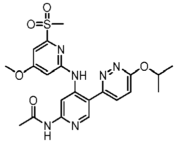 | N-(5-(6-isopropoxypyridazin-3-yl)-4-((4-methoxy-6-(methylsulfonyl)pyridin-2-yl)amino)pyridin-2-yl)acetamide | Molecular Weight: 473 |
| A31 | 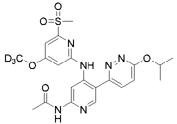 | N-(5-(6-isopropoxypyridazin-3-yl)-4-(4-(methoxy-d3)-6-(methylsulfonyl)pyridin-2-ylamino)pyridin-2-yl)acetamide | Molecular Weight: 476 |

### Example B1: Synthesis of N-(4'-((3-(methylsulfonyl)phenyl)amino)-[2,3'-bipyridin]-6'-yl)acetamide

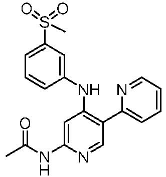

### Step 1: tert-butyl (5-bromo-2-chloropyridin-4-yl)carbamate

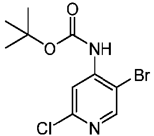

To a mixture of 5-bromo-2-chloropyridin-4-amine (30 g, 144.6 mmol), DMAP (1.8 g, 14.4 mmol), TEA (43.9 g, 434.0 mmol) in DCM (300 mL) was added Boc₂O (38.0 g, 173.0 mmol) dropwise at room temperature and the resulting mixture was stirred at this temperature for 12 h. Upon completion of the reaction, the solvent was removed in vacuo and the residue was purified by silica gel column chromatography (PE/EA = 20:1 to 5:1) to give tert-butyl (5-bromo-2-chloropyridin-4-yl)carbamate (40.0 g, 90.2% yield). ¹H NMR (400 MHz, CDCl₃) δ 8.35 (s, 1H), 8.25 (s, 1H), 7.19 (s, 1H), 1.56 (s, 9H). MS (ESI) m/e [M+1]⁺ 307.

### Step 2: tert-butyl (6'-chloro-[2,3'-bipyridin]-4'-yl)carbamate

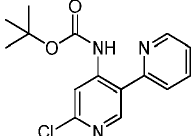

A mixture of tert-butyl (5-bromo-2-chloropyridin-4-yl)carbamate (5.0 g, 16.3 mmol), 2-bromopyridine (2.8 g, 17.9 mmol), Pd(PPh₃)₂Cl₂ (1.1 g, 1.6 mmol), Pd(PPh₃)₄ (1.9 g, 1.6 mmol) and Sn₂Me₆ (8.0 g, 24.0 mmol) in dioxane (50 mL) was stirred at 110 °C for 12 h under nitrogen atmosphere. After cooled to room temperature, the mixture was diluted with KF-H₂O (50 mL) and extracted with EA (50 mL x 3). The combined organic layers were dried over anhydrous Na₂SO₄ and concentrated under vacuum. The residue was purified by silica gel column chromatography (PE/EA = 20:1 to 10: 1) to give tert-butyl (6'-chloro-[2,3'-bipyridin]-4'-yl)carbamate (880 mg, 18.0%). MS (ESI) m/e [M+1]⁺ 306.

### Step 3: tert-butyl (6'-acetamido-[2,3'-bipyridin]-4'-yl)carbamate

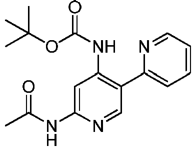

A mixture of tert-butyl (6'-chloro-[2,3'-bipyridin]-4'-yl)carbamate (2.0 g, 6.5 mmol), acetamide (773 mg, 13.1 mmol), Cs₂CO₃ (6.3 g, 19.5 mmol), Xant-Phos (753 mg, 1.3 mmol) and Pd₂(dba)₃ (595 mg, 0.65 mmol) in dioxane (20 mL) was stirred at 110 °C for 12 h under nitrogen atmosphere. After cooled to room temperature, the solvent was removed in vacuo and the residue was diluted with water (20 mL). The resulting solution was extracted with EA (30 mL x 3) and the combined organic layers were washed with 50 mL of brine, dried over anhydrous sodium sulfate. The solvent was removed in vacuo and the residue was purified by silica gel column chromatography (PE/EA = 3:1 to 1:1) to give tert-butyl (6'-acetamido-[2,3'-bipyridin]-4'-yl)carbamate (1.3 g, 61.0%). ¹H NMR (400 MHz, DMSO-*d*₆) δ 12.02 (s, 1H), 10.50 (s, 1H), 8.98 (s, 1H), 8.73 (s, 1H), 8.68 - 8.63 (m, 1H), 8.10 - 8.05 (m, 1H), 7.98 (m, 1H), 7.45 -7.40 (m, 1H), 2.11 (s, 3H), 1.50 - 1.47 (m, 9H). MS (ESI) m/e [M+1]⁺ 329.

### Step 4: N-(4'-amino-[2,3'-bipyridin]-6'-yl)acetamide

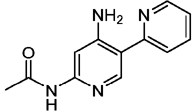

A mixture of tert-butyl (6'-acetamido-[2,3'-bipyridin]-4'-yl)carbamate (4.0 g, 12.2 mmol) in TFA (20 mL) and DCM (20 mL) was stirred at room temperature for 3h. Upon completion of the reaction, the solvent was removed in vacuo and the residue diluted with water. NaHCO₃ (40 mL) was added to adjust the pH value to 9 and the resulting solution was extracted with EA (40 mL x 3). The combined organic layers were dried over anhydrous Na₂SO₄ and concentrated in vacuo to give *N*-(4'-amino-[2,3'-bipyridin]-6'-yl)acetamide (2.7 g, 97.1%). ¹H NMR (400 MHz, DMSO-*d*₆) δ 10.35 (s, 1H), 8.60 (d*, J =* 4.9 Hz, 1H), 8.42 (s, 1H), 7.90 - 7.86 (m, 2H), 7.38 (s, 1H), 6.88 (s, 2H), 7.33 - 7.28 (m, 1H), 2.09 (s, 3H). MS (ESI) m/e [M+1]⁺ 229.

### Step 5: N-(4'-((3-(methylsulfonyl)phenyl)amino)-[2,3'-bipyridin]-6'-yl)acetamide

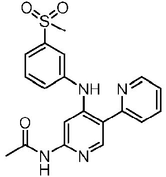

A mixture of N-(4'-amino-[2,3'-bipyridin]-6'-yl)acetamide (120 mg, 0.53 mmol), 1-bromo-3-(methyl sulfonyl)benzene (120 mg, 0.5 mmol), Pd₂dba₃ (80 mg, 0.09 mmol), Xant-Phos (60 mg, 0.1 mmol) and Cs₂CO₃ (300 mg, 0.92 mmol) in dioxane (6 mL) was stirred at 130 °C under N₂ in a sealed tube for 3 h. The reaction mixture was filtered and the solid was washed with EA (10 mL). The filtrate was concentrated and the residue was purified by prep-TLC (DCM/MeOH = 20: 1) to give N-(4'-((3-(methylsulfonyl)phenyl)amino)-[2,3'-bipyridin]-6'-yl)acetamide (24 mg, 12%). ¹H NMR (400 MHz, DMSO-*d*₆) δ 11.52 (s, 1H), 10.50 (s, 1H), 8.74 - 8.66 (m, 2H), 8.18 (s, 1H), 8.07 (d, *J =* 8.2 Hz, 1H), 7.99 - 7.90 (m, 1H), 7.83 (s, 1H), 7.67 - 7.57 (m, 3H), 7.45 - 7.37 (m, 1H), 3.29 (s, 3H), 2.07 (s, 3H). MS (ESI) m/e [M+1]⁺ 383.

The following Examples were prepared in a similar manner to the product Example B 1:

| **Example** | **Compound** | **Chemical Name** | **¹H NMR and LC /MS *m*/*z* (M+1)** |
|---|---|---|---|
| B2 | 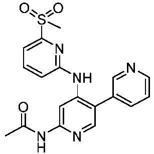 | N-(4-((6-(methylsulfonyl)pyridin-2-yl)amino)-[3,3'-bipyridin]-6-yl)acetamide | ¹H NMR (400 MHz, DMSO-*d*₆) δ 10.54 (s, 1H), 9.09 (s, 1H), 8.77 (s, 1H), 8.64 (s, 1H), 8.57 - 8.55 (m, 1H), 8.19 - 8.17 (m, 1H), 7.88 - 7.84 (m, 2H), 7.42 (d, *J* = 8.0 Hz, 2H), 7.17 (d, *J* = 8 Hz, 1H), 3.28 (s, 3H), 2.08 (s, 3H). MS (ESI) m/e [M+1]⁺ 384. |
| B3 | 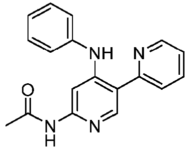 | N-(4'-(phenylamino)-[2,3'-bipyridin]-6'-yl)acetamide | ¹H NMR (400 MHz, DMSO-*d*₆) δ 11.41 (s, 1H), 10.36 (s, 1H), 8.75 - 8.65 (m, 1H), 8.65 (s, 1H), 8.10 - 8.05 (m, 2H), 8.00 - 7.90 (m, 1H), 7.42 - 7.34 (m, 2H), 7.31 (s, 2H), 7.29 (s, 1H), 7.15 - 7.10 (m, 1H), 2.05 (s, 3H). MS (ESI) m/e [M+1]⁺ 383. |
| B4 | 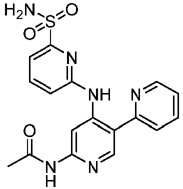 | N-(4'-((6-sulfamoylpyridin-2-yl)amino)-[2,3'-bipyridin]-6'-yl)acetamide | ¹H NMR (400 MHz, DMSO-*d*₆) δ 13.09 (s, 1H), 10.74 (s, 1H), 9.70 (s, 1H), 8.85 - 8.80 (m, 2H), 8.20 - 8.15 (m, 1H), 8.01 -7.91 (m, 2H), 7.50 - 7.45 (m, 2H), 7.38 (s, 1H), 7.35 (s, 2H), 2.18 (s, 3H). MS (ESI) m/e [M+1]⁺ 385. |
| B5 | 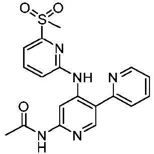 | N-(4'-((6-(methylsulfonyl)pyridin-2-yl)amino)-[2,3'-bipyridin]-6'-yl)acetamide | ¹H NMR (400 MHz, DMSO-*d*₆) δ 12.97 (s, 1H), 10.57 (s, 1H), 9.24 (s, 1H), 8.90 - 8.80 (m, 2H), 8.27 (s, 1H), 8.15 - 8.10 (m, 1H), 8.05 - 7.95 (m, 2H), 7.60 - 7.50 (m, 1H), 7.48 - 7.35 (m, 2H), 3.47 (s, 3H), 2.13 (s, 3H). MS (ESI) m/e [M+1]⁺ 384. |
| B6 | 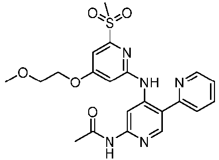 | N-(4'-((4-(2-methoxyethoxy)-6-(methylsulfonyl)pyridin-2-yl)amino)-[2,3'-bipyridin]-6'-yl)acetamide | ¹H NMR (400 MHz, DMSO-*d*₆) δ 12.71 (s, 1H), 10.54 (s, 1H), 9.15 (s, 1H), 8.82 (d, *J* = 3.6 Hz, 1H), 8.77 (s, 1H), 8.11 (d, *J* = 7.8 Hz, 1H), 7.99 (t, *J* = 7.7 Hz, 1H), 7.50 - 7.42 (m, 1H), 7.11 (s, 1H), 6.91 (s, 1H), 4.38 - 4.30 (m, 2H), 3.74 - 3.66 (m, 2H), 3.44 (s, 3H), 3.32 (s, 3H), 2.12 (s, 3H). MS (ESI) m/e [M+1]⁺ 458. |
| B7 | 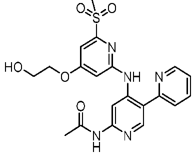 | N-(4'-((4-(2-hydroxyethoxy)-6-(methylsulfonyl)pyridin-2-yl)amino)-[2,3'-bipyridin]-6'-yl)acetamide | ¹H NMR (400 MHz, DMSO-*d*₆) δ 12.67 (s, 1H), 10.54 (s, 1H), 9.15 (s, 1H), 8.82 - 8.81 (m, 1H), 8.76 (s, 1H), 8.10 (d, *J =* 7.9 Hz, 1H), 7.99 (t*, J =* 7.3 Hz, 1H), 7.48 - 7.42 (m, 1H), 7.11 (s, 1H), 6.90 (s, 1H), 5.02 - 4.95 (m, 1H), 4.26 - 4.19 (m, 2H), 3.79 - 3.72 (m, 2H), 3.44 (s, 3H), 2.12 (s, 3H). MS (ESI) m/e [M+1]⁺ 444. |
| B8 | 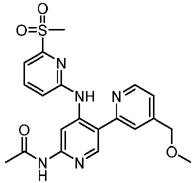 | N-(4-(methoxymethyl)-4'-((6-(methylsulfonyl)pyridin-2-yl)amino)-[2,3'-bipyridin]-6'-yl)acetamide | ¹H NMR (400 MHz, DMSO-*d*₆) δ 12.94 (s, 1H), 10.58 (s, 1H), 9.22 (s, 1H), 8.76 - 8.72 (m, 2H), 7.99 - 7.95 (m, 2H), 7.55 - 7.53 (m, 1H), 7.46 - 7.30 (m, 2H), 4.55 (s, 2H), 3.45 (s, 3H), 3.36 (s, 3H), 2.11 (s, 3H). MS (ESI) m/e [M+1]⁺ 428. |
| B9 | 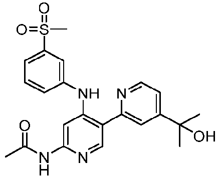 | N-(4-(2-hydroxypropan-2-yl)-4'-((3-(methylsulfonyl)phenyl)amino)-[2,3'-bipyridin]-6'-yl)acetamide | ¹H NMR (400 MHz, DMSO-*d*₆) δ 11.52 (s, 1H), 10.53 (s, 1H), 8.69 (s, 1H), 8.63 (d, *J* = 4.5 Hz, 1H), 8.18 (s, 1H), 8.03 (s, 1H), 7.81 (s, 1H), 7.63 - 7.58 (m, 3H), 7.48, (d, *J =* 4.5 Hz, 1H), 5.36 (s, 1H), 3.28 (s, 3H), 2.07 (s, 3H), 1.48 (s, 6H). MS (ESI) m/e [M+1]⁺ 441. |
| B10 | 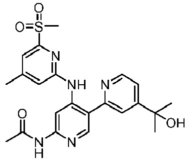 | N-(4-(2-hydroxypropan-2-yl)-4'-((4-methyl-6-(methylsulfonyl)pyridin-2-yl)amino)-[2,3'-bipyridin]-6'-yl)acetamide | ¹H NMR (400 MHz, DMSO-*d*₆) δ 12.89 (s, 1H), 10.56 (s, 1H), 9.24 (s, 1H), 8.78 (s, 1H), 8.72 (d, *J =* 4.5 Hz, 1H), 8.08 (s, 1H), 7.54 (d, *J* = 4.5 Hz, 1H), 7.43 (s, 1H), 7.22 (s, 1H), 5.39 (s, 1H), 3.45 (s, 3H), 2.42 (s, 3H), 2.13 (s, 3H), 1.50 (s, 6H). MS (ESI) m/e [M+1]⁺ 456. |
| B11 | 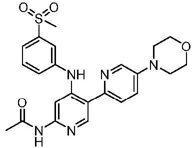 | N-(4'-((3-(methylsulfonyl)phenyl)amino)-5-morpholino-[2,3'-bipyridin]-6'-yl)acetamide | ¹H NMR (400 MHz, DMSO-*d*₆) δ 11.61 (s, 1H), 10.50 (s, 1H), 8.61 (s, 1H), 8.42 (s, 1H), 8.16 - 8.03 (m, 1H), 7.93 (d*, J* = 9.0 Hz, 1H), 7.80 (s, 1H), 7.69 - 7.51 (m, 4H), 3.81 - 3.73 (m, 4H), 3.29 (s, 3H), 3.27 - 3.22 (m, 4H), 2.07 (s, 3H). MS (ESI) m/e [M+1]⁺ 468. |
| B12 | 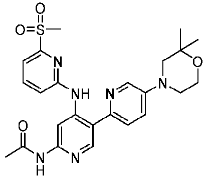 | *N-*(5-(2,2-dimethylmorpholino)-4'-((6-(methylsulfonyl)pyridin-2-yl)amino)-[2,3'-bipyridin]-6'-yl)acetamide | ¹H NMR (400 MHz, DMSO-*d*₆) *δ* 13.16 (s, 1H), 10.49 (s, 1H), 9.19 (s, 1H), 8.69 (s, 1H), 8.47 (s, 1H), 8.19 - 7.85 (m, 2H), 7.52 (d, *J* = 7.4 Hz, 2H), 7.35 (d, *J =* 8.4 Hz, 1H), 3.78 (d, *J =* 3.5 Hz, 2H), 3.46 (s, 3H), 3.22 (s, 2H), 3.12 (s, 2H), 2.09 (s, 3H), 1.23 (s, 6H). MS (ESI) m/e [M+1]⁺ 497. |
| B13 | 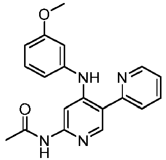 | N-(4'-((3-methoxyphenyl)amino)-[2,3'-bipyridin]-6'-yl)acetamide | ¹H NMR (400 MHz, DMSO-*d*₆) δ 11.44 (s, 1H), 10.40 (s, 1H), 8.70 - 8.69 (m, 1H), 8.65 (s, 1H), 8.15 (s, 1H), 8.06 (d, *J* = 8.1 Hz, 1H), 7.95 (d, *J =* 7.5 Hz, 1H), 7.42 - 7.37 (m, 1H), 7.28 (t, *J =* 8.0 Hz, 1H), 6.91 (s, 1H), 6.85 (d, *J* = 7.5 Hz, 1H), 6.68 (d, *J* = 7.6 Hz, 1H), 3.78 (s, 3H), 2.06 (s, 3H). MS (ESI) m/e [M+1]⁺ 335. |
| B14 | 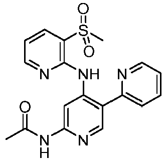 | N-(4'-((3-(methylsulfonyl)pyridin-2-yl)amino)-[2,3'-bipyridin]-6'-yl)acetamide | ¹H NMR (400 MHz, DMSO-*d*₆) δ 12.51 (s, 1H), 10.50 (s, 1H), 9.11 (s, 1H), 8.73 (s, 1H), 8.69 - 8.66 (m, 1H), 8.55 - 8.52 (m, 1H), 8.23 (d, *J =* 7.1 Hz, 1H), 8.04 (d, *J* = 7.6 Hz, 1H), 8.05 - 7.95 (m, 1H), 7.44 - 7.39 (m, 1H), 7.26 - 7.20 (m, 1H), 3.36 (s, 3H), 2.10 (s, 3H). MS (ESI) m/e [M+1]⁺ 384. |
| B15 | 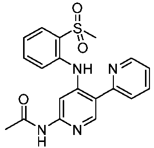 | N-(4'-((2-(methylsulfonyl)phenyl)amino)-[2,3'-bipyridin] -6'-yl)acetamide | ¹H NMR (400 MHz, DMSO-*d*₆) δ 12.14 (s, 1H), 10.52 (s, 1H), 8.77 (s, 1H), 8.60 - 8.59 (m, 1H), 8.21 (s, 1H), 8.09 (d, *J =* 8.4 Hz, 1H), 7.98 - 7.93 (m, 2H), 7.73 - 7.65 (m, 2H), 7.42 - 7.36 (m, 1H), 7.33 - 7.27 (m, 1H), 3.24 (s, 3H), 2.06 (s, 3H). MS (ESI) m/e [M+1]⁺ 383. |
| B16 | 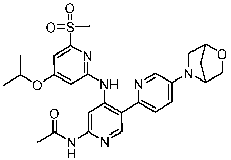 | N-(5-(2-oxa-5-azabicyclo[2.2.1]heptan-5-yl)-4'-((4-isopropoxy-6-(methylsulfonyl)pyridin-2-yl)amino)-[2,3'-bipyridin]-6'-yl)acetamide | ¹H NMR (400 MHz, DMSO-*d*₆) δ 12.76 (s, 1H), 10.41 (s, 1H), 9.09 (s, 1H), 8.64 (s, 1H), 8.25 (s, 1H), 7.91 (d, *J =* 8.0 Hz, 1H), 7.25 (d, *J* = 8.0 Hz, 1H), 7.07 (s, 1H), 6.79 (s, 1H), 4.94 - 4.91 (m, 1H), 4.75 - 4.70 (m, 2H), 3.81 - 3.79 (m, 1H), 3.71 - 3.70 (m, 1H), 3.60 - 3.58 (m, 1H), 3.44 (s, 3H), 3.14 - 3.11 (m, 1H), 2.11 (s, 3H), 1.98 - 1.90 (m, 2H), 1.33 (d, *J =* 8.0 Hz, 6H). MS (ESI) m/e [M+1]⁺ 539. |
| B17 | 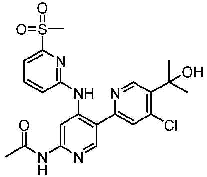 | N-(4-chloro-5-(2-hydroxypropan-2-yl)-4'-((6-(methylsulfonyl)pyridin-2-yl)amino)-[2,3'-bipyridin]-6'-yl)acetamide | ¹H NMR (400 MHz, DMSO-*d*₆) δ 12.77 (s, 1H), 10.61 (s, 1H), 9.23 (s, 1H), 9.11 (s, 1H), 8.85 (s, 1H), 8.21 (s, 1H), 7.99 (t, *J =* 7.9 Hz, 1H), 7.57 (d, *J* = 7.3 Hz, 1H), 7.48 (d, *J =* 8.3 Hz, 1H), 5.63 (s, 1H), 3.47 (s, 3H), 2.13 (s, 3H), 1.66 (s, 6H). MS (ESI) m/e [M+1]⁺ 476. |
| B18 | 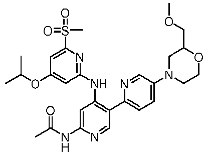 | N-(4'-((4-isopropoxy-6-(methylsulfonyl)pyridin-2-yl)amino)-5-(2-(methoxymethyl)morpholino)-[2,3'-bipyridin]-6'-yl)acetamide | ¹H NMR (400 MHz, DMSO-*d*₆) δ 12.79 (s, 1H), 10.44 (s, 1H), 9.12 (s, 1H), 8.68 (s, 1H), 8.53 (s, 1H), 7.96 (d, *J* = 8.0 Hz, 1H), 7.56 (d, *J* = 8.0 Hz, 1H), 7.07 (s, 1H), 6.82 (s, 1H), 5.03 - 4.77 (m, 1H), 4.01 - 3.98 (m, 1H), 3.82 - 3.61 (m, 4H), 3.46 - 3.44 (m, 5H), 3.32 (s, 3H), 2.86 - 2.80 (m, 1H), 2.65 - 2.59 (m, 1H), 2.11 (s, 3H), 1.33 (d, *J* = 8.0 Hz, 6H). MS (ESI) m/e [M+1]⁺ 571. |
| B19 | 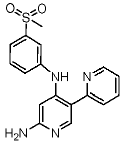 | N4'-(3-(methylsulfonyl)phenyl)-[2,3'-bipyridine]-4',6'-diamine | MS (ESI) m/e [M+1]⁺ 341. |
| B20 | 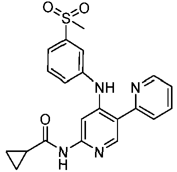 | N-(4'-((3-(methylsulfonyl)phenyl)amino)-[2,3'-bipyridin]-6'-yl)cyclopropanecarboxamide | MS (ESI) m/e [M+1]⁺ 409. |
| B21 | 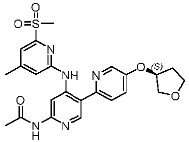 | (S)-N-(4'-((4-methyl-6-(methylsulfonyl)pyridin-2-yl)amino)-5-((tetrahydrofuran-3-yl)oxy)-[2,3'-bipyridin] -6'-yl)acetamide | ¹H NMR (400 MHz, DMSO-*d*₆) δ 12.61 (s, 1H), 10.58 (s, 1H), 9.15 (s, 1H), 8.53 (s, 1H), 8.04 (d, *J =* 7.2 Hz, 1H), 7.61 (s, 1H), 7.55 - 7.45 (m, 2H), 7.24 (s, 1H), 5.23 - 5.20 (m, 1H), 3.92 - 3.88 (m, 3H), 3.85 - 3.80 (m, 1H), 3.47 (s, 3H), 2.42 (s, 3H), 2.35 - 2.30 (m, 1H), 2.12 (s, 3H), 2.10 - 2.05 (m, 1H). MS (ESI) m/e [M+1]⁺ 484. |
| B22 | 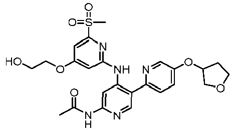 | N-(4'-((4-(2-hydroxyethoxy)-6-(methylsulfonyl)pyridin-2-yl)amino)-5-((tetrahydrofuran-3-yl)oxy)-[2,3'-bipyridin] -6'-yl)acetamide | ¹H NMR (400 MHz, DMSO-*d*₆) δ 12.35 (s, 1H), 10.50 (s, 1H), 9.13 (s, 1H), 8.68 (s, 1H), 8.53 (s, 1H), 8.02 (d, *J* = 8.0 Hz, 1H), 7.60 (d, *J* = 8.0 Hz, 1H), 7.10 (s, 1H), 6.87 (s, 1H), 5.23 - 5.20 (m, 1H), 4.98 (s, 1H), 4.25 - 4.22 (m, 2H), 3.95 - 3.75 (m, 6H), 3.43 (s, 3H), 2.33 - 2.28 (m, 1H), 2.12 (s, 3H), 2.08 - 2.05 (m, 1H). MS (ESI) m/e [M+1]⁺ 530. |
| B23 | 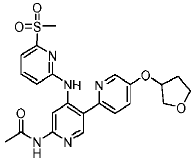 | N-(4'-((6-(methylsulfonyl)pyridin-2-yl)amino)-5-((tetrahydrofuran-3-yl)oxy)-[2,3'-bipyridin] -6'-yl)acetamide | ¹H NMR (400 MHz, DMSO-*d*₆) δ 12.64 (s, 1H), 10.51 (s, 1H), 9.21 (s, 1H), 8.72 (s, 1H), 8.51 (s, 1H), 8.07 (d, *J =* 7.4 Hz, 1H), 7.99 (t*, J =* 7.9 Hz, 1H), 7.61 (d, *J* = 8.8 Hz, 1H), 7.55 *(d, J =* 7.4 Hz, 1H), 7.40 (d, *J* = 8.3 Hz, 1H), 5.23 - 5.20 (m, 1H), 3.92 - 3.88 (m, 3H), 3.85 - 3.80 (m, 1H), 3.47 (s, 3H), 2.35 - 2.30 (m, 1H), 2.12 (s, 3H), 2.10 - 2.05 (m, 1H). MS (ESI) m/e [M+1]⁺ 470. |
| B24 | 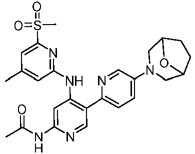 | N-(5-(8-oxa-3-azabicyclo[3.2.1]octan-3-yl)-4'-((4-methyl-6-(methylsulfonyl)pyridin-2-yl)amino)-[2,3'-bipyridin]-6'-yl)acetamide | MS (ESI) m/e [M+1]⁺ 509. |
| B25 | 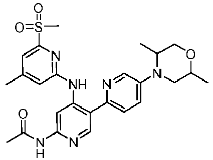 | N-(5-(2,5-dimethylmorpholino)-4'-((4-methyl-6-(methylsulfonyl)pyridin-2-yl)amino)-[2,3'-bipyridin]-6'-yl)acetamid | MS (ESI) m/e [M+1]⁺ 511. |

### Example C1: Synthesis of N-(5-(cis-2,6-dimethylmorpholino)-4'-((4-methyl-6-(methylsulfonyl)pyridin-2-yl)amino)-[2,3'-bipyridin]-6'-yl)acetamide

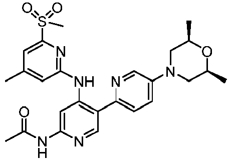

### Step 1: (cis)-4-(6-bromopyridin-3-yl)-2,6-dimethylmorpholine

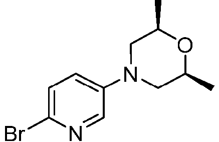

A mixture of 2-bromo-5-iodopyridine (20 g, 70.4 mmol), (Cis)-2,6-dimethylmorpholine (8.9 g, 77.5 mmol, 1.1eq), Pd₂(dba)₃ (3.22 g, 3.52 mmol, 0.05eq), Xant-Phos (2 g, 3.52 mmol, 0.05eq) and *t-*BuONa (13.5 g, 140.8 mmol, 2 eq) in toluene (300 mL) was stirred at 80 °C under N₂ for 2 hrs. The reaction mixture was cooled, filtered and the filtrate was concentrated and the residue was purified by column chromatography (PE: EA = 20: 1-10:1) to give the product (16 g, 84%) as a brown solid. MS (ESI) m/e [M+1]⁺ =271.

### Step 2: 6'-chloro-5-(Cis-2,6-dimethylmorpholino)-[2,3'-bipyridin]-4'-amine

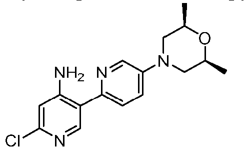

To a solution of cis-4-(6-bromopyridin-3-yl)-2,6-dimethylmorpholine (13.6g, 50.4 mmol) in dioxane/H₂O (400 mL/100 mL) were added 2-chloro-5-(4,4,5,5-tetramethyl-1,3,2-dioxaborolan-2-yl)pyridin-4-amine (15.4, 60.4 mmol), Pd(dppf)Cl₂ (3.7 g, 5.04 mmol) and K₂CO₃ (10.4 g, 75.6 mmol), the resulting mixture was stirred at 100 °C for 2 h under nitrogen atmosphere. After cooled to room temperature, the solvent was removed in vacuo and the residue was diluted with water (500 mL). The resulting solution was extracted with EA (500 mL x 3) and the combined organic layers were washed with brine, dried over anhydrous sodium sulfate. The solvent was removed in vacuo and the residue was purified by combi-flash (MeOH/DCM = 0-5%) to give 6'-chloro-5-(Cis-2,6-dimethylmorpholino)-[2,3'-bipyridin]-4'-amine (18 g, crude). MS (ESI) m/e [M+1]⁺ 319.

### Step 3: N-(4'-amino-5-(Cis-2,6-dimethylmorpholino)-[2,3'-bipyridin]-6'-yl)acetamide

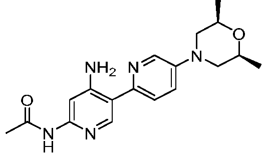

To a solution of 6'-chloro-5-(cis-2,6-dimethylmorpholino)-[2,3'-bipyridin]-4'-amine (18 g, 56.6 mmol) in 1,4-dioxane (250 mL) were added acetamide (16.7 g, 283 mmol), Pd₂(dba)₃ (5.2 g, 5.7 mmol), XantPhos (6.6 g, 11.4 mmol) and Cs₂CO₃ (37.2 g, 114 mmol), the resulting mixture was stirred at 130 °C for 4 h under nitrogen atmosphere. After cooled to room temperature, the solvent was removed in vacuo and the residue was diluted with water (20 mL). The resulting solution was extracted with EA (30 mL x 3) and the combined organic layers were washed with brine, dried over anhydrous sodium sulfate. The solvent was removed in vacuo and the residue was purified by combi-flash (MeOH/DCM = 0 - 7%) to give N-(4'-amino-5-(cis-2,6-dimethylmorpholino)-[2,3'-bipyridin]-6'-yl)acetamide (12.54 g, 65% yield). MS (ESI) m/e [M+1] ⁺ 342.

### Step 4: N-(5-(Cis-2,6-dimethylmorpholino)-4'-((4-methyl-6-(methylsulfonyl)pyridin-2-yl)amino)-[2,3'-bipyridin]-6'-yl)acetamide

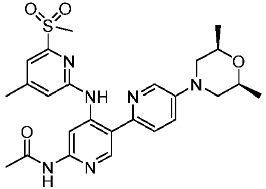

To a mixture of N-(4'-amino-5-(Cis-2,6-dimethylmorpholino)-[2,3'-bipyridin]-6'-yl)acetamide (50 mg, 0.17 mmol), 2-bromo-4-methyl-6-(methylsulfonyl)pyridine (52 mg, 0.21 mmol), Pd₂dba₃ (16 mg, 0.017 mmol), BINAP (11 mg, 0.017 mmol) and Cs₂CO₃ (111 mg, 0.34 mmol) in dioxane (10 mL) was stirred at 130 °C for 4 h. The mixture was filtrated and the filtrate as concentrated to give the residue and purified by Prep-TLC (MeOH/DCM = 1 : 20) to afford N-(5-(Cis-2,6-dimethylmorpholino)-4'-((4-methyl-6-(methylsulfonyl)pyridin-2-yl)amino)-[2,3'-bipyridin]-6'-yl)acetamide (38 mg, 51% yield). ¹H NMR (400 MHz, DMSO-*d*₆) δ 13.13 (s, 1H), 10.45 (s, 1H), 9.23 (s, 1H), 8.72 (s, 1H), 8.51 (s, 1H), 8.00 (d, *J* = 8.7 Hz, 1H), 7.58 (d, *J* = 8.7 Hz, 1H), 7.42 (s, 1H), 7.20 (s, 1H), 3.80 - 3.74 (m, 4H), 3.46 (s, 3H), 2.45 - 2.40 (m, 2H), 2.35 (s, 3H), 2.11 (s, 3H), 1.20 (d, *J* = 6.0 Hz, 6H). MS (ESI) m/e [M+1]⁺ 511*.*

### Example C9: Synthesis of N-(5-(cis-2,6-dimethylmorpholino)-4'-((4-methoxy-6-(methylsulfonyl)pyridin-2-yl)amino)-[2,3'-bipyridin]-6'-yl)acetamide

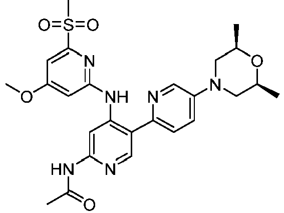

A mixture of N-(4'-amino-5-(cis-2,6-dimethylmorpholino)-[2,3'-bipyridin]-6'-yl)acetamide (1.20 g, 3.52 mmol), 2-bromo-4-methoxy-6-(methylsulfonyl)pyridine (1.15 g, 4.22 mmol), Pd₂(dba)₃ (320 mg, 0.35 mmol), BINAP (218 mg, 0.35 mmol) and Cs₂CO₃ (2.28 g, 7.04 mmol) in 1,4-Dioxane (50 mL) was stirred at 130 °C for 4 hr under nitrogen atmosphere. The reaction mixture was filtered out and the filtrate was concentrated, the residue was purified by combi-flash (MeOH/DCM=7:93) to give the crude product as brown oil, then slurry with ACN (50 mL) at RT for 30 min to give the solid by filtration and dried in vacuum drying oven to give the product (1.3 g, 70% yield). ¹H NMR (400 MHz, DMSO-*d*₆) δ 12.83 (s, 1H), 10.46 (s, 1H), 9.11 (s, 1H), 8.69 (s, 1H), 8.50 (s, 1H), 7.96 (*d, J =* 9.1 Hz, 1H), 7.57 (d*, J =* 9.1 Hz, 1H), 7.11 (s, 1H), 6.84 (s, 1H), 3.97 (s, 3H), 3.79 - 3.73 (m, 4H), 3.44 (s, 3H), 2.40 (t*, J =* 11.0 Hz, 2H), 2.11 (s, 3H), 1.19 (d, *J* = 6.0 Hz, 6H). MS (ESI) m/e [M+1] ⁺ 527.

### Example C17: Synthesis of N-(5-((cis)-2,6-dimethylmorpholino)-4'-((4-(methoxy-d3)-6-(methylsulfonyl)pyridin-2-yl)amino)-[2,3'-bipyridin]-6'-yl)acetamide

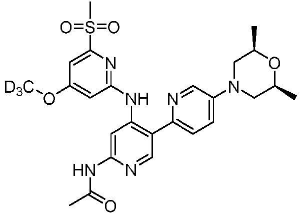

A mixture of N-(4'-amino-5-(cis-2,6-dimethylmorpholino)-[2,3'-bipyridin]-6'-yl)acetamide (2.1 g, 6.16 mmol), 2-bromo-4-(methoxy-d3)-6-(methylsulfonyl)pyridine (2.0 g, 7.39 mmol), Pd₂(dba)₃ (568 mg, 0.62 mmol), BINAP (386 mg, 0.62 mmol) and Cs₂CO₃ (4.02 g, 12.32 mmol) in 1,4-dioxane (75 mL) was stirred at 130 °C for 4 h under N₂. The solid was filtered out and the filtrate was concentrated, the residue was purified by Prep-TLC (MeOH/DCM = 1:20) to give the product (2.03 g, 62% yield). ¹H NMR (400 MHz, DMSO-*d*₆) δ 12.82 (s, 1H), 10.46 (s, 1H), 9.11 (s, 1H), 8.69 (s, 1H), 8.50 (s, 1H), 7.96 (d, *J* = 9.1 Hz, 1H), 7.55 (s, 1H), 7.11 (s, 1H), 6.84 (s, 1H), 3.79 - 3.76 (m, 4H), 3.44 (s, 3H), 2.43 - 2.37 (m, 2H), 2.11 (s, 3H), 1.19 (d, *J =* 5.3 Hz, 6H). MS (ESI) m/e [M+1]⁺ 530.

### Example C18: Synthesis of N-(5-(cis-2,6-dimethylmorpholino)-4'-((4-(1-methoxyethyl)-6-(methylsulfonyl)pyridin-2-yl)amino)-[2,3'-bipyridin]-6'-yl)acetamide

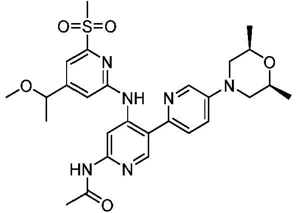

A mixture of N-(4'-amino-5-(cis-2,6-dimethylmorpholino)-[2,3'-bipyridin]-6'-yl)acetamide (60 mg, 0.17 mmol), 2-bromo-4-(1-methoxyethyl)-6-(methylsulfonyl)pyridine (62 mg, 0.21 mmol), Pd₂dba₃ (16 mg, 0.02 mmol), BINAP (11 mg, 0.02 mmol) and Cs₂CO₃ (111 mg, 0.34 mmol) in 1,4-dioxane (10 mL) was stirred at 130 °C for 4 h under nitrogen atmosphere. The solid was filtered out and the filtrate was concentrated, the residue was purified by Prep-TLC (MeOH/DCM = 1:20) to give the product (40.56 mg, 42% yield) as a yellow solid. ¹H NMR (400 MHz, DMSO-*d*₆) δ 12.99 (s, 1H), 10.46 (s, 1H), 9.21 (s, 1H), 8.71 (s, 1H), 8.49 (s, 1H), 7.98 (d, *J =* 9.0 Hz, 1H), 7.57 (d, *J* = 9.2 Hz, 1H), 7.48 (s, 1H), 7.25 (s, 1H), 4.50 - 4.49 (m, 1H), 3.77 - 3.74 (m, 4H), 3.48 (s, 3H), 3.23 (s, 3H), 2.41 (t, *J* = 11.1 Hz, 2H), 2.11 (s, 3H), 1.39 (d, *J =* 6.5 Hz, 3H), 1.20 (d, *J* = 6.0 Hz, 6H). MS (ESI) m/e [M+1] ⁺ =555.

### Example C19: Synthesis of N-(5-((cis)-2,6-dimethylmorpholino)-4'-((4-((R or S)-1-methoxyethyl)-6-(methylsulfonyl)pyridin-2-yl)amino)-[2,3'-bipyridin]-6'-yl)acetamide

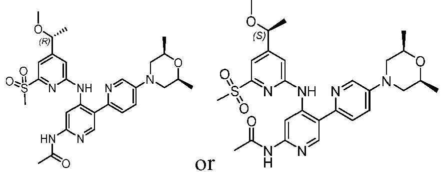

A mixture of N-(4'-amino-5-((cis)-2,6-dimethylmorpholino)-[2,3'-bipyridin]-6'-yl)acetamide (50 mg, 0.147 mmol), (R or S) 2-bromo-4-(1-methoxyethyl)-6-(methylsulfonyl)pyridine (44 mg, 0.176 mmol) (faster peak from example BB60, step 5), Pd₂(dba)₃ (13 mg, 0.0147 mmol), BINAP (18 mg, 0.0294 mmol) and Cs₂CO₃ (72 mg, 0.221 mmol) in1,4-dioxane (6 mL) was stirred at 130 °C for 4 hr under nitrogen atmosphere. The reaction was cooled to room temperature, filtered and the filtration was concentrated under vacuum. The residue was purified with Prep-TLC (DCM/MeOH = 20: 1) to afford the product (33 mg, 40%). ¹H NMR (400 MHz, DMSO-*d*₆) δ 13.01 (s, 1H), 10.49 (s, 1H), 9.21 (s, 1H), 8.71 (s, 1H), 8.50 (s, 1H), 7.99 (d, *J* = 8.9 Hz, 1H), 7.58 (d, *J* = 8.9 Hz, 1H), 7.49 (s, 1H), 7.26 (s, 1H), 4.53-4.43 (m, 1H), 3.84 - 3.70 (m, 4H), 3.49 (s, 3H), 3.23 (s, 3H), 2.41 (t*, J =* 10.9 Hz, 2H), 2.12 (s, 3H), 1.39 (d, *J* = 5.9 Hz, 3H), 1.20 (d, *J =* 5.4 Hz, 6H). MS (ESI) m/e [M+1]⁺ 555.

### Example C20: Synthesis of N-(5-((cis)-2,6-dimethylmorpholino)-4'-((4-((R or S)-1-methoxyethyl)-6-(methylsulfonyl)pyridin-2-yl)amino)-[2,3'-bipyridin]-6'-yl)acetamide

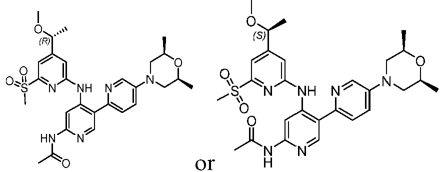

A solution of N-(4'-amino-5-(cis-2,6-dimethylmorpholino)-[2,3'-bipyridin]-6'-yl)acetamide (60 mg, 0.18 mmol), (S or R)-2-bromo-4-(1-methoxyethyl)-6-(methylsulfonyl)pyridine (slower peak from example BB60, step 5) (62 mg, 0.21 mmol), Pd₂(dba)₃ (33 mg, 0.036 mmol), Xantphos (41.7 mg, 0.072 mmol) and Cs₂CO₃ (117.4 mg, 0.36 mmol) in dioxane (3 mL) was stirred at 130 °C for 4 hr under nitrogen atmosphere. The reaction was cooled to room temperature, filtered and the filtration was concentrated under vacuum. The residue was purified with Prep-TLC (DCM/MeOH = 15: 1) to afford the product (35.83 mg, 35.89%). ¹H NMR (400 MHz, DMSO-*d*₆) δ 13.00 (s, 1H), 10.47 (s, 1H), 9.22 (s, 1H), 8.71 (s, 1H), 8.50 (s, 1H), 7.99 (d, *J =* 9.0 Hz, 1H), 7.58 (d, *J =* 9.0 Hz, 1H), 7.48 (s, 1H), 7.26 (s, 1H), 4.53 - 4.45 (m, 1H), 3.84 - 3.67 (m, 4H), 3.49 (s, 3H), 3.23 (s, 3H), 2.41 (t*, J =* 11.1 Hz, 2H), 2.11 (s, 3H), 1.39 (d, *J* = 6.3 Hz, 3H), 1.20 (d, *J* = 6.0 Hz, 6H). MS (ESI) m/e [M+1]⁺ 555.

### Example C21: Synthesis of N-(5-(cis-2,6-dimethylmorpholino)-4'-((6-(methylsulfonyl)-4-(tetrahydro-2H-pyran-4-yl)pyridin-2-yl)amino)-[2,3'-bipyridin]-6'-yl)acetamide

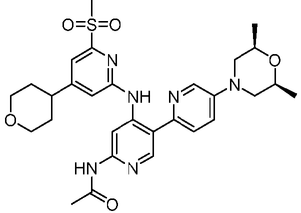

A mixture of N-(4'-amino-5-(cis-2,6-dimethylmorpholino)-[2,3'-bipyridin]-6'-yl)acetamide (60 mg, 0.17 mmol), 2-bromo-6-(methylsulfonyl)-4-(tetrahydro-2H-pyran-4-yl)pyridine (66 mg, 0.21 mmol), Pd₂(dba)₃ (16 mg, 0.02 mmol), BINAP (11 mg, 0.02 mmol) and Cs₂CO₃ (111 mg, 0.34 mmol) in 1,4-dioxane (10 mL) was stirred at 130 °C for 4 hr under N₂. The reaction mixture was filtered out and the filtrate was concentrated, the residue was purified by Prep-TLC (MeOH/DCM=1:20) to give the product (47.27 mg, 46% yield). ¹H NMR (400 MHz, DMSO-*d*₆) 12.97 (s, 1H), 10.46 (s, 1H), 9.20 (s, 1H), 8.70 (s, 1H), 8.52 (s, 1H), 7.98 (d, *J =* 8.7 Hz, 1H), 7.57 (d, *J=* 8.7 Hz, 1H), 7.46 (s, 1H), 7.21 (s, 1H), 3.98 (d, *J =* 10.2 Hz, 2H), 3.83 - 3.70 (m, 4H), 3.47 - 3.42 (m, 5H), 3.02 - 2.95 (m, 1H), 2.41 (t, *J* = 11.0 Hz, 2H), 2.11 (s, 3H), 1.76 - 1.74 (m, 4H), 1.20 (d, *J* = 6.1 Hz, 6H). MS (ESI) m/e [M+1]⁺ 581.
**Example C22: Synthesis of N-(5-(cis-2,6-dimethylmorpholino)-4'-((6-**

### (methylsulfonyl)pyrazin-2-yl)amino)-[2,3'-bipyridin]-6'-yl)acetamide

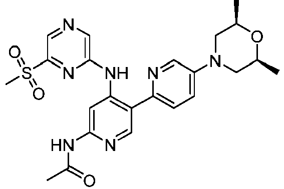

A mixture of N-(4'-amino-5-(cis-2,6-dimethylmorpholino)-[2,3'-bipyridin]-6'-yl)acetamide (60 mg, 0.17 mmol), 2-bromo-6-(methylsulfonyl)pyrazine (49 mg, 0.21 mmol), Pd₂(dba)₃ (16 mg, 0.02 mmol), BINAP (11 mg, 0.02 mmol) and Cs₂CO₃ (111 mg, 0.34 mmol) in 1,4-Dioxane (10 mL) was stirred at 130 °C for 4 hr under N₂ atmosphere. The reaction mixture was filtered out and the filtrate was concentrated, the residue was purified by Prep-TLC (MeOH/DCM = 1:20) to give the product (27.37 mg, 31% yield). ¹H NMR (400 MHz, DMSO-*d*₆) δ 14.00 (s, 1H), 10.50 (s, 1H), 9.29 (s, 1H), 8.78 - 8.75 (m, 2H), 8.55 - 8.50 (m, 2H), 8.00 (s, 1H), 7.55 (s, 1H), 3.75 - 3.69 (m, 4H), 3.53 (s, 3H), 2.37 *(t, J=* 11.0 Hz, 2H), 2.08 (s, 3H), 1.15 (d, *J=* 6.1 Hz, 6H). MS (ESI) m/e [M+1]⁺ 498.

### Example C23: Synthesis of compound N-(5-(cis-2,6-dimethylmorpholino)-4'-((4-((S)-3-methylmorpholino)-6-(methylsulfon-yl)pyridin-2-yl)amino)-[2,3'-bipyridin]-6'-yl)ace-tamide.

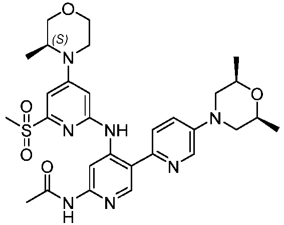

*N*-(4'-amino-5-(*cis*-2,6-dimethylmorpholino)-[2,3'-bipyridin]-6'-yl)aceta-mide (50 mg, 0.15 mmol), (*S*)-4-(2-chloro-6-(methylsulfonyl)pyridin-4-yl)-3-methyl-morpholine (47 mg, 0.16 mmol), Pd₂dba₃ (13.4 mg, 0.02 mmol), BINAP (18.3 mg, 0.03 mmol) and Cs₂CO₃ (72 mg, 0.22 mmol) were added into 1,4-dioxane (10 mL). The resulting mixture was degassed with nitrogen and stirred at 130 °C for 2 hr. After cooled to room temperature, the solid was filtered out. The filtration was concentrated under vacuum. The residue was purified with Prep-TLC (DCM/MeOH = 20/1) to give product (63.39 mg, yield: 72.7%). ¹H NMR (400 MHz, DMSO-*d*₆) δ 12.28 (s, 1H), 10.41 (s, 1H), 9.00 (s, 1H), 8.62 (s, 1H), 8.46 (s, 1H), 7.93 (d, *J=* 8.5 Hz, 1H), 7.55 (d, *J=* 8.5 Hz, 1H), 7.05 (s, 1H), 6.53 (s, 1H), 4.15 - 4.12 (m, 1H), 3.97 (d, *J=* 12.0 Hz, 1H), 3.88 - 3.57 (m, 7H), 3.52 *(t, J=* 11.6 Hz, 1H), 3.38 (s, 3H), 3.17 *(t, J=* 11.6 Hz, 1H), 2.39 (t, *J* = 11.0 Hz, 2H), 2.10 (s, 3H), 1.27 - 1.07 (m, 9H). MS (ESI) m/e [M+1]⁺ 596.

### Example C24: Synthesis of compound N-(5-(cis-2,6-dimethylmorpholino)-4'-((4-((R)-3-methylmorpholino)-6-(methylsulfon-yl)pyridin-2-yl)amino)-[2,3'-bipyridin]-6'-yl)ace-tamide.

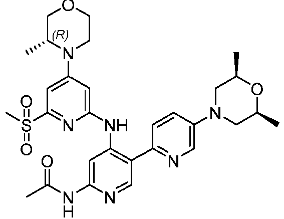

*N*-(4'-amino-5-(*cis*-2,6-dimethylmorpholino)-[2,3'-bipyridin]-6'-yl)aceta-mide (50 mg, 0.15 mmol), (R)-4-(2-chloro-6-(methylsulfonyl)pyridin-4-yl)-3-methyl-morpholine (47 mg, 0.16 mmol), Pd₂dba₃ (13.4 mg, 0.02 mmol), BINAP (18.3 mg, 0.03 mmol) and Cs₂CO₃ (72 mg, 0.22 mmol) were added into 1,4-dioxane (10 mL). The resulting mixture was degassed with nitrogen and stirred at 130 °C for 2 hr. After cooled to room temperature, the solid was filtered out. The filtration was concentrated under vacuum. The residue was purified with Prep-TLC (DCM/MeOH = 20/1) to give the product (53.8 mg, yield: 61.7%). ¹H NMR (400 MHz, DMSO-*d*₆) δ 12.27 (s, 1H), 10.40 (s, 1H), 8.99 (s, 1H), 8.62 (s, 1H), 8.46 (s, 1H), 7.92 (d, *J* = 8.6 Hz, 1H), 7.55 (d, *J=* 8.6 Hz, 1H), 7.04 (s, 1H), 6.52 (s, 1H), 4.15 - 4.12 (m, 1H), 3.96 (d, *J=* 10.4 Hz, 1H), 3.78 - 3.71 (m, 5H), 3.67 - 3.59 (m, 2H), 3.52 *(t, J=* 11.4 Hz, 1H), 3.37 (s, 3H), 3.16 *(t, J=* 11.4 Hz, 1H), 2.39 *(t, J=* 11.1 Hz, 2H), 2.09 (s, 3H), 1.24 - 1.08 (m, 9H). MS (ESI) m/e [M+1]⁺ 596.

The following Examples were prepared in a similar manner to the product Example C1:

| **Example** | **Compound** | **Chemical Name** | **¹H NMR and LC /MS *m*/*z* (M+1)** |
|---|---|---|---|
| C2 | 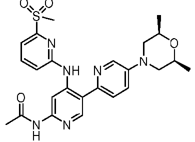 | N-(5-((cis)-2,6-dimethylmorpholino)-4'-((6-(methylsulfonyl)pyridin-2-yl)amino)-[2,3'-bipyridin]-6'-yl)acetamide | ¹H NMR (400 MHz, DMSO-*d*₆) δ 13.23 (s, 1H), 10.48 (s, 1H), 9.20 (s, 1H), 8.71 (s, 1H), 8.48 (d, *J =* 2.9 Hz, 1H), 7.97-7.94 (m, 2H), 7.57 - 7.52 (m, 2H), 7.36 (d, *J =* 8.4 Hz, 1H), 3.78 - 3.70 (m, 4H), 3.46 (s, 3H), 2.38 *(t, J=* 11.1 Hz, 2H), 2.10 (s, 3H), 1.17 (d, *J =* 6.2 Hz, 6H). MS (ESI) m/e [M+1]⁺ 497. |
| C3 | 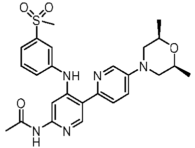 | N-(5-((cis)-2,6-dimethylmorpholino)-4'-((3-(methylsulfonyl)phenyl)amino)-[2,3'-bipyridin]-6'-yl)acetamide | ¹H NMR (400 MHz, DMSO-*d*₆) δ 11.65 (s, 1H), 10.44 (s, 1H), 8.62 (s, 1H), 8.41 (s, 1H), 8.18 (s, 1H), 7.93 *(d, J=* 9.1 Hz, 1H), 7.79 (s, 1H), 7.68-7.50 (m, 4H), 3.78 - 3.72 (m, 4H), 3.29 (s, 3H), 2.37 (t, *J=* 11.0 Hz, 2H), 2.06 (s, 3H), 1.17 (d, *J=* 5.8 Hz, 6H). MS (ESI) m/e [M+1]⁺ 496. |
| C4 | 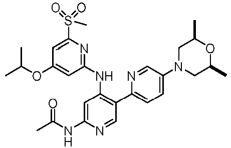 | N-(5-((cis)-2,6-dimethylmorpholino)-4'-((4-isopropoxy-6-(methylsulfonyl)pyridin-2-yl)amino)-[2,3'-bipyridin]-6'-yl)acetamide | ¹H NMR (400 MHz, DMSO-*d*₆) δ 12.80 (s, 1H), 10.44 (s, 1H), 9.11 (s, 1H), 8.68 (s, 1H), 8.51 (s, 1H), 7.96 *(d, J=* 9.2 Hz, 1H), 7.56 (d, *J=* 6.3 Hz, 1H), 7.08 (s, 1H), 6.80 (s, 1H), 4.95 - 4.92 (m, 1H), 3.79 - 3.73 (m, 4H), 3.44 (s, 3H), 2.40 (t, *J* = 10.9 Hz, 2H), 2.11 (s, 3H), 1.33 *(d, J=* 5.9 Hz, 6H), 1.19 (d, *J=* 5.9 Hz, 6H). MS (ESI) m/e [M+1]⁺ 555. |
| C5 | 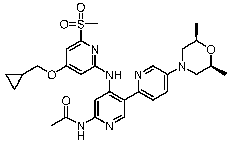 | N-(4'-((4-(cyclopropylmethoxy)-6-(methylsulfonyl)pyridin-2-yl)amino)-5-((cis)-2,6-dimethylmorpholino)-[2,3'-bipyridin] -6'-yl)acetamide | ¹H NMR (400 MHz, DMSO-*d*₆) δ 12.83 (s, 1H), 10.44 (s, 1H), 9.11 (s, 1H), 8.68 (s, 1H), 8.52 (s, 1H), 7.96 *(d, J=* 8.7 Hz, 1H), 7.56 (d, *J* = 8.7 Hz, 1H), 7.10 (s, 1H), 6.82 (s, 1H), 4.10 - 4.05 (m, 2H), 3.83 - 3.65 (m, 4H), 3.44 (s, 3H), 2.45 - 2.35 (m, 2H), 2.11 (s, 3H), 1.28 - 1.22 (m, 1H), 1.19 (d, *J =* 4.7 Hz, 6H), 0.65 - 0.60 (m, 2H), 0.40 - 0.35 (m, 2H). MS (ESI) m/e [M+1]⁺ 567. |
| C6 | 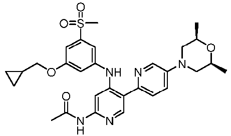 | N-(4'-((3-(cyclopropylmethoxy)-5-(methylsulfonyl)phenyl)amino)-5-((cis)-2,6-dimethylmorpholino)-[2,3'-bipyridin] -6'-yl)acetamide | ¹H NMR (400 MHz, DMSO-*d*₆) δ 11.64 (s, 1H), 10.43 (s, 1H), 8.61 (s, 1H), 8.41 (s, 1H), 8.20 (s, 1H), 7.91 *(d, J=* 9.0 Hz, 1H), 7.53 (d, *J* = 9.0 Hz, 1H), 7.34 (s, 1H), 7.15 (s, 1H), 7.06 (s, 1H), 3.99 - 3.96 (m, 2H), 3.77 - 3.74 (m, 4H), 3.27 (s, 3H), 2.40 - 2.35 (m, 2H), 2.07 (s, 3H), 1.26 - 1.22 (m, 1H), 1.17 (d, *J =* 6.0 Hz, 6H), 0.62 - 0.58 (m, 2H), 0.38 - 0.32 (m, 2H). MS (ESI) m/e [M+1]⁺ 566. |
| C7 | 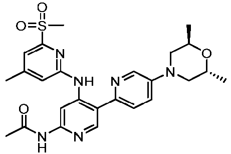 | N-(5-((trans)-2,6-dimethylmorpholino)-4'-((4-methyl-6-(methylsulfonyl)pyridin-2-yl)amino)-[2,3'-bipyridin]-6'-yl)acetamide | ¹H NMR (400 MHz, DMSO-*d*₆) δ 13.09 (s, 1H), 10.47 (s, 1H), 9.21 (s, 1H), 8.70 (s, 1H), 8.49 (s, 1H), 7.98 (d,*J =* 8.9 Hz, 1H), 7.55 (d, *J* = 8.9 Hz, 1H), 7.42 (s, 1H), 7.20 (s, 1H), 4.11 (d, *J =* 2.6 Hz, 2H), 3.46 (s, 3H), 3.40 - 3.35 (m, 2H), 3.06 - 3.02 (m, 2H), 2.43 (s, 3H), 2.12 (s, 3H), 1.24 (d, *J=* 5.8 Hz, 6H). MS (ESI) m/e [M+1]⁺ 567. |
| C8 | 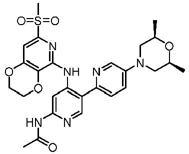 | N-(5-(Cis-2,6-dimethylmorpholino)-4'-((7-(methylsulfonyl)-2,3-dihydro-[1,4]dioxino[2,3-c]pyridin-5-yl)amino)-[2,3'-bipyridin]-6'-yl)acetamide | ¹H NMR (400 MHz, DMSO-*d₆*) δ 13.26 (s, 1H), 10.44 (s, 1H), 9.39 (s, 1H), 8.70 (s, 1H), 8.37 (s, 1H), 7.97 *(d, J=* 9.0 Hz, 1H), 7.56 (d, *J=* 9.0 Hz, 1H), 7.13 (s, 1H), 4.66 - 4.63 (m, 2H), 4.50 - 4.45 (m, 2H), 3.82 - 3.74 (m, 4H), 3.46 (s, 3H), 2.45 - 2.40 (m, 2H), 2.12 (s, 3H), 1.19 (d,*J =* 6.0 Hz, 6H). MS (ESI) m/e [M+1]⁺ 555. |
| C9 | 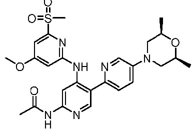 | N-(5-((cis)-2,6-dimethylmorpholino)-4'-((4-methoxy-6-(methylsulfonyl)pyridin-2-yl)amino)-[2,3'-bipyridin]-6'-yl)acetamide | MS (ESI) m/e [M+1]⁺527. |
| C10 | 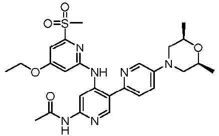 | N-(5-((cis)-2,6-dimethylmorpholino)-4'-((4-ethoxy-6-(methylsulfonyl)pyridin-2-yl)amino)-[2,3'-bipyridin]-6'-yl)acetamide | MS (ESI) m/e [M+1]⁺ 541. |
| C11 | 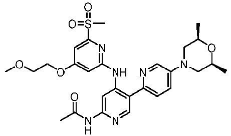 | N-(5-((cis)-2,6-dimethylmorpholino)-4'-((4-(2-methoxyethoxy)-6-(methylsulfonyl)pyridin-2-yl)amino)-[2,3'-bipyridin]-6'-yl)acetamide | MS (ESI) m/e [M+1]⁺ 571. |
| C12 | 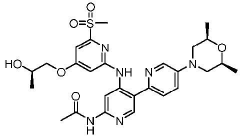 | N-(5-((cis)-2,6-dimethylmorpholino)-4'-((4-((R)-2-hydroxypropoxy)-6-(methylsulfonyl)pyridin-2-yl)amino)-[2,3'-bipyridin]-6'-yl)acetamide | MS (ESI) m/e [M+1]⁺ 571. |
| C13 | 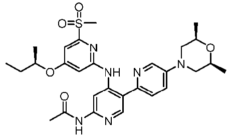 | N-(4'-((4-((R)-sec-butoxy)-6-(methylsulfonyl)pyridin-2-yl)amino)-5-((cis)-2,6-dimethylmorpholino)-[2,3'-bipyridin]-6'-yl)acetamide Molecular Weight: 568.69 | MS (ESI) m/e [M+1]⁺ 569. |
| C14 | 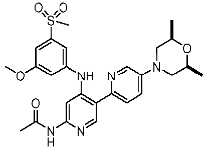 | N-(5-((cis)-2,6-dimethylmorpholino)-4'-((3-methoxy-5-(methylsulfonyl)phenyl)amino)-[2,3'-bipyridin]-6'-yl)acetamide Molecular Weight: 525.62 | MS (ESI) m/e [M+1]⁺ 526. |
| C15 | 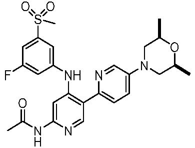 | N-(5-((cis)-2,6-dimethylmorpholino)-4'-((3-fluoro-5-(methylsulfonyl)phenyl)amino)-[2,3'-bipyridin]-6'-yl)acetamide | MS (ESI) m/e [M+1]⁺ 514. |
| C16 | 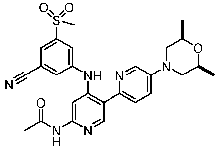 | N-(4'-((3-cyano-5-(methylsulfonyl)phenyl)amino)-5-((cis)-2,6-dimethylmorpholino)-[2,3'-bipyridin]-6'-yl)acetamide | MS (ESI) m/e [M+1]⁺ 521. |
| C25 | 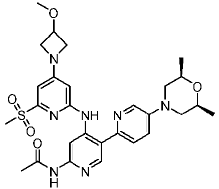 | N-(5-(cis-2,6-dimethyl morpholino)-4'-((4-(3-methoxy azetidin-1-yl)-6-(methylsulfonyl)pyridin -2-yl)amino)-[2,3'-bipyridin]-6'-yl)acetamide | 1H NMR (400 MHz, DMSO-d6) δ 12.39 (s, 1H), 10.49 (s, 1H), 9.04 (s, 1H), 8.70 (s, 1H), 8.53 (s, 1H), 8.00 (d, J = 8.0 Hz, 1H), 7.62 (d, J = 8.0 Hz, 1H), 6.70 (s, 1H), 6.20 (s, 1H), 4.45 - 4.42 (m, 1H), 4.32 - 4.30 (m, 2H), 3.97 - 3.95 (m, 2H), 3.86 - 3.80 (m, 4H), 3.43 (s, 3H), 3.34 (s, 3H), 2.49 - 2.44 (m, 2H), 2.17 (s, 3H), 1.26 - 1.23 (m, 6H). MS (ESI) m/e [M+1]+ 582. |

### Example D1: Synthesis of N-(5-(2-methoxyethoxy)-4'-((6-(methylsulfonyl)pyridin-2-yl)amino)-[2,3'-bipyridin]-6'-yl)acetamide

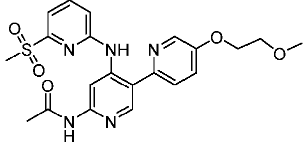

### Step 1: 2-bromo-5-(2-methoxyethoxy)pyridine

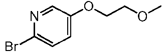

To a solution of 6-bromopyridin-3-ol (5 g, 28.7 mmol) in THF (50 mL) was added NaH (60% in mineral oil, 1.7 g, 43.1 mmol) one portions at 0 °C and the resulting mixture was stirred at this temperature for 15 mins, then 1-bromo-2-methoxyethane (8 g, 57.5 mmol) was added dropwise at 0 °C and the mixture was stirred at room temperature for 16 h. Upon completion of the reaction, the reaction mixture was poured into H₂O (200 mL) and the resulting mixture was extracted with EA (40 mL x 3). The combined organic layers were dried over Na₂SO₄ and concentrated under vacuum. The residue was purified by silica gel column chromatography (PE/EA = 20:1 to 5:1) to give 2-bromo-5-(2-methoxyethoxy)pyridine (4.8 g, 72%). MS (ESI) m/e [M+1]⁺ 232.

### Step 2: tert-butyl (6'-chloro-5-(2-methoxyethoxy)-[2,3'-bipyridin]-4'-yl)carbamate

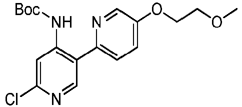

A mixture of 2-bromo-5-(2-methoxyethoxy)pyridine (2.5 g , 10.7 mmol), tert-butyl (5-bromo-2-chloropyridin-4-yl)carbamate (3.6 g, 11.8 mmol), Sn₂Me₆ (5.2 g, 16.1 mmol), Pd(PPh₃)₄ (1.2 g, 1.08 mmol) and Pd(PPh₃)₂Cl₂ (756.1 mg, 1.1 mmol) in 1,4-dioxane (30 mL) was stirred at 110 °C for 16 h under nitrogen atmosphere. After cooled to room temperature, the solvent was removed in vacuo and the residue was diluted with 20 mL of water. The resulting solution was extracted with EA (40 mL x 3) and the combined organic layers were washed with brine (50 mL), dried over anhydrous sodium sulfate. The solvent was removed in vacuo and the residue was purified by silica gel column chromatography (PE/EA = 50:1 to 0:1) to give tert-butyl (6'-chloro-5-(2-methoxyethoxy)-[2,3'-bipyridin]-4'-yl)carbamate (1.2 g, 29%). ¹H NMR (400 MHz, CDCl₃) δ 11.43 (s, 1H), 8.40 (s, 1H), 8.31 - 8.25 (m, 2H), 7.57 (d, *J =* 8.8 Hz, 1H), 7.33 - 7.27 (m, 1H), 4.19 - 4.12 (m, 2H), 3.77 - 3.60 (m, 2H), 3.36 (s, 3H), 1.42 (s, 9H). MS (ESI) m/e [M+1]⁺ 380.

### Step 3: tert-butyl (6'-acetamido-5-(2-methoxyethoxy)-[2,3'-bipyridin]-4'-yl)carbamate

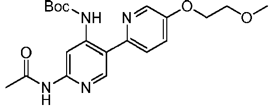

To a solution of tert-butyl (6'-chloro-5-(2-methoxyethoxy)-[2,3'-bipyridin]-4'-yl)carbamate (0.8 g, 2.1 mmol) in 1,4-dioxane (10 mL) were added acetamide (149 mg, 2.5 mmol), Cs₂CO₃ (1.3 g, 4.2 mmol), XantPhos (243 mg, 0.42 mmol) and Pd₂(dba)₃ (192.86 mg, 0.21 mmol), the resulting mixture was stirred at 110 °C for 16 h under nitrogen atmosphere. After cooled to room temperature, the solvent was removed in vacuo and the residue was diluted with 10 mL of water. The resulting solution was extracted with EA (10 mL x 3) and the combined organic layers were washed with brine (50 mL), dried over anhydrous sodium sulfate. The solvent was removed in vacuo and the residue was purified by silica gel column chromatography (PE/EA = 50:1 to 0:1) to give tert-butyl (6'-acetamido-5-(2-methoxyethoxy)-[2,3'-bipyridin]-4'-yl)carbamate (0.5 g, 58%). MS (ESI) m/e [M+1]⁺ 403.

### Step 4: N-(4'-amino-5-(2-methoxyethoxy)-[2,3'-bipyridin]-6'-yl)acetamide

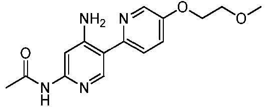

To a mixture of tert-butyl (6'-acetamido-5-(2-methoxyethoxy)-[2,3'-bipyridin]-4'-yl)carbamate (0.5 g, 1.2 mmol) in DCM (8 mL) was added TFA (2 mL) and the resulting solution was stirred at room temperature for 3 h. Upon completion of the reaction, the solvent was removed in vacuo and the residue was diluted with water, then aq. NaHCO₃ (50%) was added to adjust the pH value to 10. The resulting mixture was extracted with EA (10 mL x 3). The combined organic layers were dried over Na₂SO₄ and concentrated to give N-(4'-amino-5-(2-methoxyethoxy)-[2,3'-bipyridin]-6'-yl)acetamide (275 mg, 73%). ¹H NMR (400 MHz, DMSO-*d*₆) δ 10.09 (s, 1 H), 8.37 - 8.29 (m, 2 H), 7.82 (d, *J=* 9.0 Hz, 1 H), 7.50 - 7.45 (m, 2H), 7.44 - 7.34 (m, 2H), 4.25 - 4.18 (m, 2 H), 3.70 - 3.65 (m, 2 H), 3.31 (s, 3 H), 2.06 (s, 3 H). MS(ESI) m/e [M+1]⁺ 303.

### Step 5: N-(5-(2-methoxyethoxy)-4'-((6-(methylsulfonyl)pyridin-2-yl)amino)-[2,3'-bipyridin]-6'-yl)acetamide

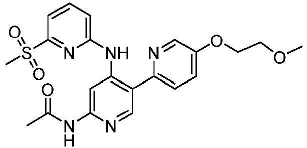

A solution of N-(4'-amino-5-(2-methoxyethoxy)-[2,3'-bipyridin]-6'-yl)acetamide (50 mg, 0.17 mmol), 2-bromo-6-(methylsulfonyl)pyridine (60 mg, 0.26 mmol), Pd₂(dba)₃ (16 mg, 0.017 mmol), BINAP (10 mg, 0.017 mmol), CsCO₃ (110 mg, 0.34 mmol) in10 ml dioxane was stirred at 120 °C in sealed tube for 2 h, the solution was concentrated and purified by Prep-TLC to give N-(5-(2-methoxyethoxy)-4'-((6-(methylsulfonyl)pyridin-2-yl)amino)-[2,3'-bipyridin]-6'-yl)acetamide (10 mg). ¹H NMR (400 MHz, DMSO-*d*₆) δ 12.66 (s, 1H), 10.50 (s, 1H), 9.19 (s, 1H), 8.70 (s, 1H), 8.55 - 8.51 (m, 1H), 8.05 - 7.95 (m, 2H), 7.62 - 7.59 (m, 1H), 7.54 -7.52 (m, 1H), 7.38 - 7.35 (m, 1H), 4.27 - 4.25 (m, 2H), 3.71 - 3.68 (m, 2H), 3.45 (s, 3H), 3.31 (s, 3H), 2.10 (s, 3H). MS (ESI) m/e [M+1]⁺ 458.

The following Examples were prepared in a similar manner to the product Example D1:

| **Example** | **Compound** | **Chemical Name** | **¹H NMR and LC /MS *m*/*z* (M+1)** |
|---|---|---|---|
| D2 | 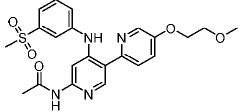 | N-(5-(2-methoxyethoxy)-4'-((3-(methylsulfonyl)pheny l)amino)-[2,3'-bipyridin]-6'-yl)acetamide | ¹H NMR (400 MHz, DMSO-*d*₆) δ 11.21 (s, 1H), 10.48 (s, 1H), 8.60 (s, 1H), 8.44 (s, 1H), 8.15 (s, 1H), 7.99 (d, *J=* 9.1 Hz, 1H), 7.81 (s, 1H), 7.61-7.58 (m, 4H), 4.25 - 4.24 (m, 2H), 3.70 - 3.69 (m, 2H), 3.32 (s, 3H), 3.29 (s, 3H), 2.07 (s, 3H). MS (ESI) m/e [M+1]⁺ 457. |
| D3 | 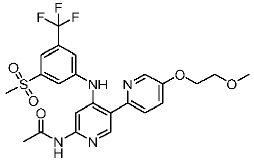 | N-(5-(2-methoxyethoxy)-4'-((3-(methylsulfonyl)-5-(trifluoromethyl)pheny l)amino)-[2,3'-bipyridin]-6'-yl)acetamide | ¹H NMR (400 MHz, DMSO-*d*₆) δ 11.17 (s, 1H), 10.53 (s, 1H), 8.61 (s, 1H), 8.45 (s, 1H), 8.21 (s, 1H), 8.06 (s, 1H), 7.94 (d, *J =* 8.2 Hz, 2H), 7.76 (s, 1H), 7.57 *(d, J=* 8.9 Hz, 1H), 4.25 - 4.20 (m, 2H), 3.70 - 3.68 (m, 2H), 3.38 (s, 3H), 3.32 (s, 3H), 2.08 (s, 3H). MS (ESI) m/e [M+1]⁺ 525. |
| D4 | 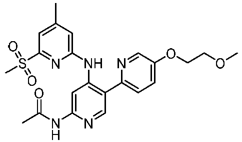 | N-(5-(2-methoxyethoxy)-4'-((4-methyl-6-(methylsulfonyl)pyridi n-2-yl)amino)-[2,3'-bipyridin]-6'-yl)acetamide | ¹H NMR (400 MHz, DMSO-*d*₆) δ 12.67 (s, 1H), 10.54 (s, 1H), 9.20 (s, 1H), 8.71 (s, 1H), 8.56 (s, 1H), 8.06 (d, *J=* 8.8 Hz, 1H), 7.64 (d, *J=* 8.9 Hz, 1H), 7.44 (s, 1H), 7.24 (d, *J =* 10.3 Hz, 2H), 4.29 - 4.22 (m, 2H), 3.72 - 3.70 (m, 2H), 3.46 (s, 3H), 3.34 (s, 3H), 2.42 (s, 3H), 2.12 (s, 3H). MS (ESI) m/e [M+1]⁺ 472. |
| D5 | 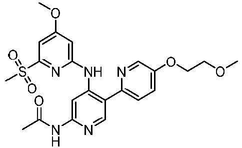 | N-(4'-((4-methoxy-6-(methylsulfonyl)pyridi n-2-yl)amino)-5-(2-methoxyethoxy)-[2,3'-bipyridin]-6'-yl)acetamide | ¹H NMR (400 MHz, DMSO-*d*₆) δ 12.39 (s, 1H), 10.51 (s, 1H), 9.12 (s, 1H), 8.68 (s, 1H), 8.56 (s, 1H), 8.03 (d, *J=* 8.6 Hz, 1H), 7.63 (d, *J=* 8.7 Hz, 1H), 7.10 (s, 1H), 6.87 (s, 1H), 4.28 - 4.25 (m, 2H), 3.96 (s, 3H), 3.75 - 3.70 (m, 2H), 3.44 (s, 3H), 3.34 (s, 3H), 2.12 (s, 3H). MS (ESI) m/e [M+1]⁺ 488. |
| D6 | 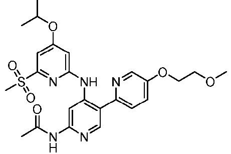 | N-(4'-((4-isopropoxy-6-(methylsulfonyl)pyridi n-2-yl)amino)-5-(2-methoxyethoxy)-[2,3'-bipyridin]-6'-yl)acetamide | ¹H NMR (400 MHz, DMSO-*d*₆) δ 12.36 (s, 1H), 10.50 (s, 1H), 9.12 (s, 1H), 8.67 (s, 1H), 8.56 (s, 1H), 8.03 (d, *J=* 9.1 Hz, 1H), 7.62 (d, *J=* 9.2 Hz, 1H), 7.06 (s, 1H), 6.83 (s, 1H), 4.95 - 4.91 (m, 1H), 4.30 - 4.25 (m, 2H), 3.75 - 3.71 (m, 2H), 3.43 (s, 3H), 3.34 (s, 3H), 2.11 (s, 3H), 1.33 *(d, J=* 5.4 Hz, 6H). MS (ESI) m/e [M+1]⁺ 516. |
| D7 | 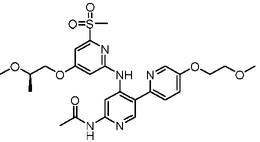 | (R)-N-(5-(2-methoxyethoxy)-4'-((4-(2-methoxypropoxy)-6-(methylsulfonyl)pyridi n-2-yl)amino)-[2,3'-bipyridin]-6'-yl)acetamide | ¹H NMR (400 MHz, DMSO-*d*₆) δ 12.41 (s, 1H), 10.52 (s, 1H), 9.12 (s, 1H), 8.68 (s, 1H), 8.57 (s, 1H), 8.03 (d, *J=* 8.8 Hz, 1H), 7.63 (d, *J=* 8.8 Hz, 1H), 7.11 (s, 1H), 6.88 (s, 1H), 4.30 - 4.25 (m, 2H), 4.20 - 4.15 (m, 2H), 3.72 (s, 3H), 3.44 (s, 3H), 3.35 - 3.28 (m, 6H), 2.12 (s, 3H), 1.19 (d, *J =* 5.6 Hz, 3H). MS (ESI) m/e [M+1]⁺ 546. |
| D8 | 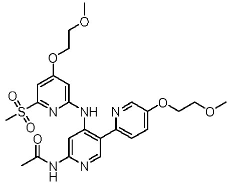 | N-(5-(2-methoxyethoxy)-4'-((4-(2-methoxyethoxy)-6-(methylsulfonyl)pyridi n-2-yl)amino)-[2,3'-bipyridin]-6'-yl)acetamide | ¹H NMR (400 MHz, DMSO-*d*₆) δ 12.45 (s, 1H), 10.49 (s, 1H), 9.13 (s, 1H), 8.68 (s, 1H), 8.56 (s, 1H), 8.03 (d, *J=* 8.9 Hz, 1H), 7.62 (d, *J=* 8.9 Hz, 1H), 7.11 (s, 1H), 6.87 (s, 1H), 4.33 - 4.28 (m, 4H), 3.75 - 3.70 (m, 4H), 3.44 (s, 3H), 3.33 (s, 3H), 3.32 (s, 3H), 2.12 (s, 3H). MS (ESI) m/e [M+1]⁺ 532. |
| D9 | 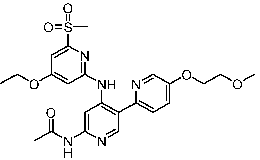 | N-(4'-((4-ethoxy-6-(methylsulfonyl)pyridi n-2-yl)amino)-5-(2-methoxyethoxy)-[2,3'-bipyridin]-6'-yl)acetamide | ¹H NMR (400 MHz, DMSO-*d*₆) δ 12.38 (s, 1H), 10.48 (s, 1H), 9.11 (s, 1H), 8.67 (s, 1H), 8.56 (s, 1H), 8.03 (d, *J=* 8.8 Hz, 1H), 7.62 (d, *J=* 8.8 Hz, 1H), 7.08 (s, 1H), 6.84 (s, 1H), 4.28 - 4.24 (m, 4H), 3.75 - 3.70 (m, 2H), 3.43 (s, 3H), 3.34 (s, 3H), 2.11 (s, 3H), 1.38 (t, *J =* 6.5 Hz, 3H). MS (ESI) m/e [M+1]⁺ 502. |
| D10 | 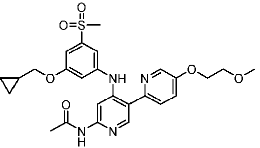 | N-(4'-((3-(cyclopropylmethoxy)-5-(methylsulfonyl)pheny l)amino)-5-(2-methoxyethoxy)-[2,3'-bipyridin]-6'-yl)acetamide | ¹H NMR (400 MHz, DMSO-*d*₆) δ 11.18 (s, 1H), 10.45 (s, 1H), 8.59 (s, 1H), 8.45 (s, 1H), 8.18 (s, 1H), 7.95 (d, *J =* 8.4 Hz, 1H), 7.58 (d, *J =* 8.4 Hz, 1H), 7.35 (s, 1H), 7.16 (s, 1H), 7.06 (s, 1H), 4.25 - 4.20 (m, 2H), 3.95 - 3.93 (m, 2H), 3.70 - 3.65 (m, 2H), 3.27 (s, 3H), 2.07 (s, 3H), 1.25 - 1.20 (m, 1H), 0.60 - 0.55 (m, 2H), 0.35 - 0.30 (m, 2H). MS (ESI) m/e [M+1]⁺ 527. |
| D11 | 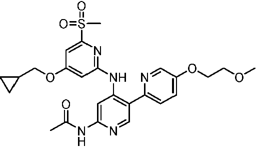 | N-(4'-((4-(cyclopropylmethoxy)-6-(methylsulfonyl)pyridi n-2-yl)amino)-5-(2-methoxyethoxy)-[2,3'-bipyridin]-6'-yl)acetamide | MS (ESI) m/e [M+1]⁺ 528. |

### Example E1: Synthesis of N-(5-(methoxymethyl)-4'-((3-(methylsulfonyl) phenyl)amino)-[2,3'-bipyridin]-6'-yl)acetamide

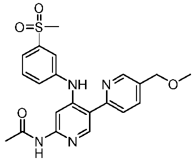

### Step 1: 2-bromo-5-(methoxymethyl)pyridine

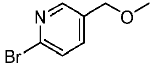

A solution of 2-bromo-5-(bromomethyl) pyridine (10 g, 39.85 mmol), NaOMe (9.6 mL, 51.8 mmol) in MeOH (150 mL) was stirred at RT overnight. The solvent was removed in vacuo and the residue was extracted between EA and H₂O. The organic layer was dried over Na₂SO₄. The organic layer was filtered, and the filtrate was concentrated under reduced pressure. The crude product was purified by silica gel column chromatography (PE/EA = 5:1) to give the desired product 2-bromo-5-(methoxymethyl) pyridine (7.8 g, 96.9% yield). MS (ESI) m/e [M+1]⁺ 202.

### Step 2: tert-butyl (6'-chloro-5-(methoxymethyl)-[2,3'-bipyridin]-4'-yl) carbamate

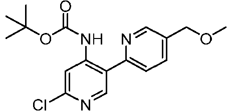

A mixture of 2-bromo-5-(methoxymethyl)pyridine (3 g, 14.84 mmol), Sn₂Me₆ (5.4 g, 16.48 mmol) and Pd(PPh₃)₄ (1.9 g, 1.65 mmol) in dioxane (50 mL) was stirred at 100 °C under N₂ overnight. The mixture was cooled to RT and then tert-butyl (5-bromo-2-chloropyridin-4-yl)carbamate (4.8 g, 15.6 mmol) and Pd(PPh₃)₂Cl₂ (1.2 g, 1.75 mmol) in dioxane (40 mL) was added under N₂. The mixture was stirred at 100 °C for 10 h under nitrogen atmosphere. After cooled to room temperature, the mixture was diluted with KF-H₂O (50 mL) and extracted with EA (100 mL x 2). The combined organic layers were dried over anhydrous Na₂SO₄ and concentrated under vacuum. The residue was purified by silica gel column chromatography (PE/EA = 10:1 to 5:1) to give tert-butyl (6'-chloro-5-(methoxymethyl)-[2,3'-bipyridin]-4'-yl)carbamate (930 mg, 17.9%). MS (ESI) m/e [M+1]⁺ 250.

### Step 3: N-(4'-amino-5-(methoxymethyl)-[2,3'-bipyridin]-6'-yl) acetamide TFA salt

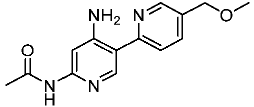

A solution of tert-butyl (6'-chloro-5-(methoxymethyl)-[2,3'-bipyridin]-4'-yl) carbamate (580 mg, 1.56 mmol) in DCM (4 mL) was added TFA (2 mL). The mixture was stirred at RT for 5 h. The solvent was removed, and the crude was used in the next step without further purification (400 mg, TFA salt). MS (ESI) m/e [M+1]⁺ 273.

### Step 4: N-(5-(methoxymethyl)-4'-((3-(methylsulfonyl)phenyl)amino)-[2,3'-bipyridin]-6'-yl)acetamide

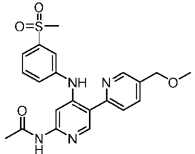

A solution of N-(4'-amino-5-(methoxymethyl)-[2,3'-bipyridin]-6'-yl) acetamide TFA salt (100 mg, 0.27 mmol), 1-bromo-3-(methylsulfonyl)benzene (174 mg, 0.74 mmol), Cs₂CO₃ (479.8 mg, 1.47 mmol), Xant-phos Pd G3 (70.2 mg, 0.074 mmol) and Xant-phos (85.2 mg, 0.15 mmol) in dioxane (3 mL) was stirred at 130 °C under N₂ overnight. The mixture was filtered and the filtrate as concentrated to give the crude residue which was purified by Prep-TLC (MeOH/DCM = 1: 10) to afford N-(5-(Cis-2,6-dimethylmorpholino)-4'-((4-methyl-6-(methylsulfonyl)pyridin-2-yl)amino)-[2,3'-bipyridin]-6'-yl)acetamide (45.9 mg, 39.9% yield). ¹H NMR (400 MHz, DMSO-*d6*) δ 11.48 (s, 1H), 10.50 (s, 1H), 8.68 (d, *J=* 7.9 Hz, 2H), 8.19 (s, 1H), 8.06 (d, *J* = 8.4 Hz, 1H), 7.89 (d, *J =* 8.4 Hz, 1H), 7.83 (s, 1H), 7.64 (s, 2H), 7.62 - 7.58 (m, 1H), 4.51 (s, 2H), 3.28 (s, 3H), 2.07 (s, 3H). MS (ESI) m/e [M+1]⁺ 427.

The following Examples were prepared in a similar manner to the product Example E1:

| **Example** | **Compound** | **Chemical Name** | **¹H NMR and LC /MS *m*/*z* (M+1)** |
|---|---|---|---|
| E2 | 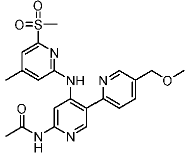 | N-(5-(methoxymethyl)-4'-((4-methyl-6-(methylsulfonyl)pyridin-2-yl)amino)-[2,3'-bipyridin]-6'-yl)acetamide | ¹H NMR (400 MHz, DMSO-*d*₆) δ 12.94 (s, 1H), 10.54 (s, 1H), 9.25 (s, 1H), 8.85 - 8.75 (m, 2H), 8.12 (d, *J=* 8.4 Hz, 1H), 7.94 (d, *J =* 8.4 Hz, 1H), 7.44 (s, 1H), 7.27 (s, 1H), 4.55 (s, 2H), 3.46 (s, 3H), 3.37 (s, 3H), 2.42 (s, 3H), 2.12 (s, 3H). MS (ESI) m/e [M+1]⁺ 442. |
| E3 | 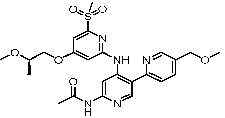 | (R)-N-(5-(methoxymethyl)-4'-((4-(2-methoxypropoxy)-6-(methylsulfonyl)pyridin-2-yl)amino)-[2,3'-bipyridin]-6'-yl)acetamide | ¹H NMR (400 MHz, DMSO-*d*₆) δ 12.69 (s, 1H), 10.55 (s, 1H), 9.16 (s, 1H), 8.80 (s, 1H), 8.77 (s, 1H), 8.11 *(d, J=* 8.5 Hz, 1H), 7.93 *(d, J* = 8.5 Hz, 1H), 7.11 (s, 1H), 6.93 (s, 1H), 4.54 (s, 2H), 4.25 - 4.12 (m, 2H), 3.71 (s, 1H), 3.44 (s, 3H), 3.37 (s, 3H), 2.12 (s, 3H), 1.20 *(d, J* = 6.2 Hz, 3H). MS (ESI) m/e [M+1]⁺ 516. |
| E4 | 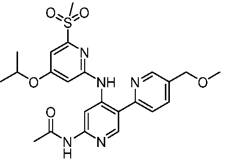 | N-(4'-((4-isopropoxy-6-(methylsulfonyl)pyridin-2-yl)amino)-5-(methoxymethyl)-[2,3'-bipyridin]-6'-yl)acetamide | ¹H NMR (400 MHz, DMSO-*d*₆) δ 12.62 (s, 1H), 10.54 (s, 1H), 9.14 (s, 1H), δ 8.79 (s, 1H), 8.77 (s, 1H), 8.10 (d, *J=* 8.4 Hz, 1H), 7.92 (d, *J* = 8.4 Hz, 1H), 7.06 (s, 1H), 6.88 (s, 1H), 5.01 - 4.83 (m, 1H), 4.54 (s, 2H), 3.43 (s, 3H), 3.37 (s, 3H), 2.12 (s, 3H), 1.33 (d, *J* = 5.9 Hz, 6H). MS (ESI) m/e [M+1]⁺ 486. |
| E5 | 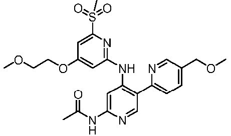 | N-(4'-((4-(2-methoxyethoxy)-6-(methylsulfonyl)pyridin-2-yl)amino)-5-(methoxymethyl)-[2,3'-bipyridin]-6'-yl)acetamide | ¹H NMR (400 MHz, DMSO- *d*₆) δ 12.74 (s, 1H), 10.55 (s, 1H), 9.17 (s, 1H), 8.80 (s, 1H), 8.75 (s, 1H), 8.11 *(d, J=* 8.1 Hz, 1H), 7.92 *(d, J* = 8.1 Hz, 1H), 7.11 (s, 1H), 6.92 (s, 1H), 4.54 (s, 2H), 4.40 - 4.30 (m, 2H), 3.75 - 3.65 (m, 2H), 3.44 (s, 3H), 3.37 (s, 3H), 3.32 (s, 3H), 2.12 (s, 3H). MS (ESI) m/e [M+1]⁺ 502. |
| E6 | 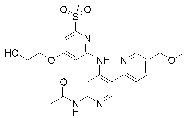 | N-(4'-((4-(2-hydroxyethoxy)-6-(methylsulfonyl)pyridin-2-yl)amino)-5-(methoxymethyl)-[2,3'-bipyridin]-6'-yl)acetamide | ¹H NMR (400 MHz, DMSO-*d*₆) δ 12.69 (s, 1H), 10.54 (s, 1H), 9.17 (s, 1H), 8.80 (s, 1H), 8.78 (s, 1H), 8.11 (d, *J=* 8.0 Hz, 1H), 7.92 *(d, J* = 8.0 Hz, 1H), 7.10 (s, 1H), 6.91 (s, 1H), 4.98 (s, 1H), 4.54 (s, 2H), 4.30 - 4.20 (m, 2H), 3.80 - 3.70 (m, 2H), 3.44 (s, 3H), 3.37 (s, 3H), 2.12 (s, 3H). MS (ESI) m/e [M+1]⁺ 488. |
| E7 | 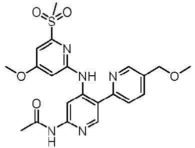 | N-(4'-((4-methoxy-6-(methylsulfonyl)pyridin-2-yl)amino)-5-(methoxymethyl)-[2,3'-bipyridin]-6'-yl)acetamide | ¹H NMR (400 MHz, DMSO-*d*₆) δ 12.68 (s, 1H), 10.55 (s, 1H), 9.15 (s, 1H), 8.78 (s, 1H), 8.72 (s, 1H), 8.11 (d, *J=* 8.4 Hz, 1H), 7.93 (d, *J* = 8.4 Hz, 1H), 7.11 (s, 1H), 6.92 (s, 1H), 4.54 (s, 2H), 3.96 (s, 3H), 3.44 (s, 3H), 3.37 (s, 3H), 2.12 (s, 3H). MS (ESI) m/e [M+1]⁺ 458. |
| E8 | 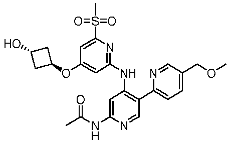 | N-(4'-((4-((1r,3r)-3-hydroxycyclobutoxy)-6-(methylsulfonyl)pyridin-2-yl)amino)-5-(methoxymethyl)-[2,3'-bipyridin]-6'-yl)acetamide | ¹H NMR (400 MHz, DMSO-*d*₆) δ = 12.56 (s, 1H), 10.54 (s, 1H), 9.12 (s, 1H), 8.76 (s, 2H), 8.09 (d, *J =* 8.1 Hz, 1H), 7.92 (d, *J=* 8.4 Hz, 1H), 6.99 (s, 1H), 6.73 (s, 1H), 5.28 (d, *J=* 5.4 Hz, 1H), 5.12 - 5.06 (m, 1H), 4.54 (s, 2H), 4.42 - 4.36 (m, 1H), 3.42 (s, 3H), 3.37 (s, 3H), 2.39 - 2.35 (m, 4H), 2.12 (s, 3H). MS (ESI) m/e [M+1]⁺ 514. |
| E9 | 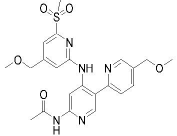 | N-(5-(methoxymethyl)-4'-((4-(methoxymethyl)-6-(methylsulfonyl)pyridin-2-yl)amino)-[2,3'-bipyridin]-6'-yl)acetamide | ¹H NMR (400 MHz, DMSO-*d*₆) δ 12.89 (s, 1H), 10.56 (s, 1H), 9.26 (s, 1H), δ 8.79 (s, 1H), 8.77 (s, 1H), 8.12 *(d, J=* 8.3 Hz, 1H), 7.93 *(d, J* = 8.3 Hz, 1H), 7.50 (s, 1H), 7.31 (s, 1H), δ 4.56 (s, 2H), 4.55 (s, 2H), 3.48 (s, 3H), 3.39 (s, 2H), 3.37 (s, 2H), 2.13 (s, 3H). MS (ESI) m/e [M+1]⁺ 472. |

### Example F1: Synthesis of N-(4'-((4-methyl-6-(methylsulfonyl)pyridin-2-yl)amino)-5-(trifluoromethyl)-[2,3'-bipyridin]-6'-yl)acetamide

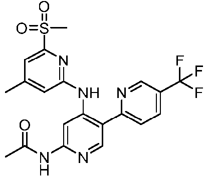

A mixture of N-(4'-amino-5-(trifluoromethyl)-[2,3'-bipyridin]-6'-yl)acetamide (100 mg, 0.34 mmol), 2-bromo-4-methyl-6-(methylsulfonyl)pyridine (100 mg, 0.4 mmol), Pd₂dba₃ (27 mg, 0.03 mmol), BINAP (18 mg, 0.03 mmol) and Cs₂CO₃ (208 mg, 0.64 mmol) in dioxane (6 mL) was stirred at 130 °C under N₂ in a sealed tube for 4 h. The reaction mixture was filtered and the solid was washed with EA (10 mL). The filtrate was concentrated and the residue was purified by prep-TLC (DCM/MeOH = 20: 1) to give N-(4'-((4-methyl-6-(methyl sulfonyl)pyridin-2-yl)amino)-5-(trifluoromethyl)-[2,3'-bipyridin]-6'-yl)acetamide (37 mg, 23 %). ¹H NMR (400 MHz, DMSO-*d*₆) δ 12.42 (s, 1H), 10.61 (s, 1H), 9.26 (s, 1H), 9.20 (s, 1H), 8.84 (s, 1H), 8.32 - 8.30 (m, 2H), 7.44 (s, 1H), 7.31 (s, 1H), 3.44 (s, 3H), 2.41 (s, 3H), 2.11 (s, 3H). MS (ESI) m/e [M+1]⁺ 466.

### Example F17: Synthesis of N-(5-fluoro-4'-((4-methyl-6-(methylsulfonyl)pyridin-2-yl)amino)-[2,3'-bipyridin]-6'-yl)acetamide

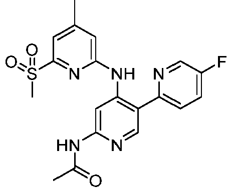

A mixture of N-(4'-amino-5-fluoro-[2,3'-bipyridin]-6'-yl)acetamide (800 mg, 3.25 mmol), 2-bromo-4-methyl-6-(methylsulfonyl)pyridine (976 mg, 3.9 mmol), Pd₂(dba)₃ (297 mg, 0.325 mmol), BINAP (405 mg, 0.65 mmol) and Cs₂CO₃ (1.59 g, 4.875 mmol) in 1,4-dioxane (14 mL) was stirred 130 °C for 3 hr under N₂ atmosphere. The reaction was cooled to room temperature, filtered and the filtration was concentrated under vacuum. The residue was purified by silica gel column chromatography (DCM/MeOH = 100 : 1) to afford the product (600 mg, 44%). ¹H NMR (400 MHz, DMSO-*d*₆) δ 12.06 (s, 1H), 10.53 (s, 1H), 9.20 (s, 1H), 8.80 (s, 1H), 8.71 (s, 1H), 8.16 (d, *J=* 7.3 Hz, 1H), 7.95 (d, *J =* 7.3 Hz, 1H), 7.44 (s, 1H), 7.23 (s, 1H), 3.44 (s, 3H), 2.41 (s, 3H), 2.12 (s, 3H). MS (ESI) m/e [M+1]⁺ 416.

### Example F22: Synthesis of N-(5-fluoro-4'-((4-methoxy-6-(methylsulfonyl)pyridin-2-yl)amino)-[2,3'-bipyridin]-6'-yl)acetamide

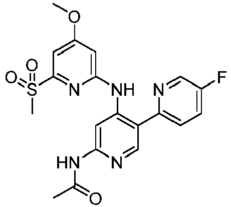

### Step 1: 6'-chloro-5-fluoro-[2,3'-bipyridin]-4'-amine

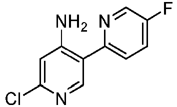

A mixture of 2-bromo-5-fluoropyridine (1.6 g, 9.09 mmol), 2-chloro-5-(4,4,5,5-tetramethyl-1,3,2-dioxaborolan-2-yl)pyridin-4-amine (2.54 g, 10 mmol), Pd(dppf)Cl₂ (664 mg, 0.909 mmol) and K₂CO₃ (1.88 g, 13.64 mmol) in 1,4-dioxane (50 mL) and H₂O (5 mL) was stirred at 100 °C for 2 hr. The reaction was cooled to room temperature and diluted with EA, washed with brine, dried and concentrated. The residue was purified by silica gel column chromatography (EA/PE = 1:3) to give the product (1.9 g). MS (ESI) m/e [M+1]⁺ 224.

### Step 2: N-(4'-amino-5-fluoro-[2,3'-bipyridin]-6'-yl)acetamide

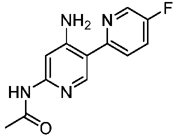

A mixture 6'-chloro-5-fluoro-[2,3'-bipyridin]-4'-amine (1.4 g, 6.25 mmol), acetamide (2.21 g, 37.5 mmol), Pd2(dba)3 (572 mg, 0.625 mmol), Xantphos (724 mg, 1.25 mmol) and Cs₂CO₃ (4.08 g, 12.5 mmol) in 1,4-dioxane (20 mL) was degassed with nitrogen and heated to 130 °C in a sealed tube stirring overnight. The reaction was cooled to room temperature and concentrated under vacuum. The residue was purified by silica gel column chromatography (DCM/MeOH = 50:1) to give the product (1.4 g, crude). MS (ESI) m/e [M+1]⁺ 247.

### Step 3: N-(5-fluoro-4'-((4-methoxy-6-(methylsulfonyl)pyridin-2-yl)amino)-[2,3'-bipyridin]-6'-yl)acetamide

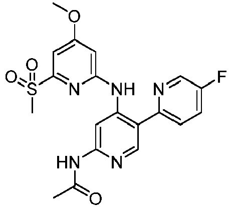

A mixture of N-(4'-amino-5-fluoro-[2,3'-bipyridin]-6'-yl)acetamide (1.5 g, 6.07 mmol), 2-bromo-4-methoxy-6-(methylsulfonyl)pyridine (1.78 g, 6.68 mmol), Pd₂(dba)₃ (555 mg, 0.607 mmol), BINAP (765 mg, 1.214 mmol) and Cs₂CO₃ (3.96 g, 12.14 mmol) in 1,4-dioxane (30 mL) was degassed with nitrogen and heated to 130 °C in a sealed tube stirring for 3 hr. The reaction was cooled to room temperature, filtered and the filtration was concentrated under vacuum. The residue was purification by column flash on silica gel eluted with DCM/MeOH (100: 1) to afford the desired product as a light yellow solid. It was then washed with 10mL of MeCN to give the product (27 mg, 30%). ¹H NMR (400 MHz, DMSO-*d*₆) δ 11.82 (s, 1H), 10.54 (s, 1H), 9.10 (s, 1H), 8.81 (s, 1H), 8.68 (s, 1H), 8.20 - 8.06 (m, 1H), 7.95 - 7.90 (m, 1H), 7.11 (s, 1H), 6.90 (s, 1H), 3.95 (s, 3H), 3.42 (s, 3H), 2.12 (s, 3H). MS (ESI) m/e [M+1]⁺ 432.

### Example F23: Synthesis of N-(4'-((3,4-dimethoxy-6-(methylsulfonyl)pyridin-2-yl)amino)-5-fluoro-[2,3'-bipyridin]-6'-yl)acetamide

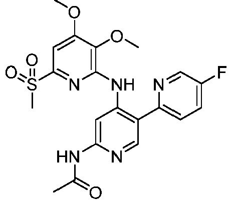

A mixture of N-(4'-amino-5-fluoro-[2,3'-bipyridin]-6'-yl)acetamide(40 mg, 0.163 mmol), 2-bromo-3,4-dimethoxy-6-(methylsulfonyl)pyridine (53 mg, 0.179 mmol), Pd₂(dba)₃ (15 mg, 0.0163 mmol), BINAP (20 mg, 0.0326 mmol) and Cs₂CO₃ (106 mg, 0.326 mmol) in 1,4-dioxane (6 mL) was degassed with nitrogen and heated to 130 °C in a sealed tube stirring for 4 hr. The reaction was cooled to room temperature, filtered and the filtration was concentrated under vacuum. The residue was applied onto Prep-TLC with (DCM/MeOH = 20: 1) to afford the product (40 mg, 30%). ¹H NMR (400 MHz, DMSO-*d*₆) δ 12.64 (s, 1H), 10.53 (s, 1H), 9.40 (s, 1H), 8.82 (s, 1H), 8.75 (s, 1H), 8.21 (d, *J* = 4.8 Hz, 1H), 7.99 *(d, J=* 6.4 Hz, 1H), 7.38 (s, 1H), 4.02 (s, 3H), 3.97 (s, 3H), 3.50 (s, 3H), 2.13 (s, 3H). MS (ESI) m/e [M+1]⁺ 462.

### Example F24: Synthesis of N-(5-fluoro-4'-((4-(methoxy-d3)-6-(methylsulfonyl)pyridine-2-yl)amino)-[2,3'-bipyridin]-6'-yl)acetamide

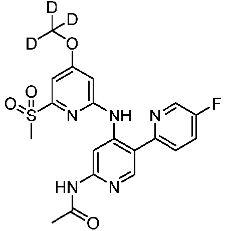

A mixture of N-(4'-amino-5-fluoro-[2,3'-bipyridin]-6'-yl)acetamide (40 mg, 0.163 mmol), 2-bromo-4-(methoxy-d3)-6-(methylsulfonyl)pyridine (48 mg, 0.179 mmol), Pd₂(dba)₃ (15 mg, 0.0163 mmol), BINAP (20 mg, 0.0326 mmol) and Cs₂CO₃ (132 mg, 0.326 mmol) in 1,4-dioxane (6 mL) was degassed with nitrogen and heated to 130 °C in a sealed tube stirring for 3 hr. The reaction was cooled to room temperature, filtered and the filtration was concentrated under vacuum. The residue was applied onto Prep-TLC (DCM/MeOH = 20: 1) to afford the product (30 mg, 30%). ¹H NMR (400 MHz, DMSO-*d*₆) δ 11.81 (s, 1H), 10.54 (s, 1H), 9.10 (s, 1H), 8.81 (s, 1H), 8.68 (s, 1H), 8.13 (d, *J=* 7.5 Hz, 1H), 7.94 (d, *J*= 7.5 Hz, 1H), 7.11 (s, 1H), 6.89 (s, 1H), 3.42 (s, 3H), 2.12 (s, 3H). MS (ESI) m/e [M+1]⁺ 435.

### Example F25: Synthesis of N-(4'-((4-cyclopropyl-6-(methylsulfonyl)pyridin-2-yl)amino)-5-fluoro-[2,3'-bipyridin]-6'-yl)acetamide

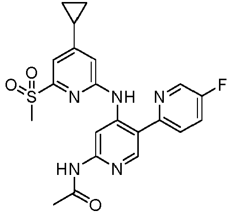

A mixture of N-(4'-amino-5-fluoro-[2,3'-bipyridin]-6'-yl)acetamide(50 mg, 0.203 mmol), 2-chloro-4-cyclopropyl-6-(methylsulfonyl)pyridine (57 mg, 0.244 mmol), Pd₂(dba)₃ (19 mg, 0.0203 mmol), BINAP (25 mg, 0.0406 mmol) and Cs₂CO₃ (132 mg, 0.406 mmol) in 1,4-dioxane (6 mL) was degassed with nitrogen and heated to 130 °C in a sealed tube stirring for 4 hr. The reaction was cooled to room temperature, filtered and the filtration was concentrated under vacuum. The residue was applied onto Prep-TLC with (DCM/MeOH = 20 : 1) to afford the product (23 mg, 25%). ¹H NMR (400 MHz, DMSO-*d*₆) δ 11.84 (s, 1H), 10.49 (s, 1H), 9.06 (s, 1H), 8.77 (s, 1H), 8.64 (s, 1H), 8.10 - 8.08 (m, 1H), 7.95 - 7.90 (m, 1H), 7.22 (s, 1H), 7.02 (s, 1H), 3.37 (s, 3H), 2.07 (s, 4H), 1.96 -1.94 (m, 1H), 1.12-1.05 (m, 2H), 0.95 - 0.88(m, 2H). MS (ESI) m/e [M+1]⁺ 442.

The following Examples were prepared in a similar manner to the product Example F1:

| **Example** | **Compound** | **Chemical Name** | **¹H NMRand LC /MS *m*/*z* (M+1)** |
|---|---|---|---|
| F2 | 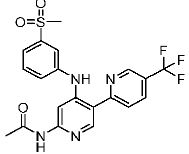 | N-(4'-((3-(methylsulfonyl)phenyl)amino)-5-(trifluoromethyl)-[2,3'-bipyridin]-6'-yl)acetamide | ¹H NMR (400 MHz, DMSO-*d*₆) δ 10.99 (s, 1H), 10.56 (s, 1H), 9.07 (s, 1H), 8.72 (s, 1H), 8.28 (s, 1H), 8.25 (s, 1H), 8.16 (s, 1H), 7.81 (s, 1H), 7.65 -7.61 (m, 3H), 3.26 (s, 3H), 2.05 (s, 3H). MS (ESI) m/e [M+1]⁺ 451. |
| F3 | 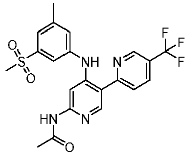 | N-(4'-((3-methyl-5-(methylsulfonyl)phenyl)amino)-5-(trifluoromethyl)-[2,3'-bipyridin]-6'-yl)acetamide | ¹H NMR (400 MHz, DMSO-*d*₆) δ 11.02 (s, 1H), 10.56 (s, 1H), 9.09 (s, 1H), 8.74 (s, 1H), 8.41 - 8.12 (m, 3H), 7.64 (s, 1H), 7.46 (s, 2H), 3.26 (s, 3H), 2.41 (s, 3H), 2.08 (s, 3H). MS (ESI) m/e [M+1]⁺ 465. |
| F4 | 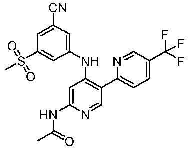 | N-(4'-((3-cyano-5-(methylsulfonyl)phenyl)amino)-5-(trifluoromethyl)-[2,3'-bipyridin]-6'-yl)acetamide | ¹H NMR (400 MHz, DMSO-*d*₆) δ 11.06 (s, 1H), 10.67 (s, 1H), 9.08 (s, 1H), 8.75 (s, 1H), 8.31 (s, 1H), 8.21 (s, 2H), 8.07 (s, 2H), 7.98 (s, 1H), 2.10 (s, 3H). MS (ESI) m/e [M+1]⁺ 476. |
| F5 | 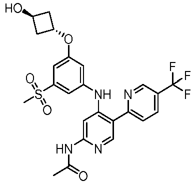 | N-(4'-((3-((trans)-3-hydroxycyclobutoxy)-5-(methylsulfonyl)phenyl)amino)-5-(trifluoromethyl)-[2,3'-bipyridin]-6'-yl)acetamide | ¹H NMR (400 MHz, DMSO-*d*₆) δ 10.92 (s, 1H), 10.56 (s, 1H), 9.09 (s, 1H), 8.72 (s, 1H), 8.29 - 8.20 (m, 3H), 7.40 (s, 1H), 7.03 (s, 1H), 6.98 (s, 1H), 5.25 - 5.19 (m, 1H), 4.95 - 4.90 (m, 1H), 4.37 - 4.32 (m, 1H), 3.27 (s, 3H), 2.35 - 2.30 (m, 4H), 2.08 (s, 3H). MS (ESI) m/e [M+1]⁺ 537. |
| F6 | 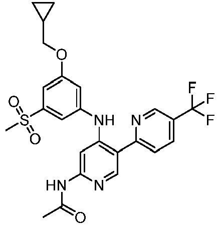 | N-(4'-((3-(cyclopropylmethoxy)-5-(methylsulfonyl)phenyl)amino)-5-(trifluoromethyl)-[2,3'-bipyridin]-6'-yl)acetamide | ¹H NMR (400 MHz, DMSO-*d*₆) δ 11.01 (s, 1H), 10.56 (s, 1H), 9.10 (s, 1H), 8.73 (s, 1H), 8.32 - 8.22 (m, 3H), 7.39 (s, 1H), 7.19 (s, 1H), 7.10 (s, 1H), 3.95 (d, *J =* 6.6 Hz, 2H), 3.27 (s, 3H), 2.08 (s, 3H), 1.30 - 1.24 (m, 1H), 0.59 - 0.58 (m, 2H), 0.35 - 0.30 (m, 2H). MS (ESI) m/e [M+1]⁺ 521. |
| F7 | 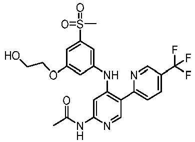 | N-(4'-((3-(2-hydroxyethoxy)-5-(methylsulfonyl)phenyl)amino)-5-(trifluoromethyl)-[2,3'-bipyridin]-6'-yl)acetamide | ¹H NMR (400 MHz, DMSO-*d*₆) δ 11.00 (s, 1H), 10.57 (s, 1H), 9.10 (s, 1H), 8.74 (s, 1H), 8.30 - 8.26 (m, 3H), 7.41 (s, 1H), 7.21 (s, 1H), 7.12 (s, 1H), 4.93 (s, 1H), 4.11 (s, 2H), 3.74 (s, 2H), 3.28 (s, 3H), 2.09 (s, 3H). MS (ESI) m/e [M+1]⁺ 511. |
| F8 | 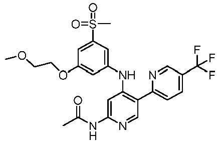 | N-(4'-((3-(2-methoxyethoxy)-5-(methylsulfonyl)phenyl)amino)-5-(trifluoromethyl)-[2,3'-bipyridin]-6'-yl)acetamide | ¹H NMR (400 MHz, DMSO-*d*₆) δ 11.02 (s, 1H), 10.58 (s, 1H), 9.10 (s, 1H), 8.74 (s, 1H), 8.34 - 8.21 (m, 3H), 7.41 (s, 1H), 7.22 (s, 1H), 7.12 (s, 1H), 4.23 (s, 2H), 3.69 (s, 2H), 3.28 (s, 3H), 2.09 (s, 3H). MS (ESI) m/e [M+1]⁺ 525. |
| F9 | 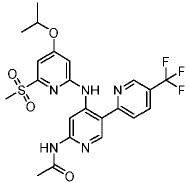 | N-(4'-((4-isopropoxy-6-(methylsulfonyl)pyridin-2-yl)amino)-5-(trifluoromethyl)-[2,3'-bipyridin]-6'-yl)acetamide | 1H NMR (400 MHz, DMSO-*d*₆) δ 12.13 (s, 1H), 10.62 (s, 1H), 9.20 - 9.15 (m, 2H), 8.83 (s, 1H), 8.40 - 8.23 (m, 2H), 7.09 (s, 1H), 6.93 (s, 1H), 4.92 (s, 1H), 3.43 (s, 3H), 2.13 (s, 3H), 1.33 (d,*J =* 5.0 Hz, 6H). MS (ESI) m/e [M+1]⁺ 510. |
| F10 | 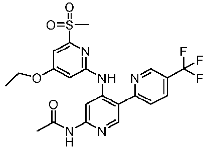 | N-(4'-((4-ethoxy-6-(methylsulfonyl)pyridin-2-yl)amino)-5-(trifluoromethyl)-[2,3'-bipyridin]-6'-yl)acetamide | ¹H NMR (400 MHz, DMSO-*d*₆) *δ* 12.21 (s, 1H), 10.62 (s, 1H), 9.22 (s, 1H), 9.19 (s, 1H), 8.83 (s, 1H), 8.35 - 8.32 (m, 2H), 7.11 (s, 1H), 6.94 (s, 1H), 4.28 - 4.24 (m, 2H), 3.44 (s, 3H), 2.13 (s, 3H), 1.40 - 1.38 (m, 3H). MS (ESI) m/e [M+1]⁺ 496. |
| F11 | 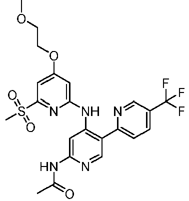 | N-(4'-((4-(2-methoxyethoxy)-6-(methylsulfonyl)pyridin-2-yl)amino)-5-(trifluoromethyl)-[2,3'-bipyridin]-6'-yl)acetamidecarboxamide | ¹H NMR (400 MHz, DMSO-*d*₆) δ 12.27 (s, 1H), 10.63 (s, 1H), 9.25 - 9.20 (m, 2H), 8.85 (s, 1H), 8.34 (d, *J=* 9.4 Hz, 2H), 7.14 (s, 1H), 6.98 (s, 1H), 4.34 (s, 2H), 3.71 (s, 2H), 3.44 (s, 3H), 2.13 (s, 3H). MS (ESI) m/e [M+1]⁺ 526. |
| F12 | 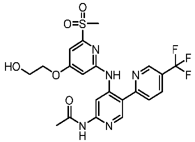 | N-(4'-((4-(2-hydroxyethoxy)-6-(methylsulfonyl)pyridin-2-yl)amino)-5-(trifluoromethyl)-[2,3'-bipyridin]-6'-yl)acetamide | ¹H NMR (400 MHz, DMSO-d6) δ 12.19 (s, 1H), 10.62 (s, 1H), 9.25 - 9.20 (m, 2H), 8.83 (s, 1H), 8.42 - 8.24 (m, 2H), 7.13 (s, 1H), 6.96 (s, 1H), 4.98 (s, 1H), 4.22 (s, 2H), 3.77 (s, 2H), 3.44 (s, 3H), 2.13 (s, 3H). MS (ESI) m/e [M+1]⁺ 512. |
| F13 | 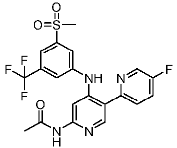 | N-(5-fluoro-4'-((3-(methylsulfonyl)-5-(trifluoromethyl)phenyl)amino)-[2,3'-bipyridin]-6'-yl)acetamide | ¹H NMR (400 MHz, DMSO-*d*₆) *δ* 10.68 (s, 1H), 10.58 (s, 1H), 8.71 (s, 1H), 8.60 (s, 1H), 8.21 (s, 1H), 8.05 (s, 2H), 7.99 - 7.90 (m, 2H), 7.77 (s, 1H), 3.37 (s, 3H), 2.09 (s, 3H). MS (ESI) m/e [M+1]⁺ 460. |
| F14 | 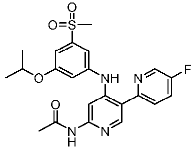 | N-(5-fluoro-4'-((3-isopropoxy-5-(methylsulfonyl)phenyl)amino)-[2,3'-bipyridin]-6'-yl)acetamide | ¹H NMR (400 MHz, DMSO-*d*₆) *δ* 10.69 (s, 1H), 10.49 (s, 1H), 8.71 (s, 1H), 8.60 (s, 1H), 8.18 (s, 1H), 8.07 (d, *J*= 7.2 Hz, 1H), 7.90 (d, *J=* 7.2 Hz, 1H), 7.35 (s, 1H), 7.11 (s, 1H), 7.05 (s, 1H), 4.73 (s, 1H), 3.27 (s, 3H), 2.07 (s, 3H), 1.30 (d, *J =* 5.1 Hz, 6H). MS (ESI) m/e [M+1]⁺ 459. |
| F15 | 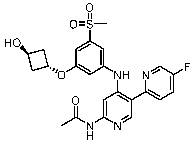 | N-(5-fluoro-4'-((3-((trans)-3-hydroxycyclobutoxy)-5-(methylsulfonyl)phenyl)amino)-[2,3'-bipyridin]-6'-yl)acetamide | ¹H NMR (400 MHz, DMSO-*d*₆) δ 10.64 (s, 1H), 10.49 (s, 1H), 8.71 (s, 1H), 8.59 (s, 1H), 8.18 (s, 1H), 8.07 (d, *J*= 7.2 Hz, 1H), 7.89 (d, *J=* 7.2 Hz, 1H), 7.37 (s, 1H), 7.00 (s, 1H), 6.95 (s, 1H), 5.20 (s, 1H), 4.94 - 4.90 (m, 1H), 4.37 - 4.32 (m, 1H), 3.26 (s, 3H), 2.35 - 2.30 (m, 4H), 2.08 (s, 3H). MS (ESI) m/e [M+1]⁺ 487. |
| F16 | 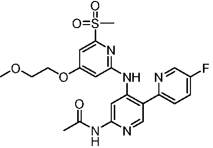 | N-(5-fluoro-4'-((4-(2-methoxyethoxy)-6-(methylsulfonyl)pyridin-2-yl)amino)-[2,3'-bipyridin]-6'-yl)acetamide | ¹H NMR (400 MHz, DMSO-*d*₆) *δ* 11.91 (s, 1H), 10.55 (s, 1H), 9.12 (s, 1H), 8.81 (s, 1H), 8.70 (s, 1H), 8.18 - 8.10 (m, 1H), 7.99 - 7.90 (m, 1H), 7.11 (s, 1H), 6.90 (s, 1H), 4.36 - 4.28 (m, 2H), 3.74 - 3.66 (m, 2H), 3.42 (s, 3H), 3.32 (s, 3H), 2.12 (s, 3H). MS (ESI) m/e [M+1]⁺ 476. |
| F18 | 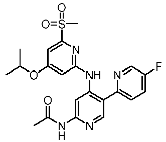 | N-(5-fluoro-4'-((4-isopropoxy-6-(methylsulfonyl)pyridin-2-yl)amino)-[2,3'-bipyridin]-6'-yl)acetamide | ¹H NMR (400 MHz, DMSO-*d*₆) δ 11.79 (s, 1H), 10.53 (s, 1H), 9.10 (s, 1H), 8.82 (s, 1H), 8.68 (s, 1H), 8.12 - 7.94 (m, 2H), 7.06 (s, 1H), 6.86 (s, 1H), 4.90 - 4.88 (m, 1H), 3.42 (s, 3H), 2.12 (s, 3H), 1.33 (d, *J=* 5.2 Hz, 6H). MS (ESI) m/e [M+1]⁺ 460. |
| F19 | 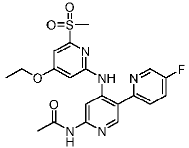 | N-(4'-((4-ethoxy-6-(methylsulfonyl)pyridin-2-yl)amino)-5-fluoro-[2,3'-bipyridin]-6'-yl)acetamide | ¹H NMR (400 MHz, DMSO-*d*₆) *δ* 11.85 (s, 1H), 10.55 (s, 1H), 9.12 (s, 1H), 8.82 (s, 1H), 8.69 (s, 1H), 8.13 -7.95 (m, 2H), 7.08 (s, 1H), 6.87 (s, 1H), 4.26 - 4.23 (m, 2H), 3.42 (s, 3H), 2.12 (s, 3H), 1.37 (t, *J =* 6.3 Hz , 3H). MS (ESI) m/e [M+1]⁺ 446. |
| F20 | 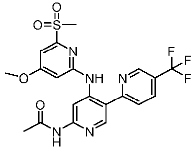 | N-(4'-((4-methoxy-6-(methylsulfonyl)pyridin-2-yl)amino)-5-(trifluoromethyl)-[2,3'-bipyridin]-6'-yl)acetamide | ¹H NMR (400 MHz, DMSO-*d*₆) δ 12.16 (s, 1H), 10.61 (s, 1H), 9.21(s, 1H), 9.17 (s, 1H), 8.83 (s, 1H), 8.36 - 8.29 (m, 2H), 7.13 (s, 1H), 6.95 (s, 1H), 3.96 (s, 3H), 3.43 (s, 3H), 2.13 (s, 3H). MS (ESI) m/e [M+1]⁺ 482. |
| F21 | 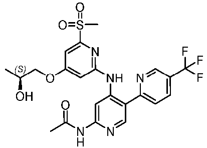 | (S)-N-(4'-((4-(2-hydroxypropoxy)-6-(methylsulfonyl)pyridin-2-yl)amino)-5-(trifluoromethyl)-[2,3'-bipyridin]-6'-yl)acetamide | ¹H NMR (400 MHz, DMSO-*d*₆) *δ* 12.10 (s, 1H), 10.60 (s, 1H), 9.22 (s, 1H), 9.18 (s, 1H), 8.82 (s, 1H), 8.35 (d, *J*= 8.3 Hz, 1H), 8.30 (d, *J* = 8.3 Hz, 1H), 7.15 - 7.13 (m, 1H), 6.99 - 6.95 (m, 1H), 4.98 (s, 1H), 4.05 - 4.00 (m, 2H), 3.43 (s, 3H), 2.13 (s, 3H), 1.99 - 1.95 (m, 1H), 1.20 - 1.15 (m, 3H). MS (ESI) m/e [M+1]⁺ 526. |

### Example G1: Synthesis of N-(4'-((3-(methylsulfonyl)phenyl)amino)-5-(3-oxomorpholino)-[2,3'-bipyridin]-6'-yl)acetamide

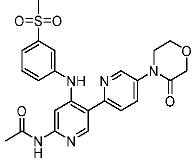

### Step 1: 2-(2-chloroethoxy)acetyl chloride

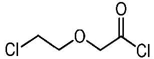

A mixture of 2-(2-chloroethoxy)acetic acid (1.0 g, 7.2 mmol) and DMF (0.1 mL) in SOCl₂ (20 mL) was heated at 70 °C for 3 h. After cooled to room temperature, the solvent was removed in vacuo to give 2-(2-chloroethoxy)acetyl chloride (1.1 g, crude).

### Step 2: N-(6-bromopyridin-3-yl)-2-(2-chloroethoxy)acetamide

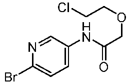

To a mixture of 2-(2-chloroethoxy)acetyl chloride (1.1 g, 7.0 mmol) in THF (20 mL) was added 6-bromopyridin-3-amine (1.2 g, 7.0 mmol) and Et₃N (2.1 g, 21.0 mmol) and the resulting mixture was stirred at room temperature for 12 h. Upon completion of the reaction, the solvent was removed in vacuo and the residue was purified by silica gel column chromatography (PE/EA = 2:1 to 1:1) to give N-(6-bromopyridin-3-yl)-2-(2-chloroethoxy)acetamide (1.5 g, 72.8%). ¹H NMR (400MHz, CDCl₃) δ 8.59 (s, 1 H), 8.48 (s, 1 H), 8.11 (d, *J=* 8.4 Hz, 1 H), 7.47 (d, *J=* 8.4 Hz, 1 H), 4.18 (s, 2 H), 3.94 - 3.89 (m, 2 H), 3.80 - 3.76 (m, 2 H). MS (ESI) m/e [M+1]⁺ 293.

### Step 3: 4-(6-bromopyridin-3-yl)morpholin-3-one

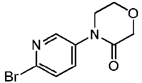

To a solution of N-(6-bromopyridin-3-yl)-2-(2-chloroethoxy)acetamide (1.5 g, 5.1 mmol) in DMF (20 mL) was added NaH (60% in mineral oil, 307 mg, 7.7 mmol) portionwise at 0 °C. Then the mixture was stirred at room temperature for 2 h. Upon completion of the reaction, water was added and the resulting mixture was extracted with EA (50 mL x 3). The combined organic layers were concentrated under vacuum to give 4-(6-bromopyridin-3-yl)morpholin-3-one (1.1 g, 84.6%). ¹H NMR (400 MHz, CDCl₃) δ 8.35 - 8.30 (m, 1 H), 7.61 (s, 1 H), 7.49 - 7.42 (m, 1 H), 4.28 (s, 2 H), 4.02 - 3.97 (m, 1 H), 4.02 - 3.97 (m, 1 H), 3.76 - 3.71 (m, 1 H), 3.76 - 3.71 (m, 1 H). MS (ESI) m/e [M+1]⁺ 412.

### Step 4: 4-(6'-amino-4'-chloro-[2,3'-bipyridin]-5-yl)morpholin-3-one

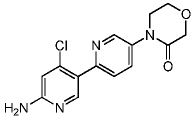

A mixture of 4-(6-bromopyridin-3-yl)morpholin-3-one (200 mg, 673.4 umol), 4-chloro-5-(4,4,5,5-tetramethyl-1,3,2-dioxaborolan-2-yl)pyridin-2-amine (340 mg, 1.4 mmol), Pd(dppf)Cl₂ (49 mg, 67.3 umol), and Na₂CO₃ (142 mg, 1.4 mmol) in ACN (10 mL)/ H₂O (2 mL) was heated at 120 °C under microwave irradiation for 20 mins under nitrogen atmosphere. After cooled to room temperature, the solvent was removed in vacuo and the residue was purified by silica gel column chromatography (EA/MeOH = 100:1 to 30:1) to give 4-(6'-amino-4'-chloro-[2,3'-bipyridin]-5-yl)morpholin-3-one (120 mg, 58.5%). MS (ESI) m/e [M+1]⁺ 305.

### Step 5: N-(4'-chloro-5-(3-oxomorpholino)-[2,3'-bipyridin]-6'-yl)acetamide

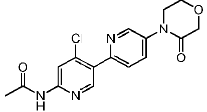

To a mixture of 4-(6'-amino-4'-chloro-[2,3'-bipyridin]-5-yl)morpholin-3-one (340 mg, 1.1 mmol) in pyridine (10 mL) was added AcCl (105 mg, 1.3 mmol) dropwise at 0 °C. Then the mixture was stirred at room temperature for 12 h. Upon completion of the reaction, water (0.2 mL) was added and the solvent was removed in vacuo. The residue was purified by silica gel column chromatography (EA/MeOH = 100:1 to 30: 1) to give N-(4'-chloro-5-(3-oxomorpholino)-[2,3'-bipyridin]-6'-yl)acetamide (150 mg, 39%). ¹H NMR (400MHz, DMSO-*d*₆) δ 10.97 (s, 1 H), 8.88 (s, 1 H), 8.61 (s, 1 H), 8.37 (s, 1 H), 8.08 (d, *J=* 8.4 Hz, 1H), 7.89 (d, *J=* 8.4 Hz, 1 H), 4.34 (s, 2 H), 4.13 - 4.05 (m, 2 H), 3.97 - 3.89 (m, 2 H), 2.21 (s, 3 H). MS (ESI) m/e [M+1]⁺ 347.

### Step 6: N-(4'-((3-(methylsulfonyl)phenyl)amino)-5-(3-oxomorpholino)-[2,3'-bipyridin]-6'-yl)acetami de

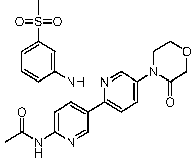

A mixture of N-(4'-chloro-5-(3-oxomorpholino)-[2,3'-bipyridin]-6'-yl)acetamide (150 mg, 432 umol), 3-(methylsulfonyl)aniline (147 mg, 865 umol), Pd₂(dba)₃ (39 mg, 43 umol), Xant-Phos (25 mg, 43 umol), and Cs₂CO₃ (421 mg, 1.3 mmol) in dioxane (5 mL) was heated at 110 °C for 12 h under nitrogen atmosphere. After cooled to room temperature, the solvent was removed in vacuo and the residue was purified by neutral prep-HPLC (column: Phenomenex Gemini-NX 150 x 30 mm x 5 um; phase: A-H₂O (10 mM NH₄HCO₃); B-ACN; B%: 10% - 40% in 20 min) to give N-(4'-((3-(methylsulfonyl)phenyl)amino)-5-(3-oxomorpholino)-[2,3'-bipyridin]-6'-yl)acetamide (5.6 mg, 2.7%). ¹H NMR (400MHz, DMSO-*d*₆) δ 11.31 (s, 1 H), 10.52 (s, 1 H), 8.84 (s, 1 H), 8.71 (s, 1 H), 8.20 (s, 1 H), 8.13 (d, *J* = 8.0 Hz, 1 H), 8.05 *(d, J* = 8.0 Hz, 1 H), 7.84 (s, 1 H), 7.69 - 7.64 (m, 2 H), 7.63 - 7.58 (m, 1 H), 4.29 (s, 2 H), 4.08 - 4.01 (m, 2 H), 3.91 - 3.84 (m, 2 H), 3.30 (s, 3 H), 2.09 (s, 3 H). MS (ESI) m/e [M+1]⁺ 482.

### Example G2: Synthesis of N-(4'-((6-(methylsulfonyl)pyridin-2-yl)amino)-5-(3-oxomorpholino)-[2,3'-bipyridin]-6'-yl)acetamide

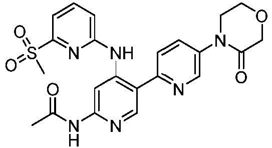

### Step 1: tert-butyl (6'-chloro-5-(3-oxomorpholino)-[2,3'-bipyridin]-4'-yl)carbamate

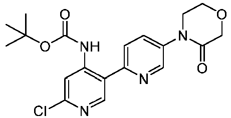

A mixture of tert-butyl (5-bromo-2-chloropyridin-4-yl)carbamate (3.0 g, 9.8 mmol), 4-(6-bromopyridin-3-yl)morpholin-3-one (2.9 g, 9.8 mmol), Pd(PPh₃)₂Cl₂ (688 mg, 977 umol), Pd(PPh₃)₄ (1.1 g, 977 umol), and Sn₂Me₆ (4.8 g, 14.7 mmol) in 1,4-dioxane (30 mL) was heated at 100 °C under nitrogen atmosphere for 12 h. After cooled to room temperature, the mixture was quenched with 10% KF solution (100 mL) and extracted with EA (100 mL x 3). The combined organic layer was washed with brine (100 mL), dried over Na₂SO₄, and concentrated under vacuum. The residue was purified by silica gel column chromatography (PE/EA = 1:1 to 1:2) to give tert-butyl (6'-chloro-5-(3-oxomorpholino)-[2,3'-bipyridin]-4'-yl)carbamate (1.1 g, 28%). ¹H NMR (400MHz, DMSO-*d*₆) δ 11.96 (s, 1 H), 8.87 (s, 1 H), 8.86 - 8.83 (m, 1 H), 8.28 (s, 1 H), 8.24 - 8.18 (m, 1 H), 8.17 - 8.10 (m, 1 H), 4.29 (s, 2 H), 4.08 - 3.99 (m, 2 H), 3.93 - 3.86 (m, 2 H), 1.50 (s, 9 H). MS (ESI) m/e [M+1]⁺ 405.

### Step 2: tert-butyl (6'-acetamido-5-(3-oxomorpholino)-[2,3'-bipyridin]-4'-yl)carbamate

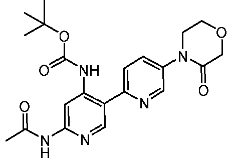

A mixture of tert-butyl (6'-chloro-5-(3-oxomorpholino)-[2,3'-bipyridin]-4'-yl)carbamate (500 mg, 1.2 mmol), acetamide (146 mg, 2.5 mmol), Pd₂(dba)₃ (113 mg, 123 umol), Xantphos (72 mg, 124 umol) and Cs₂CO₃ (808 mg, 2.5 mmol) in 1,4-dioxane (10 mL) was heated at 110 °C under nitrogen atmosphere for 12 h. After cooled to room temperature, the mixture was filterred and the filtrate was concentrated. The residue was purified by silica gel column chromatography (EA/MeOH = 100:1 to 30:1) to give tert-butyl (6'-acetamido-5-(3-oxomorpholino)-[2,3'-bipyridin]-4'-yl)carbamate (300 mg, 57%). MS (ESI) m/e [M+1]⁺ 428.

### Step 3: N-(4'-amino-5-(3-oxomorpholino)-[2,3'-bipyridin]-6'-yl)acetamide

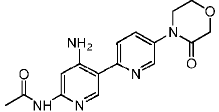

A mixture of tert-butyl (6'-acetamido-5-(3-oxomorpholino)-[2,3'-bipyridin]-4'-yl)carbamate (300 mg, 703 umol) in HCl/EA (20 mL, v: v = 1:9) was stirred at room temperature for 2 h. Upon completion of the reaction, the solvent was removed in vacuo and the residue was purified by prep-HPLC [column: Phenomenex Gemini-NX C18 75 x 30 mm x 3 um; liquid phase: [A-10 mM NH₄HCO₃ in H₂O; B-ACN] B%: 5%-45%, 8 min] to give N-(4'-amino-5-(3-oxomorpholino)-[2,3'-bipyridin]-6'-yl)acetamide (98 mg, 43%). ¹H NMR (400MHz, DMSO-*d*₆) δ 10.11 (s, 1 H), 8.63 (s, 1 H), 8.41 (s, 1 H), 7.96 - 7.86 (m, 2 H), 7.55 - 7.45 (m, 3 H), 4.22 (s, 2 H), 3.99 - 3.95 (m, 2 H), 3.82 - 3.78 (m, 2 H), 2.03 (s, 3 H). MS (ESI) m/e [M+1]⁺ 328.

### Step 4: N-(4'-((6-(methylsulfonyl)pyridin-2-yl)amino)-5-(3-oxomorpholino)-[2,3'-bipyridin]-6'-yl)acetamide

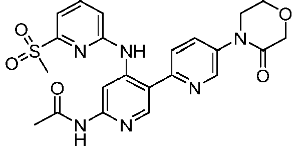

A mixture of N-(4',5-diamino-[2,3'-bipyridin]-6'-yl)acetamide (98 mg, 0.40 mmol), 2-bromo-6-(methylsulfonyl)pyridine (96 mg, 0.41 mmol), Pd₂(dba)₃ (18 mg, 0.02 mmol), BINAP (23 mg, 0.04 mmol) and K₃PO₄ (174 mg, 0.82 mmol) in 1,4-dioxane (10 mL) was heated to 100 °C for 4 h under nitrogen atmosphere. After cooled to room temperature, the mixture was filtrated and the filtrate was concentrated under vacuum. The residue was purified by Prep-TLC (MeOH/DCM = 1/15) to afford N-(4'-((6-(methylsulfonyl)pyridin-2-yl)amino) -5-(3-oxomorpholino)-[2,3'-bipyridin]-6'-yl)acetamide (15 mg, 7.6%). ¹H NMR ( 400 MHz, DMSO-*d*₆) δ 12.70 (s, 1H), 10.56 (s, 1H), 9.20 (s, 1H), 8.93 - 8.83 (m, 1H), 8.79 (s, 1H), 8.17 (d, *J=* 8.4 Hz, 1H), 8.06 (d, *J=* 8.4 Hz, 1H), 8.02 - 7.95 (m, 1H), 7.54 (s, 1H), 7.45 - 7.41 (m, 1H), 4.27 (s, 2H), 4.05 - 4.00 (m, 2H), 3.90 - 3.82 (m, 2H), 3.45 (s, 3H), 2.11 (s, 3H). MS (ESI) m/e [M+1]⁺ 483.

The following Examples were prepared in a similar manner to the product Examples G2:

| **Example** | **Compound** | **Chemical Name** | **¹H NMR and LC /MS *m*/*z* (M+1)** |
|---|---|---|---|
| G3 | 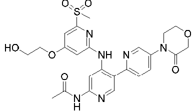 | N-(4'-((4-(2-hydroxyethoxy)-6-(methylsulfonyl)pyridin-2-yl)amino)-5-(3-oxomorpholino)-[2,3'-bipyridin]-6'-yl)acetamide | ¹H NMR (400 MHz, DMSO-*d*₆) δ 12.49 (s, 1H), 10.53 (s, 1H), 9.13 (s, 1H), 8.90 (s, 1H), 8.75 (s, 1H), 8.13 *(d, J=* 8.6 Hz, 1H), 8.05 *(d, J* = 8.6 Hz, 1H), 7.08 (s, 1H), 6.87 (s, 1H), 5.02 (s, 1H), 4.27 (s, 2H), 4.25 - 4.15 (m, 2H), 4.05 - 4.00 (m, 2H), 3.90 - 3.82 (m, 2H), 3.80 - 3.70 (m, 2H), 3.41 (s, 3H), 2.10 (s, 3H). MS (ESI) m/e [M+1]⁺ 543. |
| G4 | 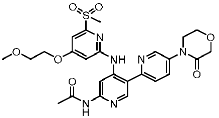 | N-(4'-((4-(2-methoxyethoxy)-6-(methylsulfonyl)pyridin-2-yl)amino)-5-(3-oxomorpholino)-[2,3'-bipyridin]-6'-yl)acetamide | ¹H NMR (400 MHz, DMSO-d6) δ 12.54 (s, 1H), 10.55 (s, 1H), 9.14 (s, 1H), 8.93 - 8.90 (m, 1H), 8.77 (s, 1H), 8.20 - 8.15 (m, 1H), 8.10 - 8.00 (m, 1H), 7.09 (s, 1H), 6.91 (s, 1H), 4.35 - 4.30 (m, 2H), 4.27 (s, 2H), 4.05 - 3.97 (m, 2H), 3.90 - 3.80 (m, 2H), 3.70 - 3.60 (m, 2H), 3.42 (s, 3H), 3.35 (s, 3H), 2.10 (s, 3H).MS (ESI) m/e [M+1]⁺ 557. |

### Example H1: Synthesis of N-(5-(2-hydroxypropan-2-yl)-4'-((3-(methylsulfonyl)phenyl)amino)-[2,3'-bipyridin]-6'-yl)acetamide

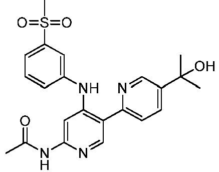

### Step 1: tert-butyl (6'-chloro-5-(2-hydroxypropan-2-yl)-[2,3'-bipyridin]-4'-yl)carbamate

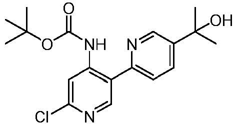

A mixture of tert-butyl (5-bromo-2-chloropyridin-4-yl)carbamate (650 mg, 2.1 mmol), 2-(6-bromopyridin-3-yl)propan-2-ol (525 mg, 2.4 mmol), Sn₂Me₆ (1.0 g, 3.1 mmol), Pd(PPh₃)₄ (244.2 mg, 211.3 umol) and Pd(PPh₃)₂Cl₂ in 1,4-dioxane (10 mL) was heated to 105 °C for 12 h under nitrogen atmosphere. After cooled to room temperature, the mixture was washed with KF (5 mL) and filtered. The organic layer was concentrated under vacuum and the residue was purified by silica gel column chromatography (PE/EA = 10:1) to give tert-butyl (6'-chloro-5-(2-hydroxypropan-2-yl)-[2,3'-bipyridin]-4'-yl)carbamate (0.4 g, 52%). ¹H NMR (400 MHz, CDCl₃) δ 11.93 (s, 1 H), 8.82 (s, 1 H), 8.59 (s, 1 H), 8.45 (s, 1 H), 7.97 (d, *J=* 8.4 Hz, 1 H), 7.74 (d, *J=* 8.4 Hz, 1 H) 1.68 (s, 6 H), 1.56 (s, 9 H). MS (ESI) m/e [M+1]⁺ 364.

### Step 2: tert-butyl (6'-acetamido-5-(2-hydroxypropan-2-yl)-[2,3'-bipyridin]-4'-yl)carbamate

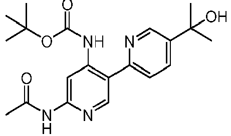

A mixture of tert-butyl (6'-chloro-5-(2-hydroxypropan-2-yl)-[2,3'-bipyridin]-4'-yl)carbamate (0.3 g, 824 umol), acetamide (97 mg, 1.6 mmol), Cs₂CO₃ (841 mg, 2.4 mmol), Xantphos (95 mg, 165 umol), Pd₂(dba)₃ (151 mg, 165 umol) in 1,4-dioxane (50 mL) was gradually warmed up to 90 °C and stirred at this temperature for 12 h under nitrogen atmosphere. After cooled to room temperature, the solution was filtered and concentrated under vacuum. The residue was purified by silica gel column chromatography (PE/EA = 1/1 to 0/1) to give tert-butyl (6'-acetamido-5-(2-hydroxypropan-2-yl)-[2,3'-bipyridin]-4'-yl)carbamate (0.22 g, 69%). ¹H NMR (400 MHz, CDCl₃) δ 11.81 (s, 1 H), 9.15 (s, 1 H), 8.79 (s, 1 H), 8.50 (s, 1 H), 7.94 (d, *J=* 8.4 Hz, 2H), 7.72 - 7.68 (m, 1 H), 2.23 (s, 3 H), 1.67 (s, 6 H), 1.66 - 1.69 (m, 1 H), 1.56 (s, 9 H). MS (ESI) m/e [M+1]⁺ 387.

### Step 3: N-(4'-amino-5-(2-hydroxypropan-2-yl)-[2,3'-bipyridin]-6'-yl)acetamide

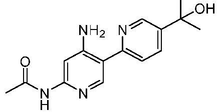

A mixture of tert-butyl (6'-acetamido-5-(2-hydroxypropan-2-yl)-[2,3'-bipyridin]-4'-yl)carbamate (150 mg, 388 umol) in TFA (1 mL) and DCM (2 mL) was stirred at 15 °C for 2 h under nitrogen atmosphere. Upon completion of the reaction, the solvent was removed in vacuo to give N-(4'-amino-5-(2-hydroxypropan-2-yl)-[2,3'-bipyridin]-6'-yl)acetamide (0.1 g, crude). It was used directly for next step without further purification. MS (ESI) m/e [M+1] ⁺ 287.

### Step 4: N-(5-(2-hydroxypropan-2-yl)-4'-((3-(methylsulfonyl)phenyl)amino)-[2,3'-bipyridin]-6'-yl)acetamide

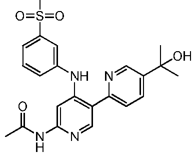

A mixture of N-(4'-amino-5-(2-hydroxypropan-2-yl)-[2,3'-bipyridin]-6'-yl)acetamide (0.1 g, 349 umol), 1-bromo-3-(methylsulfonyl)benzene (246 mg, 1.1 mmol), Cs₂CO₃ (358 mg, 1.1 mmol), Xantphos (20 mg, 35 umol), Pd₂(dba)₃ (35 mg, 35 umol) in 1,4-dioxane (5 mL) was gradually warmed up to 110 °C and stirred at this temperature for 12 h under nitrogen atmosphere. After cooled to room temperature, the solution was filtered and the filtrate was concentrated under vacuum. The residue was purified by Prep-HPLC (column: Phenomenex Gemini-NX 150 x 30 mm x 5 um; phase: A-H₂O (10 mM NH₄HCO₃); B-ACN; B%: 10% - 40% in 20 min) to give N-(5-(2-hydroxypropan-2-yl)-4'-((3-(methylsulfonyl)phenyl)amino)-[2,3'-bipyridin]-6'-yl)acetamide (11 mg, 7.1%). ¹H NMR (400 MHz, DMSO-*d*₆) δ 11.66 (s, 1 H), 10.47 (s, 1 H), 8.81 (s, 1 H), 8.69 (s, 1 H), 8.18 (s, 1 H), 8.00 (s, 2 H), 7.82 (s, 1 H), 7.66 - 7.62 (m, 2 H), 7.60 - 7.55 (m, 1 H), 5.29 (s, 1 H), 3.28 (s, 3 H), 2.07 (s, 3 H), 1.50 (s, 6 H). MS (ESI) m/e [M+1]⁺ 441.

### Example H2: Synthesis of N-(5-(2-hydroxypropan-2-yl)-4'-((4-isopropoxy-6-(methylsulfonyl)pyridin-2-yl)amino)-[2,3'-bipyridin]-6'-yl)acetamide .

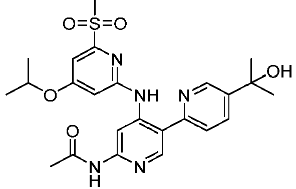

A mixture of N-(4'-amino-5-(2-hydroxypropan-2-yl)-[2,3'-bipyridin]-6'-yl)acetamide (400 mg, 1.40 mmol), 2-bromo-4-isopropoxy-6-(methylsulfonyl)pyridine (492 mg, 1.68 mmol), Pd₂(dba)₃ (128 mg, 0.14 mmol), BINAP ( 88mg, 0.14 mmol) and Cs₂CO₃ (1.368g, 4.20 mmol) in dioxane (20 mL) was stirred for 4 h at 130 °C under nitrogen atmosphere. The mixture was allowed to cool down to room temperature. The resulting mixture was filtered. The filtrate was concentrated under reduced pressure. The residue was purified by combi-flash (MeOH/DCM = 0-10%) to give the crude product. The crude product was suspended in acetonitrile to give N-(5-(2-hydroxypropan-2-yl)-4'-((4-isopropoxy-6-(methylsulfonyl)pyridin-2-yl)amino)-[2,3'-bipyridin]-6'-yl)acet amide (272.5mg, 39.0%). ¹H NMR (400 MHz, DMSO-*d*₆) δ 12.82 (s, 1H), 10.50 (s, 1H), 9.11 (s, 1H), 8.90 (s, 1H), 8.75 (s, 1H), 8.05 - 7.99 (m, 2H), 7.05 (s, 1H), 6.86 (s, 1H), 5.33 (s, 1H), 4.98 - 4.81 (m, 1H), 3.41 (s, 3H), 2.10 (s, 3H), 1.50 (s, 6H), 1.31 (d, *J=* 4.0 Hz, 6H). MS (ESI) m/e [M+1]⁺ 500.

### Example H3: Synthesis of N-(5-(2-hydroxypropan-2-yl)-4'-((3-(methylsulfonyl)-5-(trifluoro methyl)phenyl)amino)-[2,3'-bipyridin]-6'-yl)acetamide

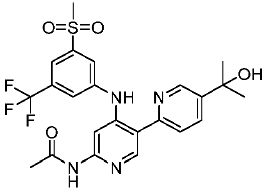

A mixture of N-(4'-amino-5-(2-hydroxypropan-2-yl)-[2,3'-bipyridin]-6'-yl)acetamide (100 mg, 0.35 mmol), 1-bromo-3-(methylsulfonyl)-5-(trifluoromethyl)benzene (127 mg, 0.42 mmol), Pd₂(dba)₃ (32 mg, 0.035 mmol), BINAP (22mg, 0.035 mmol) and Cs₂CO₃ (342 mg, 1.05 mmol) in dioxane (10 mL) was stirred for 16 h at 120 °C under nitrogen atmosphere. The mixture was allowed to cool down to room temperature. The resulting mixture was filtered. The filtrate was concentrated under reduced pressure. The residue was purified by combi-flash (MeOH/DCM = 0-10 %) to give the crude product. The crude product was suspended in acetonitrile to give N-(5-(2-hydroxypropan-2-yl)-4'-((3-(methylsulfonyl)-5-(trifluoromethyl)phenyl)amino)-[2,3'-bipyridin]-6'-yl)acetamide (18.77 mg, 10.5 %). ¹H NMR (400 MHz, DMSO-*d*₆) δ 11.66 (s, 1H), 10.50 (s, 1H), 8.76 (s, 1H), 8.64 (s, 1H), 8.17 (s, 1H), 8.03 (s, 1H), 7.92 - 7.90 (m, 3H), 7.71 (s, 1H), 5.26 (s, 1H), 3.24 (s, 3H), 2.02 (s, 3H), 1.43 (s, 6H). MS (ESI) m/e [M+1]⁺ 509.

### Example H43: Synthesis of N-(5-(2-hydroxypropan-2-yl)-4'-((3-isopropoxy-5-(methylsulfonyl)phenyl)amino)-[2,3'-bipyridin]-6'-yl)acetamide

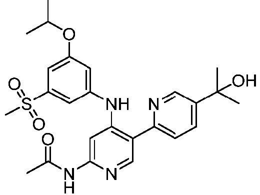

A solution of N-(4'-amino-5-(2-hydroxypropan-2-yl)-[2,3'-bipyridin]-6'-yl)acetamide (860 mg, 3 mmol), 1-bromo-3-isopropoxy-5-(methylsulfonyl)benzene (1.1 g, 3.6 mmol), Pd₂(dba)₃ (274.7 mg, 0.3 mmol), Xant-phos (347.2 mg, 0.6 mmol) and Cs₂CO₃ (1.96 g, 6 mmol) in dioxane (20 mL) was stirred at 130°C for 5 hr. The mixture was cooled to RT then filtered. The filtrate was concentrated under reduced pressure to give the crude product and purified by silica gel column chromatography (DCM/MeOH = 15: 1) to give the desired product (937.6 mg, 62.67%). ¹H NMR (400 MHz, DMSO-d6) δ 11.63 (s, 1H), 10.47 (s, 1H), 8.82 (s, 1H), 8.68 (s, 1H), 8.20 (s, 1H), 8.00 (s, 2H), 7.37 (s, 1H), 7.15 (s, 1H), 7.05 (s, 1H), 5.30 (s, 1H), 4.84 - 4.69 (m, 1H), 3.32 (s, 3H), 3.28 (s, 3H), 2.08 (s, 3H), 1.50 (s, 6H), 1.31 (d, *J=* 5.9 Hz, 6H). MS (ESI) m/e [M+1]⁺ 499.

The following Examples were prepared in a similar manner to the product Example H1:

| **Example** | **Compound** | **Chemical Name** | **¹H NMR and LC /MS *m*/*z* (M+1)** |
|---|---|---|---|
| H4 | 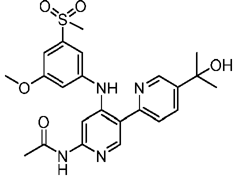 | N-(5-(2-hydroxypropan-2-yl)-4'-((3-methoxy-5-(methylsulfonyl)phenyl)amino)-[2,3'-bipyridin]-6'-yl)acetamide | ¹H NMR (400 MHz, DMSO-*d*₆) δ 11.66 (s, 1H), 10.48 (s, 1H), 8.80 (s, 1H), 8.67 (s, 1H), 8.22 (s, 1H), 8.04 - 7.92 (m, 2H), 7.36 (s, 1H), 7.22 - 7.21 (m, 1H), 7.07 (s, 1H), 5.30 (s, 1H), 3.86 (s, 3H), 3.27 (s, 3H), 2.06 (s, 3H), 1.48 (s, 6H). MS (ESI) m/e [M+1]⁺ 471. |
| H5 | 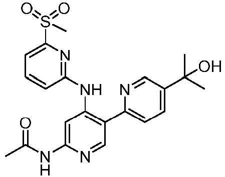 | N-(5-(2-hydroxypropan-2-yl)-4'-((6-(methylsulfonyl)pyridin-2-yl)amino)-[2,3'-bipyridin]-6'-yl)acetamide | 1H NMR (400 MHz, DMSO-*d*₆) δ 13.17 (s, 1H), 10.52 (s, 1H), 9.23 (s, 1H), 8.89 (s, 1H), 8.79 (s, 1H), 8.05 - 8.00 (m, 3H), 7.54 (d, *J=* 7.3 Hz, 1H), 7.42 (d, *J=* 8.4 Hz, 1H), 5.32 (s, 1H), 3.46 (s, 3H), 2.11 (s, 3H), 1.50 (s, 6H) MS (ESI) m/e [M+1]⁺ 442. |
| H6 | 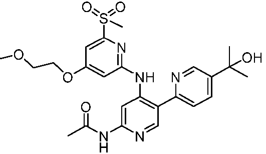 | N-(5-(2-hydroxypropan-2-yl)-4'-((4-(2-methoxyethoxy)-6-(methylsulfonyl)pyridin-2-yl)amino)-[2,3'-bipyridin]-6'-yl)acetamide | ¹H NMR (400 MHz, DMSO-*d*₆) δ 12.94 (s, 1H), 10.52 (s, 1H), 9.15 (s, 1H), 8.93 (s, 1H), 8.78 (s, 1H), 8.05 - 8.00 (m, 2H), 7.12 (s, 1H), 6.93 (s, 1H), 5.34 (s, 1H), 4.40 - 4.36 (m, 2H), 3.71 - 3.62 (m, 2H), 3.44 (s, 3H), 2.12 (s, 3H), 1.52 (s, 6H). MS (ESI) m/e [M+1]⁺ 516. |
| H7 | 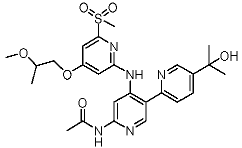 | N-(5-(2-hydroxypropan-2-yl)-4'-((4-(2-methoxypropoxy)-6-(methylsulfonyl)pyridin-2-yl)amino)-[2,3'-bipyridin]-6'-yl)acetamide | ¹H NMR (400 MHz, DMSO-*d*₆) δ 12.87 (s, 1H), 10.50 (s, 1H), 9.13 (s, 1H), 8.91 (s, 1H), 8.75 (s, 1H), 8.05 - 7.99 (m, 2H), 7.10 (s, 1H), 6.91 (s, 1H), 5.35 (s, 1H), 4.22 - 4.12 (m, 2H), 3.76 - 3.61 (m, 1H), 3.42 (s, 3H), 3.30 (s, 3H), 2.10 (s, 3H), 1.50 (s, 6H), 1.18 *(d, J* = 6.3 Hz, 3H). MS (ESI) m/e [M+1]⁺ 530. |
| H8 | 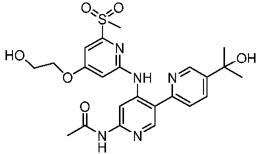 | N-(4'-((4-(2-hydroxyethoxy)-6-(methylsulfonyl)pyridin-2-yl)amino)-5-(2-hydroxypropan-2-yl)-[2,3'-bipyridin]-6'-yl)acetamide | ¹H NMR (400 MHz, DMSO-*d*₆) δ 12.89 (s, 1H), 10.51 (s, 1H), 9.14 (s, 1H), 8.97 - 8.84 (m, 1H), 8.76 (s, 1H), 8.04 - 7.99 (m, 2H), 7.09 (s, 1H), 6.89 (s, 1H), 5.33 (s, 1H), 4.97 - 4.92 (m, 1H), 4.31 - 4.12 (m, 2H), 3.76 - 3.72 (m, 2H), 3.42 (s, 3H), 2.10 (s, 3H), 1.50 (s, 6H). MS (ESI) m/e [M+1]⁺ 502. |
| H9 | 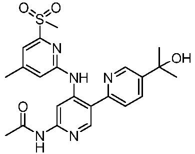 | N-(5-(2-hydroxypropan-2-yl)-4'-((4-methyl-6-(methylsulfonyl)pyridin-2-yl)amino)-[2,3'-bipyridin]-6'-yl)acetamide | ¹H NMR (400 MHz, DMSO-*d*₆) δ 13.18 (s, 1H), 10.51 (s, 1H), 9.27 (s, 1H), 8.93 (s, 1H), 8.80 (s, 1H), 8.11 - 8.03 (m, 2H), 7.44 (s, 1H), 7.27 (s, 1H), 5.36 (s, 1H), 3.47 (s, 3H), 2.43 (s, 3H), 2.13 (s, 3H), 1.53 (s, 6H). MS (ESI) m/e [M+1]⁺ 456. |
| H10 | 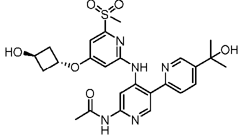 | N-(4'-((4-(trans)-3-hydroxycyclobutoxy)-6-(methylsulfonyl)pyridin-2-yl)amino)-5-(2-hydroxypropan-2-yl)-[2,3'-bipyridin]-6'-yl)acetamide | ¹H NMR (400 MHz, DMSO-*d*₆) δ 12.77 (s, 1H), 10.49 (s, 1H), 9.09 (s, 1H), 8.89 (s, 1H), 8.74 (s, 1H), 8.02 (s, 2H), 6.97 (s, 1H), 6.72 (s, 1H), 5.32 - 5.27 (m, 2H), 5.07 (s, 1H), 4.38 (s, 1H), 3.41 (s, 3H), 2.35 (s, 4H), 2.10 (s, 3H), 1.50 (s, 6H). MS (ESI) m/e [M+1]⁺ 528. |
| H11 | 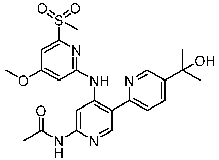 | N-(5-(2-hydroxypropan-2-yl)-4'-((4-methoxy-6-(methylsulfonyl)pyridin-2-yl)amino)-[2,3'-bipyridin]-6'-yl)acetamide | ¹H NMR (400 MHz, DMSO-*d*₆) δ 12.87 (s, 1H), 10.52 (s, 1H), 9.14 (s, 1H), 8.92 (s, 1H), 8.77 (s, 1H), 8.05 - 8.00 (m, 2H), 7.12 (s, 1H), 6.92 (s, 1H), 5.34 (s, 1H), 3.97 (s, 3H), 3.44 (s, 3H), 2.12 (s, 3H), 1.52 (s, 6H). MS (ESI) m/e [M+1]⁺ 472. |
| H12 | 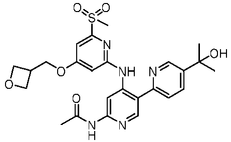 | N-(5-(2-hydroxypropan-2-yl)-4'-((6-(methylsulfonyl)-4-(oxetan-3-ylmethoxy)pyridin-2-yl)amino)-[2,3'-bipyridin]-6'-yl)acetamide | ¹H NMR (400 MHz, DMSO-*d*₆) δ 12.91 (s, 1H), 10.51 (s, 1H), 9.14 (s, 1H), 8.92 (s, 1H), 8.76 (s, 1H), 8.06 - 7.99 (m, 2H), 7.12 (s, 1H), 6.93 (s, 1H), 5.34 (s, 1H), 4.73 - 4.69 (m, 2H), 4.46 - 4.43 (m, 4H), 3.43 (s, 3H), 3.40 - 3.38 (m, 1H) 2.10 (s, 3H), 1.50 (s, 6H). MS (ESI) m/e [M+1]⁺ 528. |
| H13 | 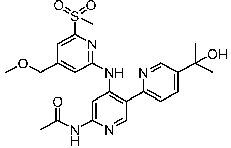 | N-(5-(2-hydroxypropan-2-yl)-4'-((4-(methoxymethyl)-6-(methylsulfonyl)pyridin-2-yl)amino)-[2,3'-bipyridin]-6'-yl)acetamide | ¹H NMR (400 MHz, DMSO-*d*₆) δ 13.07 (s, 1H), 10.47 (s, 1H), 9.20 (s, 1H), 8.84 (s, 1H), 8.74 (s, 1H), 7.82 - 7.75 (m, 2H), 7.43 (s, 1H), 7.24 (s, 1H), 5.31 (s, 1H), 4.50 (s, 2H), 3.42 (s, 3H), 3.35 (s, 3H), 2.06 (s, 3H), 1.45 (s, 6H). MS (ESI) m/e [M+1]⁺ 486. |
| H14 | 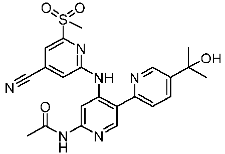 | N-(4'-((4-cyano-6-(methylsulfonyl)pyridin-2-yl)amino)-5-(2-hydroxypropan-2-yl)-[2,3'-bipyridin]-6'-yl)acetamide | ¹H NMR (400 MHz, DMSO-*d*₆) δ 13.81 (s, 1H), 10.60 (s, 1H), 9.26 (s, 1H), 8.96 (s, 1H), 8.86 (s, 1H), 8.12 (s, 1H), 8.10 (s, 1H), 8.07 (s, 1H), 8.03 (d, *J =* 8.0 Hz, 1H), 7.9 (d, *J=* 8.0 Hz, 1H), 5.36 (s, 1H), 3.52 (s, 3H), 2.11 (s, 3H), 1.51 (s, 6H). MS (ESI) m/e [M+1]⁺ 467. |
| H15 | 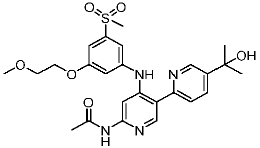 | N-(5-(2-hydroxypropan-2-yl)-4'-((3-(2-methoxyethoxy)-5-(methylsulfonyl)phenyl)amino)-[2,3'-bipyridin]-6'-yl)acetamide | ¹H NMR (400 MHz, DMSO-*d*₆) δ 11.68 (s, 1H), 10.49 (s, 1H), 8.82 (s, 1H), 8.69 (s, 1H), 8.22 (s, 1H), 8.05 - 8.00 (m, 2H), 7.39 (s, 1H), 7.22 (s, 1H), 7.09 (s, 1H), 5.31 (s, 1H), 4.28 - 4.24 (m, 2H), 3.68 - 3.62 (m, 2H), 3.28 (s, 3H), 2.08 (s, 3H), 1.50 (s, 6H). MS (ESI) m/e [M+1]⁺ 515. |
| H16 | 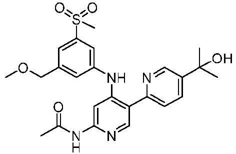 | N-(5-(2-hydroxypropan-2-yl)-4'-((3-(methoxymethyl)-5-(methylsulfonyl)phenyl)amino)-[2,3'-bipyridin]-6'-yl)acetamide | ¹H NMR (400 MHz, DMSO-*d*₆) δ 11.67 (s, 1H), 10.49 (s, 1H), 8.81 (s, 1H), 8.69 (s, 1H), 8.19 (s, 1H), 8.05 - 8.00 (m, 2H), 7.76 (s, 1H), 7.54 (d, *J=* 8.1 Hz, 2H), 5.31 (s, 1H), 4.52 (s, 2H), 3.36 (s, 3H), 3.29 (s, 3H), 2.08 (s, 3H), 1.50 (s, 6H). MS (ESI) m/e [M+1]⁺485. |
| H17 | 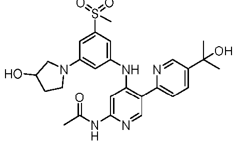 | N-(5-(2-hydroxypropan-2-yl)-4'-((3-(3-hydroxypyrrolidin-1-yl)-5-(methylsulfonyl)phenyl)amino)-[2,3'-bipyridin]-6'-yl)acetamide | ¹H NMR (400 MHz, DMSO-*d*₆) δ 11.61 (s, 1H), 10.47 (s, 1H), 8.82 (s, 1H), 8.65 (s, 1H), 8.19 (s, 1H), 8.05 - 8.00 (m, 2H), 6.98 (s, 1H), 6.69 (d, *J=* 18.3 Hz, 2H), 5.31 (s, 1H), 5.02 (s, 1H), 4.42 (s, 1H), 3.53 - 3.49 (m, 1H), 3.45 - 3.40 (m, 2H), 3.24 (s, 3H), 3.17 (d, J = 10.0 Hz, 1H), 2.08 (s, 4H), 1.96 - 1.93 (m, 1H), 1.50 (s, 6H). MS (ESI) m/e [M+1]⁺ 526. |
| H18 | 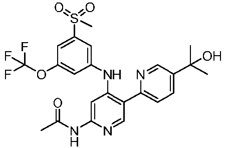 | N-(5-(2-hydroxypropan-2-yl)-4'-((3-(methylsulfonyl)-5-(trifluoromethoxy)phenyl)amino)-[2,3'-bipyridin]-6'-yl)acetamide | ¹H NMR (400 MHz, DMSO-*d*₆) δ 11.72 (s, 1H), 10.56 (s, 1H), 8.83 (s, 1H), 8.71 (s, 1H), 8.23 (s, 1H), 8.05 - 8.00 (m, 2H), 7.86 (s, 1H), 7.66 (s, 1H), 7.45 (s, 1H), 5.32 (s, 1H), 3.31 (s, 3H), 2.09 (s, 3H), 1.50 (s, 6H). MS (ESI) m/e [M+1]⁺ 525. |
| H19 | 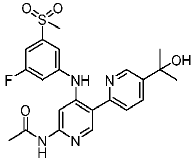 | N-(4'-((3-fluoro-5-(methylsulfonyl)phenyl)amino)-5-(2-hydroxypropan-2-yl)-[2,3'-bipyridin]-6'-yl)acetamide | ¹H NMR (400 MHz, DMSO-*d*₆) δ 11.74 (s, 1H), 10.54 (s, 1H), 8.82 (s, 1H), 8.71 (s, 1H), 8.24 (s, 1H), 8.05 - 7.99 (m, 2H), 7.67 (s, 1H), 7.55 - 7.50 (m, 1H), 7.40 - 7.36 (m, 1H), 5.31 (s, 1H), 3.34 (s, 3H), 2.09 (s, 3H), 1.50 (s, 6H). MS (ESI) m/e [M+1]⁺ 459. |
| H20 | 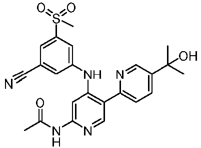 | N-(4'-((3-cyano-5-(methylsulfonyl)phenyl)amino)-5-(2-hydroxypropan-2-yl)-[2,3'-bipyridin]-6'-yl)acetamide | ¹H NMR (400 MHz, DMSO-*d*₆) δ 11.79 (s, 1H), 10.57 (s, 1H), 8.82 (s, 1H), 8.72 (s, 1H), 8.21 (s, 1H), 8.09 - 8.05 (m, 2H), 7.99 - 7.95 (m, 2H), 7.94 (s, 1H), 5.30 (s, 1H), 3.36 (s, 3H), 2.10 (s, 3H), 1.50 (s, 6H). MS (ESI) m/e [M+1]⁺ 466. |
| H21 | 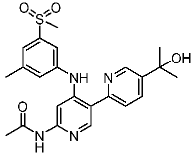 | N-(5-(2-hydroxypropan-2-yl)-4'-((3-methyl-5-(methylsulfonyl)phenyl)amino)-[2,3'-bipyridin]-6'-yl)acetamide | ¹H NMR (400 MHz, DMSO-*d*₆) δ 11.66 (s, 1H), 10.47 (s, 1H), 8.81 (s, 1H), 8.68 (s, 1H), 8.18 (s, 1H), 8.00 - 7.95 (m, 2H), 7.64 (s, 1H), 7.47 (s, 1H), 7.42 (s, 1H), 5.30 (s, 1H), 3.27 (s, 3H), 2.42 (s, 3H), 2.08 (s, 3H), 1.50 (s, 6H). MS (ESI) m/e [M+1]⁺ 455. |
| H22 | 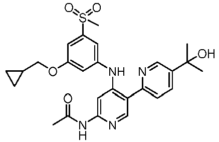 | N-(4'-((3-(cyclopropylmethoxy)-5-(methylsulfonyl)phenyl)amino)-5-(2-hydroxypropan-2-yl)-[2,3'-bipyridin]-6'-yl)acetamide | ¹H NMR (400 MHz, DMSO-*d*₆) δ 11.66 (s, 1H), 10.48 (s, 1H), 8.82 (s, 1H), 8.69 (s, 1H), 8.22 (s, 1H), 8.00 - 7.94 (m, 2H), 7.38 (s, 1H), 7.20 (s, 1H), 7.07 (s, 1H), 5.30 (s, 1H), 4.00 - 3.95 (m, 2H), 3.32 (s, 3H), 2.08 (s, 3H), 1.50 (s, 6H), 1.29 - 1.25 (m, 1H), 0.65 - 0.59 (m, 2H), 0.36 - 0.31 (m, 2H). MS (ESI) m/e [M+1]⁺ 511. |
| H23 | 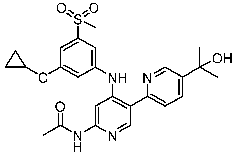 | N-(4'-((3-cyclopropoxy-5-(methylsulfonyl)phenyl)amino)-5-(2-hydroxypropan-2-yl)-[2,3'-bipyridin]-6'-yl)acetamide | ¹H NMR (400 MHz, DMSO-*d*₆) δ 11.66 (s, 1H), 10.48 (s, 1H), 8.82 (s, 1H), 8.69 (s, 1H), 8.23 (s, 1H), 8.05 - 7.99 (m, 2H), 7.42 (s, 1H), 7.33 (s, 1H), 7.19 (s, 1H), 5.30 (s, 1H), 4.06 - 4.00 (m, 1H), 3.29 (s, 3H), 2.08 (s, 3H), 1.50 (s, 6H), 0.86 - 0.67 (m, 4H). MS (ESI) m/e [M+1]⁺ 497. |
| H24 | 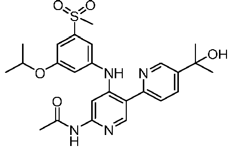 | N-(5-(2-hydroxypropan-2-yl)-4'-((3-isopropoxy-5-(methylsulfonyl)phenyl)amino)-[2,3'-bipyridin]-6'-yl)acetamide | ¹H NMR (400 MHz, DMSO-*d*₆) δ 11.62 (s, 1H), 10.47 (s, 1H), 8.82 (s, 1H), 8.68 (s, 1H), 8.19 (s, 1H), 8.00 - 7.95 (m, 2H), 7.37 (s, 1H), 7.15 (s, 1H), 7.05 (s, 1H), 5.30 (s, 1H), 4.87 - 4.65 (m, 1H), 3.28 (s, 3H), 2.08 (s, 3H), 1.50 (s, 6H), 1.31 (d, *J=* 4.0 Hz, 6H). MS (ESI) m/e [M+1]⁺ 499 |
| H25 | 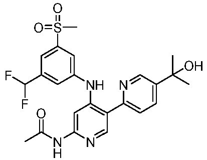 | N-(4'-((3-(difluoromethyl)-5-(methylsulfonyl)phenyl)amino)-5-(2-hydroxypropan-2-yl)-[2,3'-bipyridin]-6'-yl)acetamide | ¹H NMR (400 MHz, DMSO-*d*₆) δ 11.71 (s, 1H), 10.52 (s, 1H), 8.82 (s, 1H), 8.71 (s, 1H), 8.22 (s, 1H), 8.00 - 7.95 (m, 3H), 7.82 (s, 1H), 7.72 (s, 1H), 7.28 - 7.00 (m, 1H), 5.30 (s, 1H), 3.35 (s, 3H), 2.08 (s, 3H), 1.50 (s, 6H). MS (ESI) m/e [M+1]⁺ 491 |
| H26 | 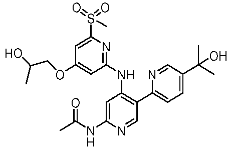 | N-(5-(2-hydroxypropan-2-yl)-4'-((4-(2-hydroxypropoxy)-6-(methylsulfonyl)pyridin-2-yl)amino)-[2,3'-bipyridin]-6'-yl)acetamide | ¹H NMR (400 MHz, DMSO-*d*₆) δ 12.83 (s, 1H), 10.51 (s, 1H), 9.14 (s, 1H), 8.92 (s, 1H), 8.77 (s, 1H), 8.04 - 8.02 (m, 2H), 7.12 (s, 1H), 6.91 (s, 1H), 5.34 (s, 1H), 5.10 (s, 1H), 4.99 - 4.92 (s, 1H), 4.06 - 4.02 (m, 2H), 3.43 (s, 3H), 2.12 (s, 3H), 1.52 (s, 6H), 1.27 - 1.17 (m, 3H). MS (ESI) m/e [M+1]⁺ 516. |
| H27 | 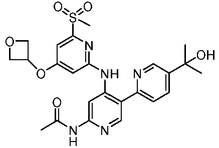 | N-(5-(2-hydroxypropan-2-yl)-4'-((6-(methylsulfonyl)-4-(oxetan-3-yloxy)pyridin-2-yl)amino)-[2,3'-bipyridin]-6'-yl)acetamide | ¹H NMR (400 MHz, DMSO-*d*₆) δ 12.90 (s, 1H), 10.53 (s, 1H), 9.11 (s, 1H), 8.93 (s, 1H), 8.78 (s, 1H), 8.07 - 8.02 (m, 2H), 7.01 (s, 1H), 6.70 (s, 1H), 5.59 (s, 1H), 5.36 (s, 1H), 4.99 - 4.91 (m, 2H), 4.66 - 4.61 (m, 2H), 3.44 (s, 3H), 2.12 (s, 3H), 1.53 (s, 6H). MS (ESI) m/e [M+1]⁺ 514. |
| H28 | 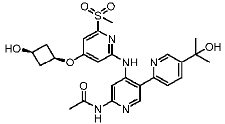 | N-(4'-((4-(cis-3-hydroxycyclobutoxy)-6-(methylsulfonyl)pyridin-2-yl)amino)-5-(2-hydroxypropan-2-yl)-[2,3'-bipyridin]-6'-yl)acetamide | ¹H NMR (400 MHz, DMSO-*d*₆) δ 12.83 (s, 1H), 10.53 (s, 1H), 9.10 (s, 1H), 8.92 (s, 1H), 8.77 (s, 1H), 8.07 - 8.04 (m, 2H), 7.02 (s, 1H), 6.79 (s, 1H), 5.45 - 5.27 (m, 2H), 4.67 - 4.45 (m, 1H), 3.90 - 3.88 (m, 1H), 3.43 (s, 3H), 2.87 - 2.82 (m, 2H), 2.12 (s, 3H), 1.98 - 1.92 (m, 2H), 1.52 (s, 6H). MS (ESI) m/e [M+1]⁺ 528. |
| H29 | 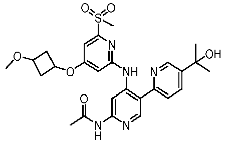 | N-(5-(2-hydroxypropan-2-yl)-4'-((4-(3-methoxycyclobutoxy)-6-(methylsulfonyl)pyridin-2-yl)amino)-[2,3'-bipyridin]-6'-yl)acetamide | ¹H NMR (400 MHz, DMSO-*d*₆) δ 12.81 (s, 1H), 10.52 (s, 1H), 9.11 (s, 1H), 8.92 (s, 1H), 8.77 (s, 1H), 8.04 - 8.01 (m, 2H), 7.04 - 7.00 (m, 1H), 6.80 - 6.76 (m, 1H), 5.35 (s, 1H), 5.10 (s, 1H), 4.10 (s, 1H), 3.43 (s, 3H), 3.18 (s, 3H), 2.46 (m, 2H), 2.42 - 2.31 (m, 2H), 2.12 (s, 3H), 1.52 (s, 6H). MS (ESI) m/e [M+1]⁺ 542. |
| H30 | 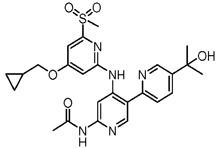 | N-(4'-((4-(cyclopropylmethoxy)-6-(methylsulfonyl)pyridin-2-yl)amino)-5-(2-hydroxypropan-2-yl)-[2,3'-bipyridin]-6'-yl)acetamide | ¹H NMR (400 MHz, DMSO-*d*₆) δ 12.87 (s, 1H), 10.52 (s, 1H), 9.14 (s, 1H), 8.94 (s, 1H), 8.78 (s, 1H), 8.08 - 8.05 (m, 2H), 7.15 - 7.10 (m, 1H), 6.91 (s, 1H), 5.35 (s, 1H), 4.08 (d, *J =* 8.0 Hz, 2H), 3.44 (s, 3H), 2.13 (s, 3H), 1.54 (s, 6H), 1.25 - 1.22 (m, 1H), 0.65 - 0.62 (m, 2H), 0.41 - 0.38 (m, 2H). MS (ESI) m/e [M+1]⁺ 512. |
| H31 | 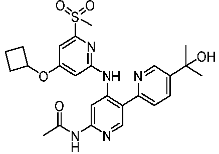 | N-(4'-((4-cyclobutoxy-6-(methylsulfonyl)pyridin-2-yl)amino)-5-(2-hydroxypropan-2-yl)-[2,3'-bipyridin]-6'-yl)acetamide | ¹H NMR (400 MHz, DMSO-*d*₆) δ 12.80 (s, 1H), 10.51 (s, 1H), 9.10 (s, 1H), 8.91 (s, 1H), 8.77 (s, 1H), 8.08 - 8.02 (m, 2H), 7.02 (s, 1H), 6.78 (s, 1H), 5.34 (s, 1H), 4.99 - 4.92 (m, 1H), 3.43 (s, 3H), 2.49 - 2.42 (m, 2H), 2.16 - 2.11 (m, 5H), 1.85 - 1.80 (m, 1H), 1.75 - 1.69 (m, 1H), 1.52 (s, 6H). MS (ESI) m/e [M+1]⁺ 512. |
| H32 | 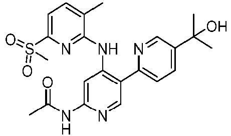 | N-(5-(2-hydroxypropan-2-yl)-4'-((3-methyl-6-(methylsulfonyl)pyridin-2-yl)amino)-[2,3'-bipyridin]-6'-yl)acetamide | ¹H NMR (400 MHz, DMSO-*d*₆) δ 13.14 (s, 1H), 10.52 (s, 1H), 9.35 (s, 1H), 8.86 (s, 1H), 8.82 (s, 1H), 8.14 *(d, J* = 8.0 Hz, 1H), 8.05 (d, *J* = 8.0 Hz, 1H), 7.90 (d, *J* = 8.0 Hz, 1H), 7.52 (d, *J* = 8.0 Hz, 1H), 5.36 (s, 1H), 3.45 (s, 3H), 2.53 (s, 3H), 2.13 (s, 3H), 1.52 (s, 6H). MS (ESI) m/e [M+1]⁺ 456. |
| H33 | 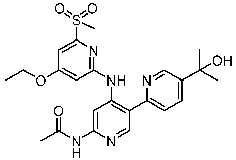 | N-(4'-((4-ethoxy-6-(methylsulfonyl)pyridin-2-yl)amino)-5-(2-hydroxypropan-2-yl)-[2,3'-bipyridin]-6'-yl)acetamide | ¹H NMR (400 MHz, DMSO-*d*₆) δ 12.88 (s, 1H), 10.50 (s, 1H), 9.14 (s, 1H), 8.92 (s, 1H), 8.77 (s, 1H), 8.12 - 7.94 (m, 2H), 7.09 (s, 1H), 6.88 - 6.85 (m, 1H), 5.34 (s, 1H), 4.29 - 4.24 (m, 2H), 3.43 (s, 3H), 2.12 (s, 3H), 1.52 (s, 6H), 1.40 - 1.36 (m, 3H). MS (ESI) m/e [M+1]⁺486. |
| H34 | 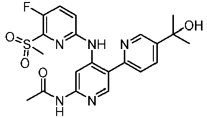 | N-(4'-((5-fluoro-6-(methylsulfonyl)pyridin-2-yl)amino)-5-(2-hydroxypropan-2-yl)-[2,3'-bipyridin]-6'-yl)acetamide | ¹H NMR (400 MHz, DMSO-*d₆*) δ 12.96 (s, 1H), 10.53 (s, 1H), 8.96 (s, 1H), 8.88 (s, 1H), 8.78 (s, 1H), 8.07 - 7.96 (m, 3H), 7.56 - 7.52 (m, 1H), 5.33 (s, 1H), 3.51 (s, 3H), 2.12 (s, 3H), 1.51 (s, 6H). MS (ESI) m/e [M+1]⁺460. |
| H35 | 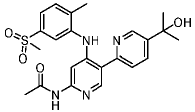 | N-(5-(2-hydroxypropan-2-yl)-4'-((2-methyl-5-(methylsulfonyl)phenyl)amino)-[2,3'-bipyridin]-6'-yl)acetamide | ¹H NMR (400 MHz, DMSO-*d₆*) δ 11.86 (s, 1H), 10.46 (s, 1H), 8.79 - 8.74 (m, 2H), 8.12 - 7.96 (m, 3H), 7.89 (s, 1H), 7.69 - 7.43 (m, 2H), 5.30 (s, 1H), 3.27 (s, 3H), 2.45 (s, 3H), 2.06 (s, 3H), 1.50 (s, 6H). MS (ESI) m/e [M+1]⁺455. |
| H36 | 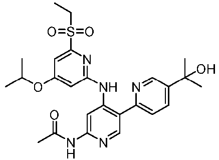 | N-(4'-((6-(ethylsulfonyl)-4-isopropoxypyridin-2-yl)amino)-5-(2-hydroxypropan-2-yl)-[2,3'-bipyridin]-6'-yl)acetamide | ¹H NMR (400 MHz, DMSO-*d₆*) δ 12.76 (s, 1H), 10.48 (s, 1H), 9.04 (s, 1H), 8.92 (s, 1H), 8.76 (s, 1H), 8.06 - 8.03 (m, 2H), 7.07 (s, 1H), 6.88 (s, 1H), 5.33 (s, 1H), 5.04 - 4.82 (m, 1H), 3.69 - 3.64 (m, 2H), 2.12 (s, 3H), 1.52 (s, 6H), 1.34 (d, *J =* 4.0 Hz, 6H), 1.19 - 1.16 (m, 3H). MS (ESI) m/e [M+1]⁺514. |
| H37 | 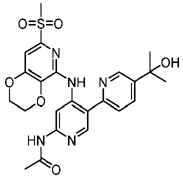 | N-(5-(2-hydroxypropan-2-yl)-4'-((7-(methylsulfonyl)-2,3-dihydro-[1,4]dioxino[2,3-c]pyridin-5-yl)amino)-[2,3'-bipyridin]-6'-yl)acetamide | ¹H NMR (400 MHz, DMSO-*d₆*) δ 13.40 (s, 1H), 10.50 (s, 1H), 9.42 (s, 1H), 8.81 - 8.80 (m, 2H), 8.10 - 8.03 (m, 2H), 7.15 (s, 1H), 5.34 (s, 1H), 4.62 - 4.51 (m, 4H), 3.46 (s, 3H), 2.13 (s, 3H), 1.53 (s, 6H). MS (ESI) m/e [M+1]⁺ 500.4 |
| H38 | 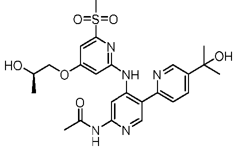 | (R)-N-(5-(2-hydroxypropan-2-yl)-4'-((4-(2-hydroxypropoxy)-6-(methylsulfonyl)pyridin-2-yl)amino)-[2,3'-bipyridin]-6'-yl)acetamide | MS (ESI) m/e [M+1]⁺ 516. |
| H39 | 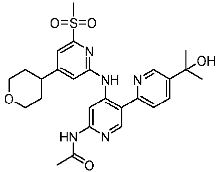 | N-(5-(2-hydroxypropan-2-yl)-4'-((6-(methylsulfonyl)-4-(tetrahydro-2H-pyran-4-yl)pyridin-2-yl)amino)-[2,3'-bipyridin]-6'-yl)acetamide | MS (ESI) m/e [M+1]⁺ 526. |
| H40 | 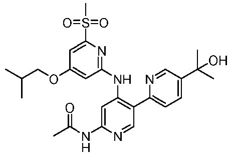 | N-(5-(2-hydroxypropan-2-yl)-4'-((4-isobutoxy-6-(methylsulfonyl)pyridin-2-yl)amino)-[2,3'-bipyridin]-6'-yl)acetamide | MS (ESI) m/e [M+1]⁺ 514. |
| H41 | 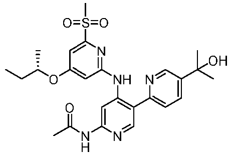 | (S)-N-(4'-((4-(sec-butoxy)-6-(methylsulfonyl)pyridin-2-yl)amino)-5-(2-hydroxypropan-2-yl)-[2,3'-bipyridin]-6'-yl)acetamide | MS (ESI) m/e [M+1]⁺ 514. |
| H42 | 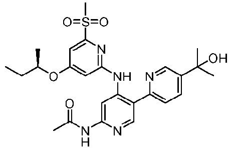 | (R)-N-(4'-((4-(sec-butoxy)-6-(methylsulfonyl)pyridin-2-yl)amino)-5-(2-hydroxypropan-2-yl)-[2,3'-bipyridin]-6'-yl)acetamide | MS (ESI) m/e [M+1]⁺ 514. |

### Example J1: Synthesis of N-(5-(1-methyl-1H-pyrazol-3-yl)-4-((4-methyl-6-(methylsulfonyl)pyridin-2-yl)amino)pyridin-2-yl)acetamide

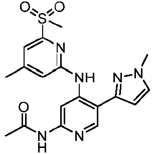

### Step 1: 1-methyl-3-(4,4,5,5-tetramethyl-1,3,2-dioxaborolan-2-yl)-1H-pyrazole

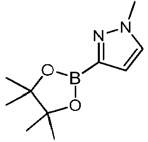

A mixture of 3-bromo-1-methyl-1H-pyrazole (4.1 g, 25.5 mmol), 4,4,4',4',5,5,5',5'-octamethyl-2,2'-bi(1,3,2-dioxaborolane) (9.7 g, 38.2 mmol), KOAc (10.0 g, 102.0 mmol) and Pd(dppf)Cl₂-CH₂Cl₂ (2.0 g, 2.5 mmol) in 1,4-dioxane (50 mL) was stirred at 95 °C under nitrogen atmosphere for 6 h. After cooled to room temperature, the solvent was removed in vacuo and the residue was diluted with 20 mL of water. The resulting solution was extracted with EA (30 mL x 3) and the combined organic layers were washed with brine (50 mL), dried over anhydrous sodium sulfate. The solvent was removed in vacuo and the residue was purified by silica gel column chromatography (PE/EA = 3:1 to 0:1) to give 1-methyl-3-(4,4,5,5-tetramethyl-1,3,2-dioxaborolan-2-yl)-1H-pyrazole (3.6 g, 68%). MS (ESI) m/e [M+1]⁺ 127.

### Step 2: tert-butyl (2-chloro-5-(1-methyl-1H-pyrazol-3-yl)pyridin-4-yl)carbamate

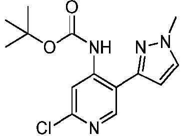

A mixture of tert-butyl (5-bromo-2-chloropyridin-4-yl)carbamate (3.5 g, 7.5 mmol), 1-methyl-3-(4,4,5,5-tetramethyl-1,3,2-dioxaborolan-2-yl)-1H-pyrazole (2.3 g, 11.0 mmol), K₃PO₄ (7.2 g, 3.3 mmol) and Pd(dppf)Cl₂ (805 mg, 1.1 mmol) in dioxane (50 mL) and H₂O (5 mL) was stirred at 100 °C for 12 h under nitrogen atmosphere. After cooled to room temperature, the solvent was removed in vacuo and the residue was diluted with 20 mL of water. The resulting solution was extracted with EA (30 mL x 3) and the combined organic layers were washed with 50 mL of brine, dried over anhydrous sodium sulfate. The solvent was removed in vacuo and the residue was purified by silica gel column chromatography (PE/EA = 10:1 to 3:1) to give tert-butyl (2-chloro-5-(1-methyl-1H-pyrazol-3-yl)pyridin-4-yl)carbamate (2.9 g, 82%). MS (ESI) m/e [M+1]⁺ 309.

### Step 3: tert-butyl (2-acetamido-5-(1-methyl-1H-pyrazol-3-yl)pyridin-4-yl)carbamate

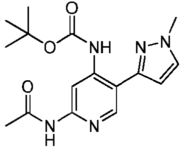

A mixture of tert-butyl (2-chloro-5-(1-methyl-1H-pyrazol-3-yl)pyridin-4-yl)carbamate (3.2 g, 10.3 mmol), acetamide (1.2 g, 20.7 mmol), Cs₂CO₃ (10.1 g, 31.0 mmol), Xant-phos (1.2 g, 2.1 mmol) and Pd₂(dba)₃ (916 mg, 1.0 mmol) in 1,4-dioxane (50 mL) was stirred at 110 °C for 12 h under nitrogen atmosphere. After cooled to room temperature, the solvent was removed in vacuo and the residue was diluted with 20 mL of water. The resulting solution was extracted with EA (30 mL x 3) and the combined organic layers were washed with brine (50 mL), dried over anhydrous sodium sulfate. The solvent was removed in vacuo and the residue was purified by silica gel column chromatography (PE/EA = 1:1 to 0:1) to give tert-butyl (2-acetamido-5-(1-methyl-1H-pyrazol-3-yl)pyridin-4-yl)carbamate (1.3 g, 38%). ¹H NMR (400 MHz, CDCl₃) δ 10.70 (s, 1H), 9.13 (s, 1H), 8.41 (s, 1H), 8.08 (s, 1H), 7.43 (d, *J =* 2.4 Hz, 1H), 6.59 (d*, J=* 2.4 Hz, 1H), 3.98 (s, 3H), 2.21 (s, 3H), 1.57 (s, 9H). MS (ESI) m/e [M+1]⁺ 332.

### Step 4: N-(4-amino-5-(1-methyl-1H-pyrazol-3-yl)pyridin-2-yl)acetamide

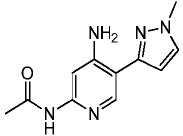

A mixture of tert-butyl (2-acetamido-5-(1-methyl-1H-pyrazol-3-yl)pyridin-4-yl)carbamate (1.3 g, 3.9 mmol) in TFA (5 mL) and DCM (5 mL) was stirred at 20 °C for 3 h. Upon completion of the reaction, the solvent was removed in vacuo and the residue was diluted with water, then sat. NaHCO₃ was added to adjust the pH value to 9. The resulting mixture was extracted with EA (30 mL x 3) and the combined organic layers were dried over Na₂SO₄. The solvent was removed to give N-(4-amino-5-(1-methyl-1H-pyrazol-3-yl)pyridin-2-yl)acetamide (810 mg, crude). MS (ESI) m/e [M+1]⁺ 232.

### Step 5: N-(5-(1-methyl-1H-pyrazol-3-yl)-4-((4-methyl-6-(methylsulfonyl)pyridin-2-yl)amino)pyridin-2-yl)acetamide

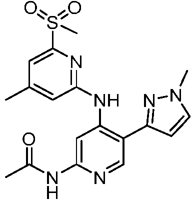

A mixture of N-(4-amino-5-(1-methyl-1H-pyrazol-3-yl)pyridin-2-yl)acetamide (70 mg, 0.3 mmol), 2-bromo-4-methyl-6-(methylsulfonyl)pyridine (91 mg, 0.36 mmol), Pd₂dba₃ (28 mg, 0.03 mmol), BINAP (19 mg, 0.03 mmol) and Cs₂CO₃ (196 mg, 0.6 mmol) in dioxane (5 mL) was stirred at 125 °C under N₂ in a sealed tube for 6 h. The reaction mixture was filtered and the solid was washed with EA (10 mL). The filtration was concentrated and the residue was purified by Prep-TLC (DCM/MeOH = 20: 1) to give the N-(5-(1-methyl-1H-pyrazol-3-yl)-4-((4-methyl-6-(methylsulfonyl)pyridin-2-yl)amino)pyridin-2-yl)acetamide (35 mg, 29%). ¹H NMR (400 MHz, DMSO-*d₆*) δ 11.47 (s, 1H), 10.43 (s, 1H), 9.27 (s, 1H), 8.64 (s, 1H), 7.90 - 7.85 (m, 1H), 7.45 (s, 1H), 7.18 (s, 1H), 6.95 - 6.90 (m, 1H), 4.04 (s, 3H), 3.48 (s, 3H), 2.44 (s, 3H), 2.11 (s, 3H). MS (ESI) m/e [M+1]⁺ 401.

### Example J2: Synthesis of N-(4-((4-isopropoxy-6-(methylsulfonyl)pyridin-2-yl)amino)-5-(1-methyl-1H-pyrazol-3-yl)pyridin-2-yl)acetamide

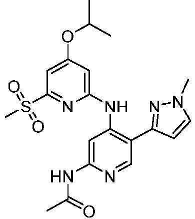

A mixture of N-(4-amino-5-(1-methyl-1H-pyrazol-3-yl)pyridin-2-yl)acetamide (600 mg, 2.6 mmol), 2-bromo-4-isopropoxy-6-(methylsulfonyl)pyridine (916 mg, 3.1 mmol), Pd₂dba₃ (238 mg, 0.26 mmol), BINAP (162 mg, 0.26 mmol) and K₂CO₃ (718 mg, 5.2 mmol) in dioxane (20 mL) was stirred at 125 °C under N₂ in a sealed tube for 4 h. The reaction mixture was filtered and the solid was washed with EA (10 mL). The filtrate was concentrated and the residue was purified by column chromatography (DCM/MeOH = 100:1 - 50:1) then combi-flash ((CH₃CN/H₂O, 0.1% HCOOH) = 0 - 100%)) to give N-(4-((4-isopropoxy-6-(methyl sulfonyl)pyridin-2-yl)amino)-5-(1-methyl-1H-pyrazol-3-yl)pyridin-2-yl)acetamide (720 mg, 62%). ¹H NMR (400 MHz, DMSO-*d₆*) δ 10.40 (s, 1H), 10.24 (s, 1H), 8.56 (s, 1H), 8.14 (s, 1H), 7.89 - 7.84 (m, 1H), 7.38 (s, 1H), 7.14 (s, 1H), 7.09 (s, 1H), 6.87 - 6.82 (m, 1H), 4.81 - 4.71 (m, 1H), 3.96 (s, 3H), 3.29 (s, 3H), 2.06 (s, 3H), 1.3 1 (d, *J =* 5.9 Hz, 6H). MS (ESI) m/e [M+1]⁺ 444.

### Example J3: Synthesis of N-(4-((4-(cyclopropylmethoxy)-6-(methylsulfonyl)pyridin-2-yl)amino)-5-(1-methyl-1H-pyrazol-3-yl)pyridin-2-yl)acetamide

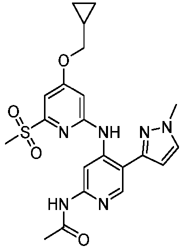

A mixture of N-(4-amino-5-(1-methyl-1H-pyrazol-3-yl)pyridin-2-yl)acetamide (300 mg, 1.3 mmol), 2-bromo-4-(cyclopropylmethoxy)-6-(methylsulfonyl)pyridine (477 mg, 1.56 mmol), Pd₂dba₃ (120 mg, 0.13 mmol), BINAP (80 mg, 0.13 mmol) and K₂CO₃ (360 mg, 2.6 mmol) in dioxane (20 mL) was stirred at 125 °C under N₂ in a sealed tube for 4 h. The reaction mixture was filtered and the solid was washed with EA (10 mL). The filtrate was concentrated and the residue was purified by column chromatography (DCM/MeOH = 200:1 - 70:1) and the impure product was washed with CH₃CN (5 mL) to give N-(4-((4-(cyclopropylmethoxy)-6-(methylsulfonyl)pyridine-2-yl)amino)-5-(1-methyl-1H-pyrazol-3-yl)pyridin-2-yl)acetamide (380 mg, 64%). ¹H NMR (400 MHz, DMSO-*d₆*) δ 10.48 (s, 1H), 10.30 (s, 1H), 8.55 (s, 1H), 8.10 (s, 1H), 7.86 (d, *J =* 2.1 Hz, 1H), 7.39 (s, 1H), 7.20 (s, 1H), 7.13 (s, 1H), 6.87 (d, *J =* 2.1 Hz, 1H), 3.96 - 3.94 (m, 5H), 3.29 (s, 3H), 2.07 (s, 3H), 1.24 - 1.22 (m, 1H), 0.65 - 0.59 (m, 2H), 0.35 - 0.34 (m, 2H). MS (ESI) m/e [M+1]⁺ 456.

The following Examples were prepared in a similar manner to the product Example J1:

| **Example** | **Compound** | **Chemical Name** | **¹H NMR and LC /MS *m*/*z* (M+1)** |
|---|---|---|---|
| J4 | 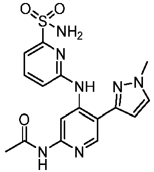 | N-(5-(1-methyl-1H-pyrazol-3-yl)-4-((6-sulfamoylpyridin-2-yl)amino)pyridin-2-yl)acetamide | ¹H NMR (400 MHz, DMSO-*d₆*) δ 11.61 (s, 1H), 10.66 (s, 1H), 9.75 (s, 1H), 8.67 (s, 1H), 8.05 - 7.88 (m, 2H), 7.47 (d, *J =* 6.4 Hz, 1H), 7.40 - 7.33 (m, 2H), 7.30 (d, *J =* 6.4 Hz, 1H), 6.97 - 6.92 (m, 1H), 4.04 (s, 3H), 2.16 (s, 3H). MS (ESI) m/e [M+1]⁺ 388. |
| J5 | 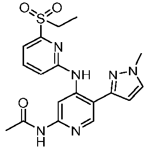 | N-(4-((6-(ethylsulfonyl)pyridin-2-yl)amino)-5-(1-methyl-1H-pyrazol-3-yl)pyridin-2-yl)acetamide | ¹H NMR (400 MHz, DMSO-*d₆*) δ 11.53 (s, 1H), 10.44 (s, 1H), 9.18 (s, 1H), 8.64 (s, 1H), 8.04 - 7.94 (m, 1H), 7.88 - 7.87 (m, 1H), 7.60 - 7.50 (m, 1H), 7.36 (d, *J =* 7.3 Hz, 1H), 6.95 - 6.91 (m, 1H), 4.00 (s, 3H), 3.73 (q, *J =* 7.3 Hz, 2H), 2.09 (s, 3H), 1.16 (t, *J =* 7.3 Hz, 3H). MS (ESI) m/e [M+1]⁺ 401. |
| J6 | 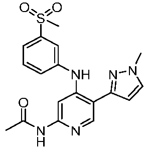 | N-(5-(1-methyl-1H-pyrazol-3-yl)-4-((3-(methylsulfonyl)pheny l)amino)pyridin-2-yl)acetamide | ¹H NMR (400 MHz, DMSO-*d₆*) δ 10.41 (s, 1H), 10.28 (s, 1H), 8.56 (s, 1H), 8.14 (s, 1H), 7.86 - 7.80 (m, 2H), 7.62 - 7.50 (m, 3H), 6.87 (s, 1H), 3.94 (s, 3H), 3.29 (s, 3H), 2.04 (s, 3H). MS (ESI) m/e [M+1]⁺ 386. |
| J7 | 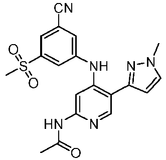 | N-(4-((3-cyano-5-(methylsulfonyl)pheny 1)amino)-5-(1-methyl-1H-pyrazol-3-yl)pyridin-2-yl)acetamide | ¹H NMR (400 MHz, DMSO-*d₆*) δ 10.49 (s, 1H), 10.36 (s, 1H), 8.59 (s, 1H), 8.16 (s, 1H), 8.08 (s, 1H), 8.07 (s, 1H), 7.97 - 7.92 (m, 1H), 7.83 (d, *J =* 2.4 Hz, 1H), 6.85 (d*, J* = 2.4 Hz, 1H), 3.94 (s, 3H), 3.35 (s, 3H), 2.05 (s, 3H). MS (ESI) m/e [M+1]⁺ 411. |
| J8 | 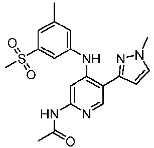 | N-(5-(1-methyl-1H-pyrazol-3-yl)-4-((3-methyl-5-(methylsulfonyl)pheny l)amino)pyridin-2-yl)acetamide | ¹H NMR (400 MHz, DMSO-*d₆*) δ 10.38 (s, 1H), 10.22 (s, 1H), 8.55 (s, 1H), 8.13 (s, 1H), 7.83 (d, *J =* 2.4 Hz, 1H), 7.63 (s, 1H), 7.44 (s, 2H), 6.86 (d, *J =* 2.4 Hz, 1H), 3.94 (s, 3H), 3.26 (s, 3H), 2.41 (s, 3H), 2.03 (s, 3H). MS (ESI) m/e [M+1]⁺ 400. |
| J9 | 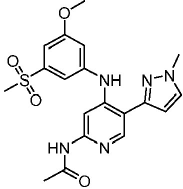 | N-(4-((3-methoxy-5-(methylsulfonyl)pheny 1)amino)-5-(1-methyl-1H-pyrazol-3-yl)pyridin-2-yl)acetamide | ¹H NMR (400 MHz, DMSO-*d₆*) δ 10.42 (s, 1H), 10.26 (s, 1H), 8.55 (s, 1H), 8.18 (s, 1H), 7.84 (d, *J =* 2.4 Hz, 1H), 7.38 - 7.32 (m, 1H), 7.25 - 7.21 (m, 1H), 7.15 - 7.10 (m, 1H), 6.86 (d, *J =* 2.4 Hz, 1H), 3.94 (s, 3H), 3.87 (s, 3H), 3.28 (s, 3H), 2.04 (s, 3H). MS (ESI) m/e [M+1]⁺ 416. |
| J10 | 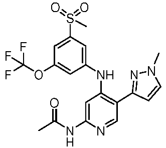 | N-(5-(1-methyl-1H-pyrazol-3-yl)-4-((3-(methylsulfonyl)-5-(trifluoromethoxy)phe nyl)amino)pyridin-2-yl)acetamide | ¹H NMR (400 MHz, DMSO-*d₆*) δ 10.49 (s, 1H), 10.34 (s, 1H), 8.60 (s, 1H), 8.21 (s, 1H), 7.86 - 7.85 (m, 2H), 7.66 (s, 1H), 7.48 (s, 1H), 6.86 - 6.85 (m, 1H), 3.96 (s, 3H), 3.38 (s, 3H), 2.07 (s, 3H). MS (ESI) m/e [M+1]⁺ 470. |
| J11 | 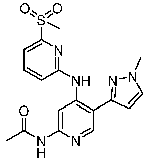 | N-(5-(1-methyl-1H-pyrazol-3-yl)-4-((6-(methylsulfonyl)pyridi n-2-yl)amino)pyridin-2-yl)acetamide | ¹H NMR (400 MHz, CDCl₃) δ 11.43 (s, 1H), 9.35 (s, 1H), 8.48 (s, 1H), 7.99 (s, 1H), 7.85-7.78 (m, 1H), 7.66 (d, *J =* 7.6 Hz, 1H), 7.47 (d, *J =* 2.4 Hz, 1H), 7.10 (d, *J =* 8.4 Hz, 1H), 6.66 (d, *J =* 2.4 Hz, 1H), 4.04 (s, 3H), 3.51 (s, 3H), 2.22 (s, 3H). MS (ESI) m/e [M+1]⁺ 387. |
| J12 | 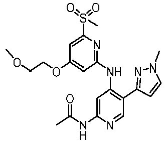 | N-(4-((4-(2-methoxyethoxy)-6-(methylsulfonyl)pyridi n-2-yl)amino)-5-(1-methyl-1H-pyrazol-3-yl)pyridin-2-yl)acetamide | ¹H NMR (400 MHz, DMSO-*d₆*) δ 11.33 (s, 1H), 10.43 (s, 1H), 9.18 (s, 1H), 8.63 (s, 1H), 7.89 - 7.84 (m, 1H), 7.14 (s, 1H), 6.95 - 6.91 (m, 1H), 6.83 (s, 1H), 4.39 - 4.32 (m, 2H), 4.03 (s, 3H), 3.74 - 3.68 (m, 2H), 3.46 (s, 3H), 2.10 (s, 3H). MS (ESI) m/e [M+1]⁺ 461. |
| J13 | 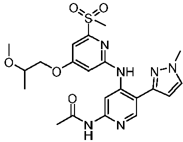 | N-(4-((4-(2-methoxypropoxy)-6-(methylsulfonyl)pyridi n-2-yl)amino)-5-(1-methyl-1H-pyrazol-3-yl)pyridin-2-yl)acetamide | ¹H NMR (400 MHz, DMSO-*d₆*) δ 11.29 (s, 1H), 10.43 (s, 1H), 9.16 (s, 1H), 8.61 (s, 1H), 7.87 - 7.82 (m, 1H), 7.12 (s, 1H), 6.90 - 6.85 (m, 1H), 6.82 (s, 1H), 4.24 - 4.15 (m, 2H), 4.00 (s, 3H), 3.73 - 3.66 (m, 1H), 3.44 (s, 3H), 2.08 (s, 3H), 1.19 - 1.15 (m, 3H). MS (ESI) m/e [M+1]⁺ 475. |
| J14 | 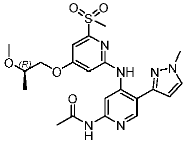 | (R)-N-(4-((4-(2-methoxypropoxy)-6-(methylsulfonyl)pyridi n-2-yl)amino)-5-(1-methyl-1H-pyrazol-3-yl)pyridin-2-yl)acetamide | ¹H NMR (400 MHz, DMSO-*d₆*) δ 11.31 (s, 1H), 10.45 (s, 1H), 9.17 (s, 1H), 8.63 (s, 1H), 7.90 - 7.82 (m, 1H), 7.15 (s, 1H), 6.96 - 6.91 (m, 1H), 6.85 (s, 1H), 4.27 - 4.13 (m, 2H), 4.03 (s, 3H), 3.78 - 3.68 (m, 1H), 3.46 (s, 3H), 3.35 (s, 3H), 2.11 (s, 3H), 1.20 (d, *J =* 6.2 Hz, 3H). MS (ESI) m/e [M+1]⁺ 475. |
| J15 | 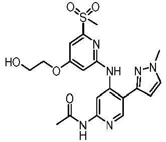 | N-(4-((4-(2-hydroxyethoxy)-6-(methylsulfonyl)pyridi n-2-yl)amino)-5-(1-methyl-1H-pyrazol-3-yl)pyridin-2-yl)acetamide | 1H NMR (400 MHz, DMSO-d6) δ 11.33 (s, 1H), 10.44 (s, 1H), 9.19 (s, 1H), 8.63 (s, 1H), 7.90 - 7.86 (m, 1H), 7.14 (s, 1H), 6.95 - 6.92 (m, 1H), 6.82 (s, 1H), 5.03 - 4.97 (m, 1H), 4.28 - 4.20 (m, 2H), 4.03 (s, 3H), 3.80 - 3.72 (m, 2H), 3.46 (s, 3H), 2.10 (s, 3H). MS (ESI) m/e [M+1]⁺ 447. |
| J16 | 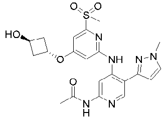 | N-(4-((4-((trans)-3-hydroxycyclobutoxy)-6-(methylsulfonyl)pyridi n-2-yl)amino)-5-(1-methyl-1H-pyrazol-3-yl)pyridin-2-yl)acetamide | ¹H NMR (400 MHz, DMSO-*d₆*) δ 11.34 (s, 1H), 10.44 (s, 1H), 9.17 (s, 1H), 8.63 (s, 1H), 7.98 - 7.91 (m, 1H), 7.01 (s, 1H), 6.96 - 6.91 (m, 1H), 6.63 (s, 1H), 5.32 - 5.26 (m, 1H), 5.12 - 5.04 (m, 1H), 4.45 - 4.35 (m, 1H), 4.02 (s, 3H), 3.46 (s, 3H), 2.46 - 2.30 (m, 4H), 2.10 (s, 3H). MS (ESI) m/e [M+1]⁺ 473. |
| J17 | 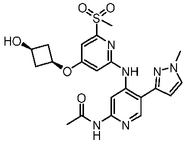 | N-(4-((4-((cis)-3-hydroxycyclobutoxy)-6-(methylsulfonyl)pyridi n-2-yl)amino)-5-(1-methyl-1H-pyrazol-3-yl)pyridin-2-yl)acetamide | ¹H NMR (400 MHz, DMSO-*d₆*) δ 11.34 (s, 1H), 10.43 (s, 1H), 9.15 (s, 1H), 8.62 (s, 1H), 7.88 (d, *J =* 2.4 Hz, 1H), 7.02 (s, 1H), 6.91 (d, *J =* 2.4 Hz, 1H), 6.67 (s, 1H), 5.29 (d, *J =* 6.8 Hz, 1H), 4.68 - 4.43 (m, 1H), 4.00 (s, 3H), 3.89 - 3.82 (m, 1H), 3.44 (s, 3H), 2.90 - 2.79 (m, 2H), 2.08 (s, 3H), 2.00 - 1.92 (m, 2H). MS (ESI) m/e [M+1]⁺ 473. |
| J18 | 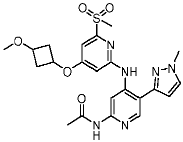 | N-(4-((4-(3-methoxycyclobutoxy)-6-(methylsulfonyl)pyridi n-2-yl)amino)-5-(1-methyl-1H-pyrazol-3-yl)pyridin-2-yl)acetamide | ¹H NMR (400 MHz, DMSO-*d₆*) δ 11.33 (s, 1H), 10.45 (s, 1H), 9.16 (s, 1H), 8.63 (s, 1H), 7.92 - 7.89 (m, 1H), 7.02 (s, 1H), 6.95 - 6.91 (m, 1H), 6.65 (s, 1H), 5.13 - 5.05 (m, 1H), 4.15 - 4.07 (m, 1H), 4.02 (s, 3H), 3.46 (s, 3H), 3.18 (s, 3H), 2.48 - 2.34 (m, 4H), 2.10 (s, 3H). MS (ESI) m/e [M+1]⁺ 487. |
| J19 | 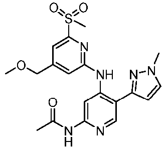 | N-(4-((4-(methoxymethyl)-6-(methylsulfonyl)pyridi n-2-yl)amino)-5-(1-methyl-1H-pyrazol-3-yl)pyridin-2-yl)acetamide | ¹H NMR (400 MHz, DMSO-*d₆*) δ 11.52 (s, 1H), 10.46 (s, 1H), 9.28 (s, 1H), 8.66 (s, 1H), 7.95 - 7.90 (m, 1H), 7.52 (s, 1H), 7.23 (s, 1H), 6.98 - 6.94 (m, 1H), 4.59 (s, 2H), 4.03 (s, 3H), 3.50 (s, 3H), 3.40 (s, 3H), 2.11 (s, 3H). MS (ESI) m/e [M+1]⁺ 431. |
| J20 | 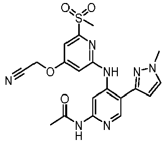 | N-(4-((4-(cyanomethoxy)-6-(methylsulfonyl)pyridi n-2-yl)amino)-5-(1-methyl-1H-pyrazol-3-yl)pyridin-2-yl)acetamide | ¹H NMR (400 MHz, DMSO-*d₆*) δ 11.51 (s, 1H), 10.46 (s, 1H), 9.21 (s, 1H), 8.66 (s, 1H), 7.90 - 7.85 (m, 1H), 7.27 (s, 1H), 6.95 - 6.90 (m, 2H), 5.46 (s, 2H), 4.04 (s, 3H), 3.50 (s, 3H), 2.11 (s, 3H). MS (ESI) m/e [M+1]⁺ 442. |
| J21 | 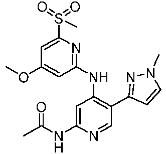 | N-(4-((4-methoxy-6-(methylsulfonyl)pyridi n-2-yl)amino)-5-(1-methyl-1H-pyrazol-3-yl)pyridin-2-yl)acetamide | ¹H NMR (400 MHz, DMSO-*d₆*) δ 11.35 (s, 1H), 10.45 (s, 1H), 9.18 (s, 1H), 8.64 (s, 1H), 7.93 - 7.89 (m, 1H), 7.14 (s, 1H), 6.96 - 6.92 (m, 1H), 6.83 (s, 1H), 4.03 (s, 3H), 3.97 (s, 3H), 3.46 (s, 3H), 2.11 (s, 3H). MS (ESI) m/e [M+1]⁺ 417. |
| J22 | 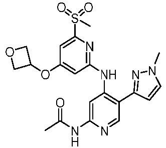 | N-(5-(1-methyl-1H-pyrazol-3-yl)-4-((6-(methylsulfonyl)-4-(oxetan-3-yloxy)pyridin-2-yl)amino)pyridin-2-yl)acetamide | ¹H NMR (400 MHz, DMSO-*d₆*) δ 11.38 (s, 1H), 10.46 (s, 1H), 9.17 (s, 1H), 8.64 (s, 1H), 7.93 - 7.89 (m, 1H), 7.03 (s, 1H), 6.95 - 6.90 (m, 1H), 6.59 (s, 1H), 5.62 - 5.54 (m, 1H), 5.05 - 4.94 (m, 2H), 4.67 - 4.60 (m, 2H), 4.03 (s, 3H), 3.47 (s, 3H), 2.11 (s, 3H). MS (ESI) m/e [M+1]⁺ 459. |
| J23 | 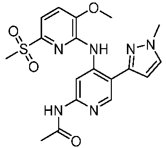 | N-(4-((3-methoxy-6-(methylsulfonyl)pyridi n-2-yl)amino)-5-(1-methyl-1H-pyrazol-3-yl)pyridin-2-yl)acetamide | ¹H NMR (400 MHz, DMSO-*d₆*) δ 11.90 (s, 1H), 10.41 (s, 1H), 9.49 (s, 1H), 8.64 (s, 1H), 7.89 - 7.86 (m, 1H), 7.60 - 7.56 (m, 1H), 7.54 - 7.50 (m, 1H), 6.92 - 6.89 (m, 1H), 4.08 (s, 3H), 3.99 (s, 3H), 3.45 (s, 3H), 2.09 (s, 3H). MS (ESI) m/e [M+1]⁺ 417. |
| J24 | 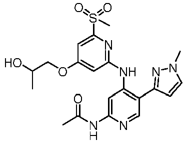 | N-(4-((4-(2-hydroxypropoxy)-6-(methylsulfonyl)pyridi n-2-yl)amino)-5-(1-methyl-1H-pyrazol-3-yl)pyridin-2-yl)acetamide | ¹H NMR (400 MHz, DMSO-*d₆*) δ 11.30 (s, 1H), 10.44 (s, 1H), 9.18 (s, 1H), 8.63 (s, 1H), 7.94 - 7.89 (m, 1H), 7.14 (s, 1H), 6.95 - 6.91 (m, 1H), 6.82 (s, 1H), 5.04 - 4.98 (m, 1H), 4.12 - 3.96 (m, 5H), 3.46 (s, 3H), 2.11 (s, 3H), 1.22 - 1.12 (m, 2H). MS (ESI) m/e [M+1]⁺ 461. |
| J25 | 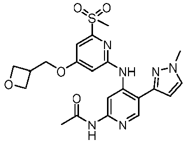 | N-(5-(1-methyl-1H-pyrazol-3-yl)-4-((6-(methylsulfonyl)-4-(oxetan-3-ylmethoxy)pyridin-2-yl)amino)pyridin-2-yl)acetamide | ¹H NMR (400 MHz, DMSO-*d₆*) δ 11.30 (s, 1H), 10.43 (s, 1H), 9.17 (s, 1H), 8.62 (s, 1H), 7.88 (d, *J =* 2.4 Hz, 1H), 7.15 (s, 1H), 6.91 (d, *J* = 2.4 Hz, 1H), 6.83 (s, 1H), 4.72 - 4.69 (m, 2H), 4.45 - 4.42 (m, 4H), 4.01 (s, 3H), 3.49 - 3.42 (m, 4H), 2.08 (s, 3H). MS (ESI) m/e [M+1]⁺ 473. |
| J26 | 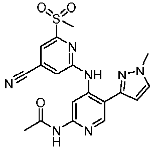 | N-(4-((4-cyano-6-(methylsulfonyl)pyridi n-2-yl)amino)-5-(1-methyl-1H-pyrazol-3-yl)pyridin-2-yl)acetamide | ¹H NMR (400 MHz, DMSO-*d₆*) δ 11.80 (s, 1H), 10.53 (s, 1H), 9.28 (s, 1H), 8.70 (s, 1H), 7.96 (s, 1H), 7.95 - 7.90 (m, 1H), 7.84 (s, 1H), 6.99 - 6.95 (m, 1H), 4.07 (s, 3H), 3.54 (s, 3H), 2.12 (s, 3H). MS (ESI) m/e [M+1]⁺ 412. |
| J27 | 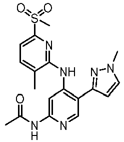 | N-(5-(1-methyl-1H-pyrazol-3-yl)-4-((3-methyl-6-(methylsulfonyl)pyridi n-2-yl)amino)pyridin-2-yl)acetamide | ¹H NMR (400 MHz, DMSO-*d₆*) δ 11.29 (s, 1H), 10.45 (s, 1H), 9.40 (s, 1H), 8.71 (s, 1H), 7.92 - 7.89 (m, 2H), 7.54 (d, *J =* 7.5 Hz, 1H), 6.98 - 6.97 (m, 1H), 3.98 (s, 3H), 3.47 (s, 3H), 2.56 (s, 3H), 2.11 (s, 3H). MS (ESI) m/e [M+1]⁺ 401. |
| J28 | 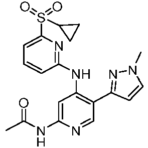 | N-(4-((6-(cyclopropylsulfonyl)p yridin-2-yl)amino)-5-(1-methyl-1H-pyrazol-3-yl)pyridin-2-yl)acetamide | ¹H NMR (400 MHz, DMSO-*d₆*) δ 11.50 (s, 1H), 10.44 (s, 1H), 9.18 (s, 1H), 8.66 (s, 1H), 8.05 - 7.95 (m, 1H), 7.90 - 7.89 (m, 1H), 7.49 (d, *J* = 7.2 Hz, 1H), 7.38 (d, *J =* 7.2 Hz, 1H), 6.95 - 6.90 (m, 1H), 4.02 (s, 3H), 3.70 - 3.65 (m, 1H), 2.09 (s, 3H), 1.15 - 1.05 (m, 4H). MS (ESI) m/e [M+1]⁺ 413. |
| J29 | 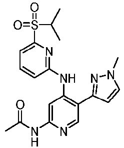 | N-(4-((6-(isopropylsulfonyl)pyri din-2-yl)amino)-5-(1-methyl-1H-pyrazol-3-yl)pyridin-2-yl)acetamide | ¹H NMR (400 MHz, DMSO-*d₆*) δ 11.52 (s, 1H), 10.44 (s, 1H), 9.17 (s, 1H), 8.66 (s, 1H), 8.01 (t, *J =* 7.6 Hz, 1H), 7.90 (d, *J =* 2.1 Hz, 1H), 7.58 (d, *J =* 8.1 Hz, 1H), 7.38 (d, *J =* 8.5 Hz, 1H), 6.94 (d, *J =* 2.1 Hz, 1H), 4.51 - 4.35 (m, 1H), 4.02 (s, 3H), 2.11 (s, 3H), 1.22 (d, *J =* 6.2 Hz, 6H). MS (ESI) m/e [M+1]⁺ 415. |
| J30 | 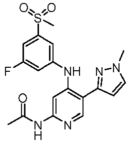 | N-(4-((3-fluoro-5-(methylsulfonyl)pheny 1)amino)-5-(1-methyl-1H-pyrazol-3-yl)pyridin-2-yl)acetamide | ¹H NMR (400 MHz, DMSO-*d₆*) δ 10.47 (s, 1H), 10.35 (s, 1H), 8.60 (s, 1H), 8.21 (s, 1H), 7.86 (s, 1H), 7.69 (s, 1H), 7.55 (d, *J =* 4.6 Hz, 1H), 7.40 (s, 1H), 6.87 (d, *J =* 4.6 Hz, 1H), 3.96 (s, 3H), 3.34 (s, 3H), 2.07 (s, 3H). MS (ESI) m/e [M+1]⁺404. |
| J31 | 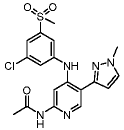 | N-(4-((3-chloro-5-(methylsulfonyl)pheny 1)amino)-5-(1-methyl-1H-pyrazol-3-yl)pyridin-2-yl)acetamide | ¹H NMR (400 MHz, DMSO-*d₆*) δ 10.47 (s, 1H), 10.30 (s, 1H), 8.59 (s, 1H), 8.17 (s, 1H), 7.85 - 7.84 (m, 1H), 7.79 (s, 1H), 7.73 (s, 1H), 7.59 (s, 1H), 6.88 - 6.85 (m, 1H), 3.96 (s, 3H), 3.35 (s, 3H), 2.07 (s, 3H). MS (ESI) m/e [M+1]⁺ 420. |
| J32 | 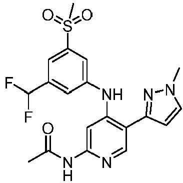 | N-(4-((3-(difluoromethyl)-5-(methylsulfonyl)pheny 1)amino)-5-(1-methyl-1H-pyrazol-3-yl)pyridin-2-yl)acetamide | ¹H NMR (400 MHz, DMSO-*d₆*) δ 10.48 (s, 1H), 10.34 (s, 1H), 8.60 (s, 1H), 8.17 (s, 1H), 8.00 (s, 1H), 7.86 -7.83 (m, 1H), 7.76 (s, 1H), 7.17 (s, 1H), 6.88 - 6.82 (m, 1H), 3.96 (s, 3H), 3.36 (s, 3H), 2.06 (s, 3H). MS (ESI) m/e [M+1]⁺436. |
| J33 | 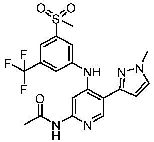 | N-(5-(1-methyl-1H-pyrazol-3-yl)-4-((3-(methylsulfonyl)-5-(trifluoromethyl)pheny 1)amino)pyridin-2-yl)acetamide | ¹H NMR (400 MHz, DMSO-*d₆*) δ 10.48 (s, 1H), 10.34 (s, 1H), 8.60 (s, 1H), 8.20 (s, 1H), 8.10 (s, 1H), 7.99 (s, 1H), 7.87 - 7.84 (m, 1H), 7.82 (s, 1H), 6.89 - 6.85 (m, 1H), 3.96 (s, 3H), 3.40 (s, 3H), 2.07 (s, 3H). MS (ESI) m/e [M+1]⁺ 454. |
| J34 | 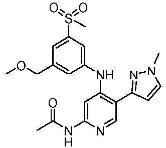 | N-(4-((3-(methoxymethyl)-5-(methylsulfonyl)pheny 1)amino)-5-(1-methyl-1H-pyrazol-3-yl)pyridin-2-yl)acetamide | ¹H NMR (400 MHz, DMSO-*d₆*) δ 10.43 (s, 1H), 10.28 (s, 1H), 8.57 (s, 1H), 8.13 (s, 1H), 7.89 - 7.85 (m, 1H), 7.77 (s, 1H), 7.58 (s, 1H), 7.55 (s, 1H), 6.89 - 6.87 (m, 1H), 4.54 (s, 2H), 3.96 (s, 3H), 3.36 (s, 3H), 3.30 (s, 3H), 2.06 (s, 3H). MS (ESI) m/e [M+1]⁺ 430. |
| J35 | 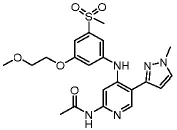 | N-(4-((3-(2-methoxyethoxy)-5-(methylsulfonyl)pheny 1)amino)-5-(1-methyl-1H-pyrazol-3-yl)pyridin-2-yl)acetamide | ¹H NMR (400 MHz, DMSO-*d₆*) δ 10.43 (s, 1H), 10.27 (s, 1H), 8.57 (s, 1H), 8.17 (s, 1H), 7.88 - 7.85 (m, 1H), 7.41 (s, 1H), 7.22 (s, 1H), 7.14 (s, 1H), 6.88 - 6.84 (m, 1H), 4.27 - 4.21 (m, 2H), 3.96 (s, 3H), 3.72 - 3.66 (m, 2H), 3.29 (s, 3H), 2.06 (s, 3H). MS (ESI) m/e [M+1]⁺ 460. |
| J36 | 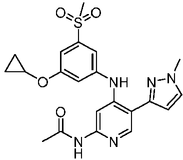 | N-(4-((3-cyclopropoxy-5-(methylsulfonyl)pheny 1)amino)-5-(1-methyl-1H-pyrazol-3-yl)pyridin-2-yl)acetamide | ¹H NMR (400 MHz, DMSO-*d₆*) *6* 10.42 (s, 1H), 10.29 (s, 1H), 8.57 (s, 1H), 8.19 (s, 1H), 7.90 - 7.80 (m, 1H), 7.44 (s, 1H), 7.32 (s, 1H), 7.24 (s, 1H), 6.90 - 6.80 (m, 1H), 4.05 - 4.00 (m, 1H), 3.96 (s, 3H), 3.30 (s, 3H), 2.06 (s, 3H), 0.82 - 0.80 (m, 2H), 0.73 - 0.71 (m, 2H). MS (ESI) m/e [M+1]⁺ 442. |
| J37 | 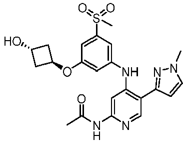 | N-(4-((3-((trans)-3-hydroxycyclobutoxy)-5-(methylsulfonyl)pheny 1)amino)-5-(1-methyl-1H-pyrazol-3-yl)pyridin-2-yl)acetamide | ¹H NMR (400 MHz, DMSO-*d₆*) δ 10.40 (s, 1H), 10.23 (s, 1H), 8.57 (s, 1H), 8.17 (s, 1H), 7.85 (d, *J* = 2.1 Hz, 1H), 7.41 (s, 1H), 7.03 (s, 1H), 6.99 (s, 1H), 6.87 (d, *J =* 2.1 Hz, 1H), 5.25 (s, 1H), 4.99 - 4.96 (m, 1H), 4.40 - 4.35 (m, 1H), 3.96 (s, 3H), 3.29 (s, 3H), 2.40 - 2.30 (m, 4H), 2.06 (s, 3H). MS (ESI) m/e [M+1]⁺ 472. |
| J38 | 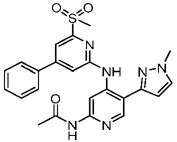 | N-(5-(1-methyl-1H-pyrazol-3-yl)-4-((6-(methylsulfonyl)-4-phenylpyridin-2-yl)amino)pyridin-2-yl)acetamide | ¹H NMR (400 MHz, DMSO-*d₆*) δ 11.54 (s, 1H), 10.47 (s, 1H), 9.28 (s, 1H), 8.67 (s, 1H), 7.94 - 7.88 (m, 3H), 7.79 (s, 1H), 7.66 - 7.52 (m, 4H), 6.95 - 6.94 (m, 1H), 4.04 (s, 3H), 3.53 (s, 3H), 2.13 (s, 3H). MS (ESI) m/e [M+1]⁺ 463. |
| J39 | 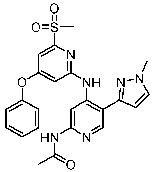 | N-(5-(1-methyl-1H-pyrazol-3-yl)-4-((6-(methylsulfonyl)-4-phenoxypyridin-2-yl)amino)pyridin-2-yl)acetamide | ¹H NMR (400 MHz, DMSO-*d₆*) δ 11.43 (s, 1H), 10.46 (s, 1H), 9.21 (s, 1H), 8.64 (s, 1H), 7.88 - 7.87 (m, 1H), 7.60 - 7.50 (m, 2H), 7.40 - 7.30 (m, 1H), 7.35 - 7.30 (m, 2H), 6.96 - 6.90 (m, 1H), 6.92 (s, 1H), 6.81 (s, 1H), 3.93 (s, 3H), 3.48 (s, 3H), 2.11 (s, 3H). MS (ESI) m/e [M+1]⁺ 479. |
| J40 | 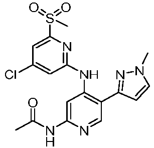 | N-(4-((4-chloro-6-(methylsulfonyl)pyridi n-2-yl)amino)-5-(1-methyl-1H-pyrazol-3-yl)pyridin-2-yl)acetamide | ¹H NMR (400 MHz, DMSO-*d₆*) δ 11.53 (s, 1H), 10.49 (s, 1H), 9.23 (s, 1H), 8.67 (s, 1H), 7.92 - 7.89 (m, 1H), 7.55 (s, 2H), 6.95 - 6.92 (m, 1H), 4.04 (s, 3H), 3.52 (s, 3H), 2.11 (s, 3H). MS (ESI) m/e [M+1]⁺ 421. |
| J41 | 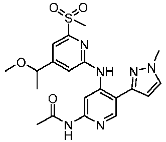 | N-(4-((4-(1-methoxyethyl)-6-(methylsulfonyl)pyridi n-2-yl)amino)-5-(1-methyl-1H-pyrazol-3-yl)pyridin-2-yl)acetamide | ¹H NMR (400 MHz, DMSO-*d₆*) δ 11.49 (s, 1H), 10.46 (s, 1H), 9.26 (s, 1H), 8.66 (s, 1H), 7.95 - 7.90 (m, 1H), 7.51 (s, 1H), 7.24 (s, 1H), 6.96 - 6.93 (m, 1H), 4.59 - 4.45 (m, 1H), 4.04 (s, 3H), 3.50 (s, 3H), 3.25 (s, 3H), 2.11 (s, 3H), 1.39 (d, *J =* 6.4 Hz, 3H). MS (ESI) m/e [M+1]⁺ 445. |
| J42 | 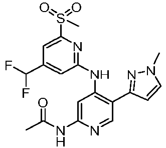 | N-(4-((4-(difluoromethyl)-6-(methylsulfonyl)pyridi n-2-yl)amino)-5-(1-methyl-1H-pyrazol-3-yl)pyridin-2-yl)acetamide | ¹H NMR (400 MHz, DMSO-*d₆*) δ 11.66 (s, 1H), 10.50 (s, 1H), 9.29 (s, 1H), 8.68 (s, 1H), 7.95 - 7.90 (m, 1H), 7.64 (s, 1H), 7.52 (s, 1H), 7.25 - 7.15 (m, 1H), 6.94 - 6.91 (m, 1H), 4.04 (s, 3H), 3.54 (s, 3H), 2.12 (s, 3H). MS (ESI) m/e [M+1]⁺ 437. |
| J43 | 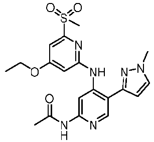 | N-(4-((4-ethoxy-6-(methylsulfonyl)pyridi n-2-yl)amino)-5-(1-methyl-1H-pyrazol-3-yl)pyridin-2-yl)acetamide | ¹H NMR (400 MHz, DMSO-*d₆*) δ 11.31 (s, 1H), 10.42 (s, 1H), 9.17 (s, 1H), 8.63 (s, 1H), 7.89 (s, 1H), 7.11 (d, *J =* 2.3 Hz, 1H), 6.92 (d, *J =* 2.3 Hz, 1H), 6.79 (s, 1H), 4.27 (d, *J =* 7.0 Hz, 2H), 4.02 (s, 3H), 3.46 (s, 3H), 2.10 (s, 3H), 1.38 (t, *J =* 6.9 Hz, 3H). MS (ESI) m/e [M+1]⁺ 431. |
| J44 | 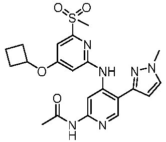 | N-(4-((4-cyclobutoxy-6-(methylsulfonyl)pyridi n-2-yl)amino)-5-(1-methyl-1H-pyrazol-3-yl)pyridin-2-yl)acetamide | ¹H NMR (400 MHz, DMSO-*d₆*) δ 11.34 (s, 1H), 10.43 (s, 1H), 9.17 (s, 1H), 8.63 (s, 1H), 7.90 (d, *J =* 2.4 Hz, 1H), 7.03 (s, 1H), 6.92 (d, *J =* 2.4 Hz, 1H), 6.68 (s, 1H), 5.00 - 4.96 (m, 1H), 4.02 (s, 3H), 3.46 (s, 3H), 2.16 - 2.10 (m, 5H), 1.85 - 1.83 (m, 1H), 1.74 - 1.69 (m, 1H). MS (ESI) m/e [M+1]⁺ 457. |
| J45 | 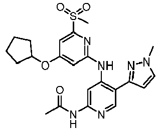 | N-(4-((4-(cyclopentyloxy)-6-(methylsulfonyl)pyridi n-2-yl)amino)-5-(1-methyl-1H-pyrazol-3-yl)pyridin-2-yl)acetamide | ¹H NMR (400 MHz, DMSO-*d₆*) δ 11.32 (s, 1H), 10.43 (s, 1H), 9.17 (s, 1H), 8.63 (s, 1H), 7.89 - 7.85 (m, 1H), 7.07 (s, 1H), 6.95 - 6.91 (m, 1H), 6.75 (s, 1H), 5.13 - 5.07 (m, 1H), 4.02 (s, 3H), 3.46 (s, 3H), 2.10 (s, 3H), 2.05 - 1.93 (m, 2H), 1.87 - 1.52 (m, 6H). MS (ESI) m/e [M+1]⁺ 471. |
| J46 | 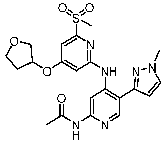 | N-(5-(1-methyl-1H-pyrazol-3-yl)-4-((6-(methylsulfonyl)-4-((tetrahydrofuran-3-yl)oxy)pyridin-2-yl)amino)pyridin-2-yl)acetamide | ¹H NMR (400 MHz, DMSO-*d₆*) δ 11.35 (s, 1H), 10.44 (s, 1H), 9.17 (s, 1H), 8.64 (s, 1H), 7.89 - 7.85 (m, 1H), 7.10 (s, 1H), 6.96 - 6.91 (m, 1H), 6.79 (s, 1H), 5.36 - 5.31 (m, 1H), 4.02 (s, 3H), 3.94 - 3.83 (m, 3H), 3.82 - 3.74 (m, 1H), 3.46 (s, 3H), 2.36 - 2.25 (m, 1H), 2.10 (s, 3H), 2.08 - 2.00 (m, 1H). MS (ESI) m/e [M+1]⁺ 473. |
| J47 | 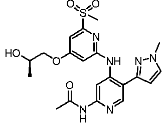 | (R)-N-(4-((4-(2-hydroxypropoxy)-6-(methylsulfonyl)pyridi n-2-yl)amino)-5-(1-methyl-1H-pyrazol-3-yl)pyridin-2-yl)acetamide | ¹H NMR (400 MHz, DMSO-*d₆*) δ 11.29 (s, 1H), 10.44 (s, 1H), 9.17 (s, 1H), 8.63 (s, 1H), 7.90 - 7.85 (m, 1H), 7.14 (s, 1H), 6.95 - 6.91 (m, 1H), 6.82 (s, 1H), 5.04 - 4.95 (m, 1H), 4.12 - 3.92 (m, 5H), 3.46 (s, 3H), 2.11 (s, 3H), 1.17 (d, *J =* 5.9 Hz, 3H). MS (ESI) m/e [M+1]⁺ 461. |
| J48 | 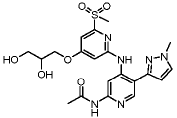 | N-(4-((4-(2,3-dihydroxypropoxy)-6-(methylsulfonyl)pyridi n-2-yl)amino)-5-(1-methyl-1H-pyrazol-3-yl)pyridin-2-yl)acetamide | ¹H NMR (400 MHz, DMSO-*d₆*) δ 11.32 (s, 1H), 10.44 (s, 1H), 9.19 (s, 1H), 8.63 (s, 1H), 7.89 - 7.86 (m, 1H), 7.15 (s, 1H), 6.95 - 6.91 (m, 1H), 6.83 (s, 1H), 5.16 - 4.97 (m, 1H), 4.84 - 4.64 (m, 1H), 4.30 - 4.22 (m, 1H), 4.12 - 4.02 (m, 1H), 4.02 (s, 3H), 3.87 - 3.80 (m, 1H), 3.49 - 3.43 (m, 5H), 2.11 (s, 3H). MS (ESI) m/e [M+1]⁺ 477. |
| J49 | 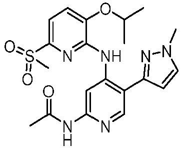 | N-(4-((3-isopropoxy-6-(methylsulfonyl)pyridi n-2-yl)amino)-5-(1-methyl-1H-pyrazol-3-yl)pyridin-2-yl)acetamide | ¹H NMR (400 MHz, DMSO-*d₆*) δ 10.97 (s, 1H), 10.38 (s, 1H), 9.25 (s, 1H), 8.58 (s, 1H), 7.86 - 7.83 (m, 1H), 7.54 - 7.50 (m, 2H), 6.89 - 6.85 (m, 1H), 4.94 - 4.86 (m, 1H), 3.96 (s, 3H), 3.36 (s, 3H), 2.05 (s, 3H), 1.40 (d, *J =* 6.0 Hz, 6H). MS (ESI) m/e [M+1]⁺ 445. |
| J50 | 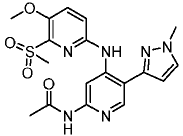 | N-(4-((5-methoxy-6-(methylsulfonyl)pyridi n-2-yl)amino)-5-(1-methyl-1H-pyrazol-3-yl)pyridin-2-yl)acetamide | ¹H NMR (400 MHz, DMSO-*d₆*) δ 11.03 (s, 1H), 10.41 (s, 1H), 8.69 (s, 1H), 8.59 (s, 1H), 7.90 (d, *J =* 2.8 Hz, 1H), 7.80 (d, *J =* 6.4 Hz, 1H), 7.48 - 7.45 (d, *J =* 6.4 Hz, 1H), 6.90 (d, *J =* 2.8 Hz, 1H), 3.99 (s, 3H), 3.93 (s, 3H), 3.39 (s, 3H), 2.08 (s, 3H). MS (ESI) m/e [M+1]⁺ 417. |
| J51 | 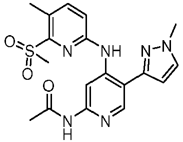 | N-(5-(1-methyl-1H-pyrazol-3-yl)-4-((5-methyl-6-(methylsulfonyl)pyridi n-2-yl)amino)pyridin-2-yl)acetamide | ¹H NMR (400 MHz, DMSO-*d₆*) δ 11.24 (s, 1H), 10.42 (s, 1H), 8.91 (s, 1H), 8.63 (s, 1H), 7.88 (s, 1H), 7.84 (d, *J* = 8.3 Hz, 1H), 7.31 (d, *J =* 8.3 Hz, 1H), 6.91 (d, *J =* 2.1 Hz, 1H), 4.00 (s, 3H), 3.47 (s, 3H), 2.53 (s, 3H), 2.09 (s, 3H). MS (ESI) m/e [M+1]⁺ 401. |
| J52 | 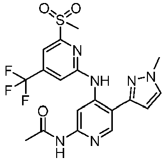 | N-(5-(1-methyl-1H-pyrazol-3-yl)-4-((6-(methylsulfonyl)-4-(trifluoromethyl)pyridi n-2-yl)amino)pyridin-2-yl)acetamide | ¹H NMR (400 MHz, DMSO-*d₆*) δ 11.61 (s, 1H), 10.52 (s, 1H), 9.24 (s, 1H), 8.68 (s, 1H), 7.90 - 7.85 (m, 1H), 7.73 (s, 1H), 7.67 (s, 1H), 6.95 - 6.91 (m, 1H), 4.03 (s, 3H), 3.55 (s, 3H), 2.12 (s, 3H). MS (ESI) m/e [M+1]⁺ 455. |
| J53 | 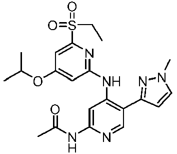 | N-(4-((6-(ethylsulfonyl)-4-isopropoxypyridin-2-yl)amino)-5-(1-methyl-1H-pyrazol-3-yl)pyridin-2-yl)acetamide | ¹H NMR (400 MHz, DMSO-*d₆*) δ 11.19 (s, 1H), 10.36 (s, 1H), 9.03 (s, 1H), 8.58 (s, 1H), 7.86 - 7.83 (m, 1H), 7.05 (s, 1H), 6.88 - 6.85 (m, 1H), 6.74 (s, 1H), 4.95 - 4.82 (m, 1H), 3.97 (s, 3H), 3.68 - 3.62 (m, 2H), 2.06 (s, 3H), 1.30 (d, *J* = 5.9 Hz, 6H), 1.13 (t, *J* = 7.3 Hz, 3H). MS (ESI) m/e [M+1]⁺ 459. |
| J54 | 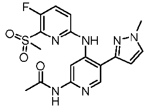 | N-(4-((5-fluoro-6-(methylsulfonyl)pyridi n-2-yl)amino)-5-(1-methyl-1H-pyrazol-3-yl)pyridin-2-yl)acetamide | ¹H NMR (400 MHz, DMSO-*d₆*) δ 11.34 (s, 1H), 10.39 (s, 1H), 8.94 (s, 1H), 8.60 (s, 1H), 7.95 (d, *J =* 9.4 Hz, 1H), 7.84 - 7.81 (m, 1H), 7.43 (d, *J =* 9.4 Hz, 1H), 6.87 - 6.82 (m, 1H), 3.96 (s, 3H), 3.49 (s, 3H), 2.05 (s, 3H). MS (ESI) m/e [M+1]⁺ 405. |
| J55 | 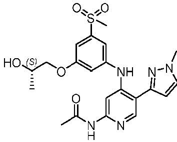 | (S)-N-(4-((3-(2-hydroxypropoxy)-5-(methylsulfonyl)pheny l)amino)-5-(1-methyl-1H-pyrazol-3-yl)pyridin-2-yl)acetamide | ¹H NMR (400 MHz, DMSO-*d₆*) δ 10.40 (s, 1H), 10.23 (s, 1H), 8.57 (s, 1H), 8.19 (s, 1H), 7.85 - 7.81 (m, 1H), 7.40 (s, 1H), 7.20 (s, 1H), 7.13 (s, 1H), 6.87 - 6.81 (m, 1H), 4.92 (s, 1H), 4.02 - 3.92 (m, 6H), 3.29 (s, 3H), 2.06 (s, 3H), 1.16 (d, *J =* 5.7 Hz, 3H). MS (ESI) m/e [M+1]⁺ 460. |
| J56 | 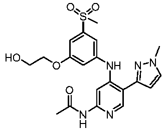 | N-(4-((3-(2-hydroxyethoxy)-5-(methylsulfonyl)pheny l)amino)-5-(1-methyl-1H-pyrazol-3-yl)pyridin-2-yl)acetamide | ¹H NMR (400 MHz, DMSO-*d₆*) δ 10.40 (s, 1H), 10.25 (s, 1H), 8.57 (s, 1H), 8.18 (s, 1H), 7.85 - 7.80 (m, 1H), 7.40 (s, 1H), 7.20 (s, 1H), 7.13 (s, 1H), 6.87 - 6.82 (m, 1H), 4.92 (s, 1H), 4.17 - 4.09 (m, 2H), 3.96 (s, 3H), 3.78 - 3.71 (m, 2H), 3.29 (s, 3H), 2.06 (s, 3H). MS (ESI) m/e [M+1]⁺ 446. |
| J57 | 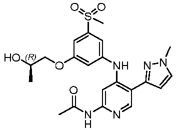 | (R)-N-(4-((3-(2-hydroxypropoxy)-5-(methylsulfonyl)pheny l)amino)-5-(1-methyl-1H-pyrazol-3-yl)pyridin-2-yl)acetamide | ¹H NMR (400 MHz, DMSO-*d₆*) δ 10.40 (s, 1H), 10.23 (s, 1H), 8.57 (s, 1H), 8.19 (s, 1H), 7.85 - 7.80 (m, 1H), 7.40 (s, 1H), 7.20 (s, 1H), 7.13 (s, 1H), 6.87 - 6.82 (m, 1H), 4.92 (s, 1H), 4.02 - 3.92 (m, 6H), 3.29 (s, 3H), 2.06 (s, 3H), 1.16 *(d, J =* 5.7 Hz, 3H). MS (ESI) m/e [M+1]⁺ 460. |
| J58 | 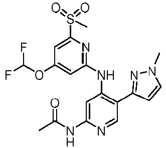 | N-(4-((4-(difluoromethoxy)-6-(methylsulfonyl)pyridi n-2-yl)amino)-5-(1-methyl-1H-pyrazol-3-yl)pyridin-2-yl)acetamide | ¹H NMR (400 MHz, DMSO-*d₆*) δ 11.51 (s, 1H), 10.47 (s, 1H), 9.22 (s, 1H), 8.66 (s, 1H), 7.90 (d, *J =* 2.8 Hz, 1H), 7.70 - 7.65 (m, 1H), 7.35 (s, 1H), 7.10 (s, 1H), 6.93 (d, *J* = 2.8 Hz, 1H), 4.03 (s, 3H), 3.52 (s, 3H), 2.11 (s, 3H). MS (ESI) m/e [M+1]⁺ 453. |
| J59 | 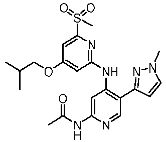 | N-(4-((4-isobutoxy-6-(methylsulfonyl)pyridi n-2-yl)amino)-5-(1-methyl-1H-pyrazol-3-yl)pyridin-2-yl)acetamide | ¹H NMR (400 MHz, DMSO-*d₆*) δ 11.26 (s, 1H), 10.42 (s, 1H), 9.16 (s, 1H), 8.62 (s, 1H), 7.89 (d, *J =* 2.2 Hz, 1H), 7.12 (s, 1H), 6.91 (d, *J =* 2.2 Hz, 1H), 6.82 (s, 1H), 4.03 (s, 3H), 4.05 - 4.00 (m, 2H), 3.45 (s, 3H), 2.10 (s, 3H), 2.09 - 2.03 (m, 1H), 1.01 (d, *J* = 6.7 Hz, 6H). MS (ESI) m/e [M+1]⁺ 459. |
| J60 | 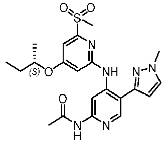 | (S)-N-(4-((4-(sec-butoxy)-6-(methylsulfonyl)pyridi n-2-yl)amino)-5-(1-methyl-1H-pyrazol-3-yl)pyridin-2-yl)acetamide | ¹H NMR (400 MHz, DMSO-*d₆*) δ 11.28 (s, 1H), 10.63 - 10.60 (m, 0H), 10.42 (s, 1H), 9.16 (s, 1H), 8.62 (s, 1H), 7.89 (d, *J =* 2.3 Hz, 1H), 7.09 - 7.05 (m, 1H), 6.91 (d, *J =* 2.3 Hz, 1H), 6.78 (s, 1H), 4.75 - 4.67 (m, 1H), 4.02 (s, 3H), 3.46 (s, 3H), 2.10 (s, 3H), 1.78 - 1.61 (m, 2H), 1.31 (d, *J=* 6.0 Hz, 3H), 0.95 (t, *J =* 7.4 Hz, 3H). MS (ESI) m/e [M+1]⁺ 459. |
| J61 | 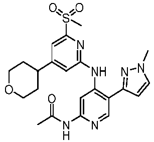 | N-(5-(1-methyl-1H-pyrazol-3-yl)-4-((6-(methylsulfonyl)-4-(tetrahydro-2H-pyran-4-yl)pyridin-2-yl)amino)pyridin-2-yl)acetamide | ¹H NMR (400 MHz, DMSO-*d₆*) δ 11.44 (s, 1H), 10.43 (s, 1H), 9.24 (s, 1H), 8.64 (s, 1H), 7.90 (d, *J =* 2.3 Hz, 1H), 7.48 (s, 1H), 7.18 (s, 1H), 6.93 (d, *J =* 2.3 Hz, 1H), 4.05 (s, 3H), 3.99 - 3.96 (m, 2H), 3.50 - 3.41 (m, 5H), 3.08 - 2.93 (m, 1H), 2.11 (s, 3H), 1.81 - 1.71 (m, 4H). MS (ESI) m/e [M+1]⁺ 471. |
| J62 | 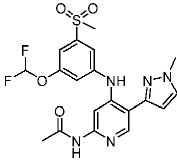 | N-(4-((3-(difluoromethoxy)-5-(methylsulfonyl)pheny l)amino)-5-(1-methyl-1H-pyrazol-3-yl)pyridin-2-yl)acetamide | ¹H NMR (400 MHz, DMSO-*d₆*) δ 10.46 (s, 1H), 10.31 (s, 1H), 8.59 (s, 1H), 8.20 (s, 1H), 7.85 *(d, J =* 2.2 Hz, 1H), 7.71 (s, 1H), 7.46 (s, 1H), 7.45 - 7.42 (m, 1H), 7.34 (s, 1H), 6.87 (d, *J =* 2.2 Hz, 1H), 3.96 (s, 3H), 3.34 (s, 3H), 2.07 (s, 3H). MS (ESI) m/e [M+1]⁺ 452. |
| J63 | 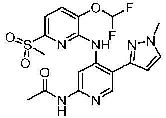 | N-(4-((3-(difluoromethoxy)-6-(methylsulfonyl)pyridi n-2-yl)amino)-5-(1-methyl-1H-pyrazol-3-yl)pyridin-2-yl)acetamide | ¹H NMR (400 MHz, DMSO-*d₆*) δ 11.79 (s, 1H), 10.48 (s, 1H), 9.45 (s, 1H), 8.70 (s, 1H), 7.90 (d, *J* = 2.1 Hz, 1H), 7.87 (d, *J* = 8.0 Hz, 1H), 7.65 (d, *J =* 8.0 Hz, 1H), 7.59 - 7.56 (m, 1H), 6.96 (d, *J=* 2.1 Hz, 1H), 3.97 (s, 3H), 3.51 (s, 3H), 2.12 (s, 3H). MS (ESI) m/e [M+1]⁺ 453. |
| J64 | 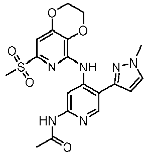 | N-(5-(1-methyl-1H-pyrazol-3-yl)-4-((7-(methylsulfonyl)-2,3-dihydro-[1,4]dioxino[2.3-c]pyridin-5-yl)amino)pyridin-2-yl)acetamide | ¹H NMR (400 MHz, DMSO-*d₆*) δ 11.83 (s, 1H), 10.41 (s, 1H), 9.45 (s, 1H), 8.65 (s, 1H), 7.89 - 7.85 (m, 1H), 7.16 (s, 1H), 6.95 - 6.90 (m, 1H), 4.63 - 4.59 (m, 2H), 4.53 - 4.48 (m, 2H), 3.99 (s, 3H), 3.47 (s, 3H), 2.11 (s, 3H). MS (ESI) m/e [M+1]⁺ 445. |
| J65 | 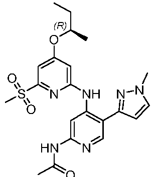 | (R)-N-(4-((4-(sec-butoxy)-6-(methylsulfonyl)pyridi n-2-yl)amino)-5-(1-methyl-1H-pyrazol-3-yl)pyridin-2-yl)acetamide | ¹H NMR (400 MHz, DMSO-*d₆*) δ 11.28 (s, 1H), 10.42 (s, 1H), 9.17 (s, 1H), 8.63 (s, 1H), 7.89 (d, *J =* 2.2 Hz, 1H), 7.09 - 7.05 (m, 1H), 6.92 (d, *J =* 2.2 Hz, 1H), 6.78 (s, 1H), 4.74 - 4.66 (m, 1H), 4.02 (s, 3H), 3.46 (s, 3H), 2.10 (s, 3H), 1.77 - 1.60 (m, 2H), 1.31 (d, *J =* 6.0 Hz, 3H), 0.95 (t, *J =* 7.4 Hz, 3H). MS (ESI) m/e [M+1]⁺ 459. |

### Example M1: Synthesis of N-(5-(1-cyclopropyl-1H-pyrazol-3-yl)-4-((6-(methylsulfonyl)pyridin-2-yl)amino)pyridin-2-yl)acetamide

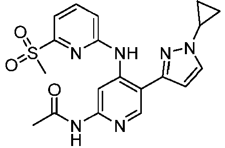

### Step 1: 3-bromo-1-cyclopropyl-1H-pyrazole

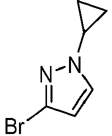

A mixture of Copper (II) acetate anhydrous (8.2 g, 41 mmol) and 2,2'bipyridyl (6.4 g, 41 mmol) in DCE (80 mL) was warmed to 50 °C and stirred for 10 min before adding to a mixture of 3-bromo-1H-pyrazole (6 g, 41 mmol), cyclopropylboronic acid (3.5 g, 41 mmol) and Na₂CO₃ (9.6 g, 90 mmol) in DCE (120 mL). The reaction mixture was stirred at 70 °C under O₂ atmosphere (O₂ balloon) for 2 d. The solvent was removed and the residue was dissolved in EA (300 mL) and washed with NH₄Cl solution (300 mL × 2) and brine (50 mL). The organic layer was dried over with Na₂SO₄ and filtered. The filtrate was concentrated and the residue was purified by column chromatography (PE/EA = 10: 1 - 5 : 1) to give 3-bromo-1-cyclopropyl-1H-pyrazole (5.5 g, impure). MS (ESI) m/e [M+1]⁺ 187.

### Step 2: tert-butyl (2-chloro-5-(1-cyclopropyl-1H-pyrazol-3-yl)pyridin-4-yl)carbamate

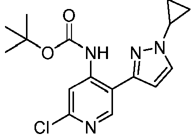

A mixture of 3-bromo-1-cyclopropyl-1H-pyrazole (5 g, 26.7 mmol), 4,4,4',4',5,5,5',5'-octamethyl-2,2'-bi(1,3,2-dioxaborolane) (1.34 g, 12 mmol), Pd(PPh₃)₂Cl₂ (702 mg, 1 mmol) and KOAc (5.2g, 53.4 mmol) in dioxane (100 mL) was stirred at 120 °C under N₂ for 6 hs. The reaction mixture was cooled to 90 °C and tert-butyl (5-bromo-2-chloropyridin-4-yl)carbamate (4.9 g, 16 mmol), K₂CO₃ (7.37 g, 53.4 mmol) and H₂O (20 mL) and the mixture was stirred at 100 °C for 2 h. The reaction mixture was cooled and diluted with EA (200 mL) and washed with H₂O (200 mL) and brine (200 mL). The organic layer was dried over with Na₂SO₄ and filtered. The filtrate was concentrated and the residue was purified by column chromatography (PE/EA = 10: 1 - 2 : 1) to give tert-butyl (2-chloro-5-(1-cyclopropyl-1H-pyrazol-3-yl)pyridin-4-yl)carbamate (4.5 g, 84%). MS (ESI) m/e [M+1]⁺ 335.

### Step 3: tert-butyl (2-acetamido-5-(1-cyclopropyl-1H-pyrazol-3-yl)pyridin-4-yl)carbamate

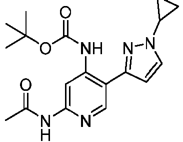

A mixture of tert-butyl (2-chloro-5-(1-cyclopropyl-1H-pyrazol-3-yl)pyridin-4-yl)carbamate (500 mg, 1.5 mmol), acetamide (177 mg, 3 mmol), Pd₂dba₃ (137 mg, 0.15 mmol), Xant-Phos (87 mg, 0.15 mmol) and Cs₂CO₃ (978 mg, 3 mmol) in dioxane (20 mL) was stirred at 130 °C under N₂ in a sealed tube for 4 hs. The reaction mixture was filtered and the solid was washed with EA (10 mL). The filtrate was concentrated and the residue was purified by column chromatography (DCM/MeOH = 70: 1 to 40 :1) to give tert-butyl (2-acetamido-5-(1-cyclopropyl-1H-pyrazol-3-yl)pyridin-4-yl)carbamate (400 mg, 75%). MS (ESI) m/e [M+1]⁺ 358.

### Step 4: N-(4-amino-5-(1-cyclopropyl-1H-pyrazol-3-yl)pyridin-2-yl)acetamide

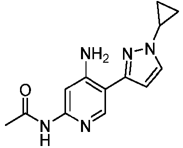

A solution of tert-butyl (2-acetamido-5-(1-cyclopropyl-1H-pyrazol-3-yl)pyridin-4-yl)carbamate (400 mg, 1.12 mmol) in TFA/DCM (14 mL, 1: 3) was stirred at rt for 4 hs. The solvent evaporated and the residue was diluted with EA (20 mL) and washed with NaHCO₃ solution (10 mL) and brine (10 mL). The organic layer was dried over with Na₂SO₄ and filtered. The filtrate was concentrated to give the crude product N-(4-amino-5-(1-cyclopropyl-1H-pyrazol-3-yl)pyridin-2-yl)acetamide (300 mg). MS (ESI) m/e [M+1]⁺ 258.

### Step 5: N-(5-(1-cyclopropyl-1H-pyrazol-3-yl)-4-((6-(methylsulfonyl)pyridin-2-yl)amino)pyridin-2-yl)acetamide

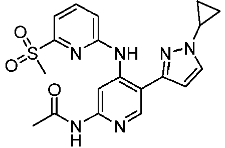

A mixture of N-(4-amino-5-(1-cyclopropyl-1H-pyrazol-3-yl)pyridin-2-yl)acetamide (60 mg, 0.23 mmol), 2-bromo-6-(methylsulfonyl)pyridine (66 mg, 0.28 mmol), Pd₂dba₃ (18 mg, 0.02 mmol), BINAP (12 mg, 0.02 mmol) and K₂CO₃ (60 mg, 0.46 mmol) in dioxane (5 mL) was stirred at 130 °C under N₂ in a sealed tube for 4 h. The reaction mixture was filtered and the solid was washed with EA (10 mL). The filtrate was concentrated and the residue was purified by prep-TLC (DCM: MeOH = 20:1) twice to give N-(5-(1-cyclopropyl-1H-pyrazol-3-yl)-4-((6-(methylsulfonyl)pyridin-2-yl)amino)pyridin-2-yl)acetamide (43 mg, 45%). ¹H NMR (400 MHz, DMSO-*d₆*) δ 11.47 (s, 1H), 10.46 (s, 1H), 9.23 (s, 1H), 8.64 (s, 1H), 8.05 - 8.00 (m, 1H), 7.98 (d, *J =* 2.4 Hz, 1H), 7.56 (d, *J* = 8.3 Hz, 1H), 7.21 (d, *J =* 8.3 Hz, 1H), 6.93 (d, *J* = 2.4 Hz, 1H), 3.96 - 3.86 (m, 1H), 3.48 (s, 3H), 2.09 (s, 3H), 1.20 - 1.16 (m, 2H), 1.08 - 1.04 (m, 2H). MS (ESI) m/e [M+1]⁺ 413.

The following Examples were prepared in a similar manner to the product Example M1:

| **Exampl e** | **Compound** | **Chemical Name** | **¹H NMR and LC /MS *m*/*z* (M+1)** |
|---|---|---|---|
| M2 | 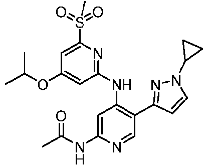 | N-(5-(1-cyclopropyl-1H-pyrazol-3-yl)-4-((4-isopropoxy-6-(methylsulfonyl)pyridin-2-yl)amino)pyridin-2-yl)acetamide | ¹H NMR (400 MHz, DMSO-d6) δ 11.34 (s, 1H), 10.46 (s, 1H), 9.14 (s, 1H), 8.64 (s, 1H), 8.02 (d, *J* = 2.4 Hz, 1H), 7.06 (s, 1H), 6.94 (d, *J =* 2.4 Hz, 1H), 6.67 (s, 1H), 4.88 - 4.82 (m, 1H), 3.96 - 3.92 (m, 1H), 3.46 (s, 3H), 2.11 (s, 3H), 1.36 (d, *J* = 5.2 Hz, 6H), 1.25 - 1.21 (m, 2H), 1.07 - 1.05 (m, 2H). MS (ESI) m/e [M+1]⁺ 471. |
| M3 | 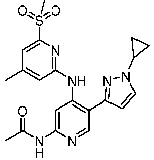 | N-(5-(1-cyclopropyl-1H-pyrazol-3-yl)-4-((4-methyl-6-(methylsulfonyl)pyridin-2-yl)amino)pyridin-2-yl)acetamide | ¹H NMR (400 MHz, DMSO-*d₆*) δ 11.40 (s, 1H), 10.46 (s, 1H), 9.22 (s, 1H), 8.65 (s, 1H), 8.00 (d, *J* = 2.4 Hz, 1H), 7.47 (s, 1H), 7.05 (s, 1H), 6.95 (d, *J =* 2.4 Hz, 1H), 3.95 - 3.92 (m, 1H), 3.47 (s, 3H), 2.45 (s, 3H), 2.11 (s, 3H), 1.20 - 1.15 (m, 2H), 1.09 - 1.05 (m, 2H). MS (ESI) m/e [M+1]⁺ 427. |
| M4 | 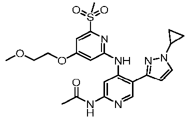 | N-(5-(1-cyclopropyl-1H-pyrazol-3-yl)-4-((4-(2-methoxyethoxy)-6-(methylsulfonyl)pyridin-2-yl)amino)pyridin-2-yl)acetamide | ¹H NMR (400 MHz, DMSO-*d₆*) δ 11.33 (s, 1H), 10.47 (s, 1H), 9.12 (s, 1H), 8.64 (s, 1H), 8.00 (d, *J* = 2.4 Hz, 1H), 7.14 (s, 1H), 6.94 (d, *J* = 2.4 Hz, 1H), 6.73 (s, 1H), 4.35 - 4.33 (m, 2H), 3.93 - 3.90 (m, 1H), 3.75 - 3.71 (m, 2H), 3.46 (s, 3H), 3.34 (s, 3H), 2.11 (s, 3H), 1.25 - 1.20 (m, 2H), 1.07 - 1.02 (m, 2H). MS (ESI) m/e [M+1]⁺ 487. |
| M5 | 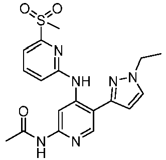 | N-(5-(1-ethyl-1H-pyrazol-3-yl)-4-((6-(methylsulfonyl)pyridin-2-yl)amino)pyridin-2-yl)acetamide | ¹H NMR (400 MHz, DMSO-*d₆*) δ 11.61 (s, 1H), 10.45 (s, 1H), 9.26 (s, 1H), 8.70 - 8.60 (m, 1H), 8.10 - 8.00 (m, 1H), 7.94 (s, 1H), 7.58 - 7.49 (m, 1H), 7.35 - 7.25 (m, 1H), 7.00 - 6.90 (m, 1H), 4.31 (q, *J =* 7.3 Hz, 2H), 3.48 (s, 3H), 2.09 (s, 3H), 1.48 (t, *J* = 7.3 Hz, 3H). MS (ESI) m/e [M+1]⁺ 401. |
| M6 | 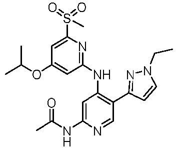 | N-(5-(1-ethyl-1H-pyrazol-3-yl)-4-((4-isopropoxy-6-(methylsulfonyl)pyridin-2-yl)amino)pyridin-2-yl)acetamide | ¹H NMR (400 MHz, DMSO-*d₆*) δ 11.41 (s, 1H), 10.44 (s, 1H), 9.16 (s, 1H), 8.64 (s, 1H), 8.00 - 7.90 (m, 1H), 7.10 - 6.95 (m, 1H), 6.93 (s, 1H), 6.73 (s, 1H), 4.95 - 4.87 (m, 1H), 4.35 - 4.27 (m, 2H), 3.46 (s, 3H), 2.11 (s, 3H), 1.50 (t, *J =* 7.2 Hz, 3H), 1.35 (d, *J =* 5.9 Hz, 6H). MS (ESI) m/e [M+1]⁺ 459. |
| M7 | 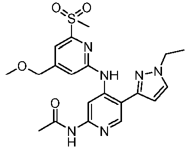 | N-(5-(1-ethyl-1H-pyrazol-3-yl)-4-((4-(methoxymethyl)-6-(methylsulfonyl)pyridin-2-yl)amino)pyridin-2-yl)acetamide | ¹H NMR (400 MHz, DMSO-*d₆*) δ 11.62 (s, 1H), 10.46 (s, 1H), 9.28 (s, 1H), 8.67 (s, 1H), 8.05 - 7.95 (m, 1H), 7.51 (s, 1H), 7.18 (s, 1H), 7.05 - 6.95 (m, 1H), 4.59 (s, 2H), 4.36 - 4.28 (m, 2H), 3.50 (s, 3H), 3.40 (s, 3H), 2.11 (s, 3H), 1.51 (t, *J =* 7.3 Hz, 3H). MS (ESI) m/e [M+1]⁺ 445. |
| M8 | 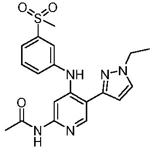 | N-(5-(1-ethyl-1H-pyrazol-3-yl)-4-((3-(methylsulfonyl)phenyl) amino)pyridin-2-yl)acetamide | ¹H NMR (400 MHz, DMSO-*d₆*) δ 10.41 (s, 1H), 10.40 (s, 1H), 8.58 (s, 1H), 8.19 (s, 1H), 7.98 - 7.87 (m, 1H), 7.84 (s, 1H), 7.72 - 7.58 (m, 3H), 6.95 - 6.85 (m, 1H), 4.29 - 4.21 (m, 2H), 3.30 (s, 3H), 2.06 (s, 3H), 1.45 (t, *J =* 7.3 Hz, 3H). MS (ESI) m/e [M+1]⁺ 400. |
| M9 | 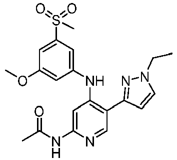 | N-(5-(1-ethyl-1H-pyrazol-3-yl)-4-((3-methoxy-5-(methylsulfonyl)phenyl) amino)pyridin-2-yl)acetamide | ¹H NMR (400 MHz, DMSO-*d₆*) δ 10.41 (s, 1H), 10.39 (s, 1H), 8.58 (s, 1H), 8.23 (s, 1H), 7.90 (d, *J* = 2.1 Hz, 1H), 7.39 (s, 1H), 7.22 (s, 1H), 7.12 (s, 1H), 6.88 (d, *J* = 2.1 Hz, 1H), 4.26 (q, *J =* 7.2 Hz, 2H), 3.89 (s, 3H), 3.29 (s, 3H), 2.07 (s, 3H), 1.45 (t, *J =* 7.2 Hz, 3H). MS (ESI) m/e [M+1]⁺ 430. |
| M10 | 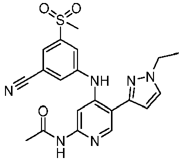 | N-(4-((3-cyano-5-(methylsulfonyl)phenyl) amino)-5-(1-ethyl-1H-pyrazol-3-yl)pyridin-2-yl)acetamide | ¹H NMR (400 MHz, DMSO-*d₆*) δ 10.50 (s, 1H), 10.44 (s, 1H), 9.12 (s, 1H), 8.62 (s, 1H), 8.18 (s, 1H), 8.08 (s, 1H), 7.97 (d, *J =* 2.2 Hz, 1H), 7.90 (s, 1H), 6.87 (d, *J =* 2.2 Hz, 1H), 4.26 (q, *J =* 7.3 Hz, 2H), 3.37 (s, 3H), 2.08 (s, 3H), 1.44 (t, *J* = 7.3 Hz, 3H). MS (ESI) m/e [M+1]⁺ 425. |
| M11 | 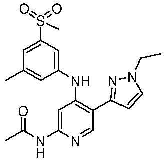 | N-(5-(1-ethyl-1H-pyrazol-3-yl)-4-((3-methyl-5-(methylsulfonyl)phenyl) amino)pyridin-2-yl)acetamide | ¹H NMR (400 MHz, DMSO-*d₆*) δ 10.38 (s, 1H), 10.33 (s, 1H), 8.57 (s, 1H), 8.17 (s, 1H), 7.90 (d, *J* = 2.1 Hz, 1H), 7.64 (s, 1H), 7.45 (s, 2H), 6.88 (d, *J =* 2.1 Hz, 1H), 4.25 (q, *J =* 7.2 Hz, 2H), 3.28 (s, 3H), 2.43 (s, 3H), 2.06 (s, 3H), 1.45 (t, *J* = 7.2 Hz, 3H). MS (ESI) m/e [M+1]⁺ 414. |
| M12 | 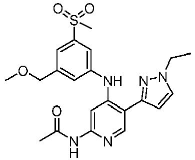 | N-(5-(1-ethyl-1H-pyrazol-3-yl)-4-((3-(methoxymethyl)-5-(methylsulfonyl)phenyl) amino)pyridin-2-yl)acetamide | ¹H NMR (400 MHz, DMSO-*d₆*) δ 10.40 (s, 1H), 10.37 (s, 1H), 8.58 (s, 1H), 8.17 (s, 1H), 7.90 (d, *J* = 2.2 Hz, 1H), 7.77 (s, 1H), 7.56 (s, 1H), 7.53 (s, 1H), 6.88 (d, *J* = 2.2 Hz, 1H), 4.54 (s, 2H), 4.25 (q, *J =* 7.3 Hz, 2H), 3.36 (s, 3H), 3.30 (s, 3H), 2.06 (s, 3H), 1.45 (t, *J =* 7.2 Hz, 3H). MS (ESI) m/e [M+1]⁺ 444. |
| M13 | 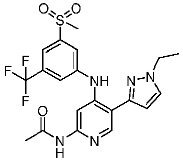 | N-(5-(1-ethyl-1H-pyrazol-3-yl)-4-((3-(methylsulfonyl)-5-(trifluoromethyl)phenyl) amino)pyridin-2-yl)acetamide | ¹H NMR (400 MHz, DMSO-*d₆*) δ 10.48 (s, 1H), 10.44 (s, 1H), 8.61 (s, 1H), 8.21 (s, 1H), 8.07 (s, 1H), 7.97 (s, 1H), 7.90 (d, *J =* 2.2 Hz, 1H), 7.80 (s, 1H), 6.86 (d, *J =* 2.2 Hz, 1H), 4.25 (q, *J =* 7.3 Hz, 2H), 3.39 (s, 3H), 2.07 (s, 3H), 1.43 (t, *J =* 7.2 Hz, 3H). MS (ESI) m/e [M+1]⁺ 468. |
| M14 | 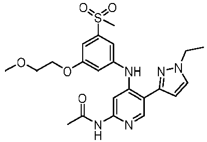 | N-(5-(1-ethyl-1H-pyrazol-3-yl)-4-((3-(2-methoxyethoxy)-5-(methylsulfonyl)phenyl) amino)pyridin-2-yl)acetamide | ¹H NMR (400 MHz, DMSO-*d₆*) δ 10.41 (s, 1H), 10.37 (s, 1H), 8.58 (s, 1H), 8.21 (s, 1H), 7.90 (d, *J* = 2.2 Hz, 1H), 7.40 (s, 1H), 7.20 (s, 1H), 7.13 (s, 1H), 6.88 (d, *J* = 2.2, 1H), 4.29 - 4.24 (m, 4H), 3.69 (s, 2H), 3.32 (s, 3H), 3.29 (s, 3H), 2.06 (s, 3H), 1.45 (t, *J =* 7.3 Hz, 3H). MS (ESI) m/e [M+1]⁺ 474. |
| M15 | 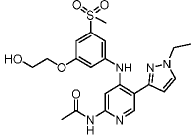 | N-(5-(1-ethyl-1H-pyrazol-3-yl)-4-((3-(2-hydroxyethoxy)-5-(methylsulfonyl)phenyl) amino)pyridin-2-yl)acetamide | ¹H NMR (400 MHz, DMSO-*d₆*) δ 10.47 (s, 1H), 10.31 (s, 1H), 8.57 (s, 1H), 7.91 (s, 1H), 7.41 (s, 1H), 7.20 (s, 1H), 7.19 (s, 1H), 6.89 (d, *J* = 2.4 Hz, 1H), 6.69 (d, *J* = 2.4 Hz, 1H), 4.26 (q, *J =* 7.4 Hz, 2H), 4.30 - 4.20 (m, 2H), 3.80 - 3.70 (m, 2H), 3.29 (s, 3H), 2.08 (s, 3H), 1.45 (t, *J* = 7.4 Hz, 3H). MS (ESI) m/e [M+1]⁺ 460. |
| M16 | 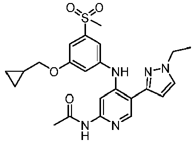 | N-(4-((3-(cyclopropylmethoxy)-5-(methylsulfonyl)phenyl) amino)-5-(1-ethyl-1H-pyrazol-3-yl)pyridin-2-yl)acetamide | 1H NMR (400 MHz, DMSO-*d₆*) δ 10.40 (s, 1H), 10.36 (s, 1H), 8.58 (s, 1H), 8.20 (s, 1H), 7.90 (d, *J* = 2.2 Hz, 1H), 7.37 (s, 1H), 7.18 (s, 1H), 7.10 (s, 1H), 6.88 (d, *J* = 2.2 Hz, 1H), 4.30 - 4.25 (m, 2H), 3.96 (d, *J =* 6.9 Hz, 2H), 3.28 (s, 3H), 2.06 (s, 3H), 1.45 (t, *J =* 7.2 Hz, 3H), 1.30 - 1.23 (m, 1H), 0.62 - 0.54 (m, 2H), 0.41 - 0.31 (m, 2H). MS (ESI) m/e [M+1]⁺ 470. |
| M17 | 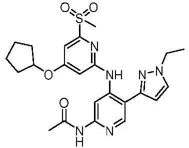 | N-(4-((4-(cyclopentyloxy)-6-(methylsulfonyl)pyridin-2-yl)amino)-5-(1-ethyl-1H-pyrazol-3-yl)pyridin-2-yl)acetamide | ¹H NMR (400 MHz, DMSO-*d₆*) δ 11.43 (s, 1H), 10.43 (s, 1H), 9.16 (s, 1H), 8.64 (s, 1H), 7.94 (s, 1H), 7.05 (s, 1H), 6.92 (d, *J =* 2.4 Hz, 1H), 6.70 (d, *J =* 2.4 Hz, 1H), 5.13 - 5.05 (m, 1H), 4.38 - 4.22 (m, 2H), 3.45 (s, 3H), 2.10 (s, 3H), 2.03 - 1.94 (m, 2H), 1.89 - 1.68 (m, 4H), 1.67 - 1.62 (m, 2H), 1.50 (t, *J =* 7.2 Hz, 3H). MS (ESI) m/e [M+1]⁺ 485. |
| M18 | 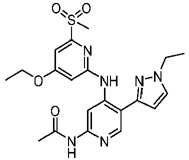 | N-(4-((4-ethoxy-6-(methylsulfonyl)pyridin-2-yl)amino)-5-(1-ethyl-1H-pyrazol-3-yl)pyridin-2-yl)acetamide | ¹H NMR (400 MHz, DMSO-*d₆*) δ 11.42 (s, 1H), 10.45 (s, 1H), 9.16 (s, 1H), 8.64 (s, 1H), 7.95 (d, *J* = 2.2 Hz, 1H), 7.10 (d, *J =* 1.8 Hz, 1H), 6.93 (d, *J =* 2.2 Hz, 1H), 6.75 (s, 1H), 4.32 - 4.30 (m, 2H), 4.29 - 4.23 (m, 2H), 3.46 (s, 3H), 2.11 (s, 3H), 1.50 (t, *J =* 7.2 Hz, 3H), 1.38 (t, *J =* 6.9 Hz, 3H). MS (ESI) m/e [M+1]⁺ 445. |
| M19 | 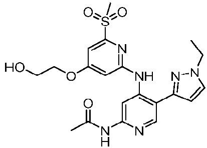 | N-(5-(1-ethyl-1H-pyrazol-3-yl)-4-((4-(2-hydroxyethoxy)-6-(methylsulfonyl)pyridin-2-yl)amino)pyridin-2-yl)acetamide | ¹H NMR (400 MHz, DMSO-*d₆*) δ 11.41 (s, 1H), 10.44 (s, 1H), 9.16 (s, 1H), 8.64 (s, 1H), 7.95 (s, 1H), 7.13 (s, 1H), 6.93 (d, *J =* 2.8 Hz, 1H), 6.77 (d, *J =* 2.8 Hz, 1H), 4.99 (t, 1H), 4.36 - 4.28 (m, 2H), 4.30 - 4.20 (m, 2H), 3.80 - 3.70 (m, 2H), 3.46 (s, 3H), 2.11 (s, 3H), 1.50 (t, *J* = 7.3 Hz, 3H). MS (ESI) m/e [M+1]⁺ 461. |
| M20 | 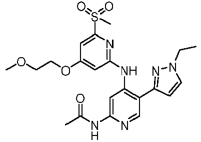 | N-(5-(1-ethyl-1H-pyrazol-3-yl)-4-((4-(2-methoxyethoxy)-6-(methylsulfonyl)pyridin-2-yl)amino)pyridin-2-yl)acetamide | ¹H NMR (400 MHz, DMSO-*d₆*) δ 11.46 (s, 1H), 10.60 (s, 1H), 9.10 (s, 1H), 8.64 (s, 1H), 7.96 (d, *J* = 2.2 Hz, 1H), 7.16 (s, 1H), 6.93 (s, 1H), 6.81 (d, *J =* 2.2 Hz, 1H), 4.37 - 4.31 (m, 4H), 3.75 - 3.71 (m, 2H), 3.45 (s, 3H), 3.32 (s, 3H), 2.12 (s, 3H), 1.50 (t, *J =* 7.2 Hz, 3H). MS (ESI) m/e [M+1]⁺ 475. |
| M21 | 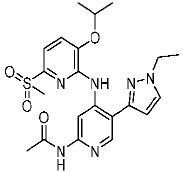 | N-(5-(1-ethyl-1H-pyrazol-3-yl)-4-((3-isopropoxy-6-(methylsulfonyl)pyridin-2-yl)amino)pyridin-2-yl)acetamide | ¹H NMR (400 MHz, DMSO-*d₆*) δ 10.97 (s, 1H), 10.43 (s, 1H), 9.24 (s, 1H), 8.63 (s, 1H), 7.95 (d, *J =* 8.4 Hz, 1H), 7.60 (d, *J =* 8.4 Hz, 1H), 6.90 (d, *J =* 2.3 Hz, 1H), 6.70 (d, *J* = 2.3 Hz, 1H), 4.99 - 4.86 (m, 1H), 4.39 - 4.23 (m, 2H), 3.39 (s, 3H), 2.10 (s, 3H), 1.49 - 1.45 (m, 6H), 1.45 - 1.40 (m, 3H). MS (ESI) m/e [M+1]⁺ 459. |
| M22 | 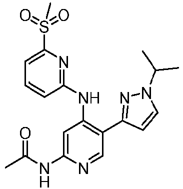 | N-(5-(1-isopropyl-1H-pyrazol-3-yl)-4-((6-(methylsulfonyl)pyridin-2-yl)amino)pyridin-2-yl)acetamide | ¹H NMR (400 MHz, DMSO-*d₆*) δ 11.65 (s, 1H), 10.46 (s, 1H), 9.26 (s, 1H), 8.68 (s, 1H), 8.04 - 7.99 (m, 2H), 7.58 (d, *J* = 7.0 Hz, 1H), 7.27 (d, *J =* 8.3 Hz, 1H), 6.95 (s, 1H), 4.72 - 4.69 (m, 1H), 3.49 (s, 3H), 2.12 (s, 3H), 1.54 (d, *J =* 6.6 Hz, 6H). MS (ESI) m/e [M+1]⁺ 415. |
| M23 | 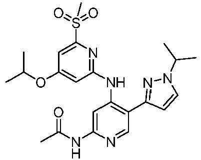 | N-(4-((4-isopropoxy-6-(methylsulfonyl)pyridin-2-yl)amino)-5-(1-isopropyl-1H-pyrazol-3-yl)pyridin-2-yl)acetamide | ¹H NMR (400 MHz, DMSO-*d₆*) δ 11.52 (s, 1H), 10.49 (s, 1H), 9.13 (s, 1H), 8.65 (s, 1H), 7.98 (d, *J* = 2.3 Hz, 1H), 7.07 (s, 1H), 6.93 (d, *J =* 2.3 Hz, 1H), 6.68 (s, 1H), 4.89 - 4.82 (m, 1H), 4.69 - 4.62 (m, 1H), 3.46 (s, 3H), 2.11 (s, 3H), 1.54 (d, *J* = 6.6 Hz, 6H), 1.35 (d, *J =* 5.8 Hz, 6H). MS (ESI) m/e [M+1]⁺ 473. |
| M24 | 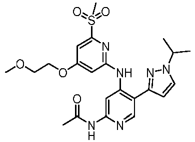 | N-(5-(1-isopropyl-1H-pyrazol-3-yl)-4-((4-(2-methoxyethoxy)-6-(methylsulfonyl)pyridin-2-yl)amino)pyridin-2-yl)acetamide | ¹H NMR (400 MHz, DMSO-*d₆*) δ 11.49 (s, 1H), 10.48 (s, 1H), 9.12 (s, 1H), 8.65 (s, 1H), 7.99 (d, *J* = 2.5 Hz, 1H), 7.14 (s, 1H), 6.94 (d, *J =* 2.5 Hz, 1H), 6.75 (s, 1H), 4.75 - 4.70 (m, 1H), 4.38 - 4.33 (m, 2H), 3.75 - 3.70 (m, 2H), 3.45 (s, 3H), 3.32 (s, 3H), 2.11 (s, 3H), 1.54 (d, *J =* 6.5 Hz, 6H). MS (ESI) m/e [M+1]⁺ 489. |
| M25 | 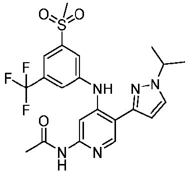 | N-(5-(1-isopropyl-1H-pyrazol-3-yl)-4-((3-(methylsulfonyl)-5-(trifluoromethyl)phenyl) amino)pyridin-2-yl)acetamide | ¹H NMR (400 MHz, DMSO-*d₆*) δ 10.58 (s, 1H), 10.52 (s, 1H), 8.63 (s, 1H), 8.23 (s, 1H), 8.06 (s, 1H), 7.95 (s, 1H),7.90 (d, *J =* 2.5 Hz, 1H), 7.81 (s, 1H), 6.88 (d, *J =* 2.5 Hz, 1H), 4.65 - 4.61 (m, 1H), 3.39 (s, 3H), 2.08 (s, 3H), 1.48 (d*, J =* 6.5 Hz, 6H). MS (ESI) m/e [M+1]⁺ 482. |
| M26 | 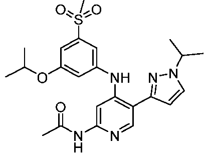 | N-(4-((3-isopropoxy-5-(methylsulfonyl)phenyl) amino)-5-(1-isopropyl-1H-pyrazol-3-yl)pyridin-2-yl)acetamide | ¹H NMR (400 MHz, DMSO-*d₆*) δ 10.53 (s, 1H), 10.48 (s, 1H), 8.58 (s, 1H), 8.13 (s, 1H), 7.95 (d, *J* = 2.5 Hz, 1H), 7.39 (s, 1H), 7.14 (s, 1H), 7.10 (s, 1H), 6.89 (d, J = 2.5 Hz, 1H), 4.78 - 4.75 (m, 1H), 4.65 - 4.61 (m, 1H), 3.29 (s, 3H), 2.08 (s, 3H), 1.49 (d, *J* = 6.6 Hz, 6H), 1.32 (d, *J =* 5.9 Hz, 6H). MS (ESI) m/e [M+1]⁺ 473. |
| M27 | 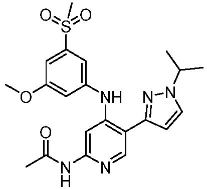 | N-(5-(1-isopropyl-1H-pyrazol-3-yl)-4-((3-methoxy-5-(methylsulfonyl)phenyl) amino)pyridin-2-yl)acetamide | ¹H NMR (400 MHz, DMSO-*d₆*) δ 10.58 (s, 1H), 10.49 (s, 1H), 8.58 (s, 1H), 8.16 (s, 1H), 7.95 (d, *J* = 2.2 Hz, 1H), 7.39 (s, 1H), 7.23 (s, 1H), 7.14 (s, 1H), 6.89 (d, *J* = 2.2 Hz, 1H), 4.65 - 4.62 (m, 1H), 3.89 (s, 3H), 3.29 (s, 3H), 2.08 (s, 3H), 1.49 (d, *J =* 6.6 Hz, 6H). MS (ESI) m/e [M+1]⁺ 444. |
| M28 | 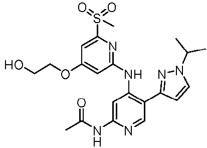 | N-(4-((4-(2-hydroxyethoxy)-6-(methylsulfonyl)pyridin-2-yl)amino)-5-(1-isopropyl-1H-pyrazol-3-yl)pyridin-2-yl)acetamide | ¹H NMR (400 MHz, DMSO-*d₆*) δ 10.54 (s, 1H), 10.38 (s, 1H), 8.58 (s, 1H), 7.90 - 7.80 (m, 1H), 7.40 (s, 1H), 7.19 (s, 1H), 7.14 (s, 1H), 6.95 - 6.88 (m, 1H), 4.95 (s, 1H), 4.70 - 4.60 (m, 1H), 4.20 - 4.15 (m, 2H), 3.80 - 3.70 (m, 2H), 3.29 (s, 3H), 2.08 (s, 3H), 1.49 (d, *J =* 6.5 Hz, 6H). MS (ESI) m/e [M+1]⁺ 475. |
| M29 | 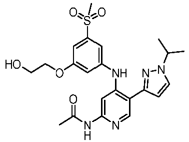 | N-(4-((3-(2-hydroxyethoxy)-5-(methylsulfonyl)phenyl) amino)-5-(1-isopropyl-1H-pyrazol-3-yl)pyridin-2-yl)acetamide | ¹H NMR (400 MHz, DMSO-*d₆*) δ10.54 (s, 1H), 10.38 (s, 1H), 8.58 (s, 1H), 8.25 - 8.20 (m, 1H), 7.95 (s, 1H), 7.40 (s, 1H), 7.19 - 7.14 (m, 2H), 6.89 (s, 1H), 5.02 - 4.83 (m, 1H), 4.65 - 4.63 (m, 1H), 4.13 - 4.10 (m, 2H), 3.76 - 3.74 (m, 2H), 3.29 (s, 3H), 2.08 (s, 3H), 1.49 (d, *J =* 6.5 Hz, 6H). MS (ESI) m/e [M+1]⁺ 474. |
| M30 | 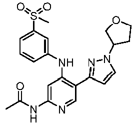 | N-(4-((3-(methylsulfonyl)phenyl) amino)-5-(1-(tetrahydrofuran-3-yl)-1H-pyrazol-3-yl)pyridin-2-yl)acetamide | MS (ESI) m/e [M+1]⁺ 442. |
| M31 | 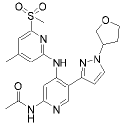 | N-(4-((4-methyl-6-(methylsulfonyl)pyridin-2-yl)amino)-5-(1-(tetrahydrofuran-3-yl)-1H-pyrazol-3-yl)pyridin-2-yl)acetamide | MS (ESI) m/e [M+1]⁺ 457. |
| M32 | 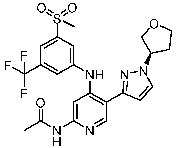 | (R)-N-(4-((3-(methylsulfonyl)-5-(trifluoromethyl)phenyl) amino)-5-(1-(tetrahydrofuran-3-yl)-1H-pyrazol-3-yl)pyridin-2-yl)acetamide | MS (ESI) m/e [M+1]⁺ 510. |
| M33 | 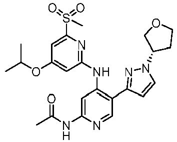 | (S)-N-(4-((4-isopropoxy-6-(methylsulfonyl)pyridin-2-yl)amino)-5-(1-(tetrahydrofuran-3-yl)-1H-pyrazol-3-yl)pyridin-2-yl)acetamide | MS (ESI) m/e [M+1]⁺ 501*.* |
| M34 | 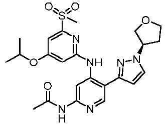 | (R)-N-(4-((4-isopropoxy-6-(methylsulfonyl)pyridin-2-yl)amino)-5-(1-(tetrahydrofuran-3-yl)-1H-pyrazol-3-yl)pyridin-2-yl)acetamide Molecular Weight: 501 | MS (ESI) m/e [M+1]⁺ 502. |
| M35 | 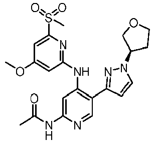 | (R)-N-(4-((4-methoxy-6-(methylsulfonyl)pyridin-2-yl)amino)-5-(1-(tetrahydrofuran-3-yl)-1H-pyrazol-3-yl)pyridin-2-yl)acetamide | MS (ESI) m/e [M+1]⁺ 473. |

### Example N1: Synthesis of N-(5-(1,5-dimethyl-1H-pyrazol-3-yl)-4-((6-(methylsulfonyl)pyridin-2-yl)amino)pyridin-2-yl)acetamide

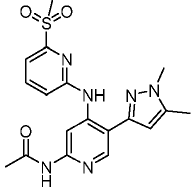

### Step 1: tert-butyl (2-chloro-5-(1,5-dimethyl-1H-pyrazol-3-yl)pyridin-4-yl)carbamate

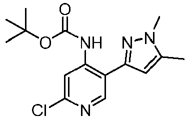

A mixture of tert-butyl (5-bromo-2-chloropyridin-4-yl)carbamate (5.0 g, 16.3 mmol), 1,5-dimethyl-3-(4,4,5,5-tetramethyl-1,3,2-dioxaborolan-2-yl)-1H-pyrazole (3.58 g, 16.28 mmol), Pd(PPh₃)₂Cl₂ (595 mg, 0.81 mmol), H₂O (1.0 mL) in dioxane (100 mL) was stirred at 100 °C for 4 h under nitrogen atmosphere. After cooled to room temperature, the mixture was filtrated and the filtration was concentrated under vacuum to give the residue, then purified by silica gel column chromatography using EA / PE (0 - 20 % ) to give tert-butyl (2-chloro-5-(1,5-dimethyl-1H-pyrazol-3-yl)pyridin-4-yl)carbamate (3.5 g, 67.3 %). MS (ESI) m/e [M+1]⁺ 323.

### Step 2: tert-butyl (2-acetamido-5-(1,5-dimethyl-1H-pyrazol-3-yl)pyridin-4-yl)carbamate

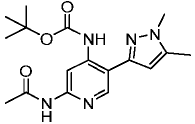

A mixture of tert-butyl (2-chloro-5-(1,5-dimethyl-1H-pyrazol-3-yl)pyridin-4-yl)carbamate (3.0 g, 9.23 mmol), acetamide (1.08 g, 18.5 mmol), Cs₂CO₃ (6.0 g, 18.5 mmol), Xant-Phos (1.06 g, 1.85 mmol) and Pd₂(dba)₃ (875 mg, 0.0.92 mmol) in dioxane (100 mL) was stirred at 110 °C for 12 h under nitrogen atmosphere. After cooled to room temperature, the solvent was removed in vacuo and the residue was diluted with 20 mL of water. The resulting solution was extracted with EA (30 mL x 3) and the combined organic layers were washed with 50 mL of brine, dried over anhydrous sodium sulfate. The solvent was removed in vacuo and the residue was purified by silica gel column chromatography using PE / EA ( 20 - 50 %) to give tert-butyl (2-acetamido-5-(1,5-dimethyl-1H-pyrazol-3-yl)pyridin-4-yl)carbamate (2.2 g, 69.2 %). MS (ESI) m/e [M+1]⁺ 345.

### Step 3: N-(4-amino-5-(1,5-dimethyl-1H-pyrazol-3-yl)pyridin-2-yl)acetamide

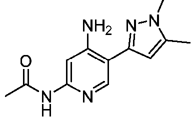

A mixture of tert-butyl (2-acetamido-5-(1,5-dimethyl-1H-pyrazol-3-yl)pyridin-4-yl)carbamate (2.2 g, 6.38 mmol) in TFA (20 mL) and DCM (10 mL) was stirred at room temperature for 16 h. Upon completion of the reaction, the solvent was removed in vacuo and the residue diluted with water. NaHCO₃ (40 mL) was added to adjust the pH value to 9 and the resulting solution was extracted with EA (10 mL x 3). The combined organic layers were dried over anhydrous Na₂SO₄ and concentrated in vacuo to give N-(4-amino-5-(1,5-dimethyl-1H-pyrazol-3-yl)pyridin-2-yl)acetamide (1.2 g, 76.9 %). MS (ESI) m/e [M+1]⁺ 246.

### Step 4: N-(5-(1,5-dimethyl-1H-pyrazol-3-yl)-4-((6-(methylsulfonyl)pyridin-2-yl)amino)pyridin-2-yl) acetamide

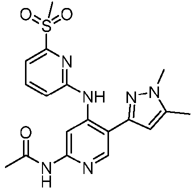

A mixture of N-(4-amino-5-(1,5-dimethyl-1H-pyrazol-3-yl)pyridin-2-yl)acetamide (50 mg, 0.20 mmol), 2-bromo-6-(methylsulfonyl)pyridine (58 mg, 0.24 mmol), Pd₂(dba)₃ (19 mg, 0.020 mmol), BINAP ( 13mg, 0.020 mmol) and Cs₂CO₃ (200 mg, 0.61 mmol) in dioxane (5 mL) was stirred for 16 h at 120 °C under nitrogen atmosphere. The mixture was allowed to cool down to room temperature. The resulting mixture was filtered. The filtrate was concentrated under reduced pressure. The residue was purified by combi-flash (MeOH/DCM = 0- 10%) to give the crude product. The crude product was suspended in acetonitrile to give N-(5-(1,5-dimethyl-1H-pyrazol-3-yl)-4-((6-(methylsulfonyl)pyridin-2-yl)amino)pyridin-2-yl)acetamide (7.66 mg, 9.6 %). ¹H NMR (400 MHz, DMSO-*d₆*) δ 11.66 (s, 1H), 10.42 (s, 1H), 9.26 (s, 1H), 8.57 (s, 1H), 8.07 - 7.89 (m, 1H), 7.55 *(d, J =* 7.2 Hz, 1H), 7.36 (d, *J =* 8.4 Hz, 1H), 6.71 (s, 1H), 3.89 (s, 3H), 3.48 (s, 3H), 2.32 (s, 3H), 2.09 (s, 3H). MS (ESI) m/e [M+1]⁺ 401.

The following Examples were prepared in a similar manner to the product Example N1:

| **Example** | **Compound** | **Chemical Name** | **¹H NMR and LC /MS *m*/*z* (M+1)** |
|---|---|---|---|
| N2 | 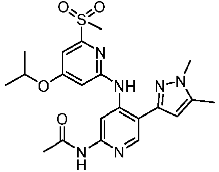 | N-(5-(1,5-dimethyl-1H-pyrazol-3-yl)-4-((4-isopropoxy-6-(methylsulfonyl)pyrid in-2-yl)amino)pyridin-2-yl)acetamide | ¹H NMR (400 MHz, DMSO-*d₆*) δ 11.39 (s, 1H), 10.43 (s, 1H), 9.15 (s, 1H), 8.56 (s, 1H), 7.08 (s, 1H), 6.77 (s, 1H), 6.71 (s, 1H), 4.95 - 4.90 (m, 1H), 3.90 (s, 3H), 3.46 (s, 3H), 2.33 (s, 3H), 2.10 (s, 3H), 1.34 (d, *J =* 5.7 Hz, 6H). MS (ESI) m/e [M+1]⁺ 459. |
| N3 | 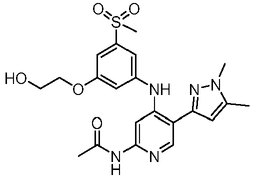 | N-(5-(1,5-dimethyl-1H-pyrazol-3-yl)-4-((3-(2-hydroxyethoxy)-5-(methylsulfonyl)phen yl)amino)pyridin-2-yl)acetamide | ¹H NMR (400 MHz, DMSO-*d₆*) δ 10.51 (s, 1H), 10.39 (s, 1H), 8.49 (s, 1H), 8.10 (s, 1H), 7.41 (s, 1H), 7.22 (s, H), 7.14 (s, H), 6.66 (s, 1H), 4.95 (s, 1H), 4.20 - 4.10 (m, 2H), 3.84 (s, 3H), 3.80 - 3.70 (m, 2H), 3.30 (s, 3H), 2.32 (s, 3H), 2.07 (s, 3H). MS (ESI) m/e [M+1]⁺ 460. |
| N4 | 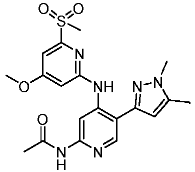 | N-(5-(1,5-dimethyl-1H-pyrazol-3-yl)-4-((4-methoxy-6-(methylsulfonyl)pyrid in-2-yl)amino)pyridin-2-yl)acetamide | ¹H NMR (400 MHz, DMSO-*d₆*) δ 11.49 (s, 1H), 10.46 (s, 1H), 9.22 (s, 1H), 8.61 (s, 1H), 7.18 (s, 1H), 6.85 (s, 1H), 6.76 (s, 1H), 4.02 (s, 3H), 3.96 (s, 3H), 3.52 (s, 3H), 2.39 (s, 3H), 2.16 (s, 3H). MS (ESI) m/e [M+1]⁺ 431. |
| N5 | 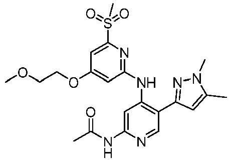 | N-(5-(1,5-dimethyl-1H-pyrazol-3-yl)-4-((4-(2-methoxyethoxy)-6-(methylsulfonyl)pyrid in-2-yl)amino)pyridin-2-yl)acetamide | ¹H NMR (400 MHz, DMSO-*d₆*) δ 11.48 (s, 1H), 10.46 (s, 1H), 9.23 (s, 1H), 8.61 (s, 1H), 7.19 (s, 1H), 6.86 (s, 1H), 6.76 (s, 1H), 4.45 - 4.40 (m, 2H), 3.97 (s, 3H), 3.80 - 3.75 (m, 2H), 3.63 (s, 3H), 3.52 (s, 3H), 2.39 (s, 3H), 2.16 (s, 3H). MS (ESI) m/e [M+1]⁺ 475. |
| N6 | 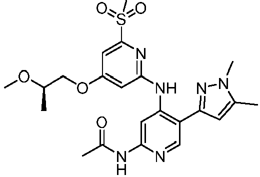 | (R)-N-(5-(1,5-dimethyl-1H-pyrazol-3-yl)-4-((4-(2-methoxypropoxy)-6-(methylsulfonyl)pyrid in-2-yl)amino)pyridin-2-yl)acetamide | ¹H NMR (400 MHz, DMSO-*d₆*) δ 11.40 (s, 1H), 10.42 (s, 1H), 9.17 (s, 1H), 8.56 (s, 1H), 7.14 (s, 1H), 6.83 (s, 1H), 6.71 (s, 1H), 4.27 - 4.13 (m, 2H), 3.91 (s, 3H), 3.72 - 3.71 (m, 1H), 3.46 (s, 3H), 3.34 (s, 3H), 2.34 (s, 3H), 2.10 (s, 3H), 1.20 (d, *J =* 6.2 Hz, 3H). MS (ESI) m/e [M+1]⁺ 489. |
| N7 | 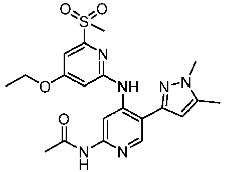 | N-(5-(1,5-dimethyl-1H-pyrazol-3-yl)-4-((4-ethoxy-6-(methylsulfonyl)pyrid in-2-yl)amino)pyridin-2-yl)acetamide | ¹H NMR (400 MHz, DMSO-*d₆*) δ 11.41 (s, 1H), 10.39 (s, 1H), 9.16 (s, 1H), 8.55 (s, 1H), 7.15 (s, 1H), 6.77 (s, 1H), 6.70 (s, 1H), 4.27 (q, *J* = 6.9 Hz, 2H), 3.57 (s, 3H), 3.46 (s, 3H), 2.33 (s, 3H), 2.10 (s, 3H), 1.38 (t, *J =* 6.9 Hz, 3H). MS (ESI) m/e [M+1]⁺ 445. |

### Example O1: Synthesis of N-(5-(1-methyl-5-morpholino-1H-pyrazol-3-yl)-4-((6-(methylsulfonyl)pyridin-2-yl)amino)pyridin-2-yl)acetamide

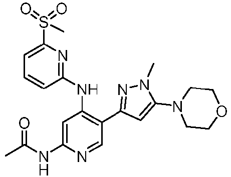

### Step 1: ethyl 3,5-dibromo-1H-pyrazole-4-carboxylate

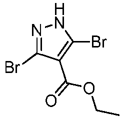

To a solution of ethyl 1H-pyrazole-4-carboxylate (10.0 g, 71.4 mmol) in EtOH (200 mL) was added NaOAc (41.0 g, 500 mmol) in H₂O (300 mL) and then added Br₂ (45.6 g, 285 mmol) at 0 °C and the resulting mixture was stirred for 5 h at room temperature under nitrogen atmosphere. Upon completion of the reaction, the mixture was poured into water (100 mL) and extracted with EA (300 mL x 2). The combined organic layer was washed with brine, dried over anhydrous Na₂SO₄, filtered and concentrated to give the residue, and purified by silica gel column chromatography (PE / EA = 100/1 to 0/1) to give ethyl 3,5-dibromo-1H-pyrazole-4-carboxylate (10.0 g, 47% yield). MS(ESI) m/e [M+1]⁺ 298.

### Step 2: ethyl 3,5-dibromo-1-methyl-1H-pyrazole-4-carboxylate

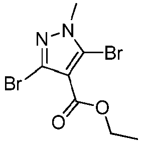

To a solution of ethyl 3,5-dibromo-1H-pyrazole-4-carboxylate (10.0 g, 33.6 mmol) in THF (100 mL) was added NaH (60% in mineral oil, 2.0 g, 50 mmol) at 0 °C and the resulting mixture was stirred at this temperature for 30 min, then MeI (5.7 g, 40.0 mmol) was added at 0 °C and the resulting mixture was stirred for 6 h at room temperature under nitrogen atmosphere. Upon completion of the reaction, the mixture was poured into water (50 mL) and the resulting mixture was extracted with EA (60 mL x 2). The combined organic phase was washed with brine, dried over Na₂SO₄, concentrated under vacuum. The residue was purified by silica gel column chromatography (PE/EA = 50/1 to 0/1) to give ethyl 3,5-dibromo-1-methyl-1H-pyrazole-4-carboxylate (5.1 g, 51%). MS(ESI) m/e [M+1] 312.

### Step 3: ethyl 3-bromo-1-methyl-5-morpholino-1H-pyrazole-4-carboxylate

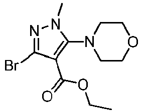

To a solution of ethyl 3,5-dibromo-1-methyl-1H-pyrazole-4-carboxylate (4.0 g, 12.8 mmol) in NMP (50 mL) were added morpholine (1.7 g, 19.2 mmol) and K₂CO₃ (4.4 g, 32.0 mmol), the resulting mixture was heated to 130 °C stirred for 25 h under nitrogen atmosphere. After cooled to room temperature, the solvent was removed and the residue was diluted with water (20 mL) then extracted with EA (30 mL x 3), the combined organic layers were washed with brine (50 mL), dried over anhydrous sodium sulfate. The solvent was removed and the residue was purified by silica gel column chromatography (PE/EA = 100/1 to 0/1) to give ethyl 3-bromo-1-methyl-5-morpholino-1H-pyrazole-4-carboxylate (2.5 g, 62%). ¹H NMR (CDCl₃) δ 4.40 - 4.35 (m, 2H), 3.79 - 3.75 (m, 4H), 3.73 (s, 3H), 3.15 - 3.10 (m, 4H), 1.40 (t, *J =* 7.0 Hz, 3H). MS(ESI) m/e [M+1]⁺ 318.

### Step 4: 4-(3-bromo-1-methyl-1H-pyrazol-5-yl)morpholine

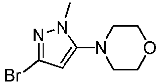

To a solution of ethyl 3-bromo-1-methyl-5-morpholino-1H-pyrazole-4-carboxylate (2.5 g, 7.9 mmol) in EtOH (20 mL) was added NaOH (1.0 g, 25.1 mmol) in water (15 mL) and the resulting mixture was stirred for 3 h at 70 °C under nitrogen atmosphere. Upon completion of the reaction, the reaction mixture was cooled down to 0 °C and H₂SO₄ (6.2 g, 62.8 mmol) was added, then the mixture was stirred for another 5 h at 70 °C under nitrogen atmosphere. After cooled to room temperature, the mixture was poured into water (10 mL) and sat Na₂CO₃ water solution was added to adjust the pH value to 8, the resulting mixture was extracted with EA (30 mL x 2). The combined organic layer was washed with brine, dried over Na₂SO₄, concentrated under vacuum. The residue was purified by silica gel column chromatography (PE/EA = 50/1 to 0/1) to give 4-(3-bromo-1-methyl-1H-pyrazol-5-yl)morpholine (1.2 mg, 62%). MS(ESI) m/e [M+1]⁺ 246.

### Step 5: (1-methyl-5-morpholino-1H-pyrazol-3-yl)boronic acid

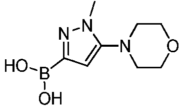

To a solution of 4-(3-bromo-1-methyl-1H-pyrazol-5-yl)morpholine (1.1 g, 4.47 mmol) in 1,4-dioxane (10 mL) was added Pin₂B₂ (1.7 g, 6.7 mmol), Pd(dppf)Cl₂.CH₂Cl₂ (365 mg, 450 umol) and AcOK (1.7 g, 17.9 mmol), the resulting mixture was heated up to 100 °C and stirred at this temperature for 5 h. After cooled to room temperature, the solvent was removed in vacuo and the residue was diluted with 20 mL of water. The resulting solution was extracted with 3 x 50 mL of EA and the combined organic layers were washed with 50 mL of brine, dried over anhydrous sodium sulfate. The solvent was removed in vacuo to give the crude product (1-methyl-5-morpholino-1H-pyrazol-3-yl)boronic acid and used directly for next step without purification. Ms (ESI) m/e [M+1]⁺ 212.

### Step 6: tert-butyl (2-chloro-5-(1-methyl-5-morpholino-1H-pyrazol-3-yl)pyridin-4-yl)carbamate

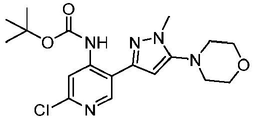

To a solution of tert-butyl (5-bromo-2-chloropyridin-4-yl)carbamate (1.5 g, 4.9 mmol) in 1,4-dioxane (10 mL) were added (1-methyl-5-morpholino-1H-pyrazol-3-yl)boronic acid (1.0 g, 4.9 mmol), Pd(dppf)Cl₂ (358.0 mg, 490 umol) and K₃PO₄ (4.1 g, 19.5 mmol), the resulting mixture was heated up to 100 °C and stirred for 12 h at this temperature under nitrogen atmosphere. After cooled to room temperature, the solvent was removed in vacuo and the residue was diluted with 20 mL of water. The resulting solution was extracted with 3 x 30 mL of EA and the combined organic layers were washed with 50 mL of brine, dried over anhydrous sodium sulfate. The solvent was removed in vacuo and the residue was purified by silica gel column chromatography (PE/EA = 100/1 to 0/1) to give tert-butyl (2-chloro-5-(1-methyl-5-morpholino-1H-pyrazol-3-yl)pyridin-4-yl)carbamate (1.0 g, 52%). ¹H NMR (CDCl₃) δ 10.95 (s, 1H) 8.46 (s, 1H) 8.38 (s, 1H) 6.19 (s, 1H) 3.89 - 3.85 (m, 4H) 3.81 (s, 3H) 3.02 - 2.96 (m, 4H) 1.56 (s, 9H). MS(ESI) m/e [M+1]⁺ 394.

### Step 7: tert-butyl (2-acetamido-5-(1-methyl-5-morpholino-1H-pyrazol-3-yl)pyridin-4-yl)carbamate

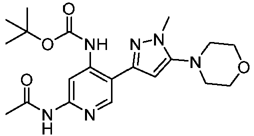

To a solution of tert-butyl (2-chloro-5-(1-methyl-5-morpholino-1H-pyrazol-3-yl)pyridin-4-yl)carbamate (1.0 g, 2.5 mmol) in 1,4-dioxane (30 mL) was added acetamide (450.0 mg, 7.6 mmol), Pd₂(dba)₃ (228.0 mg, 250.0 umol), Xantphos (144.0 mg, 250.0 umol) and Cs₂CO₃ (2.5 g, 7.6 mmol). The resulting mixture was heated up to 110 °C and stirred for 10 h at this temperature under nitrogen atmosphere. After cooled to room temperature, the solvent was removed in vacuo and the residue was diluted with 20 mL of water. The resulting solution was extracted with 3 x 20 mL of EA and the combined organic layers were washed with 50 mL of brine, dried over anhydrous sodium sulfate. The solvent was removed in vacuo and the residue was purified by silica gel column chromatography (PE / EA = 100/1 to 0/1) to give tert-butyl (2-acetamido-5-(1-methyl-5-morpholino-1H-pyrazol-3-yl)pyridin-4-yl)carbamate (750 mg, 70% yield). MS(ESI) m/e [M+1]⁺ 417.

### Step 8: N-(4-amino-5-(1-methyl-5-morpholino-1H-pyrazol-3-yl)pyridin-2-yl)acetamide

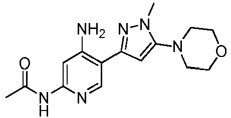

To a solution of tert-butyl (2-acetamido-5-(1-methyl-5-morpholino-1H-pyrazol-3-yl)pyridin-4-yl)carbamate (750 mg, 1.8 mmol) in DCM (2 mL) was added TFA (2 mL) and the resulting mixture was stirred at 40 °C for 5 h. After cooled to room temperature, the solvent was removed in vacuo and the residue was diluted with 20 mL of water. Then aqueous Na₂CO₃ was added to adjust the pH value to 8 and the resulting mixture was extracted with EA (10 mL x 3). The combined organic layer was washed with brine (5 mL), dried over Na₂SO₄, filtered and concentrated under vacuum. The residue was purified by silica gel column chromatography (PE: EA = 100/1 to 0/1) to give N-(4-amino-5-(1-methyl-5-morpholino-1H-pyrazol-3-yl)pyridin-2-yl)acetamide (480 mg, 80% yield). ¹H NMR (DMSO-*d₆*) δ 10.06 (s, 1H), 8.25 (s, 1H), 7.45 (s, 1H), 7.13 (s, 2H), 6.38 (s, 1H), 3.78 - 3.73 (m, 4H), 3.68 (s, 3H), 2.95 - 2.90 (m, 4H), 2.04 (s, 3H). MS(ESI) m/e [M+1]⁺ 317.

### Step 9: N-(5-(1-methyl-5-morpholino-1H-pyrazol-3-yl)-4-((6-(methylsulfonyl)pyridin-2-yl)amino)pyridin-2-yl)acetamide

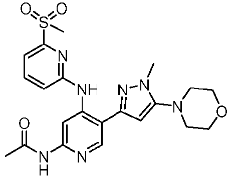

To a mixture of N-(4-amino-5-(1-methyl-5-morpholino-1H-pyrazol-3-yl)pyridin-2-yl)acetamide (100 mg, 0.32 mmol), 2-bromo-6-(methylsulfonyl)pyridine (90 mg, 0.38 mmol), Pd₂dba₃ (30 mg, 0.032 mmol), BINAP (40 mg, 0.064 mmol) and Cs₂CO₃ (205 mg, 0.64 mmol) in dioxane (10 mL) was stirred at 100 °C for 4 h. The mixture was filtrated and the filtrate as concentrated to give the residue and purified by Prep-TLC (MeOH/DCM = 1: 15) to afford N-(5-(1-methyl-5-morpholino-1H-pyrazol-3-yl)-4-((6-(methylsulfonyl)pyri dine-2-yl)amino)pyridin-2-yl)acetamide (75 mg, 52.8% yield). ¹H NMR (400 MHz, DMSO-*d*₆) δ 11.71 (s, 1H), 10.47 (s, 1H), 9.28 (s, 1H), 8.63 (s, 1H), 8.05 - 8.001 (m, 1H), 7.57 (d, *J =* 7.5 Hz, 1H), 7.36 (d, *J* = 8.6 Hz, 1H), 6.63 (s, 1H), 3.84 (s, 3H), 3.79 - 3.77 (m, 4H), 3.50 (s, 3H), 2.99 - 2.96 (m, 4H), 2.11 (s, 3H). MS (ESI) m/e [M+1]⁺ 472.

The following Examples were prepared in a similar manner to the product Example O1:

| **Example** | **Compound** | **Chemical Name** | **¹H NMR and LC /MS *m*/*z* (M+1)** |
|---|---|---|---|
| O2 | 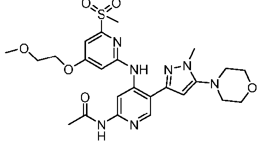 | N-(4-((4-(2-methoxyethoxy)-6-(methylsulfonyl)pyridin-2-yl)amino)-5-(1-methyl-5-morpholino-1H-pyrazol-3-yl)pyridin-2-yl)acetamide | ¹H NMR (400 MHz, DMSO-*d₆*) δ 11.46 (s, 1H), 10.45 (s, 1H), 9.18 (s, 1H), 8.60 (s, 1H), 7.14 (s, 1H), 6.80 (s, 1H), 6.61 (s, 1H), 4.35 - 4.33 (m, 2H), 3.84 (s, 3H), 3.77 - 3.75 (m, 4H), 3.71 - 3.69 (m, 2H), 3.46 (s, 3H), 3.34 (s, 3H), 2.96 - 2.94 (m, 4H), 2.10 (s, 3H). MS (ESI) m/e [M+1]⁺ 546. |
| O3 | 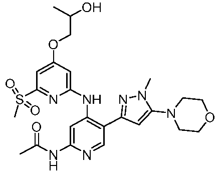 | N-(4-((4-(2-hydroxypropoxy)-6-(methylsulfonyl)pyridin-2-yl)amino)-5-(1-methyl-5-morpholino-1H-pyrazol-3-yl)pyridin-2-yl)acetamide | ¹H NMR (400 MHz, DMSO-*d*₆) δ 11.43 (s, 1H), 10.46 (s, 1H), 9.15 (s, 1H), 8.58 (s, 1H), 7.12 (s, 1H), 6.78 (s, 1H), 6.59 (s, 1H), 4.06 - 4.00 (m, 3H), 3.85 - 3.81 (m, 3H), 3.76 - 3.74 (m, 4H), 3.44 (s, 3H), 2.95 - 2.93 (m, 4H), 2.08 (s, 3H), 1.26 - 1.14 (m, 2H). MS (ESI) m/e [M+1]⁺ 546. |

### Example Q1: Synthesis of N-(5-(2,3-dihydro-[1,4]dioxino[2,3-b]pyridin-6-yl)-4-((4-isopropoxy-6-(methylsulfonyl)pyridine-2-yl)amino)pyridin-2-yl)acetamide

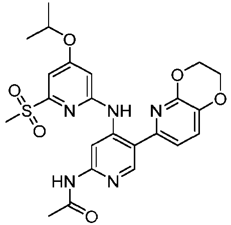

### Step 1: 2-chloro-5-(2,3-dihydro-[1,4]dioxino[2,3-b]pyridin-6-yl)pyridin-4-amine

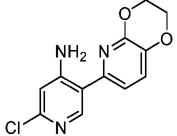

A mixture of 6-bromo-2,3-dihydro-[1,4]dioxino[2,3-b]pyridine (1 g, 4.63 mmol), 2-chloro-5-(4,4,5,5-tetramethyl-1,3,2-dioxaborolan-2-yl)pyridin-4-amine (1.29 g, 5.10 mmol), Pd(dppf)Cl₂ (338 mg, 0.46 mmol) and K₂CO₃ (958 mg, 6.95 mmol) in 1,4-dioxane (20 mL) and H₂O (2 mL) was charged with nitrogen and heated to 100 °C stirred for 2 h. The reaction was cooled to room temperature and diluted with EA, washed with brine, dried and concentrated. The residue was applied onto a silica gel column with MeOH/DCM (1 : 100) to afford 2-chloro-5-(2,3-dihydro-[1,4]dioxino[2,3-b]pyridin-6-yl)pyridin-4-amine (1.41 g). MS (ESI) m/e [M+1]⁺ 264.

### Step 2: N-(4-amino-5-(2,3-dihydro-[1,4]dioxino[2,3-b]pyridin-6-yl)pyridin-2-yl)acetamide

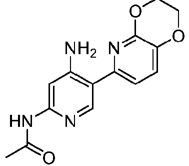

A mixture of 2-chloro-5-(2,3-dihydro-[1,4]dioxino[2,3-b]pyridin-6-yl)pyridin-4-amine (528 mg, 2 mmol), acetamide (710 mg, 12 mmol), Pd₂dba₃ (180 mg, 0.2 mmol), Xantphos (230 mg, 0.4 mmol) and Cs₂CO₃ (1.3 g, 4 mmol) in 1,4-dioxane (10 mL) was heated to 130 °C in a sealed tube stirring overnight under nitrogen atmosphere. The reaction was cooled to room temperature, filtered and the filtration was concentrated under vacuum. The residue was applied onto a silica gel column with DCM/MeOH (100:1) to afford crude N-(4-amino-5-(2,3-dihydro-[1,4]dioxino[2,3-b]pyridin-6-yl)pyridin-2-yl)acetamide (575 mg). MS (ESI) m/e [M+1]⁺ 287.

### Step 3: N-(5-(2,3-dihydro-[1,4]dioxino[2,3-b]pyridin-6-yl)-4-((4-isopropoxy-6-(methylsulfonyl)pyri din-2-yl)amino)pyridin-2-yl)acetamide

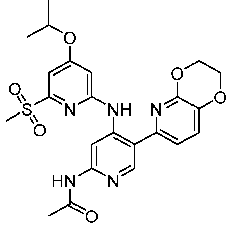

A mixture of N-(4-amino-5-(2,3-dihydro-[1,4]dioxino[2,3-b]pyridin-6-yl)pyridin-2-yl)acetamide (50 mg, 0.17 mmol), 2-bromo-4-isopropoxy-6-(methylsulfonyl)pyridine (77 mg, 0.26 mmol), Pd₂dba₃ (16 mg, 0.017 mmol), BINAP (21 mg, 0.034 mmol) and Cs₂CO₃ (111 mg, 0.34 mmol) in1,4-dioxane (6 mL) was heated to 130 °C in a sealed tube stirring for 5 h under nitrogen atmosphere. The reaction was cooled to room temperature, filtered and the filtration was concentrated under vacuum. The residue was applied onto Prep-TLC with DCM/MeOH (20 : 1) to afford N-(5-(2,3-dihydro-[1,4]dioxino[2,3-b]pyridin-6-yl)-4-((4-isopropoxy-6-(methyl sulfonyl)pyridin-2-yl)amino)pyridin-2-yl)acetamide (17 mg, 20%). ¹H NMR (400 MHz, DMSO-*d*₆) δ 11.83 (s, 1H), 10.49 (s, 1H), 9.03 (s, 1H), 8.61 (s, 1H), 7.58 (d, *J* = 8.0 Hz, 1H), 7.48 (d, *J =* 8.0 Hz, 1H), 7.08 (s, 1H), 6.59 (s, 1H), 4.85 - 4.80 (m, 1H), 4.55 - 4.52 (m, 2H), 4.36 - 4.32 (m, 2H), 3.42 (s, 3H), 2.11 (s, 3H), 1.34 (d, *J* = 4.9 Hz, 6H). MS (ESI) m/e [M+1]⁺ 500.

### Example Q21: Synthesis of compound N-(5-(2,2-dimethyl-2,3-dihydro-[1,4]dioxino[2,3-b]pyridin-6-yl)-4-((4-methyl-6-(methylsulfonyl)pyridin-2-yl)amino)pyridin-2-yl)acetamide

### Method A

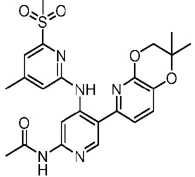

### Step 1: 1-(2,6-dibromopyridin-3-yloxy)-2-methylpropan-2-ol

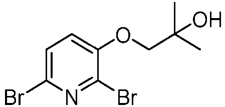

Into a 100-mL round-bottom flask, were placed 2,6-dibromopyridin-3-ol (2.00 g, 7.90 mmol), DMF (30 mL), K₂CO₃ (3.28 g, 23.73 mmol), 2,2-dimethyloxirane (0.68 g, 9.43 mmol). The resulting solution was stirred for 5 hr at 100 °C in an oil bath. After cooled to room temperature, the resulting solution was diluted with 50 mL of H₂O, extracted with 3 x 30 mL of ethyl acetate and the organic layers were combined and concentrated. The residue was purified by combi-flash (EtOAc/PE = 1: 4) to give the product (2.2 g, 85% yield). LCMS (ESI, m/z) [M+1]⁺ 324.

### Step 2: 6-bromo-2,2-dimethyl-2,3-dihydro-[1,4]dioxino[2,3-b]pyridine

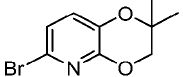

Into a 100-mL round-bottom flask, were placed 1-[(2,6-dibromopyridin-3-yl)oxy]-2-methylpropan-2-ol (2.00 g, 6.15 mmol), DMF (30 mL). This was followed by the addition of NaH (0.49 g, 60% in mineral oil, 12.30 mmol) in portions at 0 °C. The resulting solution was stirred for 3 hr at 90 °C in an oil bath. After cooled to room temperature, the reaction was then quenched by the addition of 50 mL of NH₄Cl (aq). The resulting solution was extracted with 3 x 30 mL of ethyl acetate and the organic layers were combined and concentrated. The residue was purified by combi-flash (EtOAc/PE = 1: 6) to give the product (1.06 g, 71% yield).

¹H NMR (400 MHz, CD₃Cl) δ 7.06-7.02 (m, 2H), 4.08 (s, 2H), 1.38 (s, 6H). LCMS (ESI, m/z) [M+1]⁺ 244, 246.

### Step 3: 2-chloro-5-(2,2-dimethyl-2,3-dihydro-[1,4]dioxino[2,3-b]pyridin-6-yl)pyridin-4-amine

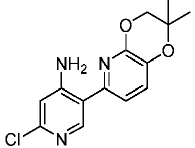

A solution of 2-chloro-5-(4,4,5,5-tetramethyl-1,3,2-dioxaborolan-2-yl)pyridin-4-amine (626.8 mg, 2.46 mmol), 6-bromo-2,2-dimethyl-2,3-dihydro-[1,4]dioxino[2,3-b]pyridine (500 mg, 2.05 mmol), Pd (dppf)Cl₂ (300 mg, 0.41 mmol), K3PO4 (869.2 mg, 4.1 mmol) in dioxane (8 mL) and H₂O (2 mL) was stirred at 75 °C for 2 hours. The mixture was cooled to rt and extracted between EA and H₂O. The organic layer was concentrated. The crude product was purified by silica gel column chromatography (PE/EA = 1:1) to give the desired product (440 mg, 73.57%). MS (ESI) m/e [M+1]⁺ 292.

### Step 4: N-(4-amino-5-(2,2-dimethyl-2,3-dihydro-[1,4]dioxino[2,3-b]pyridin-6-yl)pyridin-2-yl)acetamide

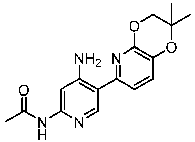

A solution of 2-chloro-5-(2,2-dimethyl-2,3-dihydro-[1,4]dioxino[2,3-b]pyridin-6-yl)pyridin-4-amine (440 mg, 1.51 mmol), acetamide (445.4 mg, 7.54 mmol), Pd₂(dba)₃ (276.6 mg, 0.3 mmol), Xant-phos (349.5 mg, 0.6 mmol) and Cs₂CO₃ (984.5 mg, 3 mmol) in dioxane (20 mL) was stirred at 130°C for 5 hours. The mixture was cooled to r.t and filtered through celite. The filtrate was concentrated under reduced pressure. The crude product was purified by silica gel column chromatography (DCM/MeOH = 20: 1) to give the desired product (305 mg, 64.26%). MS (ESI) m/e [M+1]⁺ 315.

### Step 5: N-(5-(2,2-dimethyl-2,3-dihydro-[1,4]dioxino[2,3-b]pyridin-6-yl)-4-((4-methyl-6-(methylsulfonyl)pyridin-2-yl)amino)pyridin-2-yl)acetamide

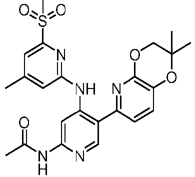

A solution of N-(4-amino-5-(2,2-dimethyl-2,3-dihydro-[1,4]dioxino[2,3-b]pyridin-6-yl)pyridin-2-yl)acetamide (50 mg, 0.16 mmol), 2-bromo-4-methyl-6-(methylsulfonyl)pyridine (48 mg, 0.19 mmol), Pd₂(dba)₃ (29.3 mg, 0.032 mmol), Xant-phos (37 mg, 0.064 mmol) and Cs₂CO₃ (104.3 mg, 0.32 mmol) in dioxane (3 mL) was stirred at 130 °C for 5 hours. The mixture was cooled to rt and the solid was removed by filtration. The filtrate was concentrated under reduced pressure. The crude product was purified by silica gel column chromatography (DCM/MeOH = 15: 1) to give the desired product (22.37 mg, 28.91%). ¹H NMR (400 MHz, DMSO-*d*₆) δ 11.97 (s, 1H), 10.47 (s, 1H), 9.11 (s, 1H), 8.63 (s, 1H), 7.61 (d, *J=* 8.3 Hz, 1H), 7.46 - 7.41 (m, 2H), 6.97 (s, 1H), 4.26 (s, 2H), 3.43 (s, 3H), 2.42 (s, 3H), 2.11 (s, 3H), 1.37 (s, 6H). MS (ESI) m/e [M+1]⁺ 484.

### Method B

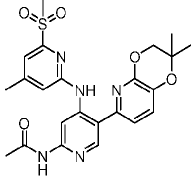

### Step 1: 1-(2,6-dibromopyridin-3-yloxy)-2-methylpropan-2-ol

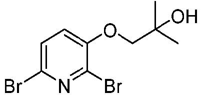

Into a 100-mL round-bottom flask, were placed 2,6-dibromopyridin-3-ol (2.00 g, 7.90 mmol), DMF (30 mL), K₂CO₃ (3.28 g, 23.73 mmol), 2,2-dimethyloxirane (0.68 g, 9.43 mmol). The resulting solution was stirred for 5 hr at 100 °C in an oil bath. After cooled to room temperature, the resulting solution was diluted with 50 mL of H₂O, extracted with 3 x 30 mL of ethyl acetate and the organic layers were combined and concentrated. The residue was purified by combi-flash (EtOAc/PE = 1/4) to give the product (2.2 g, 85% yield). LCMS (ESI, m/z) [M+1]⁺ 324.

### Step 2: 6-bromo-3,3-dimethyl-2,3-dihydro-[1,4]dioxino[2,3-b]pyridine and 6-bromo-2,2-dimethyl-2,3-dihydro-[1,4]dioxino[2,3-b]pyridine

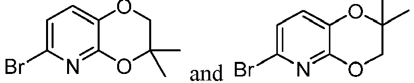

Into a 100-mL round-bottom flask purged and maintained with an inert atmosphere of nitrogen, were placed 1-[(2,6-dibromopyridin-3-yl)oxy]-2-methylpropan-2-ol (1.00 g, 3.07 mmol) and THF (15 mL). This was followed by the addition of NaH (186 mg, 4.58 mmol, 60% wt) in portions at 0 °C. The resulting solution was stirred for 16 hr at 55 °C. After cooled to room temperature, 20 mL of NH₄Cl (aq) was added to quench the reaction and the resulting solution was extracted with 3 x 10 mL of ethyl acetate and the organic layers were combined and concentrated. The residue was purified by combi-flash (EtOAc/PE = 0-35%) to give the product.

The mixture was separated by Chiral-Prep-HPLC with the following conditions, to obtain two resulted compounds: Column: CHIRAL ART Cellulose-SB, 3 x 25 cm, 5 um; Mobile Phase A: CO₂, Mobile Phase B: MeOH (0.1% 2M NH₃-MeOH); Flow rate: 60 mL/min; Gradient:15% B; Column Temperature: 35 °C; Back Pressure: 100 bar; 220 nm;. This resulted in 60 mg of 6-bromo-3,3-dimethyl-2,3-dihydro-[1,4]dioxino[2,3-b]pyridine (RT₁: 3.94 min) and 210 mg of 6-bromo-2,2-dimethyl-2,3-dihydro-[1,4]dioxino[2,3-b]pyridine (RT₂: 4.51 min).

6-Bromo-3,3-dimethyl-2,3-dihydro-[1,4]dioxino[2,3-b]pyridine: ¹H NMR (300 MHz, CD₃Cl) δ 7.06 (d, *J* = 8.0 Hz, 1H), 6.97 (d, *J* = 8.0 Hz, 1H), 3.88 (s, 2H), 1.39 (s, 6H). ¹H NMR (400 MHz, DMSO-*d*₆) δ 7.30 (d, *J* = 8.0 Hz, 1H), 7.11 (d, *J* = 8.0 Hz, 1H), 3.99 (s, 2H), 1.32 (s, 6H); LCMS (ESI, m/z): [M+H]⁺ 244, 246.

6-bromo-2,2-dimethyl-2,3-dihydro-[1,4]dioxino[2,3-b]pyridine: ¹H NMR (300 MHz, CD₃Cl) δ 7.04-7.03 (m, 2H), 4.08 (s, 2H), 1.39 (s, 6H). LCMS (ESI, m/z): [M+H]⁺ 244, 246 . And, the structure of the compound was further confirmed by single crystal structure determination.

### Step 3: 2-chloro-5-(2,2-dimethyl-2,3-dihydro-[1,4]dioxino[2,3-b]pyridin-6-yl)pyridin-4-amine

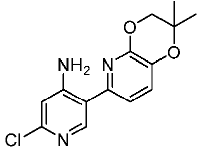

A solution of 2-chloro-5-(4,4,5,5-tetramethyl-1,3,2-dioxaborolan-2-yl)pyridin-4-amine (626.8 mg, 2.46 mmol), 6-bromo-2,2-dimethyl-2,3-dihydro-[1,4]dioxino[2,3-b]pyridine (500 mg, 2.05 mmol), Pd (dppf)Cl₂ (300 mg, 0.41 mmol), K₃PO₄ (869.2 mg, 4.1 mmol) in dioxane (8 mL) and H₂O (2 mL) was stirred at 75 °C for 2 hr. The mixture was cooled to rt and extracted between EA and H₂O. The organic layer was concentrated. The crude product was purified by silica gel column chromatography (PE/EA = 1/1) to give the desired product (440 mg, 73.57%). ¹H NMR (400 MHz, DMSO-*d*₆) δ 8.26 (s, 1H), 7.40 (d, *J* = 8.2 Hz, 2H), 7.33 (d, *J* = 12.4 Hz, 2H), 6.64 (s, 1H), 4.12 (s, 2H), 1.28 (s, 6H). MS (ESI) m/e [M+1]⁺ 292.

### Step 4: N-(4-amino-5-(2,2-dimethyl-2,3-dihydro-[1,4]dioxino[2,3-b]pyridin-6-yl)pyridin-2-yl)acetamide

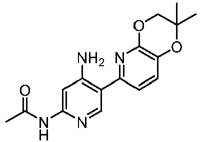

A solution of 2-chloro-5-(2,2-dimethyl-2,3-dihydro-[1,4]dioxino[2,3-b]pyridin-6-yl)pyridin-4-amine (440 mg, 1.51 mmol), acetamide (445.4 mg, 7.54 mmol), Pd₂(dba)₃ (276.6 mg, 0.3 mmol), Xant-phos (349.5 mg, 0.6 mmol) and Cs₂CO₃ (984.5 mg, 3 mmol) in dioxane (20 mL) was stirred at 130°C for 5 hours. The mixture was cooled to r.t and filtered through celite. The filtrate was concentrated under reduced pressure. The crude product was purified by silica gel column chromatography (DCM/MeOH = 20/1) to give the desired product (305 mg, 64.26%). ¹H NMR (400 MHz, DMSO-*d*₆) δ 10.07 (s, 1H), 8.26 (s, 1H), 7.48 - 7.35 (m, 2H), 7.33 - 7.17 (m, 3H), 4.11 (s, 2H), 2.01 (s, 3H), 1.28 (s, 6H). MS (ESI) m/e [M+1]⁺ 315.

### Step 5: N-(5-(2,2-dimethyl-2,3-dihydro-[1,4]dioxino[2,3-b]pyridin-6-yl)-4-((4-methyl-6-(methylsulfonyl)pyridin-2-yl)amino)pyridin-2-yl)acetamide

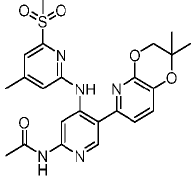

A solution of N-(4-amino-5-(2,2-dimethyl-2,3-dihydro-[1,4]dioxino[2,3-b]pyridin-6-yl)pyridin-2-yl)acetamide (50 mg, 0.16 mmol), 2-bromo-4-methyl-6-(methylsulfonyl)pyridine (48 mg, 0.19 mmol), Pd₂(dba)₃ (29.3 mg, 0.032 mmol), Xant-phos (37 mg, 0.064 mmol) and Cs₂CO₃ (104.3 mg, 0.32 mmol) in dioxane (3 mL) was stirred at 130 °C for 5 hours. The mixture was cooled to rt and the solid was removed by filtration. The filtrate was concentrated under reduced pressure. The crude product was purified by silica gel column chromatography (DCM/MeOH = 15/1) to give the desired product (22.37 mg, 28.91%). ¹H NMR (400 MHz, DMSO-*d*₆) δ 11.97 (s, 1H), 10.47 (s, 1H), 9.11 (s, 1H), 8.63 (s, 1H), 7.61 (d, *J* = 8.3 Hz, 1H), 7.46 - 7.41 (m, 2H), 6.97 (s, 1H), 4.26 (s, 2H), 3.43 (s, 3H), 2.42 (s, 3H), 2.11 (s, 3H), 1.37 (s, 6H). MS (ESI) m/e [M+1]⁺ 484.

### Example Q22: Synthesis of compound N-(5-(2,2-dimethyl-2,3-dihydro-[1,4]dioxino[2,3-b]pyridin-6-yl)-4-((6-(methylsulfonyl)-4-morpholinopyridin-2-yl)amino)pyridin-2-yl)acetamide

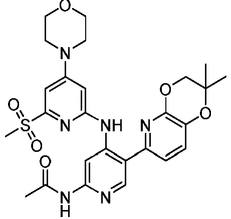

A solution of N-(4-amino-5-(2,2-dimethyl-2,3-dihydro-[1,4]dioxino[2,3-b]pyridin-6-yl)pyridin-2-yl)acetamide (600 mg, 2 mmol), 4-(2-bromo-6-(methylsulfonyl)pyridin-4-yl)morpholine (964 mg, 3 mmol), Pd₂(dba)₃ (366 mg, 0.4 mmol), Xantphos (463 mg, 0.8 mmol) and Cs₂CO₃ (1304 mg, 4 mmol) in dioxane (30 mL) was stirred at 130 °C for 5 hours. The mixture was cooled to r.t and the solid was filtered through celite. The filtrate was concentrated under reduced pressure. The crude product was purified by silica gel column chromatography (DCM/ MeOH = 15/1) to give the product (65.36 mg, 59%). ¹H NMR (400 MHz, DMSO-*d*₆) δ 11.39 (s, 1H), 10.44 (s, 1H), 8.87 (s, 1H), 8.56 (s, 1H), 7.55 (d, *J* = 8.3 Hz, 1H), 7.42 (d, *J =* 8.3 Hz, 1H), 7.10 (s, 1H), 6.52 (s, 1H), 4.24 (s, 2H), 3.77 - 3.68 (m, 4H), 3.41 - 3.36 (m, 4H), 3.35 (s, 3H), 2.09 (s, 3H), 1.36 (s, 6H). MS (ESI) m/e [M+1]⁺ 555.

### Example Q23: Synthesis of compound N-(5-(2,2-dimethyl-2,3-dihydro-[1,4]dioxino[2,3-b]pyridin-6-yl)-4-((6-(methylsulfonyl)-4-(tetrahydro-2H-pyran-4-yl)pyridin-2-yl)amino)pyridin-2-yl)acetamide

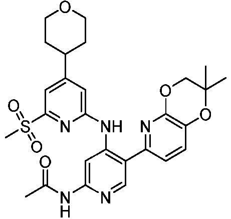

A solution of N-(4-amino-5-(2,2-dimethyl-2,3-dihydro-[1,4]dioxino[2,3-b]pyridin-6-yl)pyridin-2-yl)acetamide (60 mg, 0.2 mmol), 2-bromo-6-(methylsulfonyl)-4-(tetrahydro-2H-pyran-4-yl)pyridine (96 mg, 0.3 mmol), Pd₂(dba)₃ (36.6 mg, 0.04 mmol), Xantphos (46.3 mg, 0.08 mmol) and Cs₂CO₃ (130.4 mg, 0.4 mmol) in dioxane (3 mL) was stirred at 130 °C for 5 hr. The mixture was cooled to RT and the solid was removed by filtration. The filtrate was concentrated under reduced pressure. The crude product was purified by silica gel column chromatography (DCM/ MeOH = 15/1) to give the product (63.6 mg, 57%). ¹H NMR (400 MHz, DMSO-*d*₆) δ 11.86 (s, 1H), 10.49 (s, 1H), 9.06 (s, 1H), 8.62 (s, 1H), 7.59 (d, *J=* 8.5 Hz, 1H), 7.52 - 7.38 (m, 2H), 7.05 (s, 1H), 4.26 (s, 2H), 4.01 - 3.92 (m, 2H), 3.49 - 3.44 (m, 2H), 3.43 (s, 3H), 3.02 - 2.89 (m, 1H), 2.11 (s, 3H), 1.84 - 1.74 (m, 2H), 1.72 - 1.59 (m, 2H), 1.37 (s, 6H). MS (ESI) m/e [M+1]⁺ 554.

### Example Q24: Synthesis of compound N-(5-(2,2-dimethyl-2,3-dihydro-[1,4]dioxino[2,3-b]pyridin-6-yl)-4-((4-(methoxy-d3)-6-(methylsulfonyl)pyridin-2-yl)amino)pyridin-2-yl)acetamide

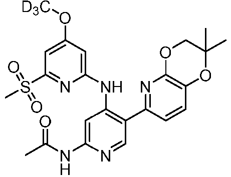

A solution of N-(4-amino-5-(2,2-dimethyl-2,3-dihydro-[1,4]dioxino[2,3-b]pyridin-6-yl)pyridin-2-yl)acetamide (60 mg, 0.2 mmol), 2-bromo-4-(methoxy-d3)-6-(methylsulfonyl)pyridine (80.7 mg, 0.3 mmol), Pd₂(dba)₃ (36.6 mg, 0.04 mmol), Xantphos (46.3 mg, 0.08 mmol) and Cs₂CO₃ (130.4 mg, 0.4 mmol) in dioxane (3 mL) was stirred at 130 °C for 5 hours. The mixture was cooled to RT and filtered through celite. The filtrate was concentrated under reduced pressure. The crude product was purified by silica gel column chromatography (DCM/MeOH = 15/1) to give the product (26.04 mg, 26 %). ¹H NMR (400 MHz, DMSO-*d*₆) δ 11.76 (s, 1H), 10.49 (s, 1H), 8.99 (s, 1H), 8.61 (s, 1H), 7.58 (d, *J* = 8.3 Hz, 1H), 7.43 (d, *J =* 8.3 Hz, 1H), 7.12 (s, 1H), 6.68 (s, 1H), 4.25 (s, 2H), 3.41 (s, 3H), 2.11 (s, 3H), 1.36 (s, 6H). MS (ESI) m/e [M+1]⁺ 503.

### Example Q25: synthesis of compound N-(5-(2,2-dimethyl-2,3-dihydro-[1,4]dioxino[2,3-b]pyridin-6-yl)-4-((4-methoxy-6-(methylsulfonyl)pyridin-2-yl)amino)pyridin-2-yl)acetamide

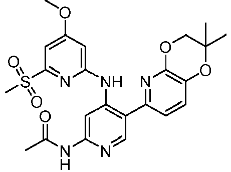

A solution of N-(4-amino-5-(2,2-dimethyl-2,3-dihydro-[1,4]dioxino[2,3-b]pyridin-6-yl)pyridin-2-yl)acetamide (60 mg, 0.2 mmol), 2-bromo-4-methoxy-6-(methylsulfonyl)pyridine (79.8 mg, 0.3 mmol), Pd₂(dba)₃ (36.6 mg, 0.04 mmol), Xant-phos (46.3 mg, 0.08 mmol) and Cs₂CO₃ (130.4 mg, 0.4 mmol) in dioxane (3 mL) was stirred at 130 °C for 5 hr. The mixture was cooled to rt and filtered through celite. The filtrate was concentrated under reduced pressure. The crude product was purified by silica gel column chromatography (DCM/MeOH = 15/1) to give the product (25.21 mg, 25%). ¹H NMR (400 MHz, DMSO-*d*₆) δ 11.77 (s, 1H), 10.49 (s, 1H), 8.99 (s, 1H), 8.61 (s, 1H), 7.58 (d, *J* = 8.4 Hz, 1H), 7.43 (d, *J =* 8.4 Hz, 1H), 7.12 (s, 1H), 6.69 (s, 1H), 4.25 (s, 2H), 3.93 (s, 3H), 3.41 (s, 3H), 2.11 (s, 3H), 1.36 (s, 6H). MS (ESI) m/e [M+1]⁺ 500.

### Example Q26: synthesis of compound N-(4-((3,4-dimethoxy-6-(methylsulfonyl)pyridin-2-yl)amino)-5-(2,2-dimethyl-2,3-dihydro-[1,4]dioxino[2,3-b]pyridin-6-yl)pyridin-2-yl)acetamide

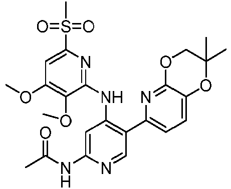

A solution of N-(4-amino-5-(2,2-dimethyl-2,3-dihydro-[1,4]dioxino[2,3-b]pyridin-6-yl)pyridin-2-yl)acetamide (60 mg, 0.2 mmol), 2-bromo-3,4-dimethoxy-6-(methylsulfonyl)pyridine (88.8 mg, 0.3 mmol), Pd₂(dba)₃ (36.6 mg, 0.04 mmol), Xant-phos (46.3 mg, 0.08 mmol) and Cs₂CO₃ (130.4 mg, 0.4 mmol) in dioxane (3 mL) was stirred at 130 °C for 5 hr. The mixture was cooled to rt and filtered through celite. The filtrate was concentrated under reduced pressure. The crude product was purified by silica gel column chromatography (DCM/MeOH = 15/1) to give the product (17.35 mg, 17%). ¹H NMR (400 MHz, DMSO-*d*₆) δ 12.11 (s, 1H), 10.47 (s, 1H), 9.34 (s, 1H), 8.59 (s, 1H), 7.57 (d, *J =* 8.3 Hz, 1H), 7.46 (d,*J* = 8.3 Hz, 1H), 7.37 (s, 1H), 4.28 (s, 2H), 4.01 (s, 3H), 3.95 (s, 3H), 3.48 (s, 3H), 2.12 (s, 3H), 1.37 (s, 6H). MS (ESI) m/e [M+1]⁺ 530.

### Example Q35: Synthesis of compound (S)-N-(5-(2,2-dimethyl-2,3-dihydro-[1,4]dioxino[2,3-b]pyridin-6-yl)-4-((4-(3-methyl-morpholino)-6-(methylsulfonyl)pyridin-2-yl)amino)-pyridin-2-yl)acetamide.

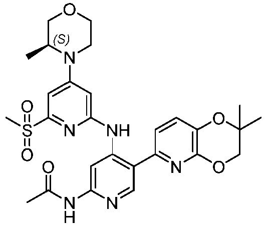

*N*-(4-amino-5-(2,2-dimethyl-2,3-dihydro-[1,4]dioxino[2,3-*b*]pyridin-6-yl)pyri-din-2-yl)acetamide (50 mg, 0.16 mmol), (*S*)-4-(2-chloro-6-(methylsulfonyl)pyridin-4-yl)-3-methyl-morpholine (50.8 mg, 0.18 mmol), Pd₂dba₃ (14.6 mg, 0.02 mmol), BINAP (20 mg, 0.03 mmol) and Cs₂CO₃ (78 mg, 0.24 mmol) were added into 1,4-dioxane (10 mL). The resulting mixture was degassed with nitrogen and heated to 130 °C with stirring for 2 hr. The reaction was cooled to room temperature. The solids were filtered out. The filtration was concentrated under vacuum. The residue was first applied onto Prep-TLC with DCM/MeOH (20/1) to give the product (65.71 mg, yield: 72.6%). ¹H NMR (400 MHz, DMSO-*d*₆) δ 11.48 (s, 1H), 10.44 (s, 1H), 8.88 (s, 1H), 8.56 (s, 1H), 7.56 (d, *J* = 8.3 Hz, 1H), 7.42 (d, *J* = 8.3 Hz, 1H), 7.06 (s, 1H), 6.45 (s, 1H), 4.24 (s, 2H), 4.03 (d, *J* = 5.1 Hz, 1H), 3.99 - 3.90 (m, 1H), 3.70 (d, *J =* 10.3 Hz, 2H), 3.52 (t, *J* = 10.3 Hz, 2H), 3.36 (s, 3H), 3.16 (d, *J =* 10.9 Hz, 1H), 2.10 (s, 3H), 1.36 (s, 6H), 1.16 (d, *J =* 6.6 Hz, 3H). MS (ESI) m/e [M+1]⁺ 569.

### Example Q36: Synthesis of compound (R)-N-(5-(2,2-dimethyl-2,3-dihydro-[1,4]dioxino[2,3-b]pyridin-6-yl)-4-((4-(3-methyl-morpholino)-6-(methylsulfonyl)pyridin-2-yl)amino)-pyridin-2-yl)acetamide.

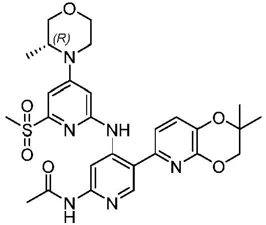

*N*-(4-amino-5-(2,2-dimethyl-2,3-dihydro-[1,4]dioxino[2,3-*b*]pyridin-6-yl)pyri-din-2-yl)acetamide (50 mg, 0.16 mmol), (*R*)-4-(2-chloro-6-(methylsulfonyl)pyridin-4-yl)-3-methyl-morpholine (50.8 mg, 0.18 mmol), Pd₂dba₃ (14.6 mg, 0.02 mmol), BINAP (20 mg, 0.03 mmol) and Cs₂CO₃ (78 mg, 0.24 mmol) were added into 1,4-dioxane (10 mL). The resulting mixture was degassed with nitrogen and heated to 130 °C with stirring for 2 hr. The reaction was cooled to room temperature. The solids were filtered out. The filtration was concentrated under vacuum. The residue was first applied onto Prep-TLC with DCM/MeOH (20/1) to give the product (49.19 mg, yield: 54.4%). ¹H NMR (400 MHz, DMSO-*d*₆) δ 11.48 (s, 1H), 10.43 (s, 1H), 8.88 (s, 1H), 8.56 (s, 1H), 7.55 (d, *J* = 8.2 Hz, 1H), 7.42 (d, *J* = 8.2 Hz, 1H), 7.05 (s, 1H), 6.45 (s, 1H), 4.23 (s, 2H), 4.03 - 4.01 (m, 1H), 3.95 (d, *J =* 10.6 Hz, 1H), 3.69 (dd, *J =* 11.4 Hz, 2H), 3.55 - 3.49 (m, 2H), 3.35 (s, 3H), 3.17 (t, *J =* 11.4 Hz, 1H), 2.09 (s, 3H), 1.35 (s, 6H), 1.16 (d, *J* = 4.1 Hz, 3H). MS (ESI) m/e [M+1]⁺ 569.

### Example Q37: synthesis of compound N-(5-(3,3-dimethyl-2,3-dihydro-[1,4]dioxino[2,3-b]pyridin-6-yl)-4-((4-(methoxy-d3)-6-(methylsulfonyl)pyridin-2-yl)amino)pyridin-2-yl)acetamide

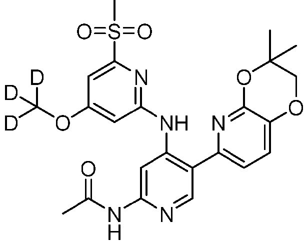

A mixture of N-(4-amino-5-(3,3-dimethyl-2,3-dihydro-[1,4]dioxino[2,3-b]pyridin-6-yl)pyridin-2-yl)acetamide (50 mg, 0.16 mmol), 2-bromo-4-(methoxy-d3)-6-(methylsulfonyl)pyridine (51 mg, 0.19 mmol), Pd₂(dba)₃ (15 mg, 0.016 mmol), BINAP ( 10 mg, 0.016 mmol) and Cs₂CO₃ (156 mg, 0.48 mmol) in dioxane (10 mL) was stirred for 5 h at 130 °C under nitrogen atmosphere. After cooled to room temperature, the solid was removed by filtration and the filtrate was concentrated under reduced pressure. The residue was purified by combi-flash (MeOH/DCM = 0- 10%) to give the product (29.01 mg, 36.4 %). ¹H NMR (400 MHz, DMSO-*d*₆) δ 12.03 (s, 1H), 10.44 (s, 1H), 8.93 (s, 1H), 8.57 (s, 1H), 7.51 (d, *J =* 8.0 Hz, 1H), 7.44 (d, *J* = 8.0 Hz, 1H), 7.03 (s, 1H), 6.57 (s, 1H), 4.01 (s, 2H), 3.35 (s, 3H), 2.05 (s, 3H), 1.33 (s, 6H). MS (ESI) m/e [M+1]⁺ 503.

### Example Q38: synthesis of compound N-(5-(3,3-dimethyl-2,3-dihydro-[1,4]dioxino[2,3-b]pyridin-6-yl)-4-((4-methoxy-6-(methylsulfonyl)pyridin-2-yl)amino)pyridin-2-yl)acetamide

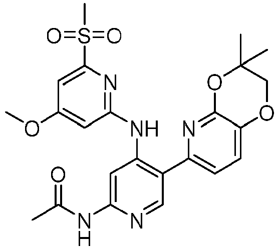

A mixture of N-(4-amino-5-(3,3-dimethyl-2,3-dihydro-[1,4]dioxino[2,3-b]pyridin-6-yl)pyridin-2-yl)acetamide (50 mg, 0.16 mmol), 2-bromo-4-methoxy-6-(methylsulfonyl)pyridine (51 mg, 0.19 mmol), Pd₂(dba)₃ (15 mg, 0.016 mmol), BINAP ( 10 mg, 0.016 mmol) and Cs₂CO₃ (156 mg, 0.48 mmol) in dioxane (10 mL) was stirred for 5 h at 130 °C under nitrogen atmosphere After cooled to room temperature, the solid was removed by filtration and the filtrate was concentrated under reduced pressure. The residue was purified by combi-flash (MeOH/DCM = 0- 10%) to give the product (39.76 mg, 50.1%). ¹H NMR (400 MHz, DMSO-*d*₆) δ 12.06 (s, 1H), 10.46 (s, 1H), 8.95 (s, 1H), 8.59 (s, 1H), 7.53 (d, *J =* 8.0 Hz, 1H), 7.46 (d, *J* = 8.0 Hz, 1H), 7.05 (s, 1H), 6.59 (s, 1H), 4.03 (s, 2H), 3.89 (s, 3H), 3.37 (s, 3H), 2.06 (s, 3H), 1.35 (s, 6H). MS (ESI) m/e [M+1]⁺ 500.

### Example Q40: synthesis of compound N-(5-(3,3-dimethyl-2,3-dihydro-[1,4]dioxino[2,3-b]pyridin-6-yl)-4-((6-(methylsulfonyl)-4-(tetrahydro-2H-pyran-4-yl)pyridin-2-yl)amino)pyridin-2-yl)acetamide

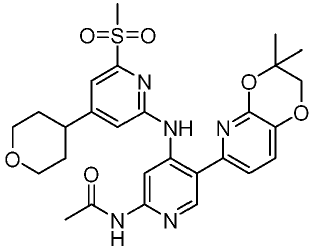

A mixture of N-(4-amino-5-(3,3-dimethyl-2,3-dihydro-[1,4]dioxino[2,3-b]pyridin-6-yl)pyridin-2-yl)acetamide (50 mg, 0.16 mmol), 2-chloro-6-(methylsulfonyl)-4-(tetrahydro-2H-pyran-4-yl)pyridine (53 mg, 0.19 mmol), Pd₂(dba)₃ (15 mg, 0.016 mmol), BINAP ( 10 mg, 0.016 mmol) and Cs₂CO₃ (156 mg, 0.48 mmol) in dioxane (10 mL) was stirred for 5 h at 130 °C under nitrogen atmosphere. After cooled to room temperature, the solid was removed by filtration and the filtrate was concentrated under reduced pressure. The residue was purified by combi-flash (MeOH/DCM = 0- 10%) to give the product (33.12 mg, 37.7 %). ¹H NMR (400 MHz, DMSO-*d*₆) δ 12.43 (s, 1H), 10.45 (s, 1H), 9.04 (s, 1H), 8.62 (s, 1H), 7.56 (d, *J* = 8.0 Hz, 1H), 7.47 (d, *J* = 8.0 Hz, 1H), 7.42 (s, 1H), 6.94 (s, 1H), 4.04 (s, 2H), 3.93 - 3.90 (m, 2H), 3.44 - 3.40 (m, 5H), 2.91 - 2.90 (m, 1H), 2.07 (s, 3H), 1.80 - 1.77 (m, 2H), 1.63 - 1.57 (m, 2H), 1.36 (s, 6H). MS (ESI) m/e [M+1]⁺ 554.

### Example Q43: synthesis of compound (S)-N-(5-(3,3-dimethyl-2,3-dihydro-[1,4]dioxino[2,3-b]pyridin-6-yl)-4-((4-(1-methoxyethyl)-6-(methylsulfonyl)pyridin-2-yl)amino)pyridin-2-yl)acetamide

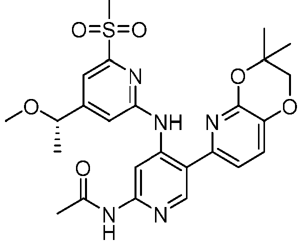

### Step 1. 2-chloro-5-(3,3-dimethyl-2,3-dihydro-[1,4]dioxino[2,3-b]pyridin-6-yl)pyridin-4-amine

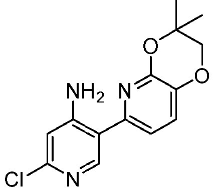

A mixture of 6-bromo-3,3-dimethyl-2,3-dihydro-[1,4]dioxino[2,3-b]pyridine (400 mg, 1.65 mmol), 2-chloro-5-(4,4,5,5-tetramethyl-1,3,2-dioxaborolan-2-yl)pyridin-4-amine (439 mg, 1.73 mmol), Pd(dppf)Cl₂(120 mg, 0.16 mmol) and K₃PO₄ (1.05 g, 4.94 mmol) in dioxane (15 mL) and H₂O (3 mL) was stirred for 2 h at 90 °C under nitrogen atmosphere. After cooled to room temperature, the solid was removed by filtration. The filtrate was concentrated under reduced pressure and the residue was purified by combi-flash (EA/PE = 0 - 60%) to give the product (450 mg, 94.1%). MS (ESI) m/e [M+1]⁺ 292.

### Step 2. N-(4-amino-5-(3,3-dimethyl-2,3-dihydro-[1,4]dioxino[2,3-b]pyridin-6-yl)pyridin-2-yl)acetamide

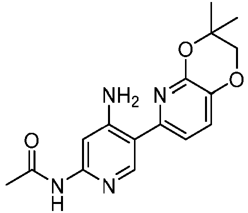

A mixture of 2-chloro-5-(3,3-dimethyl-2,3-dihydro-[1,4]dioxino[2,3-b]pyridin-6-yl)pyridin-4-amine (450 mg, 1.55 mmol), acetamide (639 mg, 10.8 mmol), Pd₂(dba)₃ (142 mg, 0.15 mmol), Xantphos (89 mg, 0.15 mmol) and Cs₂CO₃ (1.51 g, 4.64 mmol) in dioxane (15 mL) was stirred for 5 h at 130 °C under nitrogen atmosphere. After cooled to room temperature, the solid was removed by filtration and the filtrate was concentrated under reduced pressure. The residue was purified by combi-flash (MeOH/DCM = 0- 10%) to give the product (220 mg, 45.4 %). MS (ESI) m/e [M+1]⁺ 315.

### Step 3. (S)-N-(5-(3,3-dimethyl-2,3-dihydro-[1,4]dioxino[2,3-b]pyridin-6-yl)-4-((4-(1-methoxyethyl)-6-(methylsulfonyl)pyridin-2-yl)amino)pyridin-2-yl)acetamide

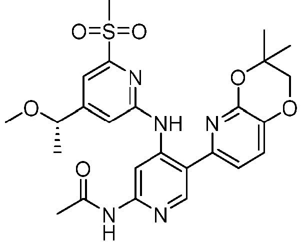

A mixture of N-(4-amino-5-(3,3-dimethyl-2,3-dihydro-[1,4]dioxino[2,3-b]pyridin-6-yl)pyridin-2-yl)acetamide (50 mg, 0.16 mmol), (S)-2-bromo-4-(1-methoxyethyl)-6-(methylsulfonyl)pyridine (56 mg, 0.19 mmol), Pd₂(dba)₃ (15 mg, 0.016 mmol), BINAP ( 10 mg, 0.016 mmol) and Cs₂CO₃ (156 mg, 0.48 mmol) in dioxane (10 mL) was stirred for 5 h at 130 °C under nitrogen atmosphere. After cooled to room temperature, the solid was removed by filtration and the filtrate was concentrated under reduced pressure. The residue was purified by combi-flash (MeOH/DCM = 0- 10%) to give the crude product. The crude product was washed with acetonitrile to give the product (7.29 mg, 8.7 %). ¹H NMR (400 MHz, DMSO-*d*₆) δ 12.26 (s, 1H), 10.47 (s, 1H), 9.05 (s, 1H), 8.61 (s, 1H), 7.55 (d, *J =* 8.0 Hz, 1H), 7.47 (d, *J* = 8.0 Hz, 1H), 7.42 (s, 1H), 7.05 (s, 1H), 4.45 - 4.44 (m, 1H), 4.04 (s, 2H), 3.41 (s, 3H), 3.22 (s, 3H), 2.07 (s, 3H), 1.40-1.29 (m, 9H). MS (ESI) m/e [M+1]⁺ 528.

### Example Q46: synthesis of (S)-N-(5-(2,2-dimethyl-2,3-dihydro-[1,4]dioxino[2,3-b]pyridin-6-yl)-4-((4-(3-methoxypyrrolidin-1-yl)-6-(methylsulfonyl)pyridin-2-yl)amino)pyridin-2-yl)acetamide.

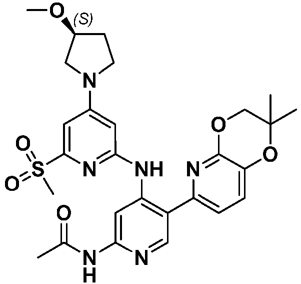

### Step 1: (S)-2,6-dichloro-4-(3-methoxypyrrolidin-1-yl)pyridine.

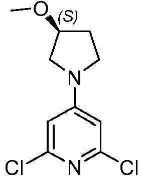

To a round bottom flask was added 2,6-dichloro-4-iodopyridine (5.0 g, 18.3 mmol), (*S*)-3-methoxypyrrolidine (2.6 g, 19.2 mmol), Pd₂dba₃ (503 mg, 0.6 mmol), Xantphos (318 mg, 0.6 mmol), *t-*BuOK (4.4 g, 45.8 mmol) and 1,4-dioxane (100 mL). The resulting mixture was charged into nitrogen and heated to 40 °C with stirring for 13 h. The reaction was cooled to room temperature. Solids were filtered out. The filtration was concentrated. The residue was applied onto a silica gel column with EtOAc/PE to afford (*S*)-2,6-dichloro-4-(3-methoxypyrrolidin-1-yl)pyridine as dark brown oil (3.8 g, 84.4%). MS (ESI) m/e [M+1] ⁺ 247.

### Step 2: (S)-2-chloro-4-(3-methoxypyrrolidin-1-yl)-6-(methylthio)pyridine.

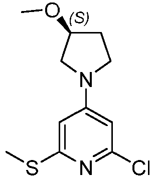

To a solution of (*S*)-2,6-dichloro-4-(3-methoxypyrrolidin-1-yl)pyridine (3.6 g, 14.6 mmol) in DMF (40 mL) was added CH₃SNa (1.3 g, 17.6 mmol). The resulting reaction was heated to 100 °C with stirring for 2 hours. The reaction mixture was cooled to room temperature. The reaction was quenched by water/ice (200 mL). The resulting solution was extracted with EtOAc (40 mL x 5). The organic layer was washed with water and brine, dried and concentrated. This resulted in 4.5 g of (*S*)-2-chloro-4-(3-methoxypyrrolidin-1-yl)-6-(methylthio)pyridine 4.5 g as yellow crude oil. MS (ESI) m/e [M+1] ⁺ 259.

### Step 3: (S)-2-chloro-4-(3-methoxypyrrolidin-1-yl)-6-(methylsulfonyl)pyridine.

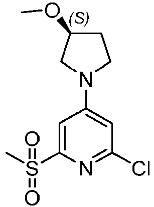

To a solution of (*S*)-2-chloro-4-(3-methoxypyrrolidin-1-yl)-6-(methylthio)py-ridine (4.5 g, 17.4 mmol) in MeOH (30 mL) was added a solution of Oxone (12.8 g, 20.8 mmol) in H₂O (30 mL) at room temperature dropwise with stirring. The resulting solution was stirred at room temperature for 1.5 h. The reaction was diluted with water (150 mL). The resulting solution was extracted with EtOAc (30 mL x 5). The organic layer was washed with water and brine, dried and concentrated. The residue was applied onto a silica gel column with DCM/EtOAc (1: 1). This resulted in 3.5 g of (S)-2-chloro-4-(3-methoxypyrrolidin-1-yl)-6-(methylsulfonyl)pyridine as a light yellow solid (Yield = 78.1% for two steps). MS (ESI) m/e [M+1]⁺ 291.

### Step 4: (S)-N-(5-(2,2-dimethyl-2,3-dihydro-[1,4]dioxino[2,3-b]pyridin-6-yl)-4-((4-(3-methoxypyrrolidin-1-yl)-6-(methylsulfonyl)pyridin-2-yl)amino)pyridin-2-yl)acetamide.

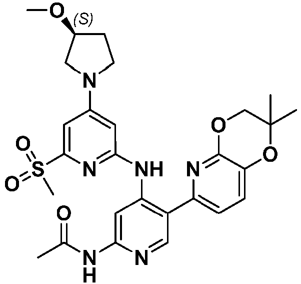

(*S*)-2-chloro-4-(3-methoxypyrrolidin-1-yl)-6-(methylsulfonyl)pyridine (100 mg, 0.3 mmol), *N*-(4-amino-5-(2,2-dimethyl-2,3-dihydro-[1,4]dioxino[2,3-*b*]pyridin-6-yl)pyridin-2-yl)acetamide (102 mg, 0.35 mmol), Pd₂dba₃ (29.2 mg, 0.03 mmol), Xantphos (37 mg, 0.06 mmol) and K₃PO₄ (135 mg, 0.6 mmol) were added into 2-methyltetrahydrofuran (9.0 mL) and H₂O (1.0 mL). The resulting mixture was degassed with nitrogen and heated to 90 °C with stirring for 16 h. The reaction was cooled to room temperature. The solids were filtered out. The filtration was concentrated under vacuum. The residue was applied onto Prep-TLC with DCM/MeOH (17:1) to afford (*S*)-*N*-(5-(2,2-dimethyl-2,3-dihydro-[1,4]dioxino[2,3-*b*]pyridin-6-yl)-4-((4-(3-metho-xypyrrolidin-1-yl)-6-(methylsulfonyl)pyridin-2-yl)amino)pyridin-2-yl)acetamide as a white solid (71.97 mg, yield: 40.0%). ¹H NMR (400 MHz, DMSO-*d*₆) δ 11.48 (s, 1H), 10.45 (s, 1H), 8.85 (s, 1H), 8.57 (s, 1H), 7.57 (d, *J =* 8.3 Hz, 1H), 7.43 (d, *J* = 8.3 Hz, 1H), 6.78 (s, 1H), 6.21 (s, 1H), 4.25 (s, 2H), 4.11 (s, 1H), 3.59 - 3.35 (m, 7H), 3.28 (s, 3H), 2.12 - 2.06 (m, 5H), 1.35 (s, 6H). MS (ESI) m/e [M+1]⁺ 569.

### Example Q49: synthesis of N-(5-(2,2-dimethyl-2,3-dihydro-[1,4]dioxino[2,3-b]pyridin-6-yl)-4-((4-(3-methoxyazetidin-1-yl)-6-(methylsulfonyl)pyridin-2-yl)amino)pyridin-2-yl)acetamide

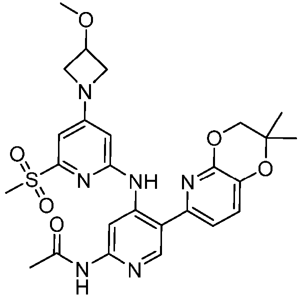

A mixture of N-(4-amino-5-(2,2-dimethyl-2,3-dihydro-[1,4]dioxino[2,3-b]pyridin-6-yl)pyridin-2-yl)acetamide (100 mg, 0.32 mmol), 2-chloro-4-(3-methoxyazetidin-1-yl)-6-(methylsulfonyl)pyridine (105 mg, 0.38 mmol), Pd₂(dba)₃ (29 mg, 0.032 mmol), BINAP ( 20 mg, 0.032 mmol) and Cs₂CO₃ (311 mg, 0.96 mmol) in 1,4-dioxane (10 mL) was stirred for 5 h at 130 °C under nitrogen atmosphere. The mixture was allowed to cool down to room temperature. The resulting mixture was filtered and the filtrate was concentrated under reduced pressure. The residue was purified by combi-flash (MeOH/DCM = 0-10%) to give the crude product. The crude product was suspended in acetonitrile to give the product (46 mg, 26.4%). ¹H NMR (400 MHz, DMSO-*d*₆) δ 11.43 (s, 1H), 10.40 (s, 1H), 8.79 (s, 1H), 8.52 (s, 1H), 7.51 (d, *J =* 8.0 Hz, 1H), 7.37 (d, *J* = 8.0 Hz, 1H), 6.58 (s, 1H), 6.01 (s, 1H), 4.33 - 4.32 (m, 1H), 4.19 - 4.18 (m, 4H), 3.81 (d, *J* = 8.0 Hz, 2H), 3.29 (s, 3H), 3.21 (s, 3H), 2.05 (s, 3H), 1.31 (s, 6H). LCMS (ESI) m/e [M+1]⁺ = 555.

### Example Q50: synthesis of (R/S)-N-(4-((4-(1,4-dioxan-2-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide

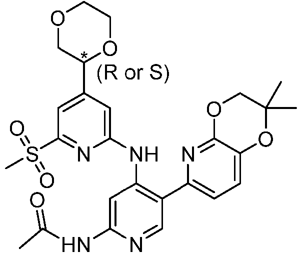

A mixture of N-(4-amino-5-(2,2-dimethyl-2,3-dihydro-[1,4]dioxino[2,3-b]pyridin-6-yl)pyridin-2-yl)acetamide (100 mg, 0.32 mmol), (R or S)-2-chloro-4-(1,4-dioxan-2-yl)-6-(methylsulfonyl)pyridine (98 mg, 0.35 mmol), Pd₂(dba)₃ (30 mg, 0.032 mmol), BINAP ( 20 mg, 0.032 mmol) and Cs₂CO₃ (312 mg, 0.96 mmol) in dioxane (10 mL) was stirred for 5 h at 130 °C under nitrogen atmosphere. The mixture was allowed to cool down to room temperature. The resulting mixture was filtered. The filtrate was concentrated under reduced pressure. The residue was purified by combi-flash (MeOH/DCM = 0-10%) to give the crude product. The crude product was suspended in acetonitrile to give the product (32 mg, 17.9%). ¹H NMR (400 MHz, DMSO-*d*₆) δ 11.78 (s, 1H), 10.44 (s, 1H), 8.98 (s, 1H), 8.55 (s, 1H), 7.57-7.44 (m, 2H), 7.37 (d, *J =* 8.0 Hz, 1H), 7.12 (s, 1H), 4.71 - 4.70 (m, 1H), 4.19 (s, 2H), 3.90 - 3.88 (m, 2H), 3.78 - 3.66 (m, 2H), 3.54 - 3.53 (m, 1H), 3.36 (s, 3H), 3.23 - 3.19 (m, 1H), 2.04 (s, 3H), 1.30 (s, 6H). MS (ESI) m/e [M+1]⁺ 556.

### Example Q51: synthesis of (S/R)-N-(4-((4-(1,4-dioxan-2-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide

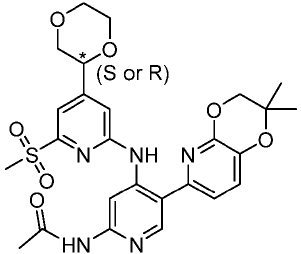

A mixture of N-(4-amino-5-(2,2-dimethyl-2,3-dihydro-[1,4]dioxino[2,3-b]pyridin-6-yl)pyridin-2-yl)acetamide (50 mg, 0.15 mmol), (S or R)-2-chloro-4-(1,4-dioxan-2-yl)-6-(methylsulfonyl)pyridine (51 mg, 0.19 mmol), Pd₂(dba)₃ (15 mg, 0.015 mmol), BINAP (10 mg, 0.015 mmol) and Cs₂CO₃ (78 mg, 0.238 mmol) in dioxane (10 mL) was stirred for 5 h at 130 °C under nitrogen atmosphere. The mixture was allowed to cool down to room temperature. The resulting mixture was filtered. The filtrate was concentrated under reduced pressure. The residue was purified by combi-flash (MeOH/DCM = 0-10%) to give the crude product. The crude product was suspended in acetonitrile to give the product (50 mg, 60%). ¹H NMR (400 MHz, DMSO-*d*₆) δ 11.80 (s, 1H), 10.46 (s, 1H), 9.00 (s, 1H), 8.57 (s, 1H), 7.60-7.45 (m, 2H), 7.39 (d, *J* = 7.9 Hz, 1H), 7.14 (s, 1H), 4.72 (d, *J* = 9.0 Hz, 1H), 4.21 (s, 2H), 3.96-3.88 (m, 2H), 3.82 - 3.67 (m, 2H), 3.58-3.55 (m, 1H), 3.38 (s, 3H), 2.06 (s, 3H), 1.32 (s, 6H). MS (ESI) m/e [M+1]⁺ = 556.

### Example Q52: synthesis of N-(5-(2,2-dimethyl-2,3-dihydro-[1,4]dioxino[2,3-b]pyridin-6-yl)-4-((4-(4-methoxypiperidin-1-yl)-6-(methylsulfonyl)pyridin-2-yl)amino)pyridin-2-yl)acetamide

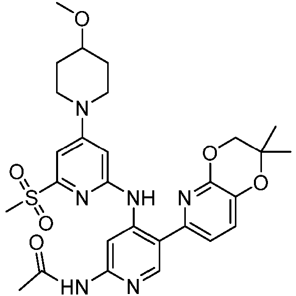

A mixture of N-(4-amino-5-(2,2-dimethyl-2,3-dihydro-[1,4]dioxino[2,3-b]pyridin-6-yl)pyridin-2-yl)acetamide (100 mg, 0.32 mmol), 2-chloro-4-(4-methoxypiperidin-1-yl)-6-(methylsulfonyl)pyridine (116 mg, 0.38 mmol), Pd₂(dba)₃ (29 mg, 0.032 mmol), BINAP ( 20mg, 0.032 mmol) and Cs₂CO₃ (311 mg, 0.96 mmol) in dioxane (10 mL) was stirred for 5 h at 130 °C under nitrogen atmosphere. The mixture was allowed to cool down to room temperature. The resulting mixture was filtered. The filtrate was concentrated under reduced pressure. The residue was purified by combi-flash (MeOH/DCM = 0-10%) to give the crude product. The crude product was suspended in acetonitrile to give the product (41 mg, 22%). ¹H NMR (400 MHz, DMSO-*d*₆) δ 11.35 (s, 1H), 10.37 (s, 1H), 8.81 (s, 1H), 8.49 (s, 1H), 7.49 (d, *J =* 8.0 Hz, 1H), 7.36 (d, *J* = 8.0 Hz, 1H), 7.00 (s, 1H), 6.44 (s, 1H), 4.17 (s, 2H), 3.60 - 3.59 (m, 2H), 3.39 - 3.38 (m, 1H), 3.28 (s, 3H), 3.31 - 3.16 (m, 5H), 2.03 (s, 3H), 1.83 - 1.82 (m, 2H), 1.43 - 1.42 (m, 2H), 1.29 (s, 6H). MS (ESI) m/e [M+1]⁺ 583.

### Example Q53: N-(5-(2,2-dimethyl-2,3-dihydro-[1,4]dioxino[2,3-b]pyridin-6-yl)-4-((4-((cis)-3-methoxycyclobutyl)-6-(methylsulfonyl)pyridin-2-yl)amino)pyridin-2-yl)acetamid

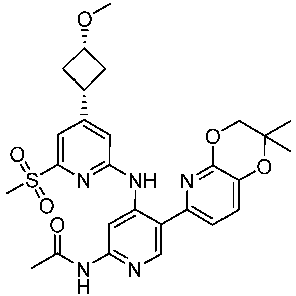

A mixture of N-(4-amino-5-(2,2-dimethyl-2,3-dihydro-[1,4]dioxino[2,3-b]pyridin-6-yl)pyridin-2-yl)acetamide (53 mg, 0.17 mmol), 2-bromo-4-((cis)-3-methoxycyclobutyl)-6-(methylsulfonyl)pyridine (47 mg, 0.17 mmol), Pd₂(dba)₃ (16 mg, 0.017 mmol), Xantphos (10 mg, 0.017 mmol), Cs₂CO₃ (110 mg, 0.34 mmol) in 5 mL 1,4-dioxane was stirred at 130 °C under nitrogen atmosphere for 2 h. After cooled to rt, the solution was concentrated in vacuo and the residue was purified by Prep TLC to give the product (20 mg). ¹H NMR (400 MHz, DMSO-*d₆*) δ 11.85 (s, 1H), 10.50 (s, 1H), 9.06 (s, 1H), 8.61 (s, 1H), 7.58 (d, *J =* 8.0 Hz, 1H), 7.43 (d, *J =* 8.0 Hz, 1H), 7.39 (s, 1H), 7.01 (s, 1H), 4.25 (s, 2H), 3.89 (m, 1H), 3.43 (s, 3H), 3.18 (m, 4H), 2.68 (m, 2H), 2.10 (s, 3H), 1.91 (m, 2H), 1.36 (s, 6H). MS (ESI) m/e [M+1]⁺ = 554.

### Example Q54: Synthesis of N-(5-(2,2-dimethyl-2,3-dihydro-[1,4]dioxino[2,3-b]pyridin-6-yl)-4-((4-((trans)-3-methoxycyclobutyl)-6-(methylsulfonyl)pyridin-2-yl)amino)pyridin-2-yl)acetamide

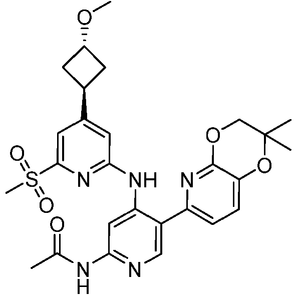

This compound was synthesized by using the similar procedure as example Q53. ¹H NMR (400 MHz, DMSO-*d*₆) δ 11.95 (s, 1H), 10.50 (s, 1H), 9.07 (s, 1H), 8.63 (s, 1H), 7.60 (d, *J =* 8.4 Hz, 1H), 7.43 (m, 2H), 7.06 (s, 1H), 4.25 (s, 2H), 4.12 - 3.97 (m, 1H), 3.71 - 3.56 (m, 1H), 3.44 (s, 3H), 3.19 (s, 3H), 2.43 - 2.32 (m, 4H), 2.11 (s, 3H), 1.36 (s, 6H). MS (ESI) m/e [M+1]⁺ 554.

### Example Q55: Synthesis of N-(5-(2,2-dimethyl-2,3-dihydro-[1,4]dioxino[2,3-b]pyridin-6-yl)-4-((4-(4-hydroxy-4-methylpiperidin-1-yl)-6-(methylsulfonyl)pyridin-2-yl)amino)pyridin-2-yl)acetamide

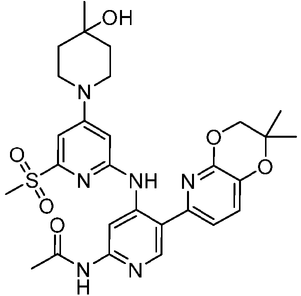

A mixture of N-(4-amino-5-(2,2-dimethyl-2,3-dihydro-[1,4]dioxino[2,3-b]pyridin-6-yl)pyridin-2-yl)acetamide (50 mg, 0.16 mmol), 1-(2-bromo-6-(methylsulfonyl)pyridin-4-yl)-4-methylpiperidin-4-ol (66 mg, 0.19 mmol), Pd₂(dba)₃ (7 mg, 0.008 mmol), XantPhos (9 mg, 0.016 mmol) and K₃PO₄ (68 mg, 0.32 mmol) in 2-MeTHF (5 mL) and water (0.5 mL) was stirred at 90 °C for 4 h under nitrogen atmosphere. After cooled to rt, the reaction mixture was concentrated and the residue was purified by Prep-TLC (MeOH: DCM = 1: 20) to give the product (39 mg, 42%). ¹H NMR (400 MHz, DMSO-*d₆*) δ 11.42 (s, 1H), 10.46 (s, 1H), 8.86 (s, 1H), 8.55 (s, 1H), 7.56 (d, *J* = 8.4 Hz, 1H), 7.43 (d, *J* = 8.3 Hz, 1H), 7.06 (s, 1H), 6.50 (s, 1H), 4.47 (s, 1H), 4.24 (s, 2H), 3.61 (d, *J =* 13.2 Hz, 2H), 3.37-3.34 (m, 5H), 2.10 (s, 3H), 1.57-1.49 (m, 4H), 1.36 (s, 6H), 1.15 (s, 3H). MS (ESI) m/e [M+H]⁺ = 583.

### Example Q63: synthesis of N-(4-((4-cyano-6-(methylsulfonyl)pyridin-2-yl)amino)-5-(2,2-dimethyl-2,3-dihydro-[1,4]dioxino[2,3-b]pyridin-6-yl)pyridin-2-yl)acetamide

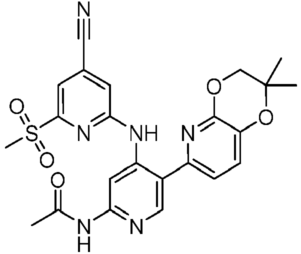

A mixture of N-(4-amino-5-(2,2-dimethyl-2,3-dihydro-[1,4]dioxino[2,3-b]pyridin-6-yl)pyridin-2-yl)acetamide (80 mg, 0.25 mmol), 2-bromo-6-(methylsulfonyl)isonicotinonitrile (80 mg, 0.31 mmol), Pd₂(dba)₃ (23 mg, 0.025 mmol), BINAP (16 mg, 0.025 mmol) and Cs₂CO₃ (249 mg, 0.76 mmol) in dioxane (10 mL) was stirred for 5 h at 130 °C under nitrogen atmosphere. The mixture was allowed to cool to room temperature. The resulting mixture was filtered. The filtrate was concentrated under reduced pressure. The residue was purified by combi-flash (MeOH/DCM = 0- 10%) to give the crude product. The crude product was suspended in acetonitrile to give the product (11.09 mg). ¹H NMR (400 MHz, DMSO-*d*₆) δ 11.94 (s, 1H), 10.59 (s, 1H), 9.05 (s, 1H), 8.65 (s, 1H), 7.82 (s, 1H), 7.63 - 7.50 (m, 2H), 7.43 (d, *J* = 8.0 Hz, 1H), 4.25 (s, 2H), 3.47 (s, 3H), 2.12 (s, 3H), 1.37 (s, 6H). MS (ESI) m/e [M+1]⁺ 495.

The following Examples were prepared in a similar manner to the product Example Q1:

| **Example** | **Compound** | **Chemical Name** | **¹H NMR and LC /MS *m*/*z* (M+1)** |
|---|---|---|---|
| | 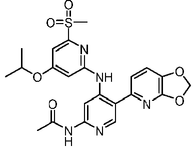 | N-(5-([1,3]dioxolo[4,5-b]pyridin-5-yl)-4-((4-isopropoxy-6-(methylsulfonyl)pyridin-2-yl)amino)pyridin-2-yl)acetamide | ¹H NMR (400 MHz, DMSO-*d*₆) δ 10.43 (s, 1H), 10.13 (s, 1H), 8.85 (s, 1H), 8.35 (s, 1H), 7.35 (d, *J =* 8.0 Hz, 1H), 7.26 (d, *J* = 8.0 Hz, 1H), 6.98 (s, 1H), 6.67 (s, 1H), 6.19 (s, 2H), 4.79 - 4.43 (m, 1H), 3.32 (s, 3H), 2.05 (s, 3H), 1.27 (d, *J =* 5.9 Hz, 6H). MS (ESI) m/e [M+1]⁺ 486. |
| Q2 | | | |
| | 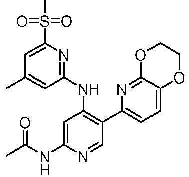 | N-(5-(2,3-dihydro-[1,4]dioxino[2,3-b]pyridin-6-yl)-4-((4-methyl-6-(methylsulfonyl)pyridin-2-yl)amino)pyridin-2-yl)acetamide | ¹H NMR (400 MHz, DMSO-*d*₆) δ 11.98 (s, 1H), 10.49 (s, 1H), 9.13 (s, 1H), 8.64 (s, 1H), 7.60 (d, *J =* 8.1 Hz, 1H), 7.54 - 7.38 (m, 2H), 6.96 (s, 1H), 4.55 - 4.52 (m, 2H), 4.38 - 4.35 (m, 2H), 3.43 (s, 3H), 2.42 (s, 3H), 2.11 (s, 3H). MS (ESI) m/e [M+1]⁺ 456. |
| Q3 | | | |
| | 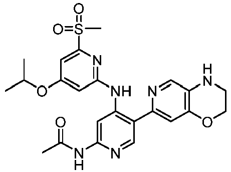 | N-(5-(3,4-dihydro-2H-pyrido[4,3-b][1,4]oxazin-7-yl)-4-((4-isopropoxy-6-(methylsulfonyl)pyridin-2-yl)amino)pyridin-2-yl)acetamide | ¹H NMR (400 MHz, DMSO-*d*₆) δ 12.31 (s, 1H), 10.41 (s, 1H), 9.03 (s, 1H), 8.53 (s, 1H), 7.51 (s, 1H), 7.12 - 7.04 (m, 3H), 6.94 (s, 1H), 5.06 - 4.79 (m, 1H), 4.19 (s, 2H), 3.53 (s, 2H), 3.41 (s, 3H), 2.09 (s, 3H), 1.34 (d, *J* = 5.9 Hz, 6H). MS (ESI) m/e [M+1]⁺ 499. |
| Q4 | | | |
| | 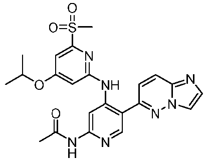 | N-(5-(imidazo[1,2-b]pyridazin-6-yl)-4-((4-isopropoxy-6-(methylsulfonyl)pyridin-2-yl)amino)pyridin-2-yl)acetamide | ¹H NMR (400 MHz, DMSO-*d*₆) δ 10.61 (s, 1H), 9.81 (s, 1H), 8.97 (s, 1H), 8.50 (s, 1H), 8.44 (s, 1H), 8.18 (d, *J =* 12.0 Hz, 1H), 7.82 (s, 1H), 7.54 (d, *J* = 12.0 Hz, 1H), 7.02 - 7.01 (m, 1H), 6.82 - 6.81 (m, 1H), 4.77 - 4.76 (m, 1H), 3.35 (s, 3H), 2.12 (s, 3H), 1.31 (d, *J =* 4.0 Hz, 6H). MS (ESI) m/e [M+1]⁺ 482. |
| Q5 | | | |
| | 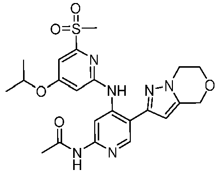 | N-(5-(6,7-dihydro-4H-pyrazolo[5,1-c][1,4]oxazin-2-yl)-4-((4-isopropoxy-6-(methylsulfonyl)pyridin-2-yl)amino)pyridin-2-yl)acetamide | ¹H NMR (400 MHz, DMSO-*d*₆) δ 11.21 (s, 1H), 10.42 (s, 1H), 9.16 (s, 1H), 8.59 (s, 1H), 7.09 (d, *J =* 1.6 Hz, 1H), 6.77 (s, 1H), 6.73 (s, 1H), 4.89 (s, 3H), 4.32 (s, 2H), 4.16 - 4.12 (m, 2H), 3.46 (s, 3H), 2.11 (s, 3H), 1.34 (d, *J* = 5.9 Hz, 6H). MS (ESI) m/e [M+1]⁺ 487. |
| Q6 | | | |
| | 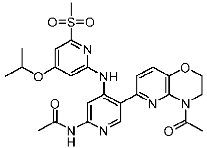 | *N*-(5-(4-acetyl-3,4-dihydro-2*H*-pyrido[3,2-*b*][1,4]oxazin-6-yl)-4-((4-isopropoxy-6-(methylsulfonyl)pyridin-2-yl)amino)pyridin-2-yl)acetamide | ¹H NMR (400 MHz, CDCl₃) δ 11.30 (s, 1H), 9.11 (s, 1H), 8.86 (s, 1H), 8.37 (s, 1H), 7.45 - 7.40 (m, 2H), 6.67 (s, 1H), 4.75 - 4.71 (m, 1H), 4.40 - 4.35 (m, 2H), 4.15 - 4.10 (m, 2H), 3.38 (s, 3H), 2.56 (s, 3H), 2.23 (s, 3H), 1.39 (d, *J* = 6.0 Hz, 6H). MS (ESI) m/e [M+1]⁺ 541. |
| Q7 | | | |
| | 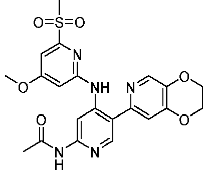 | N-(5-(2,3-dihydro-[1,4]dioxino[2,3-c]pyridin-7-yl)-4-((4-methoxy-6-(methylsulfonyl)pyridin-2-yl)amino)pyridin-2-yl)acetamide | ¹H NMR (400 MHz, DMSO-*d₆*) δ 12.51 (s, 1H), 10.46 (s, 1H), 9.10 (s, 1H), 8.63 (s, 1H), 8.38 (s, 1H), 7.61 (s, 1H), 7.09 (s, 1H), 6.84 (s, 1H), 4.43 - 4.39 (m, 4H), 3.95 (s, 3H), 3.43 (s, 3H), 2.11 (s, 3H). MS (ESI) m/e [M+1]⁺ 472. |
| Q8 | | | |
| | 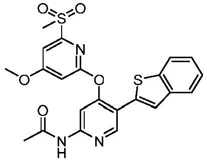 | N-(5-(benzo[b]thiophen-2-yl)-4-((4-methoxy-6-(methylsulfonyl)pyridin-2-yl)amino)pyridin-2-yl)acetamide | ¹H NMR (400 MHz, DMSO-*d₆*) δ 10.51 (s, 1H), 8.92 (s, 1H), 8.60 (s, 1H), 8.14 (s, 1H), 8.10 - 8.05 (m, 1H), 7.91 (s, 1H), 7.49 - 7.46 (m, 1H), 7.42 - 7.38 (m, 1H), 7.35 - 7.33 (m, 1H), 7.02 (s, 1H), 6.71 (s, 1H), 3.77 (s, 3H), 3.35 (s, 3H), 2.12 (s, 3H). MS (ESI) m/e [M+1]⁺ 469. |
| Q9 | | | |
| | 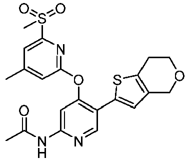 | N-(5-(6,7-dihydro-4H-thieno[3,2-c]pyran-2-yl)-4-((4-methyl-6-(methylsulfonyl)pyridin-2-yl)amino)pyridin-2-yl)acetamide | 1H NMR (400 MHz, DMSO-d6) δ 10.49 (s, 1H), 8.97 (s, 1H), 8.73 (s, 1H), 8.23 (s, 1H), 7.36 (s, 1H), 7.14 (s, 1H), 7.05 (s, 1H), 4.65 - 4.60 (m, 2H), 3.95 - 3.90 (m, 2H), 3.28 (s, 3H), 2.85 - 2.80 (m, 2H), 2.36 (s, 3H), 2.08 (s, 3H). MS (ESI) m/e [M+1]⁺ 459. |
| Q10 | | | |
| | 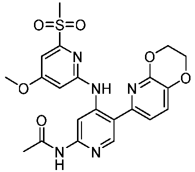 | N-(5-(2,3-dihydro-[1,4]dioxino[2,3-b]pyridin-6-yl)-4-((4-methoxy-6-(methylsulfonyl)pyridin-2-yl)amino)pyridin-2-yl)acetamide | MS (ESI) m/e [M+1]⁺ 472. |
| Q11 | | | |
| | 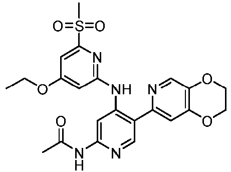 | N-(5-(2,3-dihydro-[1,4]dioxino[2,3-c]pyridin-7-yl)-4-((4-ethoxy-6-(methylsulfonyl)pyridin-2-yl)amino)pyridin-2-yl)acetamide | MS (ESI) m/e [M+1]⁺ 486. |
| Q12 | | | |
| | 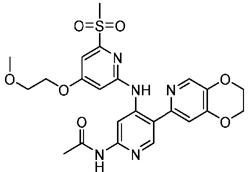 | N-(5-(2,3-dihydro-[1,4]dioxino[2,3-c]pyridin-7-yl)-4-((4-(2-methoxyethoxy)-6-(methylsulfonyl)pyridin-2-yl)amino)pyridin-2-yl)acetamide | MS (ESI) m/e [M+1]⁺ 516. |
| Q13 | | | |
| | 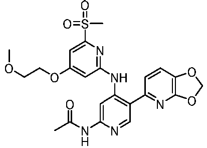 | N-(5-([1,3]dioxolo[4,5-b]pyridin-5-yl)-4-((4-(2-methoxyethoxy)-6-(methylsulfonyl)pyridin-2-yl)amino)pyridin-2-yl)acetamide | MS (ESI) m/e [M+1]⁺ 502. |
| Q14 | | | |
| | 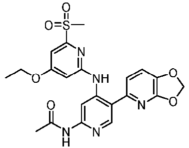 | N-(5-([1,3]dioxolo[4,5-b]pyridin-5-yl)-4-((4-ethoxy-6-(methylsulfonyl)pyridin-2-yl)amino)pyridin-2-yl)acetamide | MS (ESI) m/e [M+1]⁺ 472. |
| Q15 | | | |
| | 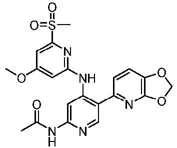 | N-(5-([1,3]dioxolo[4,5-b]pyridin-5-yl)-4-((4-methoxy-6-(methylsulfonyl)pyridin-2-yl)amino)pyridin-2-yl)acetamide | MS (ESI) m/e [M+1]⁺ 458. |
| Q16 | | | |
| | 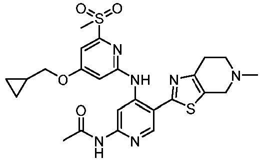 | N-(4-((4-(cyclopropylmethoxy)-6-(methylsulfonyl)pyridin-2-yl)amino)-5-(5-methyl-4,5,6,7-tetrahydrothiazolo [5,4-c]pyridin-2-yl)pyridin-2-yl)acetamide | MS (ESI) m/e [M+1]⁺ 529 |
| Q17 | | | |
| Q18 | 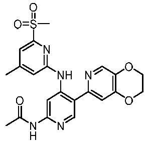 | N-(5-(2,3-dihydro-[1,4]dioxino[2,3-c]pyridin-7-yl)-4-((4-methyl-6-(methylsulfonyl)pyridin-2-yl)amino)pyridin-2-yl)acetamide | Molecular Weight: 455 |
| Q19 | 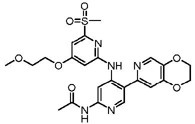 | N-(5-(2,3-dihydro-[1,4]dioxino[2,3-c]pyridin-7-yl)-4-((4-(2-methoxyethoxy)-6-(methylsulfonyl)pyridin-2-yl)amino)pyridin-2-yl)acetamide | Molecular Weight: 516 |
| Q20 | 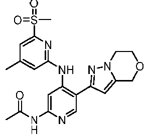 | N-(5-(6,7-dihydro-4H-pyrazolo[5,1-c][1,4]oxazin-2-yl)-4-((4-methyl-6-(methylsulfonyl)pyridin-2-yl)amino)pyridin-2-yl)acetamide | Molecular Weight: 442 |
| Q27 | 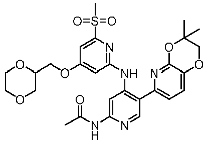 | N-(4-((4-((1,4-dioxan-2-yl)methoxy)-6-(methylsulfonyl)pyridin-2-yl)amino)-5-(3,3-dimethyl-2,3-dihydro-[1,4]dioxino[2,3-b]pyridin-6-yl)pyridin-2-yl)acetamide | Molecular Weight: 586 |
| Q28 | 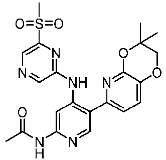 | N-(5-(3,3-dimethyl-2,3-dihydro- [1,4] dioxino [2,3-b]pyridine-6-yl)-4-((6-(methylsulfonyl)pyrazin-2-yl)amino)pyridine-2-yl)acetamide | Molecular Weight: 471 |
| Q29 | 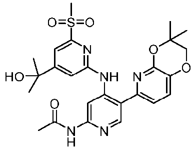 | N-(5-(3,3-dimethyl-2,3-dihydro- [1,4] dioxino [2,3-b]yridine-6-yl)-4-((4-(2-hydroxypropan-2-yl)-6-(methylsulfonyl)pyridine-2-yl)amino)pyridine-2-yl)acetamide | Molecular Weight: 528 |
| Q30 | 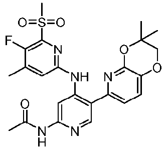 | N-(5-(3,3-dimethyl-2,3-dihydro- [1,4] dioxino [2,3-b]pyridine-6-yl)-4-((5-fluoro-4-methyl-6-(methylsulfonyl)pyridine-2-yl)amino)pyridine-2-yl)acetamide | Molecular Weight: 502 |
| Q 39 | 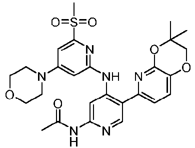 | N-(5-(3,3-dimethyl-2,3-dihydro-[1,4] dioxino [2,3-b]pyridin-6-yl)-4-((6-(methylsulfonyl)-4-morpholinopyridin-2-yl)amino)pyridin-2-yl)acetamide | Molecular Weight: 555 |
| Q 41 | 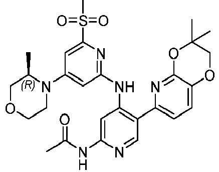 | (R)-N-(5-(3,3-dimethyl-2,3-dihydro-[1,4]dioxino[2,3-b]pyridin-6-yl)-4-((4-(3-methylmorpholino)-6-(methylsulfonyl)pyridin-2-yl)amino)pyridin-2-yl)acetamide | Molecular Weight: 569 |
| Q42 | 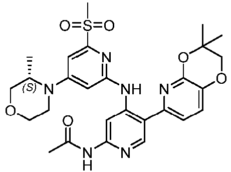 | (S)-N-(5-(3,3-dimethyl-2,3-dihydro-[1,4]dioxino[2,3-b]pyridin-6-yl)-4-((4-(3-methylmorpholino)-6-(methylsulfonyl)pyridin-2-yl)amino)pyridin-2-yl)acetamide | Molecular Weight: 569 |
| Q44 | 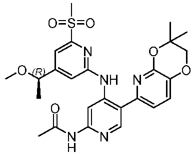 | (R)-N-(5-(3,3-dimethyl-2,3-dihydro-[1,4]dioxino[2,3-b]pyridin-6-yl)-4-((4-(1-methoxyethyl)-6-(methylsulfonyl)pyridin-2-yl)amino)pyridin-2-yl)acetamide | Molecular Weight: 528 |
| Q45 | 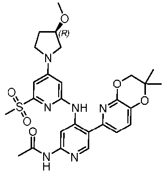 | (R)-N-(5-(2,2-dimethyl-2,3-dihydro-[1,4]dioxino[2,3-b]pyridin-6-yl)-4-((4-(3-methoxypyrrolidin-1-yl)-6-(methylsulfonyl)pyridin-2-yl)amino)pyridin-2-yl)acetamide | ¹H NMR (400 MHz, DMSO-d*₆*) δ 11.41 (s, 1H), 10.40 (s, 1H), 8.77 (s, 1H), 8.50 (s, 1H), 7.50 (d, *J* = 8.4 Hz, 1H), 7.36 (d,*J =* 8.3 Hz, 1H), 6.71 (s, 1H), 6.15 (s, 1H), 4.18 (s, 2H), 4.05 (s, 1H), 3.47 - 3.34 (m, 4H), 3.26 (s, 3H), 3.22 (s, 3H), 2.03 - 2.01 (m, 5H), 1.29 (s, 6H). MS (ESI) m/e [M+H]⁺ = 569. |
| | | | |
| Q47 | 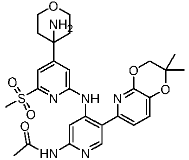 | N-(4-((4-(4-aminotetrahydro-2H-pyran-4-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide | Molecular Weight: 569 |
| Q48 | 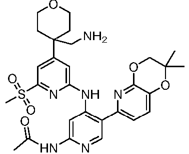 | N-(4-((4-(4-(aminomethyl)tetrahydro-2H-pyran-4-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide | Molecular Weight: 583 |
| Q56 | 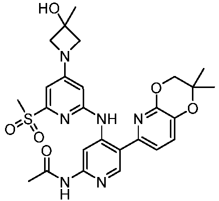 | *N*-(5-(2,2-dimethyl-2,3-dihydro- [1,4] dioxino [2,3-*b*]pyridin-6-yl)-4-((4-(3-hydroxy-3-methylazetidin-1-yl)-6-(methylsulfonyl)pyridin-2-yl)amino)pyrid-in-2-yl)acetamide | ¹H NMR (400 MHz, DMSO-*d*₆) δ 11.58 (s, 1H), 10.51 (s, 1H), 8.84 (s, 1H), 8.57 (s, 1H), 7.57 (d, *J =* 8.4 Hz, 1H), 7.44 (d,*J* = 8.4 Hz, 1H), 6.63 (s, 1H), 6.04 (s, 1H), 5.78 (s, 1H), 4.25 (s, 2H), 3.95 - 3.90 (m, 2H), 3.86 - 3.84 (m, 2H), 3.34 (s, 3H), 2.11 (s, 3H), 1.45 (s, 3H), 1.36 (s, 6H). MS (ESI) m/e [M+1]⁺ 555. |
| Q57 | 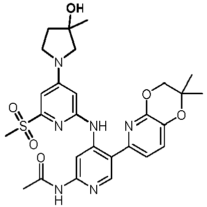 | N-(5-(2,2-dimethyl-2,3-dihydro- [1,4] dioxino [2,3-b]pyridin-6-yl)-4-((4-(3-hydroxy-3-methylpyrrolidin-1-yl)-6-(methyl sulfonyl)pyridin-2-yl)amino)pyridin-2-yl) acetamide | ¹H NMR (400 MHz, DMSO-*d*₆) δ 11.50 (s, 1H), 10.44 (s, 1H), 8.88 (s, 1H), 8.57 (s, 1H), 7.57 (d, *J=* 8.0 Hz, 1H), 7.43 (d, *J* = 8.0 Hz, 1H), 6.72 (s, 1H), 6.14 (s, 1H), 4.93 (s, 1H), 4.25 (s, 2H), 3.50 - 3.45 (m, 2H), 3.34 (s, 3H), 3.30 - 3.25 (m, 2H), 2.09 (s, 3H), 2.00 - 1.95 (m, 2H), 1.36 (s, 9H). MS (ESI) m/e [M+1]⁺ 569. |
| Q58 | 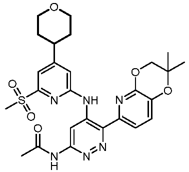 | N-(6-(2,2-dimethyl-2,3-dihydro- [1,4] dioxino [2,3-b]pyridin-6-yl)-5-((6-(methylsulfonyl)-4-(tetrahydro-2H-pyran-4-yl)pyridin-2-yl)amino)pyridazin-3-yl)acetamide | ¹H NMR (400 MHz, DMSO-*d*₆) δ 12.89 (s, 1H), 11.07 (s, 1H), 9.48 (s, 1H), 8.24 (d, *J* = 8.0 Hz, 1H), 7.62 - 7.53 (m, 2H), 7.08 (s, 1H), 4.33 (s, 2H), 4.02 - 3.96 (m, 2H), 3.55 - 3.44 (m, 5H), 3.05 - 3.00 (m, 1H), 2.18 (s, 3H), 1.86 - 1.82 (m, 2H), 1.70 - 1.65 (m, 2H), 1.40 (s, 6H). MS (ESI) m/e [M+1]⁺ 555. |
| Q59 | 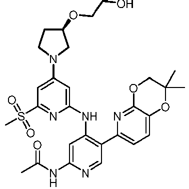 | (R)-N-(5-(2,2-dimethyl-2,3-dihydro-[1,4]dioxino[2,3-b]pyridin-6-yl)-4-((4-(3-(2-hydroxyethoxy)pyrrolidin-1-yl)-6-(methylsulfonyl)pyridin-2-yl)amino)pyridin-2-yl) acetamide | ¹H NMR (400 MHz, DMSO-*d*₆) δ 11.54 (s, 1H), 10.65 (s, 1H), 8.73 (s, 1H), 8.55 (s, 1H), 7.56 (d, *J =* 8.0 Hz, 1H), 7.45 (d, *J* = 8.0 Hz, 1H), 6.80 (s, 1H), 6.24 (s, 1H), 4.28 - 4.22 (m, 2H), 3.56 - 3.38 (m, 9H), 3.33 (s, 3H), 2.15 - 2.10 (m, 5H), 1.36 (s, 6H). MS (ESI) m/e [M+1]⁺ 599. |
| Q60 | 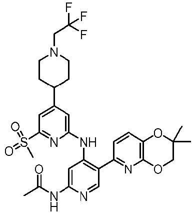 | N-(5-(2,2-dimethyl-2,3-dihydro- [1,4] dioxino [2,3-b]pyridin-6-yl)-4-((6-(methylsulfonyl)-4-(1-(2,2,2-trifluoroethyl)piperidin-4-yl)pyridin-2-yl)amino)pyridin-2-yl) acetamide | ¹H NMR (400 MHz, DMSO-*d*₆) δ 11.76 (s, 1H), 10.50 (s, 1H), 9.05 (s, 1H), 8.61 (s, 1H), 7.59 (d, *J =* 8.3 Hz, 1H), 7.46 (s, 1H), 7.44 (d, *J* = 8.3 Hz, 1H), 7.05 (s, 1H), 4.26 (s, 2H), 3.42 (s, 3H), 3.27 - 3.19 (m, 2H), 3.05 - 3.00 (m, 2H), 2.74 - 2.64 (m, 1H), 2.50 - 2.46 (m, 2H), 2.11 (s, 3H), 1.85 - 1.80 (m, 2H), 1.70 - 1.61 (m, 2H), 1.37 (s, 6H). MS (ESI) m/e [M+1]⁺ 635. |
| Q61 | 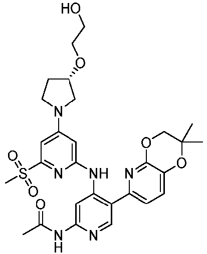 | (S)-N-(5-(2,2-dimethyl-2,3-dihydro-[1,4]dioxino[2,3-b]pyridin-6-yl)-4-((4-(3-(2-hydroxyethoxy)pyrrolidin-1-yl)-6-(methylsulfonyl)pyridin-2-yl)amino)pyridin-2-yl)acetamide | ¹H NMR (400 MHz, DMSO-*d*₆) δ 11.49 (s, 1H), 10.45 (s, 1H), 8.86 (s, 1H), 8.57 (s, 1H), 7.57 (d, *J =* 8.0 Hz, 1H), 7.43 (d, *J* = 8.0 Hz, 1H), 6.78 (s, 1H), 6.21 (s, 1H), 4.62 - 4.60 (m, 1H), 4.25 - 4.20 (m, 3H), 3.54 - 3.49 (m, 6H), 3.43 - 3.41 (m, 4H), 3.35 - 3.32 (m, 1H), 2.12 - 2.10 (m, 5H), 1.36 (s, 6H). MS (ESI) m/e [M+1]⁺ 599. |
| Q62 | 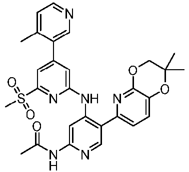 | N-(5-(2,2-dimethyl-2,3-dihydro- [1,4] dioxino [2,3-b]pyridin-6-yl)-4-((4-methyl-6'-(methylsulfonyl)-[3,4'-bipyridin]-2'-yl)amino)pyridin-2-yl)acetamide | ¹H NMR (400 MHz, DMSO-*d*₆) δ 12.01 (s, 1H), 10.54 (s, 1H), 9.16 (s, 1H), 8.65 (s, 1H), 8.55 (d, *J =* 4.0 Hz, 1H), 8.52 (s, 1H), 7.61 (d, *J =* 8.0 Hz, 1H), 7.54 (s, 1H), 7.46 - 7.40 (m, 2H), 7.17 (s, 1H), 4.20 (s, 2H), 3.50 (s, 3H), 2.34 (s, 3H), 2.13 (s, 3H), 1.33 (s, 6H). MS (ESI) m/e [M+1]⁺ 561. |
| | | | |
| Q64 | 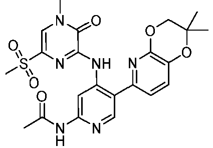 | N-(5-(2,2-dimethyl-2,3-dihydro- [1,4] dioxino [2,3-b]pyridin-6-yl)-4-((4-methyl-6-(methylsulfonyl)-3-oxo-3,4-dihydropyrazin-2-yl)amino)pyridin-2-vl)acetamide | [M+1]⁺ 501 |
| Q65 | 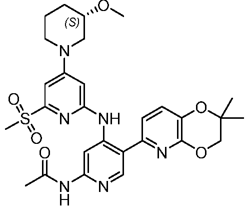 | (S)-N-(5-(2,2-dimethyl-2,3-dihydro- [1,4] dioxino [2,3-b]pyridin-6-yl)-4-((4-(3-methoxypiperidin-1-yl)-6-(methylsulfonyl)pyridin-2-yl)amino)pyridin-2-yl)acetamide | ¹H NMR (400 MHz, DMSO-*d*₆) δ 11.52 (s, 1H), 10.43 (s, 1H), 8.95 (s, 1H), 8.57 (s, 1H), 7.57 (d, *J* = 8.4 Hz, 1H), 7.43 (d, *J* = 8.3 Hz, 1H), 7.05 (d, *J* = 1.8 Hz, 1H), 6.48 (d, *J =* 1.8 Hz, 1H), 4.22 (s, 2H), 3.72 (d, *J* = 12.7 Hz, 1H), 3.66 - 3.50 (m, 1H), 3.36 (s, 3H), 3.33 - 3.19 (m, 6H), 2.10 (s, 3H), 1.98 - 1.87 (m, 1H), 1.75 - 1.73 (m, 1H), 1.55 - 1.43 (m, 2H), 1.36 (s, 6H). MS (ESI) m/e [M+1]⁺ 583 |
| Q66 | 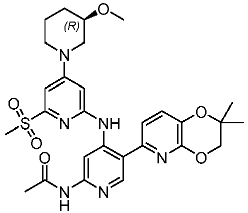 | *(R)-N*-(5-(2,2-dimethyl-2,3-dihydro-[1,4]dioxino[2,3-*b*]pyridin-6-yl)-4-((4-(3-methoxypiperidin-1-yl)-6-(methylsulfonyl)pyridin-2-yl)amino)pyridin-2-yl)acetamide | ¹H NMR (400 MHz, DMSO-*d*₆) δ 11.51 (s, 1H), 10.43 (s, 1H), 8.94 (s, 1H), 8.57 (s, 1H), 7.57 (d, *J* = 8.4 Hz, 1H), 7.43 (d, *J =* 8.3 Hz, 1H), 7.05 (d, *J =* 1.7 Hz, 1H), 6.48 (d, *J* = 1.7 Hz, 1H), 4.21 (s, 2H), 3.73 (d, *J* = 11.3 Hz, 1H), 3.58 - 3.52 (m, 1H), 3.36 (s, 3H), 3.32 - 3.19 (m, 5H), 2.09 (s, 3H), 1.96 - 1.89 (m, 1H), 1.76 - 1.74 (m, 1H), 1.63 - 1.42 (m, 2H), 1.35 (s, 6H). MS (ESI) m/e [M+1]⁺ 583 |
| Q67 | 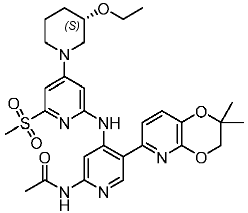 | *(S)-N*-(5-(2,2-dimethyl-2,3-dihydro-[1,4]dioxino[2,3-*b*]pyridin-6-yl)-4-((4-(3-ethoxypiperidin-1-yl)-6-(methylsulfonyl)pyridin-2-yl)amino)pyridin-2-yl)acetamide | MS (ESI) m/e [M+1]⁺ 597 |
| Q68 | 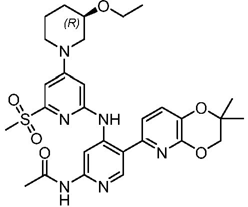 | *(R)-N*-(5-(2,2-dimethyl-2,3-dihydro-[1,4]dioxino[2,3-*b*]pyridin-6-yl)-4-((4-(3-ethoxypiperidin-1-yl)-6-(methylsulfonyl)pyridin-2-yl)amino)pyridin-2-yl)acetamide | MS (ESI) m/e [M+1]⁺ 597 |
| Q69 | 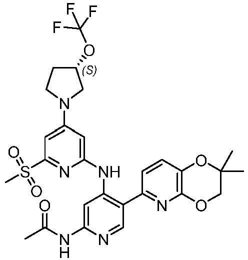 | *(S)-N-*(5-(2,2-dimethyl-2,3-dihydro-[1,4]dioxino[2,3-*b*]pyridin-6-yl)-4-((6-(methylsulfonyl)-4-(3-(trifluoromethoxy)pyrrolidin-1-yl)pyridin-2-yl)amino)pyridin-2-yl)acetamide | MS (ESI) m/e [M+1]⁺ 623 |
| Q70 | 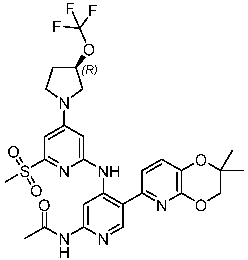 | *(R)-N*-(5-(2,2-dimethyl-2,3-dihydro-[1,4]dioxino[2,3-*b*]pyridin-6-yl)-4-((6-(methylsulfonyl)-4-(3-(trifluoromethoxy)pyrrolidin-1-yl)pyridin-2-yl)amino)pyridin-2-yl)acetamide | MS (ESI) m/e [M+1]⁺ 623 |
| Q71 | 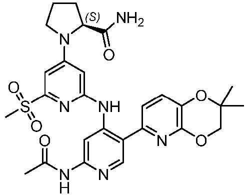 | (*S*)-1-(2-((2-acetamido-5-(2,2-dimethyl-2,3-dihydro-[1,4]dioxino[2,3-*b*]pyridin-6-yl)pyridin-4-yl)amino)-6-(methylsulfonyl)pyridin-4-yl)pyrrolidine-2-carboxamide | MS (ESI) m/e [M+1]⁺ 582 |
| Q72 | 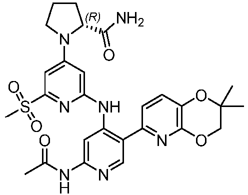 | (*R*)-1-(2-((2-acetamido-5-(2,2-dimethyl-2,3-dihydro-[1,4]dioxino[2,3-*b*]pyridin-6-yl)pyridin-4-yl)amino)-6-(methylsulfonyl)pyridin-4-yl)pyrrolidine-2-carboxamide | MS (ESI) m/e [M+1]⁺ 582 |
| Q73 | 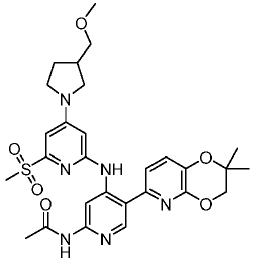 | *N*-(5-(2,2-dimethyl-2,3-dihydro-[1,4]dioxino[2,3-*b*]pyridin-6-yl)-4-((4-(3-(methoxymethyl)pyrrolidin-1-yl)-6-(methylsulfonyl)pyridin-2-yl)amino)pyridin-2-yl)acetamide | ¹H NMR (400 MHz, DMSO-*d*₆) δ 11.45 (s, 1H), 10.43 (s, 1H), 8.88 (s, 1H), 8.56 (s, 1H), 7.57 (d, *J* = 8.4 Hz, 1H), 7.43 (d, *J =* 8.3 Hz, 1H), 6.75 (d, *J* = 1.5 Hz, 1H), 6.17 (s, 1H), 4.24 (s, 2H), 3.56 - 3.42 (m, 2H), 3.42 - 3.35 (m, 3H), 3.31 (s, 3H), 3.28 (s, 3H), 3.15 - 3.11 (m, 1H), 2.61 (m, 1H), 2.13 - 2.06 (m, 4H), 1.78 (m, 1H), 1.36 (s, 6H). MS (ESI) m/e [M+1]⁺ 583 |
| Q73A | 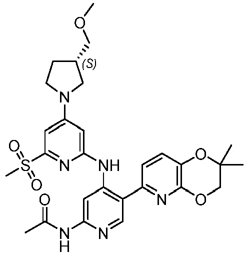 | *(S)-N*-(5-(2,2-dimethyl-2,3-dihydro-[1,4]dioxino[2,3-*b*]pyridin-6-yl)-4-((4-(3-(methoxymethyl)pyrrolidin-1-yl)-6-(methylsulfonyl)pyridin-2-yl)amino)pyridin-2-yl)acetamide | MS (ESI) m/e [M+1]⁺ 583 |
| Q74 | 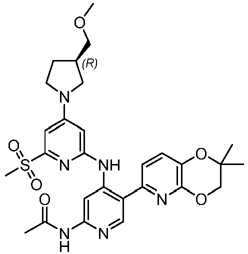 | *(R)-N*-(5-(2,2-dimethyl-2,3-dihydro-[1,4]dioxino[2,3-*b*]pyridin-6-yl)-4-((4-(3-(methoxymethyl)pyrrolidin-1-yl)-6-(methylsulfonyl)pyridin-2-yl)amino)pyridin-2-yl)acetamide | MS (ESI) m/e [M+1]⁺ 583 |
| Q75 | 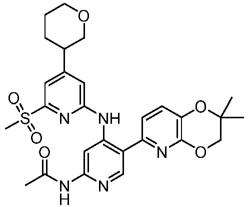 | *N*-(5-(2,2-dimethyl-2,3-dihydro-[1,4]dioxino[2,3-*b*]pyridin-6-yl)-4-((6-(methylsulfonyl)-4-(tetrahydro-2H-pyran-3-yl)pyridin-2-yl)amino)pyridin-2-yl)acetamide | ¹H NMR (400 MHz, DMSO-*d*₆) δ 11.88 (s, 1H), 10.50 (s, 1H), 9.03 (s, 1H), 8.62 (s, 1H), 7.59 (d, *J* = 8.3 Hz, 1H), 7.50 (s, 1H), 7.44 (d, *J =* 8.3 Hz, 1H), 7.10 (s, 1H), 4.26 (s, 2H), 3.90 - 3.83 (m, 2H), 3.56 - 3.38 (m, 5H), 2.98 - 2.92 (m, 1H), 2.11 (s, 3H), 2.01 - 1.98 (m, 1H), 1.91 - 1.56 (m, 3H), 1.37 (s, 6H). MS (ESI) m/e [M+1]⁺ 554 |
| Q75A | 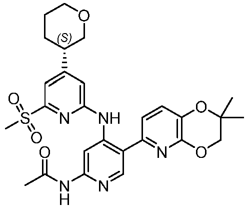 | *(S)-N-*(5-(2,2-dimethyl-2,3-dihydro-[1,4]dioxino[2,3-*b*]pyridin-6-yl)-4-((6-(methylsulfonyl)-4-(tetrahydro-2H-pyran-3-yl)pyridin-2-yl)amino)pyridin-2-yl)acetamide | MS (ESI) m/e [M+1]⁺ 554 |
| Q76 | 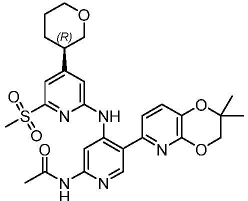 | *(R)-N*-(5-(2,2-dimethyl-2,3-dihydro-[1,4]dioxino[2,3-*b*]pyridin-6-yl)-4-((6-(methylsulfonyl)-4-(tetrahydro-2H-pyran-3-yl)pyridin-2-yl)amino)pyridin-2-yl)acetamide | MS (ESI) m/e [M+1]⁺ 554 |
| Q77 | 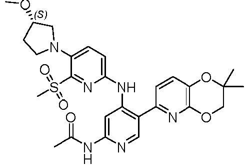 | *(S)-N*-(5-(2,2-dimethyl-2,3-dihydro-[1,4]dioxino[2,3-*b*]pyridin-6-yl)-4-((5-(3-methoxypyrrolidin-1-yl)-6-(methylsulfonyl)pyridin-2-yl)amino)pyridin-2-yl)acetamide | MS (ESI) m/e [M+1]⁺ 569 |
| Q78 | 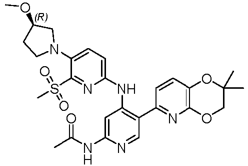 | *(R)-N*-(5-(2,2-dimethyl-2,3-dihydro-[1,4]dioxino[2,3-*b*]pyridin-6-yl)-4-((5-(3-methoxypyrrolidin-1-yl)-6-(methylsulfonyl)pyridin-2-yl)amino)pyridin-2-yl)acetamide | MS (ESI) m/e [M+1]⁺ 569 |
| Q79 | 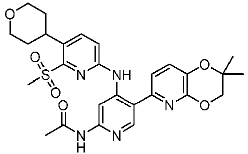 | *N*-(5-(2,2-dimethyl-2,3-dihydro-[1,4]dioxino[2,3-*b*]pyridin-6-yl)-4-((6-(methylsulfonyl)-5-(tetrahydro-2H-pyran-4-yl)pyridin-2-yl)amino)pyridin-2-yl)acetamide | ¹H NMR (400 MHz, DMSO-*d*₆) δ 11.76 (s, 1H), 10.51 (s, 1H), 8.78 (s, 1H), 8.62 (s, 1H), 8.10 (d, *J* = 8.6 Hz, 1H), 7.58 (d, *J =* 8.4 Hz, 1H), 7.42 (d, *J =* 8.3 Hz, 1H), 7.17 (d, *J* = 8.6 Hz, 1H), 4.23 (s, 2H), 3.97 (dd, *J* = 11.2, 3.1 Hz, 2H), 3.78 - 3.70 (m, 1H), 3.43 - 3.36 (m, 5H), 2.10 (s, 3H), 1.84 - 1.61 (m, 4H), 1.36 (s, 6H). MS (ESI) m/e [M+1]⁺ 554 |
| Q80 | 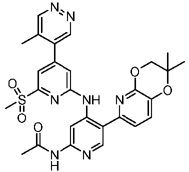 | N-(5-(2,2-dimethyl-2,3-dihydro- [1,4] dioxino [2,3-b]pyridin-6-yl)-4-((4-(5-methylpyridazin-4-yl)-6-(methylsulfonyl)pyridin-2-yl)amino)pyridin-2-yl)acetamide | MS (ESI) m/e [M+1]⁺ = 562. |
| Q81 | 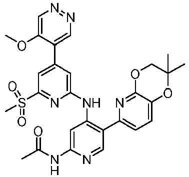 | N-(5-(2,2-dimethyl-2,3-dihydro- [1,4] dioxino [2,3-b]pyridin-6-yl)-4-((4-(5-methoxypyridazin-4-yl)-6-(methylsulfonyl)pyridin-2-yl)amino)pyridin-2-yl)acetamide | MS (ESI) m/e [M+1]⁺ = 578. |
| Q82 | 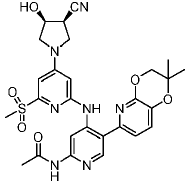 | N-(4-((4-(cis-3-cyano-4-hydroxypyrrolidin-1-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide | MS (ESI) m/e [M+1]⁺ = 580. |
| Q83 | 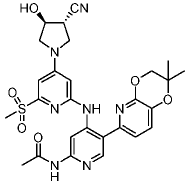 | N-(4-((4-(trans-3-cyano-4-hydroxypyrrolidin-1-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide | MS (ESI) m/e [M+1]⁺ = 580. |
| Q84 | 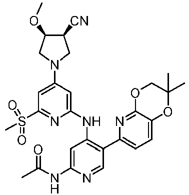 | N-(4-((4-(cis-3-cyano-4-methoxypyrrolidin-1-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide | MS (ESI) m/e [M+1]⁺ = 594. |
| Q85 | 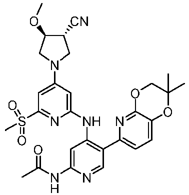 | N-(4-((4-(trans-3-cyano-4-methoxypyrrolidin-1-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide | MS (ESI) m/e [M+1]⁺ = 594. |
| Q86 | 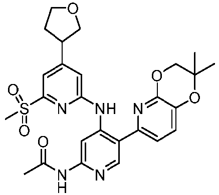 | N-(5-(2,2-dimethyl-2,3-dihydro-[1,4]dioxino[2,3-b]pyridin-6-yl)-4-((6-(methylsulfonyl)-4-(tetrahydrofuran-3-yl)pyridin-2-yl)amino)pyridin-2-yl)acetamide | MS (ESI) m/e [M+1]⁺ = 540. |
| Q86A | 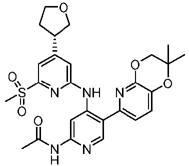 | (S)-N-(5-(2,2-dimethyl-2,3-dihydro- [1,4] dioxino [2,3-b]pyridin-6-yl)-4-((6-(methylsulfonyl)-4-(tetrahydrofuran-3-yl)pyridin-2-yl)amino)pyridin-2-yl)acetamide | MS (ESI) m/e [M+1]⁺ = 540. |
| Q87 | 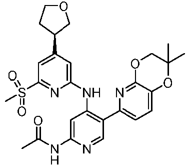 | (R)-N-(5-(2,2-dimethyl-2,3-dihydro- [1,4] dioxino [2,3-b]pyridin-6-yl)-4-((6-(methylsulfonyl)-4-(tetrahydrofuran-3-yl)pyridin-2-yl)amino)pyridin-2-yl)acetamide | MS (ESI) m/e [M+1]⁺ = 540. |
| Q88 | 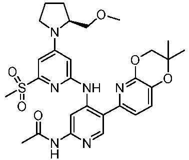 | (S)-N-(5-(2,2-dimethyl-2,3-dihydro- [1,4] dioxino [2,3-b]pyridin-6-yl)-4-((4-(2-(methoxymethyl)pyrrolidin-1-yl)-6-(methylsulfonyl)pyridin-2-yl)amino)pyridin-2-yl)acetamide | MS (ESI) m/e [M+1]⁺ = 583. |
| Q89 | 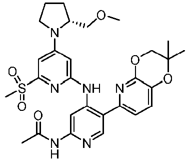 | (R)-N-(5-(2,2-dimethyl-2,3-dihydro-[1,4]dioxino[2,3-b]pyridin-6-yl)-4-((4-(2-(methoxymethyl)pyrrolidin-1-yl)-6-(methylsulfonyl)pyridin-2-yl)amino)pyridin-2-yl)acetamide | MS (ESI) m/e [M+1]⁺ = 583. |
| Q90 | 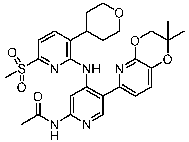 | N-(5-(2,2-dimethyl-2,3-dihydro- [1,4] dioxino [2,3-b]pyridin-6-yl)-4-((6-(methylsulfonyl)-3-(tetrahydro-2H-pyran-4-yl)pyridin-2-yl)amino)pyridin-2-yl)acetamide | MS (ESI) m/e [M+1]⁺ = 554. |
| Q91 | 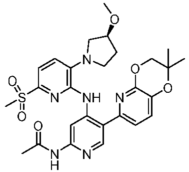 | (S)-N-(5-(2,2-dimethyl-2,3-dihydro- [1,4] dioxino [2,3-b]pyridin-6-yl)-4-((3-(3-methoxypyrrolidin-1-yl)-6-(methylsulfonyl)pyridin-2-yl)amino)pyridin-2-yl)acetamide | MS (ESI) m/e [M+1]⁺ = 569. |
| Q92 | 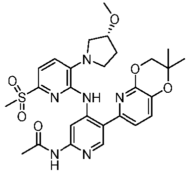 | (R)-N-(5-(2,2-dimethyl-2,3-dihydro- [1,4] dioxino [2,3-b]pyridin-6-yl)-4-((3-(3-methoxypyrrolidin-1-yl)-6-(methylsulfonyl)pyridin-2-yl)amino)pyridin-2-yl)acetamide | MS (ESI) m/e [M+1]⁺ = 569. |
| Q93 | 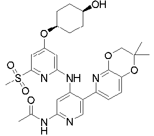 | cis-N-(5-(2,2-dimethyl-2,3-dihydro- [1,4] dioxino [2,3-b]pyridin-6-yl)-4-((4-((cis-4-hydroxycyclohexyl)oxy)-6-(methylsulfonyl)pyridin-2-yl)amino)pyridin-2-yl)acetamide | MS (ESI) m/e [M+H]⁺ = 584 |
| Q94 | 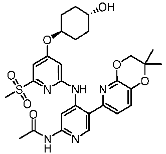 | N-(5-(2,2-dimethyl-2,3-dihydro- [1,4] dioxino [2,3-b]pyridin-6-yl)-4-((4-((trans-4-hydroxycyclohexyl)oxy)-6-(methylsulfonyl)pyridin-2-yl)amino)pyridin-2-yl)acetamide | MS (ESI) m/e [M+H]⁺ = 584 |
| Q95 | 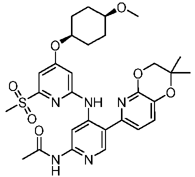 | N-(5-(2,2-dimethyl-2,3-dihydro- [1,4] dioxino [2,3-b]pyridin-6-yl)-4-((4-((cis-4-methoxycyclohexyl)oxy)-6-(methylsulfonyl)pyridin-2-yl)amino)pyridin-2-yl)acetamide | MS (ESI) m/e [M+H]⁺ = 598 |
| Q96 | 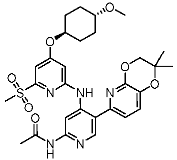 | N-(5-(2,2-dimethyl-2,3-dihydro- [1,4] dioxino [2,3-b]pyridin-6-yl)-4-((4-((trans-4-methoxycyclohexyl)oxy)-6-(methylsulfonyl)pyridin-2-yl)amino)pyridin-2-yl)acetamide | MS (ESI) m/e [M+H]⁺ = 598 |
| Q97 | 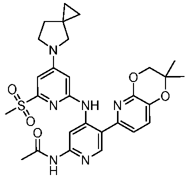 | N-(5-(2,2-dimethyl-2,3-dihydro- [1,4] dioxino [2,3-b]pyridin-6-yl)-4-((6-(methylsulfonyl)-4-(5-azaspiro[2.4]heptan-5-yl)pyridin-2-yl)amino)pyridin-2-yl)acetamide | MS (ESI) m/e [M+H]⁺ = 565 |
| Q98 | 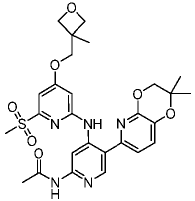 | N-(5-(2,2-dimethyl-2,3-dihydro- [1,4] dioxino [2,3-b]pyridin-6-yl)-4-((4-((3-methyloxetan-3-yl)methoxy)-6-(methylsulfonyl)pyridin-2-yl)amino)pyridin-2-yl)acetamide | ¹H NMR (400 MHz, DMSO-*d*₆) δ 11.66 (s, 1H), 10.50 (s, 1H), 9.00 (s, 1H), 8.60 (s, 1H), 7.57 (d, *J* = 8.0 Hz, 1H), 7.43 (d, *J =* 8.0 Hz, 1H), 7.18 (s, 1H), 6.72 (s, 1H), 4.52 (s, 2H), 4.32 (s, 2H), 4.26 (s, 4H), 3.42 (s, 3H), 2.11 (s, 3H), 1.38 - 1.36 (m, 9H).MS (ESI) m/e [M+1]⁺570. |
| | | | |
| Q99 | 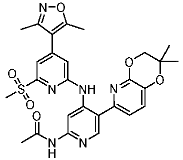 | N-(5-(2,2-dimethyl-2,3-dihydro-[1,4]dioxino[2,3-b]pyridin-6-yl)-4-((4-(3,5-dimethylisoxazol-4-yl)-6-(methylsulfonyl)pyridin-2-yl)amino)pyridin-2-yl)acetamide | MS (ESI) m/e [M+H]⁺ = 565 |
| Q100 | 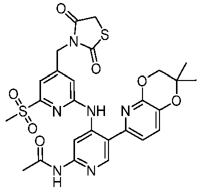 | N-(5-(2,2-dimethyl-2,3-dihydro- [1,4] dioxino [2,3-b]pyridin-6-yl)-4-((4-((2,4-dioxothiazolidin-3-yl)methyl)-6-(methylsulfonyl)pyridin-2-yl)amino)pyridin-2-yl)acetamide | MS (ESI) m/e [M+H]⁺ = 599 |
| Q101 | 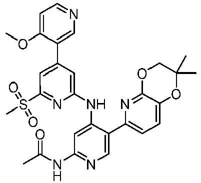 | N-(5-(2,2-dimethyl-2,3-dihydro- [1,4] dioxino [2,3-b]pyridin-6-yl)-4-((4-methoxy-6'-(methylsulfonyl)-[3, 4'-bipyridin]-2'-yl)amino)pyridin-2-yl)acetamide | MS (ESI) m/e [M+H]⁺ = 577 |
| Q102 | 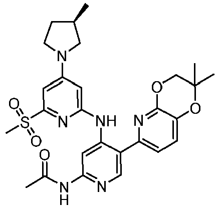 | (R)-N-(5-(2,2-dimethyl-2,3-dihydro- [1,4] dioxino [2,3-b]pyridin-6-yl)-4-((4-(3-methylpyrrolidin-1-yl)-6-(methylsulfonyl)pyridin-2-yl)amino)pyridin-2-yl)acetamide | MS (ESI) m/e [M+H]⁺ = 553 |
| Q103 | 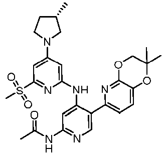 | (S)-N-(5-(2,2-dimethyl-2,3-dihydro- [1,4] dioxino [2,3-b]pyridin-6-yl)-4-((4-(3-methylpyrrolidin-1-yl)-6-(methylsulfonyl)pyridin-2-yl)amino)pyridin-2-yl)acetamide | MS (ESI) m/e [M+H]⁺ = 553 |
| Q104 | 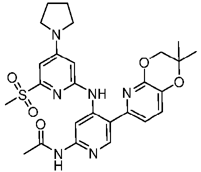 | N-(5-(2,2-dimethyl-2,3-dihydro- [1,4] dioxino [2,3-b]pyridin-6-yl)-4-((6-(methylsulfonyl)-4-(pyrrolidin-1-yl)pyridin-2-yl)amino)pyridin-2-yl)acetamide | MS (ESI) m/e [M+H]⁺ = 539 |
| Q105 | 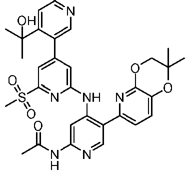 | N-(5-(2,2-dimethyl-2,3-dihydro- [1,4] dioxino [2,3-b]pyridin-6-yl)-4-((4-(2-hydroxypropan-2-yl)-6'-(methylsulfonyl)-[3, 4'-bipyridin]-2'-yl)amino)pyridin-2-yl)acetamide | MS (ESI) m/e [M+H]⁺ = 605 |
| Q106 | 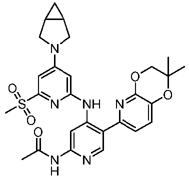 | N-(4-((4-(3-azabicyclo[3.1.0]hexan-3-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide | ¹H NMR (400 MHz, DMSO-d*₆*) δ 11.52 (s, 1H), 10.43 (s, 1H), 8.88 (s, 1H), 8.57 (s, 1H), 7.57 (d, J = 8.4 Hz, 1H), 7.43 (d, J = 8.3 Hz, 1H), 6.75 (d, J = 1.6 Hz, 1H), 6.16 (d, J = 1.6 Hz, 1H), 4.26 (s, 2H), 3.54 (d, J = 9.9 Hz, 2H), 3.44 (d, J = 9.3 Hz, 2H), 3.33 (s, 3H), 2.10 (s, 3H), 1.76-1.74 (m, 2H), 1.36 (s, 6H), 0.83-0.78 (m, 1H), 0.21-0.19 (m, 1H). MS (ESI) m/e [M+H]⁺ =551. |
| Q107 | 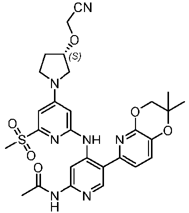 | (S)-N-(4-((4-(3-(cyanomethoxy)pyrrolidin-1-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide | MS (ESI) m/e [M+H]⁺ = 594 |
| Q108 | 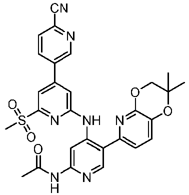 | N-(4-((6-cyano-6'-(methylsulfonyl)-[3, 4'-bipyridin]-2'-yl)amino)-5-(2,2-dimethyl-2,3-dihydro-[1,4]dioxino[2,3-b]pyridin-6-yl)pyridin-2-yl)acetamide | ¹H NMR (400 MHz, DMSO-*d₆*) δ 11.88 (s, 1H), 10.63 (s, 1H), 9.28 (d, J = 1.7 Hz, 1H), 9.12 (s, 1H), 8.70 (s, 1H), 8.61-8.59 (m, 1H), 8.31 (d, J = 8.2 Hz, 1H), 7.95 (s, 1H), 7.65-7.64 (m, 2H), 7.50 (d, J = 8.3 Hz, 1H), 4.29 (s, 2H), 3.54 (s, 3H), 2.19 (s, 3H), 1.42 (s, 6H). MS (ESI) m/e [M+H]⁺ =572. |
| Q109 | 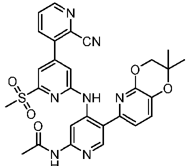 | N-(4-((2-cyano-6'-(methylsulfonyl)-[3, 4'-bipyridin]-2'-yl)amino)-5-(2,2-dimethyl-2,3-dihydro-[1,4]dioxino[2,3-b]pyridin-6-yl)pyridin-2-yl)acetamide | MS (ESI) m/e [M+H]⁺ = 572 |
| Q110 | 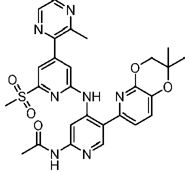 | N-(5-(2,2-dimethyl-2,3-dihydro- [1,4] dioxino [2,3-b]pyridin-6-yl)-4-((4-(3-methylpyrazin-2-yl)-6-(methylsulfonyl)pyridin-2-yl)amino)pyridin-2-yl)acetamide | ¹H NMR (400 MHz, DMSO-d*₆*) δ 12.21 (s, 1H), 10.55 (s, 1H), 9.20 (s, 1H), 8.67 (d, J = 3.6 Hz, 3H), 7.81 (s, 1H), 7.62 (d, J = 8.4 Hz, 1H), 7.44 (d, J = 8.4 Hz, 2H), 4.23 (s, 2H), 3.52 (s, 3H), 2.69 (s, 3H), 2.13 (s, 3H), 1.35 (s, 6H). MS (ESI) m/e [M+H]⁺ =562. |
| Q111 | 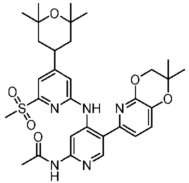 | N-(5-(2,2-dimethyl-2,3-dihydro- [1,4] dioxino [2,3-b]pyridin-6-yl)-4-((6-(methylsulfonyl)-4-(2,2,6,6-tetramethyltetrahydro-2H-pyran-4-yl)pyridin-2-yl)amino)pyridin-2-yl)acetamide | ¹H NMR (400 MHz, DMSO-*d₆*) δ 11.80 (s, 1H), 10.51 (s, 1H), 9.03 (s, 1H), 8.61 (s, 1H), 7.58 (d, J = 8.3 Hz, 1H), 7.51 (s, 1H), 7.44 (d, J = 8.3 Hz, 1H), 7.11 (s, 1H), 4.25 (s, 2H), 3.43 (s, 3H), 3.34-3.33 (m, 1H), 2.11 (s, 3H), 1.78 (d, J = 12.7 Hz, 2H), 1.41-1.39 (m, 2H), 1.37 (s, 6H), 1.31 (s, 6H), 1.17 (s, 6H). MS (ESI) m/e [M+H]⁺ =610. |
| Q112 | 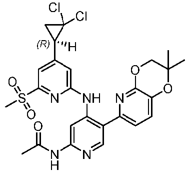 | (R)-N-(4-((4-(2,2-dichlorocyclopropyl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide | MS (ESI) m/e [M+H]⁺ = 578 |
| Q113 | 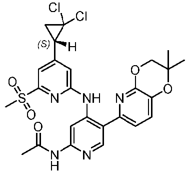 | (S)-N-(4-((4-(2,2-dichlorocyclopropyl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide | MS (ESI) m/e [M+H]⁺ = 578 |
| Q114 | 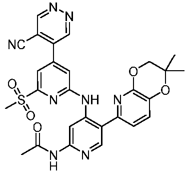 | N-(4-((4-(5-cyanopyridazin-4-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide | MS (ESI) m/e [M+H]⁺ = 573 |
| Q115 | 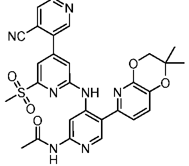 | N-(4-((4-cyano-6'-(methylsulfonyl)-[3, 4'-bipyridin]-2'-yl)amino)-5-(2,2-dimethyl-2,3-dihydro-[1,4]dioxino[2,3-b]pyridin-6-yl)pyridin-2-yl)acetamide | ¹H NMR (400 MHz, DMSO-*d₆*) δ 12.01 (s, 1H), 10.57 (s, 1H), 9.13 (s, 1H), 9.06 (s, 1H), 8.96 (d, J = 4 Hz, 1H), 8.65 (s, 1H), 8.09 (d, J = 8 Hz, 1H), 7.83 (s, 1H), 7.60 (d, J = 8 Hz, 1H), 7.48 - 7.38 (m, 2H), 4.20 (s, 2H), 3.50 (s, 3H), 2.13 (s, 3H), 1.34 (s, 6H). MS (ESI) m/e [M+H]⁺ = 572 |
| Q116 | 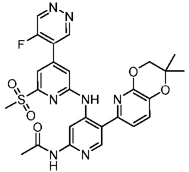 | N-(5-(2,2-dimethyl-2,3-dihydro- [1,4] dioxino [2,3-b]pyridin-6-yl)-4-((4-(5-fluoropyridazin-4-yl)-6-(methylsulfonyl)pyridin-2-yl)amino)pyridin-2-yl)acetamide | MS (ESI) m/e [M+H]⁺ = 566 |
| Q117 | 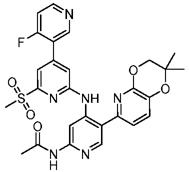 | N-(5-(2,2-dimethyl-2,3-dihydro- [1,4] dioxino [2,3-b]pyridin-6-yl)-4-((4-fluoro-6'-(methylsulfonyl)-[3,4'-bipyridin]-2'-yl)amino)pyridin-2-yl)acetamide | MS (ESI) m/e [M+H]⁺ = 565 |
| Q118 | 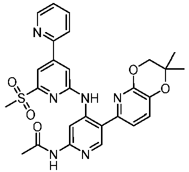 | N-(5-(2,2-dimethyl-2,3-dihydro- [1,4] dioxino [2,3-b]pyridin-6-yl)-4-((6'-(methylsulfonyl)-[2,4'-bipyridin]-2'-yl)amino)pyridin-2-yl)acetamide | MS (ESI) m/e [M+H]⁺ = 547 |
| Q119 | 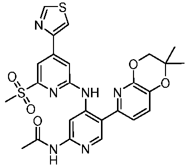 | N-(5-(2,2-dimethyl-2,3-dihydro- [1,4] dioxino [2,3-b]pyridin-6-yl)-4-((6-(methylsulfonyl)-4-(thiazol-4-yl)pyridin-2-yl)amino)pyridin-2-yl)acetamide | ¹H NMR (400 MHz, DMSO-*d₆*) δ 11.93 (s, 1H), 10.52 (s, 1H), 9.32 (d, J = 1.7 Hz, 1H), 9.11 (s, 1H), 8.73 (d, J = 1.7 Hz, 1H), 8.64 (s, 1H), 8.06 (s, 1H), 7.78 (s, 1H), 7.60 (d, J = 8 Hz, 1H), 7.44 (d, J = 8 Hz, 1H), 4.27 (s, 2H), 3.48 (s, 3H), 2.12 (s, 3H), 1.37 (s, 6H). MS (ESI) m/e [M+H]⁺ = 553 |
| Q120 | 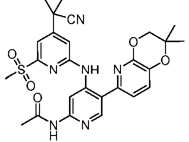 | N-(4-((4-(1-cyanocyclopropyl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide | ¹H NMR (400 MHz, DMSO-*d₆*) 11.84 (s, 1H), 10.54 (s, 1H), 8.99 (s, 1H), 8.61 (s, 1H), 7.56 (d, J = 8 Hz, 1H), 7.43 (d, *J =* 8.0 Hz, 1H), 7.29 (d, *J =* 4.0 Hz, 1H), 7.14 (d, *J* = 4.0 Hz, 1H), 4.23 (s, 2H), 3.43 (s, 3H), 2.11 (s, 3H), 2.03-1.93 (m, 2H), 1.79-1.96 (m, 2H), 1.36 (s, 6H). MS (ESI) m/e [M+H]⁺ = 535 |
| Q121 | 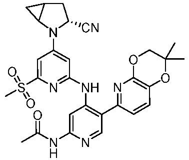 | N-(4-((4-((3R)-3-cyano-2-azabicyclo[3.1.0]hexan-2-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide | MS (ESI) m/e [M+H]⁺ =576 |
| Q122 | 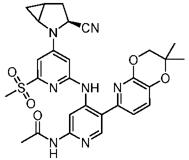 | N-(4-((4-((3S)-3-cyano-2-azabicyclo[3.1.0]hexan-2-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide | MS (ESI) m/e [M+H]⁺ =576 |
| Q123 | 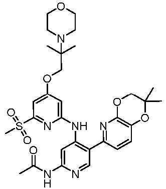 | N-(5-(2,2-dimethyl-2,3-dihydro-[1,4]dioxino[2,3-b]pyridin-6-yl)-4-((4-(2-methyl-2-morpholinopropoxy)-6-(methylsulfonyl)pyridin-2-yl)amino)pyridin-2-yl)acetamide | MS (ESI) m/e [M+H]⁺ = 627 |
| Q124 | 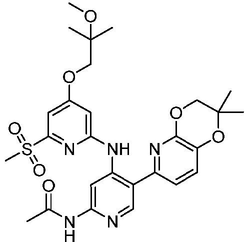 | N-(5-(2,2-dimethyl-2,3-dihydro- [1,4] dioxino [2,3-b]pyridin-6-yl)-4-((4-(2-methoxy-2-methylpropoxy)-6-(methylsulfonyl)pyridin-2-yl)amino)pyridin-2-yl)acetamide | MS (ESI) m/e [M+H]⁺ = 572 |
| Q125 | 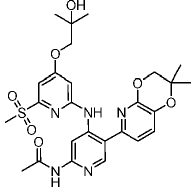 | N-(5-(2,2-dimethyl-2,3-dihydro- [1,4] dioxino [2,3-b]pyridin-6-yl)-4-((4-(2-hydroxy-2-methylpropoxy)-6-(methylsulfonyl)pyridin-2-yl)amino)pyridin-2-yl)acetamide | MS (ESI) m/e [M+H]⁺ = 558 |
| Q126 | 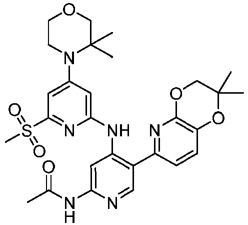 | N-(5-(2,2-dimethyl-2,3-dihydro-[1,4]dioxino[2,3-b]pyridin-6-yl)-4-((4-(3,3-dimethylmorpholino)-6-(methylsulfonyl)pyridin-2-yl)amino)pyridin-2-yl)acetamide | 1H NMR (400 MHz, DMSO-d6) δ 12.11 (s, 1H), 10.46 (s, 1H), 9.07 (s, 1H), 8.63 (s, 1H), 7.61 (d, J = 8.4 Hz, 1H), 7.44 (d, J = 8.3 Hz, 1H), 7.20 (d, J = 1.7 Hz, 1H), 6.68 (d, J = 1.8 Hz, 1H), 4.24 (s, 2H), 3.78-3.77 (m, 2H), 3.42-3.41 (m, 5H), 3.36-3.35 (m, 2H), 2.10 (s, 3H), 1.36 (s, 6H), 1.30 (s, 6H). MS (ESI) m/e [M+H]+ = 583. |
| Q127 | 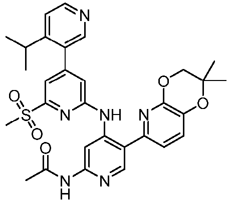 | N-(5-(2,2-dimethyl-2,3-dihydro- [1,4] dioxino [2,3-b]pyridin-6-yl)-4-((4-isopropyl-6'-(methylsulfonyl)-[3, 4'-bipyridin]-2'-yl)amino)pyridin-2-yl)acetamide | ¹H NMR (400 MHz, DMSO-*d₆*) δ 12.24 (s, 1H), 10.55 (s, 1H), 9.21 (s, 1H), 8.68 (s, 1H), 8.63 (d, J = 4 Hz, 1H), 8.47 (s, 1H), 7.64 (d, J = 8.4 Hz, 1H), 7.57 (d, J = 4 Hz, 1H), 7.53 - 7.36 (m, 2H), 7.11 (s, 1H), 4.18 (s, 2H), 3.53 (s, 3H), 3.01 - 2.96 (m, 1H), 2.13 (s, 3H), 1.33 (s, 6H), 1.21 (d, J = 8 Hz, 6H). MS (ESI) m/e [M+H]⁺ = 589 |
| Q128 | 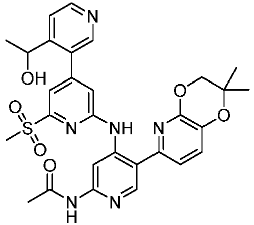 | N-(5-(2,2-dimethyl-2,3-dihydro- [1,4] dioxino [2,3-b]pyridin-6-yl)-4-((4-(1-hydroxyethyl)-6'-(methylsulfonyl)-[3, 4'-bipyridin]-2'-yl)amino)pyridin-2-yl)acetamide | MS (ESI) m/e [M+H]⁺ =591 |
| Q129 | 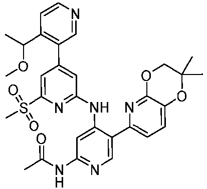 | N-(5-(2,2-dimethyl-2,3-dihydro- [1,4] dioxino [2,3-b]pyridin-6-yl)-4-((4-(1-methoxyethyl)-6'-(methylsulfonyl)-[3,4'-bipyridin]-2'-yl)amino)pyridin-2-yl)acetamide | MS (ESI) m/e [M+H]⁺ =605 |
| Q130 | 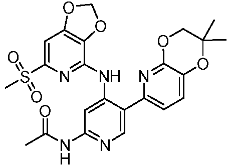 | N-(5-(2,2-dimethyl-2,3-dihydro-[1,4]dioxino[2,3-b]pyridin-6-yl)-4-((6-(methylsulfonyl)-[1,3]dioxolo[4,5-c]pyridin-4-yl)amino)pyridin-2-yl)acetamide | MS (ESI) m/e [M+H]⁺ = 514 |
| Q131 | 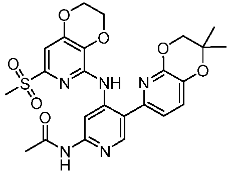 | N-(5-(2,2-dimethyl-2,3-dihydro- [1,4] dioxino [2,3-b]pyridin-6-yl)-4-((7-(methylsulfonyl)-2,3-dihydro- [1,4] dioxino [2,3-c]pyridin-5-yl)amino)pyridin-2-yl)acetamide | MS (ESI) m/e [M+H]⁺ = 528 |
| Q132 | 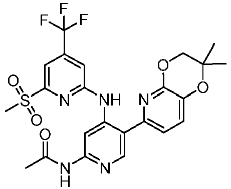 | N-(5-(2,2-dimethyl-2,3-dihydro-[1,4]dioxino[2,3-b]pyridin-6-yl)-4-((6-(methylsulfonyl)-4-(trifluoromethyl)pyridin-2-yl)amino)pyridin-2-yl)acetamide | MS (ESI) m/e [M+H]⁺ = 538 |
| Q133 | 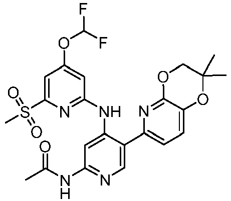 | N-(4-((4-(difluoromethoxy)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide | MS (ESI) m/e [M+H]⁺ = 536 |
| Q134 | 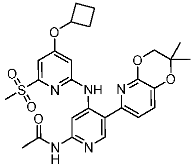 | N-(4-((4-cyclobutoxy-6-(methylsulfonyl)pyridin-2-yl)amino)-5-(2,2-dimethyl-2,3-dihydro-[1,4]dioxino[2,3-b]pyridin-6-yl)pyridin-2-yl)acetamide | MS (ESI) m/e [M+H]⁺ = 540 |
| Q135 | 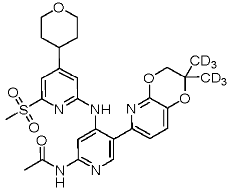 | N-(5-(2,2-bis(methyl-d3)-2,3-dihydro-[1,4]dioxino[2,3-b]pyridin-6-yl)-4-((6-(methylsulfonyl)-4-(tetrahydro-2H-pyran-4-yl)pyridin-2-yl)amino)pyridin-2-yl)acetamide | MS (ESI) m/e [M+H]+ = 560 |
| Q136 | 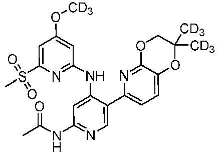 | N-(5-(2,2-bis(methyl-d3)-2,3-dihydro-[1,4]dioxino[2,3-b]pyridin-6-yl)-4-((4-(methoxy-d3)-6-(methylsulfonyl)pyridin-2-yl)amino)pyridin-2-yl)acetamide | MS (ESI) m/e [M+H]+ = 509. |

### Example R1: Synthesis of N-(5-(4,4-dimethyl-4,5-dihydrothiazol-2-yl)-4-((6-(methylsulfonyl)pyridin-2-yl)amino)pyridin-2-yl)acetamide

### Step 1: Synthesis of methyl 6-chloro-4-((4-methoxybenzyl)amino)nicotinate

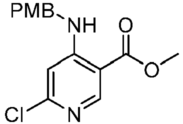

To a solution of methyl 4,6-dichloronicotinate (2 g, 9.7 mmol) and PMBNH₂ (2.1 g, 15.3 mmol) in DMF (20 mL) was added TEA (3 g, 29.7 mmol) at rt. The mixture was stirred at rt for 3h. Then H₂O was added and the mixture was extracted with EA. The organic layer was concentrated and the crude product was used in the next step without purification. MS (ESI) m/e [M+H] ⁺ = 307.

### Step 2: Synthesis of 6-chloro-4-((4-methoxybenzyl)amino)nicotinic acid

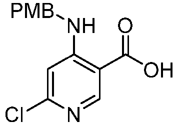

A solution of 6-chloro-4-((4-methoxybenzyl)amino)nicotinate (3.43 g, 11.2 mmol) in THF (100 mL) was added LiOH.H₂O (1.5 g, 35.7 mmol) in H₂O (50 mL). The mixture was stirred at rt for overnight. The mixture was concentrated, and the aqueous layer was acidified by HCl (1N). The white solid was collected by filtration and the filter cake was washed with H₂O, then dried to give the desired product (2.56 g, yield 90% for two steps). MS (ESI) m/e [M+H] ⁺ = 293.

### Step 3: Synthesis of 6-chloro-N-(1-hydroxy-2-methylpropan-2-yl)-4-((4-methoxybenzyl)amino)nicotinamide

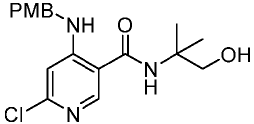

To a solution of 6-chloro-4-((4-methoxybenzyl)amino)nicotinic acid (300 mg, 1 mmol), 2-amino-2-methylpropan-1-ol (115.7 mg, 1.3 mmol), HOBT (148.5 mg, 1.1 mmol) and EDCI (383 mg, 2 mmol) in DMF (5 mL) was added DIEA (322.5 mg, 2.5 mmol) in one portion at rt. The mixture was stirred at rt for 5h. Then the reaction was quenched with H₂O and extracted with EA. The organic layer was concentrated. The crude product was purified on Prep-TLC (PE: EA = 1:1) to give the desired product (90 mg, yield 24.7%) as a colorless oil. MS (ESI) m/e [M+H] ⁺ = 364.

### Step 3: Synthesis of 6-acetamido-N-(1-hydroxy-2-methylpropan-2-yl)-4-((4-methoxybenzyl)amino)nicotinamide

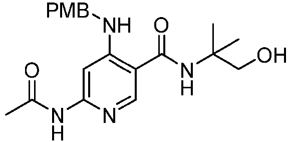

A slurry of 6-chloro-N-(1-hydroxy-2-methylpropan-2-yl)-4-((4-methoxybenzyl)amino)nicotinamide (90 mg, 0.25 mmol), acetamide (73.8 mg, 1.25 mmol), Pd₂(dba)₃ (22.9 mg, 0.025 mmol), Xantphos (28.9 mg, 0.05 mmol) and Cs₂CO₃ (163 mg, 0.05 mmol) in 1,4-dioxane (5 mL) was stirred at 120°C under N₂ for 5h.The mixture was cooled to rt and filtered through celite. The filtrate was concentrated under vacuum and the crude product was purified by Prep-TLC (DCM: MeOH = 15:1) to give the desired product as a colorless oil (230 mg, crude). MS (ESI) m/e [M+H] ⁺ = 387.

### Step 4: Synthesis of N-(5-(4,4-dimethyl-4,5-dihydrothiazol-2-yl)-4-((4-methoxybenzyl)amino)pyridin-2-yl)acetamide

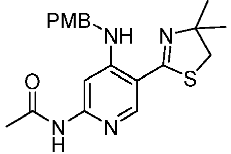

A solution of 6-acetamido-N-(1-hydroxy-2-methylpropan-2-yl)-4-((4-methoxybenzyl)amino)nicotinamide (230 mg, 0.6 mmol), Lawesson reagent (720 mg, 1.8 mmol) in dioxane (8 mL) was irritated at 100 °C under microwave for 1h. The mixture was cooled to rt and the solvent was removed under reduced pressure. The crude product was purified by silica gel column chromatography (DCM: MeOH = 15:1) to give the desired product as a yellow solid (80 mg, yield 83% for two steps). MS (ESI) m/e [M+H] ⁺ = 385.

### Step 5: Synthesis of N-(4-amino-5-(4,4-dimethyl-4,5-dihydrothiazol-2-yl)pyridin-2-yl)acetamide

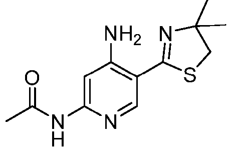

A solution of N-(5-(4,4-dimethyl-4,5-dihydrothiazol-2-yl)-4-((4-methoxybenzyl)amino)pyridin-2-yl)acetamide (80 mg, 0.21 mmol) in TFA (5 mL) was stirred at 50 °C for 16h and 70 °C for 5h. The mixture was cooled to rt and the solvent was removed under reduced pressure. The crude product was used in the next step without further purification. MS (ESI) m/e [M+H] ⁺ = 265.

### Step 6: Synthesis of N-(5-(4,4-dimethyl-4,5-dihydrothiazol-2-yl)-4-((6-(methylsulfonyl)pyridin-2-yl)amino)pyridin-2-yl)acetamide

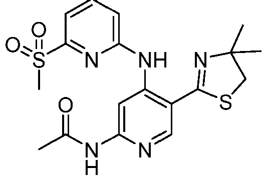

A solution of N-(4-amino-5-(4,4-dimethyl-4,5-dihydrothiazol-2-yl)pyridin-2-yl)acetamide (70 mg, 0.26 mmol), 2-bromo-6-(methylsulfonyl)pyridine (94 mg, 0.4 mmol), Pd₂(dba)₃ (24.7 mg, 0.027 mmol), Xantphos (31 mg, 0.053 mmol) and Cs₂CO₃ (173 mg, 0.53 mmol) in dioxane (5 mL) was stirred at 120 °C under N₂ for 7 h. The mixture was cooled to rt and filtered through celite. The filtrate was concentrated under reduced pressure. The crude product was purified by silica gel column chromatography (DCM: MeOH = 15:1) to give the desired product (3.19 mg, yield 3.62% for two steps) as a white solid. ¹H NMR (400 MHz, DMSO-*d*₆) *δ* 12.75 (s, 1H), 10.63 (s, 1H), 9.27 (s, 1H), 8.32 (s, 1H), 7.98 (t, *J* = 7.9 Hz, 1H), 7.56 (d, *J =* 7.4 Hz, 1H), 7.17 (d, *J =* 8.3 Hz, 1H), 3.44 (s, 3H), 3.22 (s, 2H), 2.06 (s, 3H), 1.44 (s, 6H). MS (ESI) m/e [M+H] ⁺ = 420.

The following Examples were prepared in a similar manner to the product Example R1:

| | | | |
|---|---|---|---|
| R2 | 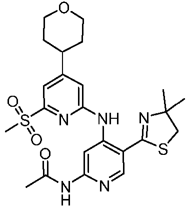 | N-(5-(4,4-dimethyl-4,5-dihydrothiazol-2-yl)-4-((6-(methylsulfonyl)-4-(tetrahydro-2H-pyran-4-yl)pyridin-2-yl)amino)pyridin-2-yl)acetamide | MS (ESI) m/e [M+H]⁺ = 504 |
| | | | |

Furtherly, all the following compounds can be obtained by similar methods of preparing the example compounds disclosed in the present disclosure.

| **#** | | **Compound** | **Chemical Name** | **Molecular Weight** |
|---|---|---|---|---|
| **1.** | | 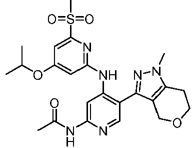 | N-(4-((4-isopropoxy-6-(methylsulfonyl)pyridin-2-yl)amino)-5-(1-methyl-1,3a,4,6,7,7a-hexahydropyrano[4,3-c]pyrazol-3-yl)pyridin-2-yl)acetamide | Molecular Weight: 502 |
| **2.** | | 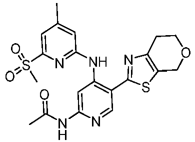 | 4-(5-(6,7-dihydro-4H-pyrano[4,3-d]thiazol-2-yl)-4-((4-methyl-6-(methylsulfonyl)pyridin-2-yl)amino)pyridin-2-yl)acetamide | Molecular Weight: 459 |
| **3.** | | 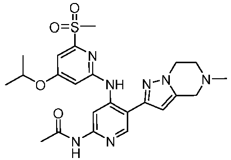 | N-(4-((4-isopropoxy-6-(methylsulfonyl)pyridin-2-yl)amino)-5-(5-methyl-4,5,6,7-tetrahydropyrazolo[1,5-a]pyrazin-2-yl)pyridin-2-yl)acetamide | Molecular Weight: 499 |
| **4.** | | 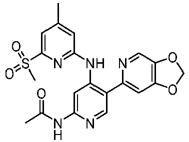 | 4-(5-([1,3]dioxolo[4,5-c]pyridin-6-yl)-4-((4-methyl-6-(methylsulfonyl)pyridin-2-yl)amino)pyridin-2-yl)acetamide | Molecular Weight: 441 |
| **5.** | | 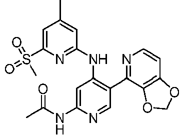 | N-(5-([1,3]dioxolo[4,5-c]pyridin-4-yl)-4-((4-methyl-6-(methylsulfonyl)pyridin-2-yl)amino)pyridin-2-yl)acetamide | Molecular Weight: 441 |
| **6.** | | 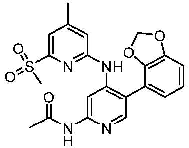 | N-(5-(benzo[d] [1,3]dioxol-4-yl)-4-((4-methyl-6-(methylsulfonyl)pyridin-2-yl)amino)pyridin-2-yl)acetamide | Molecular Weight: 440 |
| **7.** | | 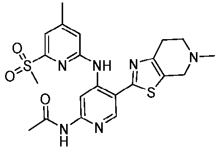 | N-(5-(5-methyl-4,5,6,7-tetrahydrothiazolo[5,4-c]pyridin-2-yl)-4-((4-methyl-6-(methylsulfonyl)pyridin-2-yl)amino)pyridin-2-yl)acetamide | Molecular Weight: 472 |
| **8.** | | 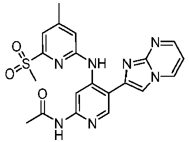 | N-(5-(imidazo[1,2-a]pyrimidin-2-yl)-4-((4-methyl-6-(methylsulfonyl)pyridin-2-yl)amino)pyridin-2-yl)acetamide | Molecular Weight: 437 |
| **9.** | | 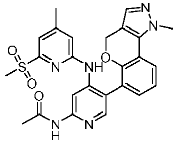 | N-(5-(1-methyl-1,4-dihydrochromeno[4,3-c]pyrazol-6-yl)-4-((4-methyl-6-(methylsulfonyl)pyridin-2-yl)amino)pyridin-2-yl)acetamide | Molecular Weight: 504 |
| **10.** | | 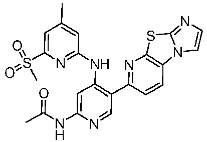 | N-(5-(imidazo[2',1':2,3]thiazolo[5,4-b]pyridin-7-yl)-4-((4-methyl-6-(methylsulfonyl)pyridin-2-yl)amino)pyridin-2-yl)acetamide | Molecular Weight: 493 |
| **11.** | | 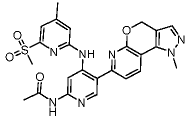 | N-(5-(1-methyl-1,4-dihydropyrazolo[3',4':4,5]pyrano[2,3-b]pyridin-7-yl)-4-((4-methyl-6-(methylsulfonyl)pyridin-2-yl)amino)pyridin-2-yl)acetamide | Molecular Weight: 505 |
| **12.** | | 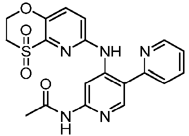 | N-(4'-((4,4-dioxido-2,3-dihydro-[1,4]oxathiino[3,2-b]pyridin-6-yl)amino)-[2,3'-bipyridin]-6'-yl)acetamide | Molecular Weight: 411 |
| **13.** | | 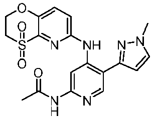 | N-(4-((4,4-dioxido-2,3-dihydro-[1,4]oxathiino[3,2-b]pyridin-6-yl)amino)-5-(1-methyl-1H-pyrazol-3-yl)pyridin-2-yl)acetamide | Molecular Weight: 414 |
| **14.** | | 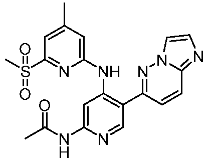 | N-(5-(imidazo[1,2-b]pyridazin-6-yl)-4-((4-methyl-6-(methylsulfonyl)pyridin-2-yl)amino)pyridin-2-yl)acetamide | Molecular Weight: 437 |
| **15.** | | 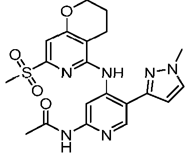 | N-(5-(1-methyl-1H-pyrazol-3-yl)-4-((7-(methylsulfonyl)-3,4-dihydro-2H-pyrano[3,2-c]pyridin-5-yl)amino)pyridin-2-yl)acetamide | Molecular Weight: 442 |
| **16.** | | 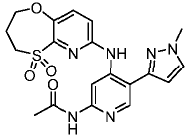 | N-(4-((5,5-dioxido-3,4-dihydro-2H-[1,4]oxathiepino[3,2-b]pyridin-7-yl)amino)-5-(1-methyl-1H-pyrazol-3-yl)pyridin-2-yl)acetamide | Molecular Weight: 428 |
| **17.** | | 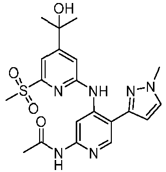 | N-(4-((4-(2-hydroxypropan-2-yl)-6-(methylsulfonyl)pyridin-2-yl)amino)-5-(1-methyl-1H-pyrazol-3-yl)pyridin-2-yl)acetamide | Molecular Weight: 444 |
| **18.** | | 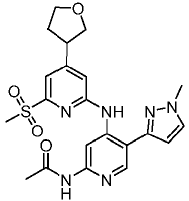 | N-(5-(1-methyl-1H-pyrazol-3-yl)-4-((6-(methylsulfonyl)-4-(tetrahydrofuran-3-yl)pyridin-2-yl)amino)pyridin-2-yl)acetamide | Molecular Weight: 456 |
| **19.** | | 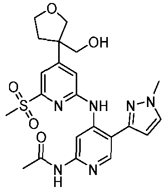 | N-(4-((4-(3-(hydroxymethyl)tetrahydrofuran-3-yl)-6-(methylsulfonyl)pyridin-2-yl)amino)-5-(1-methyl-1H-pyrazol-3-yl)pyridin-2-yl)acetamide | Molecular Weight: 486 |
| **20.** | | 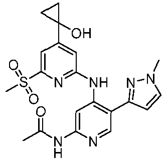 | N-(4-((4-(1-hydroxycyclopropyl)-6-(methylsulfonyl)pyridin-2-yl)amino)-5-(1-methyl-1H-pyrazol-3-yl)pyridin-2-yl)acetamide | Molecular Weight: 442 |
| **21.** | | 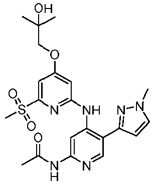 | N-(4-((4-(2-hydroxy-2-methylpropoxy)-6-(methylsulfonyl)pyridin-2-yl)amino)-5-(1-methyl-1H-pyrazol-3-yl)pyridin-2-yl)acetamide | Molecular Weight: 474 |
| **22.** | | 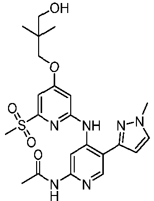 | N-(4-((4-(3-hydroxy-2,2-dimethylpropoxy)-6-(methylsulfonyl)pyridin-2-yl)amino)-5-(1-methyl-1H-pyrazol-3-yl)pyridin-2-yl)acetamide | Molecular Weight: 488 |
| **23.** | | 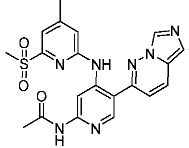 | N-(5-(imidazo[1,5-b]pyridazin-2-yl)-4-((4-methyl-6-(methylsulfonyl)pyridin-2-yl)amino)pyridin-2-yl)acetamide | Molecular Weight: 437 |
| **24.** | | 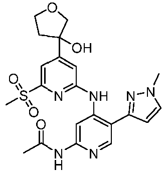 | N-(4-((4-(3-hydroxytetrahydrofuran-3-yl)-6-(methylsulfonyl)pyridin-2-yl)amino)-5-(1-methyl-1H-pyrazol-3-yl)pyridin-2-yl)acetamide | Molecular Weight: 472 |
| **25.** | | 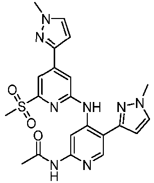 | N-(5-(1-methyl-1H-pyrazol-3-yl)-4-((4-(1-methyl-1H-pyrazol-3-yl)-6-(methylsulfonyl)pyridin-2-yl)amino)pyridin-2-yl)acetamide | Molecular Weight: 466 |
| **26.** | | 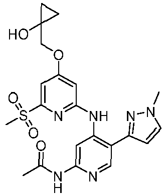 | N-(4-((4-((1-hydroxycyclopropyl)methoxy)-6-(methylsulfonyl)pyridin-2-yl)amino)-5-(1-methyl-1H-pyrazol-3-yl)pyridin-2-yl)acetamide | Molecular Weight: 472 |
| **27.** | | 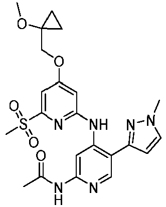 | N-(4-((4-((1-methoxycyclopropyl)methoxy)-6-(methylsulfonyl)pyridin-2-yl)amino)-5-(1-methyl-1H-pyrazol-3-yl)pyridin-2-yl)acetamide | Molecular Weight: 486 |
| **28.** | | 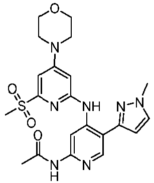 | N-(5-(1-methyl-1H-pyrazol-3-yl)-4-((6-(methylsulfonyl)-4-morpholinopyridin-2-yl)amino)pyridin-2-yl)acetamide | Molecular Weight: 471 |
| **29.** | | 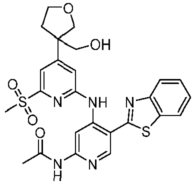 | N-(5-(benzo[d]thiazol-2-yl)-4-((4-(3-(hydroxymethyl)tetrahydrofuran-3-yl)-6-(methylsulfonyl)pyridin-2-yl)amino)pyridin-2-yl)acetamide | Molecular Weight: 540 |
| **30.** | | 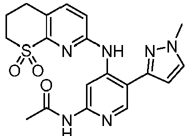 | N-(4-((1,1-dioxido-3,4-dihydro-2H-thiopyrano [2,3-b]pyridin-7-yl)amino)-5-(1-methyl-1H-pyrazol-3-yl)pyridin-2-yl)acetamide | Molecular Weight: 412 |
| **31.** | | 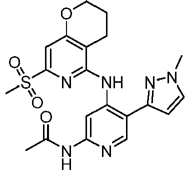 | N-(5-(1-methyl-1H-pyrazol-3-yl)-4-((7-(methylsulfonyl)-3,4-dihydro-2H-pyrano[3,2-c]pyridin-5-yl)amino)pyridin-2-yl)acetamide | Molecular Weight: 442 |
| **32.** | | 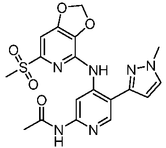 | N-(5-(1-methyl-1H-pyrazol-3-yl)-4-((6-(methylsulfonyl)-[1,3]dioxolo[4,5-c]pyridin-4-yl)amino)pyridin-2-yl)acetamide | Molecular Weight: 430 |
| **33.** | | 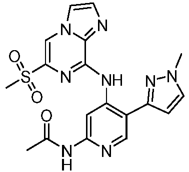 | N-(5-(1-methyl-1H-pyrazol-3-yl)-4-((6-(methylsulfonyl)imidazo[1,2-a]pyrazin-8-yl)amino)pyridin-2-yl)acetamide | Molecular Weight: 423 |
| **34.** | | 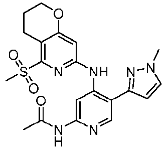 | N-(5-(1-methyl-1H-pyrazol-3-yl)-4-((5-(methylsulfonyl)-3,4-dihydro-2H-pyrano[3,2-c]pyridin-7-yl)amino)pyridin-2-yl)acetamide | Molecular Weight: 442 |
| **35.** | | 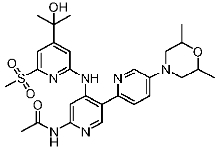 | N-(5-(2,6-dimethylmorpholino)-4'-((4-(2-hydroxypropan-2-yl)-6-(methylsulfonyl)pyridin-2-yl)amino)-[2,3'-bipyridin]-6'-yl)acetamide | Molecular Weight: 554 |
| **36.** | | 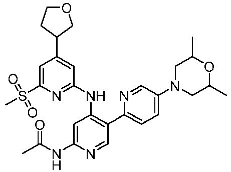 | N-(5-(2,6-dimethylmorpholino)-4'-((6-(methylsulfonyl)-4-(tetrahydrofuran-3-yl)pyridin-2-yl)amino)-[2,3'-bipyridin]-6'-yl)acetamide | Molecular Weight: 566 |
| **37.** | | 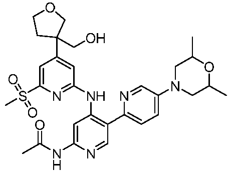 | N-(5-(2,6-dimethylmorpholino)-4'-((4-(3-(hydroxymethyl)tetrahydrofuran-3-yl)-6-(methylsulfonyl)pyridin-2-yl)amino)-[2,3'-bipyridin]-6'-yl)acetamide | Molecular Weight: 596 |
| **38.** | | 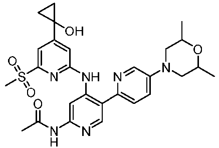 | N-(5-(2,6-dimethylmorpholino)-4'-((4-(1-hydroxycyclopropyl)-6-(methylsulfonyl)pyridin-2-yl)amino)-[2,3'-bipyridin]-6'-yl)acetamide | Molecular Weight: 552 |
| **39.** | | 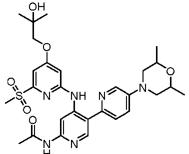 | N-(5-(2,6-dimethylmorpholino)-4'-((4-(2-hydroxy-2-methylpropoxy)-6-(methylsulfonyl)pyridin-2-yl)amino)-[2,3'-bipyridin]-6'-yl)acetamide | Molecular Weight: 584 |
| **40.** | | 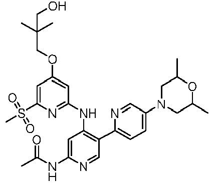 | N-(5-(2,6-dimethylmorpholino)-4'-((4-(3-hydroxy-2,2-dimethylpropoxy)-6-(methylsulfonyl)pyridin-2-yl)amino)-[2,3'-bipyridin]-6'-yl)acetamide | Molecular Weight: 598 |
| **41.** | | 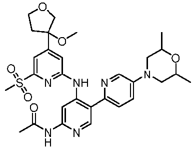 | N-(5-(2,6-dimethylmorpholino)-4'-((4-(3-methoxytetrahydrofuran-3-yl)-6-(methylsulfonyl)pyridin-2-yl)amino)-[2,3'-bipyridin]-6'-yl)acetamide | Molecular Weight: 596.70 |
| **42.** | | 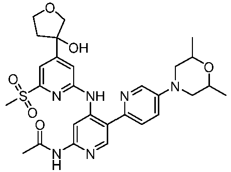 | N-(5-(2,6-dimethylmorpholino)-4'-((4-(3-hydroxytetrahydrofuran-3-yl)-6-(methylsulfonyl)pyridin-2-yl)amino)-[2,3'-bipyridin]-6'-yl)acetamide | Molecular Weight: 582 |
| **43.** | | 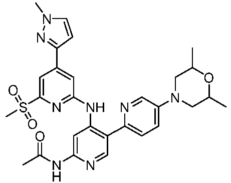 | N-(5-(2,6-dimethylmorpholino)-4'-((4-(1-methyl-1H-pyrazol-3-yl)-6-(methylsulfonyl)pyridin-2-yl)amino)-[2,3'-bipyridin]-6'-yl)acetamide | Molecular Weight: 576 |
| **44.** | | 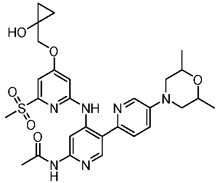 | N-(5-(2,6-dimethylmorpholino)-4'-((4-((1-hydroxycyclopropyl)methoxy)-6-(methylsulfonyl)pyridin-2-yl)amino)-[2,3'-bipyridin]-6'-yl)acetamide | Molecular Weight: 582 |
| **45.** | | 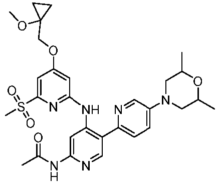 | N-(5-(2,6-dimethylmorpholino)-4'-((4-((1-methoxycyclopropyl)methoxy)-6-(methylsulfonyl)pyridin-2-yl)amino)-[2,3'-bipyridin]-6'-yl)acetamide | Molecular Weight: 596 |
| **46.** | | 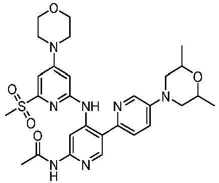 | N-(5-(2,6-dimethylmorpholino)-4'-((6-(methylsulfonyl)-4-morpholinopyridin-2-yl)amino)-[2,3'-bipyridin]-6'-yl)acetamide | Molecular Weight: 581 |
| **47.** | | 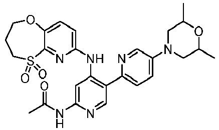 | N-(5-(2,6-dimethylmorpholino)-4'-((5,5-dioxido-3,4-dihydro-2H-[1,4]oxathiepino[3,2-b]pyridin-7-yl)amino)-[2,3'-bipyridin]-6'-yl)acetamide | Molecular Weight: 538 |
| **48.** | | 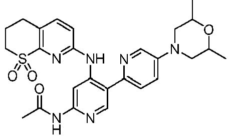 | N-(5-(2,6-dimethylmorpholino)-4'-((1,1-dioxido-3,4-dihydro-2H-thiopyrano [2,3-b]pyridin-7-yl)amino)-[2,3'-bipyridin]-6'-yl)acetamide | Molecular Weight: 522 |
| **49.** | | 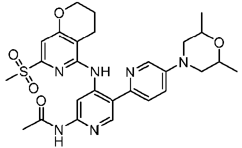 | N-(5-(2,6-dimethylmorpholino)-4'-((7-(methylsulfonyl)-3,4-dihydro-2H-pyrano[3,2-c]pyridin-5-yl)amino)-[2,3'-bipyridin]-6'-yl)acetamide | Molecular Weight: 552 |
| **50.** | | 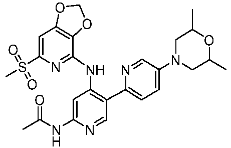 | N-(5-(2,6-dimethylmorpholino)-4'-((6-(methylsulfonyl)-[1,3]dioxolo[4,5-c]pyridin-4-yl)amino)-[2,3'-bipyridin]-6'-yl)acetamide | Molecular Weight: 540 |
| **51.** | | 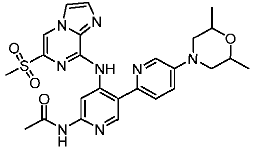 | N-(5-(2,6-dimethylmorpholino)-4'-((6-(methylsulfonyl)imidazo[1,2-a]pyrazin-8-yl)amino)-[2,3'-bipyridin]-6'-yl)acetamide | Molecular Weight: 536 |
| **52.** | | 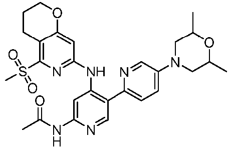 | N-(5-(2,6-dimethylmorpholino)-4'-((5-(methylsulfonyl)-3,4-dihydro-2H-pyrano[3,2-c]pyridin-7-yl)amino)-[2,3'-bipyridin]-6'-yl)acetamide | Molecular Weight: 552 |
| **53.** | | 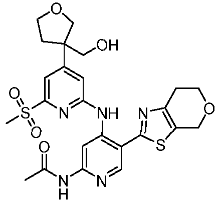 | N-(5-(6,7-dihydro-4H-pyrano[4,3-d]thiazol-2-yl)-4-((4-(3-(hydroxymethyl)tetrahydrofuran-3-yl)-6-(methylsulfonyl)pyridin-2-yl)amino)pyridin-2-yl)acetamide | Molecular Weight: 545 |
| **54.** | | 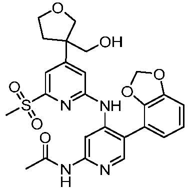 | N-(5-(benzo[d] [1,3]dioxol-4-yl)-4-((4-(3-(hydroxymethyl)tetrahydrofuran-3-yl)-6-(methylsulfonyl)pyridin-2-yl)amino)pyridin-2-yl)acetamide | Molecular Weight: 526 |
| **55.** | | 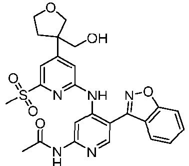 | N-(5-(benzo[d]isoxazol-3-yl)-4-((4-(3-(hydroxymethyl)tetrahydrofuran-3-yl)-6-(methylsulfonyl)pyridin-2-yl)amino)pyridin-2-yl)acetamide | Molecular Weight: 523 |
| **56.** | | 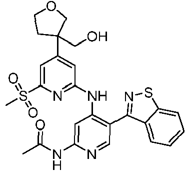 | N-(5-(benzo[d]isothiazol-3-yl)-4-((4-(3-(hydroxymethyl)tetrahydrofuran-3-yl)-6-(methylsulfonyl)pyridin-2-yl)amino)pyridin-2-yl)acetamide | Molecular Weight: 539 |
| **57.** | | 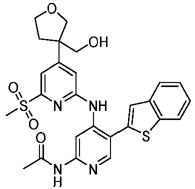 | N-(5-(benzo[b]thiophen-2-yl)-4-((4-(3-(hydroxymethyl)tetrahydrofuran-3-yl)-6-(methylsulfonyl)pyridin-2-yl)amino)pyridin-2-yl)acetamide | Molecular Weight: 538 |
| **58.** | | 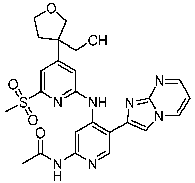 | N-(4-((4-(3-(hydroxymethyl)tetrahydrofuran-3-yl)-6-(methylsulfonyl)pyridin-2-yl)amino)-5-(imidazo[1,2-a]pyrimidin-2-yl)pyridin-2-yl)acetamide | Molecular Weight: 523 |
| **59.** | | 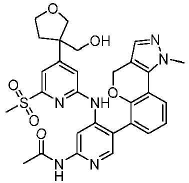 | N-(4-((4-(3-(hydroxymethyl)tetrahydrofuran-3-yl)-6-(methylsulfonyl)pyridin-2-yl)amino)-5-(1-methyl-1,4-dihydrochromeno[4,3-c]pyrazol-6-yl)pyridin-2-yl)acetamide | Molecular Weight: 590 |
| **60.** | | 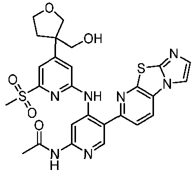 | N-(4-((4-(3-(hydroxymethyl)tetrahydrofuran-3-yl)-6-(methylsulfonyl)pyridin-2-yl)amino)-5-(imidazo[2',1':2,3]thiazolo[5,4-b]pyridin-7-yl)pyridin-2-yl)acetamide | Molecular Weight: 579 |
| **61.** | | 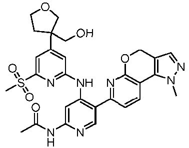 | N-(4-((4-(3-(hydroxymethyl)tetrahydrofuran-3-yl)-6-(methylsulfonyl)pyridin-2-yl)amino)-5-(1-methyl-1,4-dihydropyrazolo[3',4':4,5]pyrano[2,3-b]pyridin-7-yl)pyridin-2-yl)acetamide | Molecular Weight: 591 |
| **62.** | | 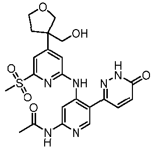 | N-(4-((4-(3-(hydroxymethyl)tetrahydrofuran-3-yl)-6-(methylsulfonyl)pyridin-2-yl)amino)-5-(6-oxo-1,6-dihydropyridazin-3-yl)pyridin-2-yl)acetamide | Molecular Weight: 500 |
| **63.** | | 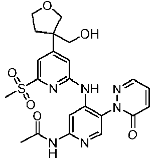 | N-(4-((4-(3-(hydroxymethyl)tetrahydrofuran-3-yl)-6-(methylsulfonyl)pyridin-2-yl)amino)-5-(6-oxopyridazin-1(6H)-yl)pyridin-2-yl)acetamide | Molecular Weight: 500 |
| **64.** | | 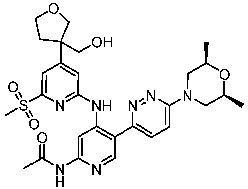 | N-(5-(6-((2S,6R)-2,6-dimethylmorpholino)pyridazin-3-yl)-4-((4-(3-(hydroxymethyl)tetrahydrofuran-3-yl)-6-(methylsulfonyl)pyridin-2-yl)amino)pyridin-2-yl)acetamide | Molecular Weight: 597 |
| **65.** | | 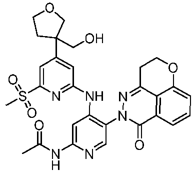 | N-(4-((4-(3-(hydroxymethyl)tetrahydrofuran-3-yl)-6-(methylsulfonyl)pyridin-2-yl)amino)-5-(3-oxo-8,9-dihydropyrano[4,3,2-de]phthalazin-2(3H)-yl)pyridin-2-yl)acetamide | Molecular Weight: 592 |
| **66.** | | 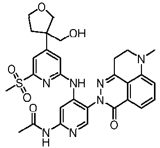 | N-(4-((4-(3-(hydroxymethyl)tetrahydrofuran-3-yl)-6-(methylsulfonyl)pyridin-2-yl)amino)-5-(7-methyl-3-oxo-8,9-dihydro-3H-pyrido[4,3,2-de]phthalazin-2(7H)-yl)pyridin-2-yl)acetamide | Molecular Weight: 605 |
| **67.** | | 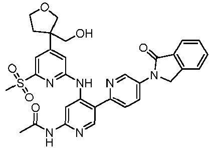 | N-(4'-((4-(3-(hydroxymethyl)tetrahydrofuran-3-yl)-6-(methylsulfonyl)pyridin-2-yl)amino)-5-(1-oxoisoindolin-2-yl)-[2,3'-bipyridin]-6'-yl)acetamide | Molecular Weight: 614 |
| **68.** | | 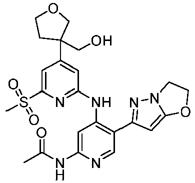 | N-(5-(2,3-dihydropyrazolo[5,1-b]oxazol-6-yl)-4-((4-(3-(hydroxymethyl)tetrahydrofuran-3-yl)-6-(methylsulfonyl)pyridin-2-yl)amino)pyridin-2-yl)acetamide | Molecular Weight: 514 |
| **69.** | | 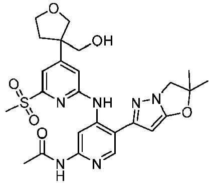 | N-(5-(2,2-dimethyl-2,3-dihydropyrazolo[5,1-b]oxazol-6-yl)-4-((4-(3-(hydroxymethyl)tetrahydrofuran-3-yl)-6-(methylsulfonyl)pyridin-2-yl)amino)pyridin-2-yl)acetamide | Molecular Weight: 542 |
| **70.** | | 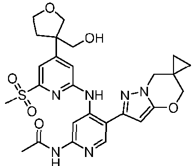 | N-(4-((4-(3-(hydroxymethyl)tetrahydrofuran-3-yl)-6-(methylsulfonyl)pyridin-2-yl)amino)-5-(5'H,7'H-spiro[cyclopropane-1,6'- | Molecular Weight: 554 |
| | | | pyrazolo[5, 1-b] [1,3]oxazin]-2'-yl)pyridin-2-yl)acetamide | |
| **71.** | | 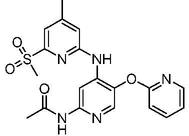 | N-(4-((4-methyl-6-(methylsulfonyl)pyridin-2-yl)amino)-5-(pyridin-2-yloxy)pyridin-2-yl)acetamide | Molecular Weight: 413 |
| **72.** | | 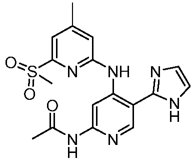 | N-(5-(1H-imidazol-2-yl)-4-((4-methyl-6-(methylsulfonyl)pyridin-2-yl)amino)pyridin-2-yl)acetamide | Molecular Weight: 386 |
| **73.** | | 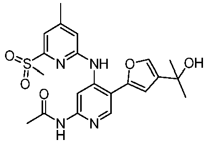 | N-(5-(4-(2-hydroxypropan-2-yl)furan-2-yl)-4-((4-methyl-6-(methylsulfonyl)pyridin-2-yl)amino)pyridin-2-yl)acetamide | Molecular Weight: 444 |
| **74.** | | 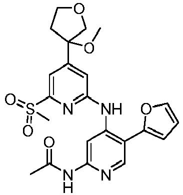 | N-(5-(furan-2-yl)-4-((4-(3-methoxytetrahydrofuran-3-yl)-6-(methylsulfonyl)pyridin-2-yl)amino)pyridin-2-yl)acetamide | Molecular Weight: 472 |
| **75.** | | 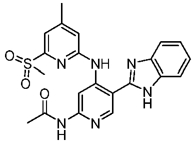 | N-(5-(1H-benzo[d]imidazol-2-yl)-4-((4-methyl-6-(methylsulfonyl)pyridin-2-yl)amino)pyridin-2-yl)acetamide | Molecular Weight: 436 |
| **76.** | | 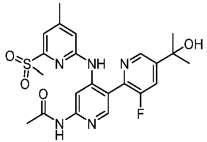 | N-(3-fluoro-5-(2-hydroxypropan-2-yl)-4'-((4-methyl-6-(methylsulfonyl)pyridin-2-yl)amino)-[2,3'-bipyridin]-6'-yl)acetamide | Molecular Weight: 473 |
| **77.** | | 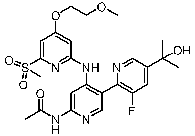 | N-(3-fluoro-5-(2-hydroxypropan-2-yl)-4'-((4-methyl-6-(methylsulfonyl)pyridin-2-yl)amino)-[2,3'-bipyridin]-6'-yl)acetamide | Molecular Weight: 473 |
| **78.** | | 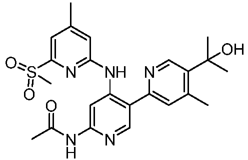 | N-(5-(2-hydroxypropan-2-yl)-4-methyl-4'-((4-methyl-6-(methylsulfonyl)pyridin-2-yl)amino)-[2,3'-bipyridin]-6'-yl)acetamide | Molecular Weight: 469 |
| **79.** | | 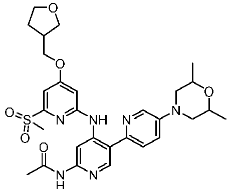 | N-(5-(2,6-dimethylmorpholino)-4'-((6-(methylsulfonyl)-4-((tetrahydrofuran-3-yl)methoxy)pyridin-2-yl)amino)-[2,3'-bipyridin]-6'-yl)acetamide | Molecular Weight: 596 |
| **80.** | | 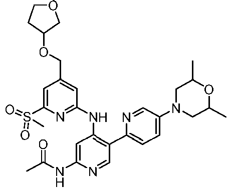 | N-(5-(2,6-dimethylmorpholino)-4'-((6-(methylsulfonyl)-4-(((tetrahydrofuran-3-yl)oxy)methyl)pyridin-2-yl)amino)-[2,3'-bipyridin]-6'-yl)acetamide | Molecular Weight: 596 |
| **81.** | | 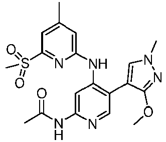 | N-(5-(3-methoxy-1-methyl-1H-pyrazol-4-yl)-4-((4-methyl-6-(methylsulfonyl)pyridin-2-yl)amino)pyridin-2-yl)acetamide | Molecular Weight: 430 |
| **82.** | | 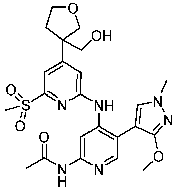 | N-(4-((4-(3-(hydroxymethyl)tetrahydrofuran-3-yl)-6-(methylsulfonyl)pyridin-2-yl)amino)-5-(3-methoxy-1-methyl-1H-pyrazol-4-yl)pyridin-2-yl)acetamide | Molecular Weight: 516 |
| **83.** | | 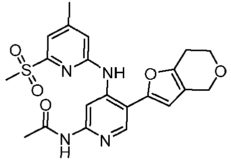 | N-(5-(6,7-dihydro-4H-furo[3,2-c]pyran-2-yl)-4-((4-methyl-6-(methylsulfonyl)pyridin-2-yl)amino)pyridin-2-yl)acetamide | Molecular Weight: 442 |
| **84.** | | 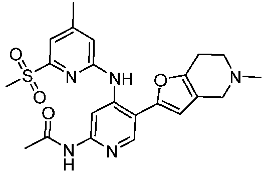 | N-(5-(5-methyl-4,5,6,7-tetrahydrofuro[3,2-c]pyridin-2-yl)-4-((4-methyl-6-(methylsulfonyl)pyridin-2-yl)amino)pyridin-2-yl)acetamide | Molecular Weight: 455 |
| **85.** | | 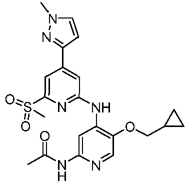 | N-(5-(cyclopropylmethoxy)-4-((4-(1-methyl-1H-pyrazol-3-yl)-6-(methylsulfonyl)pyridin-2-yl)amino)pyridin-2-yl)acetamide | Molecular Weight: 456 |
| **86.** | | 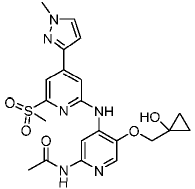 | N-(5-((1-hydroxycyclopropyl)methoxy)-4-((4-(1-methyl-1H-pyrazol-3-yl)-6-(methylsulfonyl)pyridin-2-yl)amino)pyridin-2-yl)acetamide | Molecular Weight: 472 |
| **87.** | | 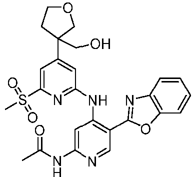 | N-(5-(benzo[d]oxazol-2-yl)-4-((4-(3-(hydroxymethyl)tetrahydrofuran-3-yl)-6-(methylsulfonyl)pyridin-2-yl)amino)pyridin-2-yl)acetamide | Molecular Weight: 524 |
| **88.** | | 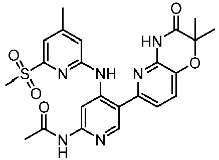 | N-(5-(2,2-dimethyl-3-oxo-3,4-dihydro-2H-pyrido[3,2-b][1,4]oxazin-6-yl)-4-((4-methyl-6-(methylsulfonyl)pyridin-2-yl)amino)pyridin-2-yl)acetamide | Molecular Weight: 497 |
| **89.** | | 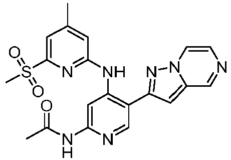 | N-(4-((4-methyl-6-(methylsulfonyl)pyridin-2-yl)amino)-5-(pyrazolo[1,5-a]pyrazin-2-yl)pyridin-2-yl)acetamide | Molecular Weight: 437 |
| **90.** | | 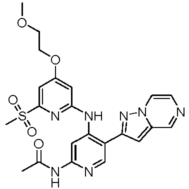 | N-(4-((4-(2-methoxyethoxy)-6-(methylsulfonyl)pyridin-2-yl)amino)-5-(pyrazolo[1,5-a]pyrazin-2-yl)pyridin-2-yl)acetamide | Molecular Weight: 498 |
| **91.** | | 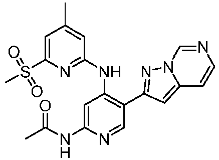 | N-(4-((4-methyl-6-(methylsulfonyl)pyridin-2-yl)amino)-5-(pyrazolo[1,5-c]pyrimidin-2-yl)pyridin-2-yl)acetamide | Molecular Weight: 437 |
| **92.** | | 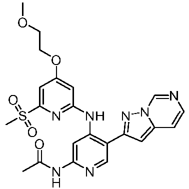 | N-(4-((4-(2-methoxyethoxy)-6-(methylsulfonyl)pyridin-2-yl)amino)-5-(pyrazolo[1,5-c]pyrimidin-2-yl)pyridin-2-yl)acetamide | Molecular Weight: 498 |
| **93.** | | 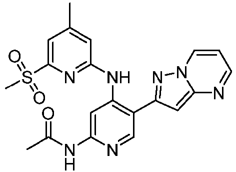 | N-(4-((4-methyl-6-(methylsulfonyl)pyridin-2-yl)amino)-5-(pyrazolo[1,5-a]pyrimidin-2-yl)pyridin-2-yl)acetamide | Molecular Weight: 437 |
| **94.** | | 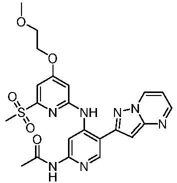 | N-(4-((4-(2-methoxyethoxy)-6-(methylsulfonyl)pyridin-2-yl)amino)-5-(pyrazolo[1,5-a]pyrimidin-2-yl)pyridin-2-yl)acetamide | Molecular Weight: 498 |
| **95.** | | 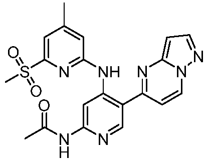 | N-(4-((4-methyl-6-(methylsulfonyl)pyridin-2-yl)amino)-5-(pyrazolo[1,5-a]pyrimidin-5-yl)pyridin-2-yl)acetamide | Molecular Weight: 437 |
| **96.** | | 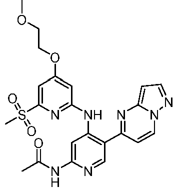 | N-(4-((4-(2-methoxyethoxy)-6-(methylsulfonyl)pyridin-2-yl)amino)-5-(pyrazolo[1,5-a]pyrimidin-5-yl)pyridin-2-yl)acetamide | Molecular Weight: 498 |
| **97.** | | 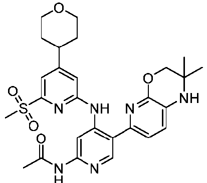 | N-(5-(2,2-dimethyl-2,3-dihydro-1H-pyrido[2,3-b][1,4]oxazin-6-yl)-4-((6-(methylsulfonyl)-4-(tetrahydro-2H-pyran-4-yl)pyridin-2-yl)amino)pyridin-2-yl)acetamide | Molecular Weight: 553 |

### Biochemical Assay and Cellular Assay

### Assay A: TYK2-JH2 biochemical assay

Compounds disclosed herein were tested for blocking of TYK2-JH2 (aa 575-869, in-house) protein with its probe in an assay based on Homogeneous Time Resolved Fluorescence. Compound dilution is done according to the following protocol: (1) Prepare 500× compounds solution in DMSO from 500uM by 5-fold dilution, total 10 doses were included; (2) Prepare 10× compounds solution in an assay buffer containing 20 mM HEPES, pH 7.5, 10 mM MgCl2, 0.005% BSA, 2mM DTT, 0.015% Brij-35 by transferring 1µl serial 500× stock solution into 49µl assay buffer. 4µl of 0.2 nM recombinant TYK2-JH2 protein was pre-incubated with 1µl of 10×serial dilution of compounds at room temperature for 0.5 hour. Then 5µl of 10 nM in-house Probe 1 (6-((3,5-dimethylphenyl)amino)-8-((4,26-dioxo-30-((3aS,4S,6aR)-2-oxohexahydro-1H-thieno[3,4-d]imidazol-4-yl)-7,10,13,16,19,22-hexaoxa-3,25-diazatriacontyl)amino)imidazo[1,2-b]pyridazine-3-carboxamide, KD = 10 nM), 5µl Mab Anti-6His Tb cryptate Gold (Cat: 61HI2TLB, Cisbio Bioassays) and Streptavidin-XL665(Cat: 610SAXLB, Cisobio Bioassays) mixture were added to plate and further incubated at room temperature for 1 hour. The HTRF signals (ex337nm, em620nm/665 nm) were read on BMG PHERAstar FS instrument. The inhibition percentage of TYK2 interaction with its probe in presence of increasing concentrations of compounds was calculated based on the ratio of fluorescence at 615 nm to that at 665nm. The IC50 for each compound was derived from fitting the data to the four-parameter logistic equation by Dotmatics.

### Assay B: JAK1-JH2 biochemical assay

Compounds disclosed herein were tested for blocking of JAK1-JH2 protein (aa 561-860, in-house) with its probe in an assay based on Homogeneous Time Resolved Fluorescence. Compound dilution is done according to the following protocol: (1) Prepare 500× compounds solution in DMSO from 500uM by 5-fold dilution, total 10 doses were included; (2)Prepare 10× compounds solution in an assay buffer containing 20 mM HEPES, pH 7.5, 10 mM MgCl2, 0.005% BSA, 2mM DTT, 0.015% Brij-35 by transferring 1µl serial 500× stock solution into 49µl assay buffer. 4µL of 1.17 nM recombinant JAK1-JH2 protein was pre-incubated with 1µl of 10×serial dilution of compounds at room temperature for 0.5 hour. Then 5µL of 2.9 nM in-house Probe 2 (N-(2-(4-(2-(methyl(4-((((Z)-2-oxoindolin-3-ylidene)(phenyl)methyl)amino)phenyl)amino)-2-oxoethyl)piperazin-1-yl)ethyl)-1-(5-((3aS,4S,6aR)-2-oxohexahydro-1H-thieno[3,4-d]imidazol-4-yl)pentanamido)-3,6,9,12,15,18-hexaoxahenicosan-21-amide, KD=2.9 nM), 5µl Mab Anti-6His Tb cryptate Gold (Cat: 61HI2TLB, Cisbio Bioassays) and Streptavidin-XL665(Cat: 610SAXLB, Cisobio Bioassays) mixture were added to plate and further incubated at room temperature for 1 hour. The HTRF signals (ex337nm, em620nm/665 nm) were read on BMG PHERAstar FS instrument. The inhibition percentage of JAK1 interaction with its probe in presence of increasing concentrations of compounds was calculated based on the ratio of fluorescence at 615 nm to that at 665nm. The IC50 for each compound was derived from fitting the data to the four-parameter logistic equation by Dotmatics.

The probes herein used in assays can also be prepared according to the conventional synthesis methods well-known by a chemist.

### Assay C: IL-12-JAK2, TYK2/ p-STAT4 (Tyr693) inhibition IC50 Cellular Assay

To evaluate the inhibition effect of compounds disclosed herein on JAK2, TYK2/ p-STAT4 (Tyr693) activated by IL-12 in NK-92 cell line, NK-92 cells were collected and washed 3 times by DPBS and resuspended in MEM- α(GIBCO,Cat#12561056) with 10% FBS (Gibco, Cat#10099, Lot#1891605), without IL-2 (R&D systems,Cat#202-IL), starved overnight. Cells were collected and resuspended in 1640 medium (phenol red free, Gibco, Cat#11835-030) with 0.1%BSA, and 12.5µl/5× 10⁴/well cell suspension were seeded to the 96-well plate (Corning, Cat# 3799).Then cells were treated with compounds diluted in 0.2%DMSO 1640 medium, at 37°C, 1h. Dilution is done according to the following protocol: (1) make 500× compounds solution in DMSO from 5mM by 4-fold dilution, total 8 doses were included; (3) make 2× compounds solution in assay medium by transferring 0.5µl serial 500× stock solution into 125µl assay medium; (4) 15µl of 2× serial solution is added to cells and incubate at 37 °C for 1h, the final compound conc. is 10000,2500,625,156.25,39,9.8,2.4 and 0.61nM, respectively. After 1h, cells were treated with 2.5µl medium containing IL-12 at 37°C (R&D systems, Cat#219-IL-005, final conc.40ng/ml), 30min. Following cells were lysed with 7.5µl lysis buffer at RT, shaking on shaker for 1h. 10 µL of cell lysate were transferred to a PE 384-well Proxiplate detection plate, and 5 µL of pre-mixed Alphascreen beads were added to each well. Covered the plate with a plate sealer, span 1000 rpm for 1 min, mix, Incubated overnight at room temperature. Read on BMG PheraStar with Alphascreen protocol. IC 50 values were calculated by fitting dependent data to the four-parameter logistic model using dotmatics software. The assay was performed by using AlphaLISA SureFire Ultra p-STAT4 (Tyr693) Assay Kit - High Volume (PE, Cat#ALSU-PST4-A-HV).

### Assay D: IL-6-JAKl/p-STAT3(Tyr705) inhibition IC50 Cellular Assay

To evaluate the inhibition effect of compounds disclosed herein on JAK1/p-STAT3(Tyr705) activated by IL-6 in TF-1 cell line, TF-1 cells were collected and washed 3 times by DPBS and resuspended in RPMI-1640 (phenol red free, Gibco, Cat#11835-030) with 0.1% FBS (Gibco, Cat#10099, Lot#1891605), without GM-CSF(R&D systems,Cat#215-GM-050), starved overnight. Cells were collected and resuspended in 1640 medium (phenol red free, Gibco, Cat#11835-030) with 0.1%BSA, and 12.5µl/10×10⁴/well cell suspension were seeded to the 96-well plate (Corning, Cat# 3799). Then cells were treated with compounds diluted in 0.2%DMSO 1640 medium, at 37°C, 1h. Dilution is done according to the following protocol: (1) make 500× compounds solution in DMSO from 5mM by 4-fold dilution, total 8 doses were included; (3) make 2× compounds solution in assay medium by transferring 0.5µl serial 500× stock solution into 125µl assay medium; (4) 15µl of 2× solution is added to cells and incubate at 37°C for 1h, the final compound conc. is 10000,2500,625,156.25,39,9.8,2.4 and 0.61nM, respectively. After 1h, cells were treated with 2.5µl medium containing IL-6 at 37°C (R&D systems, Cat#206-IL-010, final conc.50ng/ml), 30min. Following cells were lysed with 10µl lysis buffer at RT, shaking on shaker for 1h. 16 µL of cell lysate were transferred to a PE 384-well HTRF detection plate, and 4 µL of pre-mixed HTRF antibodies were added to each well. Covered the plate with a plate sealer, span 1000 rpm for 1 min, mix, Incubated overnight at room temperature. Read on BMG PheraStar with HTRF protocol (337nm-665nm-620nm). IC 50 values were calculated by fitting dependent data to the four-parameter logistic model using dotmatics software. The assay was performed by using HTRF Phospho-STAT3(Tyr705) Cellular Assay Kit (Cisbio, Cat#62AT3PEG).

Compounds disclosed herein showed picomolar to nanomolar bio-chemical activity in TYK2-JH2 binding assay and also showed nanomolar activity in cellular assay. In the meanwhile, these compounds showed excellent selectivity in TYK2 bio-chemical assay against JAK1 and in TYK2 cellular assay against JAK2. *See* the following Tables from 1 to 14.

**Table 1:**

| **Example** | | **Biochemical assay** | | | **Cellular assay** | | |
|---|---|---|---|---|---|---|---|
| | | **TYK2-JH2 IC50 (nM)** | **JAK1-JH2 IC50 (nM)** | **Selectivity fold (TYK2/JA K1)** | **pSTAT4/I L-12 IC50 (nM)** | **pSTATS/G M-CSF IC50 (nM)** | **Selectivity fold (TYK2/JAK 2)** |
| A1 | | 0.052 | 15 | 288 | 79.5 | >10000 | >125 |
| A2 | | 5.2 | 828 | 159 | -- | -- | -- |
| A3 | | 0.035 | -- | -- | 2.53 | >10000 | >3952 |
| A4 | | 0.52 | 59 | 113 | -- | -- | -- |
| A5 | | 0.49 | 59 | 120 | 296 | >10000 | >33 |
| A6 | | 0.14 | 18 | 128 | 51 | >10000 | >196 |
| A7 | | 670 | >10000 | 14 | -- | -- | -- |
| A8 | | 0.26 | -- | -- | -- | -- | -- |
| A9 | | 0.041 | -- | -- | 1.33 | >10000 | >7518 |
| A10 | | 2.4 | -- | -- | 2996 | - | -- |
| A11 | | 0.063 | -- | -- | 41.7 | >10000 | >239 |
| A12 | | 0.037 | -- | -- | 16.8 | >10000 | >595 |
| A13 | | 0.08 | -- | -- | -- | -- | -- |
| A14 | | 0.022 | -- | -- | 0.969 | >10000 | >10319 |
| A15 | | 0.04 | -- | -- | 0.687 | 3462 | 5039 |
| A16 | | 0.021 | -- | -- | 0.667 | >10000 | >14992 |
| A17 | | 0.028 | -- | -- | 1.64 | >10000 | >6097 |
| A18 | | 8.15 | -- | -- | -- | -- | -- |
| A19 | | 0.034 | -- | -- | 1.53 | >10000 | >6535 |
| A20 | | 0.03 | -- | -- | 1.12 | 2379 | 2124 |
| A21 | | -- | -- | -- | -- | -- | -- |
| A22 | | -- | -- | -- | -- | -- | -- |
| A23 | | -- | -- | -- | -- | -- | -- |
| A24 | | 0.027 | -- | -- | 1.85 | >10000 | >5405 |
| A25 | | 0.027 | -- | -- | 0.596 | >10000 | >16778 |
| A26 | | 0.032 | -- | -- | 0.405 | >10000 | >24691 |
| A27 | | 0.034 | -- | -- | 2.1 | >10000 | >4761 |
| A28 | | 0.033 | -- | -- | 1.52 | >10000 | >6578 |
| A29 | | 0.042 | -- | -- | 0.452 | >10000 | >22123 |
| A30 | | -- | -- | -- | -- | -- | -- |
| A31 | | -- | -- | -- | -- | -- | -- |

**Table 2:**

| **Example** | | **Biochemical assay** | | | **Cellular assay** | | |
|---|---|---|---|---|---|---|---|
| | | **TYK2-JH2** | **JAK1-JH2** | **Selectivity fold (TYK2/JAK1)** | **pSTAT4/IL-12 IC50 (nM)** | **pSTATS/GM-CSF IC50 (nM)** | **Selectivity fold (TYK2/JAK2)** |
| | | **IC50 (nM)** | **IC50 (nM)** | | | | |
| B1 | | 0.038 | 7.4 | 194 | 9.89 | >10000 | >1011 |
| B2 | | 5.8 | -- | -- | -- | -- | -- |
| B3 | | 1.1 | 9 | 8.1 | -- | -- | -- |
| B4 | | 0.019 | -- | -- | 2.53 | >10000 | >3952 |
| B5 | | 0.016 | 0.31 | 19 | 0.243 | >10000 | >41152 |
| B6 | | 0.018 | 1.2 | 666 | 1.21 | >10000 | >8264 |
| B7 | | 0.016 | -- | -- | 5.58 | >10000 | >1792 |
| B8 | | 0.047 | -- | -- | 1.68 | >10000 | >5952 |
| B9 | | 0.53 | -- | -- | -- | -- | -- |
| B10 | | 0.045 | -- | -- | -- | -- | -- |
| B11 | | 0.019 | -- | -- | -- | -- | -- |
| B12 | | 0.034 | -- | -- | 0.74 | 610 | 824 |
| B13 | | 0.43 | 18 | 42 | 183 | >10000 | >54 |
| B14 | | 80 | 145 | 1 | -- | -- | -- |
| B15 | | 117 | 234 | 1 | -- | -- | -- |
| B16 | | 0.03 | -- | -- | 0.513 | 4292 | 8366 |
| B17 | | 0.035 | -- | -- | 2.05 | >10000 | >4878 |
| B18 | | 0.036 | -- | -- | 1.05 | >10000 | >9523 |
| B19 | | 18 | 257 | 14.2 | -- | -- | --- |
| B20 | | 1.5 | 76 | 50 | -- | -- | -- |
| B21 | | 0.047 | -- | -- | 0.443 | 2490 | 5620 |
| B22 | | 0.025 | -- | -- | 12.61 | 2490 | 197 |
| B23 | | 0.04 | -- | -- | 0.307 | 1001 | 3260 |
| B24 | | -- | -- | -- | -- | -- | -- |
| B25 | | -- | -- | -- | -- | -- | -- |

**Table 3:**

| | | **Biochemical assay** | | | **Cellular assay** | | |
|---|---|---|---|---|---|---|---|
| **Example** | | **TYK2-JH2 IC50 (nM)** | **JAK1-JH2 IC50 (nM)** | **Selectivity fold (TYK2/JAK1)** | **pSTAT4/IL-12 IC50 (nM)** | **pSTATS/GM-CSF IC50 (nM)** | **Selectivity fold (TYK2/JAK2)** |
| C1 | | 0.027 | -- | -- | 0.881 | 563 | 639 |
| C2 | | 0.026 | -- | -- | 0.312 | 962 | 3083 |
| C3 | | 0.032 | -- | -- | 5.81 | >10000 | >1721 |
| C4 | | 0.034 | -- | -- | 4.35 | >10000 | >2298 |
| C5 | | 0.032 | -- | -- | 21.9 | >10000 | >456 |
| C6 | | 0.054 | -- | -- | 26.02 | >10000 | >381 |
| C7 | | -- | -- | -- | -- | -- | -- |
| C8 | | -- | -- | -- | -- | -- | |
| C9 | | 0.027 | -- | -- | 0.672 | >10000 | >14880 |
| C10 | | -- | -- | -- | -- | -- | |
| C11 | | -- | -- | -- | -- | -- | |
| C12 | | -- | -- | -- | -- | -- | |
| C13 | | -- | -- | -- | -- | -- | |
| C14 | | -- | -- | -- | -- | -- | -- |
| C15 | | -- | -- | -- | -- | -- | -- |
| C16 | | -- | -- | -- | -- | -- | -- |
| C17 | | 0.022 | -- | -- | 0.605 | >10000 | >16528 |
| C18 | | 0.023 | -- | -- | 0.639 | 4873 | 7625 |
| C19 | | 0.023 | -- | -- | -- | -- | -- |
| C20 | | 0.023 | -- | -- | -- | -- | -- |
| C21 | | 0.024 | -- | | 0.995 | >10000 | >10050 |
| C22 | | 0.023 | -- | -- | 0.476 | >10000 | >21008 |
| C23 | | 0.022 | -- | -- | 0.63 | 2600 | 4126 |
| C24 | | -- | -- | -- | -- | -- | -- |
| C25 | | 0.037 | -- | -- | 0.827 | 1065 | 1288 |

**Table 4:**

| **Example** | | **Biochemical assay** | | | **Cellular assay** | | |
|---|---|---|---|---|---|---|---|
| | | **TYK2-JH2 IC50 (nM)** | **JAK1-JH2 IC50 (nM)** | **Selectivity fold (TYK2/JAK1)** | **pSTAT4/IL-12 IC50 (nM)** | **pSTATS/GM-CSF IC50 (nM)** | **Selectivity fold (TYK2/JAK2)** |
| D1 | | 0.047 | -- | -- | 0.251 | >10000 | >39840 |
| D2 | | 0.038 | -- | -- | 15.6 | >10000 | >641 |
| D3 | | 0.08 | -- | -- | 4.8 | >10000 | >2083 |
| D4 | | 0.044 | -- | -- | 0.262 | >10000 | >38167 |
| D5 | | 0.036 | -- | -- | 0.99 | >10000 | >10101 |
| D6 | | 0.025 | -- | -- | 0.593 | >10000 | >16863 |
| D7 | | 0.031 | -- | -- | 0.805 | >10000 | >12422 |
| D8 | | 0.036 | -- | -- | 0.443 | >10000 | >22573 |
| D9 | | 0.021 | -- | -- | 0.479 | >10000 | >20876 |
| D10 | | 0.052 | -- | -- | 12.67 | >10000 | >789 |
| D11 | | -- | -- | -- | -- | -- | -- |

**Table 5:**

| | | **Biochemical assay** | | | **Cellular assay** | | |
|---|---|---|---|---|---|---|---|
| **Example** | | **TYK2-JH2 IC50 (nM)** | **JAK1-JH2 IC50 (nM)** | **Selectivity fold (TYK2/JAK1)** | **pSTAT4/IL-12 IC50 (nM)** | **pSTATS/GM-CSF IC50 (nM)** | **Selectivity fold (TYK2/JAK2)** |
| E1 | | 0.022 | -- | -- | 4.55 | >10000 | >2197 |
| E2 | | 0.017 | -- | -- | 0.325 | 5807 | 17867 |
| E3 | | 0.035 | -- | -- | 0.645 | >10000 | >15503 |
| E4 | | 0.022 | -- | -- | 0.536 | >10000 | >18656 |
| E5 | | 0.022 | -- | -- | 0.501 | >10000 | >19960 |
| E6 | | 0.029 | -- | -- | 2.72 | >10000 | >3676 |
| E7 | | 0.02 | -- | -- | 0.447 | >10000 | >22371 |
| E8 | | 0.034 | -- | -- | 2.86 | >10000 | >3496 |
| E9 | | 0.022 | -- | -- | 0.504 | >10000 | >19960 |

**Table 6:**

| **Example** | | **Biochemical assay** | | | **Cellular assay** | | |
|---|---|---|---|---|---|---|---|
| | | **TYK2-JH2 IC50 (nM)** | **JAK1-JH2 IC50 (nM)** | **Selectivity fold (TYK2/JAK1)** | **pSTAT4/IL-12 IC50 (nM)** | **pSTATS/GM-CSF IC50 (nM)** | **Selectivity fold (TYK2/JAK2)** |
| F1 | | 0.046 | -- | -- | 1.23 | >10000 | >8130 |
| F2 | | 0.051 | -- | -- | 22.14 | >10000 | >451 |
| F3 | | 0.068 | -- | -- | -- | -- | -- |
| F4 | | 0.324 | -- | -- | -- | -- | -- |
| F5 | | 0.061 | -- | -- | -- | -- | -- |
| F6 | | 0.532 | -- | -- | -- | -- | -- |
| F7 | | 0.085 | -- | -- | -- | -- | -- |
| F8 | | 0.087 | -- | -- | -- | -- | -- |
| F9 | | 0.06 | -- | -- | 5.45 | >10000 | > 1834 |
| F10 | | 0.04 | -- | -- | -- | -- | -- |
| F11 | | 0.037 | -- | -- | 0.705 | >10000 | >14184 |
| F12 | | 0.034 | -- | -- | 1.35 | >10000 | >7407 |
| F13 | | 0.21 | -- | -- | -- | -- | -- |
| F14 | | 0.13 | -- | -- | -- | -- | -- |
| F15 | | 0.073 | -- | -- | -- | -- | -- |
| F16 | | 0.027 | -- | -- | -- | -- | -- |
| F17 | | 0.054 | -- | -- | 1.25 | >10000 | >8000 |
| F18 | | 0.052 | -- | -- | -- | -- | -- |
| F19 | | 0.036 | -- | -- | 3.06 | >10000 | >3267 |
| F20 | | 0.031 | -- | -- | 1.45 | >10000 | >6896 |
| F21 | | 0.026 | -- | -- | 3.54 | >10000 | >2814 |
| F22 | | 0.026 | -- | -- | 1.82 | >10000 | >5494 |
| F23 | | 0.020 | -- | -- | 1.07 | >10000 | >9345 |
| F24 | | 0.033 | -- | -- | 0.82 | >10000 | >12195 |
| F25 | | 0.029 | -- | -- | 0.446 | >10000 | >22421 |

**Table 7:**

| | | **Biochemical assay** | | | **Cellular assay** | | |
|---|---|---|---|---|---|---|---|
| **Example** | | **TYK2-JH2 IC50 (nM)** | **JAK1-JH2 IC50 (nM)** | **Selectivity fold (TYK2/JAK1)** | **pSTAT4/IL-12 IC50 (nM)** | **pSTATS/GM-CSF IC50 (nM)** | **Selectivity fold (TYK2/JAK2)** |
| G1 | | 0.026 | 4.9 | 188 | 14.4 | >10000 | >694 |
| G2 | | 0.067 | -- | -- | 14.2 | >10000 | >704 |
| G3 | | 0.05 | -- | -- | 224 | >10000 | >44 |
| G4 | | 0.048 | | | 16 | >10000 | >625 |

**Table 8:**

| | | **Biochemical assay** | | | **Cellular assay** | | |
|---|---|---|---|---|---|---|---|
| **Example** | | **TYK2-JH2 IC50 (nM)** | **JAK1-JH2 IC50 (nM)** | **Selectivity fold (TYK2/JAK1)** | **pSTAT4/IL-12 IC50 (nM)** | **pSTATS/GM-CSF IC50 (nM)** | **Selectivity fold (TYK2/JAK2)** |
| H1 | | 0.016 | 1.4 | 87.5 | 2.6 | >10000 | >3846 |
| H2 | | 0.036 | -- | -- | 0.939 | >10000 | >10649 |
| H3 | | 0.048 | -- | -- | 8.52 | 7185 | >843 |
| H4 | | 0.047 | -- | -- | | | |
| H5 | | 0.015 | -- | -- | 0.323 | 1675 | 5185 |
| H6 | | 0.014 | -- | -- | 1.25 | 8872 | 7097 |
| H7 | | 0.046 | -- | -- | 1.22 | >10000 | >8196 |
| H8 | | 0.041 | -- | -- | 21.66 | >10000 | >461 |
| H9 | | 0.016 | -- | -- | 0.503 | 1583 | 3147 |
| H10 | | 0.017 | -- | -- | 16.45 | >10000 | 607 |
| H11 | | 0.027 | -- | -- | 1 | >10000 | >10000 |
| H12 | | 0.022 | -- | -- | 5.1 | >10000 | >1960 |
| H13 | | 0.042 | -- | -- | 0.676 | 4075 | 6028 |
| H14 | | 0.034 | -- | -- | 0.727 | >10000 | >13755 |
| H15 | | 0.029 | -- | -- | 4.89 | >10000 | >2044 |
| H16 | | 0.028 | -- | -- | 7.51 | >10000 | >1331 |
| H17 | | 0.034 | -- | -- | 67 | >10000 | >149 |
| H18 | | 0.032 | -- | -- | 5.54 | >10000 | >1805 |
| H19 | | 0.028 | -- | -- | 3.2 | >10000 | >3125 |
| H20 | | 0.046 | -- | -- | 35.2 | >10000 | >284 |
| H21 | | 0.024 | -- | -- | 2.24 | >10000 | >4464 |
| H22 | | 0.038 | -- | -- | 2.76 | >10000 | >3623 |
| H23 | | 0.034 | -- | -- | 5.7 | >10000 | >1754 |
| H24 | | 0.034 | -- | -- | 5.39 | >10000 | >1855 |
| H25 | | 0.038 | -- | -- | 7.53 | >10000 | >1328 |
| H26 | | 0.039 | -- | -- | 11.7 | >10000 | >854 |
| H27 | | 0.031 | -- | -- | 2.72 | >10000 | >3676 |
| H28 | | 0.03 | -- | -- | 29.2 | >10000 | >342 |
| H29 | | 0.022 | -- | -- | 1.77 | 8491 | 4797 |
| H30 | | 0.023 | -- | -- | 0.972 | >10000 | >10288 |
| H31 | | 0.023 | -- | -- | 0.935 | >10000 | >10695 |
| H32 | | 0.023 | -- | -- | 0.447 | >10000 | >22371 |
| H33 | | 0.019 | -- | -- | 0.777 | >10000 | >22371 |
| H34 | | 0.023 | -- | -- | 1.12 | >10000 | >8928 |
| H35 | | 0.028 | -- | -- | 3.17 | >10000 | >3154 |
| H36 | | 0.022 | -- | -- | 1.95 | >10000 | >5128 |
| H37 | | -- | -- | -- | -- | -- | -- |
| H38 | | -- | -- | -- | -- | -- | -- |
| H39 | | -- | -- | -- | -- | -- | -- |
| H40 | | -- | -- | -- | -- | -- | -- |
| H41 | | -- | -- | -- | -- | -- | -- |
| H42 | | -- | -- | -- | -- | -- | -- |
| H43 | | 0.034 | -- | -- | 5.39 | >10000 | >1855 |

**Table 9:**

| | | **Biochemical assay** | | | **Cellular assay** | | |
|---|---|---|---|---|---|---|---|
| **Example** | | **TYK2-JH2 IC50 (nM)** | **JAK1-JH2 IC50 (nM)** | **Selectivity fold (TYK2/JAK1)** | **pSTAT4/IL-12 IC50 (nM)** | **pSTATS/GM-CSF IC50 (nM)** | **Selectivity fold (TYK2/JAK2)** |
| J1 | | 0.019 | -- | -- | 0.446 | >10000 | >22421 |
| J2 | | 0.022 | -- | -- | 3 | >10000 | >3333 |
| J3 | | 0.026 | -- | -- | 0.286 | >10000 | >34965 |
| J4 | | 0.028 | -- | -- | 4.94 | >10000 | >2024 |
| J5 | | 0.022 | -- | -- | 2.05 | >10000 | >4878 |
| J6 | | 0.033 | -- | -- | 7.94 | >10000 | >1259 |
| J7 | | 0.19 | -- | -- | 66.23 | >10000 | >150 |
| J8 | | 0.073 | -- | -- | 7.67 | >10000 | >1303 |
| J9 | | 0.083 | -- | -- | 6.54 | >10000 | >1529 |
| J10 | | 0.05 | -- | -- | 6.34 | >10000 | >1577 |
| J11 | | 0.017 | -- | -- | 0.275 | 6415 | 23327 |
| J12 | | 0.017 | -- | -- | 0.847 | >10000 | >11806 |
| J13 | | 0.054 | -- | -- | 1.18 | >10000 | >8474 |
| J14 | | 0.033 | -- | -- | 0.792 | >10000 | >12626 |
| J15 | | 0.016 | -- | -- | 5.9 | >10000 | >1694 |
| J16 | | 0.015 | -- | -- | 2.52 | >10000 | >3968 |
| J17 | | 0.043 | | | 6.8 | >10000 | >1470 |
| J18 | | 0.028 | -- | -- | 0.399 | >10000 | >25062 |
| J19 | | 0.015 | -- | -- | 0.882 | >10000 | >1137 |
| J20 | | 0.026 | -- | -- | 3 | >10000 | >3333 |
| J21 | | 0.017 | -- | -- | 0.72 | >10000 | >13888 |
| J22 | | 0.037 | -- | -- | 1.93 | >10000 | >5181 |
| J23 | | 0.044 | -- | -- | 0.862 | 1354 | 1570 |
| J24 | | 0.028 | -- | -- | 6.05 | >10000 | >1652 |
| J25 | | 0.025 | -- | -- | 2.91 | >10000 | >3436 |
| J26 | | 0.023 | -- | -- | 5.5 | >10000 | >1818 |
| J27 | | 0.04 | -- | -- | 0.439 | >10000 | >22779 |
| J28 | | 0.053 | -- | -- | 3.14 | >10000 | >3184 |
| J29 | | 0.045 | -- | -- | 12.41 | >10000 | >805 |
| J30 | | 0.054 | -- | -- | 4.82 | >10000 | >2074 |
| J31 | | 0.076 | -- | -- | 14.97 | >10000 | >668 |
| J32 | | 0.079 | -- | -- | 8.79 | >10000 | >1137 |
| J33 | | 0.12 | -- | -- | -- | -- | -- |
| J34 | | 0.067 | -- | -- | 9.69 | >10000 | >1031 |
| J35 | | 0.036 | -- | -- | 8.13 | >10000 | >1230 |
| J36 | | 0.043 | --- | - | 5.05 | >10000 | >1980 |
| J37 | | 0.035 | -- | -- | 19.1 | >10000 | >523 |
| J38 | | 0.024 | -- | -- | 1.98 | >10000 | >5050 |
| J39 | | 0.036 | -- | -- | 10.69 | >10000 | >935 |
| J40 | | 0.029 | -- | -- | 0.326 | >10000 | >30674 |
| J41 | | 0.027 | -- | -- | 2.98 | >10000 | >3355 |
| J42 | | 0.05 | -- | -- | 0.796 | >10000 | >12562 |
| J43 | | 0.025 | -- | -- | 0.744 | >10000 | >13440 |
| J44 | | 0.022 | -- | -- | 0.358 | >10000 | >27932 |
| J45 | | 0.028 | -- | -- | 0.398 | >10000 | >25125 |
| J46 | | 0.033 | -- | -- | 2.35 | >10000 | >4255 |
| J47 | | 0.037 | -- | -- | 4.73 | >10000 | >2114 |
| J48 | | 0.035 | -- | -- | 70.2 | >10000 | >1421411 |
| J49 | | 0.13 | -- | -- | -- | -- | -- |
| J50 | | 0.049 | -- | -- | 27.68 | >10000 | >361 |
| J51 | | 0.043 | -- | -- | 1.58 | >10000 | >6329 |
| J52 | | 0.021 | -- | -- | 1.43 | >10000 | >699 |
| J53 | | 0.033 | -- | -- | 3.47 | >10000 | >2881 |
| J54 | | 0.028 | -- | -- | 2.03 | >10000 | >4926 |
| J55 | | 0.033 | -- | -- | 11.38 | >10000 | >878 |
| J56 | | 0.044 | -- | -- | 30.07 | >10000 | >332 |
| J57 | | 0.045 | -- | -- | 15.3 | >10000 | >653 |
| J58 | | 0.025 | -- | -- | 0.497 | >10000 | >20120 |
| J59 | | 0.029 | -- | -- | 1.31 | >10000 | >7633 |
| J60 | | 0.021 | -- | -- | 0.797 | >10000 | >12547 |
| J61 | | 0.019 | -- | -- | 1.42 | >10000 | >7042 |
| J62 | | 0.037 | -- | -- | 4.85 | >10000 | >2061 |
| J63 | | 0.018 | -- | -- | 0.462 | >10000 | >21645 |
| J64 | | -- | -- | -- | -- | -- | -- |
| J65 | | -- | -- | -- | -- | -- | -- |

**Table 10:**

| | | **Biochemical assay** | | | **Cellular assay** | | |
|---|---|---|---|---|---|---|---|
| **Example** | | **TYK2-JH2 IC50 (nM)** | **JAK1-JH2 IC50 (nM)** | **Selectivity fold (TYK2/JAK1)** | **pSTAT4/IL-12 IC50 (nM)** | **pSTATS/GM-CSF IC50 (nM)** | **Selectivity fold (TYK2/JAK2)** |
| M1 | | 0.055 | -- | -- | 0.174 | 2010 | 11551 |
| M2 | | 0.032 | -- | -- | 1.04 | >10000 | >9615 |
| M3 | | 0.031 | -- | -- | 0.526 | 8351 | 15876 |
| M4 | | 0.026 | -- | -- | 0.681 | >10000 | >14684 |
| M5 | | 0.032 | -- | -- | 0.189 | 1551 | 8206 |
| M6 | | 0.032 | -- | -- | 1.21 | >10000 | >8264 |
| M7 | | 0.026 | -- | -- | 0.537 | >10000 | >18621 |
| M8 | | 0.04 | -- | -- | 1.64 | >10000 | >6097 |
| M9 | | 0.035 | -- | -- | 7.06 | >10000 | >1416 |
| M10 | | 0.099 | -- | -- | -- | -- | -- |
| M11 | | 0.034 | -- | -- | 9.03 | >10000 | >1107 |
| M12 | | 0.03 | -- | -- | 9.12 | >10000 | >1096 |
| M13 | | 0.11 | -- | -- | -- | -- | -- |
| M14 | | 0.03 | -- | -- | 9.46 | >10000 | >1057 |
| M15 | | 0.048 | -- | -- | 10.23 | >10000 | >977 |
| M16 | | 0.048 | -- | -- | -- | -- | -- |
| M17 | | 0.041 | | -- | 1.09 | >10000 | >9174 |
| M18 | | 0.026 | -- | -- | 0.989 | >10000 | >10111 |
| M19 | | 0.032 | -- | -- | 2.55 | >10000 | >3921 |
| M20 | | 0.062 | -- | -- | 1.01 | >10000 | >9900 |
| M21 | | 0.035 | -- | -- | 1.69 | >10000 | >5917 |
| M22 | | 0.027 | -- | -- | 0.316 | 532 | 1683 |
| M23 | | 0.031 | -- | -- | 1.08 | 3749 | 3471 |
| M24 | | 0.032 | -- | -- | 0.487 | 2175 | 4466 |
| M25 | | 0.096 | -- | --- | 15.17 | >10000 | >659 |
| M26 | | 0.046 | -- | -- | 4.33 | >10000 | >2309 |
| M27 | | 0.035 | -- | -- | 3.85 | >10000 | >2597 |
| M28 | | 0.032 | -- | -- | 16.62 | >10000 | >601 |
| M29 | | -- | -- | -- | -- | -- | -- |
| M30 | | 0.049 | -- | -- | 11.2 | >10000 | >892 |
| M31 | | 0.031 | -- | -- | 0.828 | 1421 | 1714 |
| M32 | | 0.074 | -- | -- | -- | -- | -- |
| M33 | | 0.027 | -- | -- | 0.648 | >10000 | >15432 |
| M34 | | 0.024 | -- | -- | 0.402 | >10000 | >24875 |
| M35 | | 0.036 | -- | -- | 50.24 | >10000 | >199 |

**Table 11:**

| | | **Biochemical assay** | | | **Cellular assay** | | |
|---|---|---|---|---|---|---|---|
| **Exampl e** | | **TYK2-JH2 IC50 (nM)** | **JAK1-JH2 IC50 (nM)** | **Selectivity fold (TYK2/JA K1)** | **pSTAT4/I L-12 IC50 (nM)** | **pSTAT5/ GM-CSF IC50 (nM)** | **Selectivity fold (TYK2/JAK 2)** |
| N1 | | 0.077 | -- | -- | 0.247 | 2133 | 8635 |
| N2 | | 0.021 | -- | -- | 0.641 | >10000 | >15600 |
| N3 | | 0.033 | -- | -- | 12.63 | >10000 | >791 |
| N4 | | 0.029 | -- | -- | 0.137 | >10000 | >72992 |
| N5 | | 0.039 | -- | -- | 0.212 | >10000 | >47169 |
| N6 | | 0.039 | -- | -- | 0.274 | >10000 | >36496 |
| N7 | | 0.024 | -- | -- | 1.43 | >10000 | >6993 |

**Table 12:**

| | | **Biochemical assay** | | | **Cellular assay** | | |
|---|---|---|---|---|---|---|---|
| **Example** | | **TYK2-JH2 IC50 (nM)** | **JAK1-JH2 IC50 (nM)** | **Selectivity fold (TYK2/JAK1)** | **pSTAT4/IL-12 IC50 (nM)** | **pSTATS/GM-CSF IC50 (nM)** | **Selectivity fold (TYK2/JAK2)** |
| O1 | | 0.017 | -- | -- | 0.414 | 1213 | 2929 |
| O2 | | 0.016 | -- | -- | 1.12 | 7007 | 6265 |
| O3 | | 0.031 | -- | -- | 6.86 | 8295 | 2109 |

**Table 13:**

| | | **Biochemical assay** | | | **Cellular assay** | | |
|---|---|---|---|---|---|---|---|
| **Example** | | **TYK2-JH2 IC50 (nM)** | **JAK1-JH2 IC50 (nM)** | **Selectivity fold (TYK2/JAK1)** | **pSTAT4/IL-12 IC50 (nM)** | **pSTATS/GM-CSF IC50 (nM)** | **Selectivity fold (TYK2/JAK2)** |
| Q1 | | 0.023 | -- | -- | 1.76 | >10000 | >5681 |
| Q2 | | 0.021 | -- | -- | 1.7 | >10000 | >5882 |
| Q3 | | 0.023 | -- | -- | 1.14 | >10000 | >8771 |
| Q4 | | 0.037 | -- | -- | 1.10 | >10000 | >9090 |
| Q5 | | 0.065 | -- | -- | -- | -- | -- |
| Q6 | | 0.028 | -- | -- | 0.45 | >10000 | >22222 |
| Q7 | | -- | -- | -- | -- | -- | -- |
| Q8 | | -- | -- | -- | -- | -- | -- |
| Q9 | | -- | -- | -- | -- | -- | -- |
| Q10 | | -- | -- | -- | -- | -- | -- |
| Q11 | | -- | -- | -- | -- | -- | -- |
| Q12 | | -- | -- | -- | -- | -- | -- |
| Q13 | | -- | -- | -- | -- | -- | -- |
| Q14 | | -- | -- | -- | -- | -- | -- |
| Q15 | | -- | -- | -- | -- | -- | -- |
| Q16 | | -- | -- | -- | -- | -- | -- |
| Q17 | | -- | -- | -- | -- | -- | -- |
| Q18 | | 0.023 | -- | -- | 0.91 | >10000 | >10989 |
| Q19 | | 0.025 | -- | -- | 3.02 | >10000 | >3311 |
| Q20 | | 0.022 | -- | -- | 0.16 | 1767 | 11043 |
| Q21 | | 0.037 | -- | -- | 0.411 | 1572 | 3824 |
| Q22 | | 0.025 | -- | -- | 0.584 | 3959 | 6779 |
| Q23 | | 0.028 | -- | -- | 0.605 | 8101 | 13390 |
| Q24 | | 0.033 | -- | -- | 0.559 | 620 | 1109 |
| Q25 | | 0.026 | -- | -- | 0.530 | 1822 | 3437 |
| Q26 | | 0.032 | -- | -- | 1.53 | 700 | 457 |
| Q27 | | 0.033 | -- | -- | -- | -- | -- |
| Q28 | | 0.026 | -- | -- | 0.329 | >10000 | >30395 |
| Q29 | | 0.037 | -- | -- | -- | -- | -- |
| Q30 | | 0.065 | -- | -- | | | |
| Q35 | | 0.020 | -- | -- | 0.679 | 1810 | 2665 |
| Q36 | | -- | -- | -- | -- | -- | -- |
| Q49 | | 0.029 | -- | -- | 0.827 | 379 | 458 |
| Q50 | | 0.019 | -- | -- | 0.626 | 2023 | 3231 |
| Q51 | | 0.034 | -- | -- | 0.979 | >10000 | >10214 |
| Q52 | | 0.038 | -- | -- | 1.33 | 3420 | 2571 |
| Q53 | | 0.047 | -- | -- | 0.581 | >10000 | >17211 |
| Q54 | | 0.037 | -- | -- | 0.560 | 3985 | 7116 |
| Q55 | | 0.019 | -- | -- | -- | -- | -- |
| Q56 | | 0.031 | -- | -- | -- | -- | -- |
| Q57 | | 0.031 | -- | -- | -- | -- | -- |
| Q58 | | 0.097 | -- | -- | -- | -- | -- |
| Q59 | | 0.033 | -- | -- | -- | -- | -- |
| Q60 | | 0.059 | -- | -- | -- | -- | -- |
| Q61 | | 0.022 | -- | -- | -- | -- | -- |
| Q62 | | 0.047 | -- | -- | 1.79 | >10000 | >5586 |
| Q63 | | 0.051 | -- | -- | 0.755 | 459 | 608 |
| Q64 | | 0.034 | -- | -- | 4.61 | >10000 | >2169 |
| Q65 | | 0.029 | -- | -- | 0.685 | 1642 | 2397 |
| Q66 | | 0.029 | -- | -- | 0.792 | 3046 | 3845 |
| Q73 | | 0.025 | -- | -- | 2.84 | 2354 | 828 |
| Q75 | | 0.024 | -- | -- | 0.637 | 473 | 742 |
| Q79 | | 0.32 | -- | -- | -- | -- | -- |
| Q86 | | 0.031 | -- | -- | 1.48 | 911 | 615 |
| Q90 | | -- | -- | -- | 44 | >10000 | 227 |
| Q97 | | -- | -- | -- | 3.29 | >10000 | 3039 |
| Q98 | | 0.026 | -- | -- | 1.15 | >10000 | 8695 |
| Q99 | | 0.033 | -- | -- | -- | -- | -- |
| Q101 | | 0.03 | -- | -- | 0.904 | 373 | 412 |
| Q104 | | 0.029 | -- | -- | -- | -- | -- |
| Q106 | | 0.015 | -- | -- | -- | -- | -- |
| Q108 | | -- | -- | -- | 1.18 | >10000 | >8474 |
| Q110 | | | | | 1.08 | >10000 | >9259 |
| Q115 | | 0.034 | -- | -- | 0.302 | >10000 | >33112 |
| Q119 | | 0.031 | -- | -- | 1.19 | 1149 | 965 |
| Q120 | | 0.032 | -- | -- | -- | -- | -- |

**Table 14:**

| | | **Biochemical assay** | | | **Cellular assay** | | |
|---|---|---|---|---|---|---|---|
| **Example** | | **TYK2-JH2 IC50 (nM)** | **JAK1-JH2 IC50 (nM)** | **Selectivity fold (TYK2/JAK1)** | **pSTAT4/IL-12 IC50 (nM)** | **pSTATS/GM-CSF IC50 (nM)** | **Selectivity fold (TYK2/JAK2)** |
| R1 | | 0.041 | -- | - | 2.18 | >10000 | >4587 |

### Binding Pose of Compounds in Tyk2 JH2 Domain

The X-ray crystal structure of human Tyk2 JH2 domain in complex with BMS986165 (PDB ID: 6NZP) was downloaded from the RCSB protein data bank and prepared by the Protein Preparation Wizard in Schrödinger 2020, including removing crystallographic waters, fixing bond orders, adding hydrogens, assigning partial charges with the OPLS3e force field, and minimizing the added hydrogens. The 3D structure of the Example B5 was processed by LigPrep module of Schrödinger2020 at pH7.4. And then the ligand was docked into the binding pocket (with a radius of 10 Å around BMS986165 binding site) of the above prepared Tyk2 JH2 domain using Glide SP (standard precision). Standard default settings were used for other parameters.

The binding pose of Example B5 in Tyk2 JH2 domain showed importance of substituted position of methylsulfonyl group and acetamide group on the two pyridine rings, for the Tyk2 inhibitors of the present disclosure **(Scheme 1).** The substituted position of methylsulfonyl group was essential for potency, *meta* position was much better than *orth* position, for example, Examples B1 and B5 showed Tyk2 JH2 domain bio-chemical potency at 0.038 nM and 0.016 nM, respectively, whereas Examples B15 and B14 were 3079-fold and 5000-fold less potent, respectively, that was due to the strong interaction between methylsulfonyl group on the pyridine ring and Lys642 residue of Tyk2 **(Scheme 1).** The acetamide group on the other pyridine ring can also form crucial interaction with Lys642 of Tyk2. Furtherly, the substitution R of acetamide group on the other pyridine ring effected the Tyk2 inhibition potency, for example, the potency dramatically decreased when replacing R from methyl group (Example B1) to cyclopropane group (Example B20). and. In addition, the nitrogen (N) atom of the pyridine ring, which is at the adjacent position of methylsulfonyl, was essential for coplanarity of the two pyridine rings, for example, Example B5 is 2-fold more potent than Example B1 in bio-chemical assay and 40-fold more potent in cellular assay.

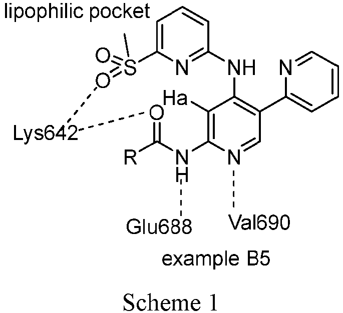

### Biological Assay: Mouse PK study

The pharmacokinetics of compounds were evaluated in male CD-1 mouse via Oral Administration (dose of 10 mg/kg). For oral administration study, test compounds were added in 0.5% MC in water and administrated to mice at 10 mg/kg by gavage. Blood was collected into EDTA-K2 anticoagulant tube at 15, and 30 min and 1, 2, 4, 8 and 24 h after administration. Approximately 30 µL blood was collected at each time point. And then the blood was centrifuged at 2000 g for 5 min at 4°C using a centrifuge to obtain the plasma. The plasma sample was transferred into a tube and stored in a freezer at approximately -70 °C until the determination of concentration by LC-MS/MS. Pharmacokinetic parameters were estimated by using WinNonlin software (version 8.1, Pharsight Corporation, CA, USA) with non-compartmental method. The following pharmacokinetic parameters were calculated, whenever possible from the plasma concentration-time data: Tₘₐₓ, Cₘₐₓ, AUCₗₐₛₜ, AUC_{inf}, T_{1/2} for PO administration. All animals were fasted before experiment. The results are shown in **Table 15.**

**Table 15:**

| **Compound** | | **Po dosing (10 mg/kg)** | | |
|---|---|---|---|---|
| | | **T_{1/2}** | **Cmax (ng/mL)** | **AUCₗₐₛₜ (h·ng/mL)** |
| Example F22 | | 1.48 | 911 | 2098 |
| Example H11 | | 2.07 | 8.73 | 19.3 |
| Example Q25 | | 3.01 | 2820 | 16245 |

The compounds disclosed herein with pyridine fused ring part showed significantly good PK. As in Table 15, taking Example Q25 with pyridine fused ring part as an example, showed significantly better PK than Examples F22 and H11. The A_{UC} (16245 h·ng/mL) and Cₘₐₓ (2820 ng/mL) data of Example Q25 in the table were at least 3-fold higher than those of Example F22 (Cₘₐₓ: 911 ng/mL; A_{UC}: 2098 h·ng/mL) and H11 (Cₘₐₓ: 7 ng/mL; A_{UC}: 19.4 h·ng/mL).

It is to be understood that, if any prior art publication is referred to herein, such reference does not constitute an admission that the publication forms a part of the common general knowledge in the art in any country.

In the claims which follow and in the preceding description of the invention, except where the context requires otherwise due to express language or necessary implication, the word "comprise" or variations such as "comprises" or "comprising" is used in an inclusive sense, i.e., to specify the presence of the stated features but not to preclude the presence or addition of further features in various embodiments of the invention.

Although the foregoing invention has been described in some detail by way of illustration and example for purposes of clarity of understanding, it is apparent to those skilled in the art that certain minor changes and modifications will be practiced. Therefore, the description and examples should not be construed as limiting the scope of the invention, which is defined by the appended claims.

## Claims

1. A compound of Formula (I) 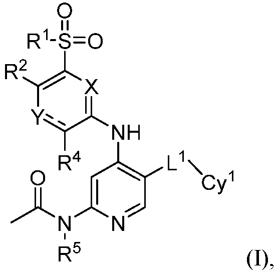 or a stereoisomer or a pharmaceutically acceptable salt thereof, wherein:
X is N or CH;
Y is N, NR³ or CR³;
L¹ is a direct bond;
R¹ is -C₁₋₆alkyl, -haloC₁₋₆alkyl, -C₁₋₆ alkoxy, -haloC₁₋₆ alkoxyl, -C₃₋₆ cycloalkyl, aryl, or -NR^{c}R^{d};
each of R², R³, and R⁴ is independently hydrogen, cyano, halogen, -C₁₋₆alkyl, -C₂₋₆alkenyl, -C₂₋₆alkynyl, -C₃₋₈cycloalkyl, heterocyclyl, aryl, heteroaryl, oxo, -NO₂, -OR^{e}, -SO₂R^{e}, -COR^{e}, -CO₂R^{e}, - CONR^{e}R^{f}, -C(=NR^{e})NR^{f}R^{g}, -NR^{e}R^{f}, -NR^{e}COR^{f}, -NR^{e}CONR^{f}R^{g}, -NR^{e}CO₂R^{f}, -NR^{e}SONR^{f}R^{g}, - NR^{e}SO₂NR^{f}R^{g}, or -NR^{e}SO₂R^{f},
wherein each of -C₁₋₆alkyl, -C₂₋₆alkenyl, -C₂₋₆alkynyl, -C₃₋₈cycloalkyl, heterocyclyl, aryl, or heteroaryl is independently and unsubstituted or substituted with at least one substituent selected from
i) cyano, -oxo-, halogen, -NR^{m}Rⁿ, -OR^{h}, -C(O)NR^{m}Rⁿ;
ii) heterocyclyl unsubstituted or substituted unsubstituted or substituted with at least one substituent independently selected from cyano, -oxo, halogen, hydroxy, -NR^{m}Rⁿ, substituted or unsubstituted -C₁₋₆alkyl, substituted or unsubstituted -C₁₋₆alkoxy or -C(O)NR^{m}R; or,
iii) C₁₋₆alkyl unsubstituted or substituted with at least one substitution independently selected from cyano, halogen, hydroxy, -NH₂or C₁₋₆alkoxy;
wherein R^{h} is hydrogen, hydroxy, -NH₂, -C₁₋₆alkyl, C₁₋₆alkyl substituted with hydroxy, or heterocyclyl,
R^{e}, R^{f}, and R^{g} are each independently hydrogen, -C₁₋₆alkyl, -C₁₋₆alkoxy, -C₂₋₆alkenyl, -C₂₋₆alkynyl, cycloalkyl, heterocyclyl, aryl, or heteroaryl, wherein each of -C₁₋₆alkyl, -C₂₋₆alkenyl, -C₂₋₆alkynyl, cycloalkyl, heterocyclyl, aryl, or heteroaryl is optionally independently substituted with one to three substituents selected from cyano, -oxo-, halogen, hydroxy, -NR^{m}Rⁿ, -C₁₋₆alkyl, -C₁₋₆alkoxy, - C₃₋₆cycloalkyl unsubstituted or substituted with halogen, hydroxy or -C₁₋₆alkoxy, -C(O)NR^{m}Rⁿ, or heterocyclyl;
R⁵ is hydrogen or C₁₋₆alkyl;
Cy¹ is 6- to 12- membered aryl or 5- to 14- membered heteroaryl, or 5- to 14- membered heterocyclyl, each of which is unsubstituted or substituted with at least one substituent Rⁱ,
Rⁱ is independently halogen, cyano, -C₁₋₆alkyl, -C₂₋₆alkenyl, -C₂₋₆alkynyl, -C₃₋₈cycloalkyl, heterocyclyl, aryl, heteroaryl, oxo, cyano (-CN), -NO₂, -OR^{j}, -SO₂R^{j}, -COR^{j}, -CO₂R^{k}, -CONR^{j}R^{k}, - C(=NR^{j})NR^{k}R^{l}, -NR^{j}R^{k}, -NR^{j}COR^{k}, -NR^{j}CONR^{k}R^{l}, -NR^{j}CO₂R^{k}, -NR^{j}SONR^{k}R^{l}, -NR^{j}SO₂NR^{k}R^{l}, or-NR^{j}SO₂R^{k},
wherein each of -C₁₋₆alkyl, -C₂₋₆alkenyl, -C₂₋₆alkynyl, -C₃₋₈cycloalkyl, heterocyclyl, aryl, or heteroaryl is unsubstituted or substituted with halogen, -OR^{m}, -C(O)R^{m}, -NR^{m}Rⁿ, -C₁₋₆alkyl, C₁₋₆alkoxy-, C₁₋₆alkyl substituted with -C₁₋₆alkoxy or -oxo-;
R^{j}, R^{k}, R^{l}, R^{m}, Rⁿ are each independently hydrogen, -C₁₋₆alkyl, C₁₋₆alkoxy-C₁₋₆alkyl-, -C₂₋₆alkenyl, -C₂₋₆alkynyl, cycloalkyl, heterocyclyl, aryl, or heteroaryl;
or (R¹ and R²), or (R² and R³), or (R³ and R⁴), together with the atoms to which they are attached, form a fused ring system, said fused ring system comprises 0-4 heteroatoms selected from oxygen (O), nitrogen (N) or sulfur (S) as ring member(s) and is optionally and independently substituted with halogen, -C₁₋₆alkyl, -C₁₋₆alkoxy, C₁₋₆alkyl substituted with halogen, C₁₋₆alkoxy substituted with halogen or -C₃₋₆cycloalkyl;
any of the said alkyl or alkoxy is optionally enriched in deuterium.

2. The compound of claim 1, wherein the compound is Formula (I-A) 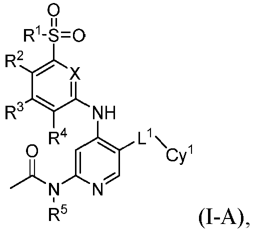
or a stereoisomer or a pharmaceutically acceptable salt thereof, wherein:
X is N or CH;
L¹ is a direct bond;
R¹ is -C₁₋₆alkyl, -haloC₁₋₆alkyl, -C₁₋₆ alkoxy, -haloC₁₋₆ alkoxyl, -C₃₋₆ cycloalkyl, aryl, or -NR^{m}Rⁿ;
each of R², R³, and R⁴ is independently hydrogen, halogen, -C₁₋₆alkyl, -C₂₋₆alkenyl, -C₂₋₆alkynyl, - C₃₋₈cycloalkyl, heterocyclyl, aryl, heteroaryl, -oxo-, -CN, -NO₂, -OR^{e}, -SO₂R^{e}, -COR^{e}, -CO₂R^{e}, - CONR^{e}R^{f}, -C(=NR^{e})NR^{f}R^{g}, -NR^{e}R^{f}, -NR^{e}COR^{f}, -NR^{e}CONR^{f}R^{g}, -NR^{e}CO₂R^{f}, -NR^{e}SONR^{f}R^{g}, - NR^{e}SO₂NR^{f}R^{g}, or -NR^{e}SO₂R^{f},
wherein each of -C₁₋₆alkyl, -C₂₋₆alkenyl, -C₂₋₆alkynyl, -C₃₋₈cycloalkyl, heterocyclyl, aryl, or heteroaryl is independently and unsubstituted or substituted with at least one substituents selected from cyano, oxo, halogen, C₁₋₆alkyl unsubstituted or substituted with halogen, -C₁₋₆alkyl substituted with hydroxy (preferably, hydroxymethyl, hydroxyethyl), -OR^{h}, -C(O)NR^{m}Rⁿ, -NH₂, C₁₋₆alkyl substituted with NH₂ or -C₁₋₆alkyl substituted with C₁₋₆alkoxy;
wherein R^{h} is hydrogen, alkyl, hydroxy-C₁₋₆alkyl or heterocyclyl,
R^{e}, R^{f}, and R^{g} are each independently hydrogen, -C₁₋₆alkyl, -C₁₋₆alkoxy, -C₂₋₆alkenyl, -C₂₋₆alkynyl, cycloalkyl, heterocyclyl, aryl, or heteroaryl, wherein each of -C₁₋₆alkyl, -C₂₋₆alkenyl, -C₂₋₆alkynyl, cycloalkyl, heterocyclyl, aryl, or heteroaryl is optionally independently substituted with one to three substituents selected from halogen, hydroxy, cyano, or -C₁₋₆alkoxy; -C₃₋₆cycloalkyl unsubstituted or substituted with halogen, hydroxy, or C₁₋₆alkoxy, or heterocyclyl;
R⁵ is hydrogen or C₁₋₆alkyl;
Cy¹ is 6- to 12- membered aryl or 5- to 14- membered heteroaryl, or 5- to 14- membered heterocyclyl, each of which is unsubstituted or substituted with at least one substituent Rⁱ,
Rⁱ is independently halogen, cyano, -C₁₋₆alkyl, -C₂₋₆alkenyl, -C₂₋₆alkynyl, -C₃₋₈cycloalkyl, heterocyclyl, aryl, heteroaryl, oxo, -CN, -NO₂, -OR^{j}, -SO₂R^{j}, -COR^{j}, -CO₂R^{k}, -CONR^{j}R^{k}, - C(=NR^{j})NR^{k}R^{l}, -NR^{j}R^{k}, -NR^{j}COR^{k}, -NR^{j}CONR^{k}R^{l}, -NR^{j}CO₂R^{k}, -NR^{j}SONR^{k}R^{l}, -NR^{j}SO₂NR^{k}R^{l}, or - NR^{j}SO₂R^{k},
wherein each of said -C₁₋₆alkyl, -C₂₋₆alkenyl, -C₂₋₆alkynyl, -C₃₋₈cycloalkyl, heterocyclyl, aryl, or heteroaryl is unsubstituted or substituted with halogen, OR^{m}, C(O)R^{m}, -NR^{m}Rⁿ, -C₁₋₆alkyl, C₁₋₆alkoxy-, C₁₋₆alkoxy-C₁₋₆alkyl-, or oxo;
R^{j}, R^{k}, R^{l}, R^{m}, Rⁿ are each independently hydrogen, -C₁₋₆alkyl, -C₁₋₆alkyl substituted with C₁₋₆alkoxy, -C₂₋₆alkenyl, -C₂₋₆alkynyl, cycloalkyl, heterocyclyl, aryl, or heteroaryl;
or (R¹ and R²), or (R² and R³), or (R³ and R⁴), together with the atoms to which they are attached, form a fused ring system, said fused ring system comprises 0-4 heteroatoms selected from oxygen, nitrogen or sulfur as ring member(s) and is optionally and independently substituted with halogen, - C₁₋₆alkyl, -C₁₋₆alkoxy, -haloC₁₋₆alkyl, -haloC₁₋₆alkoxy, or -C₃₋₆cycloalkyl;
any of the said alkyl or alkoxy is optionally enriched in deuterium.

3. The compound of claim 1 or claim 2, wherein R¹ is -C₁₋₃ alkyl, -NR^{c}R^{d} or -C₃₋₆ cycloalkyl, preferably - NH₂, methyl, ethyl, propyl, isopropyl, cyclopropyl or cyclopentyl.

4. The compound of claim 1 or claim 2, wherein R² and R⁴ are each independently hydrogen, halogen, - C₁₋₆alkyl, or -C₁₋₆alkoxy, preferably hydrogen, fluoro, methyl, methoxy, ethoxy, or isopropoxy.

5. The compound of any one of claims 1-4, wherein R³ is
- hydrogen;
- cyano;
- halogen;
- -C₁₋₄ alkyl unsubstituted or substituted with at least one substituent independently selected from halogen, 3- to 6-membered heterocyclyl or -OR^{h}, wherein
said 3- to 6-membered heterocyclyl comprises one or two heteroatoms selected from oxygen (O), nitrogen (N) or sulfur (O) as ring member(s), unsubstituted or substituted with at least one substituent independently selected from cyano, -oxo, halogen, hydroxy, -NR^{m}Rⁿ, C₁₋₆alkyl, -C₁₋₆alkoxy or -C(O)NR^{m}Rⁿ, wherein -C₁₋₆alkyl or C₁₋₆alkoxy is substituted with at least one substitution independently selected from cyano, halogen, hydroxy, -NH₂, -C₁₋₆alkyl or C₁₋₆alkoxy, and,
R^{h} is hydrogen, alkyl or heterocyclyl (preferably 3- to 6-membered heterocyclyl, e.g., tetrahydrofuranyl, thiazolidinyl);
- -C₃₋₆cycloalkyl, unsubstituted or substituted with at least one substituent independently selected from cyano, -oxo, halogen, -NR^{m}Rⁿ, hydroxy, -C₁₋₆alkyl, -C₁₋₆alkoxy or - C(O)NR^{m}Rⁿ, wherein -C₁₋₆alkyl or C₁₋₆alkoxy is substituted with at least one substitution independently selected from cyano, halogen, hydroxy, -NH₂, -C₁₋₆alkyl or C₁₋₆alkoxy;
- heterocyclyl, preferably 4- to 6-membered monocyclic saturated heterocyclyl, saturated mono-spiro heterocyclyl, saturated bicyclic fused heterocyclyl, or saturated bridged heterocyclyl, comprising one or two heteroatoms selected from oxygen (O), nitrogen (N) or sulfur (S) as ring member(s), more preferably morpholinyl, tetrahydrofuranyl, tetrahydropyranyl, pyrrolidinyl, 1,4-dioxanyl, piperidinyl or azetidinyl, unsubstituted or substituted with at least one substituent independently selected from cyano, -oxo, halogen, hydroxy, -C₁₋₆alkyl, alkoxy, -NR^{m}Rⁿ, or -C(O)NR^{m}Rⁿ, and wherein -C₁₋₆alkyl or -C₁₋₆alkoxy is substituted with at least one substitution independently selected from cyano, halogen, hydroxy, -NH₂, -C₁₋₆alkyl or C₁₋₆alkoxy;
- -OR^{e}, wherein R^{e} is -C₁₋₆alkyl, -C₃₋₆cycloalkyl, 3- to 6-membered heterocyclyl (preferably 4- to 6-membered monocyclic saturated heterocyclyl comprising one oxygen heteroatom as ring member), or aryl, wherein
i) -C₁₋₆alkyl is unsubstituted or substituted with cyano, -oxo-, halogen, hydroxy, -NR^{m}Rⁿ, -C₁₋₆alkoxy-, -C₃₋₆cycloalkyl unsubstituted or substituted with cyano, -oxo, halogen, hydroxy, -NR^{m}Rⁿ, -C₁₋₆alkyl, C₁₋₆alkoxy or - C(O)NR^{m}Rⁿ, 4- to 6-membered heterocyclyl unsubstituted or substituted with cyano, halogen, hydroxy, -C₁₋₆alkyl or -C₁₋₆alkoxy; and,
ii) -C₃₋₆cycloalkyl or 3- to 6-membered heterocyclyl is unsubstituted or substituted with cyano, -oxo, halogen, hydroxy, NR^{m}Rⁿ, C₁₋₆alkyl, C₁₋₆alkoxy or -C(O)NR^{m}Rⁿ, wherein -C₁₋₆alkyl or C₁₋₆alkoxy is substituted with at least one substitution independently selected from cyano, halogen, hydroxy, -NH₂, -C₁₋₆alkyl or C₁₋₆alkoxy;
- -C₅₋₁₀aryl; or
- heteroaryl, preferably 5- to 6-membered heteroaryl comprising one oxygen (O), nitrogen (N) or sulfur (S) heteroatom as ring member, unsubstituted or substituted with at least one substitution independently selected from cyano, -oxo, halogen, hydroxy, -NR^{m}Rⁿ, -C₁₋₆alkyl, -C₁₋₆alkoxy or -C(O)NR^{m}Rⁿ, wherein -C₁₋₆alkyl or C₁₋₆alkoxy is substituted with at least one substitution independently selected from cyano, halogen, hydroxy, -NH₂, -C₁₋₆alkyl or C₁₋₆alkoxy;
and wherein R^{m} and Rⁿ are independently selected from hydrogen or C₁₋₃alkyl.

6. The compound of claim 5, wherein R³ is
- Hydrogen;
- Methyl, 1-methoxyethyl, 2-hydroxypropan-2-yl, 1-methoxyethyl, or (2,4-dioxothiazolidin-3-yl)methyl;
- Isopropoxy, methoxy-d3, methoxy, ethoxy, difluoromethoxy, 2-methoxyethoxy, 2-methoxy-2-methylpropoxy, 2-hydroxy-2-methylpropoxy, cyclopropylmethoxy, (1,4-dioxan-2-yl)methoxy, cyclobutoxy, (4-hydroxycyclohexyl)oxy, (cis-4-hydroxycyclohexyl)oxy, (trans-4-hydroxycyclohexyl)oxy, (4-methoxycyclohexyl)oxy, (cis-4-methoxycyclohexyl)oxy, (trans-4-methoxycyclohexyl)oxy, (3-methyloxetan-3-yl)methoxy, 2-methyl-2-morpholinopropoxy;
- cyano
- 3-methoxycyclobutyl, (trans)-3-methoxycyclobutyl, (cis)-3-methoxycyclobutyl, 2,2-dichlorocyclopropyl, or 1-cyanocyclopropyl;
- Morpholino, 3-methyl-morpholino, 3(R)-methyl-morpholino, 3(S)-methyl-morpholino, 3,3-dimethylmorpho;
- tetrahydro-2H-pyran-4-yl, tetrahydro-2H-pyran-3-yl, (R)-tetrahydro-2H-pyran-3-yl, (S)-tetrahydro-2H-pyran-3-yl, 2,2,6,6-tetramethyltetrahydro-2H-pyran-4-yl;
- 3-methoxypyrrolidin-1-yl, 3(R)-methoxypyrrolidin-1-yl, 3(S)-methoxypyrrolidin-1-yl, 3-hydroxy-3-methylpyrrolidin-1-yl, 3-(2-hydroxyethoxy)pyrrolidin-1-yl, 3-(trifluoromethoxy)pyrrolidin-1-yl, 3(R)-(trifluoromethoxy)pyrrolidin-1-yl, 3(S)-(trifluoromethoxy)pyrrolidin-1-yl, 2-(aminocarbonyl)pyrrolidin-1-yl, 2(R)-(aminocarbonyl)pyrrolidin-1-yl, 2(S)-(aminocarbonyl)pyrrolidin-1-yl, 3-(methoxymethyl)pyrrolidin-1-yl, 3(R)-(methoxymethyl)pyrrolidin-1-yl, 3(S)-(methoxymethyl)pyrrolidin-1-yl, 3-cyano-4-hydroxypyrrolidin-1-yl, cis-3-cyano-4-hydroxypyrrolidin-1-yl, trans-3-cyano-4-hydroxypyrrolidin-1-yl, 3-cyano-4-methoxypyrrolidin-1-yl, cis-3-cyano-4-methoxypyrrolidin-1-yl, trans-3-cyano-4-methoxypyrrolidin-1-yl, 2-(methoxymethyl)pyrrolidin-1-yl, 2(R)-(methoxymethyl)pyrrolidin-1-yl, 2(S)-(methoxymethyl)pyrrolidin-1-yl, 3-methylpyrrolidin-1-yl, 3(R)-methylpyrrolidin-1-yl, 3(S)-methylpyrrolidin-1-yl, pyrrolidin-1-yl, 3-(cyanomethoxy)pyrrolidin-1-yl;
- 5-azaspiro[2.4]heptan-5-yl;
- tetrahydrofuran-3-yl;
- 3-methoxyazetidin-1-yl, 3-hydroxy-3-methylazetidin-1-yl;
- 1,4-dioxan-2-yl;
- 4-aminotetrahydro-2H-pyran-4-yl, 4-(aminomethyl)tetrahydro-2H-pyran-4-yl,
- 4-methoxypiperidin-1-yl, 4-hydroxy-4-methylpiperidin-1-yl, 1-(2,2,2-trifluoroethyl)piperidin-4-yl, 3-methoxypiperidin-1-yl, 3(R)-methoxypiperidin-1-yl, 3(S)-methoxypiperidin-1-yl, 3-ethoxypiperidin-1-yl, 3(R)-ethoxypiperidin-1-yl, 3(S)-ethoxypiperidin-1-yl;
- 3-cyano-2-azabicyclo[3.1.0]hexan-2-yl, (3R)-3-cyano-2-azabicyclo[3.1.0]hexan-2-yl, (3S)-3-cyano-2-azabicyclo[3.1.0]hexan-2-yl, 3-azabicyclo[3.1.0]hexan-3-yl;4-methylpyridin-3-yl, 5-methylpyridazin-4-yl, 5-methoxypyridazin-4-yl, 3,5-dimethylisoxazol-4-yl, 4-methoxypyridin-3-yl, 4-(2-hydroxypropan-2-yl)pyridin-3-yl, 6-cyanopyridin-3-yl, 4-cyanopyridin-3-yl, 2-cyanopyridin-3-yl, 3-methylpyrazin-2-yl, 5-cyanopyridazin-4-yl, 5-fluoropyridazin-4-yl, 4-fluoropyridin-3-yl, 4-isopropylpyridin-3-yl, 4-(1-hydroxyethyl)pyridin-3-yl, 4-(1-methoxyethyl)pyridin-3-yl, pyridin-2-yl, or thiazol-4-yl.

7. The compound of claim 1 or claim 2, wherein (R¹ and R²), or (R² and R³), or (R³ and R⁴), together with the atoms to which they are attached, form a fused 5- to 7-membered ring system, said fused ring system comprises 0-2 oxygen heteroatoms as ring member(s) and is optionally and independently substituted with halogen, -C₁₋₆alkyl, -C₁₋₆alkoxy, -haloC₁₋₆alkyl, -haloC₁₋₆alkoxy, or -C₃₋₆cycloalkyl.

8. The compound of claim 1 or claim 2, wherein R¹ and R², together with the atoms to which they are attached, form a fused ring system selected from 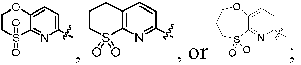 or R² and R³, together with the atoms to which they are attached, form a fused ring system 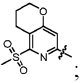 R³ and R⁴, together with the atoms to which they are attached, form a fused ring system selected from 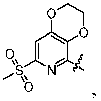 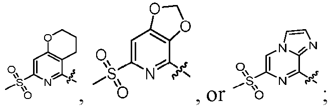 and wherein each of fused ring system is optionally and independently substituted with halogen, -C₁₋₆alkyl, -C₁₋₆alkoxy, -haloC₁₋₆alkyl, -haloC₁₋₆alkoxy, or - C₃₋₆cycloalkyl .

9. The compound of any one of claims 1-8, wherein L is a bond.

10. The compound of any one of claims 1-8, wherein Cy¹ is
a. a 5- to 7-membered monocyclic heterocyclyl or heteroaryl comprising 1, 2, 3 or 4 heteroatoms selected from oxygen, nitrogen or sulfur as ring member(s), or
b. 7- to 14- membered bicyclic or tricyclic heterocyclyl or heteroaryl having 1, 2, or 3 heteroatoms selected from oxygen, nitrogen or sulfur as ring member(s),
each of which is unsubstituted or substituted with at least one substituent Rⁱ.

11. The compound of claim 10, wherein Cy¹ is
a. said 5- to 7-membered monocyclic heteroaryl comprising 1, 2, 3 or 4 heteroatom(s) selected from oxygen (O), nitrogen (N) or sulfur (S) as ring member(s), preferably pyrazolyl, triazolyl, imidazolyl, thiazolyl, oxazolyl, furanyl, pyridinyl, pyridazinyl, pyrazinyl or pyrimidinyl, said monocyclic heteroaryl is unsubstituted or substituted with one or two substituents selected from
i. halogen;
ii. cyano;
iii. -C₁₋₆alkyl unsubstituted or substituted with halogen, hydroxy, -C₁₋₆alkoxy, - C(O)R^{m} (preferably R^{m} is morpholinyl), or -NR^{m}Rⁿ;
iv. heterocyclyl unsubstituted or substituted with halogen, C₁₋₆alkyl-, -C₁₋₆alkyl substituted with -C₁₋₆alkoxy, or oxo; preferably said heterocyclyl is selected from tetrahydrofuranyl (preferably tetrahydrofuran-3-yl), morpholinyl (preferably morpholino), 2-oxa-5-azabicyclo[2.2.1]heptanyl (preferably 2-oxa-5-azabicyclo[2.2.1]heptan-2-yl), 8-oxa-3-azabicyclo[3.2.1]octanyl (preferably 8-oxa-3-azabicyclo[3.2.1]octan-8-yl), isoindolinyl (preferably isoindolin-2-yl), each of which is unsubstituted or substituted with methyl, ethyl, propyl, isopropyl, isobutyl, tert-butyl, n-butyl, methoxymethyl, methoxyethyl, ethoxymethyl, ethoxyethyl, or oxo;
v. -C₃₋₆cycloalkyl unsubstituted or substituted with halogen,- oxo, -C₁₋₆alkyl, C₁₋₆alkoxy-, or -C₁₋₆alkyl substituted with -C₁₋₆alkoxy; or
vi. -OR^{j}, wherein R^{j} is -C₁₋₆alkyl, -C₁₋₆alkyl substituted with -C₁₋₆alkoxy, or heterocyclyl;
vii. oxo;
b. said 7- to 14-membered bicyclic or tricyclic heteroaryl comprising 1, 2, or 3 heteroatom(s) selected from oxygen, nitrogen or sulfur as ring member(s), preferably benzoimidazolyl, imidazopyrimidinyl, pyrazolopyrazinyl, pyrazolopyrimidinyl, benzothiophenyl, benzothiazolyl, benzoisoxazolyl, benzooxazolyl, benzoisothiazolyl, imidazopyridazinyl, imidazopyridazinyl; dihydro-4H-furo[3,2-c]pyranyl, 6,7-dihydro-4H-thieno[3,2-c]pyranyl, 2,3-dihydropyrazolo[5,1-b]oxazolyl, 4,5,6,7-tetrahydrofuro[3,2-c]pyridinyl, 1,3a,4,6,7,7a-hexahydropyrano[4,3-c]pyrazolyl, 4,5,6,7-tetrahydropyrazolo[1,5-a]pyrazinyl, 4,5,6,7-tetrahydropyrazolo[1,5-a]pyrimidinyl, 4,5,6,7-tetrahydropyrazolo[1,5-a]pyrazinyl, 4,5,6,7-tetrahydrothiazolo[5,4-c]pyridinyl, 4,5,6,7-tetrahydropyrazolo[1,5-a]pyrazinyl, 6,7-dihydro-4H-pyrazolo[5,1-c][1,4]oxazinyl, 2,3-dihydropyrazolo[5,1-b]oxazolyl, 2,3-dihydropyrazolo[5,1-b]oxazolyl, 1,3a,4,6,7,7a-hexahydropyrano[4,3-c]pyrazolyl, 6,7-dihydro-4H-pyrano[4,3-d]thiazolyl, [1,3]dioxolo[4,5-c]pyridinyl, 2,3-dihydro-[1,4]dioxino[2,3-c]pyridinyl, 3,4-dihydro-2H-pyrido[3,2-b][1,4]oxazinyl, 2,3-dihydro-[1,4]dioxino[2,3-b]pyridinyl, 2,3-dihydro-[1,4]dioxino[2,3-b]pyridin-6-yl, 3,4-dihydro-2H-pyrido[4,3-b][1,4]oxazinyl, or 2H-pyrido[3,2-b][1,4]oxazin-4(3H)-yl, each of which is unsubstituted or substituted with halogen, -C₁₋₆alkyl, -NH₂, or -C(O)R^{m}, wherein R^{m} is C₁₋₆alkyl; any of the said alkyl is optionally enriched in deuterium.

12. The compound of claim 10, wherein Cy¹ is
a. 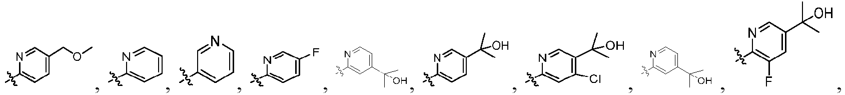 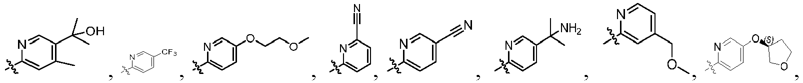 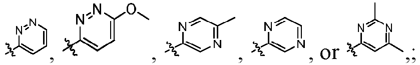
b. 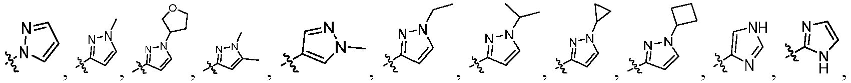 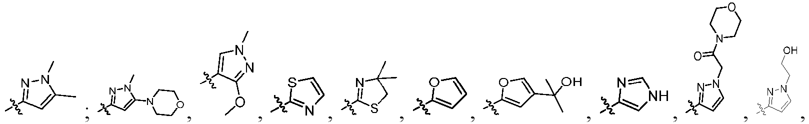 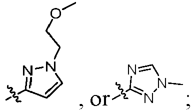
c. 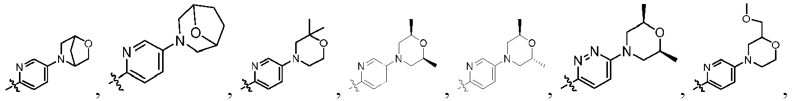 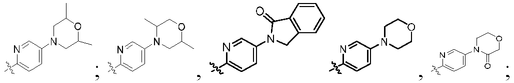
d. 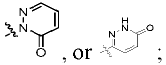
e. 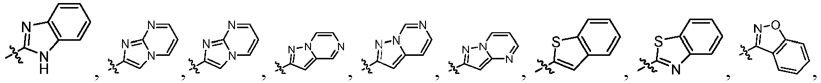 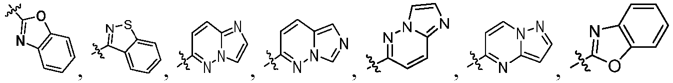
f. 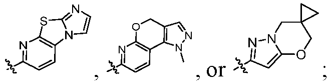
g. 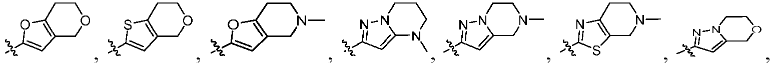 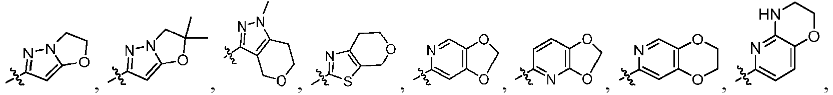 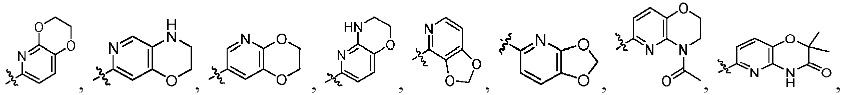 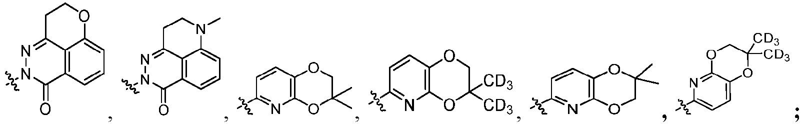
h.

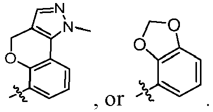

13. A compound according to any one of claims 1-12, wherein the compound is selected from:
N-(4-((3-(methylsulfonyl)phenyl)amino)-5-(pyridazin-3-yl)pyridin-2-yl)acetamide;
N-[4-[(3-methanesulfonylphenyl)amino]-5-(oxolan-2-yl)pyridin-2-yl]acetamide;
N-(5-(1H-imidazol-4-yl)-4-((6-(methylsulfonyl)pyridin-2-yl)amino)pyridin-2-yl)acetamide;
N-(5-(5-methylpyrazin-2-yl)-4-((3-(methylsulfonyl)phenyl)amino)pyridin-2-yl)acetamide;
N-(4-((3-(methylsulfonyl)phenyl)amino)-5-(pyrazin-2-yl)pyridin-2-yl)acetamide;
N-(5-(2,6-dimethylpyrimidin-4-yl)-4-((3-(methylsulfonyl)phenyl)amino)pyridin-2-yl)acetamide;
N-(4-((3-(methylsulfonyl)phenyl)amino)-5-(2-oxopyrrolidin-1-yl)pyridin-2-yl)acetamide;
N-(5-(1-methyl-1H-pyrazol-4-yl)-4-((6-(methylsulfonyl)pyridin-2-yl)amino)pyridin-2-yl)acetamide;
N-(5-(furan-2-yl)-4-((4-methyl-6-(methylsulfonyl)pyridin-2-yl)amino)pyridin-2-yl)acetamide;
N-(5-(1-methyl-1H-1,2,4-triazol-3-yl)-4-((3-(methylsulfonyl)phenyl)amino)pyridin-2-yl)acetamide;
N-(4-((3-methoxy-6-(methylsulfonyl)pyridin-2-yl)amino)-5-(1-methyl-1H-pyrazol-4-yl)pyridin-2-yl)acetamide;
N-(4-((4-isopropoxy-6-(methylsulfonyl)pyridin-2-yl)amino)-5-(1H-pyrazol-1-yl)pyridin-2-yl)acetamide;
N-(4-((4-methyl-6-(methylsulfonyl)pyridin-2-yl)amino)-5-(1-(2-morpholino-2-oxoethyl)-1H-pyrazol-3-yl)pyridin-2-yl)acetamide;
N-(5-(1-(2-hydroxyethyl)-1H-pyrazol-3-yl)-4-((4-isopropoxy-6-(methylsulfonyl)pyridin-2-yl)amino)pyridin-2-yl)acetamide;
N-(5-(1-(2-methoxyethyl)-1H-pyrazol-3-yl)-4-((4-methyl-6-(methylsulfonyl)pyridin-2-yl)amino)pyridin-2-yl)acetamide;
N-(6-cyano-4'-((4-isopropoxy-6-(methylsulfonyl)pyridin-2-yl)amino)-[2,3'-bipyridin]-6'-yl)acetamide;
N-(5-cyano-4'-((4-isopropoxy-6-(methylsulfonyl)pyridin-2-yl)amino)-[2,3'-bipyridin]-6'-yl)acetamide;
N-(5-(1H-imidazol-1-yl)-4-((4-isopropoxy-6-(methylsulfonyl)pyridin-2-yl)amino)pyridin-2-yl)acetamide;
N-(5-(1-cyclobutyl-1H-pyrazol-3-yl)-4-((4-isopropoxy-6-(methylsulfonyl)pyridin-2-yl)amino)pyridin-2-yl)acetamide;
N-(5-(2-aminopropan-2-yl)-4'-((4-methyl-6-(methylsulfonyl)pyridin-2-yl)amino)-[2,3'-bipyridin]-6'-yl)acetamide;
N-(5-(1H-imidazol-4-yl)-4-((4-(2-methoxyethoxy)-6-(methylsulfonyl)pyridin-2-yl)amino)pyridin-2-yl)acetamide;
N-(5-(1H-imidazol-4-yl)-4-((4-methyl-6-(methylsulfonyl)pyridin-2-yl)amino)pyridin-2-yl)acetamide;
N-(5-(1H-imidazol-4-yl)-4-((4-isopropoxy-6-(methylsulfonyl)pyridin-2-yl)amino)pyridin-2-yl)acetamide;
N-(4-((6-(methylsulfonyl)pyridin-2-yl)amino)-5-(pyridazin-3-yl)pyridin-2-yl)acetamide;
N-(4-((4-(cyclopropylmethoxy)-6-(methylsulfonyl)pyridin-2-yl)amino)-5-(6-methoxypyridazin-3-yl)pyridin-2-yl)acetamide;
N-(4-((4-isopropoxy-6-(methylsulfonyl)pyridin-2-yl)amino)-5-(6-methoxypyridazin-3-yl)pyridin-2-yl)acetamide;
N-(4-((4-((2S,6R)-2,6-dimethylmorpholino)-6-(methylsulfonyl)pyridin-2-yl)amino)-5-(6-methoxypyridazin-3-yl)pyridin-2-yl)acetamide;
N-(5-(6-methoxypyridazin-3-yl)-4-((6-(methylsulfonyl)-[1,3]dioxolo[4,5-c]pyridin-4-yl)amino)pyridin-2-yl)acetamide;
N-(5-(6-isopropoxypyridazin-3-yl)-4-((4-methyl-6-(methylsulfonyl)pyridin-2-yl)amino)pyridin-2-yl)acetamide;
N-(5-(6-isopropoxypyridazin-3-yl)-4-((4-methoxy-6-(methylsulfonyl)pyridin-2-yl)amino)pyridin-2-yl)acetamide;
N-(5-(6-isopropoxypyridazin-3-yl)-4-(4-(methoxy-d3)-6-(methylsulfonyl)pyridin-2-ylamino)pyridin-2-yl)acetamide;
N-(4'-((3-(methylsulfonyl)phenyl)amino)-[2,3'-bipyridin]-6'-yl)acetamide;
N-(4-((6-(methylsulfonyl)pyridin-2-yl)amino)-[3,3'-bipyridin]-6-yl)acetamide;
N-(4'-(phenylamino)-[2,3'-bipyridin]-6'-yl)acetamide;
N-(4'-((6-sulfamoylpyridin-2-yl)amino)-[2,3'-bipyridin]-6'-yl)acetamide;
N-(4'-((6-(methylsulfonyl)pyridin-2-yl)amino)-[2,3'-bipyridin]-6'-yl)acetamide;
N-(4'-((4-(2-methoxyethoxy)-6-(methylsulfonyl)pyridin-2-yl)amino)-[2,3'-bipyridin]-6'-yl)acetamide;
N-(4'-((4-(2-hydroxyethoxy)-6-(methylsulfonyl)pyridin-2-yl)amino)-[2,3'-bipyridin]-6'-yl)acetamide;
N-(4-(methoxymethyl)-4'-((6-(methylsulfonyl)pyridin-2-yl)amino)-[2,3'-bipyridin]-6'-yl)acetamide;
N-(4-(2-hydroxypropan-2-yl)-4'-((3-(methylsulfonyl)phenyl)amino)-[2,3'-bipyridin]-6'-yl)acetamide;
N-(4-(2-hydroxypropan-2-yl)-4'-((4-methyl-6-(methylsulfonyl)pyridin-2-yl)amino)-[2,3'-bipyridin]-6'-yl)acetamide;
N-(4'-((3-(methylsulfonyl)phenyl)amino)-5-morpholino-[2,3'-bipyridin]-6'-yl)acetamide;
N-(5-(2,2-dimethylmorpholino)-4'-((6-(methylsulfonyl)pyridin-2-yl)amino)-[2,3'-bipyridin]-6'-yl)acetamide;
N-(4'-((3-methoxyphenyl)amino)-[2,3'-bipyridin]-6'-yl)acetamide;
N-(4'-((3-(methylsulfonyl)pyridin-2-yl)amino)-[2,3'-bipyridin]-6'-yl)acetamide;
N-(4'-((2-(methylsulfonyl)phenyl)amino)-[2,3'-bipyridin]-6'-yl)acetamide;
N-(5-(2-oxa-5-azabicyclo[2.2.1]heptan-5-yl)-4'-((4-isopropoxy-6-(methylsulfonyl)pyridin-2-yl)amino)-[2,3'-bipyridin]-6'-yl)acetamide;
N-(4-chloro-5-(2-hydroxypropan-2-yl)-4'-((6-(methylsulfonyl)pyridin-2-yl)amino)-[2,3'-bipyridin]-6'-yl)acetamide;
N-(4'-((4-isopropoxy-6-(methylsulfonyl)pyridin-2-yl)amino)-5-(2-(methoxymethyl)morpholino)-[2,3'-bipyridin]-6'-yl)acetamide;
N4'-(3-(methylsulfonyl)phenyl)-[2,3'-bipyridine]-4',6'-diamine;
N-(4'-((3-(methylsulfonyl)phenyl)amino)-[2,3'-bipyridin]-6'-yl)cyclopropanecarboxamide;
(S)-N-(4'-((4-methyl-6-(methylsulfonyl)pyridin-2-yl)amino)-5-((tetrahydrofuran-3-yl)oxy)-[2,3'-bipyridin]-6'-yl)acetamide;
N-(4'-((4-(2-hydroxyethoxy)-6-(methylsulfonyl)pyridin-2-yl)amino)-5-((tetrahydrofuran-3-yl)oxy)-[2,3'-bipyridin]-6'-yl)acetamide;
N-(4'-((6-(methylsulfonyl)pyridin-2-yl)amino)-5-((tetrahydrofuran-3-yl)oxy)-[2,3'-bipyridin]-6'-yl)acetamide;
N-(5-(8-oxa-3-azabicyclo[3.2.1]octan-3-yl)-4'-((4-methyl-6-(methylsulfonyl)pyridin-2-yl)amino)-[2,3'-bipyridin]-6'-yl)acetamide;
N-(5-(2,5-dimethylmorpholino)-4'-((4-methyl-6-(methylsulfonyl)pyridin-2-yl)amino)-[2,3'-bipyridin]-6'-yl)acetamid;
N-(5-(cis-2,6-dimethylmorpholino)-4'-((4-methyl-6-(methylsulfonyl)pyridin-2-yl)amino)-[2,3'-bipyridin]-6'-yl)acetamide;
N-(5-((cis)-2,6-dimethylmorpholino)-4'-((6-(methylsulfonyl)pyridin-2-yl)amino)-[2,3'-bipyridin]-6'-yl)acetamide;
N-(5-((cis)-2,6-dimethylmorpholino)-4'-((3-(methylsulfonyl)phenyl)amino)-[2,3'-bipyridin]-6'-yl)acetamide;
N-(5-((cis)-2,6-dimethylmorpholino)-4'-((4-isopropoxy-6-(methylsulfonyl)pyridin-2-yl)amino)-[2,3'-bipyridin]-6'-yl)acetamide;
N-(4'-((4-(cyclopropylmethoxy)-6-(methylsulfonyl)pyridin-2-yl)amino)-5-((cis)-2,6-dimethylmorpholino)-[2,3'-bipyridin]-6'-yl)acetamide;
N-(4'-((3-(cyclopropylmethoxy)-5-(methylsulfonyl)phenyl)amino)-5-((cis)-2,6-dimethylmorpholino)-[2,3'-bipyridin]-6'-yl)acetamide;
N-(5-((trans)-2,6-dimethylmorpholino)-4'-((4-methyl-6-(methylsulfonyl)pyridin-2-yl)amino)-[2,3'-bipyridin]-6'-yl)acetamide;
N-(5-(Cis-2,6-dimethylmorpholino)-4'-((7-(methylsulfonyl)-2,3-dihydro-[1,4]dioxino[2,3-c]pyridin-5-yl)amino)-[2,3'-bipyridin]-6'-yl)acetamide;
N-(5-((cis)-2,6-dimethylmorpholino)-4'-((4-methoxy-6-(methylsulfonyl)pyridin-2-yl)amino)-[2,3'-bipyridin]-6'-yl)acetamide;
N-(5-((cis)-2,6-dimethylmorpholino)-4'-((4-ethoxy-6-(methylsulfonyl)pyridin-2-yl)amino)-[2,3'-bipyridin]-6'-yl)acetamide;
N-(5-((cis)-2,6-dimethylmorpholino)-4'-((4-(2-methoxyethoxy)-6-(methylsulfonyl)pyridin-2-yl)amino)-[2,3'-bipyridin]-6'-yl)acetamide;
N-(5-((cis)-2,6-dimethylmorpholino)-4'-((4-((R)-2-hydroxypropoxy)-6-(methylsulfonyl)pyridin-2-yl)amino)-[2,3'-bipyridin]-6'-yl)acetamide;
N-(4'-((4-((R)-sec-butoxy)-6-(methylsulfonyl)pyridin-2-yl)amino)-5-((cis)-2,6-dimethylmorpholino)-[2,3'-bipyridin]-6'-yl)acetamide;
N-(5-((cis)-2,6-dimethylmorpholino)-4'-((3-methoxy-5-(methylsulfonyl)phenyl)amino)-[2,3'-bipyridin]-6'-yl)acetamide;
N-(5-((cis)-2,6-dimethylmorpholino)-4'-((3-fluoro-5-(methylsulfonyl)phenyl)amino)-[2,3'-bipyridin]-6'-yl)acetamide;
N-(4'-((3-cyano-5-(methylsulfonyl)phenyl)amino)-5-((cis)-2,6-dimethylmorpholino)-[2,3'-bipyridin]-6'-yl)acetamide;
N-(5-(cis-2,6-dimethylmorpholino)-4'-((4-methoxy-6-(methylsulfonyl)pyridin-2-yl)amino)-[2,3'-bipyridin]-6'-yl)acetamide;
N-(5-((cis)-2,6-dimethylmorpholino)-4'-((4-(methoxy-d3)-6-(methylsulfonyl)pyridin-2-yl)amino)-[2,3'-bipyridin]-6'-yl)acetamide;
N-(5-(cis-2,6-dimethylmorpholino)-4'-((4-(1-methoxyethyl)-6-(methylsulfonyl)pyridin-2-yl)amino)-[2,3'-bipyridin]-6'-yl)acetamide;
N-(5-((cis)-2,6-dimethylmorpholino)-4'-((4-((R or S)-1-methoxyethyl)-6-(methylsulfonyl)pyridin-2-yl)amino)-[2,3'-bipyridin]-6'-yl)acetamide;
N-(5-((cis)-2,6-dimethylmorpholino)-4'-((4-((R or S)-1-methoxyethyl)-6-(methylsulfonyl)pyridin-2-yl)amino)-[2,3'-bipyridin]-6'-yl)acetamide;
N-(5-(cis-2,6-dimethylmorpholino)-4'-((6-(methylsulfonyl)-4-(tetrahydro-2H-pyran-4-yl)pyridin-2-yl)amino)-[2,3'-bipyridin]-6'-yl)acetamide;
N-(5-(cis-2,6-dimethylmorpholino)-4'-((6-(methylsulfonyl)pyrazin-2-yl)amino)-[2,3'-bipyridin]-6'-yl)acetamide;
N-(5-(cis-2,6-dimethylmorpholino)-4'-((4-((S)-3-methylmorpholino)-6-(methylsulfon-yl)pyridin-2-yl)amino)-[2,3'-bipyridin]-6'-yl)ace-tamide.;
N-(5-(cis-2,6-dimethylmorpholino)-4'-((4-((R)-3-methylmorpholino)-6-(methylsulfonyl)pyridin-2-yl)amino)-[2,3'-bipyridin]-6'-yl)ace-tamide.;
N-(5-(cis-2,6-dimethyl morpholino)-4'-((4-(3-methoxy azetidin-1-yl)-6-(methylsulfonyl)pyridin - 2-yl)amino)-[2,3'-bipyridin]-6'-yl)acetamide;
N-(5-(2-methoxyethoxy)-4'-((6-(methylsulfonyl)pyridin-2-yl)amino)-[2,3'-bipyridin]-6'-yl)acetamide;
N-(5-(2-methoxyethoxy)-4'-((3-(methylsulfonyl)phenyl)amino)-[2,3'-bipyridin]-6'-yl)acetamide;
N-(5-(2-methoxyethoxy)-4'-((3-(methylsulfonyl)-5-(trifluoromethyl)phenyl)amino)-[2,3'-bipyridin]-6'-yl)acetamide;
N-(5-(2-methoxyethoxy)-4'-((4-methyl-6-(methylsulfonyl)pyridin-2-yl)amino)-[2,3'-bipyridin]-6'-yl)acetamide;
**N-(4'-((4-methoxy-6-(methylsulfonyl)pyridin-2-yl)amino)-5-(2-methoxyethoxy)-[2,3'-bipyridin]-**6'-yl)acetamide;
N-(4'-((4-isopropoxy-6-(methylsulfonyl)pyridin-2-yl)amino)-5-(2-methoxyethoxy)-[2,3'-bipyridin]-6'-yl)acetamide;
(R)-N-(5-(2-methoxyethoxy)-4'-((4-(2-methoxypropoxy)-6-(methylsulfonyl)pyridin-2-yl)amino)-[2,3'-bipyridin]-6'-yl)acetamide;
N-(5-(2-methoxyethoxy)-4'-((4-(2-methoxyethoxy)-6-(methylsulfonyl)pyridin-2-yl)amino)-[2,3'-bipyridin]-6'-yl)acetamide;
N-(4'-((4-ethoxy-6-(methylsulfonyl)pyridin-2-yl)amino)-5-(2-methoxyethoxy)-[2,3'-bipyridin]-6'-yl)acetamide;
N-(4'-((3-(cyclopropylmethoxy)-5-(methylsulfonyl)phenyl)amino)-5-(2-methoxyethoxy)-[2,3'-bipyridin]-6'-yl)acetamide;
N-(4'-((4-(cyclopropylmethoxy)-6-(methylsulfonyl)pyridin-2-yl)amino)-5-(2-methoxyethoxy)-[2,3'-bipyridin]-6'-yl)acetamide;
N-(5-(methoxymethyl)-4'-((3-(methylsulfonyl) phenyl)amino)-[2,3'-bipyridin]-6'-yl)acetamide;
N-(5-(methoxymethyl)-4'-((4-methyl-6-(methylsulfonyl)pyridin-2-yl)amino)-[2,3'-bipyridin]-6'-yl)acetamide;
(R)-N-(5-(methoxymethyl)-4'-((4-(2-methoxypropoxy)-6-(methylsulfonyl)pyridin-2-yl)amino)-[2,3'-bipyridin]-6'-yl)acetamide;
N-(4'-((4-isopropoxy-6-(methylsulfonyl)pyridin-2-yl)amino)-5-(methoxymethyl)-[2,3'-bipyridin]-6'-yl)acetamide;
N-(4'-((4-(2-methoxyethoxy)-6-(methylsulfonyl)pyridin-2-yl)amino)-5-(methoxymethyl)-[2,3'-bipyridin]-6'-yl)acetamide;
N-(4'-((4-(2-hydroxyethoxy)-6-(methylsulfonyl)pyridin-2-yl)amino)-5-(methoxymethyl)-[2,3'-bipyridin]-6'-yl)acetamide;
N-(4'-((4-methoxy-6-(methylsulfonyl)pyridin-2-yl)amino)-5-(methoxymethyl)-[2,3'-bipyridin]-6'-yl)acetamide;
N-(4'-((4-((1r,3r)-3-hydroxycyclobutoxy)-6-(methylsulfonyl)pyridin-2-yl)amino)-5-(methoxymethyl)-[2,3'-bipyridin]-6'-yl)acetamide;
N-(5-(methoxymethyl)-4'-((4-(methoxymethyl)-6-(methylsulfonyl)pyridin-2-yl)amino)-[2,3'-bipyridin]-6'-yl)acetamide;
N-(4'-((4-methyl-6-(methylsulfonyl)pyridin-2-yl)amino)-5-(trifluoromethyl)-[2,3'-bipyridin]-6'-yl)acetamide;
N-(4'-((3-(methylsulfonyl)phenyl)amino)-5-(trifluoromethyl)-[2,3'-bipyridin]-6'-yl)acetamide;
N-(4'-((3-methyl-5-(methylsulfonyl)phenyl)amino)-5-(trifluoromethyl)-[2,3'-bipyridin]-6'-yl)acetamide;
N-(4'-((3-cyano-5-(methylsulfonyl)phenyl)amino)-5-(trifluoromethyl)-[2,3'-bipyridin]-6'-yl)acetamide;
N-(4'-((3-((trans)-3-hydroxycyclobutoxy)-5-(methylsulfonyl)phenyl)amino)-5-(trifluoromethyl)-[2,3'-bipyridin]-6'-yl)acetamide;
N-(4'-((3-(cyclopropylmethoxy)-5-(methylsulfonyl)phenyl)amino)-5-(trifluoromethyl)-[2,3'-bipyridin]-6'-yl)acetamide;
N-(4'-((3-(2-hydroxyethoxy)-5-(methylsulfonyl)phenyl)amino)-5-(trifluoromethyl)-[2,3'-bipyridin]-6'-yl)acetamide;
N-(4'-((3-(2-methoxyethoxy)-5-(methylsulfonyl)phenyl)amino)-5-(trifluoromethyl)-[2,3'-bipyridin]-6'-yl)acetamide;
N-(4'-((4-isopropoxy-6-(methylsulfonyl)pyridin-2-yl)amino)-5-(trifluoromethyl)-[2,3'-bipyridin]-6'-yl)acetamide;
N-(4'-((4-ethoxy-6-(methylsulfonyl)pyridin-2-yl)amino)-5-(trifluoromethyl)-[2,3'-bipyridin]-6'-yl)acetamide;
N-(4'-((4-(2-methoxyethoxy)-6-(methylsulfonyl)pyridin-2-yl)amino)-5-(trifluoromethyl)-[2,3'-bipyridin]-6'-yl)acetamidecarboxamide;
N-(4'-((4-(2-hydroxyethoxy)-6-(methylsulfonyl)pyridin-2-yl)amino)-5-(trifluoromethyl)-[2,3'-bipyridin]-6'-yl)acetamide;
N-(5-fluoro-4'-((3-(methylsulfonyl)-5-(trifluoromethyl)phenyl)amino)-[2,3'-bipyridin]-6'-yl)acetamide;
N-(5-fluoro-4'-((3-isopropoxy-5-(methylsulfonyl)phenyl)amino)-[2,3'-bipyridin]-6'-yl)acetamide;
N-(5-fluoro-4'-((3-((trans)-3-hydroxycyclobutoxy)-5-(methylsulfonyl)phenyl)amino)-[2,3'-bipyridin]-6'-yl)acetamide;
N-(5-fluoro-4'-((4-(2-methoxyethoxy)-6-(methylsulfonyl)pyridin-2-yl)amino)-[2,3'-bipyridin]-6'-yl)acetamide;
N-(5-fluoro-4'-((4-methyl-6-(methylsulfonyl)pyridin-2-yl)amino)-[2,3'-bipyridin]-6'-yl)acetamide;
N-(5-fluoro-4'-((4-isopropoxy-6-(methylsulfonyl)pyridin-2-yl)amino)-[2,3'-bipyridin]-6'-yl)acetamide;
N-(4'-((4-ethoxy-6-(methylsulfonyl)pyridin-2-yl)amino)-5-fluoro-[2,3'-bipyridin]-6'-yl)acetamide;
N-(4'-((4-methoxy-6-(methylsulfonyl)pyridin-2-yl)amino)-5-(trifluoromethyl)-[2,3'-bipyridin]-6'-yl)acetamide;
(S)-N-(4'-((4-(2-hydroxypropoxy)-6-(methylsulfonyl)pyridin-2-yl)amino)-5-(trifluoromethyl)-[2,3'-bipyridin]-6'-yl)acetamide;
N-(5-fluoro-4'-((4-methoxy-6-(methylsulfonyl)pyridin-2-yl)amino)-[2,3'-bipyridin]-6'-yl)acetamide;
N-(4'-((3,4-dimethoxy-6-(methylsulfonyl)pyridin-2-yl)amino)-5-fluoro-[2,3'-bipyridin]-6'-yl)acetamide;
N-(5-fluoro-4'-((4-(methoxy-d3)-6-(methylsulfonyl)pyridine-2-yl)amino)-[2,3'-bipyridin]-6'-yl)acetamide;
N-(4'-((4-cyclopropyl-6-(methylsulfonyl)pyridin-2-yl)amino)-5-fluoro-[2,3'-bipyridin]-6'-yl)acetamide;
N-(4'-((3-(methylsulfonyl)phenyl)amino)-5-(3-oxomorpholino)-[2,3'-bipyridin]-6'-yl)acetamide;
N-(4'-((6-(methylsulfonyl)pyridin-2-yl)amino)-5-(3-oxomorpholino)-[2,3'-bipyridin]-6'-yl)acetamide;
N-(4'-((4-(2-hydroxyethoxy)-6-(methylsulfonyl)pyridin-2-yl)amino)-5-(3-oxomorpholino)-[2,3'-bipyridin]-6'-yl)acetamide;
N-(4'-((4-(2-methoxyethoxy)-6-(methylsulfonyl)pyridin-2-yl)amino)-5-(3-oxomorpholino)-[2,3'-bipyridin]-6'-yl)acetamide;
N-(5-(2-hydroxypropan-2-yl)-4'-((3-(methylsulfonyl)phenyl)amino)-[2,3'-bipyridin]-6'-yl)acetamide;
N-(5-(2-hydroxypropan-2-yl)-4'-((4-isopropoxy-6-(methylsulfonyl)pyridin-2-yl)amino)-[2,3'-bipyridin]-6'-yl)acetamide .;
N-(5-(2-hydroxypropan-2-yl)-4'-((3-(methylsulfonyl)-5-(trifluoro methyl)phenyl)amino)-[2,3'-bipyridin]-6'-yl)acetamide;
N-(5-(2-hydroxypropan-2-yl)-4'-((3-methoxy-5-(methylsulfonyl)phenyl)amino)-[2,3'-bipyridin]-6'-yl)acetamide;
N-(5-(2-hydroxypropan-2-yl)-4'-((6-(methylsulfonyl)pyridin-2-yl)amino)-[2,3'-bipyridin]-6'-yl)acetamide;
N-(5-(2-hydroxypropan-2-yl)-4'-((4-(2-methoxyethoxy)-6-(methylsulfonyl)pyridin-2-yl)amino)-[2,3'-bipyridin]-6'-yl)acetamide;
N-(5-(2-hydroxypropan-2-yl)-4'-((4-(2-methoxypropoxy)-6-(methylsulfonyl)pyridin-2-yl)amino)-[2,3'-bipyridin]-6'-yl)acetamide;
N-(4'-((4-(2-hydroxyethoxy)-6-(methylsulfonyl)pyridin-2-yl)amino)-5-(2-hydroxypropan-2-yl)-[2,3'-bipyridin]-6'-yl)acetamide;
N-(5-(2-hydroxypropan-2-yl)-4'-((4-methyl-6-(methylsulfonyl)pyridin-2-yl)amino)-[2,3'-bipyridin]-6'-yl)acetamide;
N-(4'-((4-(trans)-3-hydroxycyclobutoxy)-6-(methylsulfonyl)pyridin-2-yl)amino)-5-(2-hydroxypropan-2-yl)-[2,3'-bipyridin]-6'-yl)acetamide;
N-(5-(2-hydroxypropan-2-yl)-4'-((4-methoxy-6-(methylsulfonyl)pyridin-2-yl)amino)-[2,3'-bipyridin]-6'-yl)acetamide;
N-(5-(2-hydroxypropan-2-yl)-4'-((6-(methylsulfonyl)-4-(oxetan-3-ylmethoxy)pyridin-2-yl)amino)-[2,3'-bipyridin]-6'-yl)acetamide;
N-(5-(2-hydroxypropan-2-yl)-4'-((4-(methoxymethyl)-6-(methylsulfonyl)pyridin-2-yl)amino)-[2,3'-bipyridin]-6'-yl)acetamide;
N-(4'-((4-cyano-6-(methylsulfonyl)pyridin-2-yl)amino)-5-(2-hydroxypropan-2-yl)-[2,3'-bipyridin]-6'-yl)acetamide;
N-(5-(2-hydroxypropan-2-yl)-4'-((3-(2-methoxyethoxy)-5-(methylsulfonyl)phenyl)amino)-[2,3'-bipyridin]-6'-yl)acetamide;
N-(5-(2-hydroxypropan-2-yl)-4'-((3-(methoxymethyl)-5-(methylsulfonyl)phenyl)amino)-[2,3'-bipyridin]-6'-yl)acetamide;
N-(5-(2-hydroxypropan-2-yl)-4'-((3-(3-hydroxypyrrolidin-1-yl)-5-(methylsulfonyl)phenyl)amino)-[2,3'-bipyridin]-6'-yl)acetamide;
N-(5-(2-hydroxypropan-2-yl)-4'-((3-(methylsulfonyl)-5-(trifluoromethoxy)phenyl)amino)-[2,3'-bipyridin]-6'-yl)acetamide;
N-(4'-((3-fluoro-5-(methylsulfonyl)phenyl)amino)-5-(2-hydroxypropan-2-yl)-[2,3'-bipyridin]-6'-yl)acetamide;
N-(4'-((3-cyano-5-(methylsulfonyl)phenyl)amino)-5-(2-hydroxypropan-2-yl)-[2,3'-bipyridin]-6'-yl)acetamide;
N-(5-(2-hydroxypropan-2-yl)-4'-((3-methyl-5-(methylsulfonyl)phenyl)amino)-[2,3'-bipyridin]-6'-yl)acetamide;
N-(4'-((3-(cyclopropylmethoxy)-5-(methylsulfonyl)phenyl)amino)-5-(2-hydroxypropan-2-yl)-[2,3'-bipyridin]-6'-yl)acetamide;
N-(4'-((3-cyclopropoxy-5-(methylsulfonyl)phenyl)amino)-5-(2-hydroxypropan-2-yl)-[2,3'-bipyridin]-6'-yl)acetamide;
N-(5-(2-hydroxypropan-2-yl)-4'-((3-isopropoxy-5-(methylsulfonyl)phenyl)amino)-[2,3'-bipyridin]-6'-yl)acetamide;
N-(4'-((3-(difluoromethyl)-5-(methylsulfonyl)phenyl)amino)-5-(2-hydroxypropan-2-yl)-[2,3'-bipyridin]-6'-yl)acetamide;
N-(5-(2-hydroxypropan-2-yl)-4'-((4-(2-hydroxypropoxy)-6-(methylsulfonyl)pyridin-2-yl)amino)-[2,3'-bipyridin]-6'-yl)acetamide;
N-(5-(2-hydroxypropan-2-yl)-4'-((6-(methylsulfonyl)-4-(oxetan-3-yloxy)pyridin-2-yl)amino)-[2,3'-bipyridin]-6'-yl)acetamide;
N-(4'-((4-(cis-3-hydroxycyclobutoxy)-6-(methylsulfonyl)pyridin-2-yl)amino)-5-(2-hydroxypropan-2-yl)-[2,3'-bipyridin]-6'-yl)acetamide;
N-(5-(2-hydroxypropan-2-yl)-4'-((4-(3-methoxycyclobutoxy)-6-(methylsulfonyl)pyridin-2-yl)amino)-[2,3'-bipyridin]-6'-yl)acetamide;
N-(4'-((4-(cyclopropylmethoxy)-6-(methylsulfonyl)pyridin-2-yl)amino)-5-(2-hydroxypropan-2-yl)-[2,3'-bipyridin]-6'-yl)acetamide;
N-(4'-((4-cyclobutoxy-6-(methylsulfonyl)pyridin-2-yl)amino)-5-(2-hydroxypropan-2-yl)-[2,3'-bipyridin]-6'-yl)acetamide;
N-(5-(2-hydroxypropan-2-yl)-4'-((3-methyl-6-(methylsulfonyl)pyridin-2-yl)amino)-[2,3'-bipyridin]-6'-yl)acetamide;
N-(4'-((4-ethoxy-6-(methylsulfonyl)pyridin-2-yl)amino)-5-(2-hydroxypropan-2-yl)-[2,3'-bipyridin]-6'-yl)acetamide;
N-(4'-((5-fluoro-6-(methylsulfonyl)pyridin-2-yl)amino)-5-(2-hydroxypropan-2-yl)-[2,3'-bipyridin]-6'-yl)acetamide;
N-(5-(2-hydroxypropan-2-yl)-4'-((2-methyl-5-(methylsulfonyl)phenyl)amino)-[2,3'-bipyridin]-6'-yl)acetamide;
N-(4'-((6-(ethylsulfonyl)-4-isopropoxypyridin-2-yl)amino)-5-(2-hydroxypropan-2-yl)-[2,3'-bipyridin]-6'-yl)acetamide;
N-(5-(2-hydroxypropan-2-yl)-4'-((7-(methylsulfonyl)-2,3-dihydro-[1,4]dioxino[2,3-c]pyridin-5-yl)amino)-[2,3'-bipyridin]-6'-yl)acetamide;
(R)-N-(5-(2-hydroxypropan-2-yl)-4'-((4-(2-hydroxypropoxy)-6-(methylsulfonyl)pyridin-2-yl)amino)-[2,3'-bipyridin]-6'-yl)acetamide;
N-(5-(2-hydroxypropan-2-yl)-4'-((6-(methylsulfonyl)-4-(tetrahydro-2H-pyran-4-yl)pyridin-2-yl)amino)-[2,3'-bipyridin]-6'-yl)acetamide;
N-(5-(2-hydroxypropan-2-yl)-4'-((4-isobutoxy-6-(methylsulfonyl)pyridin-2-yl)amino)-[2,3'-bipyridin]-6'-yl)acetamide;
(S)-N-(4'-((4-(sec-butoxy)-6-(methylsulfonyl)pyridin-2-yl)amino)-5-(2-hydroxypropan-2-yl)-[2,3'-bipyridin]-6'-yl)acetamide;
(R)-N-(4'-((4-(sec-butoxy)-6-(methylsulfonyl)pyridin-2-yl)amino)-5-(2-hydroxypropan-2-yl)-[2,3'-bipyridin]-6'-yl)acetamide;
N-(5-(2-hydroxypropan-2-yl)-4'-((3-isopropoxy-5-(methylsulfonyl)phenyl)amino)-[2,3'-bipyridin]-6'-yl)acetamide;
N-(5-(1-methyl-1H-pyrazol-3-yl)-4-((4-methyl-6-(methylsulfonyl)pyridin-2-yl)amino)pyridin-2-yl)acetamide;
N-(4-((4-isopropoxy-6-(methylsulfonyl)pyridin-2-yl)amino)-5-(1-methyl-1H-pyrazol-3-yl)pyridin-2-yl)acetamide;
N-(4-((4-(cyclopropylmethoxy)-6-(methylsulfonyl)pyridin-2-yl)amino)-5-(1-methyl-1H-pyrazol-3-yl)pyridin-2-yl)acetamide;
N-(5-(1-methyl-1H-pyrazol-3-yl)-4-((6-sulfamoylpyridin-2-yl)amino)pyridin-2-yl)acetamide;
N-(4-((6-(ethylsulfonyl)pyridin-2-yl)amino)-5-(1-methyl-1H-pyrazol-3-yl)pyridin-2-yl)acetamide;
N-(5-(1-methyl-1H-pyrazol-3-yl)-4-((3-(methylsulfonyl)phenyl)amino)pyridin-2-yl)acetamide;
N-(4-((3-cyano-5-(methylsulfonyl)phenyl)amino)-5-(1-methyl-1H-pyrazol-3-yl)pyridin-2-yl)acetamide;
N-(5-(1-methyl-1H-pyrazol-3-yl)-4-((3-methyl-5-(methylsulfonyl)phenyl)amino)pyridin-2-yl)acetamide;
N-(4-((3-methoxy-5-(methylsulfonyl)phenyl)amino)-5-(1-methyl-1H-pyrazol-3-yl)pyridin-2-yl)acetamide;
N-(5-(1-methyl-1H-pyrazol-3-yl)-4-((3-(methylsulfonyl)-5-(trifluoromethoxy)phenyl)amino)pyridin-2-yl)acetamide;
N-(5-(1-methyl-1H-pyrazol-3-yl)-4-((6-(methylsulfonyl)pyridin-2-yl)amino)pyridin-2-yl)acetamide;
N-(4-((4-(2-methoxyethoxy)-6-(methylsulfonyl)pyridin-2-yl)amino)-5-(1-methyl-1H-pyrazol-3-yl)pyridin-2-yl)acetamide;
N-(4-((4-(2-methoxypropoxy)-6-(methylsulfonyl)pyridin-2-yl)amino)-5-(1-methyl-1H-pyrazol-3-yl)pyridin-2-yl)acetamide;
(R)-N-(4-((4-(2-methoxypropoxy)-6-(methylsulfonyl)pyridin-2-yl)amino)-5-(1-methyl-1H-pyrazol-3-yl)pyridin-2-yl)acetamide;
N-(4-((4-(2-hydroxyethoxy)-6-(methylsulfonyl)pyridin-2-yl)amino)-5-(1-methyl-1H-pyrazol-3-yl)pyridin-2-yl)acetamide;
N-(4-((4-((trans)-3-hydroxycyclobutoxy)-6-(methylsulfonyl)pyridin-2-yl)amino)-5-(1-methyl-1H-pyrazol-3-yl)pyridin-2-yl)acetamide;
N-(4-((4-((cis)-3-hydroxycyclobutoxy)-6-(methylsulfonyl)pyridin-2-yl)amino)-5-(1-methyl-1H-pyrazol-3-yl)pyridin-2-yl)acetamide;
N-(4-((4-(3-methoxycyclobutoxy)-6-(methylsulfonyl)pyridin-2-yl)amino)-5-(1-methyl-1H-pyrazol-3-yl)pyridin-2-yl)acetamide;
N-(4-((4-(methoxymethyl)-6-(methylsulfonyl)pyridin-2-yl)amino)-5-(1-methyl-1H-pyrazol-3-yl)pyridin-2-yl)acetamide;
N-(4-((4-(cyanomethoxy)-6-(methylsulfonyl)pyridin-2-yl)amino)-5-(1-methyl-1H-pyrazol-3-yl)pyridin-2-yl)acetamide;
N-(4-((4-methoxy-6-(methylsulfonyl)pyridin-2-yl)amino)-5-(1-methyl-1H-pyrazol-3-yl)pyridin-2-yl)acetamide;
N-(5-(1-methyl-1H-pyrazol-3-yl)-4-((6-(methylsulfonyl)-4-(oxetan-3-yloxy)pyridin-2-yl)amino)pyridin-2-yl)acetamide;
N-(4-((3-methoxy-6-(methylsulfonyl)pyridin-2-yl)amino)-5-(1-methyl-1H-pyrazol-3-yl)pyridin-2-yl)acetamide;
N-(4-((4-(2-hydroxypropoxy)-6-(methylsulfonyl)pyridin-2-yl)amino)-5-(1-methyl-1H-pyrazol-3-yl)pyridin-2-yl)acetamide;
N-(5-(1-methyl-1H-pyrazol-3-yl)-4-((6-(methylsulfonyl)-4-(oxetan-3-ylmethoxy)pyridin-2-yl)amino)pyridin-2-yl)acetamide;
N-(4-((4-cyano-6-(methylsulfonyl)pyridin-2-yl)amino)-5-(1-methyl-1H-pyrazol-3-yl)pyridin-2-yl)acetamide;
N-(5-(1-methyl-1H-pyrazol-3-yl)-4-((3-methyl-6-(methylsulfonyl)pyridin-2-yl)amino)pyridin-2-yl)acetamide;
N-(4-((6-(cyclopropylsulfonyl)pyridin-2-yl)amino)-5-(1-methyl-1H-pyrazol-3-yl)pyridin-2-yl)acetamide;
N-(4-((6-(isopropylsulfonyl)pyridin-2-yl)amino)-5-(1-methyl-1H-pyrazol-3-yl)pyridin-2-yl)acetamide;
N-(4-((3-fluoro-5-(methylsulfonyl)phenyl)amino)-5-(1-methyl-1H-pyrazol-3-yl)pyridin-2-yl)acetamide;
N-(4-((3-chloro-5-(methylsulfonyl)phenyl)amino)-5-(1-methyl-1H-pyrazol-3-yl)pyridin-2-yl)acetamide;
N-(4-((3-(difluoromethyl)-5-(methylsulfonyl)phenyl)amino)-5-(1-methyl-1H-pyrazol-3-yl)pyridin-2-yl)acetamide;
N-(5-(1-methyl-1H-pyrazol-3-yl)-4-((3-(methylsulfonyl)-5-(trifluoromethyl)phenyl)amino)pyridin-2-yl)acetamide;
N-(4-((3-(methoxymethyl)-5-(methylsulfonyl)phenyl)amino)-5-(1-methyl-1H-pyrazol-3-yl)pyridin-2-yl)acetamide;
N-(4-((3-(2-methoxyethoxy)-5-(methylsulfonyl)phenyl)amino)-5-(1-methyl-1H-pyrazol-3-yl)pyridin-2-yl)acetamide;
N-(4-((3-cyclopropoxy-5-(methylsulfonyl)phenyl)amino)-5-(1-methyl-1H-pyrazol-3-yl)pyridin-2-yl)acetamide;
N-(4-((3-((trans)-3-hydroxycyclobutoxy)-5-(methylsulfonyl)phenyl)amino)-5-(1-methyl-1H-pyrazol-3-yl)pyridin-2-yl)acetamide;
N-(5-(1-methyl-1H-pyrazol-3-yl)-4-((6-(methylsulfonyl)-4-phenylpyridin-2-yl)amino)pyridin-2-yl)acetamide;
N-(5-(1-methyl-1H-pyrazol-3-yl)-4-((6-(methylsulfonyl)-4-phenoxypyridin-2-yl)amino)pyridin-2-yl)acetamide;
N-(4-((4-chloro-6-(methylsulfonyl)pyridin-2-yl)amino)-5-(1-methyl-1H-pyrazol-3-yl)pyridin-2-yl)acetamide;
N-(4-((4-(1-methoxyethyl)-6-(methylsulfonyl)pyridin-2-yl)amino)-5-(1-methyl-1H-pyrazol-3-yl)pyridin-2-yl)acetamide;
N-(4-((4-(difluoromethyl)-6-(methylsulfonyl)pyridin-2-yl)amino)-5-(1-methyl-1H-pyrazol-3-yl)pyridin-2-yl)acetamide;
N-(4-((4-ethoxy-6-(methylsulfonyl)pyridin-2-yl)amino)-5-(1-methyl-1H-pyrazol-3-yl)pyridin-2-yl)acetamide;
N-(4-((4-cyclobutoxy-6-(methylsulfonyl)pyridin-2-yl)amino)-5-(1-methyl-1H-pyrazol-3-yl)pyridin-2-yl)acetamide;
N-(4-((4-(cyclopentyloxy)-6-(methylsulfonyl)pyridin-2-yl)amino)-5-(1-methyl-1H-pyrazol-3-yl)pyridin-2-yl)acetamide;
N-(5-(1-methyl-1H-pyrazol-3-yl)-4-((6-(methylsulfonyl)-4-((tetrahydrofuran-3-yl)oxy)pyridin-2-yl)amino)pyridin-2-yl)acetamide;
(R)-N-(4-((4-(2-hydroxypropoxy)-6-(methylsulfonyl)pyridin-2-yl)amino)-5-(1-methyl-1H-pyrazol-3-yl)pyridin-2-yl)acetamide;
N-(4-((4-(2,3-dihydroxypropoxy)-6-(methylsulfonyl)pyridin-2-yl)amino)-5-(1-methyl-1H-pyrazol-3-yl)pyridin-2-yl)acetamide;
N-(4-((3-isopropoxy-6-(methylsulfonyl)pyridin-2-yl)amino)-5-(1-methyl-1H-pyrazol-3-yl)pyridin-2-yl)acetamide;
N-(4-((5-methoxy-6-(methylsulfonyl)pyridin-2-yl)amino)-5-(1-methyl-1H-pyrazol-3-yl)pyridin-2-yl)acetamide;
N-(5-(1-methyl-1H-pyrazol-3-yl)-4-((5-methyl-6-(methylsulfonyl)pyridin-2-yl)amino)pyridin-2-yl)acetamide;
N-(5-(1-methyl-1H-pyrazol-3-yl)-4-((6-(methylsulfonyl)-4-(trifluoromethyl)pyridin-2-yl)amino)pyridin-2-yl)acetamide;
N-(4-((6-(ethylsulfonyl)-4-isopropoxypyridin-2-yl)amino)-5-(1-methyl-1H-pyrazol-3-yl)pyridin-2-yl)acetamide;
N-(4-((5-fluoro-6-(methylsulfonyl)pyridin-2-yl)amino)-5-(1-methyl-1H-pyrazol-3-yl)pyridin-2-yl)acetamide;
(S)-N-(4-((3-(2-hydroxypropoxy)-5-(methylsulfonyl)phenyl)amino)-5-(1-methyl-1H-pyrazol-3-yl)pyridin-2-yl)acetamide;
N-(4-((3-(2-hydroxyethoxy)-5-(methylsulfonyl)phenyl)amino)-5-(1-methyl-1H-pyrazol-3-yl)pyridin-2-yl)acetamide;
(R)-N-(4-((3-(2-hydroxypropoxy)-5-(methylsulfonyl)phenyl)amino)-5-(1-methyl-1H-pyrazol-3-yl)pyridin-2-yl)acetamide;
N-(4-((4-(difluoromethoxy)-6-(methylsulfonyl)pyridin-2-yl)amino)-5-(1-methyl-1H-pyrazol-3-yl)pyridin-2-yl)acetamide;
N-(4-((4-isobutoxy-6-(methylsulfonyl)pyridin-2-yl)amino)-5-(1-methyl-1H-pyrazol-3-yl)pyridin-2-yl)acetamide;
(S)-N-(4-((4-(sec-butoxy)-6-(methylsulfonyl)pyridin-2-yl)amino)-5-(1-methyl-1H-pyrazol-3-yl)pyridin-2-yl)acetamide;
N-(5-(1-methyl-1H-pyrazol-3-yl)-4-((6-(methylsulfonyl)-4-(tetrahydro-2H-pyran-4-yl)pyridin-2-yl)amino)pyridin-2-yl)acetamide;
N-(4-((3-(difluoromethoxy)-5-(methylsulfonyl)phenyl)amino)-5-(1-methyl-1H-pyrazol-3-yl)pyridin-2-yl)acetamide;
N-(4-((3-(difluoromethoxy)-6-(methylsulfonyl)pyridin-2-yl)amino)-5-(1-methyl-1H-pyrazol-3-yl)pyridin-2-yl)acetamide;
N-(5-(1-methyl-1H-pyrazol-3-yl)-4-((7-(methylsulfonyl)-2,3-dihydro-[1,4]dioxino[2,3-c]pyridin-5-yl)amino)pyridin-2-yl)acetamide;
(R)-N-(4-((4-(sec-butoxy)-6-(methylsulfonyl)pyridin-2-yl)amino)-5-(1-methyl-1H-pyrazol-3-yl)pyridin-2-yl)acetamide;
N-(5-(1-cyclopropyl-1H-pyrazol-3-yl)-4-((6-(methylsulfonyl)pyridin-2-yl)amino)pyridin-2-yl)acetamide;
N-(5-(1-cyclopropyl-1H-pyrazol-3-yl)-4-((4-isopropoxy-6-(methylsulfonyl)pyridin-2-yl)amino)pyridin-2-yl)acetamide;
N-(5-(1-cyclopropyl-1H-pyrazol-3-yl)-4-((4-methyl-6-(methylsulfonyl)pyridin-2-yl)amino)pyridin-2-yl)acetamide;
N-(5-(1-cyclopropyl-1H-pyrazol-3-yl)-4-((4-(2-methoxyethoxy)-6-(methylsulfonyl)pyridin-2-yl)amino)pyridin-2-yl)acetamide;
N-(5-(1-ethyl-1H-pyrazol-3-yl)-4-((6-(methylsulfonyl)pyridin-2-yl)amino)pyridin-2-yl)acetamide;
N-(5-(1-ethyl-1H-pyrazol-3-yl)-4-((4-isopropoxy-6-(methylsulfonyl)pyridin-2-yl)amino)pyridin-2-yl)acetamide;
N-(5-(1-ethyl-1H-pyrazol-3-yl)-4-((4-(methoxymethyl)-6-(methylsulfonyl)pyridin-2-yl)amino)pyridin-2-yl)acetamide;
N-(5-(1-ethyl-1H-pyrazol-3-yl)-4-((3-(methylsulfonyl)phenyl)amino)pyridin-2-yl)acetamide;
N-(5-(1-ethyl-1H-pyrazol-3-yl)-4-((3-methoxy-5-(methylsulfonyl)phenyl)amino)pyridin-2-yl)acetamide;
N-(4-((3-cyano-5-(methylsulfonyl)phenyl)amino)-5-(1-ethyl-1H-pyrazol-3-yl)pyridin-2-yl)acetamide;
N-(5-(1-ethyl-1H-pyrazol-3-yl)-4-((3-methyl-5-(methylsulfonyl)phenyl)amino)pyridin-2-yl)acetamide;
N-(5-(1-ethyl-1H-pyrazol-3-yl)-4-((3-(methoxymethyl)-5-(methylsulfonyl)phenyl)amino)pyridin-2-yl)acetamide;
N-(5-(1-ethyl-1H-pyrazol-3-yl)-4-((3-(methylsulfonyl)-5-(trifluoromethyl)phenyl)amino)pyridin-2-yl)acetamide;
N-(5-(1-ethyl-1H-pyrazol-3-yl)-4-((3-(2-methoxyethoxy)-5-(methylsulfonyl)phenyl)amino)pyridin-2-yl)acetamide;
N-(5-(1-ethyl-1H-pyrazol-3-yl)-4-((3-(2-hydroxyethoxy)-5-(methylsulfonyl)phenyl)amino)pyridin-2-yl)acetamide;
N-(4-((3-(cyclopropylmethoxy)-5-(methylsulfonyl)phenyl)amino)-5-(1-ethyl-1H-pyrazol-3-yl)pyridin-2-yl)acetamide;
N-(4-((4-(cyclopentyloxy)-6-(methylsulfonyl)pyridin-2-yl)amino)-5-(1-ethyl-1H-pyrazol-3-yl)pyridin-2-yl)acetamide;
N-(4-((4-ethoxy-6-(methylsulfonyl)pyridin-2-yl)amino)-5-(1-ethyl-1H-pyrazol-3-yl)pyridin-2-yl)acetamide;
N-(5-(1-ethyl-1H-pyrazol-3-yl)-4-((4-(2-hydroxyethoxy)-6-(methylsulfonyl)pyridin-2-yl)amino)pyridin-2-yl)acetamide;
N-(5-(1-ethyl-1H-pyrazol-3-yl)-4-((4-(2-methoxyethoxy)-6-(methylsulfonyl)pyridin-2-yl)amino)pyridin-2-yl)acetamide;
N-(5-(1-ethyl-1H-pyrazol-3-yl)-4-((3-isopropoxy-6-(methylsulfonyl)pyridin-2-yl)amino)pyridin-2-yl)acetamide;
N-(5-(1-isopropyl-1H-pyrazol-3-yl)-4-((6-(methylsulfonyl)pyridin-2-yl)amino)pyridin-2-yl)acetamide;
N-(4-((4-isopropoxy-6-(methylsulfonyl)pyridin-2-yl)amino)-5-(1-isopropyl-1H-pyrazol-3-yl)pyridin-2-yl)acetamide;
N-(5-(1-isopropyl-1H-pyrazol-3-yl)-4-((4-(2-methoxyethoxy)-6-(methylsulfonyl)pyridin-2-yl)amino)pyridin-2-yl)acetamide;
N-(5-(1-isopropyl-1H-pyrazol-3-yl)-4-((3-(methylsulfonyl)-5-(trifluoromethyl)phenyl)amino)pyridin-2-yl)acetamide;
N-(4-((3-isopropoxy-5-(methylsulfonyl)phenyl)amino)-5-(1-isopropyl-1H-pyrazol-3-yl)pyridin-2-yl)acetamide;
N-(5-(1-isopropyl-1H-pyrazol-3-yl)-4-((3-methoxy-5-(methylsulfonyl)phenyl)amino)pyridin-2-yl)acetamide;
N-(4-((4-(2-hydroxyethoxy)-6-(methylsulfonyl)pyridin-2-yl)amino)-5-(1-isopropyl-1H-pyrazol-3-yl)pyridin-2-yl)acetamide;
N-(4-((3-(2-hydroxyethoxy)-5-(methylsulfonyl)phenyl)amino)-5-(1-isopropyl-1H-pyrazol-3-yl)pyridin-2-yl)acetamide;
N-(4-((3-(methylsulfonyl)phenyl)amino)-5-(1-(tetrahydrofuran-3-yl)-1H-pyrazol-3-yl)pyridin-2-yl)acetamide;
N-(4-((4-methyl-6-(methylsulfonyl)pyridin-2-yl)amino)-5-(1-(tetrahydrofuran-3-yl)-1H-pyrazol-3-yl)pyridin-2-yl)acetamide;
(R)-N-(4-((3-(methylsulfonyl)-5-(trifluoromethyl)phenyl)amino)-5-(1-(tetrahydrofuran-3-yl)-1H-pyrazol-3-yl)pyridin-2-yl)acetamide;
(S)-N-(4-((4-isopropoxy-6-(methylsulfonyl)pyridin-2-yl)amino)-5-(1-(tetrahydrofuran-3-yl)-1H-pyrazol-3-yl)pyridin-2-yl)acetamide;
(R)-N-(4-((4-isopropoxy-6-(methylsulfonyl)pyridin-2-yl)amino)-5-(1-(tetrahydrofuran-3-yl)-1H-pyrazol-3-yl)pyridin-2-yl)acetamide;
(R)-N-(4-((4-methoxy-6-(methylsulfonyl)pyridin-2-yl)amino)-5-(1-(tetrahydrofuran-3-yl)-1H-pyrazol-3-yl)pyridin-2-yl)acetamide;
N-(5-(1,5-dimethyl-1H-pyrazol-3-yl)-4-((6-(methylsulfonyl)pyridin-2-yl)amino)pyridin-2-yl)acetamide;
N-(5-(1,5-dimethyl-1H-pyrazol-3-yl)-4-((4-isopropoxy-6-(methylsulfonyl)pyridin-2-yl)amino)pyridin-2-yl)acetamide;
N-(5-(1,5-dimethyl-1H-pyrazol-3-yl)-4-((3-(2-hydroxyethoxy)-5-(methylsulfonyl)phenyl)amino)pyridin-2-yl)acetamide;
N-(5-(1,5-dimethyl-1H-pyrazol-3-yl)-4-((4-methoxy-6-(methylsulfonyl)pyridin-2-yl)amino)pyridin-2-yl)acetamide;
N-(5-(1,5-dimethyl-1H-pyrazol-3-yl)-4-((4-(2-methoxyethoxy)-6-(methylsulfonyl)pyridin-2-yl)amino)pyridin-2-yl)acetamide;
(R)-N-(5-(1,5-dimethyl-1H-pyrazol-3-yl)-4-((4-(2-methoxypropoxy)-6-(methylsulfonyl)pyridin-2-yl)amino)pyridin-2-yl)acetamide;
N-(5-(1,5-dimethyl-1H-pyrazol-3-yl)-4-((4-ethoxy-6-(methylsulfonyl)pyridin-2-yl)amino)pyridin-2-yl)acetamide;
N-(5-(1-methyl-5-morpholino-1H-pyrazol-3-yl)-4-((6-(methylsulfonyl)pyridin-2-yl)amino)pyridin-2-yl)acetamide;
N-(4-((4-(2-methoxyethoxy)-6-(methylsulfonyl)pyridin-2-yl)amino)-5-(1-methyl-5-morpholino-1H-pyrazol-3-yl)pyridin-2-yl)acetamide;
N-(4-((4-(2-hydroxypropoxy)-6-(methylsulfonyl)pyridin-2-yl)amino)-5-(1-methyl-5-morpholino-1H-pyrazol-3-yl)pyridin-2-yl)acetamide;
N-(5-(2,3-dihydro-[1,4]dioxino[2,3-b]pyridin-6-yl)-4-((4-isopropoxy-6-(methylsulfonyl)pyridine-2-yl)amino)pyridin-2-yl)acetamide;
N-(5-([1,3]dioxolo[4,5-b]pyridin-5-yl)-4-((4-isopropoxy-6-(methylsulfonyl)pyridin-2-yl)amino)pyridin-2-yl)acetamide;
N-(5-(2,3-dihydro-[1,4]dioxino[2,3-b]pyridin-6-yl)-4-((4-methyl-6-(methylsulfonyl)pyridin-2-yl)amino)pyridin-2-yl)acetamide;
N-(5-(3,4-dihydro-2H-pyrido[4,3-b][1,4]oxazin-7-yl)-4-((4-isopropoxy-6-(methylsulfonyl)pyridin-2-yl)amino)pyridin-2-yl)acetamide;
N-(5-(imidazo[1,2-b]pyridazin-6-yl)-4-((4-isopropoxy-6-(methylsulfonyl)pyridin-2-yl)amino)pyridin-2-yl)acetamide;
N-(5-(6,7-dihydro-4H-pyrazolo[5,1-c][1,4]oxazin-2-yl)-4-((4-isopropoxy-6-(methylsulfonyl)pyridin-2-yl)amino)pyridin-2-yl)acetamide;
N-(5-(4-acetyl-3,4-dihydro-2H-pyrido[3,2-b][1,4]oxazin-6-yl)-4-((4-isopropoxy-6-(methylsulfonyl)pyridin-2-yl)amino)pyridin-2-yl)acetamide;
N-(5-(2,3-dihydro-[1,4]dioxino[2,3-c]pyridin-7-yl)-4-((4-methoxy-6-(methylsulfonyl)pyridin-2-yl)amino)pyridin-2-yl)acetamide;
N-(5-(benzo[b]thiophen-2-yl)-4-((4-methoxy-6-(methylsulfonyl)pyridin-2-yl)amino)pyridin-2-yl)acetamide;
N-(5-(6,7-dihydro-4H-thieno[3,2-c]pyran-2-yl)-4-((4-methyl-6-(methylsulfonyl)pyridin-2-yl)amino)pyridin-2-yl)acetamide;
N-(5-(2,3-dihydro-[1,4]dioxino[2,3-b]pyridin-6-yl)-4-((4-methoxy-6-(methylsulfonyl)pyridin-2-yl)amino)pyridin-2-yl)acetamide;
N-(5-(2,3-dihydro-[1,4]dioxino[2,3-c]pyridin-7-yl)-4-((4-ethoxy-6-(methylsulfonyl)pyridin-2-yl)amino)pyridin-2-yl)acetamide;
N-(5-(2,3-dihydro-[1,4]dioxino[2,3-c]pyridin-7-yl)-4-((4-(2-methoxyethoxy)-6-(methylsulfonyl)pyridin-2-yl)amino)pyridin-2-yl)acetamide;
N-(5-([1,3]dioxolo[4,5-b]pyridin-5-yl)-4-((4-(2-methoxyethoxy)-6-(methylsulfonyl)pyridin-2-yl)amino)pyridin-2-yl)acetamide;
N-(5-([1,3]dioxolo[4,5-b]pyridin-5-yl)-4-((4-ethoxy-6-(methylsulfonyl)pyridin-2-yl)amino)pyridin-2-yl)acetamide;
N-(5-([1,3]dioxolo[4,5-b]pyridin-5-yl)-4-((4-methoxy-6-(methylsulfonyl)pyridin-2-yl)amino)pyridin-2-yl)acetamide;
N-(4-((4-(cyclopropylmethoxy)-6-(methylsulfonyl)pyridin-2-yl)amino)-5-(5-methyl-4,5,6,7-tetrahydrothiazolo[5,4-c]pyridin-2-yl)pyridin-2-yl)acetamide;
N-(5-(2,3-dihydro-[1,4]dioxino[2,3-c]pyridin-7-yl)-4-((4-methyl-6-(methylsulfonyl)pyridin-2-yl)amino)pyridin-2-yl)acetamide;
N-(5-(2,3-dihydro-[1,4]dioxino[2,3-c]pyridin-7-yl)-4-((4-(2-methoxyethoxy)-6-(methylsulfonyl)pyridin-2-yl)amino)pyridin-2-yl)acetamide;
N-(5-(6,7-dihydro-4H-pyrazolo[5,1-c][1,4]oxazin-2-yl)-4-((4-methyl-6-(methylsulfonyl)pyridin-2-yl)amino)pyridin-2-yl)acetamide;
N-(4-((4-((1,4-dioxan-2-yl)methoxy)-6-(methylsulfonyl)pyridin-2-yl)amino)-5-(3,3-dimethyl-2,3-dihydro-[1,4]dioxino[2,3-b]pyridin-6-yl)pyridin-2-yl)acetamide;
N-(5-(3,3-dimethyl-2,3-dihydro-[1,4]dioxino[2,3-b]pyridine-6-yl)-4-((6-(methylsulfonyl)pyrazin-2-yl)amino)pyridine-2-yl)acetamide;
N-(5-(3,3-dimethyl-2,3-dihydro-[1,4]dioxino[2,3-b]yridine-6-yl)-4-((4-(2-hydroxypropan-2-yl)-6-(methylsulfonyl)pyridine-2-yl)amino)pyridine-2-yl)acetamide;
N-(5-(3,3-dimethyl-2,3-dihydro-[1,4]dioxino[2,3-b]pyridine-6-yl)-4-((5-fluoro-4-methyl-6-(methylsulfonyl)pyridine-2-yl)amino)pyridine-2-yl)acetamide;
N-(5-(3,3-dimethyl-2,3-dihydro-[1,4]dioxino[2,3-b]pyridin-6-yl)-4-((6-(methylsulfonyl)-4-morpholinopyridin-2-yl)amino)pyridin-2-yl)acetamide;
N-(5-(2,2-dimethyl-2,3-dihydro-[1,4]dioxino[2,3-b]pyridin-6-yl)-4-((4-methyl-6-(methylsulfonyl)pyridin-2-yl)amino)pyridin-2-yl)acetamide;
N-(5-(2,2-dimethyl-2,3-dihydro-[1,4]dioxino[2,3-b]pyridin-6-yl)-4-((6-(methylsulfonyl)-4-morpholinopyridin-2-yl)amino)pyridin-2-yl)acetamide;
N-(5-(2,2-dimethyl-2,3-dihydro-[1,4]dioxino[2,3-b]pyridin-6-yl)-4-((6-(methylsulfonyl)-4-(tetrahydro-2H-pyran-4-yl)pyridin-2-yl)amino)pyridin-2-yl)acetamide;
N-(5-(2,2-dimethyl-2,3-dihydro-[1,4]dioxino[2,3-b]pyridin-6-yl)-4-((4-(methoxy-d3)-6-(methylsulfonyl)pyridin-2-yl)amino)pyridin-2-yl)acetamide;
N-(5-(2,2-dimethyl-2,3-dihydro-[1,4]dioxino[2,3-b]pyridin-6-yl)-4-((4-methoxy-6-(methylsulfonyl)pyridin-2-yl)amino)pyridin-2-yl)acetamide;
N-(4-((3,4-dimethoxy-6-(methylsulfonyl)pyridin-2-yl)amino)-5-(2,2-dimethyl-2,3-dihydro-[1,4]dioxino[2,3-b]pyridin-6-yl)pyridin-2-yl)acetamide;
(S)-N-(5-(2,2-dimethyl-2,3-dihydro-[1,4]dioxino[2,3-b]pyridin-6-yl)-4-((4-(3-methylmorpholino)-6-(methylsulfonyl)pyridin-2-yl)amino)-pyridin-2-yl)acetamide.;
(R)-N-(5-(2,2-dimethyl-2,3-dihydro-[1,4]dioxino[2,3-b]pyridin-6-yl)-4-((4-(3-methylmorpholino)-6-(methylsulfonyl)pyridin-2-yl)amino)-pyridin-2-yl)acetamide.;
N-(5-(3,3-dimethyl-2,3-dihydro-[1,4]dioxino[2,3-b]pyridin-6-yl)-4-((4-(methoxy-d3)-6-(methylsulfonyl)pyridin-2-yl)amino)pyridin-2-yl)acetamide;
N-(5-(3,3-dimethyl-2,3-dihydro-[1,4]dioxino[2,3-b]pyridin-6-yl)-4-((4-methoxy-6-(methylsulfonyl)pyridin-2-yl)amino)pyridin-2-yl)acetamide;
N-(5-(3,3-dimethyl-2,3-dihydro-[1,4]dioxino[2,3-b]pyridin-6-yl)-4-((6-(methylsulfonyl)-4-(tetrahydro-2H-pyran-4-yl)pyridin-2-yl)amino)pyridin-2-yl)acetamide;
(R)-N-(5-(3,3-dimethyl-2,3-dihydro-[1,4]dioxino[2,3-b]pyridin-6-yl)-4-((4-(3-methylmorpholino)-6-(methylsulfonyl)pyridin-2-yl)amino)pyridin-2-yl)acetamide;
(S)-N-(5-(3,3-dimethyl-2,3-dihydro-[1,4]dioxino[2,3-b]pyridin-6-yl)-4-((4-(3-methylmorpholino)-6-(methylsulfonyl)pyridin-2-yl)amino)pyridin-2-yl)acetamide;
(S)-N-(5-(3,3-dimethyl-2,3-dihydro-[1,4]dioxino[2,3-b]pyridin-6-yl)-4-((4-(1-methoxyethyl)-6-(methylsulfonyl)pyridin-2-yl)amino)pyridin-2-yl)acetamide;
(R)-N-(5-(3,3-dimethyl-2,3-dihydro-[1,4]dioxino[2,3-b]pyridin-6-yl)-4-((4-(1-methoxyethyl)-6-(methylsulfonyl)pyridin-2-yl)amino)pyridin-2-yl)acetamide;
(R)-N-(5-(2,2-dimethyl-2,3-dihydro-[1,4]dioxino[2,3-b]pyridin-6-yl)-4-((4-(3-methoxypyrrolidin-1-yl)-6-(methylsulfonyl)pyridin-2-yl)amino)pyridin-2-yl)acetamide;
(S)-N-(5-(2,2-dimethyl-2,3-dihydro-[1,4]dioxino[2,3-b]pyridin-6-yl)-4-((4-(3-methoxypyrrolidin-1-yl)-6-(methylsulfonyl)pyridin-2-yl)amino)pyridin-2-yl)acetamide.;
N-(4-((4-(4-aminotetrahydro-2H-pyran-4-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide;
N-(4-((4-(4-(aminomethyl)tetrahydro-2H-pyran-4-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide;
N-(5-(2,2-dimethyl-2,3-dihydro-[1,4]dioxino[2,3-b]pyridin-6-yl)-4-((4-(3-methoxyazetidin-1-yl)-6-(methylsulfonyl)pyridin-2-yl)amino)pyridin-2-yl)acetamide;
(R/S)-N-(4-((4-(1,4-dioxan-2-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide;
(S/R)-N-(4-((4-(1,4-dioxan-2-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide;
N-(5-(2,2-dimethyl-2,3-dihydro-[1,4]dioxino[2,3-b]pyridin-6-yl)-4-((4-(4-methoxypiperidin-1-yl)-6-(methylsulfonyl)pyridin-2-yl)amino)pyridin-2-yl)acetamide;
N-(5-(2,2-dimethyl-2,3-dihydro-[1,4]dioxino[2,3-b]pyridin-6-yl)-4-((4-((cis)-3-methoxycyclobutyl)-6-(methylsulfonyl)pyridin-2-yl)amino)pyridin-2-yl)acetamid;
N-(5-(2,2-dimethyl-2,3-dihydro-[1,4]dioxino[2,3-b]pyridin-6-yl)-4-((4-((trans)-3-methoxycyclobutyl)-6-(methylsulfonyl)pyridin-2-yl)amino)pyridin-2-yl)acetamide;
N-(5-(2,2-dimethyl-2,3-dihydro-[1,4]dioxino[2,3-b]pyridin-6-yl)-4-((4-(4-hydroxy-4-methylpiperidin-1-yl)-6-(methylsulfonyl)pyridin-2-yl)amino)pyridin-2-yl)acetamide;
N-(5-(2,2-dimethyl-2,3-dihydro-[1,4]dioxino[2,3-b]pyridin-6-yl)-4-((4-(3-hydroxy-3-methylazetidin-1-yl)-6-(methylsulfonyl)pyridin-2-yl)amino)pyrid-in-2-yl)acetamide ;
N-(5-(2,2-dimethyl-2,3-dihydro-[1,4]dioxino[2,3-b]pyridin-6-yl)-4-((4-(3-hydroxy-3-methylpyrrolidin-1-yl)-6-(methyl sulfonyl)pyridin-2-yl)amino)pyridin-2-yl) acetamide;
N-(6-(2,2-dimethyl-2,3-dihydro-[1,4]dioxino[2,3-b]pyridin-6-yl)-5-((6-(methylsulfonyl)-4-(tetrahydro-2H-pyran-4-yl)pyridin-2-yl)amino)pyridazin-3-yl)acetamide;
(R)-N-(5-(2,2-dimethyl-2,3-dihydro-[1,4]dioxino[2,3-b]pyridin-6-yl)-4-((4-(3-(2-hydroxyethoxy)pyrrolidin-1-yl)-6-(methylsulfonyl)pyridin-2-yl)amino)pyridin-2-yl) acetamide;
N-(5-(2,2-dimethyl-2,3-dihydro-[1,4]dioxino[2,3-b]pyridin-6-yl)-4-((6-(methylsulfonyl)-4-(1-(2,2,2-trifluoroethyl)piperidin-4-yl)pyridin-2-yl)amino)pyridin-2-yl) acetamide;
(S)-N-(5-(2,2-dimethyl-2,3-dihydro-[1,4]dioxino[2,3-b]pyridin-6-yl)-4-((4-(3-(2-hydroxyethoxy)pyrrolidin-1-yl)-6-(methylsulfonyl)pyridin-2-yl)amino)pyridin-2-yl)acetamide;
N-(5-(2,2-dimethyl-2,3-dihydro-[1,4]dioxino[2,3-b]pyridin-6-yl)-4-((4-methyl-6'-(methylsulfonyl)-[3,4'-bipyridin]-2'-yl)amino)pyridin-2-yl)acetamide;
N-(4-((4-cyano-6-(methylsulfonyl)pyridin-2-yl)amino)-5-(2,2-dimethyl-2,3-dihydro-[1,4]dioxino[2,3-b]pyridin-6-yl)pyridin-2-yl)acetamide;
N-(5-(2,2-dimethyl-2,3-dihydro-[1,4]dioxino[2,3-b]pyridin-6-yl)-4-((4-methyl-6-(methylsulfonyl)-3-oxo-3,4-dihydropyrazin-2-yl)amino)pyridin-2-yl)acetamide;
(S)-N-(5-(2,2-dimethyl-2,3-dihydro-[1,4]dioxino[2,3-b]pyridin-6-yl)-4-((4-(3-methoxypiperidin-1-yl)-6-(methylsulfonyl)pyridin-2-yl)amino)pyridin-2-yl)acetamide;
(R)-N-(5-(2,2-dimethyl-2,3-dihydro-[1,4]dioxino[2,3-b]pyridin-6-yl)-4-((4-(3-methoxypiperidin-1-yl)-6-(methylsulfonyl)pyridin-2-yl)amino)pyridin-2-yl)acetamide;
(S)-N-(5-(2,2-dimethyl-2,3-dihydro-[1,4]dioxino[2,3-b]pyridin-6-yl)-4-((4-(3-ethoxypiperidin-1-yl)-6-(methylsulfonyl)pyridin-2-yl)amino)pyridin-2-yl)acetamide;
(R)-N-(5-(2,2-dimethyl-2,3-dihydro-[1,4]dioxino[2,3-b]pyridin-6-yl)-4-((4-(3-ethoxypiperidin-1-yl)-6-(methylsulfonyl)pyridin-2-yl)amino)pyridin-2-yl)acetamide;
(S)-N-(5-(2,2-dimethyl-2,3-dihydro-[1,4]dioxino[2,3-b]pyridin-6-yl)-4-((6-(methylsulfonyl)-4-(3-(trifluoromethoxy)pyrrolidin-1-yl)pyridin-2-yl)amino)pyridin-2-yl)acetamide;
(R)-N-(5-(2,2-dimethyl-2,3-dihydro-[1,4]dioxino[2,3-b]pyridin-6-yl)-4-((6-(methylsulfonyl)-4-(3-(trifluoromethoxy)pyrrolidin-1-yl)pyridin-2-yl)amino)pyridin-2-yl)acetamide;
(S)-1-(2-((2-acetamido-5-(2,2-dimethyl-2,3-dihydro-[1,4]dioxino[2,3-b]pyridin-6-yl)pyridin-4-yl)amino)-6-(methylsulfonyl)pyridin-4-yl)pyrrolidine-2-carboxamide;
(R)-1-(2-((2-acetamido-5-(2,2-dimethyl-2,3-dihydro-[1,4]dioxino[2,3-b]pyridin-6-yl)pyridin-4-yl)amino)-6-(methylsulfonyl)pyridin-4-yl)pyrrolidine-2-carboxamide;
(S)-N-(5-(2,2-dimethyl-2,3-dihydro-[1,4]dioxino[2,3-b]pyridin-6-yl)-4-((4-(3-(methoxymethyl)pyrrolidin-1-yl)-6-(methylsulfonyl)pyridin-2-yl)amino)pyridin-2-yl)acetamide;
(R)-N-(5-(2,2-dimethyl-2,3-dihydro-[1,4]dioxino[2,3-b]pyridin-6-yl)-4-((4-(3-(methoxymethyl)pyrrolidin-1-yl)-6-(methylsulfonyl)pyridin-2-yl)amino)pyridin-2-yl)acetamide;
(S)-N-(5-(2,2-dimethyl-2,3-dihydro-[1,4]dioxino[2,3-b]pyridin-6-yl)-4-((6-(methylsulfonyl)-4-(tetrahydro-2H-pyran-3-yl)pyridin-2-yl)amino)pyridin-2-yl)acetamide;
(R)-N-(5-(2,2-dimethyl-2,3-dihydro-[1,4]dioxino[2,3-b]pyridin-6-yl)-4-((6-(methylsulfonyl)-4-(tetrahydro-2H-pyran-3-yl)pyridin-2-yl)amino)pyridin-2-yl)acetamide;
(S)-N-(5-(2,2-dimethyl-2,3-dihydro-[1,4]dioxino[2,3-b]pyridin-6-yl)-4-((5-(3-methoxypyrrolidin-1-yl)-6-(methylsulfonyl)pyridin-2-yl)amino)pyridin-2-yl)acetamide;
(R)-N-(5-(2,2-dimethyl-2,3-dihydro-[1,4]dioxino[2,3-b]pyridin-6-yl)-4-((5-(3-methoxypyrrolidin-1-yl)-6-(methylsulfonyl)pyridin-2-yl)amino)pyridin-2-yl)acetamide;
N-(5-(2,2-dimethyl-2,3-dihydro-[1,4]dioxino[2,3-b]pyridin-6-yl)-4-((6-(methylsulfonyl)-5-(tetrahydro-2H-pyran-4-yl)pyridin-2-yl)amino)pyridin-2-yl)acetamide;
N-(5-(2,2-dimethyl-2,3-dihydro-[1,4]dioxino[2,3-b]pyridin-6-yl)-4-((4-(5-methylpyridazin-4-yl)-6-(methylsulfonyl)pyridin-2-yl)amino)pyridin-2-yl)acetamide;
N-(5-(2,2-dimethyl-2,3-dihydro-[1,4]dioxino[2,3-b]pyridin-6-yl)-4-((4-(5-methoxypyridazin-4-yl)-6-(methylsulfonyl)pyridin-2-yl)amino)pyridin-2-yl)acetamide;
N-(4-((4-(cis-3-cyano-4-hydroxypyrrolidin-1-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide;
N-(4-((4-(trans-3-cyano-4-hydroxypyrrolidin-1-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide;
N-(4-((4-(cis-3-cyano-4-methoxypyrrolidin-1-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide;
N-(4-((4-(trans-3-cyano-4-methoxypyrrolidin-1-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide;
(S)-N-(5-(2,2-dimethyl-2,3-dihydro-[1,4]dioxino[2,3-b]pyridin-6-yl)-4-((6-(methylsulfonyl)-4-(tetrahydrofuran-3-yl)pyridin-2-yl)amino)pyridin-2-yl)acetamide;
(R)-N-(5-(2,2-dimethyl-2,3-dihydro-[1,4]dioxino[2,3-b]pyridin-6-yl)-4-((6-(methylsulfonyl)-4-(tetrahydrofuran-3-yl)pyridin-2-yl)amino)pyridin-2-yl)acetamide;
(S)-N-(5-(2,2-dimethyl-2,3-dihydro-[1,4]dioxino[2,3-b]pyridin-6-yl)-4-((4-(2-(methoxymethyl)pyrrolidin-1-yl)-6-(methylsulfonyl)pyridin-2-yl)amino)pyridin-2-yl)acetamide;
(R)-N-(5-(2,2-dimethyl-2,3-dihydro-[1,4]dioxino[2,3-b]pyridin-6-yl)-4-((4-(2-(methoxymethyl)pyrrolidin-1-yl)-6-(methylsulfonyl)pyridin-2-yl)amino)pyridin-2-yl)acetamide;
N-(5-(2,2-dimethyl-2,3-dihydro-[1,4]dioxino[2,3-b]pyridin-6-yl)-4-((6-(methylsulfonyl)-3-(tetrahydro-2H-pyran-4-yl)pyridin-2-yl)amino)pyridin-2-yl)acetamide;
(S)-N-(5-(2,2-dimethyl-2,3-dihydro-[1,4]dioxino[2,3-b]pyridin-6-yl)-4-((3-(3-methoxypyrrolidin-1-yl)-6-(methylsulfonyl)pyridin-2-yl)amino)pyridin-2-yl)acetamide;
(R)-N-(5-(2,2-dimethyl-2,3-dihydro-[1,4]dioxino[2,3-b]pyridin-6-yl)-4-((3-(3-methoxypyrrolidin-1-yl)-6-(methylsulfonyl)pyridin-2-yl)amino)pyridin-2-yl)acetamide;
cis-N-(5-(2,2-dimethyl-2,3-dihydro-[1,4]dioxino[2,3-b]pyridin-6-yl)-4-((4-((cis-4-hydroxycyclohexyl)oxy)-6-(methylsulfonyl)pyridin-2-yl)amino)pyridin-2-yl)acetamide;
N-(5-(2,2-dimethyl-2,3-dihydro-[1,4]dioxino[2,3-b]pyridin-6-yl)-4-((4-((trans-4-hydroxycyclohexyl)oxy)-6-(methylsulfonyl)pyridin-2-yl)amino)pyridin-2-yl)acetamide;
N-(5-(2,2-dimethyl-2,3-dihydro-[1,4]dioxino[2,3-b]pyridin-6-yl)-4-((4-((cis-4-methoxycyclohexyl)oxy)-6-(methylsulfonyl)pyridin-2-yl)amino)pyridin-2-yl)acetamide;
N-(5-(2,2-dimethyl-2,3-dihydro-[1,4]dioxino[2,3-b]pyridin-6-yl)-4-((4-((trans-4-methoxycyclohexyl)oxy)-6-(methylsulfonyl)pyridin-2-yl)amino)pyridin-2-yl)acetamide;
N-(5-(2,2-dimethyl-2,3-dihydro-[1,4]dioxino[2,3-b]pyridin-6-yl)-4-((6-(methylsulfonyl)-4-(5-azaspiro[2.4]heptan-5-yl)pyridin-2-yl)amino)pyridin-2-yl)acetamide;
N-(5-(2,2-dimethyl-2,3-dihydro-[1,4]dioxino[2,3-b]pyridin-6-yl)-4-((4-((3-methyloxetan-3-yl)methoxy)-6-(methylsulfonyl)pyridin-2-yl)amino)pyridin-2-yl)acetamide;
N-(5-(2,2-dimethyl-2,3-dihydro-[1,4]dioxino[2,3-b]pyridin-6-yl)-4-((4-(3,5-dimethylisoxazol-4-yl)-6-(methylsulfonyl)pyridin-2-yl)amino)pyridin-2-yl)acetamide;
N-(5-(2,2-dimethyl-2,3-dihydro-[1,4]dioxino[2,3-b]pyridin-6-yl)-4-((4-((2,4-dioxothiazolidin-3-yl)methyl)-6-(methylsulfonyl)pyridin-2-yl)amino)pyridin-2-yl)acetamide;
N-(5-(2,2-dimethyl-2,3-dihydro-[1,4]dioxino[2,3-b]pyridin-6-yl)-4-((4-methoxy-6'-(methylsulfonyl)-[3,4'-bipyridin]-2'-yl)amino)pyridin-2-yl)acetamide;
(R)-N-(5-(2,2-dimethyl-2,3-dihydro-[1,4]dioxino[2,3-b]pyridin-6-yl)-4-((4-(3-methylpyrrolidin-1-yl)-6-(methylsulfonyl)pyridin-2-yl)amino)pyridin-2-yl)acetamide;
(S)-N-(5-(2,2-dimethyl-2,3-dihydro-[1,4]dioxino[2,3-b]pyridin-6-yl)-4-((4-(3-methylpyrrolidin-1-yl)-6-(methylsulfonyl)pyridin-2-yl)amino)pyridin-2-yl)acetamide;
N-(5-(2,2-dimethyl-2,3-dihydro-[1,4]dioxino[2,3-b]pyridin-6-yl)-4-((6-(methylsulfonyl)-4-(pyrrolidin-1-yl)pyridin-2-yl)amino)pyridin-2-yl)acetamide;
N-(5-(2,2-dimethyl-2,3-dihydro-[1,4]dioxino[2,3-b]pyridin-6-yl)-4-((4-(2-hydroxypropan-2-yl)-6'-(methylsulfonyl)-[3,4'-bipyridin]-2'-yl)amino)pyridin-2-yl)acetamide;
N-(4-((4-(3-azabicyclo[3.1.0]hexan-3-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide;
(S)-N-(4-((4-(3-(cyanomethoxy)pyrrolidin-1-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide;
N-(4-((6-cyano-6'-(methylsulfonyl)-[3,4'-bipyridin]-2'-yl)amino)-5-(2,2-dimethyl-2,3-dihydro-[1,4]dioxino[2,3-b]pyridin-6-yl)pyridin-2-yl)acetamide;
N-(4-((2-cyano-6'-(methylsulfonyl)-[3,4'-bipyridin]-2'-yl)amino)-5-(2,2-dimethyl-2,3-dihydro-[1,4]dioxino[2,3-b]pyridin-6-yl)pyridin-2-yl)acetamide;
N-(5-(2,2-dimethyl-2,3-dihydro-[1,4]dioxino[2,3-b]pyridin-6-yl)-4-((4-(3-methylpyrazin-2-yl)-6-(methylsulfonyl)pyridin-2-yl)amino)pyridin-2-yl)acetamide;
N-(5-(2,2-dimethyl-2,3-dihydro-[1,4]dioxino[2,3-b]pyridin-6-yl)-4-((6-(methylsulfonyl)-4-(2,2,6,6-tetramethyltetrahydro-2H-pyran-4-yl)pyridin-2-yl)amino)pyridin-2-yl)acetamide;
(R)-N-(4-((4-(2,2-dichlorocyclopropyl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide;
(S)-N-(4-((4-(2,2-dichlorocyclopropyl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide;
N-(4-((4-(5-cyanopyridazin-4-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide;
N-(4-((4-cyano-6'-(methylsulfonyl)-[3,4'-bipyridin]-2'-yl)amino)-5-(2,2-dimethyl-2,3-dihydro-[1,4]dioxino[2,3-b]pyridin-6-yl)pyridin-2-yl)acetamide;
N-(5-(2,2-dimethyl-2,3-dihydro-[1,4]dioxino[2,3-b]pyridin-6-yl)-4-((4-(5-fluoropyridazin-4-yl)-6-(methylsulfonyl)pyridin-2-yl)amino)pyridin-2-yl)acetamide;
N-(5-(2,2-dimethyl-2,3-dihydro-[1,4]dioxino[2,3-b]pyridin-6-yl)-4-((4-fluoro-6'-(methylsulfonyl)-[3,4'-bipyridin]-2'-yl)amino)pyridin-2-yl)acetamide;
N-(5-(2,2-dimethyl-2,3-dihydro-[1,4]dioxino[2,3-b]pyridin-6-yl)-4-((6'-(methylsulfonyl)-[2,4'-bipyridin]-2'-yl)amino)pyridin-2-yl)acetamide;
N-(5-(2,2-dimethyl-2,3-dihydro-[1,4]dioxino[2,3-b]pyridin-6-yl)-4-((6-(methylsulfonyl)-4-(thiazol-4-yl)pyridin-2-yl)amino)pyridin-2-yl)acetamide;
N-(4-((4-(1-cyanocyclopropyl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide;
N-(4-((4-((3R)-3-cyano-2-azabicyclo[3.1.0]hexan-2-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide;
N-(4-((4-((3S)-3-cyano-2-azabicyclo[3.1.0]hexan-2-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide;
N-(5-(2,2-dimethyl-2,3-dihydro-[1,4]dioxino[2,3-b]pyridin-6-yl)-4-((4-(2-methyl-2-morpholinopropoxy)-6-(methylsulfonyl)pyridin-2-yl)amino)pyridin-2-yl)acetamide;
N-(5-(2,2-dimethyl-2,3-dihydro-[1,4]dioxino[2,3-b]pyridin-6-yl)-4-((4-(2-methoxy-2-methylpropoxy)-6-(methylsulfonyl)pyridin-2-yl)amino)pyridin-2-yl)acetamide;
N-(5-(2,2-dimethyl-2,3-dihydro-[1,4]dioxino[2,3-b]pyridin-6-yl)-4-((4-(2-hydroxy-2-methylpropoxy)-6-(methylsulfonyl)pyridin-2-yl)amino)pyridin-2-yl)acetamide;
N-(5-(2,2-dimethyl-2,3-dihydro-[1,4]dioxino[2,3-b]pyridin-6-yl)-4-((4-(3,3-dimethylmorpholino)-6-(methylsulfonyl)pyridin-2-yl)amino)pyridin-2-yl)acetamide;
N-(5-(2,2-dimethyl-2,3-dihydro-[1,4]dioxino[2,3-b]pyridin-6-yl)-4-((4-isopropyl-6'-(methylsulfonyl)-[3,4'-bipyridin]-2'-yl)amino)pyridin-2-yl)acetamide;
N-(5-(2,2-dimethyl-2,3-dihydro-[1,4]dioxino[2,3-b]pyridin-6-yl)-4-((4-(1-hydroxyethyl)-6'-(methylsulfonyl)-[3,4'-bipyridin]-2'-yl)amino)pyridin-2-yl)acetamide;
N-(5-(2,2-dimethyl-2,3-dihydro-[1,4]dioxino[2,3-b]pyridin-6-yl)-4-((4-(1-methoxyethyl)-6'-(methylsulfonyl)-[3,4'-bipyridin]-2'-yl)amino)pyridin-2-yl)acetamide;
N-(5-(2,2-dimethyl-2,3-dihydro-[1,4]dioxino[2,3-b]pyridin-6-yl)-4-((6-(methylsulfonyl)-[1,3]dioxolo[4,5-c]pyridin-4-yl)amino)pyridin-2-yl)acetamide;
N-(5-(2,2-dimethyl-2,3-dihydro-[1,4]dioxino[2,3-b]pyridin-6-yl)-4-((7-(methylsulfonyl)-2,3-dihydro-[1,4]dioxino[2,3-c]pyridin-5-yl)amino)pyridin-2-yl)acetamide;
N-(5-(2,2-dimethyl-2,3-dihydro-[1,4]dioxino[2,3-b]pyridin-6-yl)-4-((6-(methylsulfonyl)-4-(trifluoromethyl)pyridin-2-yl)amino)pyridin-2-yl)acetamide;
N-(4-((4-(difluoromethoxy)-6-(methylsulfonyl)pyridin-2-yl)amino)-5-(2,2-dimethyl-2,3-dihydro-[1,4]dioxino[2,3-b]pyridin-6-yl)pyridin-2-yl)acetamide;
N-(4-((4-cyclobutoxy-6-(methylsulfonyl)pyridin-2-yl)amino)-5-(2,2-dimethyl-2,3-dihydro-[1,4]dioxino[2,3-b]pyridin-6-yl)pyridin-2-yl)acetamide;
N-(5-(2,2-bis(methyl-d3)-2,3-dihydro-[1,4]dioxino[2,3-b]pyridin-6-yl)-4-((6-(methylsulfonyl)-4-(tetrahydro-2H-pyran-4-yl)pyridin-2-yl)amino)pyridin-2-yl)acetamide;
N-(5-(2,2-bis(methyl-d3)-2,3-dihydro-[1,4]dioxino[2,3-b]pyridin-6-yl)-4-((4-(methoxy-d3)-6-(methylsulfonyl)pyridin-2-yl)amino)pyridin-2-yl)acetamide;
N-(5-(4,4-dimethyl-4,5-dihydrothiazol-2-yl)-4-((6-(methylsulfonyl)pyridin-2-yl)amino)pyridin-2-yl)acetamide;
N-(5-(4,4-dimethyl-4,5-dihydrothiazol-2-yl)-4-((6-(methylsulfonyl)-4-(tetrahydro-2H-pyran-4-yl)pyridin-2-yl)amino)pyridin-2-yl)acetamide;
N-(4-((4-isopropoxy-6-(methylsulfonyl)pyridin-2-yl)amino)-5-(1-methyl-1,3a,4,6,7,7a-hexahydropyrano[4,3-c]pyrazol-3-yl)pyridin-2-yl)acetamide;
N-(5-(6,7-dihydro-4H-pyrano[4,3-d]thiazol-2-yl)-4-((4-methyl-6-(methylsulfonyl)pyridin-2-yl)amino)pyridin-2-yl)acetamide;
N-(4-((4-isopropoxy-6-(methylsulfonyl)pyridin-2-yl)amino)-5-(5-methyl-4,5,6,7-tetrahydropyrazolo[1,5-a]pyrazin-2-yl)pyridin-2-yl)acetamide;
N-(5-([1,3]dioxolo[4,5-c]pyridin-6-yl)-4-((4-methyl-6-(methylsulfonyl)pyridin-2-yl)amino)pyridin-2-yl)acetamide;
N-(5-([1,3]dioxolo[4,5-c]pyridin-4-yl)-4-((4-methyl-6-(methylsulfonyl)pyridin-2-yl)amino)pyridin-2-yl)acetamide;
N-(5-(benzo[d][1,3]dioxol-4-yl)-4-((4-methyl-6-(methylsulfonyl)pyridin-2-yl)amino)pyridin-2-yl)acetamide;
N-(5-(5-methyl-4,5,6,7-tetrahydrothiazolo[5,4-c]pyridin-2-yl)-4-((4-methyl-6-(methylsulfonyl)pyridin-2-yl)amino)pyridin-2-yl)acetamide;
N-(5-(imidazo[1,2-a]pyrimidin-2-yl)-4-((4-methyl-6-(methylsulfonyl)pyridin-2-yl)amino)pyridin-2-yl)acetamide;
N-(5-(1-methyl-1,4-dihydrochromeno[4,3-c]pyrazol-6-yl)-4-((4-methyl-6-(methylsulfonyl)pyridin-2-yl)amino)pyridin-2-yl)acetamide;
N-(5-(imidazo[2',1':2,3]thiazolo[5,4-b]pyridin-7-yl)-4-((4-methyl-6-(methylsulfonyl)pyridin-2-yl)amino)pyridin-2-yl)acetamide;
N-(5-(1-methyl-1,4-dihydropyrazolo[3',4':4,5]pyrano[2,3-b]pyridin-7-yl)-4-((4-methyl-6-(methylsulfonyl)pyridin-2-yl)amino)pyridin-2-yl)acetamide;
N-(4'-((4,4-dioxido-2,3-dihydro-[1,4]oxathiino[3,2-b]pyridin-6-yl)amino)-[2,3'-bipyridin]-6'-yl)acetamide;
N-(4-((4,4-dioxido-2,3-dihydro-[1,4]oxathiino[3,2-b]pyridin-6-yl)amino)-5-(1-methyl-1H-pyrazol-3-yl)pyridin-2-yl)acetamide;
N-(5-(imidazo[1,2-b]pyridazin-6-yl)-4-((4-methyl-6-(methylsulfonyl)pyridin-2-yl)amino)pyridin-2-yl)acetamide;
N-(5-(1-methyl-1H-pyrazol-3-yl)-4-((7-(methylsulfonyl)-3,4-dihydro-2H-pyrano[3,2-c]pyridin-5-yl)amino)pyridin-2-yl)acetamide;
N-(4-((5,5-dioxido-3,4-dihydro-2H-[1,4]oxathiepino[3,2-b]pyridin-7-yl)amino)-5-(1-methyl-1H-pyrazol-3-yl)pyridin-2-yl)acetamide;
N-(4-((4-(2-hydroxypropan-2-yl)-6-(methylsulfonyl)pyridin-2-yl)amino)-5-(1-methyl-1H-pyrazol-3-yl)pyridin-2-yl)acetamide;
N-(5-(1-methyl-1H-pyrazol-3-yl)-4-((6-(methylsulfonyl)-4-(tetrahydrofuran-3-yl)pyridin-2-yl)amino)pyridin-2-yl)acetamide;
N-(4-((4-(3-(hydroxymethyl)tetrahydrofuran-3-yl)-6-(methylsulfonyl)pyridin-2-yl)amino)-5-(1-methyl-1H-pyrazol-3-yl)pyridin-2-yl)acetamide;
N-(4-((4-(1-hydroxycyclopropyl)-6-(methylsulfonyl)pyridin-2-yl)amino)-5-(1-methyl-1H-pyrazol-3-yl)pyridin-2-yl)acetamide;
N-(4-((4-(2-hydroxy-2-methylpropoxy)-6-(methylsulfonyl)pyridin-2-yl)amino)-5-(1-methyl-1H-pyrazol-3-yl)pyridin-2-yl)acetamide;
N-(4-((4-(3-hydroxy-2,2-dimethylpropoxy)-6-(methylsulfonyl)pyridin-2-yl)amino)-5-(1-methyl-1H-pyrazol-3-yl)pyridin-2-yl)acetamide;
N-(5-(imidazo[1,5-b]pyridazin-2-yl)-4-((4-methyl-6-(methylsulfonyl)pyridin-2-yl)amino)pyridin-2-yl)acetamide;
N-(4-((4-(3-hydroxytetrahydrofuran-3-yl)-6-(methylsulfonyl)pyridin-2-yl)amino)-5-(1-methyl-1H-pyrazol-3-yl)pyridin-2-yl)acetamide;
N-(5-(1-methyl-1H-pyrazol-3-yl)-4-((4-(1-methyl-1H-pyrazol-3-yl)-6-(methylsulfonyl)pyridin-2-yl)amino)pyridin-2-yl)acetamide;
N-(4-((4-((1-hydroxycyclopropyl)methoxy)-6-(methylsulfonyl)pyridin-2-yl)amino)-5-(1-methyl-1H-pyrazol-3-yl)pyridin-2-yl)acetamide;
N-(4-((4-((1-methoxycyclopropyl)methoxy)-6-(methylsulfonyl)pyridin-2-yl)amino)-5-(1-methyl-1H-pyrazol-3-yl)pyridin-2-yl)acetamide;
N-(5-(1-methyl-1H-pyrazol-3-yl)-4-((6-(methylsulfonyl)-4-morpholinopyridin-2-yl)amino)pyridin-2-yl)acetamide;
N-(5-(benzo[d]thiazol-2-yl)-4-((4-(3-(hydroxymethyl)tetrahydrofuran-3-yl)-6-(methylsulfonyl)pyridin-2-yl)amino)pyridin-2-yl)acetamide;
N-(4-((1,1-dioxido-3,4-dihydro-2H-thiopyrano[2,3-b]pyridin-7-yl)amino)-5-(1-methyl-1H-pyrazol-3-yl)pyridin-2-yl)acetamide;
N-(5-(1-methyl-1H-pyrazol-3-yl)-4-((7-(methylsulfonyl)-3,4-dihydro-2H-pyrano[3,2-c]pyridin-5-yl)amino)pyridin-2-yl)acetamide;
N-(5-(1-methyl-1H-pyrazol-3-yl)-4-((6-(methylsulfonyl)-[1,3]dioxolo[4,5-c]pyridin-4-yl)amino)pyridin-2-yl)acetamide;
N-(5-(1-methyl-1H-pyrazol-3-yl)-4-((6-(methylsulfonyl)imidazo[1,2-a]pyrazin-8-yl)amino)pyridin-2-yl)acetamide;
N-(5-(1-methyl-1H-pyrazol-3-yl)-4-((5-(methylsulfonyl)-3,4-dihydro-2H-pyrano[3,2-c]pyridin-7-yl)amino)pyridin-2-yl)acetamide;
N-(5-(2,6-dimethylmorpholino)-4'-((4-(2-hydroxypropan-2-yl)-6-(methylsulfonyl)pyridin-2-yl)amino)-[2,3'-bipyridin]-6'-yl)acetamide;
N-(5-(2,6-dimethylmorpholino)-4'-((6-(methylsulfonyl)-4-(tetrahydrofuran-3-yl)pyridin-2-yl)amino)-[2,3'-bipyridin]-6'-yl)acetamide;
N-(5-(2,6-dimethylmorpholino)-4'-((4-(3-(hydroxymethyl)tetrahydrofuran-3-yl)-6-(methylsulfonyl)pyridin-2-yl)amino)-[2,3'-bipyridin]-6'-yl)acetamide;
N-(5-(2,6-dimethylmorpholino)-4'-((4-(1-hydroxycyclopropyl)-6-(methylsulfonyl)pyridin-2-yl)amino)-[2,3'-bipyridin]-6'-yl)acetamide;
N-(5-(2,6-dimethylmorpholino)-4'-((4-(2-hydroxy-2-methylpropoxy)-6-(methylsulfonyl)pyridin-2-yl)amino)-[2,3'-bipyridin]-6'-yl)acetamide;
N-(5-(2,6-dimethylmorpholino)-4'-((4-(3-hydroxy-2,2-dimethylpropoxy)-6-(methylsulfonyl)pyridin-2-yl)amino)-[2,3'-bipyridin]-6'-yl)acetamide;
N-(5-(2,6-dimethylmorpholino)-4'-((4-(3-methoxytetrahydrofuran-3-yl)-6-(methylsulfonyl)pyridin-2-yl)amino)-[2,3'-bipyridin]-6'-yl)acetamide;
N-(5-(2,6-dimethylmorpholino)-4'-((4-(3-hydroxytetrahydrofuran-3-yl)-6-(methylsulfonyl)pyridin-2-yl)amino)-[2,3'-bipyridin]-6'-yl)acetamide;
N-(5-(2,6-dimethylmorpholino)-4'-((4-(1-methyl-1H-pyrazol-3-yl)-6-(methylsulfonyl)pyridin-2-yl)amino)-[2,3'-bipyridin]-6'-yl)acetamide;
N-(5-(2,6-dimethylmorpholino)-4'-((4-((1-hydroxycyclopropyl)methoxy)-6-(methylsulfonyl)pyridin-2-yl)amino)-[2,3'-bipyridin]-6'-yl)acetamide;
N-(5-(2,6-dimethylmorpholino)-4'-((4-((1-methoxycyclopropyl)methoxy)-6-(methylsulfonyl)pyridin-2-yl)amino)-[2,3'-bipyridin]-6'-yl)acetamide;
N-(5-(2,6-dimethylmorpholino)-4'-((6-(methylsulfonyl)-4-morpholinopyridin-2-yl)amino)-[2,3'-bipyridin]-6'-yl)acetamide;
N-(5-(2,6-dimethylmorpholino)-4'-((5,5-dioxido-3,4-dihydro-2H-[1,4]oxathiepino[3,2-b]pyridin-7-yl)amino)-[2,3'-bipyridin]-6'-yl)acetamide;
N-(5-(2,6-dimethylmorpholino)-4'-((1,1-dioxido-3,4-dihydro-2H-thiopyrano[2,3-b]pyridin-7-yl)amino)-[2,3'-bipyridin]-6'-yl)acetamide;
N-(5-(2,6-dimethylmorpholino)-4'-((7-(methylsulfonyl)-3,4-dihydro-2H-pyrano[3,2-c]pyridin-5-yl)amino)-[2,3'-bipyridin]-6'-yl)acetamide;
N-(5-(2,6-dimethylmorpholino)-4'-((6-(methylsulfonyl)-[1,3]dioxolo[4,5-c]pyridin-4-yl)amino)-[2,3'-bipyridin]-6'-yl)acetamide;
N-(5-(2,6-dimethylmorpholino)-4'-((6-(methylsulfonyl)imidazo[1,2-a]pyrazin-8-yl)amino)-[2,3'-bipyridin]-6'-yl)acetamide;
N-(5-(2,6-dimethylmorpholino)-4'-((5-(methylsulfonyl)-3,4-dihydro-2H-pyrano[3,2-c]pyridin-7-yl)amino)-[2,3'-bipyridin]-6'-yl)acetamide;
N-(5-(6,7-dihydro-4H-pyrano[4,3-d]thiazol-2-yl)-4-((4-(3-(hydroxymethyl)tetrahydrofuran-3-yl)-6-(methylsulfonyl)pyridin-2-yl)amino)pyridin-2-yl)acetamide;
N-(5-(benzo[d][1,3]dioxol-4-yl)-4-((4-(3-(hydroxymethyl)tetrahydrofuran-3-yl)-6-(methylsulfonyl)pyridin-2-yl)amino)pyridin-2-yl)acetamide;
N-(5-(benzo[d]isoxazol-3-yl)-4-((4-(3-(hydroxymethyl)tetrahydrofuran-3-yl)-6-(methylsulfonyl)pyridin-2-yl)amino)pyridin-2-yl)acetamide;
N-(5-(benzo[d]isothiazol-3-yl)-4-((4-(3-(hydroxymethyl)tetrahydrofuran-3-yl)-6-(methylsulfonyl)pyridin-2-yl)amino)pyridin-2-yl)acetamide;
N-(5-(benzo[b]thiophen-2-yl)-4-((4-(3-(hydroxymethyl)tetrahydrofuran-3-yl)-6-(methylsulfonyl)pyridin-2-yl)amino)pyridin-2-yl)acetamide;
N-(4-((4-(3-(hydroxymethyl)tetrahydrofuran-3-yl)-6-(methylsulfonyl)pyridin-2-yl)amino)-5-(imidazo[1,2-a]pyrimidin-2-yl)pyridin-2-yl)acetamide;
N-(4-((4-(3-(hydroxymethyl)tetrahydrofuran-3-yl)-6-(methylsulfonyl)pyridin-2-yl)amino)-5-(1-methyl-1,4-dihydrochromeno[4,3-c]pyrazol-6-yl)pyridin-2-yl)acetamide;
N-(4-((4-(3-(hydroxymethyl)tetrahydrofuran-3-yl)-6-(methylsulfonyl)pyridin-2-yl)amino)-5-(imidazo[2',1':2,3]thiazolo[5,4-b]pyridin-7-yl)pyridin-2-yl)acetamide;
N-(4-((4-(3-(hydroxymethyl)tetrahydrofuran-3-yl)-6-(methylsulfonyl)pyridin-2-yl)amino)-5-(1-methyl-1,4-dihydropyrazolo[3',4':4,5]pyrano[2,3-b]pyridin-7-yl)pyridin-2-yl)acetamide;
N-(4-((4-(3-(hydroxymethyl)tetrahydrofuran-3-yl)-6-(methylsulfonyl)pyridin-2-yl)amino)-5-(6-oxo-1,6-dihydropyridazin-3-yl)pyridin-2-yl)acetamide;
N-(4-((4-(3-(hydroxymethyl)tetrahydrofuran-3-yl)-6-(methylsulfonyl)pyridin-2-yl)amino)-5-(6-oxopyridazin-1(6H)-yl)pyridin-2-yl)acetamide;
N-(5-(6-((2S,6R)-2,6-dimethylmorpholino)pyridazin-3-yl)-4-((4-(3-(hydroxymethyl)tetrahydrofuran-3-yl)-6-(methylsulfonyl)pyridin-2-yl)amino)pyridin-2-yl)acetamide;
N-(4-((4-(3-(hydroxymethyl)tetrahydrofuran-3-yl)-6-(methylsulfonyl)pyridin-2-yl)amino)-5-(3-oxo-8,9-dihydropyrano[4,3,2-de]phthalazin-2(3H)-yl)pyridin-2-yl)acetamide;
N-(4-((4-(3-(hydroxymethyl)tetrahydrofuran-3-yl)-6-(methylsulfonyl)pyridin-2-yl)amino)-5-(7-methyl-3-oxo-8,9-dihydro-3H-pyrido[4,3,2-de]phthalazin-2(7H)-yl)pyridin-2-yl)acetamide;
N-(4'-((4-(3-(hydroxymethyl)tetrahydrofuran-3-yl)-6-(methylsulfonyl)pyridin-2-yl)amino)-5-(1-oxoisoindolin-2-yl)-[2,3'-bipyridin]-6'-yl)acetamide;
N-(5-(2,3-dihydropyrazolo[5,1-b]oxazol-6-yl)-4-((4-(3-(hydroxymethyl)tetrahydrofuran-3-yl)-6-(methylsulfonyl)pyridin-2-yl)amino)pyridin-2-yl)acetamide;
N-(5-(2,2-dimethyl-2,3-dihydropyrazolo[5,1-b]oxazol-6-yl)-4-((4-(3-(hydroxymethyl)tetrahydrofuran-3-yl)-6-(methylsulfonyl)pyridin-2-yl)amino)pyridin-2-yl)acetamide;
N-(4-((4-(3-(hydroxymethyl)tetrahydrofuran-3-yl)-6-(methylsulfonyl)pyridin-2-yl)amino)-5-(5'H,7'H-spiro[cyclopropane-1,6'-pyrazolo[5,1-b][1,3]oxazin]-2'-yl)pyridin-2-yl)acetamide;
N-(5-(1H-imidazol-2-yl)-4-((4-methyl-6-(methylsulfonyl)pyridin-2-yl)amino)pyridin-2-yl)acetamide;
N-(5-(4-(2-hydroxypropan-2-yl)furan-2-yl)-4-((4-methyl-6-(methylsulfonyl)pyridin-2-yl)amino)pyridin-2-yl)acetamide;
N-(5-(furan-2-yl)-4-((4-(3-methoxytetrahydrofuran-3-yl)-6-(methylsulfonyl)pyridin-2-yl)amino)pyridin-2-yl)acetamide;
N-(5-(1H-benzo[d]imidazol-2-yl)-4-((4-methyl-6-(methylsulfonyl)pyridin-2-yl)amino)pyridin-2-yl)acetamide;
N-(3-fluoro-5-(2-hydroxypropan-2-yl)-4'-((4-methyl-6-(methylsulfonyl)pyridin-2-yl)amino)-[2,3'-bipyridin]-6'-yl)acetamide;
N-(3-fluoro-5-(2-hydroxypropan-2-yl)-4'-((4-methyl-6-(methylsulfonyl)pyridin-2-yl)amino)-[2,3'-bipyridin]-6'-yl)acetamide;
N-(5-(2-hydroxypropan-2-yl)-4-methyl-4'-((4-methyl-6-(methylsulfonyl)pyridin-2-yl)amino)-[2,3'-bipyridin]-6'-yl)acetamide;
N-(5-(2,6-dimethylmorpholino)-4'-((6-(methylsulfonyl)-4-((tetrahydrofuran-3-yl)methoxy)pyridin-2-yl)amino)-[2,3'-bipyridin]-6'-yl)acetamide;
N-(5-(2,6-dimethylmorpholino)-4'-((6-(methylsulfonyl)-4-(((tetrahydrofuran-3-yl)oxy)methyl)pyridin-2-yl)amino)-[2,3'-bipyridin]-6'-yl)acetamide;
N-(5-(3-methoxy-1-methyl-1H-pyrazol-4-yl)-4-((4-methyl-6-(methylsulfonyl)pyridin-2-yl)amino)pyridin-2-yl)acetamide;
N-(4-((4-(3-(hydroxymethyl)tetrahydrofuran-3-yl)-6-(methylsulfonyl)pyridin-2-yl)amino)-5-(3-methoxy-1-methyl-1H-pyrazol-4-yl)pyridin-2-yl)acetamide;
N-(5-(6,7-dihydro-4H-furo[3,2-c]pyran-2-yl)-4-((4-methyl-6-(methylsulfonyl)pyridin-2-yl)amino)pyridin-2-yl)acetamide;
N-(5-(5-methyl-4,5,6,7-tetrahydrofuro[3,2-c]pyridin-2-yl)-4-((4-methyl-6-(methylsulfonyl)pyridin-2-yl)amino)pyridin-2-yl)acetamide;
N-(5-(benzo[d]oxazol-2-yl)-4-((4-(3-(hydroxymethyl)tetrahydrofuran-3-yl)-6-(methylsulfonyl)pyridin-2-yl)amino)pyridin-2-yl)acetamide;
N-(5-(2,2-dimethyl-3-oxo-3,4-dihydro-2H-pyrido[3,2-b][1,4]oxazin-6-yl)-4-((4-methyl-6-(methylsulfonyl)pyridin-2-yl)amino)pyridin-2-yl)acetamide;
N-(4-((4-methyl-6-(methylsulfonyl)pyridin-2-yl)amino)-5-(pyrazolo[1,5-a]pyrazin-2-yl)pyridin-2-yl)acetamide;
N-(4-((4-(2-methoxyethoxy)-6-(methylsulfonyl)pyridin-2-yl)amino)-5-(pyrazolo[1,5-a]pyrazin-2-yl)pyridin-2-yl)acetamide;
N-(4-((4-methyl-6-(methylsulfonyl)pyridin-2-yl)amino)-5-(pyrazolo[1,5-c]pyrimidin-2-yl)pyridin-2-yl)acetamide;
N-(4-((4-(2-methoxyethoxy)-6-(methylsulfonyl)pyridin-2-yl)amino)-5-(pyrazolo[1,5-c]pyrimidin-2-yl)pyridin-2-yl)acetamide;
N-(4-((4-methyl-6-(methylsulfonyl)pyridin-2-yl)amino)-5-(pyrazolo[1,5-a]pyrimidin-2-yl)pyridin-2-yl)acetamide;
N-(4-((4-(2-methoxyethoxy)-6-(methylsulfonyl)pyridin-2-yl)amino)-5-(pyrazolo[1,5-a]pyrimidin-2-yl)pyridin-2-yl)acetamide;
N-(4-((4-methyl-6-(methylsulfonyl)pyridin-2-yl)amino)-5-(pyrazolo[1,5-a]pyrimidin-5-yl)pyridin-2-yl)acetamide;
N-(4-((4-(2-methoxyethoxy)-6-(methylsulfonyl)pyridin-2-yl)amino)-5-(pyrazolo[1,5-a]pyrimidin-5-yl)pyridin-2-yl)acetamide; or
N-(5-(2,2-dimethyl-2,3-dihydro-1H-pyrido[2,3-b][1,4]oxazin-6-yl)-4-((6-(methylsulfonyl)-4-(tetrahydro-2H-pyran-4-yl)pyridin-2-yl)amino)pyridin-2-yl)acetamide.

14. A pharmaceutical composition comprising one or more compounds according to any one of claims 1-13 or a stereoisomer or pharmaceutically acceptable salt thereof, and a pharmaceutically acceptable excipient.

15. A compound according to any one of claims 1-13 for use in treating an inflammatory or autoimmune disease.

## Patentansprüche

1. Verbindung der Formel (I) 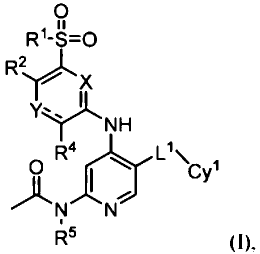
oder ein Stereoisomer oder ein pharmazeutisch annehmbares Salz davon, worin:
X N oder CH ist;
Y N, NR³ oder CR³ ist;
L¹ eine direkte Bindung ist;
R¹ -C₁₋₆-Alkyl, -Halogen-C₁₋₆-alkyl, -C₁₋₆-Alkoxy, -Halogen-C₁₋₆-alkoxy, -C₃₋₆-Cycloalkyl, Aryl oder -NR^{c}R^{d} ist;
R², R³ und R⁴ jeweils unabhängig Wasserstoff, Cyano, Halogen, -C₁₋₆-Alkyl, -C₂₋₆-Alkenyl, -C₂₋₆-Alkinyl, -C₃₋₈-Cycloalkyl, Heterocyclyl, Aryl, Heteroaryl, Oxo, -NO₂, -OR^{e}, -SO₂R^{e}, -COR^{e}, -CO₂R^{e}, -CONR^{e}R^{f}, -C(=NR^{e})NR^{f}R^{g}, -NR^{e}R^{f} -NR^{e}COR^{f}, -NR^{e}CONR^{f}R^{g}, -NR^{e}CO₂R^{f}, -NR^{e}SONR^{f}R^{g}, -NR^{e}SO₂NR^{f}R^{g} oder -NR^{e}SO₂R^{f} sind,
worin das -C₁₋₆-Alkyl, -C₂₋₆-Alkenyl, -C₂₋₆-Alkinyl, -C₃₋₈-Cycloalkyl, Heterocyclyl, Aryl und Heteroaryl jeweils unabhängig unsubstituiert oder mit zumindest einem Substituenten substituiert sind, der aus den folgenden ausgewählt ist:
i) Cyano, Oxo, Halogen, -NR^{m}Rⁿ, -OR^{h}, -C(O)NR^{m}Rⁿ,
ii) Heterocyclyl, das unsubstituiert oder mit zumindest einem Substituenten substituiert ist, der jeweils unabhängig aus Cyano, Oxo, Halogen, Hydroxy, -NR^{m}Rⁿ, substituiertem oder unsubstituiertem -C₁₋₆-Alkyl, substituiertem oder unsubstituiertem -C₁₋₆-Alkoxy und -C(O)NR^{m}R ausgewählt ist, oder
iii) C₁₋₆-Alkyl, das unsubstituiert oder mit zumindest einem Substituenten substituiert ist, der jeweils unabhängig aus Cyano, Halogen, Hydroxy, -NH₂ und C₁₋₆-Alkoxy ausgewählt ist;
worin R^{h} Wasserstoff, Hydroxy, -NH₂, -C₁₋₆-Alkyl, mit Hydroxy substituiertes C₁₋₆-Alkyl oder Heterocyclyl ist und
R^{e}, R^{f} und R^{g} jeweils unabhängig Wasserstoff, -C₁₋₆-Alkyl, -C₁₋₆-Alkoxy, -C₂₋₆-Alkenyl, -C₂₋₆-Alkinyl, Cycloalkyl, Heterocyclyl, Aryl oder Heteroaryl sind, wobei das -C₁₋₆-Alkyl, -C₂₋₆₋Alkenyl, -C₂₋₆-Alkinyl, Cycloalkyl, Heterocyclyl, Aryl und Heteroaryl jeweils unabhängig gegebenenfalls mit einem bis drei Substituenten substituiert sind, die aus Cyano, Oxo, Halogen, Hydroxy, -NR^{m}Rⁿ, -C₁₋₆-Alkyl, -C₁₋₆-Alkoxy, -C₃₋₆-Cycloalkyl, das unsubstituiert oder mit Halogen, Hydroxy oder -C₁₋₆-Alkoxy substituiert ist, -C(O)NR^{m}Rⁿ und Heterocyclyl ausgewählt sind;
R⁵ Wasserstoff oder C₁₋₆-Alkyl ist;
Cy¹ 6- bis 12-gliedriges Aryl oder 5- bis 14-gliedriges Heteroaryl oder 5- bis 14-gliedriges Heterocyclyl ist, die jeweils unsubstituiert oder mit zumindest einem Substituenten Rⁱ substituiert sind,
worin Rⁱ jeweils unabhängig Halogen, Cyano, -C₁₋₆-Alkyl, -C₂₋₆-Alkenyl, -C₂₋₆-Alkinyl, -C₃₋₈-Cycloalkyl, Heterocyclyl, Aryl, Heteroaryl, Oxo, Cyano (-CN), -NO₂, -OR^{j}, -SO₂R^{j}, -COR^{j}, -CO₂R^{k}, -CONR^{j}R^{k}, -C(=NR^{j})NR^{k}R^{l}, -NR^{j}R^{k}, -NR^{j}COR^{k}, -NR^{j}CONR^{k}R^{l}, -NR^{j}CO₂R^{k}, -NR^{j}SO-NR^{k}R^{l}, -NR^{j}SO₂NR^{k}R^{l} oder -NR^{j}SO₂R^{k} ist,
wobei das -C₁₋₆-Alkyl, -C₂₋₆-Alkenyl, -C₂₋₆-Alkinyl, -C₃₋₈-Cycloalkyl, Heterocyclyl, Aryl und Heteroaryl jeweils unsubstituiert oder mit Halogen, -OR^{m}, -C(O)R^{m}, -NR^{m}Rⁿ, -C₁₋₆-Alkyl, C₁₋₆-Alkoxy-, mit C₁₋₆-Alkoxy substituiertem C₁₋₆-Alkyl oder Oxo substituiert sind,
worin R^{j}, R^{k}, R^{l}, R^{m}, R^{h} jeweils unabhängig Wasserstoff, -C₁₋₆-Alkyl, C₁₋₆-Alkoxy-C₁₋₆-alkyl-, -C₂₋₆-Alkenyl, -C₂₋₆-Alkinyl, Cycloalkyl, Heterocyclyl, Aryl oder Heteroaryl sind;
oder (R¹ und R²) oder (R² und R³) oder (R³ und R⁴) zusammen mit den Atomen, an die sie gebunden sind, ein kondensiertes Ringsystem bilden, wobei das kondensierte Ringsystem 0 bis 4 Heteroatome, die aus Sauerstoff (O), Stickstoff (N) und Schwefel (S) ausgewählt sind, als Ringelement(e) umfasst und jeweils unabhängig gegebenenfalls mit Halogen, -C₁₋₆-Alkyl, -C₁₋₆-Alkoxy, mit Halogen substituiertem C₁₋₆-Alkyl, mit Halogen substituiertem C₁₋₆-Alkoxy oder -C₃₋₆-Cycloalkyl substituiert sind;
wobei jegliches Alkyl oder Alkoxy gegebenenfalls an Deuterium angereichert ist.

2. Verbindung nach Anspruch 1, wobei die Verbindung eine der Formel (I-A) 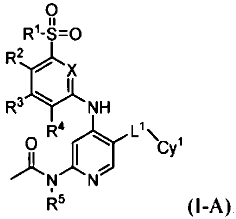
oder ein Stereoisomer oder ein pharmazeutisch annehmbares Salz davon ist, worin:
X N oder CH ist;
L¹ eine direkte Bindung ist;
R¹ -C₁₋₆-Alkyl, -Halogen-C₁₋₆-alkyl, -C₁₋₆-Alkoxy, -Halogen-C₁₋₆-alkoxy, -C₃₋₆-Cycloalkyl, Aryl oder -NR^{m}Rⁿ ist;
R², R³ und R⁴ jeweils unabhängig Wasserstoff, Halogen, -C₁₋₆-Alkyl, -C₂₋₆-Alkenyl, -C₂₋₆-Alkinyl, -C₃₋₈-Cycloalkyl, Heterocyclyl, Aryl, Heteroaryl, Oxo, -CN, -NO₂, -OR^{e}, -SO₂R^{e}, -COR^{e}, -CO₂R^{e}, -CONR^{e}R^{f}, -C(=NR^{e})NR^{f}R^{g}, -NR^{e}R^{f} -NR^{e}COR^{f}, -NR^{e}CONR^{f}R^{g}, -NR^{e}CO₂R^{f}, -NR^{e}SONR^{f}R^{g}, -NR^{e}SO₂NR^{f}R^{g} oder -NR^{e}SO₂R^{f} sind,
worin das -C₁₋₆-Alkyl, -C₂₋₆-Alkenyl, -C₂₋₆-Alkinyl, -C₃₋₈-Cycloalkyl, Heterocyclyl, Aryl und Heteroaryl jeweils unabhängig unsubstituiert oder mit zumindest einem Substituenten substituiert sind, der aus Cyano, Oxo, Halogen, C₁₋₆-Alkyl, das unsubstituiert oder mit Halogen substituiert ist, C₁₋₆-Alkyl, das mit Hydroxy substituiert ist (vorzugsweise Hydroxymethyl oder Hydroxyethyl), -OR^{h}, -C(O)NR^{m}Rⁿ, -NH₂, mit NH₂ substituiertem C₁₋₆-Alkyl und mit C₁₋₆-Alkoxy substituiertem C₁₋₆-Alkyl ausgewählt ist;
worin R^{h} Wasserstoff, Hydroxy, Alkyl, Hydroxy-C₁₋₆-alkyl oder Heterocyclyl ist und
R^{e}, R^{f} und R^{g} jeweils unabhängig Wasserstoff, -C₁₋₆-Alkyl, -C₁₋₆-Alkoxy, -C₂₋₆-Alkenyl, -C₂₋₆-Alkinyl, Cycloalkyl, Heterocyclyl, Aryl oder Heteroaryl sind, wobei das -C₁₋₆-Alkyl, -C₂₋₆₋Alkenyl, -C₂₋₆-Alkinyl, Cycloalkyl, Heterocyclyl, Aryl und Heteroaryl jeweils unabhängig gegebenenfalls mit einem bis drei Substituenten substituiert sind, die aus Halogen, Hydroxy, Cyano, -C₁₋₆-Alkoxy, -C₃₋₆-Cycloalkyl, das unsubstituiert oder mit Halogen, Hydroxy oder -C₁₋₆-Alkoxy substituiert ist, und Heterocyclyl ausgewählt sind;
R⁵ Wasserstoff oder C₁₋₆-Alkyl ist;
Cy¹ 6- bis 12-gliedriges Aryl oder 5- bis 14-gliedriges Heteroaryl oder 5- bis 14-gliedriges Heterocyclyl ist, die jeweils unsubstituiert oder mit zumindest einem Substituenten Rⁱ substituiert sind,
worin Rⁱ jeweils unabhängig Halogen, Cyano, -C₁₋₆-Alkyl, -C₂₋₆-Alkenyl, -C₂₋₆-Alkinyl, -C₃₋₈-Cycloalkyl, Heterocyclyl, Aryl, Heteroaryl, Oxo, -CN, -NO₂, -OR^{j}, -SO₂Rⁱ, -CORⁱ, -CO₂R^{k}, -CONR^{j}R^{k}, -C(=NR^{j})NR^{k}R^{l}, -NR^{j}R^{k}, -NR^{j}COR^{k}, -NR^{j}CONR^{k}R^{l}, -NRⁱCO₂R^{k}, -NR^{j}SONR^{k}R^{l}, -NR^{j}SO₂NR^{k}R^{l} oder -NR^{j}SO₂R^{k} ist,
wobei das -C₁₋₆-Alkyl, -C₂₋₆-Alkenyl, -C₂₋₆-Alkinyl, -C₃₋₈-Cycloalkyl, Heterocyclyl, Aryl und Heteroaryl jeweils unsubstituiert oder mit Halogen, -OR^{m}, -C(O)R^{m}, -NR^{m}Rⁿ, -C₁₋₆-Alkyl, C₁₋₆-Alkoxy-, -C₁₋₆-Alkoxy-C₁₋₆-alkyl oder Oxo substituiert sind,
worin R^{j}, R^{k}, R^{l}, R^{m}, Rⁿ jeweils unabhängig Wasserstoff, -C₁₋₆-Alkyl, mit C₁₋₆-Alkoxy substituiertes -C₁₋₆-Alkyl, -C₂₋₆-Alkenyl, -C₂₋₆-Alkinyl, Cycloalkyl, Heterocyclyl, Aryl oder Heteroaryl sind;
oder (R¹ und R²) oder (R² und R³) oder (R³ und R⁴) zusammen mit den Atomen, an die sie gebunden sind, ein kondensiertes Ringsystem bilden, wobei das kondensierte Ringsystem 0 bis 4 Heteroatome, die aus Sauerstoff, Stickstoff und Schwefel ausgewählt sind, als Ringelement(e) umfasst und jeweils unabhängig gegebenenfalls mit Halogen, -C₁₋₆-Alkyl, -C₁₋₆-Alkoxy, Halogen-C₁₋₆-alkyl, Halogen-C₁₋₆-alkoxy oder -C₃₋₆-Cycloalkyl substituiert sind;
wobei jegliches Alkyl oder Alkoxy gegebenenfalls an Deuterium angereichert ist.

3. Verbindung nach Anspruch 1 oder Anspruch 2, worin R¹ -C₁₋₃-Alkyl, -NR^{c}R^{d} oder -C₃₋₆-Cycloalkyl, vorzugsweise -NH₂, Methyl, Ethyl, Propyl, Isopropyl, Cyclopropyl oder Cyclopentyl, ist.

4. Verbindung nach Anspruch 1 oder Anspruch 2, worin R² und R⁴ jeweils unabhängig Wasserstoff, Halogen, -C₁₋₆-Alkyl oder -C₁₋₆-Alkoxy, vorzugsweise Wasserstoff, Fluor, Methyl, Methoxy, Ethoxy oder Isopropoxy, sind.

5. Verbindung nach einem der Ansprüche 1 bis 4, worin R³ Folgendes ist:
Wasserstoff;
Cyano;
Halogen;
-C₁₋₄-Alkyl, das unsubstituiert oder mit zumindest einem Substituenten substituiert ist, der jeweils unabhängig aus Halogen, einem 3- bis 6-gliedrigen Heterocyclyl und -OR^{h} ausgewählt ist, wobei
das 3- bis 6-gliedrige Heterocyclyl ein oder zwei Heteroatome, die aus Sauerstoff (O), Stickstoff (N) und Schwefel (S) ausgewählt sind, als Ringelement(e) umfasst und unsubstituiert oder mit zumindest einem Substituenten substituiert ist, der jeweils unabhängig aus Cyano, Oxo, Halogen, Hydroxy, -NR^{m}Rⁿ, -C₁₋₆-Alkyl, -C₁₋₆-Alkoxy und -C(O)NR^{m}Rⁿ ausgewählt ist, wobei das -C₁₋₆-Alkyl oder -C₁₋₆-Alkoxy mit zumindest einem Substituenten substituiert ist, der jeweils unabhängig aus Cyano, Halogen, Hydroxy, -NH₂, -C₁₋₆-Alkyl und C₁₋₆-Alkoxy ausgewählt ist, und
R^{h} Wasserstoff, Alkyl oder Heterocyclyl (vorzugsweise 3- bis 6-gliedriges Heterocyclyl, z. B. Tetrahydrofuranyl oder Thiazolidinyl) ist;
-C₃₋₆-Cycloalkyl, das unsubstituiert oder mit zumindest einem Substituenten substituiert ist, der jeweils unabhängig aus Cyano, Oxo, Halogen, -NR^{m}Rⁿ, Hydroxy, -C₁₋₆-Alkyl, -C₁₋₆-Alkoxy und -C(O)NR^{m}Rⁿ ausgewählt ist, wobei das -C₁₋₆-Alkyl oder C₁₋₆-Alkoxy mit zumindest einem Substituenten substituiert ist, der jeweils unabhängig aus Cyano, Halogen, Hydroxy, -NH₂, -C₁₋₆-Alkyl und C₁₋₆-Alkoxy ausgewählt ist;
Heterocyclyl, vorzugsweise 4- bis 6-gliedriges, monozyklisches gesättigtes Heterocyclyl, gesättigtes Monospiroheterocyclyl, gesättigtes, bizyklisches kondensiertes Heterocyclyl oder gesättigtes verbrücktes Heterocyclyl, das ein oder zwei Heteroatome, die aus Sauerstoff (O), Stickstoff (N) und Schwefel (S) ausgewählt sind, als Ringelement(e) umfasst, noch bevorzugter Morpholinyl, Tetrahydrofuranyl, Tetrahydropyranyl, Pyrrolidinyl, 1,4-Dioxanyl, Piperidinyl oder Azetidinyl, die unsubstituiert oder mit zumindest einem Substituenten substituiert sind, der jeweils unabhängig aus Cyano, Oxo, Halogen, Hydroxy, -C₁₋₆-Alkyl, Alkoxy, -NR^{m}Rⁿ und -C(O)NR^{m}Rⁿ ausgewählt ist, wobei das -C₁₋₆-Alkyl oder -C₁₋₆-Alkoxy mit zumindest einem Substituenten substituiert ist, der jeweils unabhängig aus Cyano, Halogen, Hydroxy, -NH₂, -C₁₋₆-Alkyl und -C₁₋₆-Alkoxy ausgewählt ist;
-OR^{e}, worin R^{e} -C₁₋₆-Alkyl, -C₃₋₆-Cycloalkyl, 3- bis 6-gliedriges Heterocyclyl (vorzugsweise 4- bis 6-gliedriges, monozyklisches gesättigtes Heterocyclyl, das ein Sauerstoff-Heteroatom als Ringelement umfasst) oder Aryl ist, wobei
i) das -C₁₋₆-Alkyl unsubstituiert oder mit Cyano, Oxo, Halogen, Hydroxy, -NR^{m}Rⁿ, -C₁₋₆-Alkoxy, -C₃₋₆-Cycloalkyl, das unsubstituiert oder mit Cyano, Oxo, Halogen, Hydroxy, -NR^{m}Rⁿ, -C₁₋₆-Alkyl, -C₁₋₆-Alkoxy oder -C(O)NR^{m}Rⁿ substituiert ist, oder 4- bis 6-gliedrigem Heterocyclyl substituiert ist, das unsubstituiert oder mit Cyano, Halogen, Hydroxy, -C₁₋₆-Alkyl oder -C₁₋₆-Alkoxy substituiert ist, und
ii) das -C₃₋₆-Cycloalkyl oder 3- bis 6-gliedrige Heterocyclyl unsubstituiert oder mit Cyano, Oxo, Halogen, Hydroxy, -NR^{m}Rⁿ, -C₁₋₆-Alkyl, -C₁₋₆-Alkoxy oder -C(O)NR^{m}Rⁿ substituiert ist, wobei das -C₁₋₆-Alkyl oder -C₁₋₆-Alkoxy mit zumindest einem Substituenten substituiert ist, der jeweils unabhängig aus Cyano, Halogen, Hydroxy, -NH₂, -C₁₋₆-Alkyl und C₁₋₆-Alkoxy ausgewählt ist;
-C₅₋₁₀-Aryl; oder
Heteroaryl, vorzugsweise 5- bis 6-gliedriges Heteroaryl, das ein Sauerstoff- (O-), Stickstoff- (N-) oder Schwefel- (S-) Heteroatom als Ringelement umfasst und das unsubstituiert oder mit zumindest einem Substituenten substituiert ist, der jeweils unabhängig aus Cyano, Oxo, Halogen, Hydroxy, -NR^{m}Rⁿ, -C₁₋₆-Alkyl, -C₁₋₆-Alkoxy und -C(O)NR^{m}Rⁿ ausgewählt ist, wobei das -C₁₋₆-Alkyl oder C₁₋₆-Alkoxy mit zumindest einem Substituenten substituiert ist, der jeweils unabhängig aus Cyano, Halogen, Hydroxy, -NH₂, -C₁₋₆-Alkyl und C₁₋₆-Alkoxy ausgewählt ist;
und worin R^{m} und Rⁿ jeweils unabhängig aus Wasserstoff oder C₁₋₃-Alkyl ausgewählt sind.

6. Verbindung nach Anspruch 5, worin R³ Folgendes ist:
Wasserstoff;
Methyl, 1-Methoxyethyl, 2-Hydroxypropan-2-yl, 1-Methoxyethyl oder (2,4-Dioxothia-zolidin-3-yl)methyl;
Isopropoxy, Methoxy-d3, Methoxy, Ethoxy, Difluormethoxy, 2-Methoxyethoxy, 2-Methoxy-2-methylpropoxy, 2-Hydroxy-2-methylpropoxy, Cyclopropylmethoxy, (1,4-Dioxan-2-yl)methoxy, (4-Hydroxycyclohexyl)oxy, (cis-4-Hydroxycyclohexyl)oxy, (trans-4-Hydroxy-cyclohexyl)oxy, (4-Methoxycyclohexyl)oxy, (cis-4-Methoxycyclohexyl)oxy, (trans-4-Methoxy-cyclohexyl)oxy oder (3-Methyloxetan-3-yl)methoxy;
Cyano
3-Methoxycyclobutyl, (trans)-3-Methoxycyclobutyl, (cis)-3-Methoxycyclobutyl, 2,2-Dichlorcyclopropyl oder 1-Cyanocyclopropyl;
Morpholino, 3-Methylmorpholino, 3(R)-Methylmorpholino, 3(S)-Methylmorpholino, 3,3-Dimethylmorpholino;
Tetrahydro-2H-pyran-4-yl, Tetrahydro-2H-pyran-3-yl, (R)-Tetrahydro-2H-pyran-3-yl, (S)-Tetrahydro-2H-pyran-3-yl, 2,2,6,6-Tetramethyltetrahydro-2H-pyran-4-yl;
3-Methoxypyrrolidin-1-yl, 3(R)-Methoxypyrrolidin-1-yl, 3(S)-Methoxypyrrolidin-1-yl, 3-Hydroxy-3-methylpyrrolidin-1-yl, 3-(2-Hydroxyethoxy)pyrrolidin-1-yl, 3-(Trifluormethoxy)-pyrrolidin-1-yl, 3(R)-(Trifluormethoxy)pyrrolidin-1-yl, 3(S)-(Trifluormethoxy)pyrrolidin-1-yl, 2-(Aminocarbonyl)pyrrolidin-1-yl, 2(R)-(Aminocarbonyl)pyrrolidin-1-yl, 2(S)-(Aminocarbonyl)-pyrrolidin-1-yl, 3-(Methoxymethyl)pyrrolidin-1-yl, 3(R)-(Methoxymethyl)pyrrolidin-1-yl, 3(S)-(Methoxymethyl)pyrrolidin-1-yl, 3-Cyano-4-hydroxypyrrolidin-1-yl, cis-3-Cyano-4-hydroxypyrrolidin-1-yl, trans-3-Cyano-4-hydroxypyrrolidin-1-yl, 3-Cyano-4-methoxypyrrolidin-1-yl, cis-3-Cyano-4-methoxypyrrolidin-1-yl, trans-3-Cyano-4-methoxypyrrolidin-1-yl, 2-(Methoxymethyl)pyrrolidin-1-yl, 2(R)-(Methoxymethyl)pyrrolidin-1-yl, 2(S)-(Methoxymethyl)-pyrrolidin-1-yl, 3-Methylpyrrolidin-1-yl, 3(R)-Methylpyrrolidin-1-yl, 3(S)-Methylpyrrolidin-1-yl, Pyrrolidin-1-yl, 3-(Cyanomethoxy)pyrrolidin-1-yl;
5-Azaspiro[2.4]heptan-5-yl;
Tetrahydrofuran-3-yl;
3-Methoxyazetidin-1-yl, 3-Hydroxy-3-methylazetidin-1-yl;
1,4-Dioxan-2-yl;
4-Aminotetrahydro-2H-pyran-4-yl, 4-(Aminomethyl)tetrahydro-2H-pyran-4-yl;
4-Methoxypiperidin-1-yl, 4-Hydroxy-4-methylpiperidin-1-yl, 1-(2,2,2-Trifluorethyl)-piperidin-4-yl, 3-Methoxypiperidin-1-yl, 3(R)-Methoxypiperidin-1-yl, 3(S)-Methoxypiperidin-1-yl, 3-Ethoxypiperidin-1-yl, 3(R)-Ethoxypiperidin-1-yl, 3(S)-Ethoxypiperidin-1-yl;
3-Cyano-2-azabicyclo[3.1.0]hexan-2-yl, (3R)-3-Cyano-2-azabicyclo[3.1.0]hexan-2-yl, (3S)-3-Cyano-2-azabicyclo[3.1.0]hexan-2-yl, 3-Azabicyclo[3.1.0]hexan-3-yl; 4-Methylpyridin-3-yl, 5-Methylpyridazin-4-yl, 5-Methoxypyridazin-4-yl, 3,5-Dimethylisoxazol-4-yl, 4-Methoxy-pyridin-3-yl, 4-(2-Hydroxypropan-2-yl)pyridin-3-yl, 6-Cyanopyridin-3-yl, 4-Cyanopyridin-3-yl, 2-Cyanopyridin-3-yl, 3-Methylpyrazin-2-yl, 5-Cyanopyridazin-4-yl, 5-Fluorpyridazin-4-yl, 4-Fluorpyridin-3-yl, 4-Isopropylpyridin-3-yl, 4-(1-Hydroxyethyl)pyridin-3-yl, 4-(1-Methoxyethyl)pyridin-3-yl, Pyridin-2-yl oder Thiazol-4-yl.

7. Verbindung nach Anspruch 1 oder Anspruch 2, worin (R¹ und R²) oder (R² und R³) oder (R³ und R⁴) zusammen mit den Atomen, an die sie gebunden sind, ein kondensiertes 5-bis 7-gliedriges Ringsystem bilden, wobei das kondensierte Ringsystem 0 bis 2 Sauerstoff-Heteroatome als Ringelement(e) umfasst und gegebenenfalls jeweils unabhängig mit Halogen, -C₁₋₆-Alkyl, -C₁₋₆-Alkoxy, -Halogen-C₁₋₆-alkyl, -Halogen-C₁₋₆-alkoxy oder -C₃₋₆-Cycloalkyl substituiert ist.

8. Verbindung nach Anspruch 1 oder Anspruch 2, worin R¹ und R² zusammen mit den Atomen, an die sie gebunden sind, ein kondensiertes Ringsystem bilden, das aus 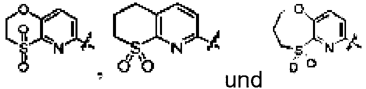 ausgewählt ist; oder R² und R³ zusammen mit den Atomen, an die sie gebunden sind, ein kondensiertes Ringsystem 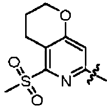 bilden; R³ und R⁴ zusammen mit den Atomen, an die sie gebunden sind, ein kondensiertes Ringsystem bilden, das aus 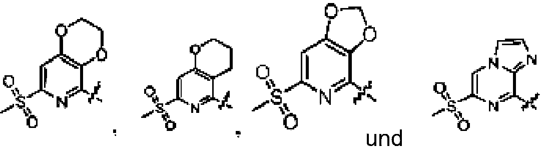 ausgewählt ist; und wobei jedes der kondensierten Ringsysteme gegebenenfalls jeweils unabhängig mit Halogen, -C₁₋₆-Alkyl, -C₁₋₆-Alkoxy, -Halogen-C₁₋₆-alkyl, -Halogen-C₁₋₆-alkoxy oder -C₃₋₆-Cycloalkyl substituiert ist.

9. Verbindung nach einem der Ansprüche 1 bis 8, worin L eine Bindung ist.

10. Verbindung nach einem der Ansprüche 1 bis 8, worin Cy¹ Folgendes ist:
a)ein 5- bis 7-gliedriges monozyklisches Heterocyclyl oder Heteroaryl, das 1, 2, 3 oder 4 Heteroatome, die aus Sauerstoff, Stickstoff und Schwefel ausgewählt sind, als Ringelement(e) umfasst, oder
b) ein 7- bis 14-gliedriges, bizyklisches oder trizyklisches Heterocyclyl oder Heteroaryl, das 1, 2 oder 3 Heteroatome, die aus Sauerstoff, Stickstoff und Schwefel ausgewählt sind, als Ringelement(e) umfasst,
die jeweils unsubstituiert oder mit zumindest einem Substituenten Rⁱ substituiert sind.

11. Verbindung nach Anspruch 10, worin Cy¹ Folgendes ist:
a) das 5- bis 7-gliedrige monozyklische Heteroaryl, das 1, 2, 3 oder 4 Heteroatome, die aus Sauerstoff (O), Stickstoff (N) und Schwefel (S) ausgewählt sind, als Ringelement(e) umfasst, vorzugsweise Pyrazolyl, Triazolyl, Imidazolyl, Thiazolyl, Oxazolyl, Furanyl, Pyridinyl, Pyridazinyl, Pyrazinyl oder Pyrimidinyl, wobei das monozyklische Heteroaryl unsubstituiert oder mit einem oder zwei Substituenten substituiert ist, die aus den folgenden ausgewählt sind:
i) Halogen;
ii) Cyano;
iii) -C₁₋₆-Alkyl, das unsubstituiert oder mit Halogen, Hydroxy, -C₁₋₆-Alkoxy, -C(O)R^{m} (worin R^{m} vorzugsweise Morpholinyl ist) oder -NR^{m}Rⁿ substituiert ist;
iv) Heterocyclyl, das unsubstituiert oder mit Halogen, C₁₋₆-Alkyl-, -C₁₋₆-Alkyl, das mit -C₁₋₆-Alkoxy substituiert ist, oder Oxo substituiert ist; wobei das Heterocyclyl vorzugsweise aus Tetrahydrofuranyl (vorzugsweise Tetrahydrofuran-3-yl), Morpholinyl (vorzugsweise Morpholino), 2-Oxa-5-azabicyclo[2.2.1]heptanyl (vorzugsweise 2-Oxa-5-azabicyclo[2.2.1]-heptan-2-yl), 8-Oxa-3-azabicyclo[3.2.1]octanyl (vorzugsweise 8-Oxa-3-azabicyclo[3.2.1]-octan-8-yl) und Isoindolinyl (vorzugsweise Isoindolin-2-yl) ausgewählt ist, die jeweils gegebenenfalls mit Methyl, Ethyl, Propyl, Isopropyl, Isobutyl, tert-Butyl, n-Butyl, Methoxymethyl, Methoxyethyl, Ethoxymethyl, Ethoxyethyl oder Oxo substituiert sind;
v) -C₃₋₆-Cycloalkyl, das unsubstituiert oder mit Halogen, Oxo, -C₁₋₆-Alkyl, C₁₋₆-Alkoxy- oder mit -C₁₋₆-Alkoxy substituiertem -C₁₋₆-Alkyl substituiert ist; oder
vi) -OR^{j}, worin R^{j} -C₁₋₆-Alkyl, mit -C₁₋₆-Alkoxy substituiertes -C₁₋₆-Alkyl oder Heterocyclyl ist;
vii) Oxo;
b) das 7- bis 14-gliedrige, bizyklische oder trizyklische Heteroaryl, das 1, 2 oder 3 Heteroatom(e), die aus Sauerstoff, Stickstoff und Schwefel ausgewählt sind, als Ringelement(e) umfasst, vorzugsweise Benzoimidazolyl, Imidazopyrimidinyl, Pyrazolopyrazinyl, Pyrazolopyrimidinyl, Benzothiophenyl, Benzothiazolyl, Benzoisoxazolyl, Benzooxazolyl, Benzoisothiazolyl, Imidazopyridazinyl, Imidazopyridazinyl, Dihydro-4H-furo[3,2-c]pyranyl, 6,7-Dihydro-4H-thieno[3,2-c]pyranyl,2,3-Dihydropyrazolo[5,1-b]oxazolyl, 4,5,6,7-Tetrahydrofuro[3,2-c]-pyridinyl, 1,3a,4,6,7,7a-Hexahydropyrano[4,3-c]pyrazolyl, 4,5,6,7-Tetrahydropyrazolo[1,5-a]-pyrazinyl, 4,5,6,7-Tetrahydropyrazolo[1,5-a]pyrimidinyl, 4,5,6,7-Tetrahydropyrazolo[1,5-a]-pyrazinyl, 4,5,6,7-Tetrahydrothiazolo[5,4-c]pyridinyl, 4,5,6,7-Tetrahydropyrazolo[1,5-a]-pyrazinyl, 6,7-Dihydro-4H-pyrazolo[5,1-c][1,4]oxazinyl, 2,3-Dihydropyrazolo[5,1-b]oxazolyl, 2,3-Dihydropyrazolo[5,1-b]oxazolyl, 1,3a,4,6,7,7a-Hexahydropyrano[4,3-c]pyrazolyl, 6,7-Di-hydro-4H-pyrano[4,3-d]thiazolyl, [1,3]Dioxolo[4,5-c]pyridinyl, 2,3-Dihydro-[1,4]dioxino[2,3-c]-pyridinyl, 3,4-Dihydro-2H-pyrido[3,2-b][1,4]oxazinyl, 2,3-Dihydro-[1,4]dioxino[2,3-b]pyridinyl, 2,3-Dihydro-[1,4]dioxino[2,3-b]pyridin-6-yl, 3,4-Dihydro-2H-pyrido[4,3-b][1,4]oxazinyl oder 2H-Pyrido[3,2-b][1,4]oxazin-4(3H)-yl, die jeweils gegebenenfalls mit Halogen, -C₁₋₆-Alkyl, -NH₂ oder -C(O)R^{m} substituiert sind, worin R^{m} C₁₋₆-Alkyl ist; wobei jegliches Alkyl gegebenenfalls an Deuterium angereichert sind.

12. Verbindung nach Anspruch 10, worin Cy¹ Folgendes ist:
a. 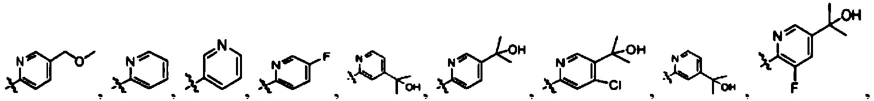 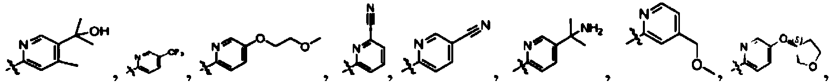 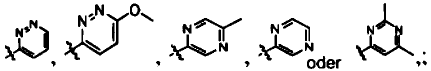
b. 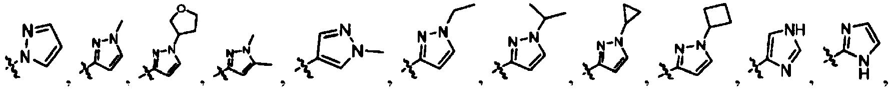 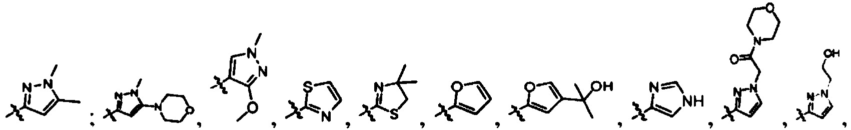 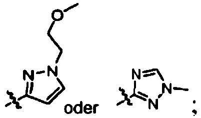
c. 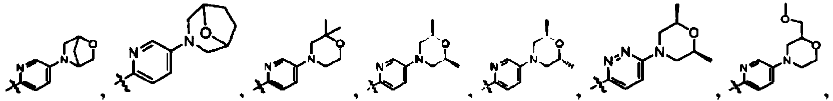 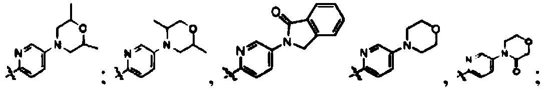
d. 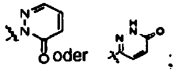
c. 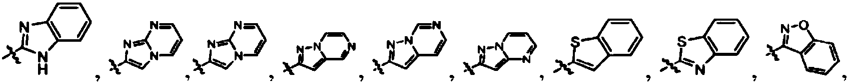 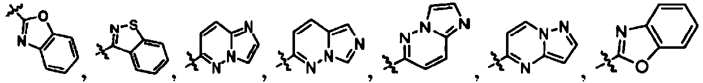
f. 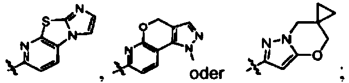
g. 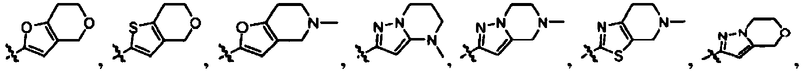 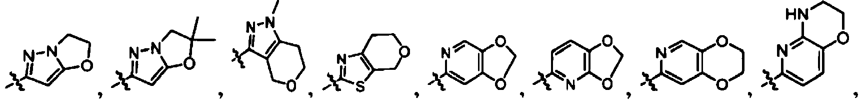 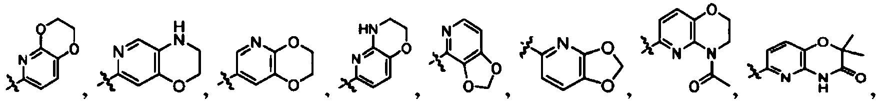 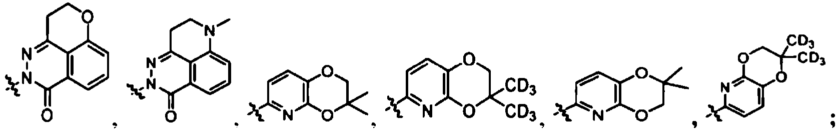
h.

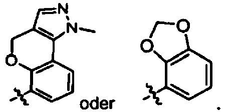

13. Verbindung nach einem der Ansprüche 1 bis 12, wobei die Verbindung aus den folgenden ausgewählt ist:
N-(4-((3-(Methylsulfonyl)phenyl)amino)-5-(pyridazin-3-yl)pyridin-2-yl)acetamid;
N-[4-[(3-Methansulfonylphenyl)amino]-5-(oxolan-2-yl)pyridin-2-yl]acetamid;
N-(5-(1H-Imidazol-4-yl)-4-((6-(methylsulfonyl)pyridin-2-yl)amino)pyridin-2-yl)acetamid;
N-(5-(5-Methylpyrazin-2-yl)-4-((3-(methylsulfonyl)phenyl)amino)pyridin-2-yl)acetamid;
N-(4-((3-(Methylsulfonyl)phenyl)amino)-5-(pyrazin-2-yl)pyridin-2-yl)acetamid;
N-(5-(2,6-Dimethylpyrimidin-4-yl)-4-((3-(methylsulfonyl)phenyl)amino)pyridin-2-yl)-acetamid;
N-(4-((3-(Methylsulfonyl)phenyl)amino)-5-(2-oxopyrrolidin-1-yl)pyridin-2-yl)acetamid;
N-(5-(1-Methyl-1H-pyrazol-4-yl)-4-((6-(methylsulfonyl)pyridin-2-yl)amino)pyridin-2-yl)-acetamid;
N-(5-(Furan-2-yl)-4-((4-methyl-6-(methylsulfonyl)pyridin-2-yl)amino)pyridin-2-yl)-acetamid;
N-(5-(1-Methyl-1H-1,2,4-triazol-3-yl)-4-((3-(methylsulfonyl)phenyl)amino)pyridin-2-yl)-acetamid;
N-(4-((3-Methoxy-6-(methylsulfonyl)pyridin-2-yl)amino)-5-(1-methyl-1H-pyrazol-4-yl)-pyridin-2-yl)acetamid;
N-(4-((4-Isopropoxy-6-(methylsulfonyl)pyridin-2-yl)amino)-5-(1H-pyrazol-1-yl)pyridin-2-yl)acetamid;
N-(4-((4-Methyl-6-(methylsulfonyl)pyridin-2-yl)amino)-5-(1-(2-morpholino-2-oxoethyl)-1H-pyrazol-3-yl)pyridin-2-yl)acetamid;
N-(5-(1-(2-Hydroxyethyl)-1H-pyrazol-3-yl)-4-((4-isopropoxy-6-(methylsulfonyl)pyridin-2-yl)amino)pyridin-2-yl)acetamid;
N-(5-(1-(2-Methoxyethyl)-1H-pyrazol-3-yl)-4-((4-methyl-6-(methylsulfonyl)pyridin-2-yl)amino)pyridin-2-yl)acetamid;
N-(6-Cyano-4'-((4-isopropoxy-6-(methylsulfonyl)pyridin-2-yl)amino)-[2,3'-bipyridin]-6'-yl)acetamid;
N-(5-Cyano-4'-((4-isopropoxy-6-(methylsulfonyl)pyridin-2-yl)amino)-[2,3'-bipyridin]-6'-yl)acetamid;
N-(5-(1H-Imidazol-1-yl)-4-((4-isopropoxy-6-(methylsulfonyl)pyridin-2-yl)amino)pyridin-2-yl)acetamid;
N-(5-(1-Cyclobutyl-1H-pyrazol-3-yl)-4-((4-isopropoxy-6-(methylsulfonyl)pyridin-2-yl)-amino)pyridin-2-yl)acetamid;
N-(5-(2-Aminopropan-2-yl)-4'-((4-methyl-6-(methylsulfonyl)pyridin-2-yl)amino)-[2,3'-bipyridin]-6'-yl)acetamid;
N-(5-(1H-Imidazol-4-yl)-4-((4-(2-methoxyethoxy)-6-(methylsulfonyl)pyridin-2-yl)-amino)pyridin-2-yl)acetamid;
N-(5-(1H-Imidazol-4-yl)-4-((4-methyl-6-(methylsulfonyl)pyridin-2-yl)amino)pyridin-2-yl)acetamid;
N-(5-(1H-Imidazol-4-yl)-4-((4-isopropoxy-6-(methylsulfonyl)pyridin-2-yl)amino)pyridin-2-yl)acetamid:
N-(4-((6-(Methylsulfonyl)pyridin-2-yl)amino)-3-(pyridazin-3-yl)pyridin-2-yl)acetamid;
N-(4-((4-(Cyclopropylmethoxy)-6-(methylsulfonyl)pyridin-2-yl)amino)-5-(6-methoxy-pyridazin-3-yl)pyridin-2-yl)acetamid;
N-(4-((4-Isopropoxy-6-(methylsulfonyl)pyridin-2-yl)amino)-5-(6-methoxypyridazin-3-yl)pyridin-2-yl)acetamid;
N-(4-((4-((2S,6R)-2,6-Dimethylmorpholino)-6-(methylsulfonyl)pyridin-2-yl)amino)-3-(6-methoxypyridazin-3-yl)pyridin-2-yl)acetamid;
N-(5-(6-Methoxypyridazin-3-yl)-4-((6-(methylsulfonyl)-[1,3]dioxolo[4,5-c]pyridin-4-yl)-amino)pyridin-2-yl)acetamid;
N-(5-(6-Isopropoxypyridazin-3-yl)-4-((4-methyl-6-(methylsulfonyl)pyridin-2-yl)amino)-pyridin-2-yl)acetamid;
N-(5-(6-Isopropoxypyridazin-3-yl)-4-((4-methoxy-6-(methylsulfonyl)pyridin-2-yl)-amino)pyridin-2-yl)acetamid;
N-(5-(6-Isopropoxypyridazin-3-yl)-4-(4-(methoxy-d3)-6-(methylsulfonyl)pyridin-2-yl)-amino)pyridin-2-yl)acetamid;
N-(4'-((3-(Methylsulfonyl)phenyl)amino)-[2,3'-bipyridin]-6'-yl)acetamid;
N-(4-((6-(Methylsulfonyl)pyridin-2-yl)amino)-[3,3'-bipyridin]-6-yl)acetamid;
N-(4'-(Phenylamino)-[2,3'-bipyridin]-6'-yl)acetamid;
N-(4'-((6-Sulfamoylpyridin-2-yl)amino)-[2,3'-bipyridin]-6'-yl)acetamid;
N-(4'-((6-(Methylsulfonyl)pyridin-2-yl)amino)-[2,3'-bipyridin]-6'-yl)acetamid;
N-(4'-((4-(2-Methoxyethoxy)-6-(methylsulfonyl)pyridin-2-yl)amino)-[2,3'-bipyridin]-6'-yl)acetamid;
N-(4'-((4-(2-Hydroxyethoxy)-6-(methylsulfonyl)pyridin-2-yl)amino)-[2,3'-bipyridin]-6'-yl)acetamid:
N-(4-(Methoxymethyl)-4'-((6-(methylsulfonyl)pyridin-2-yl)amino)-[2,3'-bipyridin]-6'-yl)-acetamid;
N-(4-(2-Hydroxypropan-2-yl)-4'-((3-(methylsulfonyl)phenyl)amino)-[2,3'-bipyridin]-6'-yl)acetamid;
N-(4-(2-Hydroxypropan-2-yl)-4'-((4-methyl-6-(methylsulfonyl)pyridin-2-yl)amino)-[2,3'-bipyridin]-6'-yl)acetamid;
N-(4'-((3-(Methylsulfonyl)phenyl)amino)-5-morpholino-[2,3'-bipyridin]-6'-yl)acetamid;
N-(5-(2,2-Dimethylmorpholino)-4'-((6-(methylsulfonyl)pyridin-2-yl)amino)-[2,3'-bipyridin]-6'-yl)acetamid;
N-(4'-((3-Methoxyphenyl)amino)-[2,3'-bipyridin]-6'-yl)acetamid;
N-(4'-((3-(Methylsulfonyl)pyridin-2-yl)amino)-[2,3'-bipyridin]-6'-yl)acetamid;
N-(4'-((2-(Methylsulfonyl)phenyl)amino)-[2,3'-bipyridin]-6'-yl)acetamid;
N-(5-(2-Oxa-5-azabicyclo[2.2.1]heptan-5-yl)-4'-((4-isopropoxy-6-(methylsulfonyl)-pyridin-2-yl)amino)-[2,3'-bipyridin]-6'-yl)acetamid;
N-(4-Chlor-5-(2-hydroxypropan-2-yl)-4'-((6-(methylsulfonyl)pyridin-2-yl)amino)-[2,3'-bipyridin]-6'-yl)acetamid;
N-(4'-((4-Isopropoxy-6-(methylsulfonyl)pyridin-2-yl)amino)-5-(2-(methoxymethyl)-morpholino)-[2,3'-bipyridin]-6'-yl)acetamid;
N-(4'-(3-(Methylsulfonyl)phenyl)-[2,3'-bipyridin]-4',6'-diamin;
N-(4'-((3-(Methylsulfonyl)phenyl)amino)-[2,3'-bipyridin]-6'-yl)cyclopropancarboxamid;
(S)-N-(4'-((4-Methyl-6-(methylsulfonyl)pyridin-2-yl)amino)-5-((tetrahydrofuran-3-yl)-oxy)-[2,3'-bipyridin]-6'-yl)acetamid:
N-(4'-((4-(2-Hydroxyethoxy)-6-(methylsulfonyl)pyridin-2-yl)amino)-5-((tetrahydrofuran-3-yl)oxv)-[2,3'-bipyridin]-6'-yl)acetamid;
N-(4'-((6-(Methylsulfonyl)pyridin-2-yl)amino)-5-((tetrahydrofuran-3-yl)oxy)-[2,3'-bipyridin]-6'-yl)acetamid;
N-(5-(8-Oxa-3-azabicyclo|3.2.1]octan-3-yl)-4'-((4-methyl-6-(methylsulfonyl)pyridin-2-yl)amino)-[2,3'-bipyridin]-6'-yl)acetamid;
N-(5-(2,5-Dimethylmorpholino)-4'-((4-methyl-6-(methylsulfonyl)pyridin-2-yl)amino)-[2,3'-bipyridin]-6'-yl)acetamid;
N-(5-(cis-2,6-Dimethylmorpholino)-4'-((4-methyl-6-(methylsulfonyl)pyridin-2-yl)amino)-[2,3'-bipyridin]-6'-yl)acetamid;
N-(5-((cis)-2,6-Dimethylmorpholino)-4'-((6-(methylsulfonyl)pyridin-2-yl)amino)-[2,3'-bipyridin]-6'-yl)acetamid;
N-(5-((cis)-2,6-Dimethylmorpholino)-4'-((3-(methylsulfonyl)phenyl)amino)-[2,3'-bipyridin]-6'-yl)acetamid:
N-(5-((cis)-2,6-Dimethylmorpholino)-4'-((4-isopropoxy-6-(methylsulfonyl)pyridin-2-yl)-amino)-[2,3'-bipyridin]-6'-yl)acetamid;
N-(4'-((4-(Cyclopropylmethoxy)-6-(methylsulfonyl)pyridin-2-yl)amino)-5-((cis)-2,6-dimethylmorpholino)-[2,3'-bipyridin]-6'-yl)acetamid;
N-(4'-((3-(Cyclopropylmethoxy)-5-(methylsulfonyl)phenyl)amino)-5-((cis)-2,6-dimethylmorpholino)-[2,3'-bipyridin]-6'-yl)acetamid;
N-(5-((trans)-2,6-Dimethylmorpholino)-4'-((4-methyl-6-(methylsulfonyl)pyridin-2-yl)-amino)-[2,3'-bipyridin]-6'-yl)acetamid;
N-(5-(cis-2,6-Dimethylmorpholino)-4'-((7-(methylsulfonyl)-2,3-dihydro[1,4]dioxino-[2,3-c]pyridin-5-yl)amino)-[2,3'-bipyridin]-6'-yl)acetamid:
N-(5-((cis)-2,6-Dimethylmorpholino)-4'-((4-methoxy-6-(methylsulfonyl)pyridin-2-yl)-amino)-[2,3'-bipyridin]-6'-yl)acetamid;
N-(5-((cis)-2,6-Dimethylmorpholino)-4'-((4-ethoxy-6-(methylsulfonyl)pyridin-2-yl)-amino)-[2,3'-bipyridin]-6'-yl)acetamid;
N-(5-((cis)-2,6-Dimethylmorpholino)-4'-((4-(2-methoxyethoxy)-6-(methylsulfonyl)-pyridin-2-yl)amino)-[2,3'-bipyridin]-6'-yl)acetamid;
N-(5-((cis)-2,6-Dimethylmorpholino)-4'-((4-((R)-2-hydroxypropoxy)-6-(methylsulfonyl)-pyridin-2-yl)amino)-[2,3'-bipyridin]-6'-yl)acetamid;
N-(4'-((4-((R)-sec-Butoxy)-6-(methylsulfonyl)pyridin-2-yl)amino)-5-((cis)-2,6-dimethylmorpholino)-[2,3'-bipyridin]-6'-yl)acetamid;
N-(5-((cis)-2,6-Dimethylmorpholino)-4'-((3-methoxy-5-(methylsulfonyl)phenyl)amino)-[2,3'-bipyridin]-6'-yl)acetamid:
N-(5-((cis)-2,6-Dimethylmorpholino)-4'-((3-fluor-5-(methylsulfonyl)phenyl)amino)-[2,3'-bipyridin]-6'-yl)acetamid;
N-(4'-((3-Cyano-5-(methylsulfonyl)phenyl)amino)-5-((cis)-2,6-dimethylmorpholino)-[2,3'-bipyridin]-6'-yl)acetamid;
N-(5-(cis-2,6-Dimethylmorpholino)-4'-((4-methoxy-6-(methylsulfonyl)pyridin-2-yl)-amino)-[2,3'-bipyridin]-6'-yl)acetamid;
N-(5-((cis)-2,6-Dimethylmorpholino)-4'-((4-(methoxy-d3)-6-(methylsulfonyl)pyridin-2-yl)amino)-[2,3'-bipyridin]-6'-yl)acetamid;
N-(5-(cis-2,6-Dimethylmorpholino)-4'-((4-(1-methoxyethyl)-6-(methylsulfonyl)pyridin-2-yl)amino)-[2,3'-bipyridin]-6'-yl)acetamid;
N-(5-((cis)-2,6-Dimethylmorpholino)-4'-((4-((R oder S)-1-methoxyethyl)-6-(methylsulfonyl)pyridin-2-yl)amino)-[2,3'-bipyridin]-6'-yl)acetamid;
N-(5-((cis)-2,6-Dimethylmorpholino)-4'-((4-((R oder S)-1-methoxyethyl)-6-(methylsulfonyl)pyridin-2-yl)amino)-[2,3'-bipyridin]-6'-yl)acetamid;
N-(5-(cis-2,6-Dimethylmorpholino)-4'-((6-(methylsulfonyl)-4-(tetrahydro-2H-pyran-4-yl)pyridin-2-yl)amino)-[2,3'-bipyridin]-6'-yl)acetamid;
N-(5-(cis-2,6-Dimethylmorpholino)-4'-((6-(methylsulfonyl)pyrazin-2-yl)amino)-[2,3'-bipyridin]-6'-yl)acetamid;
N-(5-(cis-2,6-Dimethylmorpholino)-4'-((4-((S)-3-methylmorpholino)-6-(methylsulfonyl)-pyridin-2-yl)amino)-[2,3'-bipyridin]-6'-yl)acetamid;
N-(5-(cis-2,6-Dimethylmorpholino)-4'-((4-((R)-3-methylmorpholino)-6-(methylsulfonyl)-pyridin-2-yl)amino)-[2,3'-bipyridin]-6'-yl)acetamid;
N-(5-(cis-2,6-Dimethylmorpholino)-4'-((4-(3-methoxyazetidin-1-yl)-6-(methylsulfonyl)-pyridin-2-yl)amino)-[2,3'-bipyridin]-6'-yl)acetamid;
N-(5-(2-Methoxyethoxy)-4'-((6-(methylsulfonyl)pyridin-2-yl)amino)-[2,3'-bipyridin]-6'-yl)acetamid:
N-(5-(2-Methoxyethoxy)-4'-((3-(methylsulfonyl)phenyl)amino)-[2,3'-bipyridin]-6'-yl)-acetamid;
N-(5-(2-Methoxyethoxy)-4'-((3-(methylsulfonyl)-5-(trifluormethyl)phenyl)amino)-[2,3'-bipyridin]-6'-yl)acetamid;
N-(5-(2-Methoxyethoxy)-4'-((4-methyl-6-(methylsulfonyl)pyridin-2-yl)amino)-[2,3'-bipyridin]-6'-yl)acetamid;
N-(4'-((4-Methoxy-6-(methylsulfonyl)pyridin-2-yl)amino)-5-(2-methoxyethoxy)-[2,3'-bipyridin]-6'-yl)acetamid;
N-(4'-((4-Isopropoxy-6-(methylsulfonyl)pyridin-2-yl)amino)-5-(2-methoxyethoxy)-[2,3'-bipyridin]-6'-yl)acetamid;
(R)-N-(5-(2-Methoxyethoxy)-4'-((4-(2-methoxypropoxy)-6-(methylsulfonyl)pyridin-2-yl)amino)-[2,3'-bipyridin]-6'-yl)acetamid;
N-(5-(2-Methoxyethoxy)-4'-((4-(2-methoxyethoxy )-6-(methylsulfonyl)pyridin-2-yl)-amino)-[2,3'-bipyridin]-6'-yl)acetamid;
N-(4'-((4-Ethoxy-6-(methylsulfonyl)pyridin-2-yl)amino)-5-(2-methoxyethoxy)-[2,3'-bipyridin]-6'-yl)acetamid;
N-(4'-((3-(Cyclopropylmethoxy)-5-(methylsulfonyl)phenyl)amino)-5-(2-methoxyethoxy)-[2,3'-bipyridin]-6'-yl)acetamid;
N-(4'-((4-(Cyclopropylmethoxy)-6-(methylsulfonyl)pyridin-2-yl)amino)-5-(2-methoxyethoxy)-[2,3'-bipyridin]-6'-yl)acetamid;
N-(5-(Methoxymethyl)-4'-((3-(methylsulfonyl)phenyl)amino)-[2,3'-bipyridin]-6'-yl)-acetamid;
N-(5-(Methoxymethyl)-4'-((4-methyl-6-(methylsulfonyl)pyridin-2-yl)amino)-[2,3'-bipyridin]-6'-yl)acetamid;
(R)-N-(5-(Methoxymethyl)-4'-((4-(2-methoxypropoxy)-6-(methylsulfonyl)pyridin-2-yl)-amino)-[2,3'-bipyridin]-6'-yl)acetamid;
N-(4'-((4-Isopropoxy-6-(methylsulfonyl)pyridin-2-yl)amino)-5-(methoxymethyl)-[2,3'-bipyridin]-6'-yl)acetamid;
N-(4'-((4-(2-Methoxyethoxy)-6-(methylsulfonyl)pyridin-2-yl)amino)-3-(methoxymethyl)-[2,3'-bipyridin]-6'-yl)acetamid:
N-(4'-((4-(2-Hydroxyethoxy)-6-(methylsulfonyl)pyridin-2-yl)amino)-5-(methoxymethyl)-[2,3'-bipyridin]-6'-yl)acetamid;
N-(4'-((4-Methoxy-6-(methylsulfonyl)pyridin-2-yl)amino)-5-(methoxymethyl)-[2,3'-bipyridin]-6'-yl)acetamid;
N-(4'-((4-((1r,3r)-3-Hydroxycyclobutoxy)-6-(methylsulfonyl)pyridin-2-yl)amino)-5-(methoxymethyl)-[2,3'-bipyridin]-6'-yl)acetamid;
N-(5-(Methoxymethyl)-4'-((4-(methoxymethyl)-6-(methylsulfonyl)pyridin-2-yl)amino)-[2,3'-bipyridin]-6'-yl)acetamid;
N-(4'-((4-Methyl-6-(methylsulfonyl)pyridin-2-yl)amino)-5-(trifluormethyl)-[2,3'-bipyridin]-6'-yl)acetamid;
N-(4'-((3-(Methylsulfonyl)phenyl)amino)-5-(trifluormethyl)-[2,3'-bipyridin]-6'-yl)-acetamid;
N-(4'-((3-Methyl-5-(methylsulfonyl)phenyl)amino)-3-(trifluormethyl)-[2,3'-bipyridin]-6'-yl)acetamid;
N-(4'-((3-Cyano-5-(methylsulfonyl)phenyl)amino)-5-(trifluormethyl)-[2,3'-bipyridin]-6'-yl)acetamid;
N-(4'-((3-((trans)-3-Hydroxycyclobutoxy)-3-(methylsulfonyl)phenyl)amino)-5-(trifluormethyl)-[2,3'-bipyridin]-6'-yl)acetamid;
N-(4'-((3-(Cyclopropylmethoxy)-3-(methylsulfonyl)phenyl)amino)-5-(trifluormethyl)-[2,3'-bipyridin]-6'-yl)acetamid;
N-(4'-((3-(2-Hydroxyethoxy)-3-(methylsulfonyl)phenyl)amino)-5-(trifluormethyl)-[2,3'-bipyridin]-6'-yl)acetamid;
N-(4'-((3-(2-Methoxyethoxy)-3-(methylsulfonyl)phenyl)amino)-5-(trifluormethyl)-[2,3'-bipyridin]-6'-yl)acetamid;
N-(4'-((4-Isopropoxy-6-(methylsulfonyl)pyridin-2-yl)amino)-5-(trifluormethyl)-[2,3'-bipyridin]-6'-yl)acetamid;
N-(4'-((4-Ethoxy-6-(methylsulfonyl)pyridin-2-yl)amino)-5-(trifluormethyl)-[2,3'-bipyridin]-6'-yl)acetamid;
N-(4'-((4-(2-Methoxyethoxy)-6-(methylsulfonyl)pyridin-2-yl)amino)-5-(trifluormethyl)-[2,3'-bipyridin]-6'-yl)acetamidcarboxamid;
N-(4'-((4-(2-Hydroxyethoxy)-6-(methylsulfonyl)pyridin-2-yl)amino)-5-(trifluormethyl)-[2,3'-bipyridin]-6'-yl)acetamid;
N-(5-Fluor-4'-((3-(methylsulfonyl)-5-(trifluormethyl)phenyl)amino)-[2,3'-bipyridin]-6'-yl)acetamid:
N-(5-Fluor-4'-((3-isopropoxy-5-(methylsulfonyl)phenyl)amino)-[2,3'-bipyridin]-6'-yl)-acetamid;
N-(5-Fluor-4'-((3-((trans)-3-hydroxycyclobutoxy)-5-(methylsulfonyl)phenyl)amino)-[2,3'-bipyridin]-6'-yl)acetamid;
N-(5-Fluor-4'-((4-(2-methoxyethoxy)-6-(methylsulfonyl)pyridin-2-yl)amino)-[2,3'-bipyridin]-6'-yl)acetamid;
N-(5-Fluor-4'-((4-methyl-6-(methylsulfonyl)pyridin-2-yl)amino)-[2,3'-bipyridin]-6'-yl)-acetamid;
N-(5-Fluor-4'-((4-isopropoxy-6-(methylsulfonyl)pyridin-2-yl)amino)-[2,3'-bipyridin]-6'-yl)acetamid;
N-(4'-((4-Ethoxy-6-(methylsulfonyl)pyridin-2-yl)amino)-5-fluor-[2,3'-bipyridin]-6'-yl)-acetamid;
N-(4'-((4-Methoxy-6-(methylsulfonyl)pyridin-2-yl)amino)-3-(trifluormethyl)-[2,3'-bipyridin]-6'-yl)acetamid:
(S)-N-(4'-((4-(2-Hydroxypropoxy)-6-(methylsulfonyl)pyridin-2-yl)amino)-5-(trifluormethyl)-[2,3'-bipyridin]-6'-yl)acetamid;
N-(5-Fluor-4'-((4-methoxy-6-(methylsulfonyl)pyridin-2-yl)amino)-[2,3'-bipyridin]-6'-yl)-acetamid;
N-(4'-((3,4-Dimethoxy-6-(methylsulfonyl)pyridin-2-yl)amino)-5-fluor-[2,3'-bipyridin]-6'-yl)acetamid;
N-(5-Fluor-4'-((4-(methoxy-d3)-6-(methylsulfonyl)pyridin-2-yl)amino)-[2,3'-bipyridin]-6'-yl)acetamid;
N-(4'-((4-Cyclopropyl-6-(methylsulfonyl)pyridin-2-yl)amino)-5-fluor-[2,3'-bipyridin]-6'-yl)acetamid;
N-(4'-((3-(Methylsulfonyl)phenyl)amino)-5-(3-oxomorpholino)-[2,3'-bipyridin]-6'-yl)-acetamid;
N-(4'-((6-(Methylsulfonyl)pyridin-2-yl)amino)-5-(3-oxomorpholino)-[2,3'-bipyridin]-6'-yl)acetamid;
N-(4'-((4-(2-Hydroxyethoxy)-6-(methylsulfonyl)pyridin-2-yl)amino)-5-(3-oxo-morpholino)-[2,3'-bipyridin]-6'-yl)acetamid;
N-(4'-((4-(2-Methoxyethoxy)-6-(methylsulfonyl)pyridin-2-yl)amino)-5-(3-oxo-morpholino)-[2,3'-bipyridin]-6'-yl)acetamid;
N-(5-(2-Hydroxypropan-2-yl)-4'-((3-(methylsulfonyl)phenyl)amino)-[2,3'-bipyridin]-6'-yl)acetamid:
N-(5-(2-Hydroxypropan-2-yl)-4'-((4-isopropoxy-6-(methylsulfonyl)pyridin-2-yl)amino)-[2,3'-bipyridin]-6'-yl)acetamid;
N-(5-(2-Hydroxypropan-2-yl)-4'-((3-(methylsulfonyl)-5-(trifluormethyl)phenyl)amino)-[2,3'-bipyridin]-6'-yl)acetamid;
N-(5-(2-Hydroxypropan-2-yl)-4'-((3-methoxy-5-(methylsulfonyl)phenyl)amino)-[2,3'-bipyridin]-6'-yl)acetamid;
N-(5-(2-Hydroxypropan-2-yl)-4'-((6-(methylsulfonyl)pyridin-2-yl)amino)-[2,3'-bipyridin]-6'-yl)acetamid;
N-(5-(2-Hydroxypropan-2-yl)-4'-((4-(2-methoxyethoxy)-6-(methylsulfonyl)pyridin-2-yl)-amino)-[2,3'-bipyridin]-6'-yl)acetamid;
N-(5-(2-Hydroxypropan-2-yl)-4'-((4-(2-methoxypropoxy)-6-(methylsulfonyl)pyridin-2-yl)amino)-[2,3'-bipyridin]-6'-yl)acetamid;
N-(4'-((4-(2-Hydroxyethoxy)-6-(methylsulfonyl)pyridin-2-yl)amino)-5-(2-hydroxypropan-2-yl)-[2,3'-bipyridin]-6'-yl)acetamid;
N-(5-(2-Hydroxypropan-2-yl)-4'-((4-methyl-6-(methylsulfonyl)pyridin-2-yl)amino)-[2,3'-bipyridin]-6'-yl)acetamid;
N-(4'-((4-(trans)-3-Hydroxycyclobutoxy)-6-(methylsulfonyl)pyridin-2-yl)amino)-5-(2-hydroxypropan-2-yl)-[2,3'-bipyridin]-6'-yl)acetamid;
N-(5-(2-Hydroxypropan-2-yl)-4'-((4-methoxy-6-(methylsulfonyl)pyridin-2-yl)amino)-[2,3'-bipyridin]-6'-yl)acetamid;
N-(5-(2-Hydroxypropan-2-yl)-4'-((6-(methylsulfonyl)-4-(oxetan-3-ylmethoxy)pyridin-2-yl)amino)-[2,3'-bipyridin]-6'-yl)acetamid;
N-(5-(2-Hydroxypropan-2-yl)-4'-((4-(methoxymethyl)-6-(methylsulfonyl)pyridin-2-yl)-amino)-[2,3'-bipyridin]-6'-yl)acetamid;
N-(4'-((4-Cyano-6-(methylsulfonyl)pyridin-2-yl)amino)-5-(2-hydroxypropan-2-yl)-[2,3'-bipyridin]-6'-yl)acetamid:
N-(5-(2-Hydroxypropan-2-yl)-4'-((3-(2-methoxyethoxy)-5-(methylsulfonyl)phenyl)-amino)-[2,3'-bipyridin]-6'-yl)acetamid;
N-(5-(2-Hydroxypropan-2-yl)-4'-((3-(methoxymethyl)-5-(methylsulfonyl)phenyl)amino)-[2,3'-bipyridin]-6'-yl)acetamid;
N-(5-(2-Hydroxypropan-2-yl)-4'-((3-(3-hydroxypyrrolidin-1-yl)-5-(methylsulfonyl)-phenyl)amino)-[2,3'-bipyridin]-6'-yl)acetamid;
N-(5-(2-Hydroxypropan-2-yl)-4'-((3-(methylsulfonyl)-5-(trifluormethoxy)phenyl)amino)-[2,3'-bipyridin]-6'-yl)acetamid;
N-(4'-((3-Fluor-5-(methylsulfonyl)phenyl)amino)-5-(2-hydroxypropan-2-yl)-[2,3'-bipyridin]-6'-yl)acetamid:
N-(4'-((3-Cyano-5-(methylsulfonyl)phenyl)amino)-5-(2-hydroxypropan-2-yl)-[2,3'-bipyridin]-6'-yl)acetamid;
N-(5-(2-Hydroxypropan-2-yl)-4'-((3-methyl-5-(methylsulfonyl)phenyl)amino)-[2,3'-bipyridin]-6'-yl)acetamid:
N-(4'-((3-(Cyclopropylmethoxy)-5-(methylsulfonyl)phenyl)amino)-5-(2-hydroxypropan-2-yl)-[2,3'-bipyridin]-6'-yl)acetamid;
N-(4'-((3-Cyclopropoxy-5-(methylsulfonyl)phenyl)amino)-5-(2-hydroxypropan-2-yl)-[2,3'-bipyridin]-6'-yl)acetamid:
N-(5-(2-Hydroxypropan-2-yl)-4'-((3-isopropoxy-5-(methylsulfonyl)phenyl)amino)-[2,3'-bipyridin]-6'-yl)acetamid;
N-(4'-((3-(Difluormethyl)-5-(methylsulfonyl)phenyl)amino)-5-(2-hydroxypropan-2-yl)-[2,3'-bipyridin]-6'-yl)acetamid;
N-(5-(2-Hydroxypropan-2-yl)-4'-((4-(2-hydroxypropoxy)-6-(methylsulfonyl)pyridin-2-yl)amino)-[2,3'-bipyridin]-6'-yl)acetamid;
N-(5-(2-Hydroxypropan-2-yl)-4'-((6-(methylsulfonyl)-4-(oxetan-3-yloxy)pyridin-2-yl)-amino)-[2,3'-bipyridin]-6'-yl)acetamid;
N-(4'-((4-(cis-3-Hydroxycyclobutoxy)-6-(methylsulfonyl)pyridin-2-yl)amino)-5-(2-hydroxypropan-2-yl)-[2,3'-bipyridin]-6'-yl)acetamid;
N-(5-(2-Hydroxypropan-2-yl)-4'-((4-(3-methoxycyclobutoxy)-6-(methylsulfonyl)pyridin-2-yl)amino)-[2,3'-bipyridin]-6'-yl)acetamid;
N-(4'-((4-(Cyclopropylmethoxy)-6-(methylsulfonyl)pyridin-2-yl)amino)-5-(2-hydroxypropan-2-yl)-[2,3'-bipyridin]-6'-yl)acetamid;
N-(4'-((4-Cyclobutoxy-6-(methylsulfonyl)pyridin-2-yl)amino)-5-(2-hydroxypropan-2-yl)-[2,3'-bipyridin]-6'-yl)acetamid;
N-(5-(2-Hydroxypropan-2-yl)-4'-((3-methyl-6-(methylsulfonyl)pyridin-2-yl)amino)-[2,3'-bipyridin]-6'-yl)acetamid:
N-(4'-((4-Ethoxy-6-(methylsulfonyl)pyridin-2-yl)amino)-5-(2-hydroxypropan-2-yl)-[2,3'-bipyridin]-6'-yl)acetamid;
N-(4'-((5-Fluor-6-(methylsulfonyl)pyridin-2-yl)amino)-5-(2-hydroxypropan-2-yl)-[2,3'-bipyridin]-6'-yl)acetamid;
N-(5-(2-Hydroxypropan-2-yl)-4'-((2-methyl-5-(methylsulfonyl)phenyl)amino)-[2,3'-bipyridin]-6'-yl)acetamid;
N-(4'-((6-(Ethylsulfonyl)-4-isopropoxypyridin-2-yl)amino)-5-(2-hydroxypropan-2-yl)-[2,3'-bipyridin]-6'-yl)acetamid;
N-(5-(2-Hydroxypropan-2-yl)-4'-((7-(methylsulfonyl)-2,3-dihydro-[1,4]dioxino[2,3-c]-pyridin-5-yl)amino)-[2,3'-bipyridin]-6'-yl)acetamid;
(R)-N-(5-(2-Hydroxypropan-2-yl)-4'-((4-(2-hydroxypropoxy)-6-(methylsulfonyl)pyridin-2-yl)amino)-[2,3'-bipyridin]-6'-yl)acetamid;
N-(5-(2-Hydroxypropan-2-yl)-4'-((6-(methylsulfonyl)-4-(tetrahydro-2H-pyran-4-yl)-pyridin-2-yl)amino)-[2,3'-bipyridin]-6'-yl)acetamid;
N-(5-(2-Hydroxypropan-2-yl)-4'-((4-isobutoxy-6-(methylsulfonyl)pyridin-2-yl)amino)-[2,3'-bipyridin]-6'-yl)acetamid;
(S)-N-(4'-((4-(sec-Butoxy)-6-(methylsulfonyl)pyridin-2-yl)amino)-3-(2-hydroxypropan-2-yl)-[2,3'-bipyridin]-6'-yl)acetamid;
(R)-N-(4'-((4-(sec-Butoxy)-6-(methylsulfonyl)pyridin-2-yl)amino)-5-(2-hydroxypropan-2-yl)-[2,3'-bipyridin]-6'-yl)acetamid;
N-(5-(2-Hydroxypropan-2-yl)-4'-((3-isopropoxy-5-(methylsulfonyl)phenyl)amino)-[2,3'-bipyridin]-6'-yl)acetamid;
N-(5-(1-Methyl-1H-pyrazol-3-yl)-4-((4-methyl-6-(methylsulfonyl)pyridin-2-yl)amino)-pyridin-2-yl)acetamid;
N-(4-((4-Isopropoxy-6-(methylsulfonyl)pyridin-2-yl)amino)-5-(1-methyl-1H-pyrazol-3-yl)pyridin-2-yl)acetamid;
N-(4-((4-(Cyclopropylmethoxy)-6-(methylsulfonyl)pyridin-2-yl)amino)-5-(1-methyl-1H-pyrazol-3-yl)pyridin-2-yl)acetamid;
N-(5-(1-Methyl-1H-pyrazol-3-yl)-4-((6-sulfamoylpyridin-2-yl)amino)pyridin-2-yl)-acetamid;
N-(4-((6-(Ethylsulfonyl)pyridin-2-yl)amino)-5-(1-methyl-1H-pyrazol-3-yl)pyridin-2-yl)-acetamid;
N-(5-(1-Methyl-1H-pyrazol-3-yl)-4-((3-(methylsulfonyl)phenyl)amino)pyridin-2-yl)-acetamid;
N-(4-((3-Cyano-5-(methylsulfonyl)phenyl)amino)-5-(1-methyl-1H-pyrazol-3-yl)pyridin-2-yl)acetamid;
N-(5-(1-Methyl-1H-pyrazol-3-yl)-4-((3-methyl-5-(methylsulfonyl)phenyl)amino)pyridin-2-yl)acetamid;
N-(4-((3-Methoxy-5-(methylsulfonyl)phenyl)amino)-5-(1-methyl-1H-pyrazol-3-yl)-pyridin-2-yl)acetamid;
N-(5-(1-Methyl-1H-pyrazol-3-yl)-4-((3-(methylsulfonyl)-5-(trifluormethoxy)phenyl)-amino)pyridin-2-yl)acetamid;
N-(5-(1-Methyl-1H-pyrazol-3-yl)-4-((6-(methylsulfonyl)pyridin-2-yl)amino)pyridin-2-yl)-acetamid;
N-(4-((4-(2-Methoxyethoxy)-6-(methylsulfonyl)pyridin-2-yl)amino)-3-(1-methyl-1H-pyrazol-3-yl)pyridin-2-yl)acetamid;
N-(4-((4-(2-Methoxypropoxy)-6-(methylsulfonyl)pyridin-2-yl)amino)-5-(1-methyl-1H-pyrazol-3-yl)pyridin-2-yl)acetamid;
(R)-N-(4-((4-(2-Methoxypropoxy)-6-(methylsulfonyl)pyridin-2-yl)amino)-5-(1-methyl-1 H-pyrazol-3-yl)pyridin-2-yl)acetamid;
N-(4-((4-(2-Hydroxyethoxy)-6-(methylsulfonyl)pyridin-2-yl)amino)-5-(1-methyl-1H-pyrazol-3-yl)pyridin-2-yl)acetamid;
N-(4-((4-((trans)-3-Hydroxycyclobutoxy)-6-(methylsulfonyl)pyridin-2-yl)amino)-3-(1-methyl-1 H-pyrazol-3-yl)pyridin-2-yl)acetamid;
N-(4-((4-((cis)-3-Hydroxycyclobutoxy)-6-(methylsulfonyl)pyridin-2-yl)amino)-5-(1-methyl-1 H-pyrazol-3-yl)pyridin-2-yl)acetamid;
N-(4-((4-(3-Methoxycyclobutoxy)-6-(methylsulfonyl)pyridin-2-yl)amino)-5-(1-methyl-1 H-pyrazol-3-yl)pyridin-2-yl)acetamid;
N-(4-((4-(Methoxymethyl)-6-(methylsulfonyl)pyridin-2-yl)amino)-5-(1-methyl-1H-pyrazol-3-yl)pyridin-2-yl)acetamid;
N-(4-((4-(Cyanomethoxy)-6-(methylsulfonyl)pyridin-2-yl)amino)-5-(1-methyl-1H-pyrazol-3-yl)pyridin-2-yl)acetamid;
N-(4-((4-Methoxy-6-(methylsulfonyl)pyridin-2-yl)amino)-5-(1-methyl-1H-pyrazol-3-yl)-pyridin-2-yl)acetamid;
N-(5-(1-Methyl-1H-pyrazol-3-yl)-4-((6-(methylsulfonyl)-4-(oxetan-3-yloxy)pyridin-2-yl)-amino)pyridin-2-yl)acetamid;
N-(4-((3-Methoxy-6-(methylsulfonyl)pyridin-2-yl)amino)-5-(1-methyl-1H-pyrazol-3-yl)-pyridin-2-yl)acetamid;
N-(4-((4-(2-Hydroxypropoxy)-6-(methylsulfonyl)pyridin-2-yl)amino)-5-(1-methyl-1H-pyrazol-3-yl)pyridin-2-yl)acetamid;
N-(5-(1-Methyl-1H-pyrazol-3-yl)-4-((6-(methylsulfonyl)-4-(oxetan-3-ylmethoxy)pyridin-2-yl)amino)pyridin-2-yl)acetamid;
N-(4-((4-Cyano-6-(methylsulfonyl)pyridin-2-yl)amino)-5-(1-methyl-1H-pyrazol-3-yl)-pyridin-2-yl)acetamid;
N-(5-(1-Methyl-1H-pyrazol-3-yl)-4-((3-methyl-6-(methylsulfonyl)pyridin-2-yl)amino)-pyridin-2-yl)acetamid;
N-(4-((6-(Cyclopropylsulfonyl)pyridin-2-yl)amino)-5-(1-methyl-1H-pyrazol-3-yl)pyridin-2-yl)acetamid;
N-(4-((6-(Isopropylsulfonyl)pyridin-2-yl)amino)-5-(1-methyl-1H-pyrazol-3-yl)pyridin-2-yl)acetamid;
N-(4-((3-Fluor-3-(methylsulfonyl)phenyl)amino)-3-(1-methyl-1H-pyrazol-3-yl)pyridin-2-yl)acetamid;
N-(4-((3-Chlor-5-(methylsulfonyl)phenyl)amino)-5-(1-methyl-1H-pyrazol-3-yl)pyridin-2-yl)acetamid:
N-(4-((3-(Difluormethyl)-5-(methylsulfonyl)phenyl)amino)-5-(1-methyl-1H-pyrazol-3-yl)pyridin-2-yl)acetamid;
N-(5-(1-Methyl-1H-pyrazol-3-yl)-4-((3-(methylsulfonyl)-5-(trifluormethyl)phenyl)amino)-pyridin-2-yl)acetamid;
N-(4-((3-(Methoxymethyl)-5-(methylsulfonyl)phenyl)amino)-5-(1-methyl-1H-pyrazol-3-yl)pyridin-2-yl)acetamid;
N-(4-((3-(2-Methoxyethoxy)-5-(methylsulfonyl)phenyl)amino)-5-(1-methyl-1H-pyrazol-3-yl)pyridin-2-yl)acetamid;
N-(4-((3-Cyclopropoxy-5-(methylsulfonyl)phenyl)amino)-5-(1-methyl-1H-pyrazol-3-yl)-pyridin-2-yl)acetamid;
N-(4-((3-((trans)-3-Hydroxycyclobutoxy)-5-(methylsulfonyl)phenyl)amino)-5-(1-methyl-1H-pyrazol-3-yl)pyridin-2-yl)acetamid;
N-(5-(1-Methyl-1H-pyrazol-3-yl)-4-((6-(methylsulfonyl)-4-phenylpyridin-2-yl)amino)-pyridin-2-yl)acetamid;
N-(5-(1-Methyl-1H-pyrazol-3-yl)-4-((6-(methylsulfonyl)-4-phenoxypyridin-2-yl)amino)-pyridin-2-yl)acetamid;
N-(4-((4-Chlor-6-(methylsulfonyl)pyridin-2-yl)amino)-5-(1-methyl-1H-pyrazol-3-yl)-pyridin-2-yl)acetamid:
N-(4-((4-(1-Methoxyethyl)-6-(methylsulfonyl)pyridin-2-yl)amino)-5-(1-methyl-1H-pyrazol-3-yl)pyridin-2-yl)acetamid;
N-(4-((4-(Difluormethyl)-6-(methylsulfonyl)pyridin-2-yl)amino)-5-(1-methyl-1H-pyrazol-3-yl)pyridin-2-yl)acetamid;
N-(4-((4-Ethoxy-6-(methylsulfonyl)pyridin-2-yl)amino)-5-(1-methyl-1H-pyrazol-3-yl)-pyridin-2-yl)acetamid;
N-(4-((4-Cyclobutoxy-6-(methylsulfonyl)pyridin-2-yl)amino)-5-(1-methyl-1H-pyrazol-3-yl)pyridin-2-yl)acetamid;
N-(4-((4-(Cyclopentyloxy)-6-(methylsulfonyl)pyridin-2-yl)amino)-5-(1-methyl-1H-pyrazol-3-yl)pyridin-2-yl)acetamid;
N-(5-(1-Methyl-1H-pyrazol-3-yl)-4-((6-(methylsulfonyl)-4-((tetrahydrofuran-3-yl)oxy)-pyridin-2-yl)amino)pyridin-2-yl)acetamid;
(R)-N-(4-((4-(2-Hydroxypropoxy)-6-(methylsulfonyl)pyridin-2-yl)amino)-5-(1-methyl-1H-pyrazol-3-yl)pyridin-2-yl)acetamid;
N-(4-((4-(2,3-Dihydroxypropoxy)-6-(methylsulfonyl)pyridin-2-yl)amino)-5-(1-methyl-1H-pyrazol-3-yl)pyridin-2-yl)acetamid;
N-(4-((3-Isopropoxy-6-(methylsulfonyl)pyridin-2-yl)amino)-5-(1-methyl-1H-pyrazol-3-yl)pyridin-2-yl)acetamid;
N-(4-((5-Methoxy-6-(methylsulfonyl)pyridin-2-yl)amino)-5-(1-methyl-1H-pyrazol-3-yl)-pyridin-2-yl)acetamid;
N-(5-(1-Methyl-1H-pyrazol-3-yl)-4-((5-methyl-6-(methylsulfonyl)pyridin-2-yl)amino)-pyridin-2-yl)acetamid:
N-(5-(1-Methyl-1H-pyrazol-3-yl)-4-((6-(methylsulfonyl)-4-(trifluormethyl)pyridin-2-yl)-amino)pyridin-2-yl)acetamid;
N-(4-((6-(Ethylsulfonyl)-4-isopropoxypyridin-2-yl)amino)-5-(1-methyl-1H-pyrazol-3-yl)-pyridin-2-yl)acetamid;
N-(4-((5-Fluor-6-(methylsulfonyl)pyridin-2-yl)amino)-5-(1-methyl-1H-pyrazol-3-yl)-pyridin-2-yl)acetamid;
(S)-N-(4-((3-(2-Hydroxypropoxy)-5-(methylsulfonyl)phenyl)amino)-5-(1-methyl-1H-pyrazol-3-yl)pyridin-2-yl)acetamid;
N-(4-((3-(2-Hydroxyethoxy)-5-(methylsulfonyl)phenyl)amino)-5-(1-methyl-1H-pyrazol-3-yl)pyridin-2-yl)acetamid;
(R)-N-(4-((3-(2-Hydroxypropoxy)-5-(methylsulfonyl)phenyl)amino)-5-(1-methyl-1H-pyrazol-3-yl)pyridin-2-yl)acetamid;
N-(4-((4-(Difluormethoxy)-6-(methylsulfonyl)pyridin-2-yl)amino)-5-(1-methyl-1H-pyrazol-3-yl)pyridin-2-yl)acetamid;
N-(4-((4-Isobutoxy-6-(methylsulfonyl)pyridin-2-yl)amino)-5-(1-methyl-1H-pyrazol-3-yl)-pyridin-2-yl)acetamid;
(S)-N-(4-((4-(sec-Butoxy)-6-(methylsulfonyl)pyridin-2-yl)amino)-5-(1-methyl-1H-pyrazol-3-yl)pyridin-2-yl)acetamid;
N-(5-(1-Methyl-1H-pyrazol-3-yl)-4-((6-(methylsulfonyl)-4-(tetrahydro-2H-pyran-4-yl)-pyridin-2-yl)amino)pyridin-2-yl)acetamid;
N-(4-((3-(Difluormethoxy)-5-(methylsulfonyl)phenyl)amino)-5-(1-methyl-1H-pyrazol-3-yl)pyridin-2-yl)acetamid;
N-(4-((3-(Difluormethoxy)-6-(methylsulfonyl)pyridin-2-yl)amino)-5-(1-methyl-1H-pyrazol-3-yl)pyridin-2-yl)acetamid;
N-(5-(1-Methyl-1H-pyrazol-3-yl)-4-((7-(methylsulfonyl)-2,3-dihydro-[1,4]dioxino[2,3-c]-pyridin-5-yl)amino)pyridin-2-yl)acetamid;
(R)-N-(4-((4-(sec-Butoxy)-6-(methylsulfonyl)pyridin-2-yl)amino)-5-(1-methyl-1H-pyrazol-3-yl)pyridin-2-yl)acetamid;
N-(5-(1-Cyclopropyl-1H-pyrazol-3-yl)-4-((6-(methylsulfonyl)pyridin-2-yl)amino)pyridin-2-yl)acetamid;
N-(5-(1-Cyclopropyl-1H-pyrazol-3-yl)-4-((4-isopropoxy-6-(methylsulfonyl)pyridin-2-yl)-amino)pyridin-2-yl)acetamid;
N-(5-(1-Cyclopropyl-1H-pyrazol-3-yl)-4-((4-methyl-6-(methylsulfonyl)pyridin-2-yl)-amino)pyridin-2-yl)acetamid;
N-(5-(1-Cyclopropyl-1H-pyrazol-3-yl)-4-((4-(2-methoxyethoxy)-6-(methylsulfonyl)-pyridin-2-yl)amino)pyridin-2-yl)acetamid;
N-(5-(1-Ethyl-1H-pyrazol-3-yl)-4-((6-(methylsulfonyl)pyridin-2-yl)amino)pyridin-2-yl)-acetamid;
N-(5-(1-Ethyl-1H-pyrazol-3-yl)-4-((4-isopropoxy-6-(methylsulfonyl)pyridin-2-yl)amino)-pyridin-2-yl)acetamid;
N-(5-(1-Ethyl-1H-pyrazol-3-yl)-4-((4-(methoxymethyl)-6-(methylsulfonyl)pyridin-2-yl)-amino)pyridin-2-yl)acetamid;
N-(5-(1-Ethyl-1H-pyrazol-3-yl)-4-((3-(methylsulfonyl)phenyl)amino)pyridin-2-yl)-acetamid;
N-(5-(1-Ethyl-1H-pyrazol-3-yl)-4-((3-methoxy-5-(methylsulfonyl)phenyl)amino)pyridin-2-yl)acetamid;
N-(4-((3-Cyano-5-(methylsulfonyl)phenyl)amino)-5-(1-ethyl-1H-pyrazol-3-yl)pyridin-2-yl)acetamid;
N-(5-(1-Ethyl-1H-pyrazol-3-yl)-4-((3-methyl-5-(methylsulfonyl)phenyl)amino)pyridin-2-yl)acetamid:
N-(5-(1-Ethyl-1H-pyrazol-3-yl)-4-((3-(methoxymethyl)-5-(methylsulfonyl)phenyl)-amino)pyridin-2-yl)acetamid;
N-(5-(1-Ethyl-1H-pyrazol-3-yl)-4-((3-(methylsulfonyl)-5-(trifluormethyl)phenyl)amino)-pyridin-2-yl)acetamid;
N-(5-(1-Ethyl-1H-pyrazol-3-yl)-4-((3-(2-methoxyethoxy)-5-(methylsulfonyl)phenyl)-amino)pyridin-2-yl)acetamid;
N-(5-(1-Ethyl-1H-pyrazol-3-yl)-4-((3-(2-hydroxyethoxy)-5-(methylsulfonyl)phenyl)-amino)pyridin-2-yl)acetamid;
N-(4-((3-(Cyclopropylmethoxy)-3-(methylsulfonyl)phenyl)amino)-5-(1-ethyl-1H-pyrazol-3-yl)pyridin-2-yl)acetamid;
N-(4-((4-(Cyclopentyloxy)-6-(methylsulfonyl)pyridin-2-yl)amino)-3-(1-ethyl-1H-pyrazol-3-yl)pyridin-2-yl)acetamid;
N-(4-((4-Ethoxy-6-(methylsulfonyl)pyridin-2-yl)amino)-3-(1-ethyl-1H-pyrazol-3-yl)-pyridin-2-yl)acetamid:
N-(5-(1-Ethyl-1H-pyrazol-3-yl)-4-((4-(2-hydroxyethoxy)-6-(methylsulfonyl)pyridin-2-yl)-amino)pyridin-2-yl)acetamid;
N-(5-(1-Ethyl-1H-pyrazol-3-yl)-4-((4-(2-methoxyethoxy)-6-(methylsulfonyl)pyridin-2-yl)amino)pyridin-2-yl)acetamid;
N-(5-(1-Ethyl-1H-pyrazol-3-yl)-4-((3-isopropoxy-6-(methylsulfonyl)pyridin-2-yl)amino)-pyridin-2-yl)acetamid;
N-(5-(1-Isopropyl-1H-pyrazol-3-yl)-4-((6-(methylsulfonyl)pyridin-2-yl)amino)pyridin-2-yl)acetamid;
N-(4-((4-Isopropoxy-6-(methylsulfonyl)pyridin-2-yl)amino)-3-(1-isopropyl-1H-pyrazol-3-yl)pyridin-2-yl)acetamid;
N-(5-(1-Isopropyl-1H-pyrazol-3-yl)-4-((4-(2-methoxyethoxy)-6-(methylsulfonyl)pyridin-2-yl)amino)pyridin-2-yl)acetamid;
N-(5-(1-Isopropyl-1H-pyrazol-3-yl)-4-((3-(methylsulfonyl)-5-(trifluormethyl)phenyl)-amino)pyridin-2-yl)acetamid;
N-(4-((3-Isopropoxy-5-(methylsulfonyl)phenyl)amino)-5-(1-isopropyl-1H-pyrazol-3-yl)-pyridin-2-yl)acetamid;
N-(5-(1-Isopropyl-1H-pyrazol-3-yl)-4-((3-methoxy-5-(methylsulfonyl)phenylamino)-pyridin-2-yl)acetamid;
N-(4-((4-(2-Hydroxyethoxy)-6-(methylsulfonyl)pyridin-2-yl)amino)-5-(1-isopropyl-1H-pyrazol-3-yl)pyridin-2-yl)acetamid;
N-(4-((3-(2-Hydroxyethoxy)-5-(methylsulfonyl)phenyl)amino)-5-(1-isopropyl-1H-pyrazol-3-yl)pyridin-2-yl)acetamid;
N-(4-((3-(Methylsulfonyl)phenyl)amino)-5-(1-(tetrahydrofuran-3-yl)-1H-pyrazol-3-yl)-pyridin-2-yl)acetamid:
N-(4-((4-Methyl-6-(methylsulfonyl)pyridin-2-yl)amino)-5-(1-(tetrahydrofuran-3-yl)-1H-pyrazol-3-yl)pyridin-2-yl)acetamid;
(R)-N-(4-((3-(Methylsulfonyl)-5-(trifluormethyl)phenyl)amino)-5-(1-(tetrahydrofuran-3-yl)-1H-pyrazol-3-yl)pyridin-2-yl)acetamid;
(S)-N-(4-((4-Isopropoxy-6-(methylsulfonyl)pyridin-2-yl)amino)-5-(1-(tetrahydrofuran-3-yl)-1H-pyrazol-3-yl)pyridin-2-yl)acetamid;
(R)-N-(4-((4-Isopropoxy-6-(methylsulfonyl)pyridin-2-yl)amino)-5-(1-(tetrahydrofuran-3-yl)-1H-pyrazol-3-yl)pyridin-2-yl)acetamid;
(R)-N-(4-((4-Methoxy-6-(methylsulfonyl)pyridin-2-yl)amino)-3-(1-(tetrahydrofuran-3-yl)-1H-pyrazol-3-yl)pyridin-2-yl)acetamid;
N-(5-(1,5-Dimethyl-1H-pyrazol-3-yl)-4-((6-(methylsulfonyl)pyridin-2-yl)amino)pyridin-2-yl)acetamid;
N-(5-(1,5-Dimethyl-1H-pyrazol-3-yl)-4-((4-isopropoxy-6-(methylsulfonyl)pyridin-2-yl)-amino)pyridin-2-yl)acetamid;
N-(5-(1,5-Dimethyl-1H-pyrazol-3-yl)-4-((3-(2-hydroxyethoxy)-5-(methylsulfonyl)-phenyl)amino)pyridin-2-yl)acetamid;
N-(5-(1,5-Dimethyl-1H-pyrazol-3-yl)-4-((4-methoxy-6-(methylsulfonyl)pyridin-2-yl)-amino)pyridin-2-yl)acetamid;
N-(5-(1,5-Dimethyl-1H-pyrazol-3-yl)-4-((4-(2-methoxyethoxy)-6-(methylsulfonyl)-pyridin-2-yl)amino)pyridin-2-yl)acetamid;
(R)-N-(5-(1,5-Dimethyl-1H-pyrazol-3-yl)-4-((4-(2-methoxypropoxy)-6-(methylsulfonyl)-pyridin-2-yl)amino)pyridin-2-yl)acetamid;
N-(5-(1,5-Dimethyl-1H-pyrazol-3-yl)-4-((4-ethoxy-6-(methylsulfonyl)pyridin-2-yl)-amino)pyridin-2-yl)acetamid;
N-(5-(1-Methyl-5-morpholino-1H-pyrazol-3-yl)-4-((6-(methylsulfonyl)pyridin-2-yl)-amino)pyridin-2-yl)acetamid;
N-(4-((4-(2-Methoxyethoxy)-6-(methylsulfonyl)pyridin-2-yl)amino)-5-(1-methyl-5-morpholino-1H-pyrazol-3-yl)pyridin-2-yl)acetamid;
N-(4-((4-(2-Hydroxypropoxy)-6-(methylsulfonyl)pyridin-2-yl)amino)-5-(1-methyl-5-morpholino-1H-pyrazol-3-yl)pyridin-2-yl)acetamid;
N-(5-(2,3-Dihydro-[1,4]dioxino[2,3-b]pyridin-6-yl)-4-((4-isopropoxy-6-(methylsulfonyl)-pyridin-2-yl)amino)pyridin-2-yl)acetamid;
N-(5-([1,3]Dioxolo[4,5-b]pyridin-5-yl)-4-((4-isopropoxy-6-(methylsulfonyl)pyridin-2-yl)-amino)pyridin-2-yl)acetamid;
N-(5-(2,3-Dihydro-[1,4]dioxino[2,3-b]pyridin-6-yl)-4-((4-methyl-6-(methylsulfonyl)-pyridin-2-yl)amino)pyridin-2-yl)acetamid;
N-(5-(3,4-Dihydro-2H-pyrido[4,3-b][1,4]oxazin-7-yl)-4-((4-isopropoxy-6-(methylsulfonyl)pyridin-2-yl)amino)pyridin-2-yl)acetamid;
N-(5-(Imidazo[1,2-b]pyridazin-6-yl)-4-((4-isopropoxy-6-(methylsulfonyl)pyridin-2-yl)-amino)pyridin-2-yl)acetamid;
N-(5-(6,7-Dihydro-4H-pyrazolo[5,1-c][1,4]oxazin-2-yl)-4-((4-isopropoxy-6-(methylsulfonyl)pyridin-2-yl)amino)pyridin-2-yl)acetamid;
N-(5-(4-Acetyl-3,4-dihydro-2H-pyrido[3,2-b][1,4]oxazin-6-yl)-4-((4-isopropoxy-6-(methylsulfonyl)pyridin-2-yl)amino)pyridin-2-yl)acetamid;
N-(5-(2,3-Dihydro-[1,4]dioxino[2,3-c]pyridin-7-yl)-4-((4-methoxy-6-(methylsulfonyl)-pyridin-2-yl)amino)pyridin-2-yl)acetamid;
N-(5-(Benzo[b]thiophen-2-yl)-4-((4-methoxy-6-(methylsulfonyl)pyridin-2-yl)amino)-pyridin-2-yl)acetamid;
N-(5-(6,7-Dihydro-4H-thieno[3.2-c]pyran-2-yl)-4-((4-methyl-6-(methylsulfonyl)pyridin-2-yl)amino)pyridin-2-yl)acetamid;
N-(5-(2,3-Dihydro-[1,4]dioxino[2,3-b]pyridin-6-yl)-4-((4-methoxy-6-(methylsulfonyl)-pyridin-2-yl)amino)pyridin-2-yl)acetamid;
N-(5-(2,3-Dihydro-[1,4]dioxino[2,3-c]pyridin-7-yl)-4-((4-ethoxy-6-(methylsulfonyl)-pyridin-2-yl)amino)pyridin-2-yl)acetamid;
N-(5-(2,3-Dihydro-[1,4]dioxino[2,3-c]pyridin-7-yl)-4-((4-(2-methoxyethoxy)-6-(methylsulfonyl)pyridin-2-yl)amino)pyridin-2-yl)acetamid;
N-(5-([1,3]Dioxolo[4,5-b]pyridin-5-yl)-4-((4-(2-methoxyethoxy)-6-(methylsulfonyl)-pyridin-2-yl)amino)pyridin-2-yl)acetamid;
N-(5-([1,3]Dioxolo[4,5-b]pyridin-5-yl)-4-((4-ethoxy-6-(methylsulfonyl)pyridin-2-yl)-amino)pyridin-2-yl)acetamid;
N-(5-([1,3]Dioxolo[4,5-b]pyridin-5-yl)-4-((4-methoxy-6-(methylsulfonyl)pyridin-2-yl)-amino)pyridin-2-yl)acetamid;
N-(4-((4-(Cyclopropylmethoxy)-6-(methylsulfonyl)pyridin-2-yl)amino)-5-(5-methyl-4,5,6,7-tetrahydrothiazolo[5,4-c]pyridin-2-yl)pyridin-2-yl)acetamid;
N-(5-(2,3-Dihydro-[1,4]dioxino[2,3-c]pyridin-7-yl)-4-((4-methyl-6-(methylsulfonyl)-pyridin-2-yl)amino)pyridin-2-yl)acetamid;
N-(5-(2,3-Dihydro-[1,4]dioxino[2,3-c]pyridin-7-yl)-4-((4-(2-methoxyethoxy)-6-(methylsulfonyl)pyridin-2-yl)amino)pyridin-2-yl)acetamid;
N-(5-(6,7-Dihydro-4H-pyrazolo[5,1-c][1,4]oxazin-2-yl)-4-((4-methyl-6-(methylsulfonyl)-pyridin-2-yl)amino)pyridin-2-yl)acetamid;
N-(4-((4-((1,4-Dioxan-2-yl)methoxy)-6-(methylsulfonyl)pyridin-2-yl)amino)-5-(3,3-dimethyl-2,3-dihydro-[1,4]dioxino[2,3-b]pyridin-6-yl)pyridin-2-yl)acetamid;
N-(5-(3,3-'Dimethyl-2,3-dihydro-[1,4]dioxino[2,3-b]pyridin-6-yl)-4-((6-(methylsulfonyl)-pyrazin-2-yl)amino)pyridin-2-yl)acetamid;
N-(5-(3,3-Dimethyl-2,3-dihydro-[1,4]dioxino[2,3-b]pyridin-6-yl)-4-((4-(2-hydroxypropan-2-yl)-6-(methylsulfonyl)pyridin-2-yl)amino)pyridin-2-yl)acetamid;
N-(5-(3,3-Dimethyl-2,3-dihydro-[1,4]dioxino[2,3-b]pyridin-6-yl)-4-((5-fluor-4-methyl-6-(methylsulfonyl)pyridin-2-yl)amino)pyridin-2-yl)acetamid;
N-(5-(3,3-Dimethyl-2,3-dihydro-[1,4]dioxino[2,3-b]pyridin-6-yl)-4-((6-(methylsulfonyl)-4-morpholinopyridin-2-yl)amino)pyridin-2-yl)acetamid;
N-(5-(2,2-Dimethyl-2,3-dihydro-[1,4]dioxino[2,3-b]pyridin-6-yl)-4-((4-methyl-6-(methylsulfonyl)pyridin-2-yl)amino)pyridin-2-yl)acetamid;
N-(5-(2,2-Dimethyl-2,3-dihydro-[1,4]dioxino[2,3-b]pyridin-6-yl)-4-((6-(methylsulfonyl)-4-morpholinopyridin-2-yl)amino)pyridin-2-yl)acetamid;
N-(5-(2,2-Dimethyl-2,3-dihydro-[1,4]dioxino[2,3-b]pyridin-6-yl)-4-((6-(methylsulfonyl)-4-(tetrahydro-2H-pyran-4-yl)pyridin-2-yl)amino)pyridin-2-yl)acetamid;
N-(5-(2,2-Dimethyl-2,3-dihydro-[1,4]dioxino|[2,3-b]pyridin-6-yl)-4-((4-(methoxy-d3)-6-(methylsulfonyl)pyridin-2-yl)amino)pyridin-2-yl)acetamid;
N-(5-(2,2-Dimethyl-2,3-dihydro-[1,4]dioxino[2,3-b]pyridin-6-yl)-4-((4-methoxy-6-(methylsulfonyl)pyridin-2-yl)amino)pyridin-2-yl)acetamid;
N-(4-((3,4-Dimethoxy-6-(methylsulfonyl)pyridin-2-yl)amino)-5-(2,2-dimethyl-2,3-dihydro-[1,4]dioxino[2,3-b]pyridin-6-yl)pyridin-2-yl)acetamid;
(S)-N-(5-(2,2-Dimethyl-2,3-dihydro-[1,4]dioxino[2,3-b]pyridin-6-yl)-4-((4-(3-methylmorpholino)-6-(methylsulfonyl)pyridin-2-yl)amino)-pyridin-2-yl)acetamid;
(R)-N-(5-(2,2-Dimethyl-2,3-dihydro-[1,4]dioxino[2,3-b]pyridin-6-yl)-4-((4-(3-methylmorpholino)-6-(methylsulfonyl)pyridin-2-yl)amino)-pyridin-2-yl)acetamid;
N-(5-(3,3-Dimethyl-2,3-dihydro-[1,4]dioxino[2,3-b]pyridin-6-yl)-4-((4-(methoxy-d3)-6-(methylsulfonyl)pyridin-2-yl)amino)pyridin-2-yl)acetamid;
N-(5-(3,3-Dimethyl-2,3-dihydro-[1,4]dioxino[2,3-b]pyridin-6-yl)-4-((4-methoxy-6-(methylsulfonyl)pyridin-2-yl)amino)pyridin-2-yl acetamid;
N-(5-(3,3-Dimethyl-2,3-dihydro-[1,4]dioxino[2,3-b]pyridin-6-yl)-4-((6-(methylsulfonyl)-4-(tetrahydro-2H-pyran-4-yl)pyridin-2-yl)amino)pyridin-2-yl)acetamid;
(R)-N-(5-(3,3-Dimethyl-2,3-dihydro-[1,4]dioxino[2,3-b]pyridin-6-yl)-4-((4-(3-methylmorpholino)-6-(methylsulfonyl)pyridin-2-yl)amino)pyridin-2-yl)acetamid;
(S)-N-(5-(3,3-Dimethyl-2,3-dihydro-[1,4|dioxino[2,3-b]pyridin-6-yl)-4-((4-(3-methyl-morpholino)-6-(methylsulfonyl)pyridin-2-yl)amino)pyridin-2-yl)acetamid;
(S)-N-(5-(3,3-Dimethyl-2,3-dihydro-[1,4]dioxino[2,3-b]pyridin-6-yl)-4-((4-(1-methoxyethyl)-6-(methylsulfonyl)pyridin-2-yl)amino)pyridin-2-yl)acetamid;
(R)-N-(5-(3,3-Dimethyl-2,3-dihydro-[1,4]dioxino[2,3-b]pyridin-6-yl)-4-((4-(1-methoxyethyl)-6-(methylsulfonyl)pyridin-2-yl)amino)pyridin-2-yl)acetamid;
(R)-N-(5-(2,2-Dimethyl-2,3-dihydro-[1,4]dioxino[2,3-b]pyridin-6-yl)-4-((4-(3-methoxy-pyrrolidin-1-yl)-6-(methylsulfonyl)pyridin-2-yl)amino)pyridin-2-yl)acetamid;
(S)-N-(5-(2,2-Dimethyl-2,3-dihydro-[1,4]dioxino[2,3-b]pyridin-6-yl)-4-((4-(3-methoxy-pyrrolidin-1-yl)-6-(methylsulfonyl)pyridin-2-yl)amino)pyridin-2-yl)acetamid;
N-(4-((4-(4-Aminotetrahydro-2H-pyran-4-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamid;
N-(4-((4-(4-(Aminomethyl)tetrahydro-2H-pyran-4-yl)-6-(methylsulfonyl)pyridin-2-yl)-amino)-5-(2,2-dimethyl-2,3-dihydro-[1,4]dioxino[2,3-b]pyridin-6-yl)pyridin-2-yl)acetamid;
N-(5-(2,2-Dimethyl-2,3-dihydro-[1,4|dioxino[2,3-b]pyridin-6-yl)-4-((4-(3-methoxy-azetidin-1-yl)-6-(methylsulfonyl)pyridin-2-yl)amino)pyridin-2-yl)acetamid;
(R/S)-N-(4-((4-(1,4-Dioxan-2-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamid;
(S/R)-N-(4-((4-(1,4-Dioxan-2-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamid;
N-(5-(2,2-Dimethyl-2,3-dihydro-[1,4]dioxino[2,3-b]pyridin-6-yl)-4-((4-(4-methoxy-piperidin-1-yl)-6-(methylsulfonyl)pyridin-2-yl)amino)pyridin-2-yl)acetamid;
N-(5-(2,2-Dimethyl-2,3-dihydro-[1,4]dioxino[2,3-b]pyridin-6-yl)-4-((4-((cis)-3-methoxy-cyclobutyl)-6-(methylsulfonyl)pyridin-2-yl)amino)pyridin-2-yl)acetamid;
N-(5-(2,2-Dimethyl-2,3-dihydro-[1,4]dioxino[2,3-b]pyridin-6-yl)-4-((4-((trans)-3-methoxycyclobutyl)-6-(methylsulfonyl)pyridin-2-yl)amino)pyridin-2-yl)acetamid;
N-(5-(2,2-Dimethyl-2,3-dihydro-[1,4]dioxino[2,3-b]pyridin-6-yl)-4-((4-(4-hydroxy-4-methylpiperidin-1-yl)-6-(methylsulfonyl)pyridin-2-yl)amino)pyridin-2-yl)acetamid;
N-(5-(2,2-Dimethyl-2,3-dihydro-[1,4]dioxino[2,3-b]pyridin-6-yl)-4-((4-(3-hydroxy-3-methylazetidin-1-yl)-6-(methylsulfonyl)pyridin-2-yl)amino)pyrid-in-2-yl)acetamid;
N-(5-(2,2-Dimethyl-2,3-dihydro-[1,4]dioxino[2,3-b]pyridin-6-yl)-4-((4-(3-hydroxy-3-methylpyrrolidin-1-yl)-6-(methylsulfonyl)pyridin-2-yl)amino)pyridin-2-yl)acetamid;
N-(6-(2,2-Dimethyl-2,3-dihydro-[1,4]dioxino[2,3-b]pyridin-6-yl)-5-((6-(methylsulfonyl)-4-(tetrahydro-2H-pyran-4-yl)pyridin-2-yl)amino)pyridazin-3-yl)acetamid;
(R)-N-(5-(2,2-Dimethyl-2,3-dihydro-[1,4]dioxino[2,3-b]pyridin-6-yl)-4-((4-(3-(2-hydroxy-ethoxy)pyrrolidin-1-yl)-6-(methylsulfonyl)pyridin-2-yl)amino)pyridin-2-yl)acetamid;
N-(5-(2,2-Dimethyl-2,3-dihydro-[1,4]dioxino[2,3-b]pyridin-6-yl)-4-((6-(methylsulfonyl)-4-(1-(2,2,2-trifluorethyl)piperidin-4-yl)pyridin-2-yl)amino)pyridin-2-yl)acetamid;
(S)-N-(5-(2,2-Dimethyl-2,3-dihydro-[1,4]dioxino[2,3-b]pyridin-6-yl)-4-((4-(3-(2-hydroxy-ethoxy)pyrrolidin-1-yl)-6-(methylsulfonyl)pyridin-2-yl)amino)pyridin-2-yl)acetamid;
N-(5-(2,2-Dimethyl-2,3-dihydro-[1,4]dioxino[2,3-b]pyridin-6-yl)-4-((4-methyl-6'-(methylsulfonyl)-[3,4'-bipyridin]-2'-yl)amino)pyridin-2-yl)acetamid;
N-(4-((4-Cyano-6-(methylsulfonyl)pyridin-2-yl)amino)-5-(2,2-dimethyl-2,3-dihydro-[1,4]dioxino[2,3-b]pyridin-6-yl)pyridin-2-yl)acetamid;
N-(5-(2,2-Dimethyl-2,3-dihydro-[1,4]dioxino[2,3-b]pyridin-6-yl)-4-((4-methyl-6-(methylsulfonyl)-3-oxo-3,4-dihydropyrazin-2-yl)amino)pyridin-2-yl)acetamid;
(S)-N-(5-(2,2-Dimethyl-2,3-dihydro-[1,4]dioxino[2,3-b]pyridin-6-yl)-4-((4-(3-methoxy-piperidin-1-yl)-6-(methylsulfonyl)pyridin-2-yl)amino)pyridin-2-yl)acetamid;
(R)-N-(5-(2,2-Dimethyl-2,3-dihydro-[1,4]dioxino[2,3-b]pyridin-6-yl)-4-((4-(3-methoxy-piperidin-1-yl)-6-(methylsulfonyl)pyridin-2-yl)amino)pyridin-2-yl)acetamid;
(S)-N-(5-(2,2-Dimethyl-2,3-dihydro-[1,4]dioxino[2,3-b]pyridin-6-yl)-4-((4-(3-ethoxy-piperidin-1-yl)-6-(methylsulfonyl)pyridin-2-yl)amino)pyridin-2-yl)acetamid;
(R)-N-(5-(2,2-Dimethyl-2,3-dihydro-[1,4]dioxino[2,3-b]pyridin-6-yl)-4-((4-(3-ethoxy-piperidin-1-yl)-6-(methylsulfonyl)pyridin-2-yl)amino)pyridin-2-yl)acetamid;
(S)-N-(5-(2,2-Dimethyl-2,3-dihydro-[1,4]dioxino[2,3-b]pyridin-6-yl)-4-((6-(methylsulfonyl)-4-(3-(trifluormethoxy)pyrrolidin-1-yl)pyridin-2-yl)amino)pyridin-2-yl)acetamid;
(R)-N-(5-(2,2-Dimethyl-2,3-dihydro-[1,4]dioxino[2,3-b]pyridin-6-yl)-4-((6-(methylsulfonyl)-4-(3-(trifluormethoxy)pyrrolidin-1-yl)pyridin-2-yl)amino)pyridin-2-yl)acetamid;
(S)-1-(2-((2-Acetamido-5-(2,2-dimethyl-2,3-dihydro-[1,4]dioxino[2,3-b]pyridin-6-yl)-pyridin-4-yl)amino)-6-(methylsulfonyl)pyridin-4-yl)pyrrolidin-2-carboxamid;
(R)-1-(2-((2-Acetamido-5-(2,2-dimethyl-2,3-dihydro-[1,4]dioxino[2,3-b]pyridin-6-yl)-pyridin-4-yl)amino)-6-(methylsulfonyl)pyridin-4-yl)pyrrolidin-2-carboxamid;
(S)-N-(5-(2,2-Dimethyl-2,3-dihydro-[1,4]dioxino[2,3-b]pyridin-6-yl)-4-((4-(3-(methoxymethyl)pyrrolidin-1-yl)-6-(methylsulfonyl)pyridin-2-yl)amino)pyridin-2-yl)acetamid;
(R)-N-(5-(2,2-Dimethyl-2,3-dihydro-[1,4]dioxino[2,3-b]pyridin-6-yl)-4-((4-(3-(methoxymethyl)pyrrolidin-1-yl)-6-(methylsulfonyl)pyridin-2-yl)amino)pyridin-2-yl)acetamid;
(S)-N-(5-(2,2-Dimethyl-2,3-dihydro-[1,4]dioxino[2,3-b]pyridin-6-yl)-4-((6-(methylsulfonyl)-4-(tetrahydro-2H-pyran-3-yl)pyridin-2-yl)amino)pyridin-2-yl)acetamid;
(R)-N-(5-(2,2-Dimethyl-2,3-dihydro-[1,4]dioxino[2,3-b]pyridin-6-yl)-4-((6-(methylsulfonyl)-4-(tetrahydro-2H-pyran-3-yl)pyridin-2-yl)amino)pyridin-2-yl)acetamid;
(S)-N-(5-(2,2-Dimethyl-2,3-dihydro-[1,4|dioxino[2,3-b]pyridin-6-yl)-4-((5-(3-methoxy-pyrrolidin-1-yl)-6-(methylsulfonyl)pyridin-2-yl)amino)pyridin-2-yl)acetamid;
(R)-N-(5-(2,2-Dimethyl-2,3-dihydro-[1,4]dioxino[2,3-b]pyridin-6-yl)-4-((5-(3-methoxy-pyrrolidin-1-yl)-6-(methylsulfonyl)pyridin-2-yl)amino)pyridin-2-yl)acetamid;
N-(5-(2,2-Dimethyl-2,3-dihydro-[1,4]dioxino[2,3-b]pyridin-6-yl)-4-((6-(methylsulfonyl)-5-(tetrahydro-2H-pyran-4-yl)pyridin-2-yl)amino)pyridin-2-yl)acetamid;
N-(5-(2,2-Dimethyl-2,3-dihydro-[1,4]dioxino[2,3-b]pyridin-6-yl)-4-((4-(5-methyl-pyridazin-4-yl)-6-(methylsulfonyl)pyridin-2-yl)amino)pyridin-2-yl)acetamid;
N-(5-(2,2-Dimethyl-2,3-dihydro-[1,4]dioxino[2,3-b]pyridin-6-yl)-4-((4-(5-methoxy-pyridazin-4-yl)-6-(methylsulfonyl)pyridin-2-yl)amino)pyridin-2-yl)acetamid;
N-(4-((4-(cis-3-Cyano-4-hydroxypyrrolidin-1-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamid;
N-(4-((4-(trans-3-Cyano-4-hydroxypyrrolidin-1-yl)-6-(methylsulfonyl)pyridin-2-yl)-amino)-5-(2,2-dimethyl-2,3-dihydro-[1,4]dioxino[2,3-b]pyridin-6-yl)pyridin-2-yl)acetamid;
N-(4-((4-(cis-3-Cyano-4-methoxypyrrolidin-1-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamid;
N-(4-((4-(trans-3-Cyano-4-methoxypyrrolidin-1-yl)-6-(methylsulfonyl)pyridin-2-yl)-amino)-5-(2,2-dimethyl-2,3-dihydro-[1,4]dioxino[2,3-b]pyridin-6-yl)pyridin-2-yl)acetamid;
(S)-N-(5-(2,2-Dimethyl-2,3-dihydro-[1,4]dioxino[2,3-b]pyridin-6-yl)-4-((6-(methylsulfonyl)-4-(tetrahydrofuran-3-yl)pyridin-2-yl)amino)pyridin-2-yl)acetamid;
(R)-N-(5-(2,2-Dimethyl-2,3-dihydro-[1,4]dioxino[2,3-b]pyridin-6-yl)-4-((6-(methylsulfonyl)-4-(tetrahydrofuran-3-yl)pyridin-2-yl)amino)pyridin-2-yl)acetamid;
(S)-N-(5-(2,2-Dimethyl-2,3-dihydro-[1,4|dioxino[2,3-b]pyridin-6-yl)-4-((4-(2-(methoxymethyl)pyrrolidin-1-yl)-6-(methylsulfonyl)pyridin-2-yl)amino)pyridin-2-yl)acetamid;
(R)-N-(5-(2,2-Dimethyl-2,3-dihydro-[1,4]dioxino[2,3-b]pyridin-6-yl)-4-((4-(2-(methoxymethyl)pyrrolidin-1-yl)-6-(methylsulfonyl)pyridin-2-yl)amino)pyridin-2-yl)acetamid;
N-(5-(2,2-Dimethyl-2,3-dihydro-[1,4]dioxino[2,3-b]pyridin-6-yl)-4-((6-(methylsulfonyl)-3-(tetrahydro-2H-pyran-4-yl)pyridin-2-yl)amino)pyridin-2-yl)acetamid;
(S)-N-(5-(2,2-Dimethyl-2,3-dihydro-[1,4]dioxino[2,3-b]pyridin-6-yl)-4-((3-(3-methoxy-pyrrolidin-1-yl)-6-(methylsulfonyl)pyridin-2-yl)amino)pyridin-2-yl)acetamid;
(R)-N-(5-(2,2-Dimethyl-2,3-dihydro-[1,4]dioxino[2,3-b]pyridin-6-yl)-4-((3-(3-methoxy-pyrrolidin-1-yl)-6-(methylsulfonyl)pyridin-2-yl)amino)pyridin-2-yl)acetamid;
cis-N-(5-(2,2-Dimethyl-2,3-dihydro-[1,4]dioxino[2,3-b]pyridin-6-yl)-4-((4-((cis-4-hydroxycyclohexyl)oxy)-6-(methylsulfonyl)pyridin-2-yl)amino)pyridin-2-yl)acetamid;
N-(5-(2,2-Dimethyl-2,3-dihydro-[1,4]dioxino[2,3-b]pyridin-6-yl)-4-((4-((trans-4-hydroxy-cyclohexyl)oxy)-6-(methylsulfonyl)pyridin-2-yl)amino)pyridin-2-yl)acetamid;
N-(5-(2,2-Dimethyl-2,3-dihydro-[1,4]dioxino[2,3-b]pyridin-6-yl)-4-((4-((cis-4-methoxy-cyclohexyl)oxy)-6-(methylsulfonyl)pyridin-2-yl)amino)pyridin-2-yl)acetamid;
N-(5-(2,2-Dimethyl-2,3-dihydro-[1,4]dioxino[2,3-b]pyridin-6-yl)-4-((4-((trans-4-methoxycyclohexyl)oxy)-6-(methylsulfonyl)pyridin-2-yl)amino)pyridin-2-yl)acetamid;
N-(5-(2,2-Dimethyl-2,3-dihydro-[1,4]dioxino|[2,3-b]pyridin-6-yl)-4-((6-(methylsulfonyl)-4-(5-azaspiro[2,4]heptan-5-yl)pyridin-2-yl)amino)pyridin-2-yl)acetamid;
N-(5-(2,2-Dimethyl-2,3-dihydro-[1,4]dioxino[2,3-b]pyridin-6-yl)-4-((4-((3-methyloxetan-3-yl)methoxy)-6-(methylsulfonyl)pyridin-2-yl)amino)pyridin-2-yl)acetamid;
N-(5-(2,2-Dimethyl-2,3-dihydro-[1,4]dioxino[2,3-b]pyridin-6-yl)-4-((4-(3,5-dimethyl-isoxazol-4-yl)-6-(methylsulfonyl)pyridin-2-yl)amino)pyridin-2-yl)acetamid;
N-(5-(2,2-Dimethyl-2,3-dihydro-[1,4]dioxino[2,3-b]pyridin-6-yl)-4-((4-((2,4-dioxo-thiazolidin-3-yl)methyl)-6-(methylsulfonyl)pyridin-2-yl)amino)pyridin-2-yl)acetamid;
N-(5-(2,2-Dimethyl-2,3-dihydro-[1,4]dioxino[2,3-b]pyridin-6-yl)-4-((4-methoxy-6'-(methylsulfonyl)-[3,4'-bipyridin]-2'-yl)amino)pyridin-2-yl)acetamid;
(R)-N-(5-(2,2-Dimethyl-2,3-dihydro-[1,4]dioxino[2,3-b]pyridin-6-yl)-4-((4-(3-methylpyrrolidin-1-yl)-6-(methylsulfonyl)pyridin-2-yl)amino)pyridin-2-yl)acetamid;
(S)-N-(5-(2,2-Dimethyl-2,3-dihydro-[1,4]dioxino[2,3-b]pyridin-6-yl)-4-((4-(3-methylpyrrolidin-1-yl)-6-(methylsulfonyl)pyridin-2-yl)amino)pyridin-2-yl)acetamid;
N-(5-(2,2-Dimethyl-2,3-dihydro-[1,4]dioxino[2,3-b[pyridin-6-yl)-4-((6-(methylsulfonyl)-4-(pyrrolidin-1-yl)pyridin-2-yl)amino)pyridin-2-yl)acetamid;
N-(5-(2,2-Dimethyl-2,3-dihydro-[1,4]dioxino[2,3-b]pyridin-6-yl)-4-((4-(2-hydroxypropan-2-yl)-6'-(methylsulfonyl)-[3,4'-bipyridin]-2'-yl)amino)pyridin-2-yl)acetamid;
N-(4-((4-(3-Azabicyclo[3,1,0]hexan-3-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamid;
(S)-N-(4-((4-(3-(Cyanomethoxy)pyrrolidin-1-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamid;
N-(4-((6-Cyano-6'-(methylsulfonyl)-[3,4'-bipyridin]-2'-yl)amino)-3-(2,2-dimethyl-2,3-dihydro-[1,4]dioxino[2,3-b]pyridin-6-yl)pyridin-2-yl)acetamid;
N-(4-((2-Cyano-6'-(methylsulfonyl)-[3,4'-bipyridin]-2'-yl)amino)-5-(2,2-dimethyl-2,3-dihydro-[1,4]dioxino[2,3-b]pyridin-6-yl)pyridin-2-yl)acetamid;
N-(5-(2,2-Dimethyl-2,3-dihydro-[1,4]dioxino[2,3-b]pyridin-6-yl)-4-((4-(3-methylpyrazin-2-yl)-6-(methylsulfonyl)pyridin-2-yl)amino)pyridin-2-yl)acetamid;
N-(5-(2,2-Dimethyl-2,3-dihydro-[1,4]dioxino[2,3-b]pyridin-6-yl)-4-((6-(methylsulfonyl)-4-(2,2,6,6-tetramethyltetrahydro-2H-pyran-4-yl)pyridin-2-yl)amino)pyridin-2-yl)acetamid;
(R)-N-(4-((4-(2,2-Dichlorcyclopropyl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamid;
(S)-N-(4-((4-(2,2-Dichlorcyclopropyl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamid;
N-(4-((4-(5-Cyanopyridazin-4-yl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamid;
N-(4-((4-Cyano-6'-(methylsulfonyl)-[3,4'-bipyridin]-2'-yl)amino)-3-(2,2-dimethyl-2,3-dihydro-[1,4]dioxino[2,3-b]pyridin-6-yl)pyridin-2-yl)acetamid;
N-(5-(2,2-Dimethyl-2,3-dihydro-[1,4]dioxino[2,3-b]pyridin-6-yl)-4-((4-(5-fluorpyridazin-4-yl)-6-(methylsulfonyl)pyridin-2-yl)amino)pyridin-2-yl)acetamid;
N-(5-(2,2-Dimethyl-2,3-dihydro-[1,4]dioxino[2,3-b]pyridin-6-yl)-4-((4-fluor-6'-(methylsulfonyl)-[3,4'-bipyridin]-2'-yl)amino)pyridin-2-yl)acetamid;
N-(5-(2,2-Dimethyl-2,3-dihydro-[1,4]dioxino[2,3-b]pyridin-6-yl)-4-((6'-(methylsulfonyl)-[2,4'-bipyridin]-2'-yl)amino)pyridin-2-yl)acetamid;
N-(5-(2,2-Dimethyl-2,3-dihydro-[1,4]dioxino[2,3-b]pyridin-6-yl)-4-((6-(methylsulfonyl)-4-(thiazol-4-yl)pyridin-2-yl)amino)pyridin-2-yl)acetamid;
N-(4-((4-(1-Cyanocyclopropyl)-6-(methylsulfonyl)pyridin-2-yl)amino)-5-(2,2-dimethyl-2,3-dihydro-[1,4]dioxino[2,3-b]pyridin-6-yl)pyridin-2-yl)acetamid;
N-(4-((4-((3R)-3-Cyano-2-azabicyclo[3,1,0]hexan-2-yl)-6-(methylsulfonyl)pyridin-2-yl)-amino)-5-(2,2-dimethyl-2,3-dihydro-[1,4]dioxino[2,3-b]pyridin-6-yl)pyridin-2-yl)acetamid;
N-(4-((4-((3S)-3-Cyano-2-azabicyclo[3,1,0]hexan-2-yl)-6-(methylsulfonyl)pyridin-2-yl)-amino)-5-(2,2-dimethyl-2,3-dihydro-[1,4]dioxino[2,3-b]pyridin-6-yl)pyridin-2-yl)acetamid;
N-(5-(2,2-Dimethyl-2,3-dihydro-[1,4]dioxino[2,3-b]pyridin-6-yl)-4-((4-(2-methyl-2-morpholinopropoxy)-6-(methylsulfonyl)pyridin-2-yl)amino)pyridin-2-yl)acetamid:
N-(5-(2,2-Dimethyl-2,3-dihydro-[1,4]dioxino[2,3-b]pyridin-6-yl)-4-((4-(2-methoxy-2-methylpropoxy)-6-(methylsulfonyl)pyridin-2-yl)amino)pyridin-2-yl)acetamid;
N-(5-(2,2-Dimethyl-2,3-dihydro-[1,4]dioxino[2,3-b]pyridin-6-yl)-4-((4-(2-hydroxy-2-methylpropoxy)-6-(methylsulfonyl)pyridin-2-yl)amino)pyridin-2-yl)acetamid;
N-(5-(2,2-Dimethyl-2,3-dihydro-[1,4]dioxino[2,3-b[pyridin-6-yl)-4-((4-(3,3-dimethyl-morpholino)-6-(methylsulfonyl)pyridin-2-yl)amino)pyridin-2-yl)acetamid;
N-(5-(2,2-Dimethyl-2,3-dihydro-[1,4]dioxino[2,3-b]pyridin-6-yl)-4-((4-isopropyl-6'-(methylsulfonyl)-[3,4'-bipyridin]-2'-yl)amino)pyridin-2-yl)acetamid;
N-(5-(2,2-Dimethyl-2,3-dihydro-[1,4]dioxino[2,3-b]pyridin-6-yl)-4-((4-(1-hydroxyethyl)-6'-(methylsulfonyl)-[3,4'-bipyridin]-2'-yl)amino)pyridin-2-yl)acetamid;
N-(5-(2,2-Dimethyl-2,3-dihydro-[1,4]dioxino[2,3-b]pyridin-6-yl)-4-((4-(1-methoxyethyl)-6'-(methylsulfonyl)-[3,4'-bipyridin]-2'-yl)amino)pyridin-2-yl)acetamid;
N-(5-(2,2-Dimethyl-2,3-dihydro-[1,4]dioxino[2,3-b]pyridin-6-yl)-4-((6-(methylsulfonyl)-[1,3]dioxolo[4,5-c]pyridin-4-yl)amino)pyridin-2-yl)acetamid;
N-(5-(2,2-Dimethyl-2,3-dihydro-[1,4]dioxino[2,3-b]pyridin-6-yl)-4-((7-(methylsulfonyl)-2,3-dihydro-[1,4]dioxino[2,3-c]pyridin-3-yl)amino)pyridin-2-yl)acetamid;
N-(5-(2,2-Dimethyl-2,3-dihydro-[1,4]dioxino[2,3-b]pyridin-6-yl)-4-((6-(methylsulfonyl)-4-(trifluormethyl)pyridin-2-yl)amino)pyridin-2-yl)acetamid;
N-(4-((4-(Difluormethoxy)-6-(methylsulfonyl)pyridin-2-yl)amino)-5-(2,2-dimethyl-2,3-dihydro-[1,4]dioxino[2,3-b]pyridin-6-yl)pyridin-2-yl)acetamid;
N-(4-((4-Cyclobutoxy-6-(methylsulfonyl)pyridin-2-yl)amino)-5-(2,2-dimethyl-2,3-dihydro-[1,4]dioxino[2,3-b]pyridin-6-yl)pyridin-2-yl)acetamid;
N-(5-(2,2-Bis(methyl-d3)-2,3-dihydro-[1,4]dioxino[2,3-b]pyridin-6-yl)-4-((6-(methylsulfonyl)-4-(tetrahydro-2H-pyran-4-yl)pyridin-2-yl)amino)pyridin-2-yl)acetamid;
N-(5-(2,2-Bis(methyl-d3)-2,3-dihydro-[14|dioxino[2,3-b]pyridin-6-yl)-4-((4-(methoxy-d3)-6-(methylsulfonyl)pyridin-2-yl)amino)pyridin-2-yl acetamid;
N-(5-(4,4-Dimethyl-4,5-dihydrothiazol-2-yl)-4-((6-(methylsulfonyl)pyridin-2-yl)amino)-pyridin-2-yl)acetamid;
N-(5-(4,4-Dimethyl-4,5-dihydrothiazol-2-yl)-4-((6-(methylsulfonyl)-4-(tetrahydro-2H-pyran-4-yl)pyridin-2-yl)amino)pyridin-2-yl)acetamid;
N-(4-((4-Isopropoxy-6-(methylsulfonyl)pyridin-2-yl)amino)-5-(1-methyl-1,3a,4,6,7,7a-hexahydropyrano[4,3-c]pyrazol-3-yl)pyridin-2-yl)acetamid;
N-(5-(6,7-Dihydro-4H-pyrano[4,3-d]thiazol-2-yl)-4-((4-methyl-6-(methylsulfonyl)pyridin-2-yl)amino)pyridin-2-yl)acetamid;
N-(4-((4-Isopropoxy-6-(methylsulfonyl)pyridin-2-yl)amino)-3-(5-methyl-4,5,6,7-tetra-hydropyrazolo[1,5-a]pyrazin-2-yl)pyridin-2-yl)acetamid;
N-(5-([1,3]Dioxolo[4,5-c]pyridin-6-yl)-4-((4-methyl-6-(methylsulfonyl)pyridin-2-yl)-amino)pyridin-2-yl)acetamid;
N-(5-([1,3]Dioxolo[4,5-c]pyridin-4-yl)-4-((4-methyl-6-(methylsulfonyl)pyridin-2-yl)-amino)pyridin-2-yl)acetamid;
N-(5-(Benzo[d][1,3]dioxol-4-yl)-4-((4-methyl-6-(methylsulfonyl)pyridin-2-yl)amino)-pyridin-2-yl)acetamid;
N-(5-(5-Methyl-4,5,6,7-tetrahydrothiazolo[5,4-c]pyridin-2-yl)-4-((4-methyl-6-(methylsulfonyl)pyridin-2-yl)amino)pyridin-2-yl)acetamid;
N-(5-(Imidazo[1,2-a]pyrimidin-2-yl)-4-((4-methyl-6-(methylsulfonyl)pyridin-2-yl)amino)-pyridin-2-yl)acetamid;
N-(5-(1-Methyl-1,4-dihydrochromeno[4,3-c]pyrazol-6-yl)-4-((4-methyl-6-(methylsulfonyl)pyridin-2-yl)amino)pyridin-2-yl)acetamid;
N-(5-(imidazo[2',1':2,3]thiazolo[5,4-b]pyridin-7-yl)-4-((4-methyl-6-(methylsulfonyl)-pyridin-2-yl)amino)pyridin-2-yl)acetamid;
N-(5-(1-Methyl-1,4-dihydropyrazolo[3',4':4,5]pyrano[2,3-b]pyridin-7-yl)-4-((4-methyl-6-(methylsulfonyl)pyridin-2-yl)amino)pyridin-2-yl)acetamid;
N-(4'-((4,4-Dioxido-2,3-dihydro-[1,4]oxathiino[3,2-b]pyridin-6-yl)amino)-[2,3'-bipyridin]-6'-yl)acetamid:
N-(4-((4,4-Dioxido-2,3-dihydro-[1,4]oxathiino[3,2-b]pyridin-6-yl)amino)-5-(1-methyl-1H-pyrazol-3-yl)pyridin-2-yl)acetamid;
N-(5-(Imidazo[1,2-b]pyridazin-6-yl)-4-((4-methyl-6-(methylsulfonyl)pyridin-2-yl)amino)-pyridin-2-yl)acetamid;
N-(5-(1-Methyl-1H-pyrazol-3-yl)-4-((7-(methylsulfonyl)-3.4-dihydro-2H-pyrano[3,2-c]-pyridin-5-yl)amino)pyridin-2-yl)acetamid;
N-(4-((5,5-Dioxido-3,4-dihydro-2H-[1,4]oxathiepino[3,2-b]pyridin-7-yl)amino)-5-(1-methyl-1H-pyrazol-3-yl)pyridin-2-yl)acetamid;
N-(4-((4-(2-Hydroxypropan-2-yl)-6-(methylsulfonyl)pyridin-2-yl)amino)-5-(1-methyl-1H-pyrazol-3-yl)pyridin-2-yl)acetamid;
N-(5-(1-Methyl-1H-pyrazol-3-yl)-4-((6-(methylsulfonyl)-4-(tetrahydrofuran-3-yl)pyridin-2-yl)amino)pyridin-2-yl)acetamid;
N-(4-((4-(3-(Hydroxymethyl)tetrahydrofuran-3-yl)-6-(methylsulfonyl)pyridin-2-yl)-amino)-3-(1-methyl-1H-pyrazol-3-yl)pyridin-2-yl)acetamid;
N-(4-((4-(1-Hydroxycyclopropyl)-6-(methylsulfonyl)pyridin-2-yl)amino)-5-(1-methyl-1H-pyrazol-3-yl)pyridin-2-yl)acetamid;
N-(4-((4-(2-Hydroxy-2-methylpropoxy)-6-(methylsulfonyl)pyridin-2-yl)amino)-5-(1-methyl-1H-pyrazol-3-yl)pyridin-2-yl)acetamid;
N-(4-((4-(3-Hydroxy-2,2-dimethylpropoxy)-6-(methylsulfonyl)pyridin-2-yl)amino)-5-(1-methyl-1H-pyrazol-3-yl)pyridin-2-yl)acetamid;
N-(5-(Imidazo[1,5-b]pyridazin-2-yl)-4-((4-methyl-6-(methylsulfonyl)pyridin-2-yl)amino)-pyridin-2-yl)acetamid;
N-(4-((4-(3-Hydroxytetrahydrofuran-3-yl)-6-(methylsulfonyl)pyridin-2-yl)amino)-5-(1-methyl-1H-pyrazol-3-yl)pyridin-2-yl)acetamid;
N-(5-(1-Methyl-1H-pyrazol-3-yl)-4-((4-(1-methyl-1H-pyrazol-3-yl)-6-(methylsulfonyl)-pyridin-2-yl)amino)pyridin-2-yl)acetamid;
N-(4-((4-((1-Hydroxycyclopropyl)methoxy)-6-(methylsulfonyl)pyridin-2-yl)amino)-5-(1-methyl-1H-pyrazol-3-yl)pyridin-2-yl)acetamid;
N-(4-((4-((1-Methoxycyclopropyl)methoxy)-6-(methylsulfonyl)pyridin-2-yl)amino)-5-(1-methyl-1H-pyrazol-3-yl)pyridin-2-yl)acetamid;
N-(5-(1-Methyl-1H-pyrazol-3-yl)-4-((6-(methylsulfonyl)-4-morpholinopyridin-2-yl)-amino)pyridin-2-yl)acetamid;
N-(5-(Benzol|d|thiazol-2-yl)-4-((4-(3-(hydroxymethyl)tetrahydrofuran-3-yl)-6-(methylsulfonyl)pyridin-2-yl)amino)pyridin-2-yl)acetamid;
N-(4-((1,1-Dioxido-3,4-dihydro-2H-thiopyrano[2,3-b]pyridin-7-yl)amino)-5-(1-methyl-1H-pyrazol-3-yl)pyridin-2-yl)acetamid;
N-(5-(1-Methyl-1H-pyrazol-3-yl)-4-((7-(methylsulfonyl)-3.4-dihydro-2H-pyrano[3,2-c]-pyridin-5-yl)amino)pyridin-2-yl)acetamid;
N-(5-(1-Methyl-1H-pyrazol-3-yl)-4-((6-(methylsulfonyl)-[1,3]dioxolo[4,5-c|pyridin-4-yl)-amino)pyridin-2-yl)acetamid;
N-(5-(1-Methyl-1H-pyrazol-3-yl)-4-((6-(methylsulfonyl)imidazo[1,2-a]pyrazin-8-yl)-amino)pyridin-2-yl)acetamid;
N-(5-(1-Methyl-1H-pyrazol-3-yl)-4-((5-(methylsulfonyl)-3.4-dihydro-2H-pyrano[3,2-c]-pyridin-7-yl)amino)pyridin-2-yl)acetamid;
N-(5-(2,6-Dimethylmorpholino)-4'-((4-(2-hydroxypropan-2-yl)-6-(methylsulfonyl)-pyridin-2-yl)amino)-[2,3'-bipyridin]-6'-yl)acetamid;
N-(5-(2,6-Dimethylmorpholino)-4'-((6-(methylsulfonyl)-4-(tetrahydrofuran-3-yl)pyridin-2-yl)amino)-[2,3'-bipyridin]-6'-yl)acetamid;
N-(5-(2,6-Dimethylmorpholino)-4'-((4-(3-(hydroxymethyl)tetrahydrofuran-3-yl)-6-(methylsulfonyl)pyridin-2-yl)amino)-[2,3'-bipyridin]-6'-yl)acetamid;
N-(5-(2,6-Dimethylmorpholino)-4'-((4-(1-hydroxycyclopropyl)-6-(methylsulfonyl)-pyridin-2-yl)amino)-[2,3'-bipyridin]-6'-yl)acetamid;
N-(5-(2,6-Dimethylmorpholino)-4'-((4-(2-hydroxy-2-methylpropoxy)-6-(methylsulfonyl)-pyridin-2-yl)amino)-[2,3'-bipyridin]-6'-yl)acetamid;
N-(5-(2,6-Dimethylmorpholino)-4'-((4-(3-hydroxy-2,2-dimethylpropoxy)-6-(methylsulfonyl)pyridin-2-yl)amino)-[2,3'-bipyridin]-6'-yl)acetamid;
N-(5-(2,6-Dimethylmorpholino)-4'-((4-(3-methoxytetrahydrofuran-3-yl1)-6-(methylsulfonyl)pyridin-2-yl)amino)-[2,3'-bipyridin]-6'-yl)acetamid;
N-(5-(2,6-Dimethylmorpholino)-4'-((4-(3-hydroxytetrahydrofuran-3-yl)-6-(methylsulfonyl)pyridin-2-yl)amino)-[2,3'-bipyridin]-6'-yl)acetamid;
N-(5-(2,6-Dimethylmorpholino)-4'-((4-(1-methyl-1H-pyrazol-3-yl)-6-(methylsulfonyl)-pyridin-2-yl)amino)-[2,3'-bipyridin]-6'-yl)acetamid;
N-(5-(2,6-Dimethylmorpholino)-4'-((4-((1-hydroxycyclopropyl)methoxy)-6-(methylsulfonyl)pyridin-2-yl)amino)-[2,3'-bipyridin]-6'-yl)acetamid;
N-(5-(2,6-Dimethylmorpholino)-4'-((4-((1-methoxycyclopropyl)methoxy)-6-(methylsulfonyl)pyridin-2-yl)amino)-[2,3'-bipyridin]-6'-yl)acetamid;
N-(5-(2,6-Dimethylmorpholino)-4'-((6-(methylsulfonyl)-4-morpholinopyridin-2-yl)-amino)-[2,3'-bipyridin]-6'-yl)acetamid;
N-(5-(2,6-Dimethylmorpholino)-4'-((5,5-dioxido-3.4-dihydro-2H-[1,4]oxathiepino[3,2-b]-pyridin-7-yl)amino)-[2,3'-bipyridin]-6'-yl)acetamid;
N-(5-(2,6-Dimethylmorpholino)-4'-((1,1-dioxido-3.4-dihydro-2H-thiopyrano[2,3-b]-pyridin-7-yl)amino)-[2,3'-bipyridin]-6'-yl)acetamid;
N-(5-(2,6-Dimethylmorpholino)-4'-((7-(methylsulfonyl)-3,4-dihydro-2H-pyrano[3,2-c]-pyridin-5-yl)amino)-[2,3'-bipyridin]-6'-yl)acetamid;
N-(5-(2,6-Dimethylmorpholino)-4'-((6-(methylsulfonyl)-[1,3]dioxolo[4,5-c]pyridin-4-yl)-amino)-[2,3'-bipyridin]-6'-yl)acetamid;
N-(5-(2,6-Dimethylmorpholino)-4'-((6-(methylsulfonyl)imidazo[1,2-a]pyrazin-8-yl)-amino)-[2,3'-bipyridin]-6'-yl)acetamid;
N-(5-(2,6-Dimethylmorpholino)-4'-((5-(methylsulfonyl)-3,4-dihydro-2H-pyrano[3,2-c]-pyridin-7-yl)amino)-[2,3'-bipyridin]-6'-yl)acetamid;
N-(5-(6,7-Dihydro-4H-pyrano[4,3-d]thiazol-2-yl)-4-((4-(3-(hydroxymethyl)-tetrahydrofuran-3-yl)-6-(methylsulfonyl)pyridin-2-yl)amino)pyridin-2-yl)acetamid;
N-(5-(Benzo[d][1,3]dioxol-4-yl)-4-((4-(3-(hydroxymethyl)tetrahydrofuran-3-yl)-6-(methylsulfonyl)pyridin-2-yl)amino)pyridin-2-yl)acetamid;
N-(5-(Benzo|d]isoxazol-3-yl)-4-((4-(3-(hydroxymethyl)tetrahydrofuran-3-yl)-6-(methylsulfonyl)pyridin-2-yl)amino)pyridin-2-yl)acetamid;
N-(5-(Benzo[d]isothiazol-3-yl)-4-((4-(3-(hydroxymethyl)tetrahydrofuran-3-yl)-6-(methylsulfonyl)pyridin-2-yl)amino)pyridin-2-yl)acetamid;
N-(5-(Benzo|b]thiophen-2-yl)-4-((4-(3-(hydroxymethyl)tetrahydrofuran-3-yl)-6-(methylsulfonyl)pyridin-2-yl)amino)pyridin-2-yl)acetamid;
N-(4-((4-(3-(Hydroxymethyl)tetrahydrofuran-3-yl)-6-(methylsulfonyl)pyridin-2-yl)-amino)-3-(imidazo[1,2-a]pyrimidin-2-yl)pyridin-2-yl)acetamid;
N-(4-((4-(3-(Hydroxymethyl)tetrahydrofuran-3-yl)-6-(methylsulfonyl)pyridin-2-yl)-amino)-3-(1-methyl-1,4-dihydrochromeno(4,3-c]pyrazol-6-yl)pyridin-2-yl)acetamid;
N-(4-((4-(3-(Hydroxymethyl)tetrahydrofuran-3-yl)-6-(methylsulfonyl)pyridin-2-yl)-amino)-5-(imidazo(2',1':2,3]thiazolo[5,4-b]pyridin-7-yl)pyridin-2-yl)acetamid;
N-(4-((4-(3-(Hydroxymethyl)tetrahydrofuran-3-yl)-6-(methylsulfonyl)pyridin-2-yl)-amino)-3-(1-methyl-1,4-dihydropyrazolo[3'.4':4,5|pyrano[2,3-b]pyridin-7-yl)pyridin-2-yl)-acetamid;
N-(4-((4-(3-(Hydroxymethyl)tetrahydrofuran-3-yl)-6-(methylsulfonyl)pyridin-2-yl)-amino)-3-(6-oxo-1,6-dihydropyridazin-3-yl)pyridin-2-yl)acetamid;
N-(4-((4-(3-(Hydroxymethyl)tetrahydrofuran-3-yl)-6-(methylsulfonyl)pyridin-2-yl)-amino)-3-(6-oxopyridazin-1(6H)-yl)pyridin-2-yl)acetamid;
N-(5-(6-((25,6R)-2,6-Dimethylmorpholino)pyridazin-3-yl)-4-((4-(3-(hydroxymethyl)-tetrahydrofuran-3-yl)-6-(methylsulfonyl)pyridin-2-yl)amino)pyridin-2-yl)acetamid;
N-(4-((4-(3-(Hydroxymethyl)tetrahydrofuran-3-yl)-6-(methylsulfonyl)pyridin-2-yl)-amino)-3-(3-oxo-8,9-dihydropyrano[4,3,2-de]phthalazin-2(3H)-yl)pyridin-2-yl)acetamid;
N-(4-((4-(3-(Hydroxymethyl)tetrahydrofuran-3-yl)-6-(methylsulfonyl)pyridin-2-yl)-amino)-3-(7-methyl-3-oxo-8,9-dihydro-3H-pyrido[4,3,2-de]phthalazin-2(7H)-yl)pyridin-2-yl)-acetamid;
N-(4'-((4-(3-(Hydroxymethyl)tetrahydrofuran-3-yl)-6-(methylsulfonyl)pyridin-2-yl)-amino)-5-(1-oxoisoindolin-2-yl)-[2,3'-bipyridin]-6'-yl)acetamid;
N-(5-(2,3-Dihydropyrazolo[5,1-b]oxazol-6-yl)-4-((4-(3-(hydroxymethyl)tetrahydrofuran-3-yl)-6-(methylsulfonyl)pyridin-2-yl)amino)pyridin-2-yl)acetamid;
N-(5-(2,2-Dimethyl-2,3-dihydropyrazolo[5,1-b]oxazol-6-yl)-4-((4-(3-(hydroxymethyl)-tetrahydrofuran-3-yl)-6-(methylsulfonyl)pyridin-2-yl)amino)pyridin-2-yl)acetamid;
N-(4-((4-(3-(Hydroxymethyl)tetrahydrofuran-3-yl)-6-(methylsulfonyl)pyridin-2-yl)-amino)-3-(5'H,7'H-spiro[cyclopropan-1,6'-pyrazolo[5,1-b][1,3]oxazin]-2'-y])pyridin-2-yl)-acetamid;
N-(5-(1H-Imidazol-2-yl)-4-((4-methyl-6-(methylsulfonyl)pyridin-2-yl)amino)pyridin-2-yl)acetamid;
N-(5-(4-(2-Hydroxypropan-2-yl)furan-2-yl)-4-((4-methyl-6-(methylsulfonyl)pyridin-2-yl)amino)pyridin-2-yl)acetamid;
N-(5-(Furan-2-yl)-4-((4-(3-methoxytetrahydrofuran-3-yl)-6-(methylsulfonyl)pyridin-2-yl)amino)pyridin-2-yl)acetamid;
N-(5-(1H-Benzo[d]imidazol-2-yl)-4-((4-methyl-6-(methylsulfonyl)pyridin-2-yl)amino)-pyridin-2-yl)acetamid;
N-(3-Fluor-5-(2-hydroxypropan-2-yl)-4'-((4-methyl-6-(methylsulfonyl)pyridin-2-yl)-amino)-[2,3'-bipyridin]-6'-yl)acetamid;
N-(3-Fluor-5-(2-hydroxypropan-2-yl)-4'-((4-methyl-6-(methylsulfonyl)pyridin-2-yl)-amino)-[2,3'-bipyridin]-6'-yl)acetamid;
N-(5-(2-Hydroxypropan-2-yl)-4-methyl-4'-((4-methyl-6-(methylsulfonyl)pyridin-2-yl)-amino)-[2,3'-bipyridin]-6'-yl)acetamid;
N-(5-(2,6-Dimethylmorpholino)-4'-((6-(methylsulfonyl)-4-((tetrahydrofuran-3-yl)-methoxy)pyridin-2-yl)amino)-[2,3'-bipyridin]-6"-yl)acetamid;
N-(5-(2,6-Dimethylmorpholino)-4'-((6-(methylsulfonyl)-4-(((tetrahydrofuran-3-yl)oxy)-methyl)pyridin-2-yl)amino)-[2,3'-bipyridin]-6'-yl)acetamid;
N-(5-(3-Methoxy-1-methyl-1H-pyrazol-4-yl)-4-((4-methyl-6-(methylsulfonyl)pyridin-2-yl)amino)pyridin-2-yl)acetamid;
N-(4-((4-(3-(Hydroxymethyl)tetrahydrofuran-3-yl)-6-(methylsulfonyl)pyridin-2-yl)-amino)-3-(3-methoxy-1-methyl-1H-pyrazol-4-yl)pyridin-2-yl)acetamid;
N-(5-(6,7-Dihydro-4H-furo|3,2-c]pyran-2-yl)-4-((4-methyl-6-(methylsulfonyl)pyridin-2-yl)amino)pyridin-2-yl)acetamid;
N-(5-(5-Methyl-4,5,6,7-tetrahydrofuro[3,2-c]pyridin-2-yl)-4-((4-methyl-6-(methylsulfonyl)pyridin-2-yl)amino)pyridin-2-yl)acetamid;
N-(5-(Benzo|d]oxazol-2-yl)-4-((4-(3-(hydroxymethyl)tetrahydrofuran-3-yl)-6-(methylsulfonyl)pyridin-2-yl)amino)pyridin-2-yl)acetamid;
N-(5-(2,2-Dimethyl-3-oxo-3,4-dihydro-2H-pyrido[3,2-b][1,4]oxazin-6-yl)-4-((4-methyl-6-(methylsulfonyl)pyridin-2-yl)amino)pyridin-2-yl)acetamid;
N-(4-((4-Methyl-6-(methylsulfonyl)pyridin-2-yl)amino)-5-(pyrazolo[1,5-a]pyrazin-2-yl)-pyridin-2-yl)acetamid;
N-(4-((4-(2-Methoxyethoxy)-6-(methylsulfonyl)pyridin-2-yl)amino)-5-(pyrazolo[1,5-a]-pyrazin-2-yl)pyridin-2-yl)acetamid;
N-(4-((4-Methyl-6-(methylsulfonyl)pyridin-2-yl)amino)-5-(pyrazolo[1,5-c|pyrimidin-2-yl)pyridin-2-yl)acetamid;
N-(4-((4-(2-Methoxyethoxy)-6-(methylsulfonyl)pyridin-2-yl)amino)-3-(pyrazolo[1,5-c]-pyrimidin-2-yl)pyridin-2-yl)acetamid;
N-(4-((4-Methyl-6-(methylsulfonyl)pyridin-2-yl)amino)-5-(pyrazolo[1,5-a]pyrimidin-2-yl)pyridin-2-yl)acetamid;
N-(4-((4-(2-Methoxyethoxy)-6-(methylsulfonyl)pyridin-2-yl)amino)-5-(pyrazolo[1,5-a]-pyrimidin-2-yl)pyridin-2-yl)acetamid;
N-(4-((4-Methyl-6-(methylsulfonyl)pyridin-2-yl)amino)-5-(pyrazolo[1,5-a]pyrimidin-5-yl)pyridin-2-yl)acetamid;
N-(4-((4-(2-Methoxyethoxy)-6-(methylsulfonyl)pyridin-2-yl)amino)-3-(pyrazolo[1,5-a]-pyrimidin-5-yl)pyridin-2-yl)acetamid; oder
N-(5-(2,2-Dimethyl-2,3-dihydro-1H-pyrido[2,3-b][1,4]oxazin-6-yl)-4-((6-(methylsulfonyl)-4-(tetrahydro-2H-pyran-4-yl)pyridin-2-yl)amino)pyridin-2-yl)acetamid.

14. Pharmazeutische Zusammensetzung, die eine oder mehrere Verbindungen nach einem der Ansprüche 1 bis 13 oder ein pharmazeutisch annehmbares Salz davon und einen pharmazeutisch annehmbaren Hilfsstoff umfasst.

15. Verbindung nach einem der Ansprüche 1 bis 13 zur Verwendung bei der Behandlung einer Entzündungs- oder Autoimmunerkrankung.

## Revendications

1. Composé de formule (1) 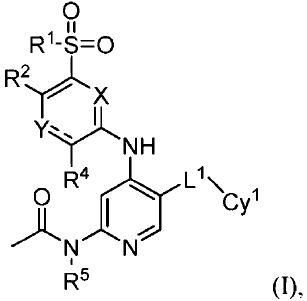 ou un stéréoisomère ou un sel pharmaceutiquement acceptable de celui-ci, dans lequel :
X est N ou CH ;
Y est N, NR³ ou CR³ ;
L¹ est une liaison directe ;
R¹ est -C₁₋₆ alkyle, -haloC₁₋₆ alkyle, -C₁₋₆ alcoxy, - haloC₁₋₆ alcoxy, -C₃₋₆ cycloalkyle, aryle ou -NR^{c}R^{d} ;
chacun de R², R³ et R⁴ est indépendamment hydrogène, cyano, halogène, -C₁₋₆ alkyle, -C₂₋₆ alcényle, -C₂₋₆ alcynyle, -C₃₋₆ cycloalkyle, hétérocyclyle, aryle, hétéroaryle, oxo, - NO₂, -OR^{e}, -SO₂R^{e}, -COR^{e}, -CO₂R^{e}, -CONR^{e}R^{f}, -C(=NR^{e})NR^{f}R^{g}, -NR^{e}R^{f}, -NR^{e}COR^{f}, ⁻NR^{e}CONR^{f}R^{g}, -NR^{e}CO₂R^{f}, -NR^{e}SONR^{f}R^{g}, -NR^{e}SO₂NR^{r}R^{g} ou - NR^{e}SO₂R^{f},
dans lequel chacun des -C₁₋₆ alkyle, -C₂₋₆ alcényle, -C₂₋₆ alcynyle, -C₃₋₆ cycloalkyle, hétérocyclyle, aryle ou hétéroaryle est indépendamment non substitué ou substitué par au moins un substituant sélectionné parmi
i) cyano, -oxo-, halogène, -NR^{m}Rⁿ, -OR^{h}, - C(O)NR^{m}Rⁿ ;
ii) hétérocyclyle non substitué ou substitué, non substitué ou substitué par au moins un substituant sélectionné indépendamment parmi cyano, -oxo, halogène, hydroxy, -NR^{m}Rⁿ, -C₁₋₆ alkyle substitué ou non substitué, -C₁₋₆ alcoxy substitué ou non substitué ou -C(O)NR^{m}Rⁿ ; ou
iii) C₁₋₆ alkyle non substitué ou substitué par au moins une substitution sélectionnée indépendamment parmi cyano, halogène, hydroxy, -NH₂ ou C₁₋₆ alcoxy ;
dans lequel R^{h} est hydrogène, hydroxy, -NH₂, -C₁₋₆ alkyle, C₁₋₆ alkyle substitué par hydroxy, ou hétérocyclyle,
R^{e}, R^{r} et R^{g} sont chacun indépendamment hydrogène, -C₁₋₆ alkyle, -C₁₋₆ alcoxy, -C₂₋₆ alcényle, -C₂₋₆ alcynyle, cycloalkyle, hétérocyclyle, aryle ou hétéroaryle, dans lequel chacun des -C₁₋₆ alkyle, -C₂₋₆ alcényle, -C₂₋₆ alcynyle, cycloalkyle, hétérocyclyle, aryle ou hétéroaryle est éventuellement substitué indépendamment par un à trois substituants sélectionnés parmi cyano, -oxo-, halogène, hydroxy, -NR^{m}Rⁿ, -C₁₋₆ alkyle, -C₁₋₆ alcoxy, -C₃₋₆ cycloalkyle non substitué ou substitué par halogène, hydroxy ou -C₁₋₆ alcoxy, -C(O)NR^{m}Rⁿ ou hétérocyclyle ;
R⁵ est hydrogène ou C₁₋₆ alkyle ;
Cy¹ est un aryle à 6 à 12 chaînons ou un hétéroaryle à 5 à 14 chaînons, ou un hétérocyclyle à 5 à 14 chaînons, chacun étant non substitué ou substitué par au moins un substituant Rⁱ,
Rⁱ est indépendamment halogène, cyano, -C₁₋₆ alkyle, -C₂₋₆ alcényle, -C₂₋₆ alcynyle, -C₃₋₆ cycloalkyle, hétérocyclyle, aryle, hétéroaryle, oxo, cyano (-CN), -NO₂, -OR^{j}, -SO₂R^{j}, - COR^{j}, -CO₂R^{k}, -CONR^{j}R^{k}, -C(=NR^{j})NR^{k}R^{l}, -NR^{j}R^{k}, -NR^{j}COR^{k}, - NR^{j}CONR^{k}R^{l}, -NR^{j}CO₂R^{k}, -NRⁱSONR^{k}R^{l}, -NR^{j}SO₂NR^{k}R^{l} ou -NR^{j}SO₂R^{k},
dans lequel chacun des -C₁₋₆ alkyle, -C₂₋₆ alcényle, -C₂₋₆ alcynyle, -C₃₋₆ cycloalkyle, hétérocyclyle, aryle ou hétéroaryle est non substitué ou substitué par halogène, - OR^{m}, -C (O)R^{m}, -NR^{m}Rⁿ, -C₁₋₆ alkyle, C₁₋₆ alcoxy-, C₁₋₆ alkyle substitué par -C₁₋₆ alcoxy ou -oxo- ;
R^{j}, R^{k}, R^{l}, R^{m}, Rⁿ sont chacun indépendamment hydrogène, -C₁₋₆ alkyle, C₁₋₆ alcoxy-C₁₋₆ alkyle-, -C₂₋₆ alcényle, -C₂₋₆ alcynyle, cycloalkyle, hétérocyclyle, aryle ou hétéroaryle ;
ou (R¹ et R²), ou (R² et R³), ou (R³ et R⁴), ensemble avec les atomes auxquels ils sont attachés, forment un système cyclique fusionné, ledit système cyclique fusionné comprend 0-4 hétéroatomes sélectionnés parmi l'oxygène (O), l'azote (N) ou le soufre (S) en tant que membre(s) du cycle et est éventuellement et indépendamment substitué par halogène, -C₁₋₆ alkyle, -C₁₋₆ alcoxy, C₁₋₆ alkyle substitué par halogène, C₁₋₆ alcoxy substitué par halogène ou -C₃₋₆ cycloalkyle ;
l'un quelconque desdits alkyle ou alcoxy est éventuellement enrichi en deutérium.

2. Composé selon la revendication 1, dans lequel le composé est de formule (1-A) 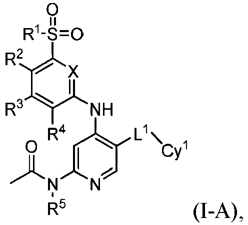 ou un stéréoisomère ou un sel pharmaceutiquement acceptable de celui-ci, dans lequel :
X est N ou CH ;
L¹ est une liaison directe ;
R¹ est -C₁₋₆ alkyle, -haloC₁₋₆ alkyle, -C₁₋₆ alcoxy, - haloC₁₋₆ alcoxy, -C₃₋₆ cycloalkyle, aryle ou -NR^{m}Rⁿ ;
chacun de R², R³ et R⁴ est indépendamment hydrogène, halogène, -C₁₋₆ alkyle, -C₂₋₆ alcényle, -C₂₋₆ alcynyle, -C₃₋₆ cycloalkyle, hétérocyclyle, aryle, hétéroaryle, -oxo-, -CN, -NO₂, -OR^{e}, -SO₂R^{e}, -COR^{e}, -CO₂R^{e}, -CONR^{e}R^{f}, -C(=NR^{e})NR^{f}R^{g}, - NR^{e}R^{f}, -NR^{e}COR^{f}, -NR^{e}CONR^{f}R^{g}, -NR^{e}CO₂R^{f}, -NR^{e}SONR^{f}R^{g}, -NR^{e}SO₂NR^{f}R⁹ ou -NR^{e}SO₂R^{f},
dans lequel chacun des -C₁₋₆ alkyle, -C₂₋₆ alcényle, -C₂₋₆ alcynyle, -C₃₋₆ cycloalkyle, hétérocyclyle, aryle ou hétéroaryle est indépendamment non substitué ou substitué par au moins un substituant sélectionné parmi cyano, oxo, halogène, C₁₋₆ alkyle non substitué ou substitué par halogène, -C₁₋₆ alkyle substitué par hydroxy (de préférence, hydroxyméthyle, hydroxyéthyle), -OR^{h}, -C(O)NR^{m}Rⁿ, -NH₂, C₁₋₆ alkyle substitué par NH₂ ou -C₁₋₆ alkyle substitué par C₁₋₆ alcoxy ;
dans lequel R^{h} est hydrogène, alkyle, hydroxy-C₁₋₆ alkyle ou hétérocyclyle,
R^{e}, R^{f} et R^{g} sont chacun indépendamment hydrogène, -C₁₋₆ alkyle, -C₁₋₆ alcoxy, -C₂₋₆ alcényle, -C₂₋₆ alcynyle, cycloalkyle, hétérocyclyle, aryle ou hétéroaryle, dans lequel chacun des -C₁₋₆ alkyle, -C₂₋₆ alcényle, -C₂₋₆ alcynyle, cycloalkyle, hétérocyclyle, aryle ou hétéroaryle est éventuellement substitué indépendamment par un à trois substituants sélectionnés parmi halogène, hydroxy, cyano ou -C₁₋₆ alcoxy ; -C₃₋₆ cycloalkyle non substitué ou substitué par halogène, hydroxy ou C₁₋₆ alcoxy, ou hétérocyclyle ;
R⁵ est hydrogène ou C₁₋₆ alkyle ;
Cy¹ est un aryle à 6 à 12 chaînons ou un hétéroaryle à 5 à 14 chaînons, ou un hétérocyclyle à 5 à 14 chaînons, chacun étant non substitué ou substitué par au moins un substituant Rⁱ,
Rⁱ est indépendamment halogène, cyano, -C₁₋₆ alkyle, -C₂₋₆ alcényle, -C₂₋₆ alcynyle, -C₃₋₆ cycloalkyle, hétérocyclyle, aryle, hétéroaryle, oxo, -CN, -NO₂, -OR^{j}, -SO₂R^{j}, -COR^{j}, - CO₂R^{k}, -CONR^{j}R^{k}, -C (=NR^{j}) NR^{k}R^{l}, -NR^{j}R^{k}, -NR^{j}COR^{k}, -NR^{j}CONR^{k}R^{l}, - NR^{j}CO₂R^{k}, -NR^{j}SONR^{k}R^{l}, -NR O₂NR^{k}R^{l} ou -NR^{j}SO₂R^{k},
dans lequel chacun desdits -C₁₋₆ alkyle, -C₂₋₆ alcényle, -C₂₋₆ alcynyle, -C₃₋₆ cycloalkyle, hétérocyclyle, aryle ou hétéroaryle est non substitué ou substitué par halogène, OR^{m}, C (O)R^{m}, -NR^{m}Rⁿ, -C₁₋₆ alkyle, C₁₋₆ alcoxy-, C₁₋₆ alcoxy-C₁₋₆ alkyle- ou oxo ;
R^{j}, R^{k}, R^{l}, R^{m}, Rⁿ sont chacun indépendamment hydrogène, -C₁₋₆ alkyle, -C₁₋₆ alkyle substitué par C₁₋₆ alcoxy, -C₂₋₆ alcényle, -C₂₋₆ alcynyle, cycloalkyle, hétérocyclyle, aryle ou hétéroaryle ;
ou (R¹ et R²), ou (R² et R³), ou (R³ et R⁴), ensemble avec les atomes auxquels ils sont attachés, forment un système cyclique fusionné, ledit système cyclique fusionné comprend 0-4 hétéroatomes sélectionnés parmi l'oxygène, l'azote ou le soufre en tant que membre(s) du cycle et est éventuellement et indépendamment substitué par halogène, -C₁₋₆ alkyle, -C₁₋₆ alcoxy, -haloC₁₋₆ alkyle, -haloC₁₋₆ alcoxy ou -C₃₋₆ cycloalkyle ;
l'un quelconque desdits alkyle ou alcoxy est éventuellement enrichi en deutérium.

3. Composé selon la revendication 1 ou la revendication 2, dans lequel R¹ est -C₁₋₃ alkyle, -NR^{c}R^{d} ou - C₃₋₆ cycloalkyle, de préférence -NH₂, méthyle, éthyle, propyle, isopropyle, cyclopropyle ou cyclopentyle.

4. Composé selon la revendication 1 ou la revendication 2, dans lequel R² et R⁴ sont chacun indépendamment hydrogène, halogène, -C₁₋₆ alkyle ou -C₁₋₆ alcoxy, de préférence hydrogène, fluoro, méthyle, méthoxy, éthoxy ou isopropoxy.

5. Composé selon l'une quelconque des revendications 1 à 4, dans lequel R³ est
- hydrogène ;
- cyano ;
- halogène ;
- -C₁₋₄ alkyle non substitué ou substitué par au moins un substituant sélectionné indépendamment parmi halogène, hétérocyclyle à 3 à 6 chaînons ou -OR^{h}, dans lequel
ledit hétérocyclyle à 3 à 6 chaînons comprend un ou deux hétéroatomes sélectionnés parmi l'oxygène (O), l'azote (N) ou le soufre (S) en tant que membre(s) du cycle, non substitué ou substitué par au moins un substituant sélectionné indépendamment parmi cyano, -oxo, halogène, hydroxy, -NR^{m}Rⁿ, C₁₋₆ alkyle, -C₁₋₆ alcoxy ou -C(O)NR^{m}Rⁿ, dans lequel -C₁₋₆ alkyle ou C₁₋₆ alcoxy est substitué par au moins une substitution sélectionnée indépendamment parmi cyano, halogène, hydroxy, -NH₂, -C₁₋₆ alkyle ou C₁₋₆ alcoxy, et
R^{h} est hydrogène, alkyle ou hétérocyclyle (de préférence hétérocyclyle à 3 à 6 chaînons, par exemple, tétrahydrofuranyle, thiazolidinyle) ;
- -C₃₋₆ cycloalkyle, non substitué ou substitué par au moins un substituant sélectionné indépendamment parmi cyano, -oxo, halogène, -NR^{m}Rⁿ, hydroxy, -C₁₋₆ alkyle, -C₁₋₆ alcoxy ou -C(O)NR^{m}Rⁿ, dans lequel -C₁₋₆ alkyle ou C₁₋₆ alcoxy est substitué par au moins une substitution sélectionnée indépendamment parmi cyano, halogène, hydroxy, -NH₂, -C₁₋₆ alkyle ou C₁₋₆ alcoxy ;
- hétérocyclyle, de préférence hétérocyclyle saturé monocyclique à 4 à 6 chaînons, hétérocyclyle saturé monospiro, hétérocyclyle saturé bicyclique fusionné, ou hétérocyclyle saturé ponté, comprenant un ou deux hétéroatomes sélectionnés parmi l'oxygène (O), l'azote (N) ou le soufre (S) en tant que membre(s) du cycle, plus préférentiellement morpholinyle, tétrahydrofuranyle, tétrahydropyranyle, pyrrolidinyle, 1,4-dioxanyle, pipéridinyle ou azétidinyle, non substitué ou substitué par au moins un substituant sélectionné indépendamment parmi cyano, -oxo, halogène, hydroxy, -C₁₋₆ alkyle, alcoxy, -NR^{m}Rⁿ ou -C(O)NR^{m}Rⁿ, et dans lequel -C₁₋₆ alkyle ou -C₁₋₆ alcoxy est substitué par au moins une substitution sélectionnée indépendamment parmi cyano, halogène, hydroxy, -NH₂, -C₁₋₆ alkyle ou C₁₋₆ alcoxy ;
- -OR^{e}, dans lequel R^{e} est -C₁₋₆ alkyle, -C₃₋₆ cycloalkyle, hétérocyclyle à 3 à 6 chaînons (de préférence hétérocyclyle saturé monocyclique à 4 à 6 chaînons comprenant un hétéroatome oxygène en tant que membre du cycle) ou aryle, dans lequel
i) -C₁₋₆ alkyle est non substitué ou substitué par cyano, -oxo-, halogène, hydroxy, -NR^{m}Rⁿ, -C₁₋₆ alcoxy-, -C₃₋₆ cycloalkyle non substitué ou substitué par cyano, -oxo, halogène, hydroxy, -NR^{m}Rⁿ, -C₁₋₆ alkyle, C₁₋₆ alcoxy ou - C(O)NR^{m}Rⁿ, hétérocyclyle à 4 à 6 chaînons non substitué ou substitué par cyano, halogène, hydroxy, -C₁₋₆ alkyle ou -C₁₋₆ alcoxy ; et
- -C₃₋₆ cycloalkyle ou hétérocyclyle à 3 à 6 chaînons est non substitué ou substitué par cyano, -oxo, halogène, hydroxy, NR^{m}Rⁿ, C₁₋₆ alkyle, C₁₋₆ alcoxy ou -C(O)NR^{m}Rⁿ, dans lequel -C₁₋₆ alkyle ou C₁₋₆ alcoxy est substitué par au moins une substitution sélectionnée indépendamment parmi cyano, halogène, hydroxy, -NH₂, -C₁₋₆ alkyle ou C₁₋₆ alcoxy ;
- -C₅₋₁₀ aryke ; ou
- hétéroaryle, de préférence hétéroaryle à 5 à 6 chaînons comprenant un hétéroatome oxygène (O), azote (N) ou soufre (S) en tant que membre du cycle, non substitué ou substitué par au moins une substitution sélectionnée indépendamment parmi cyano, -oxo, halogène, hydroxy, -NR^{m}Rⁿ, -C₁₋₆ alkyle, -C₁₋₆ alcoxy ou -C(O)NR^{m}Rⁿ, dans lequel -C₁₋₆ alkyle ou C₁₋₆ alcoxy est substitué par au moins une substitution sélectionnée indépendamment parmi cyano, halogène, hydroxy, -NH₂, -C₁₋₆ alkyle ou C₁₋₆ alcoxy ;
et dans lequel R^{m} et Rⁿ sont indépendamment sélectionnés parmi hydrogène ou C₁₋₃ alkyle.

6. Composé selon la revendication 5, dans lequel R³ est
- Hydrogène ;
- méthyle, 1-méthoxyéthyle, 2-hydroxypropan-2-yle, 1-méthoxyéthyle ou (2,4-dioxothiazolidin-3-yl)méthyle ;
- isopropoxy, méthoxy-d3, méthoxy, éthoxy, difluorométhoxy, 2-méthoxyéthoxy, 2-méthoxy-2-méthylpropoxy, 2-hydroxy-2-méthylpropoxy, cyclopropylméthoxy, (1,4-dioxan-2-yl)méthoxy, cyclobutoxy, (4-hydroxycyclohexyl)oxy, (cis-4-hydroxycyclohexyl)oxy, (trans-4-hydroxycyclohexyl)oxy, (4-méthoxycyclohexyl)oxy, (cis-4-méthoxycyclohexyl)oxy, (trans-4-méthoxycyclohexyl)oxy, (3-méthyloxétan-3-yl)méthoxy, 2-méthyl-2-morpholinopropoxy ;
- cyano
- 3-méthoxycyclobutyle, (trans)-3-méthoxycyclobutyle, (cis)-3-méthoxycyclobutyle, 2,2-dichlorocyclopropyle ou 1-cyanocyclopropyle ;
- morpholino, 3-méthyl-morpholino, 3(R)-méthyl-morpholino, 3(S)-méthyl-morpholino, 3,3-diméthylmorpho ;
- tétrahydro-2H-pyran-4-yle, tétrahydro-2H-pyran-3-yle, (R)-tétrahydro-2H-pyran-3-yle, (S)-tétrahydro-2H-pyran-3-yle, 2,2,6,6-tétraméthyltétrahydro-2H-pyran-4-yle ;
- 3-méthoxypyrrolidin-1-yle, 3(R)-méthoxypyrrolidin-1-yle, 3(S)-méthoxypyrrolidin-1-yle, 3-hydroxy-3-méthylpyrrolidin-1-yle, 3-(2-hydroxyéthoxy)pyrrolidin-1-yle, 3-(trifluorométhoxy)pyrrolidin-1-yle, 3(R)-(trifluorométhoxy)pyrrolidin-1-yle, 3(S)-(trifluorométhoxy)pyrrolidin-1-yle, 2-(aminocarbonyl)pyrrolidin-1-yle, 2(R)-(aminocarbonyl)pyrrolidin-1-yle, 2(S)-(aminocarbonyl)pyrrolidin-1-yle, 3-(méthoxyméthyl)pyrrolidin-1-yle, 3(R)-(méthoxyméthyl)pyrrolidin-1-yle, 3(S)-(méthoxyméthyl)pyrrolidin-1-yle, 3-cyano-4-hydroxypyrrolidin-1-yle, cis-3-cyano-4-hydroxypyrrolidin-1-yle, trans-3-cyano-4-hydroxypyrrolidin-1-yle, 3-cyano-4-méthoxypyrrolidin-1-yle, cis-3-cyano-4-méthoxypyrrolidin-1-yle, trans-3-cyano-4-méthoxypyrrolidin-1-yle, 2-(méthoxyméthyl)pyrrolidin-1-yle, 2(R)-(méthoxyméthyl)pyrrolidin-1-yle, 2(S)-(méthoxyméthyl)pyrrolidin-1-yle, 3-méthylpyrrolidin-1-yle, 3(R)-méthylpyrrolidin-1-yle, 3(S)-méthylpyrrolidin-1-yle, pyrrolidin-1-yle, 3-(cyanométhoxy)pyrrolidin-1-yle ;
- 5-azaspiro[2.4]heptan-5-yle ;
- tétrahydrofurane-3-yle ;
- 3-méthoxyazétidin-1-yle, 3-hydroxy-3-méthylazétidin-1-yle ;
- 1,4-dioxan-2-yle ;
- 4-aminotétrahydro-2H-pyran-4-yle, 4-(aminométhyl)tétrahydro-2H-pyran-4-yle,
- 4-méthoxypipéridin-1-yle, 4-hydroxy-4-méthylpipéridin-1-yle, 1-(2,2,2-trifluoroéthyl)pipéridin-4-yle, 3-méthoxypipéridin-1-yle, 3(R)-méthoxypipéridin-1-yle, 3(S)-méthoxypipéridin-1-yle, 3-éthoxypipéridin-1-yle, 3(R)-éthoxypipéridin-1-yle, 3(S)-éthoxypipéridin-1-yle ;
- 3-cyano-2-azabicyclo[3.1.0]hexan-2-yle, (3R)-3-cyano-2-azabicyclo[3.1.0]hexan-2-yle, (3S)-3-cyano-2-azabicyclo[3.1.0]hexan-2-yle, 3-azabicyclo[3.1.0]hexan-3-yle, 4-méthylpyridin-3-yle, 5-méthylpyridazin-4-yle, 5-méthoxypyridazin-4-yle, 3,5-diméthylisoxazol-4-yle, 4-méthoxypyridin-3-yle, 4-(2-hydroxypropan-2-yl)pyridin-3-yle, 6-cyanopyridin-3-yle, 4-cyanopyridin-3-yle, 2-cyanopyridin-3-yle, 3-méthylpyrazin-2-yle, 5-cyanopyridazin-4-yle, 5-fluoropyridazin-4-yle, 4-fluoropyridin-3-yle, 4-isopropylpyridin-3-yle, 4-(1-hydroxyéthyl)pyridin-3-yle, 4-(1-méthoxyéthyl)pyridin-3-yle, pyridin-2-yle ou thiazol-4-yle.

7. Composé selon la revendication 1 ou la revendication 2, dans lequel (R¹ et R²), ou (R² et R³), ou (R³ et R⁴), ensemble avec les atomes auxquels ils sont attachés, forment un système cyclique fusionné à 5 à 7 chaînons, ledit système cyclique fusionné comprend 0 à 2 hétéroatomes oxygène en tant que membre(s) du cycle et est éventuellement et indépendamment substitué par halogène, -C₁₋₆ alkyle, -C₁₋₆ alcoxy, -haloC₁₋₆ alkyle, -haloC₁₋₆ alcoxy ou -C₃₋₆ cycloalkyle.

8. Composé selon la revendication 1 ou la revendication 2, dans lequel R¹ et R², ensemble avec les atomes auxquels ils sont attachés, forment un système cyclique fusionné sélectionné parmi 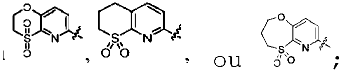 ou R² et R³, ensemble avec les atomes auxquels ils sont attachés, forment un système cyclique fusionné 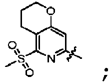 R³ et R⁴, ensemble avec les atomes auxquels ils sont attachés, forment un système cyclique fusionné sélectionné parmi 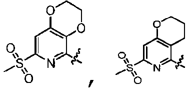 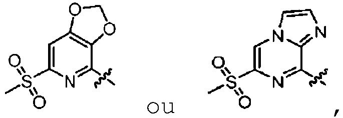 et dans lequel chacun desdits systèmes cycliques fusionnés est éventuellement et indépendamment substitué par halogène, -C₁₋₆ alkyle, -C₁₋₆ alcoxy, -haloC₁₋₆ alkyle, -haloC₁₋₆ alcoxy ou -C₃₋₆ cycloalkyle.

9. Composé selon l'une quelconque des revendications 1 à 8, dans lequel L est une liaison.

10. Composé selon l'une quelconque des revendications 1 à 8, dans lequel Cy¹ est:
a) un hétérocyclyle ou hétéroaryle monocyclique à 5 à 7 chaînons comprenant 1, 2, 3 ou 4 hétéroatomes sélectionnés parmi l'oxygène, l'azote ou le soufre en tant que membre(s) du cycle, ou
b) un hétérocyclyle ou hétéroaryle bicyclique ou tricyclique à 7 à 14 chaînons ayant 1, 2 ou 3 hétéroatomes sélectionnés parmi l'oxygène, l'azote ou le soufre en tant que membre(s) du cycle,
chacun étant non substitué ou substitué par au moins un substituant Rⁱ.

11. Composé selon la revendication 10, dans lequel Cy¹ est
a. ledit hétéroaryle monocyclique à 5 à 7 chaînons comprenant 1, 2, 3 ou 4 hétéroatome(s) sélectionné(s) parmi l'oxygène (O), l'azote (N) ou le soufre (S) en tant que membre(s) du cycle, de préférence pyrazolyle, triazolyle, imidazolyle, thiazolyle, oxazolyle, furanyle, pyridinyle, pyridazinyle, pyrazinyle ou pyrimidinyle, ledit hétéroaryle monocyclique étant non substitué ou substitué par un ou deux substituants sélectionnés parmi
i. halogène ;
ii. cyano ;
iii. -C₁₋₆ alkyle non substitué ou substitué par halogène, hydroxy, -C₁₋₆ alcoxy, -C(O)R^{m} (de préférence R^{m} est morpholinyle) ou -NR^{m}Rⁿ ;
iv. hétérocyclyle non substitué ou substitué par halogène, C₁₋₆ alkyle-, -C₁₋₆ alkyle substitué par -C₁₋₆ alcoxy ou oxo ; de préférence ledit hétérocyclyle est sélectionné parmi tétrahydrofuranyle (de préférence tétrahydrofuran-3-yle), morpholinyle (de préférence morpholino), 2-oxa-5-azabicyclo[2.2.1]heptanyle (de préférence 2-oxa-5-azabicyclo[2.2.1]heptan-2-yle), 8-oxa-3-azabicyclo[3.2.1]octanyle (de préférence 8-oxa-3-azabicyclo[3.2.1]octan-8-yle), isoindolinyle (de préférence isoindolin-2-yle), chacun étant non substitué ou substitué par méthyle, éthyle, propyle, isopropyle, isobutyle, tertbutyle, n-butyle, méthoxyméthyle, méthoxyéthyle, éthoxyméthyle, éthoxyéthyle ou oxo ;
v. -C₃₋₆ cycloalkyle non substitué ou substitué par halogène, -oxo, -C₁₋₆ alkyle, C₁₋₆ alcoxy- ou -C₁₋₆ alkyle substitué par -C₁₋₆ alcoxy ; ou
vi. -OR^{j}, dans lequel R^{j} est -C₁₋₆ alkyle, -C₁₋₆ alkyle substitué par -C₁₋₆ alcoxy ou hétérocyclyle ;
vii. oxo ;
b. ledit hétéroaryle bicyclique ou tricyclique à 7 à 14 chaînons comprenant 1, 2 ou 3 hétéroatome(s) sélectionné(s) parmi l'oxygène, l'azote ou le soufre en tant que membre(s) du cycle, de préférence benzoimidazolyle, imidazopyrimidinyle, pyrazolopyrazinyle, pyrazolopyrimidinyle, benzothiophényle, benzothiazolyle, benzoisoxazolyle, benzooxazolyle, benzoisothiazolyle, imidazopyridazinyle ; dihydro-4H-furo[3,2-c]pyranyle, 6,7-dihydro-4H-thiéno[3,2-c]pyranyle, 2,3-dihydropyrazolo[5,1-b]oxazolyle, 4,5,6,7-tétrahydrofuro[3,2-c]pyridinyle, 1,3a,4,6,7,7a-hexahydropyrano[4,3-c]pyrazolyle, 4,5,6,7-tétrahydropyrazolo[1,5-a]pyrazinyle, 4,5,6,7-tétrahydropyrazolo[1,5-a]pyrimidinyle, 4,5,6,7-tétrahydropyrazolo[1,5-a]pyrazinyle, 4,5,6,7-tétrahydrothiazolo[5,4-c]pyridinyle, 4,5,6,7-tétrahydropyrazolo[1,5-a]pyrazinyle, 6,7-dihydro-4H-pyrazolo[5,1-c][1,4]oxazinyle, 2,3-dihydropyrazolo[5,1-b]oxazolyle, 1,3a,4,6,7,7a-hexahydropyrano[4,3-c]pyrazolyle, 6,7-dihydro-4H-pyrano[4,3-d]thiazolyle, [1,3]dioxolo[4,5-c]pyridinyle, 2,3-dihydro-[1,4]dioxino[2,3-c]pyridinyle, 3,4-dihydro-2H-pyrido[3,2-b][1,4]oxazinyle, 2,3-dihydro-[1,4]dioxino[2,3-b]pyridinyle, 2,3-dihydro-[1,4]dioxino[2,3-b]pyridin-6-yle, 3,4-dihydro-2H-pyrido[4,3-b][1,4]oxazinyle, ou 2H-pyrido[3,2-b][1,4]oxazin-4(3H)-yle, chacun étant non substitué ou substitué par halogène, -C₁₋₆ alkyle, -NH₂, ou - C(O)R^{m}, dans lequel R^{m} est C₁₋₆ alkyle ; l'un quelconque desdits groupes alkyle est éventuellement être enrichi en deutérium.

12. Composé selon la revendication 10, dans lequel Cy¹ est
a. 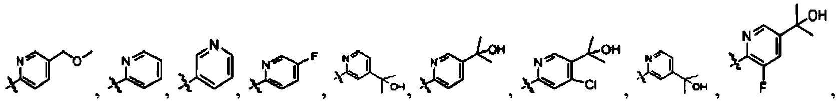 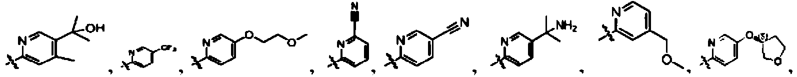 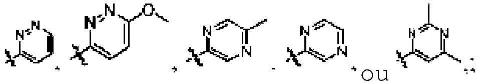
b. 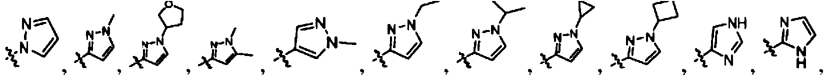 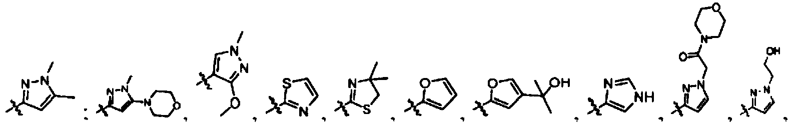 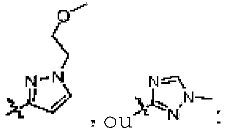
**c.** 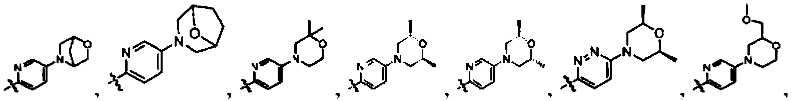 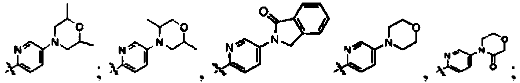
**d.** 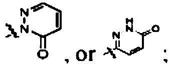
**c.** 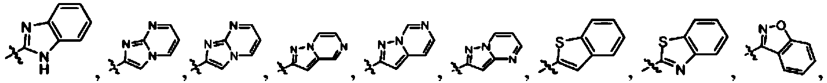 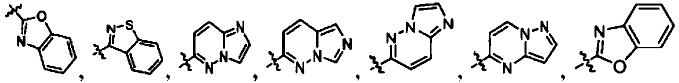
**f.** 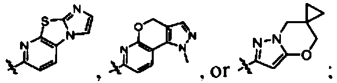
g. 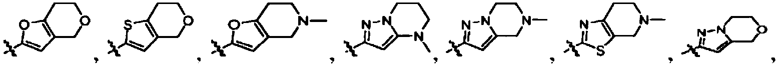 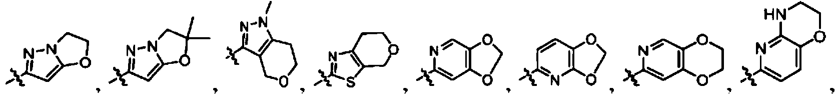 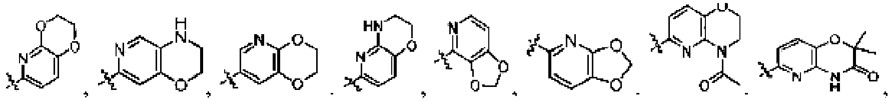 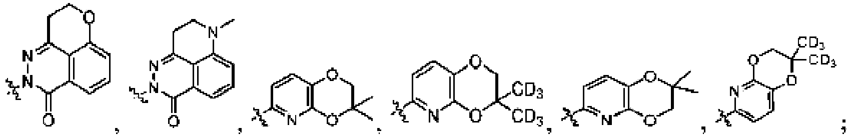
h.

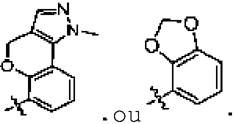

13. Composé selon l'une quelconque des revendications 1 à 12, dans lequel le composé est sélectionné parmi :
N-(4-((3-(méthylsulfonyl)phényl)amino)-5-(pyridazin-3-yl)pyridin-2-yl)acétamide ;
N-[4-[(3-méthanesulfonylphényl)amino]-5-(oxolan-2-yl)pyridin-2-yl]acétamide ;
N-(5-(1H-imidazol-4-yl)-4-((6-(méthylsulfonyl)pyridin-2-yl)amino)pyridin-2-yl)acétamide ;
N-(5-(5-méthylpyrazin-2-yl)-4-((3-(méthylsulfonyl)phényl)amino)pyridin-2-yl)acétamide ;
N-(4-((3-(méthylsulfonyl)phényl)amino)-5-(pyrazin-2-yl)pyridin-2-yl)acétamide ;
N-(5-(2,6-diméthylpyrimidin-4-yl)-4-((3-(méthylsulfonyl)phényl)amino)pyridin-2-yl)acétamide ;
N-(4-((3-(méthylsulfonyl)phényl)amino)-5-(2-oxopyrrolidin-1-yl)pyridin-2-yl)acétamide ;
N-(5-(1-méthyl-1H-pyrazol-4-yl)-4-((6-(méthylsulfonyl)pyridin-2-yl)amino)pyridin-2-yl)acétamide ;
N-(5-(furan-2-yl)-4-((4-méthyl-6-(méthylsulfonyl)pyridin-2-yl)amino)pyridin-2-yl)acétamide ;
N-(5-(1-méthyl-1H-1,2,4-triazol-3-yl)-4-((3-(méthylsulfonyl)phényl)amino)pyridin-2-yl)acétamide ;
N-(4-((3-méthoxy-6-(méthylsulfonyl)pyridin-2-yl)amino)-5-(1-méthyl-1H-pyrazol-4-yl)pyridin-2-yl)acétamide ;
N-(4-((4-isopropoxy-6-(méthylsulfonyl)pyridin-2-yl)amino)-5-(1H-pyrazol-1-yl)pyridin-2-yl)acétamide ;
N-(4-((4-méthyl-6-(méthylsulfonyl)pyridin-2-yl)amino)-5-(1-(2-morpholino-2-oxoéthyl)-1H-pyrazol-3-yl)pyridin-2-yl)acétamide ;
N-(5-(1-(2-hydroxyéthyl)-1H-pyrazol-3-yl)-4-((4-isopropoxy-6-(méthylsulfonyl)pyridin-2-yl)amino)pyridin-2-yl)acétamide ;
N-(5-(1-(2-méthoxyéthyl)-1H-pyrazol-3-yl)-4-((4-méthyl-6-(méthylsulfonyl)pyridin-2-yl)amino)pyridin-2-yl)acétamide ;
N-(6-cyano-4'-((4-isopropoxy-6-(méthylsulfonyl)pyridin-2-yl)amino)-[2,3'-bipyridin]-6'-yl)acétamide ;
N-(5-cyano-4'-((4-isopropoxy-6-(méthylsulfonyl)pyridin-2-yl)amino)-[2,3'-bipyridin]-6'-yl)acétamide ;
N-(5-(1H-imidazol-1-yl)-4-((4-isopropoxy-6-(méthylsulfonyl)pyridin-2-yl)amino)pyridin-2-yl)acétamide ;
N-(5-(1-cyclobutyl-1H-pyrazol-3-yl)-4-((4-isopropoxy-6-(méthylsulfonyl)pyridin-2-yl)amino)pyridin-2-yl)acétamide ;
N-(5-(2-aminopropan-2-yl)-4'-((4-méthyl-6-(méthylsulfonyl)pyridin-2-yl)amino)-[2,3'-bipyridin]-6'-yl)acétamide ;
N-(5-(1H-imidazol-4-yl)-4-((4-(2-méthoxyéthoxy)-6-(méthylsulfonyl)pyridin-2-yl)amino)pyridin-2-yl)acétamide ;
N-(5-(1H-imidazol-4-yl)-4-((4-méthyl-6-(méthylsulfonyl)pyridin-2-yl)amino)pyridin-2-yl)acétamide ;
N-(5-(1H-imidazol-4-yl)-4-((4-isopropoxy-6-(méthylsulfonyl)pyridin-2-yl)amino)pyridin-2-yl)acétamide ;
N-(4-((6-(méthylsulfonyl)pyridin-2-yl)amino)-5-(pyridazin-3-yl)pyridin-2-yl)acétamide ;
N-(4-((4-(cyclopropylméthoxy)-6-(méthylsulfonyl)pyridin-2-yl)amino)-5-(6-méthoxypyridazin-3-yl)pyridin-2-yl)acétamide ;
N-(4-((4-isopropoxy-6-(méthylsulfonyl)pyridin-2-yl)amino)-5-(6-méthoxypyridazin-3-yl)pyridin-2-yl)acétamide ;
N-(4-((4-((2S,6R)-2,6-diméthylmorpholino)-6-(méthylsulfonyl)pyridin-2-yl)amino)-5-(6-méthoxypyridazin-3-yl)pyridin-2-yl)acétamide ;
N-(5-(6-méthoxypyridazin-3-yl)-4-((6-(méthylsulfonyl)-[1,3]dioxolo[4,5-c]pyridin-4-yl)amino)pyridin-2-yl)acétamide ;
N-(5-(6-isopropoxypyridazin-3-yl)-4-((4-méthyl-6-(méthylsulfonyl)pyridin-2-yl)amino)pyridin-2-yl)acétamide ;
N-(5-(6-isopropoxypyridazin-3-yl)-4-((4-méthoxy-6-(méthylsulfonyl)pyridin-2-yl)amino)pyridin-2-yl)acétamide ;
N-(5-(6-isopropoxypyridazin-3-yl)-4-(4-(méthoxy-d3)-6-(méthylsulfonyl)pyridin-2-ylamino)pyridin-2-yl)acétamide ;
N-(4'-((3-(méthylsulfonyl)phényl)amino)-[2,3'-bipyridin]-6'-yl)acétamide ;
N-(4-((6-(méthylsulfonyl)pyridin-2-yl)amino)-[3,3'-bipyridin]-6-yl)acétamide ;
N-(4'-(phénylamino)-[2,3'-bipyridin]-6'-yl)acétamide ;
N-(4'-((6-sulfamoylpyridin-2-yl)amino)-[2,3'-bipyridin]-6'-yl)acétamide ;
N-(4'-((6-(méthylsulfonyl)pyridin-2-yl)amino)-[2,3'-bipyridin]-6'-yl)acétamide ;
N-(4'-((4-(2-méthoxyéthoxy)-6-(méthylsulfonyl)pyridin-2-yl)amino)-[2,3'-bipyridin]-6'-yl)acétamide ;
N-(4'-((4-(2-hydroxyéthoxy)-6-(méthylsulfonyl)pyridin-2-yl)amino)-[2,3'-bipyridin]-6'-yl)acétamide ;
N-(4-(méthoxyméthyl)-4'-((6-(méthylsulfonyl)pyridin-2-yl)amino)-[2,3'-bipyridin]-6'-yl)acétamide ;
N-(4-(2-hydroxypropan-2-yl)-4'-((3-(méthylsulfonyl)phényl)amino)-[2,3'-bipyridin]-6'-yl)acétamide ;
N-(4-(2-hydroxypropan-2-yl)-4'-((4-méthyl-6-(méthylsulfonyl)pyridin-2-yl)amino)-[2,3'-bipyridin]-6'-yl)acétamide ;
N-(4'-((3-(méthylsulfonyl)phényl)amino)-5-morpholino-[2,3'-bipyridin]-6'-yl)acétamide ;
N-(5-(2,2-diméthylmorpholino)-4'-((6-(méthylsulfonyl)pyridin-2-yl)amino)-[2,3'-bipyridin]-6'-yl)acétamide ;
N-(4'-((3-méthoxyphényl)amino)-[2,3'-bipyridin]-6'-yl)acétamide ;
N-(4'-((3-(méthylsulfonyl)pyridin-2-yl)amino)-[2,3'-bipyridin]-6'-yl)acétamide ;
N-(4'-((2-(méthylsulfonyl)phényl)amino)-[2,3'-bipyridin]-6'-yl)acétamide ;
N-(5-(2-oxa-5-azabicyclo[2.2.1]heptan-5-yl)-4'-((4-isopropoxy-6-(méthylsulfonyl)pyridin-2-yl)amino)-[2,3'-bipyridin]-6'-yl)acétamide ;
N-(4-chloro-5-(2-hydroxypropan-2-yl)-4'-((6-(méthylsulfonyl)pyridin-2-yl)amino)-[2,3'-bipyridin]-6'-yl)acétamide ;
N-(4'-((4-isopropoxy-6-(méthylsulfonyl)pyridin-2-yl)amino)-5-(2-(méthoxyméthyl)morpholino)-[2,3'-bipyridin]-6'-yl)acétamide ;
N4'-(3-(méthylsulfonyl)phényl)-[2,3'-bipyridine]-4',6'-diamine ;
N-(4'-((3-(méthylsulfonyl)phényl)amino)-[2,3'-bipyridin]-6'-yl)cyclopropanecarboxamide ;
(S)-N-(4'-((4-méthyl-6-(méthylsulfonyl)pyridin-2-yl)amino)-5-((tétrahydrofuran-3-yl)oxy)-[2,3'-bipyridin]-6'-yl)acétamide ;
N-(4'-((4-(2-hydroxyéthoxy)-6-(méthylsulfonyl)pyridin-2-yl)amino)-5-((tétrahydrofuran-3-yl)oxy)-[2,3'-bipyridin]-6'-yl)acétamide ;
N-(4'-((6-(méthylsulfonyl)pyridin-2-yl)amino)-5-((tétrahydrofuran-3-yl)oxy)-[2,3'-bipyridin]-6'-yl)acétamide ;
N-(5-(8-oxa-3-azabicyclo[3.2.1]octan-3-yl)-4'-((4-méthyl-6-(méthylsulfonyl)pyridin-2-yl)amino)-[2,3'-bipyridin]-6'-yl)acétamide ;
N-(5-(2,5-diméthylmorpholino)-4'-((4-méthyl-6-(méthylsulfonyl)pyridin-2-yl)amino)-[2,3'-bipyridin]-6'-yl)acétamide ;
N-(5-(cis-2,6-diméthylmorpholino)-4'-((4-méthyl-6-(méthylsulfonyl)pyridin-2-yl)amino)-[2,3'-bipyridin]-6'-yl)acétamide ;
N-(5-((cis)-2,6-diméthylmorpholino)-4'-((6-(méthylsulfonyl)pyridin-2-yl)amino)-[2,3'-bipyridin]-6'-yl)acétamide ;
N-(5-((cis)-2,6-diméthylmorpholino)-4'-((3-(méthylsulfonyl)phényl)amino)-[2,3'-bipyridin]-6'-yl)acétamide ;
N-(5-((cis)-2,6-diméthylmorpholino)-4'-((4-isopropoxy-6-(méthylsulfonyl)pyridin-2-yl)amino)-[2,3'-bipyridin]-6'-yl)acétamide ;
N-(4'-((4-(cyclopropylméthoxy)-6-(méthylsulfonyl)pyridin-2-yl)amino)-5-((cis)-2,6-diméthylmorpholino)-[2,3'-bipyridin]-6'-yl)acétamide ;
N-(4'-((3-(cyclopropylméthoxy)-5-(méthylsulfonyl)phényl)amino)-5-((cis)-2,6-diméthylmorpholino)-[2,3'-bipyridin]-6'-yl)acétamide ;
N-(5-((trans)-2,6-diméthylmorpholino)-4'-((4-méthyl-6-(méthylsulfonyl)pyridin-2-yl)amino)-[2,3'-bipyridin]-6'-yl)acétamide ;
N-(5-(cis-2,6-diméthylmorpholino)-4'-((7-(méthylsulfonyl)-2,3-dihydro-[1,4]dioxino[2,3-c]pyridin-5-yl)amino)-[2,3'-bipyridin]-6'-yl)acétamide ;
N-(5-((cis)-2,6-diméthylmorpholino)-4'-((4-méthoxy-6-(méthylsulfonyl)pyridin-2-yl)amino)-[2,3'-bipyridin]-6'-yl)acétamide ;
N-(5-((cis)-2,6-diméthylmorpholino)-4'-((4-éthoxy-6-(méthylsulfonyl)pyridin-2-yl)amino)-[2,3'-bipyridin]-6'-yl)acétamide ;
N-(5-((cis)-2,6-diméthylmorpholino)-4'-((4-(2-méthoxyéthoxy)-6-(méthylsulfonyl)pyridin-2-yl)amino)-[2,3'-bipyridin]-6'-yl)acétamide ;
N-(5-((cis)-2,6-diméthylmorpholino)-4'-((4-((R)-2-hydroxypropoxy)-6-(méthylsulfonyl)pyridin-2-yl)amino)-[2,3'-bipyridin]-6'-yl)acétamide ;
N-(4'-((4-((R)-sec-butoxy)-6-(méthylsulfonyl)pyridin-2-yl)amino)-5-((cis)-2,6-diméthylmorpholino)-[2,3'-bipyridin]-6'-yl)acétamide ;
N-(5-((cis)-2,6-diméthylmorpholino)-4'-((3-méthoxy-5-(méthylsulfonyl)phényl)amino)-[2,3'-bipyridin]-6'-yl)acétamide ;
N-(5-((cis)-2,6-diméthylmorpholino)-4'-((3-fluoro-5-(méthylsulfonyl)phényl)amino)-[2,3'-bipyridin]-6'-yl)acétamide ;
N-(4'-((3-cyano-5-(méthylsulfonyl)phényl)amino)-5-((cis)-2,6-diméthylmorpholino)-[2,3'-bipyridin]-6'-yl)acétamide ;
N-(5-(cis-2,6-diméthylmorpholino)-4'-((4-méthoxy-6-(méthylsulfonyl)pyridin-2-yl)amino)-[2,3'-bipyridin]-6'-yl)acétamide ;
N-(5-((cis)-2,6-diméthylmorpholino)-4'-((4-(méthoxy-d3)-6-(méthylsulfonyl)pyridin-2-yl)amino)-[2,3'-bipyridin]-6'-yl)acétamide ;
N-(5-(cis-2,6-diméthylmorpholino)-4'-((4-(1-méthoxyéthyl)-6-(méthylsulfonyl)pyridin-2-yl)amino)-[2,3'-bipyridin]-6'-yl)acétamide ;
N-(5-((cis)-2,6-diméthylmorpholino)-4'-((4-((R ou S)-1-méthoxyéthyl)-6-(méthylsulfonyl)pyridin-2-yl)amino)-[2,3'-bipyridin]-6'-yl)acétamide ;
N-(5-((cis)-2,6-diméthylmorpholino)-4'-((4-((R ou S)-1-méthoxyéthyl)-6-(méthylsulfonyl)pyridin-2-yl)amino)-[2,3'-bipyridin]-6'-yl)acétamide ;
N-(5-(cis-2,6-diméthylmorpholino)-4'-((6-(méthylsulfonyl)-4-(tétrahydro-2H-pyran-4-yl)pyridin-2-yl)amino)-[2,3'-bipyridin]-6'-yl)acétamide ;
N-(5-(cis-2,6-diméthylmorpholino)-4'-((6-(méthylsulfonyl)pyrazin-2-yl)amino)-[2,3'-bipyridin]-6'-yl)acétamide ;
N-(5-(cis-2,6-diméthylmorpholino)-4'-((4-((S)-3-méthylmorpholino)-6-(méthylsulfonyl)pyridin-2-yl)amino)-[2,3'-bipyridin]-6'-yl)acétamide ;
N-(5-(cis-2,6-diméthylmorpholino)-4'-((4-((R)-3-méthylmorpholino)-6-(méthylsulfonyl)pyridin-2-yl)amino)-[2,3'-bipyridin]-6'-yl)acétamide ;
N-(5-(cis-2,6-diméthylmorpholino)-4'-((4-(3-méthoxyazétidin-1-yl)-6-(méthylsulfonyl)pyridin-2-yl)amino)-[2,3'-bipyridin]-6'-yl)acétamide ;
N-(5-(2-méthoxyéthoxy)-4'-((6-(méthylsulfonyl)pyridin-2-yl)amino)-[2,3'-bipyridin]-6'-yl)acétamide ;
N-(5-(2-méthoxyéthoxy)-4'-((3-(méthylsulfonyl)phényl)amino)-[2,3'-bipyridin]-6'-yl)acétamide ;
N-(5-(2-méthoxyéthoxy)-4'-((3-(méthylsulfonyl)-5-(trifluorométhyl)phényl)amino)-[2,3'-bipyridin]-6'-yl)acétamide ;
N-(5-(2-méthoxyéthoxy)-4'-((4-méthyl-6-(méthylsulfonyl)pyridin-2-yl)amino)-[2,3'-bipyridin]-6'-yl)acétamide ;
N-(4'-((4-méthoxy-6-(méthylsulfonyl)pyridin-2-yl)amino)-5-(2-méthoxyéthoxy)-[2,3'-bipyridin]-6'-yl)acétamide ;
N-(4'-((4-isopropoxy-6-(méthylsulfonyl)pyridin-2-yl)amino)-5-(2-méthoxyéthoxy)-[2,3'-bipyridin]-6'-yl)acétamide ;
(R)-N-(5-(2-méthoxyéthoxy)-4'-((4-(2-méthoxypropoxy)-6-(méthylsulfonyl)pyridin-2-yl)amino)-[2,3'-bipyridin]-6'-yl)acétamide ;
N-(5-(2-méthoxyéthoxy)-4'-((4-(2-méthoxyéthoxy)-6-(méthylsulfonyl)pyridin-2-yl)amino)-[2,3'-bipyridin]-6'-yl)acétamide ;
N-(4'-((4-éthoxy-6-(méthylsulfonyl)pyridin-2-yl)amino)-5-(2-méthoxyéthoxy)-[2,3'-bipyridin]-6'-yl)acétamide ;
N-(4'-((3-(cyclopropylméthoxy)-5-(méthylsulfonyl)phényl)amino)-5-(2-méthoxyéthoxy)-[2,3'-bipyridin]-6'-yl)acétamide ;
N-(4'-((4-(cyclopropylméthoxy)-6-(méthylsulfonyl)pyridin-2-yl)amino)-5-(2-méthoxyéthoxy)-[2,3'-bipyridin]-6'-yl)acétamide ;
N-(5-(méthoxyméthyl)-4'-((3-(méthylsulfonyl)phényl)amino)-[2,3'-bipyridin]-6'-yl)acétamide ;
N-(5-(méthoxyméthyl)-4'-((4-méthyl-6-(méthylsulfonyl)pyridin-2-yl)amino)-[2,3'-bipyridin]-6'-yl)acétamide ;
(R)-N-(5-(méthoxyméthyl)-4'-((4-(2-méthoxypropoxy)-6-(méthylsulfonyl)pyridin-2-yl)amino)-[2,3'-bipyridin]-6'-yl)acétamide ;
N-(4'-((4-isopropoxy-6-(méthylsulfonyl)pyridin-2-yl)amino)-5-(méthoxyméthyl)-[2,3'-bipyridin]-6'-yl)acétamide ;
N-(4'-((4-(2-méthoxyéthoxy)-6-(méthylsulfonyl)pyridin-2-yl)amino)-5-(méthoxyméthyl)-[2,3'-bipyridin]-6'-yl)acétamide ;
N-(4'-((4-(2-hydroxyéthoxy)-6-(méthylsulfonyl)pyridin-2-yl)amino)-5-(méthoxyméthyl)-[2,3'-bipyridin]-6'-yl)acétamide ;
N-(4'-((4-méthoxy-6-(méthylsulfonyl)pyridin-2-yl)amino)-5-(méthoxyméthyl)-[2,3'-bipyridin]-6'-yl)acétamide ;
N-(4'-((4-((11,3r)-3-hydroxycyclobutoxy)-6-(méthylsulfonyl)pyridin-2-yl)amino)-5-(méthoxyméthyl)-[2,3'-bipyridin]-6'-yl)acétamide ;
N-(5-(méthoxyméthyl)-4'-((4-(méthoxyméthyl)-6-(méthylsulfonyl)pyridin-2-yl)amino)-[2,3'-bipyridin]-6'-yl)acétamide ;
N-(4'-((4-méthyl-6-(méthylsulfonyl)pyridin-2-yl)amino)-5-(trifluorométhyl)-[2,3'-bipyridin]-6'-yl)acétamide ;
N-(4'-((3-(méthylsulfonyl)phényl)amino)-5-(trifluorométhyl)-[2,3'-bipyridin]-6'-yl)acétamide ;
N-(4'-((3-méthyl-5-(méthylsulfonyl)phényl)amino)-5-(trifluorométhyl)-[2,3'-bipyridin]-6'-yl)acétamide ;
N-(4'-((3-cyano-5-(méthylsulfonyl)phényl)amino)-5-(trifluorométhyl)-[2,3'-bipyridin]-6'-yl)acétamide ;
N-(4'-((8-((trans)-3-hydroxycyclobutoxy)-5-(méthylsulfonyl)phényl)amino)-5-(trifluorométhyl)-[2,3'-bipyridin]-6'-yl)acétamide ;
N-(4'-((3-(cyclopropylméthoxy)-5-(méthylsulfonyl)phényl)amino)-5-(trifluorométhyl)-[2,3'-bipyridin]-6'-yl)acétamide ;
N-(4'-((3-(2-hydroxyéthoxy)-5-(méthylsulfonyl)phényl)amino)-5-(trifluorométhyl)-[2,3'-bipyridin]-6'-yl)acétamide ;
N-(4'-((3-(2-méthoxyéthoxy)-5-(méthylsulfonyl)phényl)amino)-5-(trifluorométhyl)-[2,3'-bipyridin]-6'-yl)acétamide ;
N-(4'-((4-isopropoxy-6-(méthylsulfonyl)pyridin-2-yl)amino)-5-(trifluorométhyl)-[2,3'-bipyridin]-6'-yl)acétamide ;
N-(4'-((4-éthoxy-6-(méthylsulfonyl)pyridin-2-yl)amino)-5-(trifluorométhyl)-[2,3'-bipyridin]-6'-yl)acétamide ;
N-(4'-((4-(2-méthoxyéthoxy)-6-(méthylsulfonyl)pyridin-2-yl)amino)-5-(trifluorométhyl)-[2,3'-bipyridin]-6'-yl)acétamidecarboxamide ;
N-(4'-((4-(2-hydroxyéthoxy)-6-(méthylsulfonyl)pyridin-2-yl)amino)-5-(trifluorométhyl)-[2,3'-bipyridin]-6'-yl)acétamide ;
N-(5-fluoro-4'-((3-(méthylsulfonyl)-5-(trifluorométhyl)phényl)amino)-[2,3'-bipyridin]-6'-yl)acétamide ;
N-(5-fluoro-4'-((3-isopropoxy-5-(méthylsulfonyl)phényl)amino)-[2,3'-bipyridin]-6'-yl)acétamide ;
N-(5-fluoro-4'-((3-((trans)-3-hydroxycyclobutoxy)-5-(méthylsulfonyl)phényl)amino)-[2,3'-bipyridin]-6'-yl)acétamide ;
N-(5-fluoro-4'-((4-(2-méthoxyéthoxy)-6-(méthylsulfonyl)pyridin-2-yl)amino)-[2,3'-bipyridin]-6'-yl)acétamide ;
N-(5-fluoro-4'-((4-méthyl-6-(méthylsulfonyl)pyridin-2-yl)amino)-[2,3'-bipyridin]-6'-yl)acétamide ;
N-(5-fluoro-4'-((4-isopropoxy-6-(méthylsulfonyl)pyridin-2-yl)amino)-[2,3'-bipyridin]-6'-yl)acétamide ;
N-(4'-((4-éthoxy-6-(méthylsulfonyl)pyridin-2-yl)amino)-5-fluoro-[2,3'-bipyridin]-6'-yl)acétamide ;
N-(4'-((4-méthoxy-6-(méthylsulfonyl)pyridin-2-yl)amino)-5-(trifluorométhyl)-[2,3'-bipyridin]-6'-yl)acétamide ;
(S)-N-(4'-((4-(2-hydroxypropoxy)-6-(méthylsulfonyl)pyridin-2-yl)amino)-5-(tnifluorométhyl)-[2,3'-bipyridin]-6'-yl)acétamide ;
N-(5-fluoro-4'-((4-méthoxy-6-(méthylsulfonyl)pyridin-2-yl)amino)-[2,3'-bipyridin]-6'-yl)acétamide ;
N-(4'-((3,4-diméthoxy-6-(méthylsulfonyl)pyridin-2-yl)amino)-5-fluoro-[2,3'-bipyridin]-6'-yl)acétamide ;
N-(5-fluoro-4'-((4-(méthoxy-d3)-6-(méthylsulfonyl)pyridine-2-yl)amino)-[2,3'-bipyridin]-6'-yl)acétamide ;
N-(4'-((4-cyclopropyl-6-(méthylsulfonyl)pyridin-2-yl)amino)-5-fluoro-[2,3'-bipyridin]-6'-yl)acétamide ;
N-(4'-((8-(méthylsulfonyl)phényl)amino)-5-(3-oxomorpholino)-[2,3'-bipyridin]-6'-yl)acétamide ;
N-(4'-((6-(méthylsulfonyl)pyridin-2-yl)amino)-5-(3-oxomorpholino)-[2,3'-bipyridin]-6'-yl)acétamide ;
N-(4'-((4-(2-hydroxyéthoxy)-6-(méthylsulfonyl)pyridin-2-yl)amino)-5-(3-oxomorpholino)-[2,3'-bipyridin]-6'-yl)acétamide ;
N-(4'-((4-(2-méthoxyéthoxy)-6-(méthylsulfonyl)pyridin-2-yl)amino)-5-(3-oxomorpholino)-[2,3'-bipyridin]-6'-yl)acétamide ;
N-(5-(2-hydroxypropan-2-yl)-4'-((3-(méthylsulfonyl)phényl)amino)-[2,3'-bipyridin]-6'-yl)acétamide ;
N-(5-(2-hydroxypropan-2-yl)-4'-((4-isopropoxy-6-(méthylsulfonyl)pyridin-2-yl)amino)-[2,3'-bipyridin]-6'-yl)acétamide ;
N-(5-(2-hydroxypropan-2-yl)-4'-((3-(méthylsulfonyl)-5-(trifluoro méthyl)phényl)amino)-[2,3'-bipyridin]-6'-yl)acétamide ;
N-(5-(2-hydroxypropan-2-yl)-4'-((3-méthoxy-5-(méthylsulfonyl)phényl)amino)-[2,3'-bipyridin]-6'-yl)acétamide ;
N-(5-(2-hydroxypropan-2-yl)-4'-((6-(méthylsulfonyl)pyridin-2-yl)amino)-[2,3'-bipyridin]-6'-yl)acétamide ;
N-(5-(2-hydroxypropan-2-yl)-4'-((4-(2-méthoxyéthoxy)-6-(méthylsulfonyl)pyridin-2-yl)amino)-[2,3'-bipyridin]-6'-yl)acétamide ;
N-(5-(2-hydroxypropan-2-yl)-4'-((4-(2-méthoxypropoxy)-6-(méthylsulfonyl)pyridin-2-yl)amino)-[2,3'-bipyridin]-6'-yl)acétamide ;
N-(4'-((4-(2-hydroxyéthoxy)-6-(méthylsulfonyl)pyridin-2-yl)amino)-5-(2-hydroxypropan-2-yl)-[2,3'-bipyridin]-6'-yl)acétamide ;
N-(5-(2-hydroxypropan-2-yl)-4'-((4-méthyl-6-(méthylsulfonyl)pyridin-2-yl)amino)-[2,3'-bipyridin]-6'-yl)acétamide ;
N-(4'-((4-(trans)-3-hydroxycyclobutoxy)-6-(méthylsulfonyl)pyridin-2-yl)amino)-5-(2-hydroxypropan-2-yl)-[2,3'-bipyridin]-6'-yl)acétamide ;
N-(5-(2-hydroxypropan-2-yl)-4'-((4-méthoxy-6-(méthylsulfonyl)pyridin-2-yl)amino)-[2,3'-bipyridin]-6'-yl)acétamide ;
N-(5-(2-hydroxypropan-2-yl)-4'-((6-(méthylsulfonyl)-4-(oxétan-3-ylméthoxy)pyridin-2-yl)amino)-[2,3'-bipyridin]-6'-yl)acétamide ;
N-(5-(2-hydroxypropan-2-yl)-4'-((4-(méthoxyméthyl)-6-(méthylsulfonyl)pyridin-2-yl)amino)-[2,3'-bipyridin]-6'-yl)acétamide ;
N-(4'-((4-cyano-6-(méthylsulfonyl)pyridin-2-yl)amino)-5-(2-hydroxypropan-2-yl)-[2,3'-bipyridin]-6'-yl)acétamide ;
N-(5-(2-hydroxypropan-2-yl)-4'-((3-(2-méthoxyéthoxy)-5-(méthylsulfonyl)phényl)amino)-[2,3'-bipyridin]-6'-yl)acétamide ;
N-(5-(2-hydroxypropan-2-yl)-4'-((3-(méthoxyméthyl)-5-(méthylsulfonyl)phényl)amino)-[2,3'-bipyridin]-6'-yl)acétamide ;
N-(5-(2-hydroxypropan-2-yl)-4'-((3-(3-hydroxypyrrolidin-1-yl)-5-(méthylsulfonyl)phényl)amino)-[2,3'-bipyridin]-6'-yl)acétamide ;
N-(5-(2-hydroxypropan-2-yl)-4'-((3-(méthylsulfony])-5-(trifluorométhoxy)phényl)amino)-[2,3'-bipyridin]-6'-yl)acétamide ;
N-(4'-((3-fluoro-5-(méthylsulfonyl)phényl)amino)-5-(2-hydroxypropan-2-yl)-[2,3'-bipyridin]-6'-yl)acétamide ;
N-(4'-((3-cyano-5-(méthylsulfonyl)phényl)amino)-5-(2-hydroxypropan-2-yl)-[2,3'-bipyridin]-6'-yl)acétamide ;
N-(5-(2-hydroxypropan-2-yl)-4'-((3-méthyl-5-(méthylsulfonyl)phényl)amino)-[2,3'-bipyridin]-6'-yl)acétamide ;
N-(4'-((3-(cyclopropylméthoxy)-5-(méthylsulfonyl)phényl)amino)-5-(2-hydroxypropan-2-yl)-[2,3'-bipyridin]-6'-yl)acétamide ;
N-(4'-((3-cyclopropoxy-5-(méthylsulfonyl)phényl)amino)-5-(2-hydroxypropan-2-yl)-[2,3'-bipyridin]-6'-yl)acétamide ;
N-(5-(2-hydroxypropan-2-yl)-4'-((3-isopropoxy-5-(méthylsulfonyl)phényl)amino)-[2,3'-bipyridin]-6'-yl)acétamide ;
N-(4'-((3-(difluorométhyl)-5-(méthylsulfonyl)phényl)amino)-5-(2-hydroxypropan-2-yl)-[2,3'-bipyridin]-6'-yl)acétamide ;
N-(5-(2-hydroxypropan-2-yl)-4'-((4-(2-hydroxypropoxy)-6-(méthylsulfonyl)pyridin-2-yl)amino)-[2,3'-bipyridin]-6'-yl)acétamide ;
N-(5-(2-hydroxypropan-2-yl)-4'-((6-(méthylsulfonyl)-4-(oxétan-3-yl)oxy)pyridin-2-yl)amino)-[2,3'-bipyridin]-6'-yl)acétamide ;
N-(4'-((4-(cis-3-hydroxycyclobutoxy)-6-(méthylsulfonyl)pyridin-2-yl)amino)-5-(2-hydroxypropan-2-yl)-[2,3'-bipyridin]-6'-yl)acétamide ;
N-(5-(2-hydroxypropan-2-yl)-4'-((4-(3-méthoxycyclobutoxy)-6-(méthylsulfonyl)pyridin-2-yl)amino)-[2,3'-bipyridin]-6'-yl)acétamide ;
N-(4'-((4-(cyclopropylméthoxy)-6-(méthylsulfonyl)pyridin-2-yl)amino)-5-(2-hydroxypropan-2-yl)-[2,3'-bipyridin]-6'-yl)acétamide ;
N-(4'-((4-cyclobutoxy-6-(méthylsulfonyl)pyridin-2-yl)amino)-5-(2-hydroxypropan-2-yl)-[2,3'-bipyridin]-6'-yl)acétamide ;
N-(5-(2-hydroxypropan-2-yl)-4'-((3-méthyl-6-(méthylsulfonyl)pyridin-2-yl)amino)-[2,3'-bipyridin]-6'-yl)acétamide ;
N-(4'-((4-éthoxy-6-(méthylsulfonyl)pyridin-2-yl)amino)-5-(2-hydroxypropan-2-yl)-[2,3'-bipyridin]-6'-yl)acétamide ;
N-(4'-((5-fluoro-6-(méthylsulfonyl)pyridin-2-yl)amino)-5-(2-hydroxypropan-2-yl)-[2,3'-bipyridin]-6'-yl)acétamide ;
N-(5-(2-hydroxypropan-2-yl)-4'-((2-méthyl-5-(méthylsulfonyl)phényl)amino)-[2,3'-bipyridin]-6'-yl)acétamide ;
N-(4'-((6-(éthylsulfonyl)-4-isopropoxypyridin-2-yl)amino)-5-(2-hydroxypropan-2-yl)-[2,3'-bipyridin]-6'-yl)acétamide ;
N-(5-(2-hydroxypropan-2-yl)-4'-((7-(méthylsulfony])-2,3-dihydro-[1,4]dioxino[2,3-c]pyridin-5-yl)amino)-[2,3'-bipyridin]-6'-yl)acétamide ;
(R)-N-(5-(2-hydroxypropan-2-yl)-4'-((4-(2-hydroxypropoxy)-6-(méthylsulfonyl)pyridin-2-yl)amino)-[2,3'-bipyridin]-6'-yl)acétamide ; N-(5-(2-hydroxypropan-2-yl)-4'-((6-(méthylsulfonyl)-4-(tétrahydro-2H-pyran-4-yl)pyridin-2-yl)amino)-[2,3'-bipyridin]-6'-yl)acétamide ;
N-(5-(2-hydroxypropan-2-yl)-4'-((4-isobutoxy-6-(méthylsulfonyl)pyridin-2-yl)amino)-[2,3'-bipyridin]-6'-yl)acétamide ;
(S)-N-(4'-((4-(sec-butoxy)-6-(méthylsulfonyl)pyridin-2-yl)amino)-5-(2-hydroxypropan-2-yl)-[2,3'-bipyridin]-6'-yl)acétamide ;
(R)-N-(4'-((4-(sec-butoxy)-6-(méthylsulfonyl)pyridin-2-yl)amino)-5-(2-hydroxypropan-2-yl)-[2,3'-bipyridin]-6'-yl)acétamide ;
N-(5-(2-hydroxypropan-2-yl)-4'-((3-isopropoxy-5-(méthylsulfonyl)phényl)amino)-[2,3'-bipyridin]-6'-yl)acétamide ;
N-(5-(1-méthyl-1H-pyrazol-3-yl)-4-((4-méthyl-6-(méthylsulfonyl)pyridin-2-yl)amino)pyridin-2-yl)acétamide ;
N-(4-((4-isopropoxy-6-(méthylsulfonyl)pyridin-2-yl)amino)-5-(1-méthyl-1H-pyrazol-3-yl)pyridin-2-yl)acétamide ;
N-(4-((4-(cyclopropylméthoxy)-6-(méthylsulfonyl)pyridin-2-yl)amino)-5-(1-méthyl-1H-pyrazol-3-yl)pyridin-2-yl)acétamide ;
N-(5-(1-méthyl-1H-pyrazol-3-yl)-4-((6-sulfamoylpyridin-2-yl)amino)pyridin-2-yl)acétamide ;
N-(4-((6-(éthylsulfonyl)pyridin-2-yl)amino)-5-(1-méthyl-1H-pyrazol-3-yl)pyridin-2-yl)acétamide ;
N-(5-(1-méthyl-1H-pyrazol-3-yl)-4-((3-(méthylsulfonyl)phényl)amino)pyridin-2-yl)acétamide ;
N-(4-((3-cyano-5-(méthylsulfonyl)phényl)amino)-5-(1-méthyl-1H-pyrazol-3-yl)pyridin-2-yl)acétamide ;
N-(5-(1-méthyl-1H-pyrazol-3-yl)-4-((3-méthyl-5-(méthylsulfonyl)phényl)amino)pyridin-2-yl)acétamide ;
N-(4-((3-méthoxy-5-(méthylsulfonyl)phényl)amino)-5-(1-méthyl-1H-pyrazol-3-yl)pyridin-2-yl)acétamide ;
N-(5-(1-méthyl-1H-pyrazol-3-yl)-4-((3-(méthylsulfonyl)-5-(trifluorométhoxy)phényl)amino)pyridin-2-yl)acétamide ;
N-(5-(1-méthyl-1H-pyrazol-3-yl)-4-((6-(méthylsulfonyl)pyridin-2-yl)amino)pyridin-2-yl)acétamide ;
N-(4-((4-(2-méthoxyéthoxy)-6-(méthylsulfonyl)pyridin-2-yl)amino)-5-(1-méthyl-1H-pyrazol-3-yl)pyridin-2-yl)acétamide ;
N-(4-((4-(2-méthoxypropoxy)-6-(méthylsulfonyl)pyridin-2-yl)amino)-5-(1-méthyl-1H-pyrazol-3-yl)pyridin-2-yl)acétamide ;
(R)-N-(4-((4-(2-méthoxypropoxy)-6-(méthylsulfonyl)pyridin-2-yl)amino)-5-(1-méthyl-1H-pyrazol-3-yl)pyridin-2-yl)acétamide ;
N-(4-((4-(2-hydroxyéthoxy)-6-(méthylsulfonyl)pyridin-2-yl)amino)-5-(1-méthyl-1H-pyrazol-3-yl)pyridin-2-yl)acétamide ;
N-(4-((4-((trans)-3-hydroxycyclobutoxy)-6-(méthylsulfonyl)pyridin-2-yl)amino)-5-(1-méthyl-1H-pyrazol-3-yl)pyridin-2-yl)acétamide ;
N-(4-((4-((cis)-3-hydroxycyclobutoxy)-6-(méthylsulfonyl)pyridin-2-yl)amino)-5-(1-méthyl-1H-pyrazol-3-yl)pyridin-2-yl)acétamide ;
N-(4-((4-(3-méthoxycyclobutoxy)-6-(méthylsulfonyl)pyridin-2-yl)amino)-5-(1-méthyl-1H-pyrazol-3-yl)pyridin-2-yl)acétamide ;
N-(4-((4-(méthoxyméthyl)-6-(méthylsulfonyl)pyridin-2-yl)amino)-5-(1-méthyl-1H-pyrazol-3-yl)pyridin-2-yl)acétamide ;
N-(4-((4-(cyanométhoxy)-6-(méthylsulfonyl)pyridin-2-yl)amino)-5-(1-méthyl-1H-pyrazol-3-yl)pyridin-2-yl)acétamide ;
N-(4-((4-méthoxy-6-(méthylsulfonyl)pyridin-2-yl)amino)-5-(1-méthyl-1H-pyrazol-3-yl)pyridin-2-yl)acétamide ;
N-(5-(1-méthyl-1H-pyrazol-3-yl)-4-((6-(méthylsulfonyl)-4-(oxétan-3-yl)oxy)pyridin-2-yl)amino)pyridin-2-yl)acétamide ;
N-(4-((3-méthoxy-6-(méthylsulfonyl)pyridin-2-yl)amino)-5-(1-méthyl-1H-pyrazol-3-yl)pyridin-2-yl)acétamide ;
N-(4-((4-(2-hydroxypropoxy)-6-(méthylsulfonyl)pyridin-2-yl)amino)-5-(1-méthyl-1H-pyrazol-3-yl)pyridin-2-yl)acétamide ;
N-(5-(1-méthyl-1H-pyrazol-3-yl)-4-((6-(méthylsulfonyl)-4-(oxétan-3-ylméthoxy)pyridin-2-yl)amino)pyridin-2-yl)acétamide ;
N-(4-((4-cyano-6-(méthylsulfonyl)pyridin-2-yl)amino)-5-(1-méthyl-1H-pyrazol-3-yl)pyridin-2-yl)acétamide ;
N-(5-(1-méthyl-1H-pyrazol-3-yl)-4-((3-méthyl-6-(méthylsulfonyl)pyridin-2-yl)amino)pyridin-2-yl)acétamide ;
N-(4-((6-(cyclopropylsulfonyl)pyridin-2-yl)amino)-5-(1-méthyl-1H-pyrazol-3-yl)pyridin-2-yl)acétamide ;
N-(4-((6-(isopropylsulfonyl)pyridin-2-yl)amino)-5-(1-méthyl-1H-pyrazol-3-yl)pyridin-2-yl)acétamide ;
N-(4-((3-fluoro-5-(méthylsulfonyl)phényl)amino)-5-(1-méthyl-1H-pyrazol-3-yl)pyridin-2-yl)acétamide ;
N-(4-((3-chloro-5-(méthylsulfonyl)phényl)amino)-5-(1-méthyl-1H-pyrazol-3-yl)pyridin-2-yl)acétamide ;
N-(4-((3-(difluorométhyl)-5-(méthylsulfonyl)phényl)amino)-5-(1-méthyl-1H-pyrazol-3-yl)pyridin-2-yl)acétamide ;
N-(5-(1-méthyl-1H-pyrazol-3-yl)-4-((3-(méthylsulfonyl)-5-(trifluorométhyl)phényl)amino)pyridin-2-yl)acétamide ;
N-(4-((3-(méthoxyméthyl)-5-(méthylsulfonyl)phényl)amino)-5-(1-méthyl-1H-pyrazol-3-yl)pyridin-2-yl)acétamide ;
N-(4-((3-(2-méthoxyéthoxy)-5-(méthylsulfonyl)phényl)amino)-5-(1-méthyl-1H-pyrazol-3-yl)pyridin-2-yl)acétamide ;
N-(4-((3-cyclopropoxy-5-(méthylsulfonyl)phényl)amino)-5-(1-méthyl-1H-pyrazol-3-yl)pyridin-2-yl)acétamide ;
N-(4-((3-((trans)-3-hydroxycyclobutoxy)-5-(méthylsulfonyl)phényl)amino)-5-(1-méthyl-1H-pyrazol-3-yl)pyridin-2-yl)acétamide ;
N-(5-(1-méthyl-1H-pyrazol-3-yl)-4-((6-(méthylsulfonyl)-4-phénylpyridin-2-yl)amino)pyridin-2-yl)acétamide ;
N-(5-(1-méthyl-1H-pyrazol-3-yl)-4-((6-(méthylsulfonyl)-4-phenoxypyridin-2-yl)amino)pyridin-2-yl)acétamide ;
N-(4-((4-chloro-6-(méthylsulfonyl)pyridin-2-yl)amino)-5-(1-méthyl-1H-pyrazol-3-yl)pyridin-2-yl)acétamide ;
N-(4-((4-(1-méthoxyéthyl)-6-(méthylsulfonyl)pyridin-2-yl)amino)-5-(1-méthyl-1H-pyrazol-3-yl)pyridin-2-yl)acétamide ;
N-(4-((4-(difluorométhyl)-6-(méthylsulfonyl)pyridin-2-yl)amino)-5-(1-méthyl-1H-pyrazol-3-yl)pyridin-2-yl)acétamide ;
N-(4-((4-éthoxy-6-(méthylsulfonyl)pyridin-2-yl)amino)-5-(1-méthyl-1H-pyrazol-3-yl)pyridin-2-yl)acétamide ;
N-(4-((4-cyclobutoxy-6-(méthylsulfonyl)pyridin-2-yl)amino)-5-(1-méthyl-1H-pyrazol-3-yl)pyridin-2-yl)acétamide ;
N-(4-((4-(cyclopentyl)oxy)-6-(méthylsulfonyl)pyridin-2-yl)amino)-5-(1-méthyl-1H-pyrazol-3-yl)pyridin-2-yl)acétamide ;
N-(5-(1-méthyl-1H-pyrazol-3-yl)-4-((6-(méthylsulfonyl)-4-((tétrahydrofuran-3-yl)oxy)pyridin-2-yl)amino)pyridin-2-yl)acétamide ;
(R)-N-(4-((4-(2-hydroxypropoxy)-6-(méthylsulfonyl)pyridin-2-yl)amino)-5-(1-méthyl-1H-pyrazol-3-yl)pyridin-2-yl)acétamide ;
N-(4-((4-(2,3-dihydroxypropoxy)-6-(méthylsulfonyl)pyridin-2-yl)amino)-5-(1-méthyl-1H-pyrazol-3-yl)pyridin-2-yl)acétamide ;
N-(4-((3-isopropoxy-6-(méthylsulfonyl)pyridin-2-yl)amino)-5-(1-méthyl-1H-pyrazol-3-yl)pyridin-2-yl)acétamide ;
N-(4-((5-méthoxy-6-(méthylsulfonyl)pyridin-2-yl)amino)-5-(1-méthyl-1H-pyrazol-3-yl)pyridin-2-yl)acétamide ;
N-(5-(1-méthyl-1H-pyrazol-3-yl)-4-((5-méthyl-6-(méthylsulfonyl)pyridin-2-yl)amino)pyridin-2-yl)acétamide ;
N-(5-(1-méthyl-1H-pyrazol-3-yl)-4-((6-(méthylsulfonyl)-4-(trifluorométhyl)pyridin-2-yl)amino)pyridin-2-yl)acétamide ;
N-(4-((6-(éthylsulfonyl)-4-isopropoxypyridin-2-yl)amino)-5-(1-méthyl-1H-pyrazol-3-yl)pyridin-2-yl)acétamide ;
N-(4-((5-fluoro-6-(méthylsulfonyl)pyridin-2-yl)amino)-5-(1-méthyl-1H-pyrazol-3-yl)pyridin-2-yl)acétamide ;
(S)-N-(4-((3-(2-hydroxypropoxy)-5-(méthylsulfonyl)phényl)amino)-5-(1-méthyl-1H-pyrazol-3-yl)pyridin-2-yl)acétamide ;
N-(4-((3-(2-hydroxyéthoxy)-5-(méthylsulfonyl)phényl)amino)-5-(1-méthyl-1H-pyrazol-3-yl)pyridin-2-yl)acétamide ;
(R)-N-(4-((3-(2-hydroxypropoxy)-5-(méthylsulfonyl)phényl)amino)-5-(1-méthyl-1H-pyrazol-3-yl)pyridin-2-yl)acétamide ;
N-(4-((4-(difluorométhoxy)-6-(méthylsulfonyl)pyridin-2-yl)amino)-5-(1-méthyl-1H-pyrazol-3-yl)pyridin-2-yl)acétamide ;
N-(4-((4-isobutoxy-6-(méthylsulfonyll)pyridin-2-yl)amino)-5-(1-méthyl-1H-pyrazol-3-yl)pyridin-2-yl)acétamide ;
(S)-N-(4-((4-(sec-butoxy)-6-(méthyllsulfonyl)pyridin-2-yl)amino)-5-(1-méthyl-1H-pyrazol-3-yl)pyridin-2-yl)acétamide ;
N-(5-(1-méthyl-1H-pyrazol-3-yl)-4-((6-(méthylsulfonyl)-4-(tétrahydro-2H-pyran-4-yl)pyridin-2-yl)amino)pyridin-2-yl)acétamide ;
N-(4-((3-(difluorométhoxy)-5-(méthylsulfonyl)phényl)amino)-5-(1-méthyl-1H-pyrazol-3-yl)pyridin-2-yl)acétamide ;
N-(4-((3-(difluorométhoxy)-6-(méthylsulfonyl)pyridin-2-yl)amino)-5-(1-méthyl-1H-pyrazol-3-yl)pyridin-2-yl)acétamide ;
N-(5-(1-méthyl-1H-pyrazol-3-yl)-4-((7-(méthylsulfonyl)-2,3-dihydro-[1,4]dioxino[2,3-c]pyridin-5-yl)amino)pyridin-2-yl)acétamide ;
(R)-N-(4-((4-(sec-butoxy)-6-(méthylsulfonyl)pyridin-2-yl)amino)-5-(1-méthyl-1H-pyrazol-3-yl)pyridin-2-yl)acétamide ;
N-(5-(1-cyclopropyl-1H-pyrazol-3-yl)-4-((6-(méthylsulfonyl)pyridin-2-yl)amino)pyridin-2-yl)acétamide ;
N-(5-(1-cyclopropyl-1H-pyrazol-3-yl)-4-((4-isopropoxy-6-(méthylsulfonyl)pyridin-2-yl)amino)pyridin-2-yl)acétamide ;
N-(5-(1-cyclopropyl-1H-pyrazol-3-yl)-4-((4-méthyl-6-(méthylsulfonyl)pyridin-2-yl)amino)pyridin-2-yl)acétamide ;
N-(5-(1-cyclopropyl-1H-pyrazol-3-yl)-4-((4-(2-méthoxyéthoxy)-6-(méthylsulfonyl)pyridin-2-yl)amino)pyridin-2-yl)acétamide ;
N-(5-(1-éthyl-1H-pyrazol-3-yl)-4-((6-(méthylsulfonyl)pyridin-2-yl)amino)pyridin-2-yl)acétamide ;
N-(5-(1-éthyl-1H-pyrazol-3-yl)-4-((4-isopropoxy-6-(méthylsulfonyl)pyridin-2-yl)amino)pyridin-2-yl)acétamide ;
N-(5-(1-éthyl-1H-pyrazol-3-yl)-4-((4-(méthoxyméthyl)-6-(méthylsulfonyl)pyridin-2-yl)amino)pyridin-2-yl)acétamide ;
N-(5-(1-éthyl-1H-pyrazol-3-yl)-4-((3-(méthylsulfonyl)phényl)amino)pyridin-2-yl)acétamide ;
N-(5-(1-éthyl-1H-pyrazol-3-yl)-4-((3-méthoxy-5-(méthylsulfonyl)phényl)amino)pyridin-2-yl)acétamide ;
N-(4-((3-cyano-5-(méthylsulfonyl)phényl)amino)-5-(1-éthyl-1H-pyrazol-3-yl)pyridin-2-yl)acétamide ;
N-(5-(1-éthyl-1H-pyrazol-3-yl)-4-((3-méthyl-5-(méthylsulfonyl)phényl)amino)pyridin-2-yl)acétamide ;
N-(5-(1-éthyl-1H-pyrazol-3-yl)-4-((3-(méthoxyméthyl)-5-(méthylsulfonyl)phényl)amino)pyridin-2-yl)acétamide ;
N-(5-(1-éthyl-1H-pyrazol-3-yl)-4-((3-(méthylsulfonyl)-5-(trifluorométhyl)phényl)amino)pyridin-2-yl)acétamide ;
N-(5-(1-éthyl-1H-pyrazol-3-yl)-4-((3-(2-méthoxyéthoxy)-5-(méthylsulfonyl)phényl)amino)pyridin-2-yl)acétamide ;
N-(5-(1-éthyl-1H-pyrazol-3-yl)-4-((3-(2-hydroxyéthoxy)-5-(méthylsulfonyl)phényl)amino)pyridin-2-yl)acétamide ;
N-(4-((3-(cyclopropylméthoxy)-5-(méthylsulfonyl)phényl)amino)-5-(1-éthyl-1H-pyrazol-3-yl)pyridin-2-yl)acétamide ;
N-(4-((4-(cyclopentyl)oxy)-6-(méthylsulfonyl)pyridin-2-yl)amino)-5-(1-éthyl-1H-pyrazol-3-yl)pyridin-2-yl)acétamide ;
N-(4-((4-éthoxy-6-(méthylsulfonyl)pyridin-2-yl)amino)-5-(1-éthyl-1H-pyrazol-3-yl)pyridin-2-yl)acétamide ;
N-(5-(1-éthyl-1H-pyrazol-3-yl)-4-((4-(2-hydroxyéthoxy)-6-(méthylsulfonyl)pyridin-2-yl)amino)pyridin-2-yl)acétamide ;
N-(5-(1-éthyl-1H-pyrazol-3-yl)-4-((4-(2-méthoxyéthoxy)-6-(méthylsulfonyl)pyridin-2-yl)amino)pyridin-2-yl)acétamide ;
N-(5-(1-éthyl-1H-pyrazol-3-yl)-4-((3-isopropoxy-6-(méthylsulfonyl)pyridin-2-yl)amino)pyridin-2-yl)acétamide ;
N-(5-(1-isopropyl-1H-pyrazol-3-yl)-4-( (6-(méthylsulfonyl)pyridin-2-yl)amino)pyridin-2-yl)acétamide ;
N-(4-((4-isopropoxy-6-(méthylsulfonyl)pyridin-2-yl)amino)-5-(1-isopropyl-1H-pyrazol-3-yl)pyridin-2-yl)acétamide ;
N-(5-(1-isopropyl-1H-pyrazol-3-yl)-4-((4-(2-méthoxyéthoxy)-6-(méthylsulfonyl)pyridin-2-yl)amino)pyridin-2-yl)acétamide ;
N-(5-(1-isopropyl-1H-pyrazol-3-yl)-4-((3-(méthylsulfonyl)-5-(trifluorométhyl)phényl)amino)pyridin-2-yl)acétamide ;
N-(4-((3-isopropoxy-5-(méthylsulfonyl)phényl)amino)-5-(1-isopropyl-1H-pyrazol-3-yl)pyridin-2-yl)acétamide ;
N-(5-(1-isopropyl-1H-pyrazol-3-yl)-4-((3-méthoxy-5-(méthylsulfonyl)phényl)amino)pyridin-2-yl)acétamide ;
N-(4-((4-(2-hydroxyéthoxy)-6-(méthylsulfonyl)pyridin-2-yl)amino)-5-(1-isopropyl-1H-pyrazol-3-yl)pyridin-2-yl)acétamide ;
N-(4-((3-(2-hydroxyéthoxy)-5-(méthylsulfonyl)phényl)amino)-5-(1-isopropyl-1H-pyrazol-3-yl)pyridin-2-yl)acétamide ;
N-(4-((3-(méthylsulfonyl)phényl)amino)-5-(1-(tétrahydrofuran-3-yl)-1H-pyrazol-3-yl)pyridin-2-yl)acétamide ;
N-(4-((4-méthyl-6-(méthylsulfonyl)pyridin-2-yl)amino)-5-(1-(tétrahydrofuran-3-yl)-1H-pyrazol-3-yl)pyridin-2-yl)acétamide ;
(R)-N-(4-((3-(méthylsulfonyl)-5-(trifluorométhyl)phényl)amino)-5-(1-(tétrahydrofuran-3-yl)-1H-pyrazol-3-yl)pyridin-2-yl)acétamide ;
(S)-N-(4-((4-isopropoxy-6-(méthylsulfonyl)pyridin-2-yl)amino)-5-(1-(tétrahydrofuran-3-yl)-1H-pyrazol-3-yl)pyridin-2-yl)acétamide ;
(R)-N-(4-((4-isopropoxy-6-(méthylsulfonyl)pyridin-2-yl)amino)-5-(1-(tétrahydrofuran-3-yl)-1H-pyrazol-3-yl)pyridin-2-yl)acétamide ;
(R)-N-(4-((4-méthoxy-6-(méthylsulfonyl)pyridin-2-yl)amino)-5-(1-(tétrahydrofuran-3-yl)-1H-pyrazol-3-yl)pyridin-2-yl)acétamide ;
N-(5-(1,5-diméthyl-1H-pyrazol-3-yl)-4-((6-(méthylsulfonyl)pyridin-2-yl)amino)pyridin-2-yl)acétamide ;
N-(5-(1,5-diméthyl-1H-pyrazol-3-yl)-4-((4-isopropoxy-6-(méthylsulfonyl)pyridin-2-yl)amino)pyridin-2-yl)acétamide ;
N-(5-(1,5-diméthyl-1H-pyrazol-3-yl)-4-((3-(2-hydroxyéthoxy)-5-(méthylsulfonyl)phényl)amino)pyridin-2-yl)acétamide ;
N-(5-(1,5-diméthyl-1H-pyrazol-3-yl)-4-((4-méthoxy-6-(méthylsulfonyl)pyridin-2-yl)amino)pyridin-2-yl)acétamide ;
N-(5-(1,5-diméthyl-1H-pyrazol-3-yl)-4-((4-(2-méthoxyéthoxy)-6-(méthylsulfonyl)pyridin-2-yl)amino)pyridin-2-yl)acétamide ;
(R)-N-(5-(1,5-diméthyl-1H-pyrazol-3-yl)-4-((4-(2-méthoxypropoxy)-6-(méthylsulfonylpyridin-2-yl)amino)pyridin-2-yl)acétamide ;
N-(5-(1,5-diméthyl-1H-pyrazol-3-yl)-4-((4-éthoxy-6-(méthylsulfonyl)pyridin-2-yl)amino)pyridin-2-yl)acétamide ;
N-(5-(1-méthyl-5-morpholino-1H-pyrazol-3-yl)-4-((6-(méthylsulfonyl)pyridin-2-yl)amino)pyridin-2-yl)acétamide ;
N-(4-((4-(2-méthoxyéthoxy)-6-(méthylsulfonyl)pyridin-2-yl)amino)-5-(1-méthyl-5-morpholino-1H-pyrazol-3-yl)pyridin-2-yl)acétamide ;
N-(4-((4-(2-hydroxypropoxy)-6-(méthylsulfonyl)pyridin-2-yl)amino)-5-(1-méthyl-5-morpholino-1H-pyrazol-3-yl)pyridin-2-yl)acétamide ;
N-(5-(2,3-dihydro-[1,4]dioxino[2,3-b]pyridin-6-yl)-4-((4-isopropoxy-6-(méthylsulfonyl)pyridine-2-yl)amino)pyridin-2-yl)acétamide ;
N-(5-([1,3]dioxolo[4,5-b]pyridin-5-yl)-4-((4-isopropoxy-6-(méthylsulfonyl)pyridin-2-yl)amino)pyridin-2-yl)acétamide ;
N-(5-(2,3-dihydro-[1,4]dioxino[2,3-b]pyridin-6-yl)-4-((4-méthyl-6-(méthylsulfonyl)pyridin-2-yl)amino)pyridin-2-yl)acétamide ;
N-(5-(3,4-dihydro-2H-pyrido[4,3-b][1,4]oxazin-7-yl)-4-((4-isopropoxy-6-(méthylsulfonyl)pyridin-2-yl)amino)pyridin-2-yl)acétamide ;
N-(5-(imidazo[1,2-b]pyridazin-6-yl)-4-((4-isopropoxy-6-(méthylsulfonyl)pyridin-2-yl)amino)pyridin-2-yl)acétamide ;
N-(5-(6,7-dihydro-4H-pyrazolo[5,1-c][1,4]oxazin-2-yl)-4-((4-isopropoxy-6-(méthylsulfonyl)pyridin-2-yl)amino)pyridin-2-yl)acétamide ;
N-(5-(4-acétyl-3,4-dihydro-2H-pyrido[3,2-b][1,4]oxazin-6-yl)-4-((4-isopropoxy-6-(méthylsulfonyl)pyridin-2-yl)amino)pyridin-2-yl)acétamide ;
N-(5-(2,3-dihydro-[1,4]dioxino[2,3-c]pyridin-7-yl)-4-((4-méthoxy-6-(méthylsulfonyl)pyridin-2-yl)amino)pyridin-2-yl)acétamide ;
N-(5-(benzo[b]thiophén-2-yl)-4-((4-méthoxy-6-(méthylsulfonyl)pyridin-2-yl)amino)pyridin-2-yl)acétamide ;
N-(5-(6,7-dihydro-4H-thiéno[3,2-c]pyran-2-yl)-4-((4-méthyl-6-(méthylsulfonyl)pyridin-2-yl)amino)pyridin-2-yl)acétamide ;
N-(5-(2,3-dihydro-[1,4]dioxino[2,3-b]pyridin-6-yl)-4-((4-méthoxy-6-(méthylsulfonyl)pyrnidin-2-yl)amino)pyridin-2-yl)acétamide ;
N-(5-(2,3-dihydro-[1,4]dioxino[2,3-c]pyridin-7-yl)-4-((4-éthoxy-6-(méthylsulfonyl)pyridin-2-yl)amino)pyridin-2-yl)acétamide ;
N-(5-(2,3-dihydro-[1,4]dioxino[2,3-c]pyridin-7-yl)-4-((4-(2-méthoxyéthoxy)-6-(méthylsulfonyl)pyridin-2-yl)amino)pyridin-2-yl)acétamide ;
N-(5-([1,3]dioxolo[4,5-b]pyridin-5-yl)-4-((4-(2-méthoxyéthoxy)-6-(méthylsulfonyl)pyridin-2-yl)amino)pyridin-2-yl)acétamide ;
N-(5-([1,3]dioxolo[4,5-b]pyridin-5-yl)-4-((4-éthoxy-6-(méthylsulfonyl)pyridin-2-yl)amino)pyridin-2-yl)acétamide ;
N-(5-([1,3]dioxolo[4,5-b]]pyridin-5-yl)-4-((4-méthoxy-6-(méthylsulfonyl)pyridin-2-yl)amino)pyridin-2-yl)acétamide ;
N-(4-((4-(cyclopropylméthoxy)-6-(méthylsulfonyl)pyridin-2-yl)amino)-5-(5-méthyl-4,5,6,7-tétrahydrothiazolo[5,4-c]pyridin-2-yl)pyridin-2-yl)acétamide ;
N-(5-(2,3-dihydro-[1,4]dioxino[2,3-c]pyridin-7-yl)-4-((4-méthyl-6-(méthylsulfonyl)pyridin-2-yl)amino)pyridin-2-yl)acétamide ;
N-(5-(2,3-dihydro-[1,4]dioxino[2,3-c]pyridin-7-yl)-4-((4-(2-méthoxyéthoxy)-6-(méthylsulfonyl)pyridin-2-yl)amino)pyridin-2-yl)acétamide ;
N-(5-(6,7-dihydro-4H-pyrazolo[5,1-c][1,4]oxazin-2-yl)-4-((4-méthyl-6-(méthylsulfonyl)pyridin-2-yl)amino)pyridin-2-yl)acétamide ;
N-(4-((4-((1,4-dioxan-2-yl)méthoxy)-6-(méthylsulfonyl)pyridin-2-yl)amino)-5-(3,3-diméthyl-2,3-dihydro-[1,4]dioxino[2,3-b]pyridin-6-yl)pyridin-2-yl)acétamide ;
N-(5-(3,3-diméthyl-2,3-dihydro-[1,4]dioxino[2,3-b]pyridine-6-yl)-4-((6-(méthylsulfonyl)pyrazin-2-yl)amino)pyridine-2-yl)acétamide ;
N-(5-(3,3-diméthyl-2,3-dihydro-[1,4]dioxino[2,3-b]yridine-6-yl)-4-((4-(2-hydroxypropan-2-yl)-6-(méthylsulfonyl)pyridine-2-yl)amino)pyridine-2-yl)acétamide ;
N-(5-(3,3-diméthyl-2,3-dihydro-[1,4]dioxino[2,3-b]pyridine-6-yl)-4-((5-fluoro-4-méthyl-6-(méthylsulfonyl)pyridine-2-yl)amino)pyridine-2-yl)acétamide ;
N-(5-(3,3-diméthyl-2,3-dihydro-[1,4]dioxino[2,3-b]pyridin-6-yl)-4-((6-(méthylsulfonyl)-4-morpholinopyridin-2-yl)amino)pyridin-2-yl)acétamide ;
N-(5-(2,2-diméthyl-2,3-dihydro-[1,4]dioxino[2,3-b]pyridin-6-yl)-4-((4-méthyl-6-(méthylsulfonyl)pyridin-2-yl)amino)pyridin-2-yl)acétamide ;
N-(5-(2,2-diméthyl-2,3-dihydro-[1,4]dioxino[2,3-b]pyridin-6-yl)-4-((6-(méthylsulfonyl)-4-morpholinopyridin-2-yl)amino)pyridin-2-yl)acétamide ;
N-(5-(2,2-diméthyl-2,3-dihydro-[1,4]dioxino[2,3-b]pyridin-6-yl)-4-((6-(méthylsulfonyl)-4-(tétrahydro-2H-pyran-4-yl)pyridin-2-yl)amino)pyridin-2-yl)acétamide ;
N-(5-(2,2-diméthyl-2,3-dihydro-[1,4]dioxino[2,3-b]pyridin-6-yl)-4-((4-(méthoxy-d3)-6-(méthylsulfonyl)pyridin-2-yl)amino)pyridin-2-yl)acétamide ;
N-(5-(2,2-diméthyl-2,3-dihydro-[1,4]dioxino[2,3-b]pyridin-6-yl)-4-((4-méthoxy-6-(méthylsulfonyl)pyridin-2-yl)amino)pyridin-2-yl)acétamide ;
N-(4-((3,4-diméthoxy-6-(méthylsulfonyl)pyridin-2-yl)amino)-5-(2,2-diméthyl-2,3-dihydro-[1,4]dioxino[2,3-b]pyridin-6-yl)pyridin-2-yl)acétamide ; (S)-N-(5-(2,2-diméthyl-2,3-dihydro-[1,4]dioxino[2,3-b]pyridin-6-yl)-4-((4-(3-méthyl-morpholino)-6-(méthylsulfonyl)pyridin-2-yl)amino)-pyridin-2-yl)acétamide ;
(R)-N-(5-(2,2-diméthyl-2,3-dihydro-[1,4]dioxino[2,3-b]pyridin-6-yl)-4-((4-(3-méthyl-morpholino)-6-(méthylsulfonyl)pyridin-2-yl)amino)-pyridin-2-yl)acétamide ;
N-(5-(3,3-diméthyl-2,3-dihydro-[1,4]dioxino[2,3-b]pyridin-6-yl)-4-((4-(méthoxy-d3)-6-(méthylsulfonyl)pyridin-2-yl)amino)pyridin-2-yl)acétamide ;
N-(5-(3,3-diméthyl-2,3-dihydro-[1,4]dioxino[2,3-b]pyridin-6-yl)-4-((4-méthoxy-6-(méthylsulfonyl)pyridin-2-yl)amino)pyridin-2-yl)acétamide ;
N-(5-(3,3-diméthyl-2,3-dihydro-[1,4]dioxino[2,3-b]pyridin-6-yl)-4-((6-(méthylsulfonyl)-4-(tétrahydro-2H-pyran-4-yl)pyridin-2-yl)amino)pyridin-2-yl)acétamide ;
(R)-N-(5-(3,3-diméthyl-2,3-dihydro-[1,4]dioxino[2,3-b]pyridin-6-yl)-4-((4-(3-méthylmorpholino)-6-(méthylsulfonyl)pyridin-2-yl)amino)pyridin-2-yl)acétamide ;
(S)-N-(5-(3,3-diméthyl-2,3-dihydro-[1,4]dioxino[2,3-b]pyridin-6-yl)-4-((4-(3-méthylmorpholino)-6-(méthylsulfonyl)pyridin-2-yl)amino)pyridin-2-yl)acétamide ;
(S)-N-(5-(3,3-diméthyl-2,3-dihydro-[1,4]dioxino[2,3-b]pyridin-6-yl)-4-((4-(1-méthoxyéthyl)-6-(méthylsulfonyl)pyridin-2-yl)amino)pyridin-2-yl)acétamide ;
(R)-N-(5-(3,3-diméthyl-2,3-dihydro-[1,4]dioxino[2,3-b]pyridin-6-yl)-4-((4-(1-méthoxyéthyl)-6-(méthylsulfonyl)pyridin-2-yl)amino)pyridin-2-yl)acétamide ;
(R)-N-(5-(2,2-diméthyl-2,3-dihydro-[1,4]dioxino[2,3-b]pyridin-6-yl)-4-((4-(3-méthoxypyrrolidin-1-yl)-6-(méthylsulfonyl)pyridin-2-yl)amino)pyridin-2-yl)acétamide ;
(S)-N-(5-(2,2-diméthyl-2,3-dihydro-[1,4]dioxino[2,3-b]pyridin-6-yl)-4-((4-(3-méthoxypyrrolidin-1-yl)-6-(méthylsulfonyl)pyridin-2-yl)amino)pyridin-2-yl)acétamide ;
N-(4-((4-(4-aminotétrahydro-2H-pyran-4-yl)-6-(méthylsulfonyl)pyridin-2-yl)amino)-5-(2,2-diméthyl-2,3-dihydro-[1,4]dioxino[2,3-b]pyridin-6-yl)pyridin-2-yl)acétamide ;
N-(4-((4-(4-(aminométhyl)tétrahydro-2H-pyran-4-yl)-6-(méthylsulfonyl)pyridin-2-yl)amino)-5-(2,2-diméthyl-2,3-dihydro-[1,4]dioxino[2,3-b]pyridin-6-yl)pyridin-2-yl)acétamide ;
N-(5-(2,2-diméthyl-2,3-dihydro-[1,4]dioxino[2,3-b]pyridin-6-yl)-4-((4-(3-méthoxyazétidin-1-yl)-6-(méthylsulfonyl)pyridin-2-yl)amino)pyridin-2-yl)acétamide ;
(R/S)-N-(4-((4-(1,4-dioxan-2-yl)-6-(méthylsulfonyl)pyridin-2-yl)amino)-5-(2,2-diméthyl-2,3-dihydro-[1,4]dioxino[2,3-b]pyridin-6-yl)pyridin-2-yl)acétamide ;
(S/R)-N-(4-((4-(1,4-dioxan-2-yl)-6-(méthylsulfonyl)pyridin-2-yl)amino)-5-(2,2-diméthyl-2,3-dihydro-[1,4]dioxino[2,3-b]pyridin-6-yl)pyridin-2-yl)acétamide ;
N-(5-(2,2-diméthyl-2,3-dihydro-[1,4]dioxino[2,3-b]pyridin-6-yl)-4-((4-(4-méthoxypipéridin-1-yl)-6-(méthylsulfonyl)pyridin-2-yl)amino)pyridin-2-yl)acétamide ;
N-(5-(2,2-diméthyl-2,3-dihydro-[1,4]dioxino[2,3-b]pyridin-6-yl)-4-((4-((cis)-3-méthoxycyclobutyl)-6-(méthylsulfonyl)pyridin-2-yl)amino)pyridin-2-yl)acétamide ;
N-(5-(2,2-diméthyl-2,3-dihydro-[1,4]dioxino[2,3-b]pyridin-6-yl)-4-((4-((trans)-3-méthoxycyclobutyl)-6-(méthylsulfonyl)pyridin-2-yl)amino)pyridin-2-yl)acétamide ;
N-(5-(2,2-diméthyl-2,3-dihydro-[1,4]dioxino[2,3-b]pyridin-6-yl)-4-((4-(4-hydroxy-4-méthylpipéridin-1-yl)-6-(méthylsulfonyl)pyridin-2-yl)amino)pyridin-2-yl)acétamide ;
N-(5-(2,2-diméthyl-2,3-dihydro-[1,4]dioxino[2,3-b]pyridin-6-yl)-4-((4-(3-hydroxy-3-méthylazétidin-1-yl)-6-(méthylsulfonyl)pyridin-2-yl)amino)pyrid-in-2-yl)acétamide ;
N-(5-(2,2-diméthyl-2,3-dihydro-[1,4]dioxino[2,3-b]pyridin-6-yl)-4-((4-(3-hydroxy-3-méthylpyrrolidin-1-yl)-6-(méthyl sulfonyl)pyridin-2-yl)amino)pyridin-2-yl) acétamide ;
N-(6-(2,2-diméthyl-2,3-dihydro-[1,4]dioxino[2,3-b]pyridin-6-yl)-5-((6-(méthylsulfonyl)-4-(tétrahydro-2H-pyran-4-yl)pyridin-2-yl)amino)pyridazin-3-yl)acétamide ;
(R)-N-(5-(2,2-diméthyl-2,3-dihydro-[1,4]dioxino[2,3-b]pyridin-6-yl)-4-((4-(3-(2-hydroxyéthoxy)pyrrolidin-1-yl)-6-(méthylsulfonyl)pyridin-2-yl)amino)pyridin-2-yl)acétamide ;
N-(5-(2,2-diméthyl-2,3-dihydro-[1,4]dioxino[2,3-b]pyridin-6-yl)-4-((6-(méthylsulfonyl)-4-(1-(2,2,2-trifluoroéthyl)pipéridin-4-yl)pyridin-2-yl)amino)pyridin-2-yl) acétamide ;
(S)-N-(5-(2,2-diméthyl-2,3-dihydro-[1,4]dioxino[2,3-b]pyridin-6-yl)-4-((4-3-(2-hydroxyéthoxy)pyrrolidin-1-yl)-6-(méthylsulfonyl)pyridin-2-yl)amino)pyridin-2-yl)acétamide ;
N-(5-(2,2-diméthyl-2,3-dihydro-[1,4]dioxino[2,3-b]pyridin-6-yl)-4-((4-méthyl-6'-(méthylsulfonyl)-[3,4'-bipyridin]-2'-yl)amino)pyridin-2-yl)acétamide ;
N-(4-((4-cyano-6-(méthylsulfonyl)pyridin-2-yl)amino)-5-(2,2-diméthyl-2,3-dihydro-[1,4]dioxino[2,3-b]pyridin-6-yl)pyridin-2-yl)acétamide ;
N-(5-(2,2-diméthyl-2,3-dihydro-[1,4]dioxino[2,3-b]pyridin-6-yl)-4-((4-méthyl-6-(méthylsulfonyl)-3-oxo-3,4-dihydropyrazin-2-yl)amino)pyridin-2-yl)acétamide ;
(S)-N-(5-(2,2-diméthyl-2,3-dihydro-[1,4]dioxino[2,3-b]pyridin-6-yl)-4-((4-(3-méthoxypipéridin-1-yl)-6-(méthylsulfonyl)pyridin-2-yl)amino)pyridin-2-yl)acétamide ;
(R)-N-(5-(2,2-diméthyl-2,3-dihydro-[1,4]dioxino[2,3-b]pyridin-6-yl)-4-((4-(3-méthoxypipéridin-1-yl)-6-(méthylsulfonyl)pyridin-2-yl)amino)pyridin-2-yl)acétamide ;
(S)-N-(5-(2,2-diméthyl-2,3-dihydro-[1,4]dioxino[2,3-b]pyridin-6-yl)-4-((4-(3-éthoxypipéridin-1-yl)-6-(méthylsulfonyl)pyridin-2-yl)amino)pyridin-2-yl)acétamide ;
(R)-N-(5-(2,2-diméthyl-2,3-dihydro-[1,4]dioxino[2,3-b]pyridin-6-yl)-4-((4-(3-éthoxypipéridin-1-yl)-6-(méthylsulfonyl)pyridin-2-yl)amino)pyridin-2-yl)acétamide ;
(S)-N-(5-(2,2-diméthyl-2,3-dihydro-[1,4]dioxino[2,3-b]pyridin-6-yl)-4-((6-(méthylsulfonyl)-4-(3-(trifluorométhoxy)pyrrolidin-1-yl)pyridin-2-yl)amino)pyridin-2-yl)acétamide ;
(R)-N-(5-(2,2-diméthyl-2,3-dihydro-[1,4]dioxino[2,3-b]pyridin-6-yl)-4-((6-(méthylsulfonyl)-4-(3-(trifluorométhoxy)pyrrolidin-1-yl)pyridin-2-yl)amino)pyridin-2-yl)acétamide ;
(S)-1-(2-((2-acétamido-5-(2,2-diméthyl-2,3-dihydro-[1,4]dioxino[2,3-b]pyridin-6-yl)pyridin-4-yl)amino)-6-(méthylsulfonyl)pyridin-4-yl)pyrrolidine-2-carboxamide ;
(R)-1-(2-((2-acétamido-5-(2,2-diméthyl-2,3-dihydro-[1,4]dioxino[2,3-b]pyridin-6-yl)pyridin-4-yl)amino)-6-(méthylsulfonyl)pyridin-4-yl)pyrrolidine-2-carboxamide ;
(S)-N-(5-(2,2-diméthyl-2,3-dihydro-[1,4]dioxino[2,3-b]pyridin-6-yl)-4-((4-3-(méthoxyméthyl)pyrrolidin-1-yl)-6-(méthylsulfonyl)pyridin-2-yl)amino)pyridin-2-yl)acétamide ;
(R)-N-(5-(2,2-diméthyl-2,3-dihydro-[1,4]dioxino[2,3-b]pyridin-6-yl)-4-((4-(3-(méthoxyméthyl)pyrrolidin-1-yl)-6-(méthylsulfonyl)pyridin-2-yl)amino)pyridin-2-yl)acétamide ;
(S)-N-(5-(2,2-diméthyl-2,3-dihydro-[1,4]dioxino[2,3-b]pyridin-6-yl)-4-((6-(méthylsulfonyl)-4-(tétrahydro-2H-pyran-3-yl)pyridin-2-yl)amino)pyridin-2-yl)acétamide ;
(R)-N-(5-(2,2-diméthyl-2,3-dihydro-[1,4]dioxino[2,3-b]pyridin-6-yl)-4-((6-(méthylsulfonyl)-4-(tétrahydro-2H-pyran-3-yl)pyridin-2-yl)amino)pyridin-2-yl)acétamide ;
(S)-N-(5-(2,2-diméthyl-2,3-dihydro-[1,4]dioxino[2,3-b]pyridin-6-yl)-4-((5-3-méthoxypyrrolidin-1-yl)-6-(méthylsulfonyl)pyridin-2-yl)amino)pyridin-2-yl)acétamide ;
(R)-N-(5-(2,2-diméthyl-2,3-dihydro-[1,4]dioxino[2,3-b]pyridin-6-yl)-4-((5-(3-méthoxypyrrolidin-1-yl)-6-(méthylsulfonyl)pyridin-2-yl)amino)pyridin-2-yl)acétamide ;
N-(5-(2,2-diméthyl-2,3-dihydro-[1,4]dioxino[2,3-b]pyridin-6-yl)-4-((6-(méthylsulfonyl)-5-(tétrahydro-2H-pyran-4-yl)pyridin-2-yl)amino)pyridin-2-yl)acétamide ;
N-(5-(2,2-diméthyl-2,3-dihydro-[1,4]dioxino[2,3-b]pyridin-6-yl)-4-((4-(5-méthylpynidazin-4-yl)-6-(méthylsulfonyl)pyridin-2-yl)amino)pyridin-2-yl)acétamide ;
N-(5-(2,2-diméthyl-2,3-dihydro-[1,4]dioxino[2,3-b]pyridin-6-yl)-4-((4-(5-méthoxypyridazin-4-yl)-6-(méthylsulfonyl)pyridin-2-yl)amino)pyridin-2-yl)acétamide ;
N-(4-((4-(cis-3-cyano-4-hydroxypyrrolidin-1-yl)-6-(méthylsulfonyl)pyridin-2-yl)amino)-5-(2,2-diméthyl-2,3-dihydro-[1,4]dioxino[2,3-b]pyridin-6-yl)pyridin-2-yl)acétamide ;
N-(4-((4-(trans-3-cyano-4-hydroxypyrrolidin-1-yl)-6-(méthylsulfonyl)pyridin-2-yl)amino)-5-(2,2-diméthyl-2,3-dihydro-[1,4]dioxino[2,3-b]pyridin-6-yl)pyridin-2-yl)acétamide ;
N-(4-((4-(cis-3-cyano-4-méthoxypyrrolidin-1-yl)-6-(méthylsulfonyl)pyridin-2-yl)amino)-5-(2,2-diméthyl-2,3-dihydro-[1,4]dioxino[2,3-b]pyridin-6-yl)pyridin-2-yl)acétamide ;
N-(4-((4-(trans-3-cyano-4-méthoxypyrrolidin-1-yl)-6-(méthylsulfonyl)pyridin-2-yl)amino)-5-(2,2-diméthyl-2,3-dihydro-[1,4]dioxino[2,3-b]pyridin-6-yl)pyridin-2-yl)acétamide ;
(S)-N-(5-(2,2-diméthyl-2,3-dihydro-[1,4]dioxino[2,3-b]pyridin-6-yl)-4-((6-(méthylsulfonyl)-4-(tétrahydrofuran-3-yl)pyridin-2-yl)amino)pyridin-2-yl)acétamide ;
(R)-N-(5-(2,2-diméthyl-2,3-dihydro-[1,4]dioxino[2,3-b]pyridin-6-yl)-4-((6-(méthylsulfonyl)-4-(tétrahydrofuran-3-yl)pyridin-2-yl)amino)pyridin-2-yl)acétamide ;
(S)-N-(5-(2,2-diméthyl-2,3-dihydro-[1,4]dioxino[2,3-b]pyridin-6-yl)-4-((4-(2-(méthoxyméthyl)pyrrolidin-1-yl)-6-(méthylsulfonyl)pyridin-2-yl)amino)pyridin-2-yl)acétamide ;
(R)-N-(5-(2,2-diméthyl-2,3-dihydro-[1,4]dioxino[2,3-b]pyridin-6-yl)-4-((4-(2-(méthoxyméthyl)pyrrolidin-1-yl)-6-(méthylsulfonyl)pyridin-2-yl)amino)pyridin-2-yl)acétamide ; N-(5-(2,2-diméthyl-2,3-dihydro-[1,4]dioxino[2,3-b]pyridin-6-yl)-4-((6-(méthylsulfonyl)-3-(tétrahydro-2H-pyran-4-yl)pyridin-2-yl)amino)pyridin-2-yl)acétamide ;
(S)-N-(5-(2,2-diméthyl-2,3-dihydro-[1,4]dioxino[2,3-b]pyridin-6-yl)-4-((3-(3-méthoxypyrrolidin-1-yl)-6-(méthylsulfonyl)pyridin-2-yl)amino)pyridin-2-yl)acétamide ;
(R)-N-(5-(2,2-diméthyl-2,3-dihydro-[1,4]dioxino[2,3-b]pyridin-6-yl)-4-((3-(3-méthoxypyrrolidin-1-yl)-6-(méthylsulfonyl)pyridin-2-yl)amino)pyridin-2-yl)acétamide ;
cis-N-(5-(2,2-diméthyl-2,3-dihydro-[1,4]dioxino[2,3-b]pyridin-6-yl)-4-((4-((cis-4-hydroxycyclohexyl)oxy)-6-(méthylsulfonyl)pyridin-2-yl)amino)pyridin-2-yl)acétamide ;
N-(5-(2,2-diméthyl-2,3-dihydro-[1,4]dioxino[2,3-b]pyridin-6-yl)-4-((4-((trans-4-hydroxycyclohexyl)oxy)-6-(méthylsulfonyl)pyridin-2-yl)amino)pyridin-2-yl)acétamide ;
N-(5-(2,2-diméthyl-2,3-dihydro-[1,4]dioxino[2,3-b]pyridin-6-yl)-4-((4-((cis-4-méthoxycyclohexyl)oxy)-6-(méthylsulfonyl)pyridin-2-yl)amino)pyridin-2-yl)acétamide ;
N-(5-(2,2-diméthyl-2,3-dihydro-[1,4]dioxino[2,3-b]pyridin-6-yl)-4-((4-((trans-4-méthoxycyclohexyl)oxy)-6-(méthylsulfonyl)pyridin-2-yl)amino)pyridin-2-yl)acétamide ;
N-(5-(2,2-diméthyl-2,3-dihydro-[1,4]dioxino[2,3-b]pyridin-6-yl)-4-((6-(méthylsulfonyl)-4-(5-azaspiro[2.4]heptan-5-yl)pyridin-2-yl)amino)pyridin-2-yl)acétamide ;
N-(5-(2,2-diméthyl-2,3-dihydro-[1,4]dioxino[2,3-b]pyridin-6-yl)-4-((4-((3-méthyloxétan-3-yl)méthoxy)-6-(méthylsulfonyl)pyridin-2-yl)amino)pyridin-2-yl)acétamide ;
N-(5-(2,2-diméthyl-2,3-dihydro-[1,4]dioxino[2,3-b]pyridin-6-yl)-4-((4-(3,5-diméthylisoxazol-4-yl)-6-(méthylsulfonyl)pyridin-2-yl)amino)pyridin-2-yl)acétamide ;
N-(5-(2,2-diméthyl-2,3-dihydro-[1,4]dioxino[2,3-b]pyridin-6-yl)-4-((4-((2,4-dioxothiazolidin-3-yl)méthyl)-6-(méthylsulfonyl)pyridin-2-yl)amino)pyridin-2-yl)acétamide ;
N-(5-(2,2-diméthyl-2,3-dihydro-[1,4]dioxino[2,3-b]pyridin-6-yl)-4-((4-méthoxy-6'-(méthylsulfonyl)-[3,4'-bipyridin]-2'-yl)amino)pyridin-2-yl)acétamide ;
(R)-N-(5-(2,2-diméthyl-2,3-dihydro-[1,4]dioxino[2,3-blpyridin-6-yl)-4-((4-(3-méthylpyrrolidin-1-yl)-6-(méthylsulfonyl)pyridin-2-yl)amino)pyridin-2-yl)acétamide ;
(S)-N-(5-(2,2-diméthyl-2,3-dihydro-[1,4]dioxino[2,3-b]pyridin-6-yl)-4-((4-(3-méthylpyrrolidin-1-yl)-6-(méthylsulfonyl)pyridin-2-yl)amino)pyridin-2-yl)acétamide ;
N-(5-(2,2-diméthyl-2,3-dihydro-[1,4]dioxino[2,3-b]pyridin-6-yl)-4-((6-(méthylsulfonyl)-4-(pyrrolidin-1-yl)pyridin-2-yl)amino)pyridin-2-yl)acétamide ;
N-(5-(2,2-diméthyl-2,3-dihydro-[1,4]dioxino[2,3-b]pyridin-6-yl)-4-((4-(2-hydroxypropan-2-yl)-6'-(méthylsulfonyl)-[3,4'-bipyridin]-2'-yl)amino)pyridin-2-yl)acétamide ;
N-(4-((4-(-azabicyclo[3.1.0]hexan-3-yl)-6-(méthylsulfonyl)pyridin-2-yl)amino)-5-(2,2-diméthyl-2,3-dihydro-[1,4]dioxino[2,3-b]pyridin-6-yl)pyridin-2-yl)acétamide ;
(S)-N-(4-((4-(3-(cyanométhoxy)pyrrolidin-1-yl)-6-(méthylsulfonyl)pyridin-2-yl)amino)-5-(2,2-diméthyl-2,3-dihydro-[1,4]dioxino[2,3-b]pyridin-6-yl)pyridin-2-yl)acétamide ;
N-(4-((6-cyano-6'-(méthylsulfonyl)-[3,4'-bipyridin]-2'-yl)amino)-5-(2,2-diméthyl-2,3-dihydro-[1,4]dioxino[2,3-b]pyridin-6-yl)pyridin-2-yl)acétamide ;
N-(4-((2-cyano-6'-(méthylsulfonyl)-[3,4'-bipyridin]-2'-yl)amino)-5-(2,2-diméthyl-2,3-dihydro-[1,4]dioxino[2,3-b]pyridin-6-yl)pyridin-2-yl)acétamide ;
N-(5-(2,2-diméthyl-2,3-dihydro-[1,4]dioxino[2,3-b]pyridin-6-yl)-4-((4-(3-méthylpyrazin-2-yl)-6-(méthylsulfonyl)pyridin-2-yl)amino)pyridin-2-yl)acétamide ;
N-(5-(2,2-diméthyl-2,3-dihydro-[1,4]dioxino[2,3-b]pyridin-6-yl)-4-((6-(méthylsulfonyl)-4-(2,2,6,6-tétraméthyltétrahydro-2H-pyran-4-yl)pyridin-2-yl)amino)pyridin-2-yl)acétamide ;
(R)-N-(4-((4-(2,2-dichlorocyclopropyl)-6-(méthylsulfonyl)pyridin-2-yl)amino)-5-(2,2-diméthyl-2,3-dihydro-[1,4]dioxino[2,3-b]pyridin-6-yl)pyridin-2-yl)acétamide ;
(S)-N-(4-((4-(2,2-dichlorocyclopropyl)-6-(méthylsulfonyl)pyridin-2-yl)amino)-5-(2,2-diméthyl-2,3-dihydro-[1,4]dioxino[2,3-b]pyridin-6-yl)pyridin-2-yl)acétamide ;
N-(4-((4-(5-cyanopyridazin-4-yl)-6-(méthylsulfonyl)pyridin-2-yl)amino)-5-(2,2-diméthyl-2,3-dihydro-[1,4]dioxino[2,3-b]pyridin-6-yl)pyridin-2-yl)acétamide ;
N-(4-((4-cyano-6'-(méthylsulfonyl)-[3,4'-bipyridin]-2'-yl)amino)-5-(2,2-diméthyl-2,3-dihydro-[1,4]dioxino[2,3-b]pyridin-6-yl)pyridin-2-yl)acétamide ;
N-(5-(2,2-diméthyl-2,3-dihydro-[1,4]dioxino[2,3-b]pyridin-6-yl)-4-((4-(5-fluoropyridazin-4-yl)-6-(méthylsulfonyl)pyridin-2-yl)amino)pyridin-2-yl)acétamide ;
N-(5-(2,2-diméthyl-2,3-dihydro-[1,4]dioxino[2,3-b]pyridin-6-yl)-4-((4-fluoro-6'-(méthylsulfonyl)-[3,4'-bipyridin]-2'-yl)amino)pyridin-2-yl)acétamide ;
N-(5-(2,2-diméthyl-2,3-dihydro-[1,4]dioxino[2,3-b]pyridin-6-yl)-4-((6'-(méthylsulfonyl)-[2,4'-bipyridin]-2'-yl)amino)pyridin-2-yl)acétamide ;
N-(5-(2,2-diméthyl-2,3-dihydro-[1,4]dioxino[2,3-b]pyridin-6-yl)-4-((6-(méthylsulfonyl)-4-(thiazol-4-yl)pyridin-2-yl)amino)pyridin-2-yl)acétamide ;
N-(4-((4-(1-cyanocyclopropy])-6-(méthylsulfonyl)pyridin-2-yl)amino)-5-(2,2-diméthyl-2,3-dihydro-[1,4]dioxino[2,3-b]pyridin-6-yl)pyridin-2-yl)acétamide ;
N-(4-((4-((3R)-3-cyano-2-azabicyclo[3.1.0]hexan-2-yl)-6-(méthylsulfonyl)pyridin-2-yl)amino)-5-(2,2-diméthyl-2,3-dihydro-[1,4]dioxino[2,3-b]pyridin-6-yl)pyridin-2-yl)acétamide ;
N-(4-((4-((3S)-3-cyano-2-azabicyclo[3.1.0]hexan-2-yl)-6-(méthylsulfonyl)pyridin-2-yl)amino)-5-(2,2-diméthyl-2,3-dihydro-[1,4]dioxino[2,3-b]pyridin-6-yl)pyridin-2-yl)acétamide ;
N-(5-(2,2-diméthyl-2,3-dihydro-[1,4]dioxino[2,3-b]pyridin-6-yl)-4-((4-(2-méthyl-2-morpholinopropoxy)-6-(méthylsulfonyl)pyridin-2-yl)amino)pyridin-2-yl)acétamide ;
N-(5-(2,2-diméthyl-2,3-dihydro-[1,4]dioxino[2,3-b]pyridin-6-yl)-4-((4-(2-méthoxy-2-méthylpropoxy)-6-(méthylsulfonyl)pyridin-2-yl)amino)pyridin-2-yl)acétamide ;
N-(5-(2,2-diméthyl-2,3-dihydro-[1,4]dioxino[2,3-b]pyridin-6-yl)-4-((4-(2-hydroxy-2-méthylpropoxy)-6-(méthylsulfonyl)pyridin-2-yl)amino)pyridin-2-yl)acétamide ;
N-(5-(2,2-diméthyl-2,3-dihydro-[1,4]dioxino[2,3-b]pyridin-6-yl)-4-((4-(3,3-diméthylmorpholino)-6-(méthylsulfonyl)pyridin-2-yl)amino)pyridin-2-yl)acétamide ;
N-(5-(2,2-diméthyl-2,3-dihydro-[1,4]dioxino[2,3-b]pyridin-6-yl)-4-((4-isopropyl-6'-(méthylsulfonyl)-[3,4'-bipyridin]-2'-yl)amino)pyridin-2-yl)acétamide ;
N-(5-(2,2-diméthyl-2,3-dihydro-[1,4]dioxino[2,3-b]pyridin-6-yl)-4-((4-(1-hydroxyéthyl)-6'-(méthylsulfonyl)-[3,4'-bipyridin]-2'-yl)amino)pyridin-2-yl)acétamide ;
N-(5-(2,2-diméthyl-2,3-dihydro-[1,4]dioxino[2,3-b]pyridin-6-yl)-4-((4-(1-méthoxyéthyl)-6'-(méthylsulfonyl)-[3,4'-bipyridin]-2'-yl)amino)pyridin-2-yl)acétamide ;
N-(5-(2,2-diméthyl-2,3 -dihydro-[1,4]dioxino[2,3-b]pyridin-6-yl)-4-((6-(méthylsulfonyl)-[1,3]dioxolo[4,5-c]pyridin-4-yl)amino)pyridin-2-yl)acétamide ;
N-(5-(2,2-diméthyl-2,3-dihydro-[1,4]dioxino[2,3-b]pyridin-6-yl)-4-((7-(méthylsulfonyl)-2,3-dihydro-[1,4]dioxino[2,3-c]pyridin-5-yl)amino)pyridin-2-yl)acétamide ;
N-(5-(2,2-diméthyl-2,3-dihydro-[1,4]dioxino[2,3-b]pyridin-6-yl)-4-((6-(méthylsulfonyl)-4-(trifluorométhyl)pyridin-2-yl)amino)pyridin-2-yl)acétamide ;
N-(4-((4-(difluorométhoxy)-6-(méthylsulfonyl)pyridin-2-yl)amino)-5-(2,2-diméthyl-2,3-dihydro-[1,4]dioxino[2,3-b]pyridin-6-yl)pyridin-2-yl)acétamide ;
N-(4-((4-cyclobutoxy-6-(méthylsulfonyl)pyridin-2-yl)amino)-5-(2,2-diméthyl-2,3-dihydro-[1,4]dioxino[2,3-b]pyridin-6-yl)pyridin-2-yl)acétamide ;
N-(5-(2,2-bis(méthyl-d3)-2,3-dihydro-[1,4]dioxino[2,3-b]pyridin-6-yl)-4-((6-(méthylsulfonyl)-4-(tétrahydro-2H-pyran-4-yl)pyridin-2-yl)amino)pyridin-2-yl)acétamide ;
N-(5-(2,2-bis(méthyl-d3)-2,3-dihydro-[1,4]dioxino[2,3-b]pyridin-6-yl)-4-((4-(méthoxy-d3)-6-(méthylsulfonyl)pyridin-2-yl)amino)pyridin-2-yl)acétamide ;
N-(5-(4,4-diméthyl-4,5-dihydrothiazol-2-yl)-4-((6-(méthylsulfonyl)pyridin-2-yl)amino)pyridin-2-yl)acétamide ;
N-(5-(4,4-diméthyl-4,5-dihydrothiazol-2-yl)-4-((6-(méthylsulfonyl)-4-(tétrahydro-2H-pyran-4-yl)pyridin-2-yl)amino)pyridin-2-yl)acétamide ;
N-(4-((4-isopropoxy-6-(méthylsulfonyl)pyridin-2-yl)amino)-5-(1-méthyl-1,3a,4,6,7,7a-hexahydropyrano[4,3-c]pyrazol-3-yl)pyridin-2-yl)acétamide ;
N-(5-(6,7-dihydro-4H-pyrano[4,3-d]thiazol-2-yl)-4-((4-méthyl-6-(méthylsulfonyl)pyridin-2-yl)amino)pyridin-2-yl)acétamide ;
N-(4-((4-isopropoxy-6-(méthylsulfonyl)pyridin-2-yl)amino)-5-(5-méthyl-4,5,6,7-tétrahydropyrazolo[1,5-a]pyrazin-2-yl)pyridin-2-yl)acétamide ;
N-(5-([1,3]dioxolo[4,5-c]pyridin-6-yl)-4-((4-méthyl-6-(méthylsulfonyl)pyridin-2-yl)amino)pyridin-2-yl)acétamide ;
N-(5-([1,3]dioxolo[4,5-c]pyridin-4-yl)-4-((4-méthyl-6-(méthylsulfonyl)pyridin-2-yl)amino)pyridin-2-yl)acétamide ;
N-(5-(benzo[d][1,3]dioxol-4-yl)-4-((4-méthyl-6-(méthylsulfonyl)pyridin-2-yl)amino)pyridin-2-yl)acétamide ;
N-(5-(5-méthyl-4,5,6,7-tétrahydrothiazolo[5,4-c]pyridin-2-yl)-4-((4-méthyl-6-(méthylsulfonyl)pyridin-2-yl)amino)pyridin-2-yl)acétamide ;
N-(5-(imidazo[1,2-a]pyrimidin-2-yl)-4-((4-méthyl-6-(méthylsulfonyl)pyridin-2-yl)amino)pyridin-2-yl)acétamide ;
N-(5-(1-méthyl-1,4-dihydrochroméno[4,3-c]pyrazol-6-yl)-4-((4-méthyl-6-(méthylsulfonyl)pyridin-2-yl)amino)pyridin-2-yl)acétamide ;
N-(5-(imidazo[2',1':2,3}thiazolo[5,4-b]pyridin-7-yl)-4-((4-méthyl-6-(méthylsulfonyl)pyridin-2-yl)amino)pyridin-2-yl)acétamide ;
N-(5-(1-méthyl-1,4-dihydropyrazolo[3',4':4,5]pyrano[2,3-b]pyridin-7-yl)-4-((4-méthyl-6-(méthylsulfonyl)pyridin-2-yl)amino)pyridin-2-yl)acétamide ;
N-(4'-((4,4-dioxido-2,3-dihydro-[1,4]oxathiino[3,2-b]pyridin-6-yl)amino)-[2,3'-bipyridin]-6'-yl)acétamide ;
N-(4-((4,4-dioxido-2,3-dihydro-[1,4]oxathiino[3,2-b]pyridin-6-yl)amino)-5-(1-méthyl-1H-pyrazol-3-yl)pyridin-2-yl)acétamide ;
N-(5-(imidazo[1,2-b]pyridazin-6-yl)-4-((4-méthyl-6-(méthylsulfonyl)pyridin-2-yl)amino)pyridin-2-yl)acétamide ;
N-(5-(1-méthyl-1H-pyrazol-3-yl)-4-((7-(méthylsulfonyl)-3,4-dihydro-2H-pyrano[3,2-c]pyridin-5-yl)amino)pyridin-2-yl)acétamide ;
N-(4-((5,5-dioxido-3,4-dihydro-2H-[1,4]oxathiépino[3,2-b]pyridin-7-yl)amino)-5-(1-méthyl-1H-pyrazol-3-yl)pyridin-2-yl)acétamide ;
N-(4-((4-(2-hydroxypropan-2-yl)-6-(méthylsulfonyl)pyridin-2-yl)amino)-5-(1-méthyl-1H-pyrazol-3-yl)pyridin-2-yl)acétamide ;
N-(5-(1-méthyl-1H-pyrazol-3-yl)-4-((6-(méthylsulfonyl)-4-(tétrahydrofuran-3-yl)pyridin-2-yl)amino)pyridin-2-yl)acétamide ;
N-(4-((4-(3-(hydroxyméthyl)tétrahydrofuran-3-yl)-6-(méthylsulfonyl)pyridin-2-yl)amino)-5-(1-méthyl-1H-pyrazol-3-yl)pyridin-2-yl)acétamide ;
N-(4-((4-(1-hydroxycyclopropyl)-6-(méthylsulfonyl)pyridin-2-yl)amino)-5-(1-méthyl-1H-pyrazol-3-yl)pyridin-2-yl)acétamide ;
N-(4-((4-(2-hydroxy-2-méthylpropoxy)-6-(méthylsulfonyl)pyridin-2-yl)amino)-5-(1-méthyl-1H-pyrazol-3-yl)pyridin-2-yl)acétamide ;
N-(4-((4-(-hydroxy-2,2-diméthylpropoxy)-6-(méthylsulfonyl)pyridin-2-yl)amino)-5-(1-méthyl-1H-pyrazol-3-yl)pyridin-2-yl)acétamide ;
N-(5-(imidazo[1,5-b]pyridazin-2-yl)-4-((4-méthyl-6-(méthylsulfonyl)pyridin-2-yl)amino)pyridin-2-yl)acétamide ; N-(4-((4-(3-hydroxytétrahydrofuran-3-yl)-6-(méthylsulfonyl)pyridin-2-yl)amino)-5-(1-méthyl-1H-pyrazol-3-yl)pyridin-2-yl)acétamide ;
N-(5-(1-méthyl-1H-pyrazol-3-yl)-4-((4-(1-méthyl-1H-pyrazol-3-yl)-6-(méthylsulfonyl)pyridin-2-yl)amino)pyridin-2-yl)acétamide ;
N-(4-((4-((1-hydroxycyclopropyl)méthoxy)-6-(méthylsulfonyl)pyridin-2-yl)amino)-5-(1-méthyl-1H-pyrazol-3-yl)pyridin-2-yl)acétamide ;
N-(4-((4-((1-méthoxycyclopropyl)méthoxy)-6-(méthylsulfonyl)pyridin-2-yl)amino)-5-(1-méthyl-1H-pyrazol-3-yl)pyridin-2-yl)acétamide ;
N-(5-(1-méthyl-1H-pyrazol-3-yl)-4-((6-(méthylsulfonyl)-4-morpholinopyridin-2-yl)amino)pyridin-2-yl)acétamide ;
N-(5-(benzo[d]thiazol-2-yl)-4-((4-(3-(hydroxyméthyl)tétrahydrofuran-3-yl)-6-(méthylsulfonyl)pyridin-2-yl)amino)pyridin-2-yl)acétamide ;
N-(4-((1,1-dioxido-3,4-dihydro-2H-thiopyrano[2,3-b]pyridin-7-yl)amino)-5-(1-méthyl-1H-pyrazol-3-yl)pyridin-2-yl)acétamide ;
N-(5-(1-méthyl-1H-pyrazol-3-yl)-4-((7-(méthylsulfonyl)-3,4-dihydro-2H-pyrano[3,2-c]pyridin-5-yl)amino)pyridin-2-yl)acétamide ;
N-(5-(1-méthyl- 1H-pyrazol-3-yl)-4-((6-(méthylsulfonyl)-[1,3]dioxolo[4,5-c]pyridin-4-yl)amino)pyridin-2-yl)acétamide ;
N-(5-(1-méthyl-1H-pyrazol-3-yl)-4-((6-(méthylsulfonyl)imidazo[1,2-a]pyrazin-8-yl)amino)pyridin-2-yl)acétamide ;
N-(5-(1-méthyl-1H-pyrazol-3-yl)-4-((5-(méthylsulfonyl)-3,4-dihydro-2H-pyrano[3,2-c]pyridin-7-yl)amino)pyridin-2-yl)acétamide ;
N-(5-(2,6-diméthylmorpholino)-4'-((4-(2-hydroxypropan-2-yl)-6-(méthylsulfonyl)pyridin-2-yl)amino)-[2,3'-bipyridin]-6'-yl)acétamide ;
N-(5-(2,6-diméthylmorpholino)-4'-((6-(méthylsulfonyl)-4-(tétrahydrofuran-3-yl)pyridin-2-yl)amino)-[2,3'-bipyridin]-6'-yl)acétamide ;
N-(5-(2,6-diméthylmorpholino)-4'-((4-(3-(hydroxyméthyl)tétrahydrofuran-3-yl)-6-(méthylsulfonyl)pyridin-2-yl)amino)-[2,3'-bipyridin]-6'-yl)acétamide ;
N-(5-(2,6-diméthylmorpholino)-4'-((4-(1-hydroxycyclopropyl)-6-(méthylsulfonyl)pyridin-2-yl)amino)-[2,3'-bipyridin]-6'-yl)acétamide ;
N-(5-(2,6-diméthylmorpholino)-4'-((4-(2-hydroxy-2-méthylpropoxy)-6-(méthylsulfonyl)pyridin-2-yl)amino)-[2,3'-bipyridin]-6'-yl)acétamide ;
N-(5-(2,6-diméthylmorpholino)-4'-((4-3-hydroxy-2,2-diméthylpropoxy)-6-(méthylsulfonyl)pyridin-2-yl)amino)-[2,3'-bipyridin]-6'-yl)acétamide ;
N-(5-(2,6-diméthylmorpholino)-4'-((4-(3-méthoxytétrahydrofuran-3-yl)-6-(méthylsulfonyl)pyridin-2-yl)amino)-[2,3'-bipyridin]-6'-yl)acétamide ;
N-(5-(2,6-diméthylmorpholino)-4'-((4-(3-hydroxytétrahydrofuran-3-yl)-6-(méthylsulfonyl)pyridin-2-yl)amino)-[2,3'-bipyridin]-6'-yl)acétamide ;
N-(5-(2,6-diméthylmorpholino)-4'-((4-(1-méthyl-1H-pyrazol-3-yl)-6-(méthylsulfonyl)pyridin-2-yl)amino)-[2,3'-bipyridin]-6'-yl)acétamide ;
N-(5-(2,6-diméthylmorpholino)-4'-((4-((1-hydroxycyclopropyl)méthoxy)-6-(méthylsulfonyl)pyridin-2-yl)amino)-[2,3'-bipyridin]-6'-yl)acétamide ;
N-(5-(2,6-diméthylmorpholino)-4'-((4-((1-méthoxycyclopropyl)méthoxy)-6-(méthylsulfonyl)pyridin-2-yl)amino)-[2,3'-bipyridin]-6'-yl)acétamide ;
N-(5-(2,6-diméthylmorpholino)-4'-((6-(méthylsulfonyl)-4-morpholinopyridin-2-yl)amino)-[2,3'-bipyridin]-6'-yl)acétamide ;
N-(5-(2,6-diméthylmorpholino)-4'-((5,5-dioxido-3,4-dihydro-2H-[1,4]oxathiepino[3,2-b]pyridin-7-yl)amino)-[2,3'-bipyridin]-6'-yl)acétamide ;
N-(5-(2,6-diméthylmorpholino)-4'-((1,1-dioxido-3,4-dihydro-2H-thiopyrano[2,3-b]pyridin-7-yl)amino)-[2,3'-bipyridin]-6'-yl)acétamide ;
N-(5-(2,6-diméthylmorpholino)-4'-((7-(méthylsulfonyl)-3,4-dihydro-2H-pyrano[3,2-c]pyridin-5-yl)amino)-[2,3'-bipyridin]-6'-yl)acétamide ;
N-(5-(2,6-diméthylmorpholino)-4'-((6-(méthylsulfonyl)-[1,3]dioxolo[4,5-c]pyridin-4-yl)amino)-[2,3'-bipyridin]-6'-yl)acétamide ;
N-(5-(2,6-diméthylmorpholino)-4'-((6-(méthylsulfonyl)imidazo[1,2-a]pyrazin-8-yl)amino)-[2,3'-bipyridin]-6'-yl)acétamide ;
N-(5-(2,6-diméthylmorpholino)-4'-((5-(méthylsulfonyl)-3,4-dihydro-2H-pyrano[3,2-c]pyridin-7-yl)amino)-[2,3'-bipyridin]-6'-yl)acétamide ;
N-(5-(6,7-dihydro-4H-pyrano[4,3-d]thiazol-2-yl)-4-((4-(3-(hydroxyméthyl)tétrahydrofuran-3-yl)-6-(méthylsulfonyl)pyridin-2-yl)amino)pyridin-2-yl)acétamide ;
N-(5-(benzo[d][1,3]dioxol-4-yl)-4-((4-(3-(hydroxyméthyl)tétrahydrofuran-3-yl)-6-(méthylsulfonyl)pyridin-2-yl)amino)pyridin-2-yl)acétamide ;
N-(5-(benzo[d]isoxazol-3-yl)-4-((4-(3-(hydroxyméthyl)tétrahydrofuran-3-yl)-6-(méthylsulfonyl)pyridin-2-yl)amino)pyridin-2-yl)acétamide ;
N-(5-(benzo[d]isothiazol-3-yl)-4-((4-(3-(hydroxyméthyl)tétrahydrofuran-3-yl)-6-(méthylsulfonyl)pyridin-2-yl)amino)pyridin-2-yl)acétamide ;
N-(5-(benzo[b]thiophén-2-yl)-4-((4-(3-(hydroxyméthyl)tétrahydrofuran-3-yl)-6-(méthylsulfonyl)pyridin-2-yl)amino)pyridin-2-yl)acétamide ;
N-(4-((4-(3-(hydroxyméthyl)tétrahydrofuran-3-yl)-6-(méthylsulfonyl)pyridin-2-yl)amino)-5-(imidazo[1,2-a]pyrimidin-2-yl)pyridin-2-yl)acétamide ;
N-(4-((4-(3-(hydroxyméthyl)tétrahydrofuran-3-yl)-6-(méthylsulfonyl)pyridin-2-yl)amino)-5-(1-méthyl-1,4-dihydrochroméno[4,3-c]pyrazol-6-yl)pyridin-2-yl)acétamide ;
N-(4-((4-(3-(hydroxyméthyl)tétrahydrofuran-3-yl)-6-(méthylsulfonyl)pyridin-2-yl)amino)-5-(imidazo[2',1':2,3]thiazolo[5,4-b]pyridin-7-yl)pyridin-2-yl)acétamide ;
N-(4-((4-(3-(hydroxyméthyl)tétrahydrofuran-3-yl)-6-(méthylsulfonyl)pyridin-2-yl)amino)-5-(1-méthyl-1,4-dihydropyrazolo[3',4':4,5]pyrano[2,3-b]pyridin-7-yl)pyridin-2-yl)acétamide ;
N-(4-((4-(3-(hydroxyméthyl)tétrahydrofuran-3-yl)-6-(méthylsulfonyl)pyridin-2-yl)amino)-5-(6-oxo-1,6-dihydropyridazin-3-yl)pyridin-2-yl)acétamide ;
N-(4-((4-(3-(hydroxyméthyl)tétrahydrofuran-3-yl)-6-(méthylsulfonyl)pyridin-2-yl)amino)-5-(6-oxopyridazin-1(6H)-yl)pyridin-2-yl)acétamide ;
N-(5-(6-((2S,6R)-2,6-diméthylmorpholino)pyridazin-3-yl)-4-((4-(3-(hydroxyméthyl)tétrahydrofuran-3-yl)-6-(méthylsulfonyl)pyridin-2-yl)amino)pyridin-2-yl)acétamide ;
N-(4-((4-(3-(hydroxyméthyl)tétrahydrofuran-3-yl)-6-(méthylsulfonyl)pyridin-2-yl)amino)-5-(3-oxo-8,9-dihydropyrano[4,3,2-de]phthalazin-2(3H)-yl)pyridin-2-yl)acétamide ;
N-(4-((4-(3-(hydroxyméthyl)tétrahydrofuran-3-yl)-6-(méthylsulfonyl)pyridin-2-yl)amino)-5-(7-méthyl-3-oxo-8,9-dihydro-3H-pyrido[4,3,2-de]phthalazin-2(7H)-yl)pyridin-2-yl)acétamide ;
N-(4'-((4-(3-(hydroxyméthyl)tétrahydrofuran-3-yl)-6-(méthylsulfonyl)pyridin-2-yl)amino)-5-(1-oxoisoindolin-2-yl)-[2,3'-bipyridin]-6'-yl)acétamide ;
N-(5-(2,3-dihydropyrazolo[5,1-b]oxazol-6-yl)-4-((4-(3-(hydroxyméthyl)tétrahydrofuran-3-yl)-6-(méthylsulfonyl)pyridin-2-yl)amino)pyridin-2-yl)acétamide ;
N-(5-(2,2-diméthyl-2,3-dihydropyrazolo[5,1-b]oxazol-6-yl)-4-((4-(3-(hydroxyméthyl)tétrahydrofuran-3-yl)-6-(méthylsulfonyl)pyridin-2-yl)amino)pyridin-2-yl)acétamide ;
N-(4-((4-(3-(hydroxyméthyl)tétrahydrofuran-3-yl)-6-(méthylsulfonyl)pyridin-2-yl)amino)-5-(5'H,7'H-spiro[cyclopropane-1,6'-pyrazolo[5,1-b][1,3]oxazin]-2'-yl)pyridin-2-yl)acétamide ;
N-(5-(1H-imidazol-2-yl)-4-((4-méthyl-6-(méthylsulfonyl)pyridin-2-yl)amino)pyridin-2-yl)acétamide ;
N-(5-(4-(2-hydroxypropan-2-yl)furan-2-yl)-4-((4-méthyl-6-(méthylsulfonyl)pyridin-2-yl)amino)pyridin-2-yl)acétamide ;
N-(5-(furan-2-yl)-4-((4-(3-méthoxytétrahydrofuran-3-yl)-6-(méthylsulfonyl)pyridin-2-yl)amino)pyridin-2-yl)acétamide ;
N-(5-(1H-benzo[d]imidazol-2-yl)-4-((4-méthyl-6-(méthylsulfonyl)pyridin-2-yl)amino)pyridin-2-yl)acétamide ;
N-(3-fluoro-5-(2-hydroxypropan-2-yl)-4'-((4-méthyl-6-(méthylsulfonyl)pyridin-2-yl)amino)-[2,3'-bipyridin]-6'-yl)acétamide ;
N-(3-fluoro-5-(2-hydroxypropan-2-yl)-4'-((4-méthyl-6-(méthylsulfonyl)pyridin-2-yl)amino)-[2,3'-bipyridin]-6'-yl)acétamide ;
N-(5-(2-hydroxypropan-2-yl)-4-méthyl-4'-((4-méthyl-6-(méthylsulfonyl)pyridin-2-yl)amino)-[2,3'-bipyridin]-6'-yl)acétamide ;
N-(5-(2,6-diméthylmorpholino)-4'-((6-(méthylsulfonyl)-4-((tétrahydrofuran-3-yl)méthoxy)pyridin-2-yl)amino)-[2,3'-bipyridin]-6'-yl)acétamide ;
N-(5-(2,6-diméthylmorpholino)-4'-((6-(méthylsulfonyl)-4-(((tétrahydrofuran-3-yl)oxy)méthyl)pyridin-2-yl)amino)-[2,3'-bipyridin]-6'-yl)acétamide ;
N-(5-(3-méthoxy-1-méthyl-1H-pyrazol-4-yl)-4-((4-méthyl-6-(méthylsulfonyl)pyridin-2-yl)amino)pyridin-2-yl)acétamide ;
N-(4-((4-(3-(hydroxyméthyl)tétrahydrofuran-3-yl)-6-(méthylsulfonyl)pyridin-2-yl)amino)-5-(3-méthoxy-1-méthyl-1H-pyrazol-4-yl)pyridin-2-yl)acétamide ;
N-(5-(6,7-dihydro-4H-furo[3,2-c]pyran-2-yl)-4-((4-méthyl-6-(méthylsulfonyl)pyridin-2-yl)amino)pyridin-2-yl)acétamide ;
N-(5-(5-méthyl-4,5,6,7-tétrahydrofuro[3,2-c]pyridin-2-yl)-4-((4-méthyl-6-(méthylsulfonyl)pyridin-2-yl)amino)pyridin-2-yl)acétamide ;
N-(5-(benzo[d]oxazol-2-yl)-4-((4-(3-(hydroxyméthyl)tétrahydrofuran-3-yl)-6-(méthylsulfonyl)pyridin-2-yl)amino)pyridin-2-yl)acétamide ;
N-(5-(2,2-diméthyl-3-oxo-3,4-dihydro-2H-pyrido[3,2-b][1,4]oxazin-6-yl)-4-((4-méthyl-6-(méthylsulfonyl)pyridin-2-yl)amino)pyridin-2-yl)acétamide ;
N-(4-((4-méthyl-6-(méthylsulfonyl)pyridin-2-yl)amino)-5-(pyrazolo[1,5-a]pyrazin-2-yl)pyridin-2-yl)acétamide ;
N-(4-((4-(2-méthoxyéthoxy)-6-(méthylsulfonyl)pyridin-2-yl)amino)-5-(pyrazolo[1,5-a]pyrazin-2-yl)pyridin-2-yl)acétamide ;
N-(4-((4-méthyl-6-(méthylsulfonyl)pyridin-2-yl)amino)-5-(pyrazolo[1,5-c]pyrimidin-2-yl)pyridin-2-yl)acétamide ;
N-(4-((4-(2-méthoxyéthoxy)-6-(méthylsulfonyl)pyridin-2-yl)amino)-5-(pyrazolo[1,5-c]pyrimidin-2-yl)pyridin-2-yl)acétamide ;
N-(4-((4-méthyl-6-(méthylsulfonyl)pyridin-2-yl)amino)-5-(pyrazolo[1,5-a]pyrimidin-2-yl)pyridin-2-yl)acétamide ;
N-(4-((4-(2-méthoxyéthoxy)-6-(méthylsulfonyl)pyridin-2-yl)amino)-5-(pyrazolo[1,5-a]pyrimidin-2-yl)pyridin-2-yl)acétamide ;
N-(4-((4-méthyl-6-(méthylsulfonyl)pyridin-2-yl)amino)-5-(pyrazolo[1,5-a]pyrimidin-5-yl)pyridin-2-yl)acétamide ;
N-(4-((4-(2-méthoxyéthoxy)-6-(méthylsulfonyl)pyridin-2-yl)amino)-5-(pyrazolo[1,5-a]pyrimidin-5-yl)pyridin-2-yl)acétamide ; ou
N-(5-(2,2-diméthyl-2,3-dihydro-1H-pyrido[2,3-b][1,4]oxazin-6-yl)-4-((6-(méthylsulfonyl)-4-(tétrahydro-2H-pyran-4-yl)pyridin-2-yl)amino)pyridin-2-yl)acétamide.

14. Composition pharmaceutique comprenant un ou plusieurs composés selon l'une quelconque des revendications 1 à 13, ou un stéréoisomère ou un sel pharmaceutiquement acceptable de celui-ci, et un excipient pharmaceutiquement acceptable.

15. Composé selon l'une quelconque des revendications 1 à 13 pour une utilisation dans le traitement d'une maladie inflammatoire ou auto-immune.
